(19) Europäisches Patentamt / European Patent Office / Office européen des brevets

(11) **EP 3 901 147 B1**

(12) **EUROPEAN PATENT SPECIFICATION**


(45) Date of publication and mention of the grant of the patent:
**22.11.2023 Bulletin 2023/47**

(21) Application number: **20745487.7**

(22) Date of filing: **17.01.2020**

(51) International Patent Classification (IPC):
***C07D 401/12*** (2006.01) ***C07D 401/14*** (2006.01)
***C07D 405/14*** (2006.01) ***C07D 413/12*** (2006.01)
***C07D 417/12*** (2006.01) ***A61K 31/415*** (2006.01)
***A61P 17/06*** (2006.01) ***A61P 35/00*** (2006.01)
***A61P 37/00*** (2006.01)

(52) Cooperative Patent Classification (CPC):
**C07D 401/12; A61K 31/415; A61P 17/06; A61P 35/00; A61P 37/00; C07D 401/14; C07D 405/14; C07D 413/12; C07D 417/12**

(86) International application number:
**PCT/CN2020/072738**

(87) International publication number:
**WO 2020/151589 (30.07.2020 Gazette 2020/31)**


(54) **ACYLAMINO BRIDGED HETEROCYCLIC COMPOUND, AND COMPOSITION AND APPLICATION THEREOF**

ACYLAMINOVERBRÜCKTE HETEROCYCLISCHE VERBINDUNG SOWIE ZUSAMMENSETZUNG UND DEREN VERWENDUNG

COMPOSÉ HÉTÉROCYCLIQUE À PONT ACYLAMINO, COMPOSITION ET UTILISATION ASSOCIÉES


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2019 CN 201910071476**
**10.01.2020 CN 202010027582**

(43) Date of publication of application:
**27.10.2021 Bulletin 2021/43**



(73) Proprietor: **Beijing Scitech-MQ Pharmaceuticals Limited**
**Beijing 101320 (CN)**

(72) Inventors:
- **ZHANG, Qiang**
  **Beijing 101320 (CN)**
- **LIU, Yansheng**
  **Beijing 101320 (CN)**
- **LI, Xingfu**
  **Beijing 101320 (CN)**
- **ZHANG, Shaohua**
  **Beijing 101320 (CN)**
- **HU, Chenming**
  **Beijing 101320 (CN)**

(74) Representative: **Maiwald GmbH**
**Elisenhof**
**Elisenstraße 3**
**80335 München (DE)**



(56) References cited:
**WO-A1-2005/040152** **WO-A1-2014/125444**
**WO-A1-2017/109724** **WO-A1-2018/044783**
**WO-A1-2018/109097** **WO-A2-2011/025706**
**WO-A2-2017/136727**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 3 901 147 B1

## Description

### TECHNICAL FIELD

The present disclosure belongs to the field of medicinal chemistry, and particularly relates to acylamino bridged heterocyclic compounds, or diastereomers, enantiomers, solvates, or pharmaceutically acceptable salts thereof, and to pharmaceutical compositions thereof, and their use in the preparation of medicaments for the treatment of autoimmune diseases, tumors and neurodegenerative diseases related to receptor-interacting protein 1 kinase (RIPK1).

### BACKGROUND OF THE INVENTION

In the early days, it was thought that there are two main ways of cell death, i.e., apoptosis and necrosis. Apoptosis is the autonomous and orderly death of cells controlled by genes in order to maintain the stability of the body's internal environment. It plays an important role in the evolution of the organism, the stability of the internal environment, and the development of the system. Cell necrosis is a pathological process in which cells are affected by physical, chemical and other environmental factors, such as mechanical damage, poisons, microorganisms, radiation, etc., causing cell death. In 2005, Degterev A et al. first discovered and reported an orderly cell necrosis process regulated by a series of biochemical molecules, and named it Necroptosis (also known as programmed necrosis). This process is a kind of programmed cell necrosis produced by stimulating death receptors with TNF-$\alpha$, FasL or TRAIL. Morphologically, it is manifested as cell swelling, cell volume increasing, organelle dysfunction, damage to cell membrane integrity, release of cell contents, and mass production of ROS.

Receptor-interacting protein 1 kinase (RIPK1) belongs to the TKL family of serine/threonine protein kinases. Members of the RIPK serine/threonine kinase family have the same N-terminal kinase domain, but different binding domains. Studies have shown that receptor-interacting protein 1 can regulate the process of cell apoptosis. The death domain of RIPK-1 binds to death receptors such as TNFR1, Fas, TRAILR1, TRAILR2, etc., and they can also bind to other proteins containing death domains, such as TRADD, FADD, etc. Binding with the latter is a necessary condition for activating caspase-8 and inducing apoptosis. The intermediate structure of RIPK-1 is the RIPK isotype interaction target, through which RIPK-1 can interact with RIPK-3. Kelliher et al. found that mice with congenital defects of RIPK-1 died less than 3 days after birth due to a large number of cell apoptosis, which indicates that RIPK-1 is also involved in regulating cell apoptosis. In addition, cells with congenital RIPK-1 deficiency are quite sensitive to TNF-induced cell death, perhaps because such cells cannot effectively activate NF-kB.

On the other hand, studies have also shown that RIPK-1 and RIPK-3 are also involved in the process of cell necroptosis. In most cell types, death receptors TNFR1, Fas and TRAILR mediate cell apoptosis. Activating TNFR1 can trigger the ubiquitination of RIPK-1 through cIAP1 and cIAP2, and the ubiquitinated RIPK-1 determines whether the cell will continue to survive or die. When the apoptosis pathway is blocked by the pan-caspase inhibitor z-VAD-fmk, cell death will move toward to necroptosis. In this process, the activity of RIPK-1 is a key factor, which is regulated by FasL, TNF and TRAIL death receptors.

Recent studies have shown that the process of necroptosis is related to a variety of diseases, including tumors, autoimmune diseases, degenerative diseases, inflammatory diseases and the like. Based on this, it can be known that RIP family kinases are closely related to the occurrence of tumors, autoimmune diseases, degenerative diseases, inflammatory diseases and other diseases.

For example, in the study of Alzheimer's disease, Claudia Balducci et al. found that activated microglia plays an important role in the evolution of Alzheimer's disease (Pharmacological Research. 2018; 130: 402-413). At the same time, microglias highly expresses RIPK-1, and RIPK-1 inhibitors can protect from A$\beta$-induced neuronal death in vitro and reduce the proliferation of microglias. Moreover, in Alzheimer-like mouse brains, RIPK-1 inhibitors can improve their learning and memory abilities. In addition to Alzheimer's disease, RIPK-1 inhibitors are expected to be used in a variety of other neurodegenerative diseases including Parkinson's disease, amyotrophic lateral sclerosis, Huntington disease, etc.

At present, there are not many studies on RIPK-1 inhibitors, and only a small number of studies have entered the clinical stage. There is an urgent need for more research and development of drugs based on RIPK-1 inhibitors.

The present disclosure provides a class of acylamino bridged heterocyclic compounds, which exhibit good RIPK-1 inhibitory activity and can be used as RIPK-1 inhibitors in the preparation of a medicament for the treatment of tumors, autoimmune diseases, degenerative diseases, and inflammatory diseases.

There are RIPK1 inhibitors disclosed in the WO 2018/109097, WO 2014/125444, WO 2017/109724, WO 2017/136727, WO 2018/044783, WO 2011/025706, and WO 2005/040152.

## SUMMARY OF THE INVENTION

The compound represented by formula (I) provided by the present disclosure, or pharmaceutically acceptable salts, diastereomers, enantiomers, hydrates, or solvates thereof, can be used to prepare medicaments for the treatment or prevention of diseases related to RIPK1.

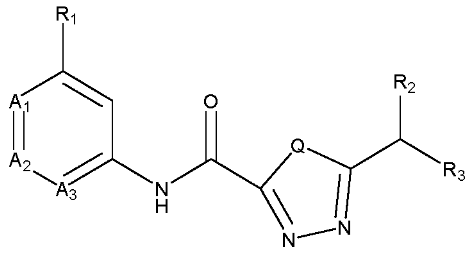

formula (I)

In formula (I),

Q is NH, O or S;

$A_1$, $A_2$, and $A_3$ are each independently selected from N or $CR_4$ and at least one of $A_1$, $A_2$, and $A_3$ is N, $R_4$ is H, F, Cl or methyl;

$R_1$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or aryl or heteroaryl, which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$, -SO-$R_5$, -CO-$R_5$, -CONH-$R_5$, -NHCO-$R_5$, -R'-COO-R", -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl and/or $C_1$-$C_6$ alkoxy, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms, the 4- to 8-membered heteroalicyclic group is a 4- to 8-membered heteroalicyclic group containing 1-2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is substituted with 1 to 3 susbtituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

R' is $C_2$-$C_6$ alkenylene, or $C_1$-$C_6$ alkylene,

R" is hydrogen, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_2$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_7$-$C_{12}$ bridged cyclyl, which is unsubstituted or substituted with 1 to 3 substituents selected from the group

consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_1$-$C_{10}$ alkyl, which is unsubstituted or substituted with 1 to 3 substituents selected from the group consisting of $C_1$-$C_6$ alkoxy, $C_1$-$C_6$ alkylthio, $C_1$-$C_3$ acyl, hydroxyl, halogen, cyano, -$CONH_2$, $C_3$-$C_8$ cycloalkyl, and -$NR^cR^d$,

or -$(CH_2)$n-$R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, $C_3$-$C_6$ cycloalkyl, phenyl, naphthyl, $C_2$-$C_6$ alkynyl, $C_2$-$C_6$ alkenyl, and -$NR^cR^d$, wherein n is an integer from 0 to 3,

or -$(CH_2)$m-$R^f$, wherein $R^f$ is 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$, m is an integer from 0 to 3,

the 4- to 8-membered heteroalicyclic group is a 4- to 8-membered heteroalicyclic group containing 1 to 2 atoms selected from N, O, or S as ring atoms,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms,

$R^c$ and $R^d$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_3$ is hydrogen, $C_1$-$C_3$ alkyl, hydroxyl, halogen, trifluoromethyl, or cyano.

In some embodiments, alternatively, Q is NH, O or S;

$A_1$, $A_2$, and $A_3$ are each independently selected from N or $CR_4$ and at least one of $A_1$, $A_2$, and $A_3$ is N, $R_4$ is H, F, Cl or methyl;

$R_1$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or aryl or heteroaryl, which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$, -SO-$R_5$, -CO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms, the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is substituted with 1 to 3 susbtituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_2$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_7$-$C_{12}$ bridged cyclyl, which is unsubstituted or substituted with 1 to 3 substituents selected from the group

consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_1$-$C_{10}$ alkyl, which is unsubstituted or substituted with 1 to 3 substituents selected from the group consisting of $C_1$-$C_6$ alkoxy, $C_1$-$C_6$ alkylthio, $C_1$-$C_3$ acyl, hydroxyl, halogen, cyano, -$CONH_2$, $C_3$-$C_8$ cycloalkyl, and -$NR^cR^d$,

or -$(CH_2)$n-$R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, $C_2$-$C_6$ alkynyl, $C_2$-$C_6$ alkenyl, and -$NR^cR^d$, wherein n is an integer from 0 to 3,

or -$(CH_2)$m-$R^f$, wherein $R^f$ is 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$, m is an integer from 0 to 3,

the 4- to 8-membered heteroalicyclic group is a 4- to 8-membered heteroalicyclic group containing 1 to 2 atoms selected from N, O, or S as ring atoms,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms,

$R^c$ and $R^d$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_3$ is hydrogen, $C_1$-$C_3$ alkyl, hydroxyl, halogen, trifluoromethyl, or cyano.

In some embodiments, alternatively, $A_3$ is N, $A_1$, and $A_2$ are each independently selected from N or $CR_4$, $R_4$ is H, F, Cl or methyl; still alternatively, $A_3$ is N, $A_1$, and $A_2$ are each independently CH.

In some embodiments, alternatively, $A_3$ is N, $A_1$, $A_2$ are each independently selected from N or $CR_4$, $R_4$ is H or F.

In some embodiments, still alternatively, Q is NH.

In some embodiments, alternatively, $R_1$ is $C_3$-$C_8$ cycloalkyl, 4- to 8-membered heteroalicyclic group, or aryl or heteroaryl which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is phenyl, naphthyl,

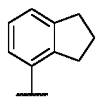 、 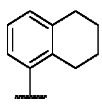 ,

the heteroaryl group is pyrrolyl, furyl, pyridyl, thienyl, imidazolyl, thiazolyl, isothiazolyl, indazolyl, indolyl, indazolyl, isoindolyl, indolinyl, isoindolinyl, isoquinolinyl, indolizinyl, isoxazolyl, 1,5-naphthyridinyl, 1,6-naphthyridinonyl, oxadiazolyl, oxazolyl, 1-phenyl-1H-pyrrolyl, phenazinyl, phenothiazinyl, phenoxazinyl, phthalazinyl, pteridinyl, purinyl, pyranyl, pyrazolyl, pyrazolo[3,4-d]pyrimidinyl, pyridyl, pyrido[3,2-d]pyrimidinyl, pyrido[3,4-d]pyrimidinyl, pyrazinyl, pyrimidinyl, pyridazinyl, quinazolinyl, quinoxalinyl, quinolinyl, isoquinolinyl,

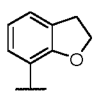 、 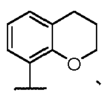 、 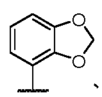 、 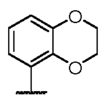 、 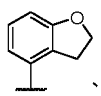 、 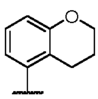 ,

the 4- to 8-membered heteroalicyclic group is a 4-to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -CO-$R_5$, -$SO_2$-$R_5$, -SO-$R_5$, $C_2$-$C_6$ alkynyl, $C_2$-$C_6$ alkenyl, $C_1$-$C_4$

alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, or $C_1$-$C_6$ alkylthio,
$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl.

In some embodiments, alternatively, $R_1$ is cyclopropyl, cyclobutyl, cyclopentyl, cyclohexyl, oxetan-3-yl, tetrahydrofuran-3-yl, tetrahydropyran-4-yl, tetrahydropyran-3-yl, pyrrolidinyl, piperidinyl, piperazinyl, morpholinyl, thiomorpholinyl, or aryl or heteroaryl which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$, -SO-$R_5$, -CO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is phenyl,

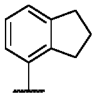 、 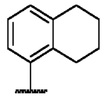 ,

the heteroaryl group is pyrazolyl, pyridyl, pyrimidinyl, thiazolyl, oxazolyl,

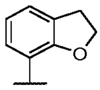 、 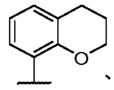 、 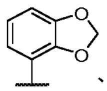 、 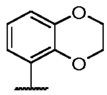 、 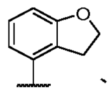 、 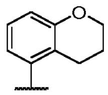 ,

the 4- to 8-membered heteroalicyclic group is a 4- to 8-membered heteroalicyclic group containing 1 to 2 atoms selected from N, O, or S as ring atoms,
$R_5$ is hydrogen, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,
$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, or $C_1$-$C_6$ alkylthio,
$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl.

In some embodiments, alternatively, $R_1$ is

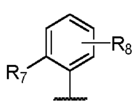 , 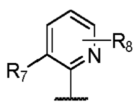 , 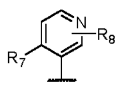 , 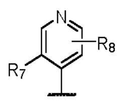 ,

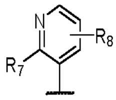 , 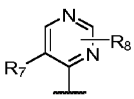 , 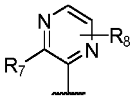 , or 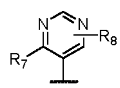 ,

$R_7$ is hydrogen, $C_1$-$C_4$ alkyl, $C_1$-$C_3$ alkoxy, halogen, hydroxyl, trifluoromethyl, trifluoromethoxy, cyano, $C_2$-$C_4$ alkynyl, $C_2$-$C_4$ alkenyl, $C_3$-$C_4$ cycloalkyl, or $C_1$-$C_3$ acyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, hydroxyl $C_1$-$C_{10}$ alkyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or -O-$R_6$,

the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl.

In some embodiments, alternatively, $R_1$ is:

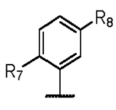 , 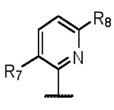 , 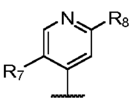 , 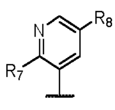 , 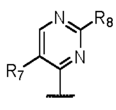 , or 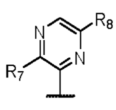 ,

$R_7$ is hydrogen, methyl, ethyl, propyl, isopropyl, methoxy, ethoxy, propoxy, isopropoxy, fluorine, chlorine, hydroxyl, trifluoromethyl, trifluoromethoxy, cyano, ethynyl, propynyl, vinyl, propenyl, cyclopropyl, cyclobutyl, formyl, or acetyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or -O-$R_6$,

the 4- to 8-membered heteroalicyclic group is heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl.

In some embodiments, alternatively, $R_5$ is hydrogen, hydroxyl, $C_1$-$C_4$ alkyl, $C_1$-$C_3$ alkoxy $C_1$-$C_3$ alkyl, $C_1$-$C_3$ alkyl substituted with hydroxyl, cyclopropyl, cyclobutyl, cyclopentyl, cyclohexyl, or $C_1$-$C_3$ alkyl which is substituted with 4-6 membered heteroalicyclic group selected from oxetanyl, azetidinyl, tetrahydrofuranyl, tetrahydro-2H-pyranyl, tetrahydropyrrolyl, piperidinyl, piperazinyl, morpholinyl, or thiomorpholinyl,

In some embodiments, alternatively, $R_6$ is Ci-Cs alkyl, $C_3$-$C_6$ cycloalkyl, 4-6 membered heteroalicyclic group, or $C_1$-$C_{10}$ alkyl which is unsubstituted or substituted with 1 to 3 substituents selected from the group consisting of hydroxyl, methoxy, ethoxy, propoxy, isopropoxy, butoxy, tert-butoxy, pentoxy, neopentoxy, cyano, amino, dimethylamino, diethylamino, cyclopropyloxy, cyclobutyloxy, cyclopentyloxy, cyclohexyloxy, -CONH-$R_5$, cyclopropyl, cyclobutyl, cyclopentyl, cyclohexyl, cycloheptyl, cyclooctyl, $C_3$-$C_8$ cycloalkyl substituted with hydroxyl and/or $C_1$-$C_4$ alkyl, carboxyl, fluorine, chlorine, trifluoromethoxy, trichloromethoxy, methylsulfone, ethylsulfone, -SO-$R_5$, -CO-$R_5$, ethynyl, vinyl, methoxyethoxy, meth-

oxypropoxy, ethoxyethoxy, ethoxypropoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, methylthio, ethylthio, and propylthio. Still alternatively, 4- to 8-membered heteroalicyclic group described herein is oxetanyl, azetidinyl, tetrahydrofuranyl, tetrahydro-2H-pyranyl, tetrahydropyrrolyl, piperidinyl, piperazinyl, morpholinyl, thiomorpholinyl, 1,3-dioxolan-2-yl, etc.

In some embodiments, still alternatively, $R_6$ is 4-ethyl-4-hydroxyhexyl, 4-methyl-4-hydroxypentyl, 5-methyl-5-hydroxyhexyl, 2-methyl-2-hydroxypropyl, tetrahydro-2H-pyran-4-ylethyl, tetrahydro-2H-pyran-4-ylmethyl, tetrahydro-2H-pyran-2-ylmethyl, tetrahydro-2H-pyran-2-ylethyl, tetrahydrofuran-2-ylmethyl, tetrahydrofuran-3-ylmethyl, tetrahydrofuran-2-ylethyl, tetrahydrofuran-3-ylethyl, methoxypropyl, ethoxypropyl, tert-butoxypropyl, isobutoxypropyl, isopropoxypropyl, ethoxyethyl, tert-butoxyethyl, isobutoxyethyl, isopropoxyethyl, methoxyethyl, methoxybutyl, ethoxybutyl, tert-butoxybutyl, isobutoxybutyl, isopropoxybutyl, methoxyethoxyethyl, piperidin-1-ylethyl, piperidin-1-ylpropyl, 1-methylpiperidin-4-ylmethyl, 1-methylpiperidin-4-ylethyl, 1-methylpiperidin-4-ylpropyl, 1-methylpiperazin-4-ylethyl, 1-methylpiperazin-4-ylpropyl, methyl, ethyl, propyl, isopropyl, butyl, isobutyl, tert-butyl, pentyl, isopentyl, neopentyl, hexyl, heptyl, 3,3-dimethylbutyl, octyl, hydroxymethyl, hydroxyethyl, hydroxypropyl, hydroxybutyl, hydroxypentyl, hydroxyoctyl, hydroxyheptyl, cyanomethyl, cyanoethyl, cyanopropyl, cyanobutyl, 4-cyanopentyl, 4,4,4-trifluorobutyl, N,N-dimethylpropyl, N,N-dimethylethyl, N,N-diethylpropyl, N,N-diethylethyl, methylthiobutyl, methylthiomethyl, methylthioethyl, methylthiopropyl, methylsulfonylbutyl, methylsulfonylmethyl, methylsulfonylethyl, methylsulfonylpropyl, cycloheptylethyl, cycloheptylpropyl, cyclohexylethyl, cyclohexylpropyl, cyclopentylethyl, cyclopentylpropyl, allyl, penten-1-yl, oxetan-3-yl, tetrahydro-2H-pyran-4-yl, oxetan-3-ylmethyl, oxetan-3-ylethyl, fluoroethyl, fluoropropyl, fluorobutyl, cyclopropylethyl, cyclopropylpropyl, cyclopropylbutyl, cyclobutoxypropyl, cyclobutoxyethyl, cyclobutoxymethyl, cyclopropyl, cyclobutyl, cyclopentyl, cyclohexyl, cycloheptyl, cyclooctyl, $-CH_2CONH_2$, $-(CH_2)_2CONH_2$, $-(CH_2)_3CONH_2$,

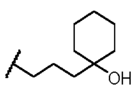, 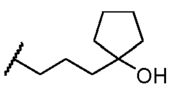, 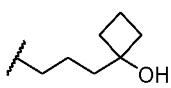, 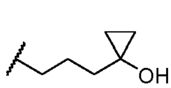, 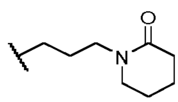, or 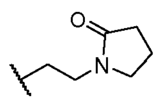.

In some embodiments, alternatively, $R^a$ and $R^b$ are each independently selected from hydrogen, $C_1$-$C_3$ alkyl, $C_3$-$C_6$ cycloalkyl, $C_1$-$C_3$ alkyl substituted with $C_1$-$C_3$ alkoxy, $C_1$-$C_3$ alkyl substituted with hydroxyl, $C_3$-$C_6$ cycloalkyl $C_1$-$C_3$ alkyl, $C_1$-$C_3$ alkyl substituted with 4-6 membered heteroalicyclic group, $C_1$-$C_3$ alkyl substituted with $C_1$-$C_3$ alkylthio, or $C_1$-$C_3$ alkyl substituted with substituted amino or unsubstituted amino, wherein the substituted amino is substituted with mono- or di-$C_1$-$C_3$ alkyl.

In some embodiments, still alternatively, $R_7$ is hydrogen, fluorine, chlorine, methyl, or trifluoromethyl.

In some embodiments, alternatively, $R_1$ is

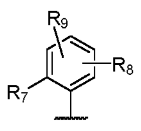, 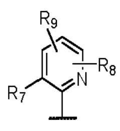, 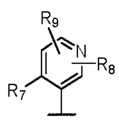, 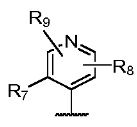,

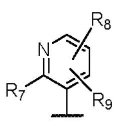, 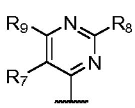, 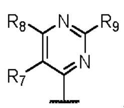, 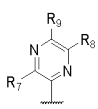, 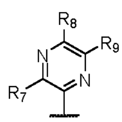, 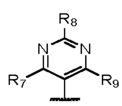 or 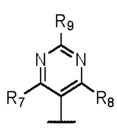,

$R_7$ is hydrogen, $C_1$-$C_4$ alkyl, $C_1$-$C_3$ alkoxy, halogen, hydroxyl, trifluoromethyl, trifluoromethoxy, cyano, $C_2$-$C_4$ alkynyl, $C_2$-$C_4$ alkenyl, $C_3$-$C_4$ cycloalkyl, or $C_1$-$C_3$ acyl; still alternatively, $R_7$ is hydrogen, fluorine, chlorine, methyl, or trifluoromethyl.

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, $-CO-R_5$, $-SO_2-R_5$, $-SO-R_5$, $-CONH-R_5$, $-NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or $-O-R_6$,

the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cy-

cloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_9$ is hydrogen, $C_1$-$C_4$ alkyl, $C_1$-$C_3$ alkoxy, halogen, hydroxy, trifluoromethyl, trifluoromethoxy, cyano, $C_2$-$C_4$ alkynyl, $C_2$-$C_4$ alkenyl, $C_3$-$C_4$ Cycloalkyl, or $C_1$-$C_3$ acyl.

In some embodiments, alternatively, $R_2$ is $C_3$-$C_8$ cycloalkyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino,

or $C_7$-$C_{10}$ bridged cyclyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino,

or $C_1$-$C_{10}$ alkyl which is unsubstituted or substituted with 1 to 3 substituents selected from the group consisting of methoxy, ethoxy, propoxy, isopropoxy, methylthio, ethylthio, propylthio, formyl, acetyl, hydroxyl, fluorine, chlorine, cyano, -$CONH_2$, cyclopropyl, cyclobutyl, cyclopentyl, cyclohexyl, amino, dimethylamino, and diethylamino,

or -$(CH_2)n$-$R^e$, wherein $R^e$ is aryl or heteroaryl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, ethynyl, vinyl, amino, dimethylamino, and diethylamino, n is an integer from 0 to 3,

or -$(CH_2)m$-$R^f$, wherein $R^f$ is 4- to 8-membered heteroalicyclic group, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino, m is an integer from 0 to 3,

the 4- to 8-membered heteroalicyclic group contains 1 to 2 atoms selected from N, O, or S as ring atoms,

the aryl group is phenyl, and the heteroaryl group is pyridyl, pyrimidinyl, pyrazolyl, oxazolyl, isoxazolyl, or thiazolyl.

In some embodiments, alternatively, $R_2$ is cyclopropyl, cyclobutyl, cyclopentyl, cyclohexyl, cycloheptyl, cyclooctyl, 4,4-difluorocyclohexyl, bicyclo[2.2.1]heptyl, adamantyl, methyl, ethyl, propyl, isopropyl, butyl, isobutyl, sec-butyl, tert-butyl, pentyl, neopentyl, hexyl, 2-hydroxy-2-methylpropyl, 3,3-dimethylbutyl, 3-hydroxy-3-methylbutyl, cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, benzyl, phenethyl, phenyl, 2-fluorophenyl, 2-chlorophenyl, 2-methoxyphenyl, 2-cyanophenyl, 2-ethynylphenyl, 3-fluorophenyl, 3-chlorophenyl, 3-methoxyphenyl, 3-cyanophenyl, 3-ethynylphenyl, 4-fluorophenyl, 4-chlorophenyl, 4-methoxyphenyl, 4-cyanophenyl, 4-ethynylphenyl, 3,4-difluorophenyl, 3-cyano-4-methylphenyl, pyridin-2-yl, pyridin-3-yl, pyridine-4-yl, pyrimidin-2-yl, pyrimidin-4-yl, pyrimidin-5-yl, pyrazinyl, pyrazolyl, oxazolyl, isoxazolyl, thiazolyl, oxetan-3-yl, tetrahydrofuran-3-yl, tetrahydro-2H-pyran-4-yl, tetrahydropyrrolyl, piperidin-1-yl, piperazin-1-yl, morpholin-4-yl, methylpiperazin-4-yl, 1-methylpiperidin-4-yl, 1-acetylpiperidin-4-yl, 4-hydroxypiperidin-1-yl, 4-methyl-4-hydroxypiperidin-1-yl,

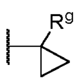, 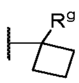, 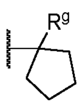, 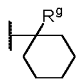, 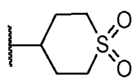,

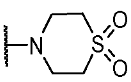, 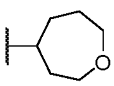, 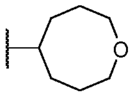, 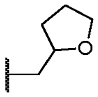, 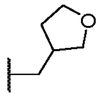, 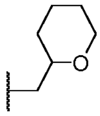, 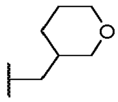, or 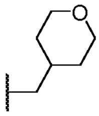,

$R^g$ is $-CH_3$ or - OH.

In some embodiments, alternatively, $R_2$ is 2,3-difluorophenyl, 3,5-difluorophenyl, 2,5-difluorophenyl, 2,4-difluorophenyl, 2,3,4-trifluorophenyl, 2,3,5-trifluorophenyl, 2,3,6-trifluorophenyl, 3,4,5-trifluorophenyl, 2,4,5-trifluorophenyl, 3-chloro-2-fluorophenyl, 2-chloro-3-fluorophenyl, 5-chloro-2-fluorophenyl, 2-chloro-5-fluorophenyl, 5-chloro-3-fluorophenyl, 3-chloro-5-fluorophenyl, 3-chloro-4-fluorophenyl, 4-chloro-3-fluorophenyl, 2-chloro-4-fluorophenyl, 4-chloro-2-fluorophenyl, 3-chloro-2,4-difluorophenyl, 5-chloro-2,4-difluorophenyl, 3-chloro-2,5-difluorophenyl, 3-chloro-2,6-difluorophenyl, 3-chloro-4,5-difluorophenyl, 2-chloro-4,5-difluorophenyl, 2-chloro-3,4-difluorophenyl, 2-chloro-3,5-difluorophenyl, 2-chloro-3,6-difluorophenyl, 4-chloro-2,3-difluorophenyl, 4-chloro-3,5-difluorophenyl, 4-chloro-2,5-difluorophenyl, 5-chloro-2,3 -difluorophenyl, 5 -chloro-3,4-difluorophenyl, 6-chloro-2,3 -difluorophenyl, 3 -fluoro-5 -methylphenyl, 4-fluoro-3-methylphenyl, 2-fluoro-3-methylphenyl, 2-fluoro-4-methylphenyl, 2-fluoro-5-methylphenyl, 3-fluoro-4-methylphenyl, 3-fluoro-2-methylphenyl, 3-fluoro-5-methoxyphenyl, 4-fluoro-3-methoxyphenyl, 2-fluoro-3-methoxyphenyl, 2-fluoro-4-methoxyphenyl, 2-fluoro-5-methoxyphenyl, 3-fluoro-4-methoxyphenyl, 3-fluoro-2-methoxyphenyl, 2-fluoro-3-trifluoromethylphenyl, 2-fluoro-4-trifluoromethylphenyl, 2-fluoro-5-trifluoromethylphenyl, 3-fluoro-2-trifluoromethylphenyl, 4-fluoro-2-trifluoromethylphenyl, 5-fluoro-2-trifluoromethylphenyl, 4-fluoro-3-trifluoromethylphenyl, 3-fluoro-4-trifluoromethylphenyl, 3-fluoro-5-trifluoromethylphenyl, 2-fluoro-5-ethylphenyl, 2-fluoro-5-cyclopropylphenyl, or 2-fluoro-5-phenylphenyl.

In some embodiments, alternatively, $R_3$ is hydrogen, methyl, ethyl, hydroxyl, cyano, trifluoromethyl, fluorine, or chlorine.

Disclosed is a compound represented by formula (I), pharmaceutically acceptable salts, diastereomer, enantiomer, or solvate thereof,

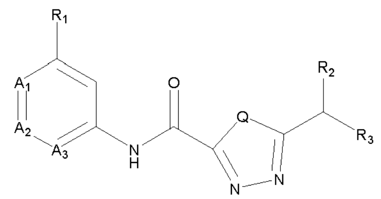

formula (I)

wherein, Q is NH;

$A_3$ is N, $A_1$, and $A_2$ are each independently selected from N or $CR_4$, $R_4$ is H, F, Cl or methyl, alternatively H or F;
$R_1$ is

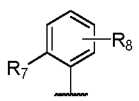, 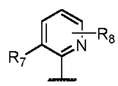, 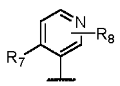, 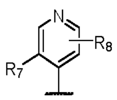, 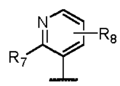, 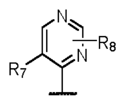, 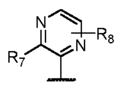,

or

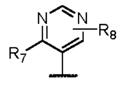,

$R_7$ is hydrogen, $C_1$-$C_4$ alkyl, $C_1$-$C_3$ alkoxy, halogen, hydroxyl, trifluoromethyl, trifluoromethoxy, cyano, $C_2$-$C_4$ alkynyl, $C_2$-$C_4$ alkenyl, $C_3$-$C_4$ cycloalkyl, or $C_1$-$C_3$ acyl;
$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, $-CO-R_5$, $-SO_2-R_5$, $-SO-R_5$, $-CONH-R_5$, $-NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, hydroxyl $C_1$-$C_{10}$ alkyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or $-O-R_6$,
the 4- to 8-membered heteroalicyclic group is a 4- to 8-membered heteroalicyclic group containing 1 to 2 atoms selected from N, O, or S as ring atoms,
$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,
$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, $-NR^aR^b$,

$C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, or $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl,

still alternatively, $R_1$ is :

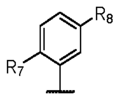, 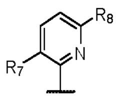, 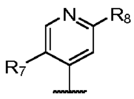, 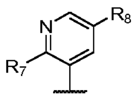, 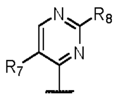, or 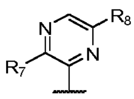,

$R_7$ is hydrogen, methyl, ethyl, propyl, isopropyl, methoxy, ethoxy, propoxy, isopropoxy, fluorine, chlorine, hydroxyl, trifluoromethyl, trifluoromethoxy, cyano, ethynyl, propynyl, vinyl, propenyl, cyclopropyl, cyclobutyl, formyl, or acetyl; alternatively $R_7$ is hydrogen, fluorine, chlorine, methyl, or trifluoromethyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or -O-$R_6$,

the 4- to 8-membered heteroalicyclic group is heteroalicyclic group containing 1 to 2 atoms selected from N, O, or S as ring atoms,

$R_5$ is hydrogen, hydroxyl, $C_1$-$C_4$ alkyl, $C_1$-$C_3$ alkoxy $C_1$-$C_3$ alkyl, $C_1$-$C_3$ alkyl substituted with hydroxyl, cyclopropyl, cyclobutyl, cyclopentyl, cyclohexyl, or $C_1$-$C_3$ alkyl which is substituted with 4-6 membered heteroalicyclic group selected from oxetanyl, azetidinyl, tetrahydrofuranyl, tetrahydro-2H-pyranyl, tetrahydropyrrolyl, piperidinyl, piperazinyl, morpholinyl, or thiomorpholinyl,

$R_6$ is Ci-Cs alkyl, $C_3$-$C_6$ cycloalkyl, 4-6 membered heteroalicyclic group, or $C_1$-$C_{10}$ alkyl which is unsubstituted or substituted with 1 to 3 substituents selected from the group consisting of hydroxyl, methoxy, ethoxy, propoxy, isopropoxy, butoxy, tert-butoxy, pentoxy, neopentoxy, cyano, amino, dimethylamino, diethylamino, cyclopropyloxy, cyclobutyloxy, cyclopentyloxy, cyclohexyloxy, -CONH-$R_5$, cyclopropyl, cyclobutyl, cyclopentyl, cyclohexyl, cycloheptyl, cyclooctyl, $C_3$-$C_8$ cycloalkyl substituted with hydroxyl and/or $C_1$-$C_4$ alkyl, carboxyl, fluorine, chlorine, trifluoromethoxy, trichloromethoxy, methylsulfone, ethylsulfone, -SO-$R_5$, -CO-$R_5$, ethynyl, vinyl, methoxyethoxy, methoxypropoxy, ethoxyethoxy, ethoxypropoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, methylthio, ethylthio, and propylthio. Still alternatively, 4- to 8-membered heteroalicyclic group described herein is oxetanyl, azetidinyl, tetrahydrofuranyl, tetrahydro-2H-pyranyl, tetrahydropyrrolyl, piperidinyl, piperazinyl, morpholinyl, thiomorpholinyl, 1,3-dioxolan-2-yl, etc.,

still alternatively, $R_6$ is 4-ethyl-4-hydroxyhexyl, 4-methyl-4-hydroxypentyl, 5-methyl-5-hydroxyhexyl, 2-methyl-2-hydroxypropyl, tetrahydro-2H-pyran-4-ylethyl, tetrahydro-2H-pyran-4-ylmethyl, tetrahydro-2H-pyran-2-ylmethyl, tetrahydro-2H-pyran-2-ylethyl, tetrahydrofuran-2-ylmethyl, tetrahydrofuran-3 -ylmethyl, tetrahydrofuran-2-ylethyl, tetrahydrofuran-3-ylethyl, methoxypropyl, ethoxypropyl, tert-butoxypropyl, isobutoxypropyl, isopropoxypropyl, ethoxyethyl, tert-butoxyethyl, isobutoxyethyl, isopropoxyethyl, methoxyethyl, methoxybutyl, ethoxybutyl, tert-butoxybutyl, isobutoxybutyl, isopropoxybutyl, methoxyethoxyethyl, piperidin-1-ylethyl, piperidin-1-ylpropyl, 1-methylpiperidin-4-ylmethyl, 1-methylpiperidin-4-ylethyl, 1-methylpiperidin-4-ylpropyl, 1-methylpiperazin-4-ylethyl, 1-methylpiperazin-4-ylpropyl, methyl, ethyl, propyl, isopropyl, butyl, isobutyl, tert-butyl, pentyl, isopentyl, neopentyl, hexyl, heptyl, 3,3-dimethylbutyl, octyl, hydroxymethyl, hydroxyethyl, hydroxypropyl, hydroxybutyl, hydroxypentyl, hydroxyoctyl, hydroxyheptyl, cyanomethyl, cyanoethyl, cyanopropyl, cyanobutyl, 4-cyanopentyl, 4,4,4-trifluorobutyl, N,N-dimethylpropyl, N,N-dimethylethyl, N,N-diethylpropyl, N,N-diethylethyl, methylthiobutyl, methylthiomethyl, methylthioethyl, methylthiopropyl, methylsulfonylbutyl, methylsulfonylmethyl, methylsulfonylethyl, methylsulfonylpropyl, cycloheptylethyl, cycloheptylpropyl, cyclohexylethyl, cyclohexylpropyl, cyclopentylethyl, cyclopentylpropyl, allyl, penten-1-yl, oxetan-3-yl, tetrahydro-2H-pyran-4-yl, oxetan-3-ylmethyl, oxetan-3-ylethyl, fluoroethyl, fluoropropyl, fluorobutyl, cyclopropylethyl, cyclopropylpropyl, cyclopropylbutyl, cyclobutoxypropyl, cyclobutoxyethyl, cyclobutoxymethyl, cyclopropyl, cyclobutyl, cyclopentyl, cyclohexyl, cycloheptyl, cyclooctyl, -$CH_2CONH_2$, -$(CH_2)_2CONH_2$, -$(CH_2)_3CONH_2$,

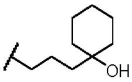, 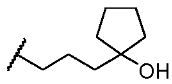, 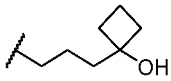, 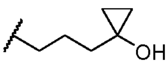, 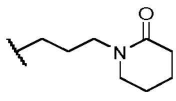, or 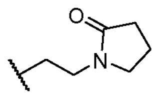.

$R^a$ and $R^b$ are each independently selected from hydrogen, $C_1$-$C_3$ alkyl, $C_3$-$C_6$ cycloalkyl, $C_1$-$C_3$ alkyl substituted with $C_1$-$C_3$ alkoxy, $C_1$-$C_3$ alkyl substituted with hydroxyl, $C_3$-$C_6$ cycloalkyl $C_1$-$C_3$ alkyl, $C_1$-$C_3$ alkyl substituted with 4-6 membered heteroalicyclic group, $C_1$-$C_3$ alkyl substituted with $C_1$-$C_3$ alkylthio, or $C_1$-$C_3$ alkyl substituted with substituted amino or unsubstituted amino, wherein the substituted amino is substituted with mono- or di-$C_1$-$C_3$ alkyl;

$R_2$ is $C_3$-$C_8$ cycloalkyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, $-CONH_2$, oxo (=O), amino, dimethylamino, and diethylamino,

or $C_7$-$C_{10}$ bridged cyclyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, $-CONH_2$, oxo (=O), amino, dimethylamino, and diethylamino,

or $C_1$-$C_{10}$ alkyl which is unsubstituted or substituted with 1 to 3 substituents selected from the group consisting of methoxy, ethoxy, propoxy, isopropoxy, methylthio, ethylthio, propylthio, formyl, acetyl, hydroxyl, fluorine, chlorine, cyano, $-CONH_2$, cyclopropyl, cyclobutyl, cyclopentyl, cyclohexyl, amino, dimethylamino, and diethylamino,

or $-(CH_2)n-R^e$, wherein $R^e$ is aryl or heteroaryl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, $-CONH_2$, ethynyl, vinyl, amino, dimethylamino, and diethylamino, n is an integer from 0 to 3,

or $-(CH_2)m-R^f$, wherein $R^f$ is 4- to 8-membered heteroalicyclic group, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, $-CONH_2$, oxo (=O), amino, dimethylamino, and diethylamino, m is an integer from 0 to 3,

the 4- to 8-membered heteroalicyclic group contains 1 to 2 atoms selected from N, O, or S as ring atoms,

the aryl group is phenyl, and the heteroaryl group is pyridyl, pyrimidinyl, pyrazolyl, oxazolyl, isoxazolyl, or thiazolyl;

still alternatively, $R_2$ is cyclopropyl, cyclobutyl, cyclopentyl, cyclohexyl, cycloheptyl, cyclooctyl, 4,4-difluorocyclohexyl, bicyclo[2.2.1]heptyl, adamantyl, methyl, ethyl, propyl, isopropyl, butyl, isobutyl, sec-butyl, tert-butyl, pentyl, neopentyl, hexyl, 2-hydroxy-2-methylpropyl, 3,3-dimethylbutyl, 3-hydroxy-3-methylbutyl, cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, benzyl, phenethyl, phenyl, 2-fluorophenyl, 2-chlorophenyl, 2-methoxyphenyl, 2-cyanophenyl, 2-ethynylphenyl, 3-fluorophenyl, 3-chlorophenyl, 3-methoxyphenyl, 3-cyanophenyl, 3-ethynylphenyl, 4-fluorophenyl, 4-chlorophenyl, 4-methoxyphenyl, 4-cyanophenyl, 4-ethynylphenyl, 3,4-difluorophenyl, 3-cyano-4-methylphenyl, pyridin-2-yl, pyridin-3-yl, pyridine-4-yl, pyrimidin-2-yl, pyrimidin-4-yl, pyrimidin-5-yl, pyrazinyl, pyrazolyl, oxazolyl, isoxazolyl, thiazolyl, oxetan-3-yl, tetrahydrofuran-3-yl, tetrahydro-2H-pyran-4-yl, tetrahydropyrrolyl, piperidin-1-yl, piperazin-1-yl, morpholin-4-yl, methylpiperazin-4-yl, 1-methylpiperidin-4-yl, 1-acetylpiperidin-4-yl, 4-hydroxypiperidin-1-yl, 4-methyl-4-hydroxypiperidin-1-yl, 2,3-difluorophenyl, 3,5-difluorophenyl, 2,5-difluorophenyl, 2,4-difluorophenyl, 2,3,4-trifluorophenyl, 2,3,5-trifluorophenyl, 2,3,6-trifluorophenyl, 3,4,5-trifluorophenyl, 2,4,5-trifluorophenyl, 3-chloro-2-fluorophenyl, 2-chloro-3-fluorophenyl, 5-chloro-2-fluorophenyl, 2-chloro-5-fluorophenyl, 5-chloro-3-fluorophenyl, 3-chloro-5-fluorophenyl, 3-chloro-4-fluorophenyl, 4-chloro-3-fluorophenyl, 2-chloro-4-fluorophenyl, 4-chloro-2-fluorophenyl, 3-chloro-2,4-difluorophenyl, 5-chloro-2,4-difluorophenyl, 3-chloro-2,5-difluorophenyl, 3-chloro-2,6-difluorophenyl, 3-chloro-4,5-difluorophenyl, 2-chloro-4,5-difluorophenyl, 2-chloro-3,4-difluorophenyl, 2-chloro-3,5-difluorophenyl, 2-chloro-3,6-difluorophenyl, 4-chloro-2,3-difluorophenyl, 4-chloro-3,5-difluorophenyl, 4-chloro-2,5 -difluorophenyl, 5 -chloro-2,3 -difluorophenyl, 5 -chloro-3,4-difluorophenyl, 6-chloro-2,3 - difluorophenyl, 3-fluoro-5-methylphenyl, 4-fluoro-3-methylphenyl, 2-fluoro-3-methylphenyl, 2-fluoro-4-methylphenyl, 2-fluoro-5-methylphenyl, 3-fluoro-4-methylphenyl, 3-fluoro-2-methylphenyl, 3-fluoro-5-methoxyphenyl, 4-fluoro-3-methoxyphenyl, 2-fluoro-3-methoxyphenyl, 2-fluoro-4-methoxyphenyl, 2-fluoro-5-methoxyphenyl, 3-fluoro-4-methoxyphenyl, 3-fluoro-2-methoxyphenyl, 2-fluoro-3-trifluoromethylphenyl, 2-fluoro-4-trifluoromethylphenyl, 2-fluoro-5 -trifluoromethylphenyl, 3 -fluoro-2-trifluoromethylphenyl, 4-fluoro-2-trifluoromethylphenyl, 5 -fluoro-2-trifluoromethylphenyl, 4-fluoro-3 -trifluoromethylphenyl, 3 -fluoro-4-trifluoromethylphenyl, 3 -fluoro-5 -trifluoromethylphenyl, 2-fluoro-5 -ethylphenyl, 2-fluoro-5 -cyclopropylphenyl, 2-fluoro-5 -phenylphenyl,

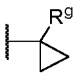, 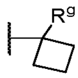, 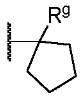, 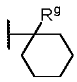, 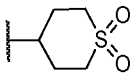, 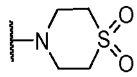, 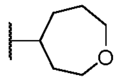,

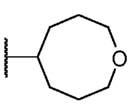, 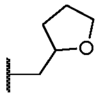, 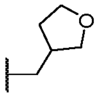, 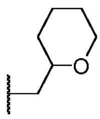, 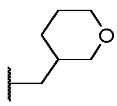, or 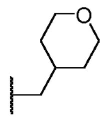,

$R^g$ is $-CH_3$ or -OH.
$R_3$ is hydrogen, methyl, ethyl, hydroxyl, cyano, trifluoromethyl, fluorine, or chlorine, still alternatively hydrogen, fluorine, hydroxyl, or chlorine.

In one particularly preferred embodiment, in formula (I),

Q is NH;
$A_1$, and $A_2$ are each CH;
$A_3$ is N;
$R_1$ is

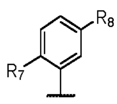, 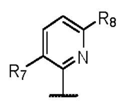, 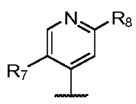, 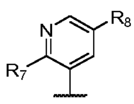, 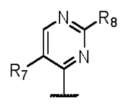, or 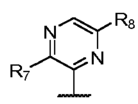;

$R_7$ is methyl, ethyl, propyl, isopropyl, fluorine, chlorine, trifluoromethyl, vinyl, or propenyl;
$R_8$ is cyano, $C_1$-$C_3$ alkoxy $C_1$-$C_6$ alkylthio, or -O-$R_6$;
$R_5$ is $C_1$-$C_6$ alkyl;
$R_6$ is 4- to 8-membered heteroalicyclic group, or $C_1$-$C_{10}$ alkyl which is substituted with 1 to 3 substituents selected from the group consisting of hydroxyl, $C_1$-$C_6$ alkoxy, cyano, $C_3$-$C_8$ cycloalkyloxy, $C_3$-$C_8$ cycloalkyl, - $SO_2$-$R_5$, 4- to 8-membered heteroalicyclic group, and $C_1$-$C_6$ alkylthio;
$R_2$ is $-(CH_2)n-R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, $-CONH_2$, $C_2$-$C_6$ alkynyl, $C_2$-$C_6$ alkenyl, and $-NR^cR^d$, wherein n is an integer from 0 to 3; $R^c$ and $R^d$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;
the 4- to 8-membered heteroalicyclic group is a 4- to 8-membered heteroalicyclic group containing 1 to 2 atoms selected from N, O, or S as ring atoms;
the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms; and
$R_3$ is hydrogen.

In said particularly preferred embodiment, it is especially preferred that

Q is NH;
$A_1$, and $A_2$ are each CH;
$A_3$ is N;
$R_1$ is

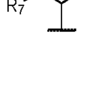, 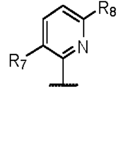, 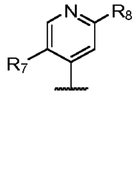, 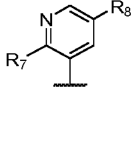, 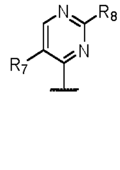, or 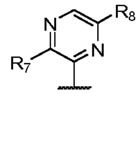;

$R_7$ is methyl, chlorine, or trifluoromethyl; $R_8$ is cyano, $C_1$-$C_3$ alkoxy $C_1$-$C_6$ alkylthio, or -O-$R_6$; $R_6$ is $C_1$-$C_{10}$ alkyl which is substituted with 1 to 3 substituents selected from hydroxyl; $R_2$ is -$(CH_2)$n-$R^e$, wherein $R^e$ is aryl or heteroaryl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, ethynyl, vinyl, amino, dimethylamino, and diethylamino, n is an integer from 0 to 3,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms; and

$R_3$ is hydrogen.

According to some embodiments of the present disclosure, the pharmaceutically acceptable salt of the compound is selected from the group consisting of one or more of the following salts: hydrochloride, hydrobromide, hydroiodide, perchlorate, sulfate, nitrate, phosphate, formate, acetate, propionate, glycolate, lactate, succinate, maleate, tartrate, malate, citrate, fumarate, gluconate, benzoate, mandelate, methanesulfonate, isethionate, benzenesulfonate, oxalate, palmitate, 2-naphthalenesulfonate, p-toluenesulfonate, cyclohexylsulfamate, salicylate, hexonate, trifluoroacetate, aluminum salt, calcium salt, chloroprocaine salt, choline salt, diethanolamine salt, ethylenediamine salt, lithium salt, magnesium salt, potassium salt, sodium salt and zinc salt.

Another aspect of the present disclosure relates to the application of the compound, pharmaceutically acceptable salt(s), diastereomer(s), enantiomer(s), or solvate(s) thereof in the preparation of a medicament for the treatment of RIP1 related diseases, Wherein, the RIP1 related disease include ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic myeloid leukemia, acute myeloid leukemia, non-Hodgkin's lymphoma, nasopharyngeal cancer, esophageal cancer, brain tumor, B-cell and T-cell lymphoma, lymphoma, multiple myeloma, biliary cancer and sarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behget's disease, interleukin-1 converzyme-related fever syndrome, chronic obstructive pulmonary disease, tumor necrosis factor receptor related periodic syndrome and periodontitis.

Another aspect of the present disclosure provides a pharmaceutical composition, which includes the acylamino bridged heterocyclic compounds of the present disclosure, or diastereomers, enantiomers, solvates, or pharmaceutically acceptable salts thereof, and one or more pharmaceutically acceptable carriers or excipients.

According to some embodiments of the application, the pharmaceutical composition may also include one or more other therapeutic agents.

The present disclosure also relates to a therapeutically effective amount of a compound of formula (I) or a salt thereof for use in treating diseases or disorders mediated by RIP1 kinase in a patient (human or other mammals, especially human) in need thereof. The RIP1 kinase-mediated diseases or disorders include those mentioned above.

## Description of the figures

Figure 1 shows a Western Blot picture of the inhibition of RIPK1 phosphorylation by the compound of Example 2;
Figure 2 shows the inhibitory rate of the compound of Example 2 on the inhibition of RIPK1 phosphorylation;
Figure 3 shows the dilution flow chart for the compound of Assay Example 2.

## DETAILED DESCRIPTION OF THE EMBODIMENTS

Unless otherwise stated, the following terms used in this application (including the description and claims) have the definitions given below. In addition, the use of the term "comprising" and other forms such as "including", "containing" and "having" is not limiting. The chapter headings used herein are for organizational purposes only and should not be

interpreted as limitations on the topics described.

Detailed description

Unless otherwise specified, alkyl represents a saturated straight-chain, or branched-chain hydrocarbon group with the specified number of carbon atoms. The term $C_1$-$C_{10}$ alkyl represents an alkyl moiety containing 1 to 10 carbon atoms, similarly $C_1$-$C_3$ alkyl represents an alkyl moiety containing 1 to 3 carbon atoms, such as, $C_1$-$C_6$ alkyl including methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl and 2-methylpentyl etc.

When substituent terms such as "alkyl" are used in combination with other substituent terms, for example in the term "$C_1$-$C_3$ alkoxy $C_1$-$C_6$ alkylthio" or "$C_1$-$C_{10}$ alkyl substituted with hydroxyl", this linking substituent term (e.g. alkyl or alkylthio) is intended to include a divalent moiety, wherein the connection point is through the connection substituent. Examples of "$C_1$-$C_3$ alkoxy $C_1$-$C_6$ alkylthio" include, but are not limited to, methoxymethylthio, methoxyethylthio and ethoxypropylthio, etc. Examples of "$C_1$-$C_{10}$ alkyl substituted with hydroxyl" include, but are not limited to, hydroxymethyl, hydroxyethyl, and hydroxyisopropyl.

Alkoxy is an alkyl-O- group formed by the previously described linear or branched alkyl group and - O-, for example, methoxy, ethoxy, etc. Similarly, alkylthio is an alkyl-S- group formed by the previously described linear or branched alkyl group and -S-, for example, methylthio, ethylthio, etc.

Alkenyl and alkynyl include straight chain, or branched alkenyl or alkynyl, the term $C_2$-$C_6$ alkenyl or $C_2$-$C_6$ alkynyl means a straight or branched chain hydrocarbon group having at least one alkenyl or alkynyl group.

The term "halogenated $C_1$-$C_{10}$ alkyl" represents a group having one or more halogen atoms, which may be the same or different, on one or more carbon atoms of the alkyl moiety comprising 1 to 10 carbon atoms. Examples of "halogenated $C_1$-$C_{10}$ alkyl" may include, but are not limited to, $-CF_3$ (trifluoromethyl), $-CCl_3$ (trichloromethyl), 1,1-difluoroethyl, 2,2,2-trifluoroethyl and hexafluoroisopropyl, etc. Similarly, the term "halogenated $C_1$-$C_{10}$ alkoxy" represents a haloalkyl-O- group formed by the halogenated $C_1$-$C_{10}$ alkyl group and -O-, for example, trifluoromethoxy, trichloromethoxy, etc.

The term "$C_1$-$C_3$ acyl" includes formyl (-CHO), acetyl ($CH_3CO$-), propionyl ($C_2H_5CO$-).

The terms "$-CO-R_5$, $-SO_2-R_5$, $-SO-R_5$, $-CONH-R_5$" represent

“ 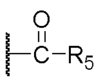 , 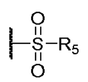 , 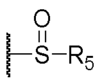 ,

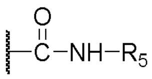 ”,

respectively.

"Cycloalkyl" represents a non-aromatic, saturated, cyclic hydrocarbon group containing the specified number of carbon atoms. For example, the term "($C_3$-$C_6$) cycloalkyl" refers to a non-aromatic cyclic hydrocarbon ring with 3-6 ring carbon atoms. Exemplary "($C_3$-$C_6$) cycloalkyl" includes cyclopropyl, cyclobutyl, cyclopentyl and cyclohexyl.

The term "aryl" represents a group or moiety containing an aromatic monocyclic or bicyclic hydrocarbon atom group, which contains 6 to 12 carbon ring atoms and has at least one aromatic ring. Examples of "aryl" are phenyl, naphthyl, indenyl and indanyl. Generally, in the compounds of the present disclosure, the aryl group is a phenyl group.

The term "heteroalicyclic group" as used herein, unless otherwise specified, represents an unsubstituted or substituted stable 4- to 8-membered non-aromatic monocyclic saturated ring system, which consists of carbon atoms and 1 to 3 heteroatoms selected from N, O, or S, wherein, N and S heteroatoms can be arbitrarily oxidized, and N heteroatoms can also be arbitrarily quaternized. Examples of such heterocycles include, but are not limited to, azetidinyl, oxetanyl, thietanyl, pyrrolidinyl, pyrrolinyl, pyrazolidinyl, pyrazolinyl, imidazolidinyl, imidazolinyl, oxazolinyl, thiazolinyl, tetrahydrofuranyl, dihydrofuranyl, tetrahydrothienyl, 1,3-dioxolanyl, piperidinyl, piperazinyl, tetrahydropyranyl, dihydropyranyl, tetrahydrothiopyranyl, 1,3-dioxanyl, 1,4-dioxanyl, 1,3-oxathiolanyl, 1,3-oxathianyl, 1,3-dithianyl, 1,4-oxathiolanyl, 1,4-oxathianyl, 1,4-dithianyl, morpholinyl, and thiomorpholinyl.

The term "heteroaryl" as used herein represents a group or moiety containing an aromatic monocyclic or bicyclic group (which contains 5 to 10 ring atoms), which includes 1 to 3 heteroatoms independently selected from nitrogen, oxygen and sulfur. The term also includes bicyclic heterocyclic aryl, which contains an aryl ring moiety fused to a heterocycloalkyl ring moiety, or a heteroaryl ring moiety fused to a cycloalkyl ring moiety. Unless otherwise specified, it represents an unsubstituted or substituted stable 5- or 6-membered monocyclic aromatic ring system, and can also represents an unsubstituted or substituted benzene fused heteroaromatic ring system or bicyclic heteroaromatic ring

system with 9 or 10 ring atoms, which are composed of carbon atoms and 1 to 3 heteroatoms selected from N, O, or S, where N, S heteroatoms can be oxidized, and N heteroatoms can also be quaternized. Heteroaryl groups can be connected to any heteroatom or carbon atom to form a stable structure. Illustrative examples of heteroaryl groups include, but are not limited to, furanyl, thienyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridyl, oxo-pyridyl (pyridyl-N-oxide), pyridazinyl, pyrazinyl, pyrimidinyl, triazinyl, benzofuranyl, isobenzofuranyl, 2,3-dihydrobenzofuranyl, 1,3-benzodioxolyl, dihydrobenzodioxinyl, benzothienyl, indazinyl, indolyl, isoindolyl, indolinyl, benzimidazolyl, dihydrobenzimidazolyl, benzoxazolyl, dihydrobenzoxazolyl, benzothiazolyl, benzoisothiazolyl, dihydrobenzisothiazolyl, indazolyl, imidazopyridyl, pyrazolopyridyl, benzotriazolyl, triazolopyridyl, purinyl, quinolinyl, tetrahydroquinolinyl, isoquinolinyl, tetrahydroisoquinolinyl, quinoxalinyl, cinnolinyl, phthalazinyl, quinazolinyl, 1,5-naphthyridinyl, 1,6- naphthyridinyl, 1,7- naphthyridinyl, 1,8- naphthyridinyl, and pteridyl.

The term "carbonyl" refers to the -C(O)- group. The terms "halogen" and "halo" represent chlorine, fluorine, bromine or iodine substituents. "Oxo" represents the oxygen moiety with double bond; for example, it forms a carbonyl moiety (C=O) when it is directly attached to a carbon atom. "Hydroxy" is intended to mean the -OH group. As used herein, the term "cyano" refers to the group -CN.

The term "each independently" means that when more than one substituents are selected from a number of possible substituents, those substituents may be the same or different.

It is clear that the compounds of formula I, or diastereomers, enantiomers, or crystal forms and pharmaceutically acceptable salts thereof may exist in solvated and unsolvated forms. For example, the solvated form can be a hydrate form. The present disclosure includes all these solvated forms and unsolvated forms.

The compounds of the present disclosure may have asymmetric carbon atoms. According to their physical and chemical differences, such diastereomeric mixtures can be separated into single diastereomers by known, technically mature methods, such as chromatography or fractional crystallization. The separation of enantiomers can be carried out by first reacting with a suitable optically active compound, converting the enantiomeric mixture into a diastereomeric mixture, separating the diastereoisomers, and then the single diastereomers are transformed (hydrolyzed) into the corresponding pure enantiomers. All such isomers, including diastereomeric mixtures and pure enantiomers, are considered as part of the invention.

The compound of the present disclosure as an active ingredient, and the method of preparing the same, are both included in the present disclosure. Moreover, the crystalline form of some of the compounds may exist as polymorphs, and such forms may also be included in the present disclosure. Additionally, some of the compounds may form solvates with water (i.e., hydrates) or common organic solvents, and such solvates are also included within the scope of the disclosure.

The compounds of the disclosure may be used in the free form for treatment or, when appropriate, in the form of a pharmaceutically acceptable salt or other derivative for treatment. As used herein, the term "pharmaceutically acceptable salt" refers to organic and inorganic salts of the compounds of the present disclosure which are suitable for use in human and lower animals without undue toxicity, irritation, allergic response, etc., and have reasonable benefit/risk ratio. Pharmaceutically acceptable salts of amines, carboxylic acids, phosphonates, and other types of compounds are well known in the art. The salt can be formed by reacting a compound of the disclosure with a suitable free base or acid, including, but not limited to, salts with inorganic acids such as hydrochloric acid, hydrobromic acid, phosphoric acid, sulfuric acid, perchloric acid or with organic acids such as acetic acid, oxalic acid, maleic acid, tartaric acid, citric acid, succinic acid, malonic acid. Or the salts may be obtained by methods well known in the art, such as ion exchange. Other pharmaceutically acceptable salts include adipate, alginate, ascorbate, aspartate, besylate, benzoate, bisulfate, borate, butanoate, camphorate, camphorsulfonate, citrate, digluconate, lauryl sulfate, ethanesulfonate, formate, fumarate, glucoheptonate, glycerol phosphate, glyconate, hemisulfate, hexanoate, hydroiodide, 2-hydroxyethanesulfonate, lactobionate, lactate, laurate, lauryl sulfate, malate, maleate, mesylate, 2-naphthalenesulfonate, nicotinate, nitrate, oleate, palmitate, pamoate, pectate, persulphate, per-3-phenylpropionate, phosphate, picrate, propionate, stearate, sulfate, thiocyanate, p-toluenesulfonate, undecanoate, and the like. Representative alkali or alkaline earth metal salts include salts of sodium, lithium, potassium, calcium, magnesium, and the like. Other pharmaceutically acceptable salts include suitable non-toxic salts of ammonium, quaternary ammonium, and amine cations formed from halides, hydroxides, carboxylates, sulfates, phosphates, nitrates, lower alkyl sulfonates and aryl sulfonates.

Pharmaceutical compositions of this disclosure comprise the compound of formula (I) described herein or a pharmaceutically acceptable salt thereof; an additional agent selected from a kinase inhibitory agent (small molecule, polypeptide, antibody, etc.), an immunosuppressant, an anticancer agent, an anti-viral agent, antiinflammatory agent, antifungal agent, antibiotic, or an anti-vascular hyperproliferation compound; and any pharmaceutically acceptable carrier, adjuvant or vehicle.

The compounds of the present disclosure may be used alone or in combination with one or more of other compounds of the present disclosure or with one or more of other agents. When administered in combination, the therapeutic agents can be formulated for simultaneous or sequential administration at different times, or the therapeutic agents can be administered as a single composition. By "combination therapy", it refers to the use of a compound of

the disclosure in combination with another agent in the form of co-administration of each agent or sequential administration of each agent, in either case, for the purpose of achieving the optimal results. Co-administration includes dosage form for simultaneous delivery, as well as separate dosage forms for each compound. Thus, administration of the compounds of the disclosure can be combined with other therapies known in the art, for example, radiation therapy or cytostatic agents, cytotoxic agents, other anticancer agents, and the like as used in the treatment of cancer, in order to improve the symptoms of cancer. The administration sequence is not limited in the present disclosure. The compounds of the present disclosure may be administered before, simultaneously, or after other anticancer or cytotoxic agents.

To prepare the pharmaceutical ingredient of the present disclosure, one or more compounds of formula (I) or salts thereof as an active ingredient can be intimately mixed with a pharmaceutical carrier, which is carried out according to a conventional pharmaceutical formulation technique. The carrier can be used in a wide variety of forms depending on the form of preparation which is designed for different administration modes (for example, oral or parenteral administration). Suitable pharmaceutically acceptable carriers are well known in the art. A description of some of these pharmaceutically acceptable carriers can be found in the Handbook of Pharmaceutical Excipients, published jointly by the American Pharmaceutical Association and the Pharmaceutical Society of Great Britain.

The pharmaceutical composition of the present disclosure may have the following forms, for example, those suitable for oral administration, such as tablets, capsules, pills, powders, sustained release forms, solutions or suspensions; those for parenteral injections such as clear solutions, suspensions, emulsion; or those for topical use such as ointments, creams; or as a suppository for rectal administration. The pharmaceutical ingredients may also be presented in unit dosage form for single administration in a precise dosage. The pharmaceutical ingredient will include a conventional pharmaceutical carrier or excipient and a compound as an active ingredient prepared according to the present disclosure, and may also include other medical or pharmaceutical preparations, carriers, adjuvants, and the like.

Therapeutic compounds can also be administered to mammals other than humans. The drug dosage for a mammal will depend on the species of the animal and its disease condition or its disordered condition. The therapeutic compound can be administered to the animal in the form of a capsule, a bolus, or a tablet or liquid. The therapeutic compound can also be introduced into the animal by injection or infusion. These drug forms are prepared in a traditional manner complying with standard veterinary practice. As an alternative, the therapeutic compounds can be mixed with the animal feed and fed to the animal, so that the concentrated feed additive or premix can be prepared by mixing ordinary animal feed.

It is a further object of the present disclosure to provide a therapeutically effective amount of a composition containing the compound of the present disclosure for use in treating cancer in a subject in need thereof.

The present disclosure also includes the use of the compound of the present disclosure or a pharmaceutically acceptable derivative thereof, in the manufacture of medicaments for treating RIP1 related diseases, including ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic myeloid leukemia, acute myeloid leukemia, non-Hodgkin's lymphoma, nasopharyngeal cancer, esophageal cancer, brain tumor, B-cell and T-cell lymphoma, lymphoma, multiple myeloma, biliary cancer and sarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behget's disease, interleukin-1 converzyme-related fever syndrome, chronic obstructive pulmonary disease, tumor necrosis factor receptor related periodic syndrome and periodontitis.

The present disclosure also provides a method for preparing the corresponding compounds. A variety of synthetic methods can be used to prepare the compounds described herein, including the method involved in the following examples. The compounds of the present disclosure, or pharmaceutically acceptable salts, diastereomers, enantiomers or hydrates thereof, can be synthesized using the following methods, synthetic methods known in the field of organic chemical synthesis, or variations of these methods understood by those skilled in the art. Preferred methods include, but are not limited to, the following methods.

In order to make the objectives, technical solutions and advantages of the present disclosure more clear, the present disclosure will be further described in detail below in conjunction with specific examples. It should be understood that the specific examples described here are only used to explain the present disclosure and are not intended to limit the present disclosure. If no specific technology or conditions are indicated in examples, the technology or conditions

described in the literature in the art or the product specification shall be followed. If reagents or instruments used do not indicate manufacturers, they are all conventional products that are commercially available. The term "and/or" as used herein includes any and all combinations of one or more related listed items. The Example provided below can better illustrate the present disclosure, unless otherwise specified, all temperatures are in °C. The names of some compounds in this disclosure are generated by Chemdraw and translated into Chinese.

**Preparation of some intermediates**

I. Preparation of A series intermediates

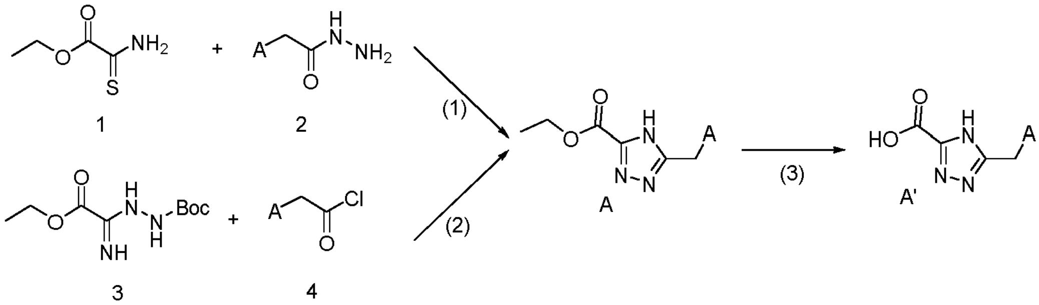


A series intermediates may include A and A', where A' can be further formed by hydrolysis of A. A can be prepared by the above two routes. In Route 2, Intermediate A is generated by the reaction of compound 3 and acid chloride 4 in a pyridine solvent at 110 degrees, and in Route 1, Intermediate A is synthesized by refluxing compound 1 and hydrazide 2.

**Synthesis of Intermediate A-1** (ethyl 5-benzyl-4H-1,2,4-triazole-3-carboxylate) and **A-2** (5-benzyl-4H-1,2,4-triazole-3-carboxylic acid)

The synthetic method refers to WO2014125444A1.

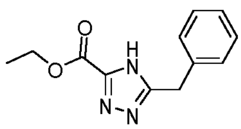
A-1

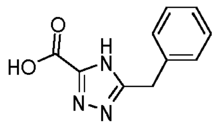
A-2

**Synthesis of Intermediate A-3** (ethyl 5-(1-phenylethyl)-4H-1,2,4-triazole-3-carboxylate) and **A-4** (ethyl 5-(1-phenylethyl)-1,3,4-oxadiazole-2-carboxylate)

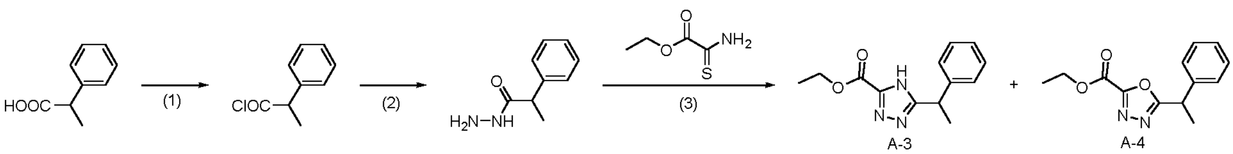


Step 1: Synthesis of 2-phenylpropionyl chloride

2-phenylpropionic acid (1g, 6.66mmol) was dissolved in thionyl chloride (10mL), and reacted at 85°C for 1 hour. The reaction solution was diluted with toluene and spin dried. The crude product was used directly in the next step.

Step 2: Synthesis of 2-phenylpropionyl hydrazide

2-phenylpropionyl chloride was dissolved in methanol (20mL), and reacted at 25°C for 2 hours. Hydrazine hydrate (10mL) was then added, and reacted at 80°C for 16 hours. The reaction solution was cooled, extracted with ethyl acetate, washed with saturated brine, dried over anhydrous sodium sulfate, filtered and evaporated to dryness, to afford 900mg of colorless oil. MS: 165 $[M+H]^+$

Step 3: Synthesis of A-3 and A-4

2-phenylpropionyl hydrazide (900mg, 5.5mmol) was dissolved in ethanol (15mL), ethyl thiooxamate (805mg, 6mmol) was adde, and reacted at 70°C for 3 hours. The reaction solution was cooled and filtered to afford 800mg of white solid. Xylene (20mL) was added to the solid, and reacted at 160°C under refluxing to remove water for 24 hours. The reaction solution was cooled and concentrated, the crude product was rinsed with mixture of petroleum ether/ethyl acetate (10:1), to afford 300mg of ethyl 5-(1-phenylethyl)-4H-1,2,4-triazol-3-carboxylate as white solid. MS: 246 $[M+H]^+$. The mother liquor was evaporated to dryness to afford 200mg of ethyl 5-(1-phenylethyl)-1,3,4-oxadiazol-2-carboxylate as yellow solid. MS: 247 $[M+H]^+$.

**Synthesis of Intermediate A-5** (ethyl 5-(cyclopentylmethyl)-4H-1,2,4-triazole-3-carboxylate) and **A-6** (ethyl 5-(cyclopentylmethyl)-1,3,4-oxadiazol-2-carboxylate)

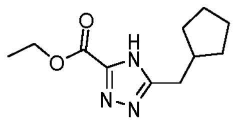

A-5

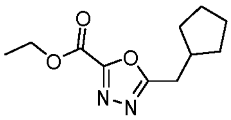

A-6

The preparation was carried out in a similar manner to intermediate A-3 and A-4 (step 2 to step 3), except that in step 2, cyclopentylacetyl chloride was used in place of 2-phenylpropionyl chloride.

**Synthesis of Intermediate A-7** ethyl 5-(morpholinomethyl)-4H-1,2,4-triazole-3-carboxylate

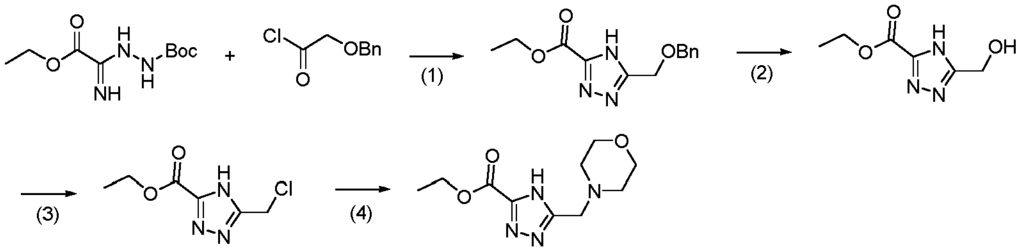


Step 1: Tert-butyl 2-(2-ethoxy-1-imino-2-oxoethyl)hydrazine-1-carboxylate and 2-(benzyloxy)acetyl chloride in pyridine were reacted at 110°C to afford ethyl 5-((benzyloxy)methyl)-4H-1,2,4-triazol-3-carboxylate (see M. V Chudinov et al./ Bioorg. Med. Chem. Lett. 26(2016) 3223-3225 for detailed operations)

Step 2: Synthesis of Ethyl 5-(hydroxymethyl)-4H-1,2,4-triazol-3-carboxylate

Ethyl 5-((benzyloxy)methyl)-4H-1,2,4-triazole-3-carboxylate (250mg, 1mmol) was dissolved in a mixture of ethanol and ethyl acetate (2:1, 3mL), palladium on carbon (10%, 25mg) was added, and was reacted for 5 hours under hydrogen atmosphere. The reaction was filtered through diatomaceous earth and the filtrate was evaporated to get 150mg of yellow solid. MS: 172 $[M+H]^+$

Step 3: Synthesis of ethyl 5-(chloromethyl)-4H-1,2,4-triazole-3-carboxylate

Ethyl 5-(hydroxymethyl)-4H-1,2,4-triazole-3-carboxylate (150mg, 0.9mmol) was dissolved in thionyl chloride (2mL), and reacted at 85°C for 7 hours. The reaction was cooled and diluted with toluene, and the resultant was spin dried to afford 160mg of yellow oil. MS: 190 $[M+H]^+$

Step 4: Synthesis of ethyl 5-(morpholinomethyl)-4H-1,2,4-triazole-3-carboxylate

Ethyl 5-(chloromethyl)-4H-1,2,4-triazole-3-carboxylate (160mg, 0.9mmol) was dissolved in tetrahydrofuran (2mL), diisopropylethylamine (220mg, 1.7mmol) and morpholine (150mg, 1.7mmol) were added, and reacted at 25°C for 5 hours. The reaction solution was evaporated to dryness, and purified by column chromatography to get 180mg of yellow solid. MS: 241 $[M+H]^+$

Table 1 below shows the structure and synthesis of intermediates A-8 to A-28 and mass spectral data thereof, wherein intermediates A-8 to A-12 were prepared in a similar manner to intermediate A-3, without collecting the oxadiazole by-product, and the synthesis of intermediates A-13 and A-28 were carried out in a similar manner to the first step of synthesis of intermediate A-7.

Table 1. Structures and synthesis of intermediates A-8 to A-28 and mass spectral data thereof

| Intermediate | Structure | Name | Synthesis | MS: $[M+H]^+$ |
|---|---|---|---|---|
| A-8 | 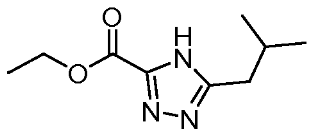 | Ethyl 5-isobutyl-4H-1,2,4-triazol-5-carboxylate | The operation was the same as the steps 2 to 3 of the preparation of intermediate A-3, but 3-methylbutyryl chloride was used in place of 2-phenylpropionyl chloride | 198 |
| A-9 | 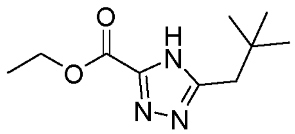 | Ethyl 5-neopentyl-4H-1,2,4-triazol-5-carboxylate | The operation was the same as the steps 2 to 3 of the preparation of intermediate A-3, but 3,3-dimethylbutyryl chloride was used in place of 2-phenylpropionyl chloride | 212 |
| A-10 | 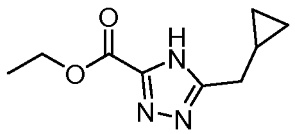 | Ethyl 5-(cyclopropylmethyl)-4H-1,2,4-triazol-5-carboxylate | The operation was the same as the steps 1 to 3 of the preparation of intermediate A-3, but 2-cyclopropylacetic acid was used in place of 2-phenylpropionic acid | 196 |
| A-11 | 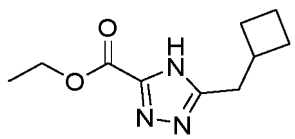 | Ethyl 5-(cyclobutylmethyl)-4H-1,2,4-triazol-5-carboxylate | The operation was the same as the steps 1 to 3 of the preparation of intermediate A-3, but 2-cyclobutylacetic acid was used in place of 2-phenylpropionic acid | 210 |
| A-12 | 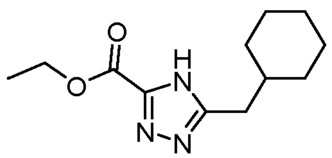 | Ethyl 5-(cyclohexylmethyl)-4H-1,2,4-triazol-3-carboxylate | The operation was the same as the steps 1 to 3 of the preparation of intermediate A-3, but cyclohexylacetic acid was used in place of 2-phenylpropionic acid | 238 |

(continued)

| Intermediate | Structure | Name | Synthesis | MS: [M+H]+ |
|---|---|---|---|---|
| A-13 | 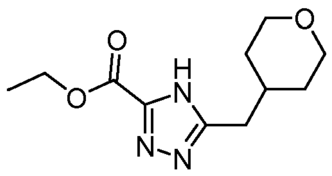 | Ethyl 5-((tetrahydro-2H-pyran-4-yl)methyl)-4H-1,2,4-triazol-3-carboxylate | The operation was the same as the step 1 of the preparation of intermediate A-7, but 2-(tetrahydro-2H-pyran-4-yl)acetyl chloride was used in place of 2-(benzyloxy)acetyl chloride | 240 |
| A-14 | 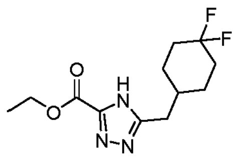 | Ethyl 5-((4,4-difluorocyclohexyl) methyl )-4H-1,2,4-triazol-3-carboxylate | The operation was the same as the step 1 of the preparation of intermediate A-7, but 2-(4,4-difluorocyclohexyl) acetyl chloride was used in place of 2-(benzyloxy) acetyl chloride | 274 |
| A-15 | 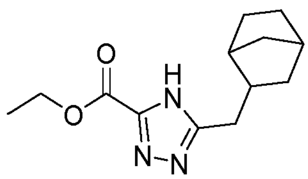 | Ethyl 5-(bicyclo[2.2.1] heptan-2-ylmethyl)-4H-1,2,4-triazol-3 -carboxylate | The operation was the same as the step 1 of the preparation of intermediate A-7, but 2-(bicyclo[2.2.1]heptan-2-yl)acetyl chloride was used in place of 2-(benzyloxy)acetyl chloride | 250 |
| A-16 | 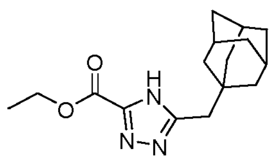 | Ethyl 5-(adamantan-1-yl)methyl)-4H-1,2,4-triazol-3 -carboxylate | The operation was the same as the step 1 of the preparation of intermediate A-7, but 2-(adamantan-1-yl)acetyl chloride was used in place of 2-(benzyloxy)acetyl chloride | 290 |
| A-17 | 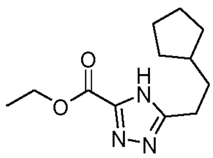 | Ethyl 5-(2-cyclopentylethyl)-4H-1,2,4-triazol-3-carboxylate | The operation was the same as the step 1 of the preparation of intermediate A-7, but 3-cyclopentylpropionyl chloride was used in place of 2-(benzyloxy)acetyl chloride | 238 |
| A-18 | 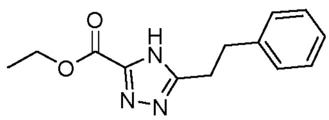 | Ethyl 5-(phenethyl)-4H-1,2,4-triazol-3-carboxylate | The operation was the same as the step 1 of the preparation of intermediate A-7, but 3-phenylpropionyl chloride was used in place of 2-(benzyloxy)acetyl chloride | 246 |

(continued)

| Intermediate | Structure | Name | Synthesis | MS: $[M+H]^+$ |
|---|---|---|---|---|
| A-19 | 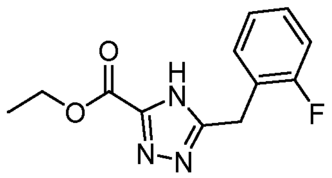 | Ethyl 5-(2-fluorobenzyl)-4H-1,2,4-triazol-3-carboxylate | The operation was the same as the step 1 of the preparation of intermediate A-7, but 2-(2-fluorophenyl)acetyl chloride was used in place of 2-(benzyloxy)acetyl chloride | 250 |
| A-20 | 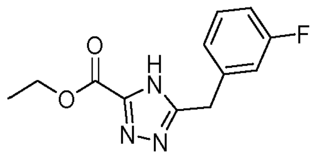 | Ethyl 5-(3-fluorobenzyl)-4H-1,2,4-triazol-3-carboxylate | The operation was the same as the step 1 of the preparation of intermediate A-7, but 2-(3-fluorophenyl)acetyl chloride was used in place of 2-(benzyloxy)acetyl chloride | 250 |
| A-21 | 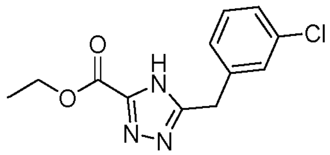 | Ethyl 5-(3-chlorobenzyl)-4H-1,2,4-triazol-3-carboxylate | The operation was the same as the step 1 of the preparation of intermediate A-7, but 2-(3-chlorophenyl)acetyl chloride was used in place of 2-(benzyloxy)acetyl chloride | 266 |
| A-22 | 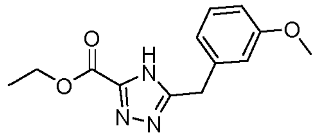 | Ethyl 5-(3-methoxybenzyl)-4H-1,2,4-triazol-3 -carboxylate | The operation was the same as the step 1 of the preparation of intermediate A-7, but 2-(3-methoxyphenyl) acetyl chloride was used in place of 2-(benzyloxy) acetyl chloride | 262 |
| A-23 | 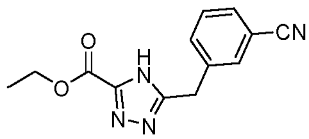 | Ethyl 5-(3-cyanobenzyl)-4H-1,2,4-triazol-3-carboxylate | The operation was the same as the step 1 of the preparation of intermediate A-7, but 2-(3-cyanophenyl)acetyl chloride was used in place of 2-(benzyloxy)acetyl chloride | 257 |
| A-24 | 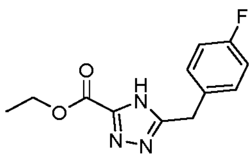 | Ethyl 5-(4-fluorobenzyl)-4H-1,2,4-triazol-3-carboxylate | The operation was the same as the step 1 of the preparation of intermediate A-7, but 2-(4-fluorophenyl)acetyl chloride was used in place of 2-(benzyloxy)acetyl chloride | 250 |

(continued)

| Intermediate | Structure | Name | Synthesis | MS: $[M+H]^+$ |
|---|---|---|---|---|
| A-25 | 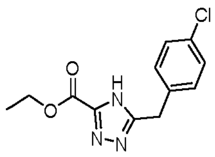 | Ethyl 5-(4-chlorobenzyl)-4H-1,2,4-triazol-3-carboxylate | The operation was the same as the step 1 of the preparation of intermediate A-7, but 2-(4-chlorophenyl)acetyl chloride was used in place of 2-(benzyloxy)acetyl chloride | 266 |
| A-26 | 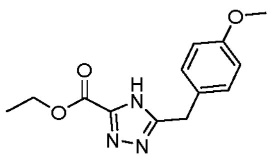 | Ethyl 5-(4-methoxybenzyl)-4H-1,2,4-triazol-3 -carboxylate | The operation was the same as the step 1 of the preparation of intermediate A-7, but 2-(4-methoxyphenyl) acetyl chloride was used in place of 2-(benzyloxy) acetyl chloride | 262 |
| A-27 | 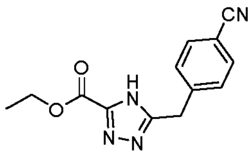 | Ethyl 5-(4-cyanobenzyl)-4H-1,2,4-triazol-3-carboxylate | The operation was the same as the step 1 of the preparation of intermediate A-7, but 2-(4-cyanophenyl)acetyl chloride was used in place of 2-(benzyloxy)acetyl chloride | 257 |
| A-28 | 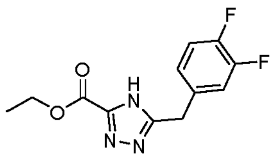 | Ethyl 5-(3,4-difluorobenzyl)-4H-1,2,4-triazol-3 -carboxylate | The operation was the same as the step 1 of the preparation of intermediate A-7, but 2-(3,4-difluorobenzyl) acetyl chloride was used in place of 2-(benzyloxy) acetyl chloride | 268 |

Synthesis of Intermediate A-29 methyl 5-benzyl-1,3,4-thiadiazole-2-carboxylate

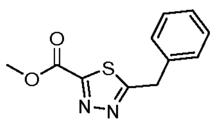

Phenylacetylhydrazine (5g, 33.3mmol) was dissolved in anhydrous dichloromethane (100mL), triethylamine (6.74g, 66.7mmol) was added, and under argon protection, methyl oxalyl chloride (4.1g, 33.5mmol) was added dropwise at 0°C. The reaction solution was slowly warmed to 25°C. After 16 hours reaction, the solvent was evaporated to dryness, rinsed with mixture of petroleum ether/ethyl acetate, filtered, and the filter cake was re-dissolved in toluene (100mL), Lawson's reagent (26.9g, 66.5mmol) was added, heated to 110°C and reacted for 6 hours. The reaction was cooled and diluted with ethyl acetate, washed with water, saturated aqueous solution of sodium bicarbonate and brine, the organic phase was dried over anhydrous sodium sulfate, filtered and evaporated to dryness, and purified by column chromatography to get 4g product. MS: 235 $[M+H]^+$.

**Examples**

Example 1: 5-benzyl-N-(4-phenylpyridin-2-yl)-4H-1,2,4-triazole-3-carboxamide

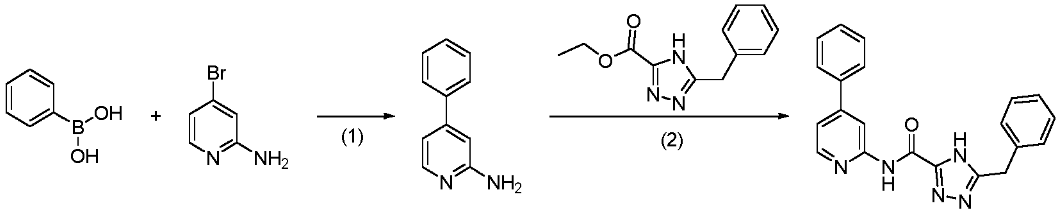


Step 1: Synthesis of 4-phenylpyridin-2-amine

4-Bromopyridin-2-amine (173mg, 1mmol), phenylboronic acid (146mg, 1.2mmol), Pd(dppf)$Cl_2$ (73mg, 0.1mmol) and sodium carbonate (160mg, 1.5mmol) were placed in a mixture of dioxane ( 4mL) and water (0.8mL), which was reacted at 100°C for 16 hours under argon protection. After cooling, the reaction solution was evaporated to dryness and subjected to column chromatography to afford 120mg of brown solid. MS: 171 $[M+H]^+$.

Step 2: Synthesis of 5-benzyl-N-(4-phenylpyridin-2-yl)-4H-1,2,4-triazole-3-carboxamide

4-Phenylpyridin-2-amine (120mg, 0.7mmol) was placed in dry xylene (3mL), to which trimethyl aluminum (3M, 0.7mL) was slowly added under argon protection, and reacted at 25°C for 1 hour. Then, intermediate 1 (197mg, 0.9mmol) was added, and reacted at 100°C for 16 hours. The reaction solution was cooled and diluted with methanol, evaporated to dryness and subjected to column chromatography and preparative liquid chromatography to afford 50mg of white solid. $^1$H NMR (400 MHz, DMSO-$d_6$) $\delta$ 14.65 (s, 1H), 9.90 (s, 1H), 8.49 - 8.38 (m, 2H), 7.82 - 7.71 (m, 2H), 7.62 - 7.45 (m, 4H), 7.40 - 7.30 (m, 4H), 7.30 - 7.18 (m, 1H), 4.18 (s, 2H). MS: 356 $[M+H]^+$.

Example 2: 5-benzyl-N-(4-(o-methylphenyl)pyridin-2-yl)-4H-1,2,4-triazole-3-carboxamide

The preparation was carried out in a similar manner to Example 1, except that in step 1, o-methylphenylboronic acid was used in place of phenylboronic acid. $^1$H NMR (400 MHz, DMSO-$d_6$) $\delta$ 14.55 (s, 1H), 9.86 (s, 1H), 8.42 (d, $J$ = 5.1 Hz, 1H), 8.13 (s, 1H), 7.51 - 7.34 (m, 3H), 7.34 - 7.30 (m, 4H), 7.30 - 7.23 (m, 2H), 7.21 (d, $J$ = 5.1 Hz, 1H), 4.19 (s, 2H), 2.28 (s, 3H). MS: 370 $[M+H]^+$.

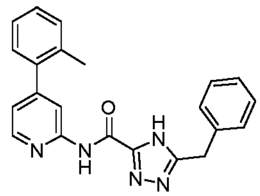

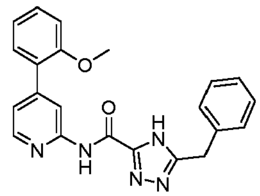

Example 2 Example 3

Example 3: 5-benzyl-N-(4-(2-methoxyphenyl)pyridin-2-yl)-4H-1,2,4-triazole-3-carboxamide

The preparation was carried out in a similar manner to Example 1, except that in step 1, o-methoxyphenylboronic acid was used in place of phenylboronic acid. $^1$H NMR (400 MHz, DMSO-$d_6$) $\delta$ 14.58 (s, 1H), 9.82 (s, 1H), 8.37 (d, $J$ = 5.2 Hz, 1H), 8.33 - 8.17 (m, 1H), 7.60 - 6.91 (m, 10H), 4.18 (s, 2H), 3.81 (s, 3H). MS: 386 $[M+H]^+$.

Example 4: 5-benzyl-N-(4-(2-ethoxyphenyl)pyridin-2-yl)-4H-1,2,4-triazole-3-carboxamide

The preparation was carried out in a similar manner to Example 1, except that in step 1, o-ethoxyphenylboronic acid was used in place of phenylboronic acid. $^1$H NMR (400 MHz, DMSO-$d_6$) $\delta$ 14.60 (s, 1H), 9.80 (s, 1H), 8.62 - 8.40 (m, 1H), 8.38 (d, $J$ = 5.2 Hz, 1H), 7.60 - 6.94 (m, 10H), 4.18 (s, 2H), 4.10 (q, $J$ = 7.0 Hz, 2H), 1.32 (t, $J$ = 6.9 Hz, 3H).

MS: 400 $[M+H]^+$.

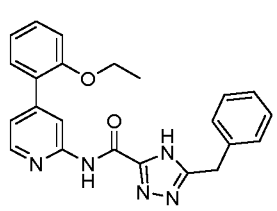
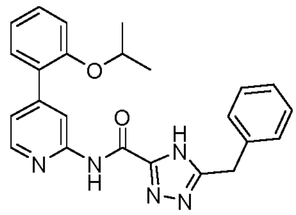

Example 4 Example 5

Example 5: 5-benzyl-N-(4-(2-isopropyloxyphenyl)pyridin-2-yl)-4H-1,2,4-triazole-3-carboxamide

The preparation was carried out in a similar manner to Example 1, except that in step 1, 2-isopropyloxyphenylboronic acid was used in place of phenylboronic acid. $^1$H NMR (400 MHz, DMSO-$d_6$) δ 14.55 (s, 1H), 9.74 (s, 1H), 8.56 - 8.26 (m, 2H), 7.45 - 7.38 (m, 2H), 7.38 - 7.29 (m, 5H), 7.27 (d, *J* = 7.3 Hz, 1H), 7.19 (d, *J* = 8.6 Hz, 1H), 7.09 - 7.02 (m, 1H), 4.69 (p, *J* = 6.0 Hz, 1H), 4.19 (s, 2H), 1.27 (d, *J* = 6.0 Hz, 6H). MS: 414 $[M+H]^+$.

Example 6: 5-benzyl-N-(4-(2-(trifluoromethoxy)phenyl)pyridin-2-yl)-4H-1,2,4-triazole-3-carboxamide

The preparation was carried out in a similar manner to Example 1, except that in step 1, 2-(trifluoromethoxy)phenylboronic acid was used in place of phenylboronic acid. $^1$H NMR (400 MHz, DMSO-$d_6$) δ 14.64 (s, 1H), 9.96 (s, 1H), 8.47 (d, *J* = 5.2 Hz, 1H), 8.30 (d, *J* = 1.5 Hz, 1H), 7.70-7.51 (m, 4H), 7.41-7.18 (m, 6H), 4.18 (s, 2H). MS: 440 $[M+H]^+$.

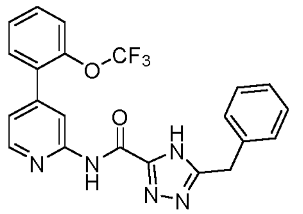
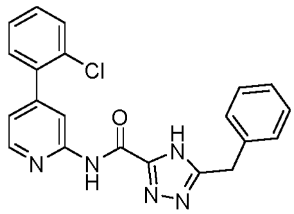

Example 6 Example 7

Example 7: 5-benzyl-N-(4-(2-chlorophenyl)pyridin-2-yl)-4H-1,2,4-triazole-3-carboxamide

The preparation was carried out in a similar manner to Example 1, except that in step 1, 2-chlorophenylboronic acid was used in place of phenylboronic acid. $^1$H NMR (600 MHz, DMSO-$d_6$) δ 14.65 (s, 1H), 9.95 (s, 1H), 8.46 (d, *J* = 5.1 Hz, 1H), 8.23 (d, *J* = 1.5 Hz, 1H), 7.68 - 7.60 (m, 1H), 7.56 - 7.46 (m, 3H), 7.39 -7.30 (m, 4H), 7.30 - 7.21 (m, 2H), 4.18 (s, 2H). MS: 390 $[M+H]^+$.

Example 8: 5-benzyl-N-(4-(2-cyclopropylphenyl)pyridin-2-yl)-4H-1,2,4-triazole-3-carboxamide

The preparation was carried out in a similar manner to Example 1, except that in step 1, 2-cyclopropylphenylboronic acid was used in place of phenylboronic acid. $^1$H NMR (400 MHz, DMSO-$d_6$) δ 14.56 (s, 1H), 9.87 (s, 1H), 8.43 (d, *J* = 5.1 Hz, 1H), 8.27 (s, 1H), 7.42 - 7.30 (m, 5H), 7.30 -7.20 (m, 4H), 7.10 - 7.01 (m, 1H), 4.18 (s, 2H), 1.91 - 1.77 (m, 1H), 0.95 - 0.81 (m, 2H), 0.79 - 0.63 (m, 2H). MS: 396 $[M+H]^+$.

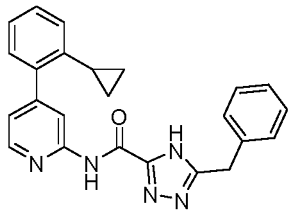
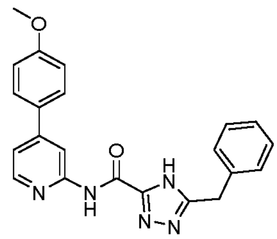

Example 8 Example 9

Example 9: 5-benzyl-N-(4-(4-methoxyphenyl)pyridin-2-yl)-4H-1,2,4-triazole-3-carboxamide

The preparation was carried out in a similar manner to Example 1, except that in step 1, 4-methoxyphenylboronic acid was used in place of phenylboronic acid. $^1$H NMR (400 MHz, DMSO-$d_6$) δ 14.60 (s, 1H), 9.82 (s, 1H), 8.48 - 8.29 (m, 2H), 7.80 - 7.66 (m, 2H), 7.47 (dd, *J* = 5.2, 1.8 Hz, 1H), 7.42 - 7.18 (m, 5H), 7.18 - 6.98 (m, 2H), 4.18 (s, 2H), 3.83 (s, 3H). MS: 386 $[M+H]^+$.

Example 10: 5-benzyl-N-(4-(4-methoxy-2-methylphenyl)pyridin-2-yl)-4H-1,2,4-triazole-3-carboxamide

The preparation was carried out in a similar manner to Example 1, except that in step 1, 4-methoxy-2-methylphenylboronic acid was used in place of phenylboronic acid. $^1$H NMR (400 MHz, DMSO-$d_6$) δ 14.59 (s, 1H), 9.85 (s, 1H), 8.38 (d, *J* = 5.1 Hz, 1H), 8.10 (s, 1H), 7.47 -7.02 (m, 7H), 7.04 - 6.76 (m, 2H), 4.18 (s, 2H), 3.80 (s, 3H), 2.29 (s, 3H). MS: 400 $[M+H]^+$.

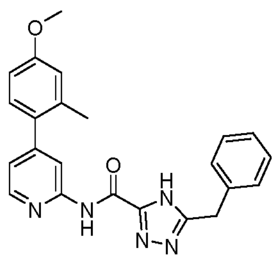
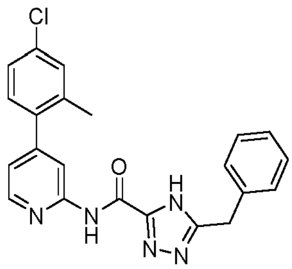

Example 10 Example 11

Example 11: 5-benzyl-N-(4-(4-chloro-2-methylphenyl)pyridin-2-yl)-4H-1,2,4-triazole-3-carboxamide

The preparation was carried out in a similar manner to Example 1, except that in step 1, 4-chloro-2-methylphenylboronic acid was used in place of phenylboronic acid. $^1$H NMR (400 MHz, DMSO-$d_6$) δ 14.59 (s, 1H), 9.93 (s, 1H), 8.43 (d, *J* = 5.1 Hz, 1H), 8.10 (s, 1H), 7.48 (d, *J* = 2.1 Hz, 1H), 7.43-7.11 (m, 8H), 4.18 (s, 2H), 2.28 (s, 3H). MS: 404 $[M+H]^+$.

Example 12: 5-benzyl-N-(4-(4-cyano-2-methylphenyl)pyridin-2-yl)-4H-1,2,4-triazole-3-carboxamide

The preparation was carried out in a similar manner to Example 1, except that in step 1, 4-cyano-2-methylphenylboronic acid was used in place of phenylboronic acid. $^1$H NMR (400 MHz, DMSO-$d_6$) δ 14.60 (s, 1H), 10.00 (s, 1H), 8.47 (d, *J* = 5.1 Hz, 1H), 8.12 (s, 1H), 7.89 (s, 1H), 7.81 (d, *J* = 7.8 Hz, 1H), 7.49 (d, *J* = 7.9 Hz, 1H), 7.41 - 7.29 (m, 4H), 7.29 - 7.15 (m, 2H), 4.18 (s, 2H), 2.30 (s, 3H). MS: 395 $[M+H]^+$.

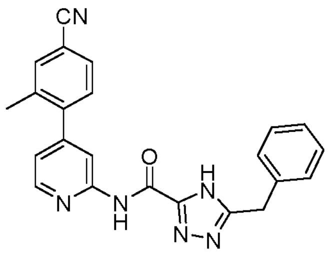

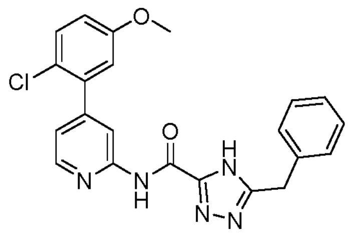

Example 12 Example 13

Example 13: Synthesis of 5-benzyl-N-(4-(2-chloro-5-methoxyphenyl)pyridin-2-yl)-4H-1,2,4-triazole-3-carboxamide

The preparation was carried out in a similar manner to Example 1, except that in step 1, 2-chloro-5-methoxyphenylboronic acid was used in place of phenylboronic acid. $^1$H NMR (600 MHz, DMSO-$d_6$) δ 14.56 (s, 1H), 9.91 (s, 1H), 8.46 (d, *J* = 5.1 Hz, 1H), 8.23 (s, 1H), 7.53 (d, *J* = 8.8 Hz, 1H), 7.45 - 7.30 (m, 4H), 7.30 - 7.19 (m, 2H), 7.08 (dd, *J* = 8.8, 3.1 Hz, 1H), 7.03 (d, *J* = 3.0 Hz, 1H), 4.18 (s, 2H), 3.81 (s, 3H). MS: 420 $[M+H]^+$.

Example 14: 5-benzyl-N-(4-(5-fluoro-2-methoxyphenyl)pyridin-2-yl)-4H-1,2,4-triazole-3-carboxamide

The preparation was carried out in a similar manner to Example 1, except that in step 1, 5-fluoro-2-methoxyphenylboronic acid was used in place of phenylboronic acid. $^1$H NMR (400 MHz, DMSO-$d_6$) δ 14.60 (s, 1H), 9.87 (s, 1H), 8.40 (d, *J* = 5.2 Hz, 1H), 8.32 (s, 1H), 7.51 -7.03 (m, 9H), 4.18 (s, 2H), 3.80 (s, 3H). MS: 404 $[M+H]^+$.

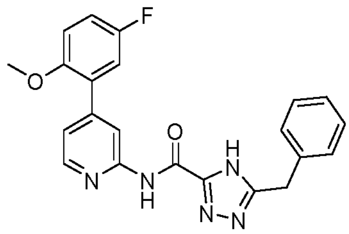

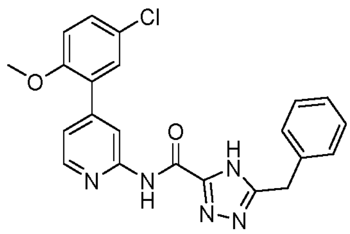

Example 14 Example 15

Example 15: 5-benzyl-N-(4-(5-chloro-2-methoxyphenyl)pyridin-2-yl)-4H-1,2,4-triazole-3-carboxamide

The preparation was carried out in a similar manner to Example 1, except that in step 1, 5-chloro-2-methoxyphenylboronic acid was used in place of phenylboronic acid. $^1$H NMR (400 MHz, DMSO-$d_6$) δ 14.57 (s, 1H), 9.86 (s, 1H), 8.39 (d, *J* = 5.2 Hz, 1H), 8.28 (s, 1H), 7.50 (dd, *J* = 8.8, 2.7 Hz, 1H), 7.43 (d, *J* = 2.7 Hz, 1H), 7.40 - 7.29 (m, 5H), 7.29 - 7.15 (m, 2H), 4.18 (s, 2H), 3.81 (s, 3H). MS: 420 $[M+H]^+$.

Example 16: 5-benzyl-N-(4-(2-methoxy-5-methylphenyl)pyridin-2-yl)-4H-1,2,4-triazole-3-carboxamide

The preparation was carried out in a similar manner to Example 1, except that in step 1, 2-methoxy-5-methylphenylboronic acid was used in place of phenylboronic acid. $^1$H NMR (400 MHz, DMSO-$d_6$) δ 14.55 (s, 1H), 9.79 (s, 1H), 8.36 (d, *J* = 5.2 Hz, 1H), 8.28 (s, 1H), 7.38 - 7.29 (m, 5H), 7.29 - 7.21 (m, 2H), 7.20 (d, *J* = 2.2 Hz, 1H), 7.07 (d, *J* = 8.4 Hz, 1H), 4.18 (s, 2H), 3.77 (s, 3H), 2.31 (s, 3H). MS: 400 $[M+H]^+$.

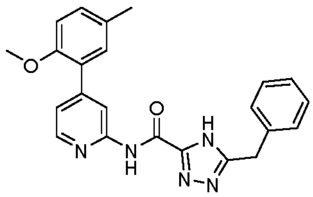 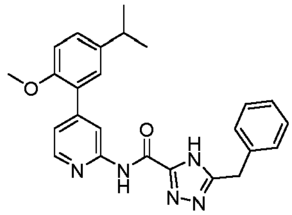

Example 16 Example 17

Example 17: 5-benzyl-N-(4-(5-isopropyl-2-methoxyphenyl)pyridin-2-yl)-4H-1,2,4-triazole-3-carboxamide

The preparation was carried out in a similar manner to Example 1, except that in step 1, 5-isopropyl-2-methoxyphenylboronic acid was used in place of phenylboronic acid. $^1$H NMR (400 MHz, DMSO-$d_6$) δ 14.62 (s, 1H), 9.84 (s, 1H), 8.37 (d, *J* = 5.2 Hz, 1H), 8.29 (d, *J* = 1.5 Hz, 1H), 7.41 - 7.19 (m, 8H), 7.09 (d, *J* = 8.5 Hz, 1H), 4.18 (s, 2H), 3.78 (s, 3H), 2.91 (p, *J* = 6.9 Hz, 1H), 1.21 (d, *J* = 6.9 Hz, 6H). MS: 428 [M+H]$^+$.

Example 18: 5-benzyl-N-(4-(2,5-dimethoxyphenyl)pyridin-2-yl)-4H-1,2,4-triazole-3-carboxamide

The preparation was carried out in a similar manner to Example 1, except that in step 1, 2,5-dimethoxyphenylboronic acid was used in place of phenylboronic acid. $^1$H NMR (400 MHz, DMSO-$d_6$) δ 14.62 (s, 1H), 9.83 (s, 1H), 8.38 (d, *J* = 5.2 Hz, 1H), 8.29 (d, *J* = 1.5 Hz, 1H), 7.45 - 7.29 (m, 5H), 7.29 - 7.22 (m, 1H), 7.12 (d, *J* = 9.0 Hz, 1H), 7.02 (dd, *J* = 9.0, 3.1 Hz, 1H), 6.95 (d, *J* = 3.1 Hz, 1H), 4.18 (s, 2H), 3.76 (s, 3H), 3.75 (s, 3H). MS: 416 [M+H]$^+$.

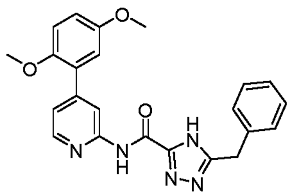 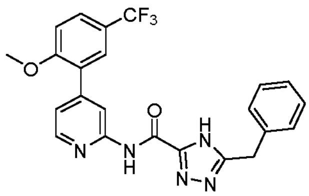

Example 18 Example 19

Example 19: 5-benzyl-N-(4-(2-methoxy-5-(trifluoromethyl)phenyl)pyridin-2-yl)-4H-1,2,4-triazole-3-carboxamide

The preparation was carried out in a similar manner to Example 1, except that in step 1, 2-methoxy-5-(trifluoromethyl)phenylboronic acid was used in place of phenylboronic acid. $^1$H NMR (400 MHz, DMSO-$d_6$) δ 14.55 (s, 1H), 9.86 (s, 1H), 8.42 (d, *J* = 5.2 Hz, 1H), 8.29 (s, 1H), 7.83 (dd, *J* = 8.9, 2.3 Hz, 1H), 7.69 (d, *J* = 2.3 Hz, 1H), 7.46 - 7.18 (m, 7H), 4.19 (s, 2H), 3.90 (s, 3H). MS: 454 [M+H]$^+$.

Example 20: 5-benzyl-N-(2-methyl-[3,4'-bipyridin]-2'-yl)-4H-1,2,4-triazole-3-carboxamide

The preparation was carried out in a similar manner to Example 1, except that in step 1, 2-methyl-3-(4,4,5,5-tetramethyl-1,3,2-dioxaborolan-2-yl)pyridine was used in place of phenylboronic acid. $^1$H NMR (400 MHz, DMSO-$d_6$) δ 14.63 (s, 1H), 9.99 (s, 1H), 8.57 (dd, *J* = 4.9, 1.7 Hz, 1H), 8.48 (d, *J* = 5.1 Hz, 1H), 8.18 (d, *J* = 1.5 Hz, 1H), 7.73 (dd, *J* = 7.7, 1.8 Hz, 1H), 7.44 - 7.32 (m, 5H), 7.32 - 7.24 (m, 2H), 4.21 (s, 2H), 2.50 (s, 3H). MS: 371 [M+H]$^+$.

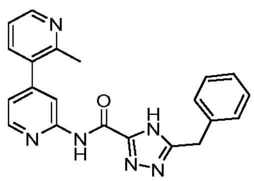 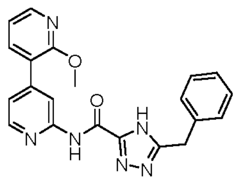

Example 20 Example 21

Example 21: 5-benzyl-N-(2-methoxy-[3,4'-bipyridin]-2'-yl)-4H-1,2,4-triazole-3-carboxamide

The preparation was carried out in a similar manner to Example 1, except that in step 1, (2-methoxypyridin-3-yl)boronic acid was used in place of phenylboronic acid. $^1H$ NMR (400 MHz, DMSO-$d_6$) δ 14.61 (s, 1H), 9.88 (s, 1H), 8.41 (d, *J* = 5.2 Hz, 1H), 8.37 (d, *J* = 1.5 Hz, 1H), 8.28 (dd, *J* = 5.0, 1.9 Hz, 1H), 7.88 (dd, *J* = 7.4, 1.9 Hz, 1H), 7.40 (dd, *J* = 5.2, 1.6 Hz, 1H), 7.38 - 7.29 (m, 4H), 7.29 - 7.21 (m, 1H), 7.17 (dd, *J* = 7.4, 4.9 Hz, 1H), 4.18 (s, 2H), 3.92 (s, 3H). MS: 387 $[M+H]^+$.

Example 22: 5-benzyl-N-(2-ethoxy-[3,4'-bipyridin]-2'-yl)-4H-1,2,4-triazole-3-carboxamide

The preparation was carried out in a similar manner to Example 1, except that in step 1, (2-ethoxypyridin-3-yl)boronic acid was used in place of phenylboronic acid. $^1H$ NMR (400 MHz, DMSO-$d_6$) δ 14.58 (s, 1H), 9.83 (s, 1H), 8.48 (s, 1H), 8.42 (d, *J* = 5.2 Hz, 1H), 8.26 (dd, *J* = 4.9, 1.9 Hz, 1H), 7.90 (dd, *J* = 7.4, 1.9 Hz, 1H), 7.42 (dd, *J* = 5.2, 1.6 Hz, 1H), 7.39 -7.30 (m, 4H), 7.30 - 7.21 (m, 1H), 7.15 (dd, *J* = 7.4, 4.9 Hz, 1H), 4.41 (q, *J* = 7.0 Hz, 2H), 4.18 (s, 2H), 1.34 (t, *J* = 7.0 Hz, 3H). MS: 401 $[M+H]^+$.

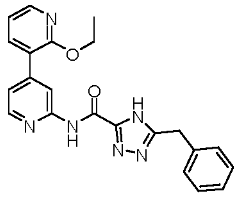
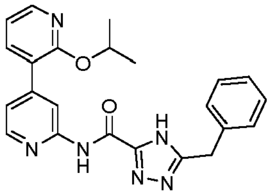

Example 22 Example 23

Example 23: 5-benzyl-N-(2-isopropyloxy-[3,4'-bipyridin]-2'-yl)-4H-1,2,4-triazole-3-carboxamide

The preparation was carried out in a similar manner to Example 1, except that in step 1, (2-isopropyloxypyridin-3-yl)boronic acid was used in place of phenylboronic acid. $^1H$ NMR (400 MHz, DMSO-$d_6$) δ 14.59 (s, 1H), 9.81 (s, 1H), 8.52 (s, 1H), 8.41 (d, *J* = 5.2 Hz, 1H), 8.25 (dd, *J* = 4.9, 1.9 Hz, 1H), 7.89 (dd, *J* = 7.4, 1.9 Hz, 1H), 7.40 (dd, *J* = 5.2, 1.6 Hz, 1H), 7.38 -7.30 (m, 4H), 7.29 - 7.21 (m, 1H), 7.12 (dd, *J* = 7.4, 4.9 Hz, 1H), 5.39 (p, *J* = 6.2 Hz, 1H), 4.18 (s, 2H), 1.33 (d, *J* = 6.2 Hz, 6H). MS: 415 $[M+H]^+$.

Example 24: 5-benzyl-N-(3'-methyl-[4,4'-bipyridin]-2-yl)-4H-1,2,4-triazole-3-carboxamide

The preparation was carried out in a similar manner to Example 1, except that in step 1, (3-methylpyridin-4-yl)boronic acid was used in place of phenylboronic acid. $^1H$ NMR (400 MHz, DMSO-$d_6$) δ 14.58 (s, 1H), 9.97 (s, 1H), 8.58 (s, 1H), 8.53 (d, *J* = 5.0 Hz, 1H), 8.49 (d, *J* = 5.1 Hz, 1H), 8.15 (s, 1H), 7.39 - 7.30 (m, 5H), 7.30 - 7.22 (m, 2H), 4.18 (s, 2H), 2.28 (s, 3H). MS: 371 $[M+H]^+$.

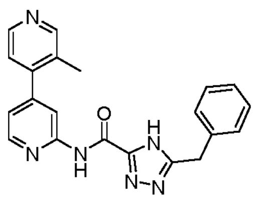
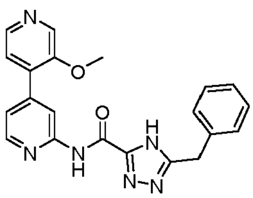

Example 24 Example 25

Example 25: 5-benzyl-N-(3'-methoxy-[4,4'-bipyridin]-2-yl)-4H-1,2,4-triazole-3-carboxamide

The preparation was carried out in a similar manner to Example 1, except that in step 1, (3-methoxypyridin-4-yl)boronic acid was used in place of phenylboronic acid. $^1H$ NMR (400 MHz, DMSO-$d_6$) δ 14.56 (s, 1H), 9.91 (s, 1H), 8.56 (s, 1H), 8.45 (d, *J* = 5.2 Hz, 1H), 8.38 - 8.33 (m, 2H), 7.44 (d, *J* = 4.8 Hz, 1H), 7.39 (dd, *J* = 5.2, 1.6 Hz, 1H), 7.37 - 7.29 (m, 4H), 7.29 - 7.21 (m, 1H), 4.18 (s, 2H), 3.94 (s, 3H). MS: 387 $[M+H]^+$.

Example 26: 5-benzyl-N-(4-(1-methyl-1H-pyrazol-5-yl)pyridin-2-yl)-4H-1,2,4-triazole-3-carboxamide

The preparation was carried out in a similar manner to Example 1, except that in step 1, 1-methyl-5-(4,4,5,5-tetramethyl-1,3,2-dioxaborolan-2-yl)-1H-pyrazole was used in place of phenylboronic acid. $^1$H NMR (400 MHz, DMSO-$d_6$) $\delta$ 14.66 (s, 1H), 10.00 (s, 1H), 8.47 (d, *J* = 5.2 Hz, 1H), 8.28 (d, *J* = 1.4 Hz, 1H), 7.55 (d, *J* = 1.9 Hz, 1H), 7.40 (dd, *J* = 5.2, 1.5 Hz, 1H), 7.38 - 7.29 (m, 4H), 7.29 - 7.21 (m, 1H), 6.63 (d, *J* = 1.9 Hz, 1H), 4.18 (s, 2H), 3.96 (s, 3H). MS: 360 [M+H]$^+$.

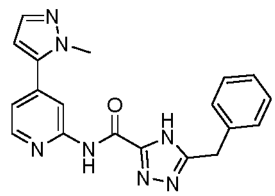

Example 26

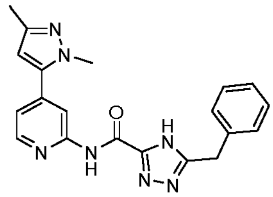

Example 27

Example 27: 5-benzyl-N-(4-(1,3-dimethyl-1H-pyrazol-5-yl)pyridin-2-yl)-4H-1,2,4-triazole-3-carboxamide

The preparation was carried out in a similar manner to Example 1, except that in step 1, 1,3-dimethyl-5-(4,4,5,5-tetramethyl-1,3,2-dioxaborolan-2-yl)-1H-pyrazole was used in place of phenylboronic acid. $^1$H NMR (400 MHz, DMSO-$d_6$) $\delta$ 14.62 (s, 1H), 9.97 (s, 1H), 8.45 (d, *J* = 5.2 Hz, 1H), 8.25 (s, 1H), 7.42 - 7.29 (m, 5H), 7.29 - 7.20 (m, 1H), 6.40 (s, 1H), 4.18 (s, 2H), 3.87 (s, 3H), 2.19 (s, 3H). MS: 374 [M+H]$^+$.

Example 28: 5-benzyl-N-(4-(1-methyl-3-(trifluoromethyl)-1H-pyrazol-5-yl)pyridin-2-yl)-4H-1,2,4-triazole-3-carboxamide

The preparation was carried out in a similar manner to Example 1, except that in step 1, 1-methyl-5-(4,4,5,5-tetramethyl-1,3,2-dioxaborolan-2-yl)-3-(trifluoromethyl)-1H-pyrazole was used in place of phenylboronic acid. $^1$H NMR (400 MHz, DMSO-$d_6$) $\delta$ 14.59 (s, 1H), 10.08 (s, 1H), 8.65 - 8.41 (m, 1H), 8.31 (d, *J* = 3.7 Hz, 1H), 7.63 - 7.41 (m, 1H), 7.41 - 7.19 (m, 5H), 7.15 (d, *J* = 3.8 Hz, 1H), 4.19 (s, 2H), 4.04 (s, 3H). MS: 428 [M+H]$^+$.

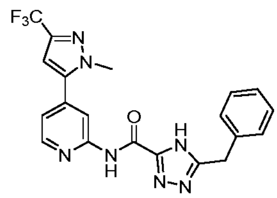

Example 28

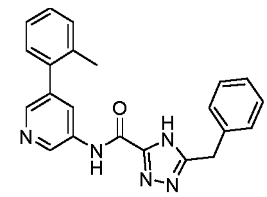

Example 29

Example 29: 5-benzyl-N-(5-(o-methylphenyl)pyridin-3-yl)-4H-1,2,4-triazole-3-carboxamide

The preparation was carried out in a similar manner to Example 1, except that in step 1, o-methylphenylboronic acid was used in place of phenylboronic acid, and 5-bromopyridin-3-amine was used in place of 4-bromopyridin-2-amine. $^1$H NMR (600 MHz, DMSO-$d_6$) $\delta$ 14.61 (s, 1H), 10.80 (s, 1H), 9.02 (d, *J* = 2.4 Hz, 1H), 8.31 (d, *J* = 2.0 Hz, 1H), 8.27 - 8.11 (m, 1H), 7.57 - 7.12 (m, 9H), 4.17 (s, 2H), 2.27 (s, 3H). MS: 370 [M+H]$^+$.

Example 30: 5-benzyl-N-(5-(2-chlorophenyl)pyridin-3-yl)-4H-1,2,4-triazole-3-carboxamide

The preparation was carried out in a similar manner to Example 1, except that in step 1, 2-chlorophenylboronic acid was used in place of phenylboronic acid, 5-bromopyridin-3-amine was used in place of 4-bromopyridin-2-amine. $^1$H NMR (600 MHz, DMSO-$d_6$) $\delta$ 14.59 (s, 1H), 10.84 (s, 1H), 9.04 (s, 1H), 8.53 - 8.23 (m, 2H), 7.66 - 7.58 (m, 1H), 7.54 - 7.45 (m, 3H), 7.37 - 7.31 (m, 2H), 7.31 - 7.28 (m, 2H), 7.28 - 7.22 (m, 1H), 4.18 (s, 2H). MS: 390 [M+H]$^+$.

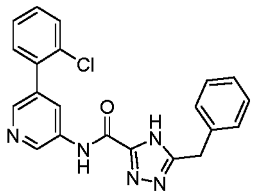

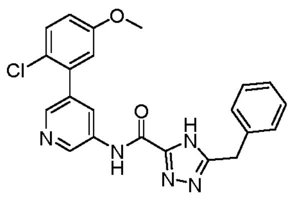

Example 30 Example 31

Example 31: 5-benzyl-N-(5-(2-chloro-5-methoxyphenyl)pyridin-3-yl)-4H-1,2,4-triazole-3-carboxamide

The preparation was carried out in a similar manner to Example 1, except that in step 1, 2-chloro-5-methoxyphenylboronic acid was used in place of phenylboronic acid, 5-bromopyridin-3-amine was used in place of 4-bromopyridin-2-amine. $^1$H NMR (600 MHz, DMSO-$d_6$) $\delta$ 14.59 (s, 1H), 10.82 (s, 1H), 9.04 (d, $J$ = 2.4 Hz, 1H), 8.49 - 8.23 (m, 2H), 7.59 - 7.44 (m, 1H), 7.37 - 7.31 (m, 2H), 7.31 - 7.27 (m, 2H), 7.27 - 7.22 (m, 1H), 7.07 -7.02 (m, 2H), 4.18 (s, 2H), 3.81 (s, 3H). MS: 420 $[M+H]^+$.

Example 32: 5-benzyl-N-(4-(1-isopropyl-3-(trifluoromethyl)-1H-pyrazol-5-yl)pyridin-2-yl)-4H-1,2,4-triazole-3-carboxamide

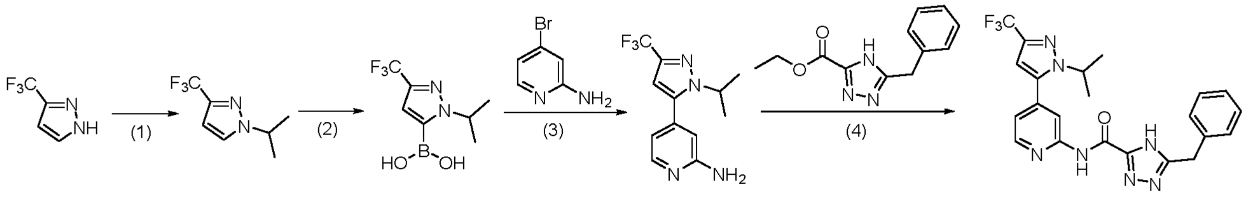


Step 1: Synthesis of 1-isopropyl-3-(trifluoromethyl)-1H-pyrazole

3-(Trifluoromethyl)-1H-pyrazole (1.09g, 8mmol) was dissolved in anhydrous tetrahydrofuran (24mL), and under argon protection at 0°C, sodium hydride was added in batches (60%, 385mg, 9.6mmol). After reacting for 40 minutes, 2-bromopropane (1.28g, 10.4mmol) was added dropwise, and the reaction was warmed to 25°C and reacted for 16 hours. The reaction solution was quenched with saturated aqueous solution of ammonium chloride, and extracted with ethyl acetate. The organic phase was washed with saturated brine, dried over anhydrous sodium sulfate, filtered and evaporated to dryness, and purified by column chromatography to afford 300 mg of product, MS: 179 $[M+H]^+$.

Step 2: Synthesis of (1-isopropyl-3-(trifluoromethyl)-1H-pyrazol-5-yl)boronic acid

1-Isopropyl-3-(trifluoromethyl)-1H-pyrazole (300mg, 1.7mmol) was dissolved in anhydrous tetrahydrofuran (6mL), and under argon protection at -78°C, n-butyllithium (2.5M, 1mL, 2.5mmol) was added dropwise. After reacting at this temperature for 1 hour, trimethyl borate (351mg, 3.4mmol) was added, and slowly warmed up overnight. The reaction solution was quenched with saturated aqueous solution of ammonium chloride, and extracted with ethyl acetate. The organic phase was washed with saturated brine, dried over anhydrous sodium sulfate, filtered and evaporated to dryness, and purified by column chromatography to afford 70 mg of product, MS: 223 $[M+H]^+$.

Step 3 to Step 4: 5-benzyl-N-(4-(1-isopropyl-3-(trifluoromethyl)-1H-pyrazol-5-yl)pyridin-2-yl)-4H-1,2,4-triazole-3-carboxamide

The preparation was carried out in a similar manner to Example 1, except that in step 1, (1-isopropyl-3-(trifluoromethyl)-1H-pyrazol-5-yl)boronic acid was used in place of phenylboronic acid. $^1$H NMR (400 MHz, DMSO-$d_6$) $\delta$ 14.61 (s, 1H), 10.08 (s, 1H), 8.54 (d, $J$ = 5.1 Hz, 1H), 8.26 (s, 1H), 7.41 - 7.29 (m, 5H), 7.29 - 7.21 (m, 1H), 7.04 (s, 1H), 4.86 - 4.60 (m, 1H), 4.18 (s, 2H), 1.45 (d, $J$ = 6.5 Hz, 6H). MS: 456 $[M+H]^+$.

Example 33: 5-benzyl-N-(4-(5-fluoro-2-methylphenyl)pyridin-2-yl)-4H-1,2,4-triazole-3-carboxamide

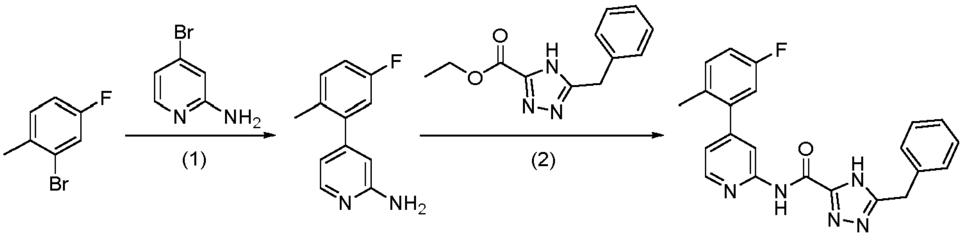


Step 1: Synthesis of 4-(5-fluoro-2-methylphenyl)pyridin-2-amine

4-Bromopyridin-2-amine (400mg, 2.3mmol), pinacol diborate (645mg, 2.5mmol), Pd(dppf)$Cl_2$ (170mg, 0.2mmol) and potassium acetate (564mg, 5.8mmol) were placed in dioxane (12mL), and reacted at 85°C for 16 hours under argon protection. The reaction solution was cooled to 25°C, and 2-bromo-4-fluoro-1-methylbenzene (321mg, 1.7mmol), Pd(dppf)$Cl_2$ (150mg, 0.2mmol) and an aqueous solution (1mL) of sodium carbonate (360mg, 3.4mmol) was added, and continued to react under argon protection at 100°C for 5 hours. The reaction solution was cooled and evaporated to dryness, and subjected to column chromatography to afford 240 mg of brown solid. MS: 203 $[M+H]^+$.

Step 2: Synthesis of 5-benzyl-N-(4-(5-fluoro-2-methylphenyl)pyridin-2-yl)-4H-1,2,4-triazole-3-carboxamide

4-(5-Fluoro-2-methylphenyl)pyridin-2-amine (0.7mmol) was placed in dry xylene (3mL), and trimethyl aluminum (3M, 0.7mL) was slowly added under argon protection. After reacting at 25°C for 1 hour, intermediate 1 (197mg, 0.9mmol) was added, and reacted at 100°C for 16 hours. The reaction solution was cooled and diluted with methanol, evaporated to dryness and subjected to column chromatography and preparative liquid chromatography to prepare the product. $^1$H NMR (400 MHz, DMSO-$d_6$) δ 14.62 (s, 1H), 9.96 (s, 1H), 8.44 (d, $J$ = 5.1 Hz, 1H), 8.12 (d, $J$ = 1.5 Hz, 1H), 7.54 -7.00 (m, 9H), 4.18 (s, 2H), 2.24 (s, 3H). MS: 388 $[M+H]^+$.

Example 34: 5-benzyl-N-(4-(5-chloro-2-methylphenyl)pyridin-2-yl)-4H-1,2,4-triazole-3-carboxamide

The preparation was carried out in a similar manner to Example 33, except that in step 1, 2-bromo-4-chloro-1-methylbenzene was used in place of 2-bromo-4-fluoro-1-methylbenzene. $^1$H NMR (400 MHz, DMSO-$d_6$) δ 14.50 (s, 1H), 9.96 (s, 1H), 8.44 (d, $J$ = 5.1 Hz, 1H), 8.11 (s, 1H), 7.57 -7.06 (m, 9H), 4.17 (s, 2H), 2.24 (s, 3H). MS: 404 $[M+H]^+$.

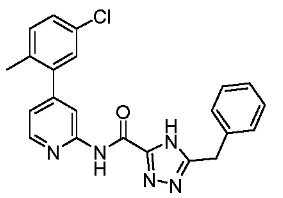
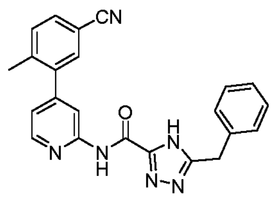

Example 34 Example 35

Example 35: 5-benzyl-N-(4-(5-cyano-2-methylphenyl)pyridin-2-yl)-4H-1,2,4-triazole-3-carboxamide

The preparation was carried out in a similar manner to Example 33, except that in step 1, 2-bromo-4-cyano-1-methylbenzene was used in place of 2-bromo-4-fluoro-1-methylbenzene. $^1$H NMR (400 MHz, DMSO-$d_6$) δ 14.56 (s, 1H), 9.93 (s, 1H), 8.46 (d, $J$ = 5.1 Hz, 1H), 8.14 (s, 1H), 7.74 (dd, $J$ = 8.1, 2.0 Hz, 1H), 7.68 - 7.53 (m, 2H), 7.38 - 7.30 (m, 4H), 7.30 - 7.23 (m, 2H), 4.20 (s, 2H), 2.35 (s, 3H). MS: 395 $[M+H]^+$.

Example 36: 5-benzyl-N-(4-(2-methyl-5-(trifluoromethyl)phenyl)pyridin-2-yl)-4H-1,2,4-triazole-3-carboxamide

The preparation was carried out in a similar manner to Example 33, except that in step 1, 2-bromo-4-trifluoromethyl-1-methylbenzene was used in place of 2-bromo-4-fluoro-1-methylbenzene. $^1$H NMR (400 MHz, DMSO-$d_6$) δ

14.56 (s, 1H), 9.93 (s, 1H), 8.46 (d, *J* = 5.1 Hz, 1H), 8.14 (s, 1H), 7.74 (dd, *J* = 8.1, 2.0 Hz, 1H), 7.65 - 7.57 (m, 2H), 7.39 - 7.23 (m, 6H), 4.20 (s, 2H), 2.35 (s, 3H). MS: 438 $[M+H]^+$.

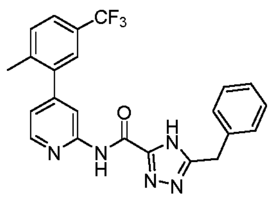

Example 36

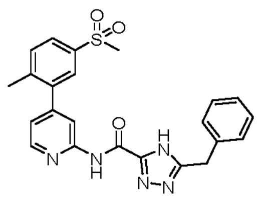

Example 37

Example 37: 5-benzyl-N-(4-(2-methyl-5-(methylsulfonyl)phenyl)pyridin-2-yl)-4H-1,2,4-triazole-3-carboxamide

The preparation was carried out in a similar manner to Example 33, except that in step 1, 2-bromo-4-methylsulfonyl-1-methylbenzene was used in place of 2-bromo-4-fluoro-1-methylbenzene. $^1H$ NMR (400 MHz, DMSO-$d_6$) δ 14.61 (s, 1H), 10.00 (s, 1H), 8.48 (d, *J* = 5.1 Hz, 1H), 8.15 (s, 1H), 7.91 (dd, *J* = 8.0, 2.1 Hz, 1H), 7.79 (d, *J* = 2.0 Hz, 1H), 7.66 (d, *J* = 8.1 Hz, 1H), 7.40 - 7.21 (m, 6H), 4.18 (s, 2H), 3.26 (s, 3H), 2.37 (s, 3H). MS: 448 $[M+H]^+$.

Example 38: 5-benzyl-N-(4-(2,3-dihydrobenzofuran-7-yl)pyridin-2-yl)-4H-1,2,4-triazole-3-carboxamide

The preparation was carried out in a similar manner to Example 33, except that in step 1, 7-bromo-2,3-dihydrobenzofuran was used in place of 2-bromo-4-fluoro-1-methylbenzene. $^1H$ NMR (600 MHz, DMSO-$d_6$) δ 14.55 (s, 1H), 9.78 (s, 1H), 8.53 (s, 1H), 8.39 (d, *J* = 5.3 Hz, 1H), 7.52 (d, *J* = 5.3 Hz, 1H), 7.41 (d, *J* = 7.8 Hz, 1H), 7.38 - 7.30 (m, 5H), 7.27 (d, *J* = 7.2 Hz, 1H), 7.03 - 6.98 (m, 1H), 4.63 (t, *J* = 8.7 Hz, 2H), 4.18 (s, 2H), 3.27 (t, *J* = 8.7 Hz, 2H). MS: 398 $[M+H]^+$.

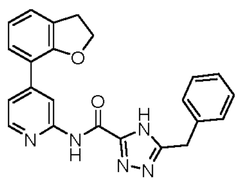

Example 38

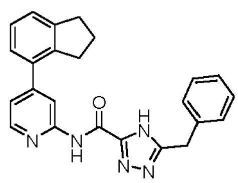

Example 39

Example 39: 5-benzyl-N-(4-(2,3-dihydro-1H-inden-4-yl)pyridin-2-yl)-4H-1,2,4-triazole-3-carboxamide

The preparation was carried out in a similar manner to Example 33, except that in step 1, 4-bromo-2,3-dihydro-1H-indene was used in place of 2-bromo-4-fluoro-1-methylbenzene. $^1H$ NMR (400 MHz, DMSO-$d_6$) δ 14.62 (s, 1H), 9.89 (s, 1H), 8.42 (d, *J* = 5.1 Hz, 1H), 8.27 (d, *J* = 1.5 Hz, 1H), 7.60 - 7.12 (m, 9H), 4.18 (s, 2H), 3.04 - 2.88 (m, 4H), 2.11 - 1.95 (m, 2H). MS: 396 $[M+H]^+$.

Example 40: 5-benzyl-N-(4-(chroman-5-yl)pyridin-2-yl)-4H-1,2,4-triazole-3-carboxamide

The preparation was carried out in a similar manner to Example 33, except that in step 1, 5-bromochroman was used in place of 2-bromo-4-fluoro-1-methylbenzene. $^1H$ NMR (400 MHz, DMSO-$d_6$) δ 14.60 (s, 1H), 9.90 (s, 1H), 8.41 (d, *J* = 5.1 Hz, 1H), 8.11 (s, 1H), 7.44 -7.30 (m, 4H), 7.30 -7.14 (m, 3H), 6.91 - 6.76 (m, 2H), 4.27 - 4.09 (m, 4H), 2.61 (t, *J* = 6.5 Hz, 2H), 1.95 - 1.78 (m, 2H). MS: 412 $[M+H]^+$.

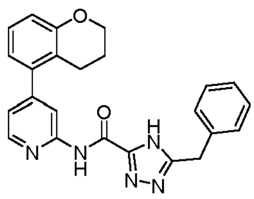 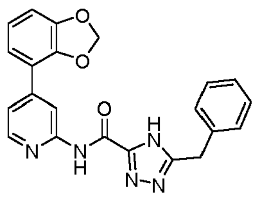

Example 40 Example 41

Example 41: N-(4-(benzo[d][1,3]dioxol-4-yl)pyridin-2-yl)-5-benzyl-4H-1,2,4-triazole-3-carboxamide

The preparation was carried out in a similar manner to Example 33, except that in step 1, 4-bromobenzo[d][1,3]dioxole was used in place of 2-bromo-4-fluoro-1-methylbenzene. $^1$H NMR (600 MHz, DMSO-$d_6$) δ 14.55 (s, 1H), 9.85 (s, 1H), 8.57 (s, 1H), 8.44 (d, *J* = 5.3 Hz, 1H), 7.55 (d, *J* = 5.3 Hz, 1H), 7.38 - 7.29 (m, 4H), 7.29 - 7.19 (m, 2H), 7.08 - 6.97 (m, 2H), 6.15 (s, 2H), 4.18 (s, 2H). MS: 400 $[M+H]^+$.

Example 42: 5-benzyl-N-(4-(2,3-dihydrobenzo[b][1,4]dioxin-5-yl)pyridin-2-yl)-4H-1,2,4-triazole-3-carboxamide

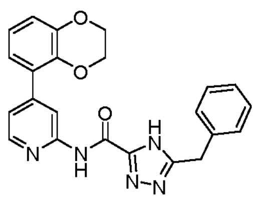

The preparation was carried out in a similar manner to Example 33, except that in step 1, 5-bromo-2,3-dihydrobenzo[b][1,4]dioxine was used in place of 2-bromo-4-fluoro-1-methylbenzene. $^1$H NMR (600 MHz, DMSO-$d_6$) δ 14.59 (s, 1H), 9.84 (s, 1H), 8.38 (d, *J* = 5.2 Hz, 1H), 8.29 (s, 1H), 7.39 - 7.29 (m, 5H), 7.29 - 7.21 (m, 1H), 7.02 - 6.92 (m, 3H), 4.34 - 4.25 (m, 4H), 4.17 (s, 2H). MS: 414 $[M+H]^+$.

Example 43: 5-benzyl-N-(4-(5,6,7,8-tetrahydronaphthalen-1-yl)pyridin-2-yl)-4H-1,2,4-triazole-3-carboxamide

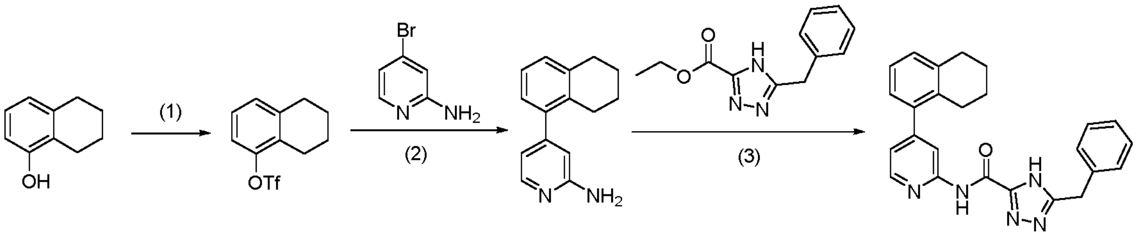


Step 1: Synthesis of 5,6,7,8-tetrahydronaphthalen-1-yl trifluoromethanesulfonate

5,6,7,8-tetrahydronaphthalen-1-ol was dissolved in dichloromethane (600mg, 4mmol) and pyridine (480mg, 6.1mmol), trifluoromethanesulfonic anhydride (1.37g, 4.8mmol) was added dropwise under argon protection at 0°C, and the reaction was warmed to 25°C and reacted for 5 hours. The reaction solution was diluted with dichloromethane, washed with 1M HCl, washed with saturated brine, dried over anhydrous sodium sulfate, filtered and evaporated to dryness to afford 444mg of colorless oil. MS: 281 $[M+H]^+$.

Step 2 to Step 3: The preparation was carried out in a similar manner to Example 33, except that in step 1, 5,6,7,8-tetrahydronaphthalen-1-yl trifluoromethanesulfonate was used in place of 2-bromo-4-fluoro-1-methylbenzene. $^1$H NMR (400 MHz, DMSO-$d_6$) δ 14.59 (s, 1H), 9.88 (s, 1H), 8.40 (d, *J* = 5.1 Hz, 1H), 8.07 (s, 1H), 7.40 - 7.29 (m, 4H), 7.29 - 7.12 (m, 4H), 7.03 (dd, *J* = 7.3, 1.6 Hz, 1H), 4.17 (s, 2H), 2.81 (t, *J* = 6.4 Hz, 2H), 2.56 (t, *J* = 6.2 Hz, 2H), 1.81 -

1.71 (m, 2H), 1.71 - 1.59 (m, 2H). MS: 410 [M+H]+.

Example 44: 5 -benzyl-N-(5 -cyclopropyl-2-methoxy- [3,4 '-bipyridin] -2' -yl)-4H-1,2,4-triazole-3 - carboxamide

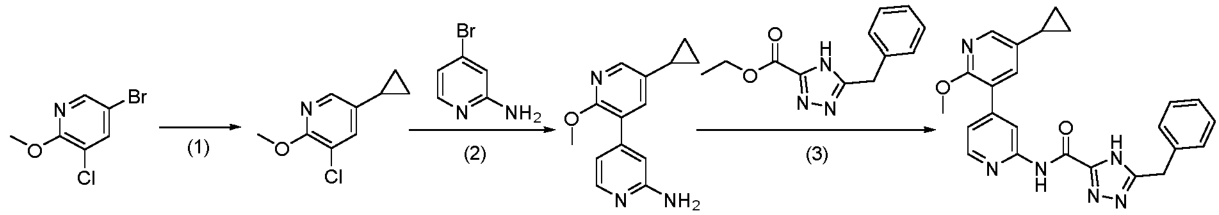


Step 1: Synthesis of 3-chloro-5-cyclopropyl-2-methoxypyridine

5-bromo-3-chloro-2-methoxypyridine (500mg, 2.2mmol), cyclopropylboronic acid (212mg, 2.5mmol), Pd(dppf)$Cl_2$ (145mg, 0.2mmol) and potassium carbonate (930mg, 6.7 mmol) were placed in a mixture of dioxane (8mL) and water (2mL), and reacted for 16 hours under argon protection at 80°C. The reaction solution was evaporated to dryness and subjected to column chromatography to afford 165 mg of product, MS: 184 [M+H]+.

Step 2 to Step 3: The preparation was carried out in a similar manner to Example 33, except that in step 1, 3-chloro-5-cyclopropyl-2-methoxypyridine was used in place of 2-bromo-4-fluoro-1-methylbenzene, and reacted at 120°C. $^1$H NMR (400 MHz, DMSO-$d_6$) $\delta$ 14.59 (s, 1H), 9.87 (s, 1H), 8.40 (d, $J$ = 5.2 Hz, 1H), 8.34 (s, 1H), 8.09 (d, $J$ = 2.4 Hz, 1H), 7.51 (d, $J$ = 2.4 Hz, 1H), 7.43 - 7.30 (m, 5H), 7.30 - 7.21 (m, 1H), 4.18 (s, 2H), 3.88 (s, 3H), 2.05 - 1.90 (m, 1H), 1.04 - 0.88 (m, 2H), 0.82 - 0.67 (m, 2H). MS: 427 [M+H]+.

Example 45: 5-benzyl-N-(4-(5-cyclopropyl-2-methylphenyl)pyridin-2-yl)-4H-1,2,4-triazole-3-carboxamide

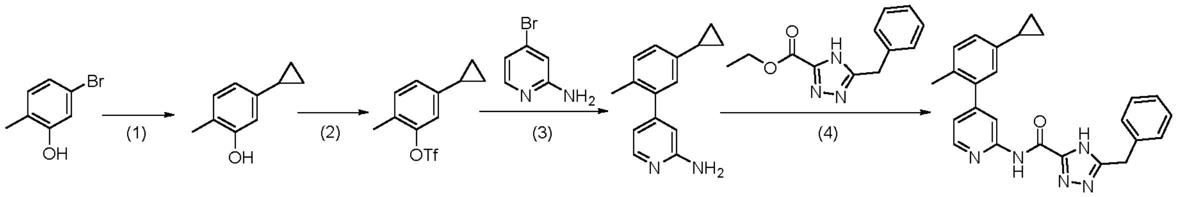


Step 1: Synthesis of 5-cyclopropyl-2-methylphenol

The operation is the same as step 1 of Example 44, but 5-bromo-2-methylphenol was used in place of 5-bromo-3-chloro-2-methoxypyridine. MS: 149 [M+H]+.

Step 2 to Step 4:

The preparation was carried out in a similar manner to Example 43, except that in step 1, but 5-cyclopropyl-2-methylphenol was used in place of 5,6,7,8-tetrahydronaphthalen-1-ol. $^1$H NMR (400 MHz, DMSO-$d_6$) $\delta$ 14.58 (s, 1H), 9.90 (s, 1H), 8.41 (d, $J$ = 5.1 Hz, 1H), 8.10 (s, 1H), 7.46 - 7.11 (m, 7H), 7.11 - 6.87 (m, 2H), 4.17 (s, 2H), 2.21 (s, 3H), 2.02 - 1.84 (m, 1H), 1.00 - 0.87 (m, 2H), 0.76 - 0.56 (m, 2H). MS: 410 [M+H]+.

Example 46: 5-benzyl-N-(4-(5-cyclopropyl-2-methoxyphenyl)pyridin-2-yl)-4H-1,2,4-triazole-3-carboxamide

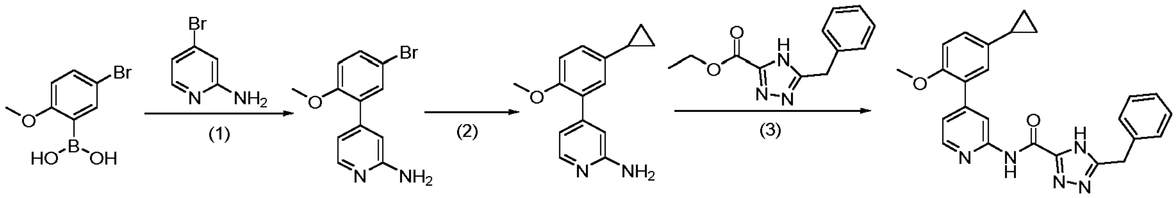


Step 1: Synthesis of 4-(5-bromo-2-methoxyphenyl)pyridin-2-amine

The operation is the same as step 1 of Example 1, but (5-bromo-2-methoxyphenyl)boronic acid was used in place of phenylboronic acid. MS: 279 $[M+H]^+$.

Step 2: Synthesis of 4-(5-cyclopropyl-2-methoxyphenyl)pyridin-2-amine

The operation is the same as step 1 of Example 44, but 4-(5-bromo-2-methoxyphenyl)pyridin-2-amine was used in place of 5-bromo-3-chloro-2-methoxypyridine. MS: 241 $[M+H]^+$.

Step 3: Synthesis of 5-benzyl-N-(4-(5-cyclopropyl-2-methoxyphenyl)pyridin-2-yl)-4H-1,2,4-triazole-3-carboxamide

The operation is the same as step 2 of Example 1, but 4-(5-cyclopropyl-2-methoxyphenyl)pyridin-2-amine was used in place of 4-phenylpyridin-2-amine. $^1$H NMR (400 MHz, DMSO-$d_6$) δ 14.54 (s, 1H), 9.79 (s, 1H), 8.36 (d, *J* = 5.2 Hz, 1H), 8.27 (s, 1H), 7.40-7.29 (m, 5H), 7.29-7.21 (m, 1H), 7.14 (dd, *J* = 8.5, 2.3 Hz, 1H), 7.11-7.03 (m, 2H), 4.18 (s, 2H), 3.76 (s, 3H), 2.00-1.88 (m, 1H), 0.97-0.86 (m, 2H), 0.70-0.58 (m, 2H). MS: 426 $[M+H]^+$.

Example 47: 5-benzyl-N-(4-cyclopentylpyridin-2-yl)-4H-1,2,4-triazole-3-carboxamide

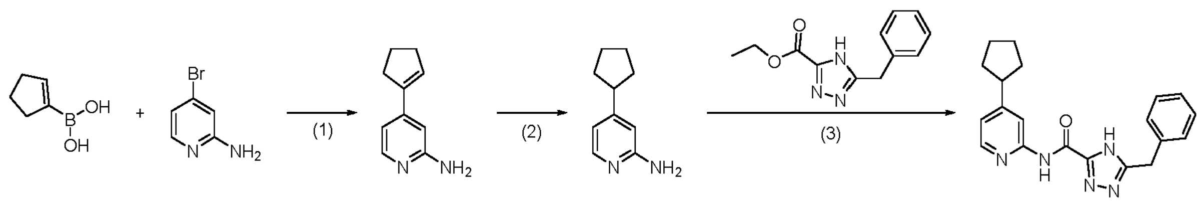


Step 1: Synthesis of 4-(cyclopent-1-en-1-yl)pyridin-2-amine

The operation is the same as step 1 of Example 1, but cyclopent-1-en-1-ylboronic acid was used in place of phenylboronic acid. MS: 161 $[M+H]^+$.

Step 2: Synthesis of 4-cyclopentylpyridin-2-amine

4-(Cyclopent-1-en-1-yl)pyridin-2-amine (160mg, 1mmol) was dissolved in a mixture of ethanol (2mL) and ethyl acetate (1mL), to which a drop of hydrochloric acid and palladium on carbon (10%, 10mg) was added, and reacted for 16 hours under hydrogen atmosphere. The reaction solution was filtered through diatomaceous earth. The filtrate was evaporated to dryness, diluted with dichloromethane, washed with saturated aqueous solution of sodium carbonate, dried over anhydrous sodium sulfate, filtered and evaporated to dryness to afford 140 mg of yellow oil. MS: 163 $[M+H]^+$.

Step 3: Synthesis of 5-benzyl-N-(4-cyclopentylpyridin-2-yl)-4H-1,2,4-triazole-3-carboxamide

The operation is the same as step 2 of Example 1, but 4-cyclopentylpyridin-2-amine was used in place of 4-phenylpyridin-2-amine. $^1$H NMR (400 MHz, DMSO-$d_6$) δ 14.61 (s, 1H), 9.70 (s, 1H), 8.23 (d, *J* = 5.1 Hz, 1H), 8.06 (d, *J* = 1.5 Hz, 1H), 7.42-7.29 (m, 4H), 7.29-7.18 (m, 1H), 7.09 (dd, *J* = 5.1, 1.5 Hz, 1H), 4.16 (s, 2H), 3.11-2.95 (m, 1H), 2.15-1.97 (m, 2H), 1.86-1.73 (m, 2H), 1.73-1.60 (m, 2H), 1.60-1.44 (m, 2H). MS: 348 $[M+H]^+$.

Example 48: 5-benzyl-N-(4-cyclohexylpyridin-2-yl)-4H-1,2,4-triazole-3-carboxamide

The preparation was carried out in a similar manner to Example 47, except that in step 1, cyclohex-1-en-1-ylboronic acid was used in place of cyclopent-1-en-1-ylboronic acid. $^1H$ NMR (400 MHz, DMSO-$d_6$) δ 14.58 (s, 1H), 9.68 (s, 1H), 8.23 (d, $J$ = 5.2 Hz, 1H), 8.04 (s, 1H), 7.39 - 7.28 (m, 4H), 7.28 - 7.19 (m, 1H), 7.12 -7.03 (m, 1H), 4.15 (s, 2H), 2.64 - 2.53 (m, 1H), 1.89 - 1.74 (m, 4H), 1.75 - 1.64 (m, 1H), 1.48 - 1.31 (m, 4H), 1.30 - 1.18 (m, 1H). MS: 362 $[M+H]^+$.

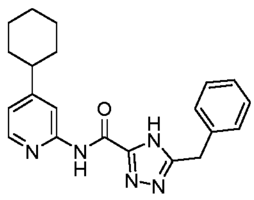

Example 48

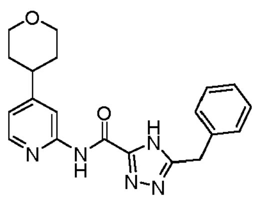

Example 49

Example 49: 5-benzyl-N-(4-(tetrahydro-2H-pyran-4-yl)pyridin-2-yl)-4H-1,2,4-triazole-3-carboxamide

The preparation was carried out in a similar manner to Example 47, except that in step 1, (3,6-dihydro-2H-pyran-4-yl)boronic acid was used in place of cyclopent-1-en-1-ylboronic acid. $^1H$ NMR (400 MHz, DMSO-$d_6$) δ 14.53 (s, 1H), 9.69 (s, 1H), 8.27 (d, $J$ = 5.2 Hz, 1H), 8.08 (s, 1H), 7.42-7.18 (m, 5H), 7.12 (d, $J$ = 5.3 Hz, 1H), 4.19 (s, 2H), 4.02-3.86 (m, 2H), 3.53-3.37 (m, 2H), 2.95-2.78 (m, 1H), 1.81-1.52 (m, 4H).MS: 364 $[M+H]^+$.

Example 50: 5-benzyl-N-(2-methoxy-5-methyl-[3,4'-bipyridin]-2'-yl)-4H-1,2,4-triazole-3-carboxamide

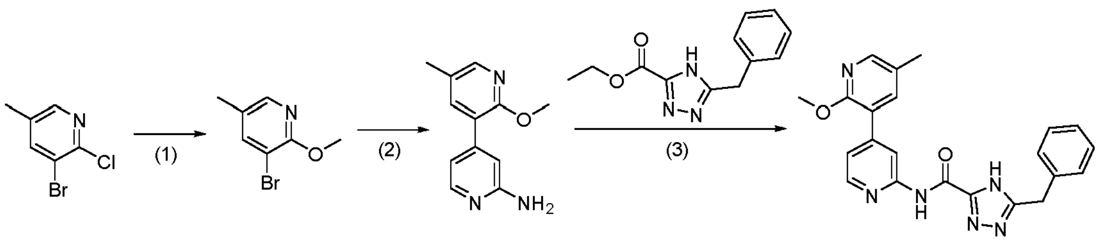


Step 1: Synthesis of 3-bromo-2-methoxy-5-methylpyridine

Anhydrous methanol (100mg, 3.1mmol) was dissolved in anhydrous DMF (3mL), and sodium hydride (60%, 115mg, 2.9mmol) was added under argon protection at 0°C. After reacting for 1 hour, 3-bromo-2-chloro-5-methylpyridine (400mg, 1.9mmol) in DMF (1mL) was added dropwise, and reacted at 60°C for 16 hours. MS: 202 $[M+H]^+$.

Step 2 to Step 3: The preparation was carried out in a similar manner to Example 33, except that in step 1, 3-bromo-2-methoxy-5-methylpyridine was used in place of 2-bromo-4-fluoro-1-methylbenzene. $^1H$ NMR (400 MHz, DMSO-$d_6$) δ 14.58 (s, 1H), 9.86 (s, 1H), 8.41 (d, $J$ = 5.2 Hz, 1H), 8.36 (d, $J$ = 1.5 Hz, 1H), 8.09 (d, $J$ = 2.2 Hz, 1H), 7.72 (d, $J$ = 2.3 Hz, 1H), 7.39 (dd, $J$ = 5.2, 1.6 Hz, 1H), 7.38 - 7.29 (m, 4H), 7.29 - 7.21 (m, 1H), 4.18 (s, 2H), 3.88 (s, 3H), 2.30 (s, 3H). MS: 401 $[M+H]^+$.

Example 51: 5-benzyl-N-(2-ethoxy-5-methyl-[3,4'-bipyridin]-2'-yl)-4H-1,2,4-triazole-3-carboxamide

The preparation was carried out in a similar manner to Example 50, except that in step 1, anhydrous ethanol was used in place of anhydrous methanol. $^1H$ NMR (400 MHz, DMSO-$d_6$) δ 14.55 (s, 1H), 9.80 (s, 1H), 8.47 (s, 1H), 8.40 (d, $J$ = 5.2 Hz, 1H), 8.07 (dd, $J$ = 2.3, 1.0 Hz, 1H), 7.73 (d, $J$ = 2.3 Hz, 1H), 7.41 (dd, $J$ = 5.2, 1.6 Hz, 1H), 7.38 - 7.29 (m, 4H), 7.29 - 7.21 (m, 1H), 4.37 (q, $J$ = 7.1 Hz, 2H), 4.18 (s, 2H), 2.29 (s, 3H), 1.32 (t, $J$ = 7.0 Hz, 3H). MS: 415 $[M+H]^+$.

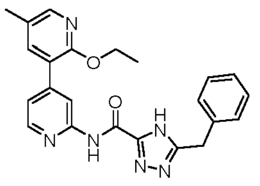

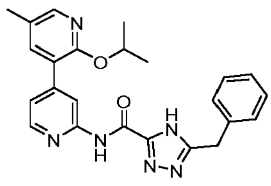

Example 51 Example 52

Example 52: 5-benzyl-N-(2-isopropoxy-5-methyl-[3,4'-bipyridin]-2'-yl)-4H-1,2,4-triazole-3-carboxamide

The preparation was carried out in a similar manner to Example 50, except that in step 1, anhydrous isopropanol was used in place of anhydrous methanol. $^{1}$H NMR (400 MHz, DMSO-$d_6$) δ 14.63 (s, 1H), 9.91 - 9.71 (m, 1H), 8.51 (s, 1H), 8.40 (d, *J* = 5.2 Hz, 1H), 8.06 (d, *J* = 2.3 Hz, 1H), 7.73 (d, *J* = 2.4 Hz, 1H), 7.44 - 7.18 (m, 6H), 5.34 (p, *J* = 6.2 Hz, 1H), 4.18 (s, 2H), 2.28 (s, 3H), 1.31 (d, *J* = 6.2 Hz, 6H). MS: 429 $[M+H]^+$.

Example 53: 5-benzyl-N-(6-ethoxy-5-methyl-[2,4'-bipyridin]-2'-yl)-4H-1,2,4-triazole-3-carboxamide

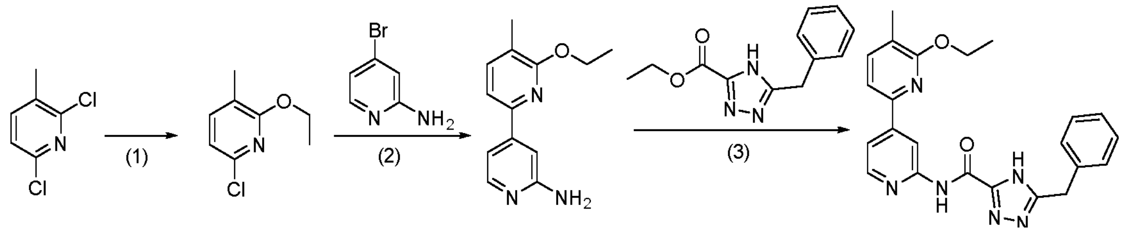


Step 1: Synthesis of 6-chloro-2-ethoxy-3-methylpyridine

Anhydrous ethanol (830mg, 18mmol) was dissolved in anhydrous tetrahydrofuran (10mL), and sodium hydride (60%, 600mg, 15mmol) was added at 0°C. After reacting for 30 minutes, 2,6-dichloro-3-methylpyridine (810mg, 5mmol) in tetrahydrofuran (5mL) was added, and reacted at 40°C for 24 hours. The reaction solution was quenched with saturated ammonium chloride, extracted with ethyl acetate, washed with saturated brine, dried over anhydrous sodium sulfate, filtered and evaporated to dryness, and purified by column chromatography to afford 300 mg of product. MS: 172 $[M+H]^+$.

Step 2 to Step 3: The preparation was carried out in a similar manner to Example 33, except that in step 1, 6-chloro-2-ethoxy-3-methylpyridine was used in place of 2-bromo-4-fluoro-1-methylbenzene, and reacted at 120°C. $^{1}$H NMR (400 MHz, DMSO-$d_6$) δ 14.21 (s, 1H), 9.86 (s, 1H), 8.86 (d, *J* = 3.6 Hz, 1H), 8.53-8.32 (m, 1H), 7.84-7.77 (m, 1H), 7.71 (dd, *J* = 7.8, 3.6 Hz, 1H), 7.59 (dd, *J* = 7.8, 3.8 Hz, 1H), 7.40-7.30 (m, 4H), 7.27 (d, *J* = 7.0 Hz, 1H), 4.56-4.43 (m, 2H), 4.18 (s, 2H), 2.21 (s, 3H), 1.52-1.36 (m, 3H). MS: 415 $[M+H]^+$.

Example 54: 5-benzyl-N-(6-(2-methoxyethoxy)-5-methyl-[2,4'-bipyridin]-2'-yl)-4H-1,2,4-triazole-3-carboxamide

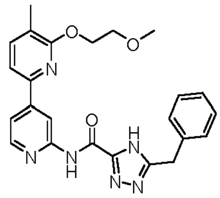

The preparation was carried out in a similar manner to Example 53, except that in step 1, 2-methoxyethanol was used in place of anhydrous ethanol. $^{1}$H NMR (400 MHz, DMSO-$d_6$) δ 14.54 (s, 1H), 9.87 (s, 1H), 8.85 (d, *J* = 1.3 Hz, 1H), 8.44 (d, *J* = 5.3 Hz, 1H), 7.81 (dd, *J* = 5.3, 1.6 Hz, 1H), 7.76 - 7.69 (m, 1H), 7.61 (d, *J* = 7.4 Hz, 1H), 7.40 - 7.29 (m, 4H), 7.29 - 7.20 (m, 1H), 4.64 - 4.51 (m, 2H), 4.18 (s, 2H), 3.82 - 3.73 (m, 2H), 3.45 (s, 3H), 2.22 (s, 3H). MS: 445 $[M+H]^+$.

Example 55: 5-benzyl-N-(4-(5-((2-methoxyethyl)(methyl)amino)-2-methylphenyl)pyridin-2-yl)-4H-1,2,4-triazole-3-carboxamide

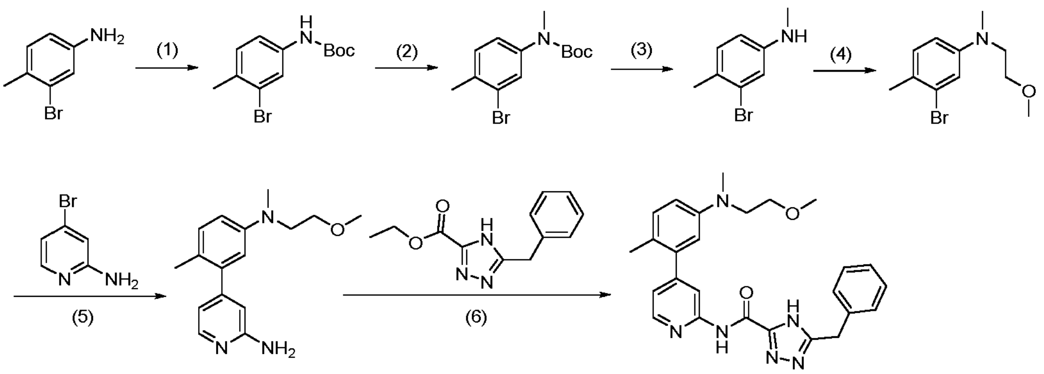


Step 1: Synthesis of tert-butyl (3-bromo-4-methylphenyl)carbamate

3-Bromo-4-methylaniline (1.86g, 10mmol) was placed in dichloromethane (25mL), 4-dimethylaminopyridine (120mg, 1mmol) and triethylamine (2g, 20mmol) were added, a solution of di-tert-butyl dicarbonate (2.4g, 11mmol) in dichloromethane (5mL) was added dropwise under argon protection at 0°C, and reacted at 25°C for 4 hours after the addition. The reaction solution was diluted with dichloromethane, washed with saturated aqueous solution of ammonium chloride, washed with brine, dried over anhydrous sodium sulfate, evaporated to dryness and filtered to afford 2.84g crude product.

Step 2: Synthesis of tert-butyl (3-bromo-4-methylphenyl)(methyl)carbamate

Tert-butyl (3-bromo-4-methylphenyl)carbamate (2.84 g, 9.9mmol) was dissolved in DMF, and sodium hydride (60%, 475mg, 11.9mmol) was added in batches at 0°C. After reacting at 0°C for 30minutes, iodomethane (2.1g, 14.8mmol) was added dropwise, and reacted at 25°C for 3 hours. The reaction solution was quenched with saturated aqueous solution of ammonium chloride, diluted with ethyl acetate, washed with saturated aqueous solution of ammonium chloride and brine, dried over anhydrous sodium sulfate, filtered and evaporated to dryness, purified by column chromatography to afford 2.5 g.

Step 3: Synthesis of 3-bromo-N,4-dimethylaniline

Tert-butyl (3-bromo-4-methylphenyl)(methyl)carbamate (2.5g, 8.3mmol) was dissolved in dichloromethane (25mL), trifluoroacetic acid (10mL) was added dropwise at 0°C, and reacted at 25°C for 3 hours. The reaction solution was concentrated, an aqueous solution of sodium carbonate was added dropwise to adjust the pH to 9, extracted with dichloromethane, dried over anhydrous sodium sulfate, filtered and evaporated to dryness to afford 1.5g of crude product. MS: 200 $[M+H]^+$.

Step 4: Synthesis of 3-bromo-N-(2-methoxyethyl)-N,4-dimethylaniline

3-Bromo-N,4-dimethylaniline (200mg, 1mmol) was dissolved in DMF, potassium carbonate (275mg, 1.5mmol) and 1-bromo-2-methoxyethane (180mg, 1.3mmol) were added, and reacted for 24 hours at 70°C in sealed tube. The reaction solution was cooled, diluted with ethyl acetate, washed with saturated ammonium chloride and brine, dried over anhydrous sodium sulfate, filtered and evaporated to dryness to afford 180 mg of the product. MS: 258 $[M+H]^+$.

Step 5 to Step 6: The preparation was carried out in a similar manner to Example 33, except that in step 1, 3-bromo-N-(2-methoxyethyl)-N,4-dimethylaniline was used in place of 2-bromo-4-fluoro-1-methylbenzene. $^1H$ NMR (400 MHz, DMSO-$d_6$) δ 14.58 (s, 1H), 9.86 (s, 1H), 8.40 (d, *J* = 5.1 Hz, 1H), 8.11 (s, 1H), 7.38 - 7.29 (m, 4H), 7.27 (dd, *J* = 6.1, 2.5 Hz, 1H), 7.19 (dd, *J* = 5.1, 1.5 Hz, 1H), 7.12 (d, *J* = 8.5 Hz, 1H), 6.72 (dd, *J* = 8.5, 2.8 Hz, 1H), 6.55 (d, *J* = 2.8 Hz, 1H), 4.17 (s, 2H), 3.54 - 3.43 (m, 4H), 3.24 (s, 3H), 2.90 (s, 3H), 2.13 (s, 3H). MS: 457 $[M+H]^+$.

Example 56: 5-benzyl-N-(4-(5-((2-ethoxyethyl)(methyl)amino)-2-methylphenyl)pyridin-2-yl)-4H-1,2,4-triazole-3-carboxamide

The operation is the same as Example 55, but 1-bromo-2-ethoxyethane was used in place of 1-bromo-2-methoxyethane in step 4. $^1$H NMR (400 MHz, DMSO-$d_6$) δ 14.56 (s, 1H), 9.85 (s, 1H), 8.40 (d, $J$ = 5.1 Hz, 1H), 8.11 (s, 1H), 7.46 - 7.29 (m, 4H), 7.27 (d, $J$ = 6.5 Hz, 1H), 7.19 (dd, $J$ = 5.2, 1.5 Hz, 1H), 7.12 (d, $J$ = 8.5 Hz, 1H), 6.72 (dd, $J$ = 8.5, 2.8 Hz, 1H), 6.55 (d, $J$ = 2.8 Hz, 1H), 4.18 (s, 2H), 3.56 - 3.45 (m, 4H), 3.41 (q, $J$ = 7.0 Hz, 2H), 2.90 (s, 3H), 2.13 (s, 3H), 1.07 (t, $J$ = 7.0 Hz, 3H). MS: 471 [M+H]$^+$.

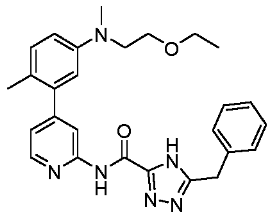
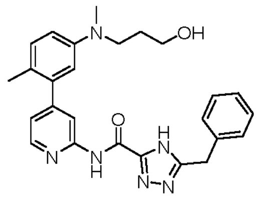

Example 56 Example 57

Example 57: 5-benzyl-N-(4-(5-((3-hydroxypropyl)(methyl)amino)-2-methylphenyl)pyridin-2-yl)-4H-1,2,4-triazole-3-carboxamide

The operation is the same as Example 55, but 3-bromo-1-propanol was used in place of 1-bromo-2-methoxyethane in step 4. $^1$H NMR (400 MHz, DMSO-$d_6$) δ 14.58 (s, 1H), 9.88 (s, 1H), 8.40 (d, $J$ = 5.1 Hz, 1H), 8.11 (s, 1H), 7.42 - 7.29 (m, 4H), 7.29 - 7.22 (m, 1H), 7.19 (dd, $J$ = 5.1, 1.5 Hz, 1H), 7.12 (d, $J$ = 8.5 Hz, 1H), 6.72 (dd, $J$ = 8.5, 2.7 Hz, 1H), 6.54 (d, $J$ = 2.7 Hz, 1H), 4.49 (t, $J$ = 5.0 Hz, 1H), 4.17 (s, 2H), 3.51 - 3.36 (m, 4H), 2.86 (s, 3H), 2.13 (s, 3H), 1.73 - 1.55 (m, 2H). MS: 457 [M+H]$^+$.

Example 58: 5-benzyl-N-(4-(5-((3-methoxypropyl)(methyl)amino)-2-methylphenyl)pyridin-2-yl)-4H-1,2,4-triazole-3-carboxamide

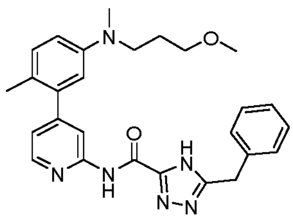

The operation is the same as Example 55, but 1-bromo-3-methoxypropane was used in place of 1-bromo-2-methoxyethane in step 4. $^1$H NMR (400 MHz, DMSO-$d_6$) δ 14.56 (s, 1H), 9.88 (s, 1H), 8.40 (d, $J$ = 5.1 Hz, 1H), 8.11 (s, 1H), 7.43 - 7.29 (m, 4H), 7.29 - 7.22 (m, 1H), 7.19 (dd, $J$ = 5.1, 1.6 Hz, 1H), 7.13 (d, $J$ = 8.5 Hz, 1H), 6.70 (dd, $J$ = 8.5, 2.8 Hz, 1H), 6.54 (d, $J$ = 2.8 Hz, 1H), 4.17 (s, 2H), 3.40 - 3.34 (m, 4H), 3.19 (s, 3H), 2.86 (s, 3H), 2.13 (s, 3H), 1.77 - 1.63 (m, 2H). MS: 471 [M+H]$^+$.

Example 59: 5-benzyl-N-(4-(5-(3-methoxypropylthio)-2-methylphenyl)pyridin-2-yl)-4H-1,2,4-triazole-3-carboxamide

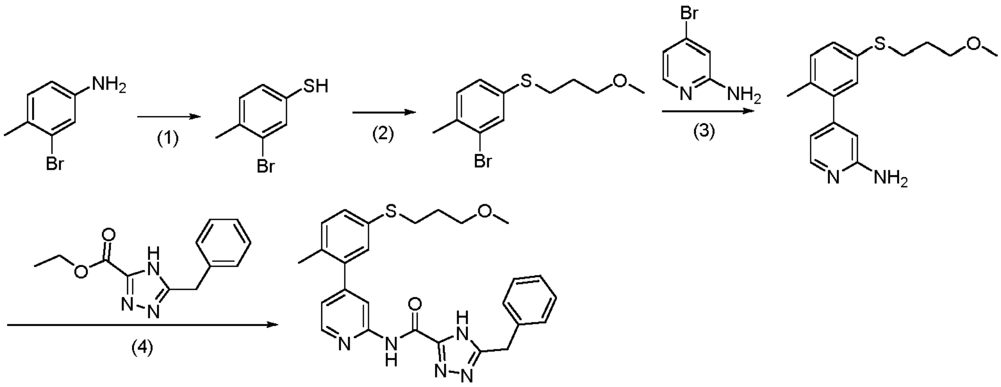


Step 1: Synthesis of 3-bromo-4-methylbenzenethiol

3-bromo-4-methylaniline (1.12g, 6mmol) was dissolved in 6M hydrochloric acid solution, and at - 10°C, an aqueous solution (6mL) of sodium nitrite (1.04g, 15mmol) was slowly added dropwise. After reacting for 30 minutes, this mixture was added dropwise to an aqueous solution (15 mL) of potassium ethylxanthate (3.36g, 21mmol), reacted at 80°C for 20 minutes, and then cooled. The reaction solution was diluted with water, extracted with ether, the organic phase was dried over anhydrous sodium sulfate, filtered and evaporated to dryness. The crude product was dissolved in ethanol (30mL), and potassium hydroxide (3.36g, 60mmol) in water (10mL) was added dropwise at 0°C, and reacted at 50 °C for 3 hours. The reaction solution was concentrated, diluted with water, adjusted to pH 4 with 6M hydrochloric acid, and extracted with ether. The organic phase was dried over anhydrous sodium sulfate, filtered and evaporated to dryness to afford 1g of crude product.MS: 203 $[M+H]^+$.

Step 2 to Step 4: Synthesis of 5-benzyl-N-(4-(5-((3-methoxypropyl)thio)-2-methylphenyl)pyridin-2-yl)-4H-1,2,4-triazole-3-carboxamide

The operation is the same as steps 4 to 6 of Example 55, but 3-bromo-4-methylbenzenethiol was used in place of 3-bromo-N,4-dimethylaniline in the fourth step, and 1-bromo-3-methoxypropane was used in place of 1-bromo-2-methoxyethane in the fourth step. $^1$H NMR (400 MHz, DMSO-$d_6$) $\delta$ 14.59 (s, 1H), 9.92 (s, 1H), 8.43 (d, $J$ = 5.1 Hz, 1H), 8.10 (s, 1H), 7.41 - 7.29 (m, 6H), 7.29 - 7.13 (m, 3H), 4.18 (s, 2H), 3.40 (t, $J$ = 6.1 Hz, 2H), 3.19 (s, 3H), 3.00 (t, $J$ = 7.2 Hz, 2H), 2.23 (s, 3H), 1.84 - 1.72 (m, 2H). MS: 474 $[M+H]^+$.

Example 60: 5-benzyl-N-(3-methoxy-6-methyl-[2,4'-bipyridin]-2'-yl)-4H-1,2,4-triazole-3-carboxamide

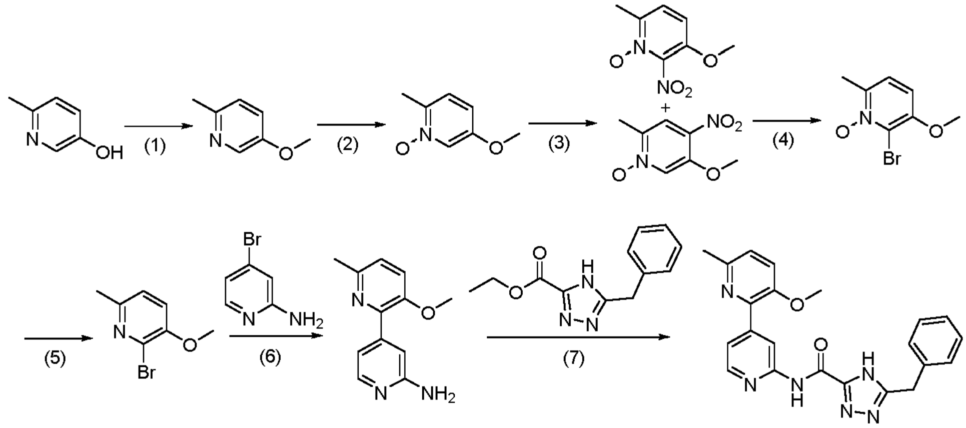


Step 1: Synthesis of 5-methoxy-2-methylpyridine

The operation is the same as step 4 of Example 55, but iodomethane was used in place of 1-bromo-2-methoxyethane, and reacted at 50°C in sealed tube. MS: 124 $[M+H]^+$.

Step 2 to Step 5: Synthesis of 2-bromo-3-methoxy-6-methylpyridine

Synthesis was made with reference to the method in Gonzalez, Javier et al/ PCT Int. Appl., 2006018725, 23 Feb 2006. In the third step, the separated 3-methoxy-6-methyl-2-nitropyridine N-oxide was used for the subsequent reaction.

Step 6 and 7: The preparation was carried out in a similar manner to Example 33, except that in step 1, 2-bromo-3-methoxy-6-methylpyridine was used in place of 2-bromo-4-fluoro-1-methylbenzene. $^1$H NMR (400 MHz, DMSO-$d_6$) δ 14.62 (s, 1H), 9.82 (s, 1H), 8.71 (s, 1H), 8.41 (d, *J* = 5.3 Hz, 1H), 7.68 (dd, *J* = 5.3, 1.5 Hz, 1H), 7.58 (d, *J* = 8.5 Hz, 1H), 7.40 - 7.29 (m, 5H), 7.29 - 7.22 (m, 1H), 4.18 (s, 2H), 3.87 (s, 3H), 3.33 (s, 3H). MS: 401 [M+H]$^+$.

Example 61: 5-benzyl-N-(5'-methoxy-2'-methyl-[4,4'-bipyridin]-2-yl)-4H-1,2,4-triazole-3-carboxamide

The operation is the same as step of Example 60, but 5-methoxy-2-methyl-4-nitropyridine N-oxide separated in the third step was used for the subsequent reaction. $^1$H NMR (400 MHz, DMSO-$d_6$) δ 14.61 (s, 1H), 9.93 (s, 1H), 8.46 (d, *J* = 5.2 Hz, 1H), 8.41 (s, 1H), 8.36 (s, 1H), 7.43 - 7.33 (m, 5H), 7.33 - 7.26 (m, 2H), 4.21 (s, 2H), 3.92 (s, 3H), 2.50 (s, 3H). MS: 401 [M+H]$^+$.

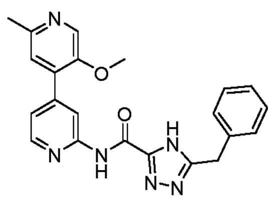

Example 61

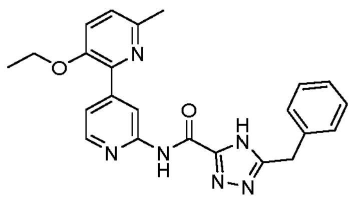

Example 62

Example 62: 5-benzyl-N-(3-ethoxy-6-methyl-[2,4'-bipyridin]-2'-yl)-4H-1,2,4-triazole-3-carboxamide

The operation is the same as Example 60, but bromoethane was used in place of iodomethane in the first step, and in the third step, the nitration process only gets 3-ethoxy-6-methyl-2-nitropyridine N-oxide. $^1$H NMR (400 MHz, DMSO-$d_6$) δ 14.70 (s, 1H), 9.80 (s, 1H), 8.84 (s, 1H), 8.41 (d, *J* = 5.3 Hz, 1H), 7.74 (dd, *J* = 5.3, 1.5 Hz, 1H), 7.55 (d, *J* = 8.5 Hz, 1H), 7.39-7.29 (m, 5H), 7.29-7.22 (m, 1H), 4.25-4.08 (m, 4H), 2.48 (s, 3H), 1.39 (t, *J* = 6.9 Hz, 3H). MS: 415 [M+H]$^+$.

Example 63: 5-benzyl-N-(2'-ethyl-5'-methoxy-[4,4'-bipyridin]-2-yl)-4H-1,2,4-triazole-3-carboxamide

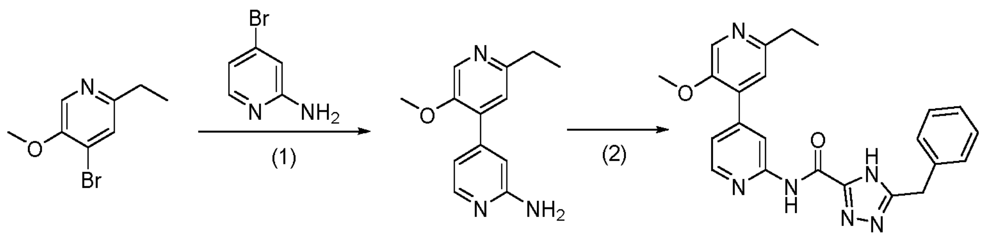


The preparation was carried out in a similar manner to Example 33, except that in step 1, 4-bromo-2-ethyl-5-methoxypyridine (see Gonzalez, Javier et al/PCT Int. Appl., 2006018725, 23 Feb 2006 for its synthetic method) was used in place of 2-bromo-4-fluoro-1-methylbenzene. $^1$H NMR (400 MHz, DMSO-$d_6$) δ 14.66 (s, 1H), 9.93 (s, 1H), 8.50 - 8.38 (m, 2H), 8.33 (d, *J* = 1.4 Hz, 1H), 7.43 -7.30 (m, 5H), 7.30 - 7.21 (m, 2H), 4.18 (s, 2H), 3.90 (s, 3H), 2.76 (q, *J* = 7.6 Hz, 2H), 1.24 (t, *J* = 7.6 Hz, 3H). MS: 415 [M+H]$^+$.

Example 64: 5-benzyl-N-(4-(5-(2-(tert-butoxy)ethoxy)-2-methylphenyl)pyridin-2-yl)-4H-1,2,4-triazole-3-carboxamide

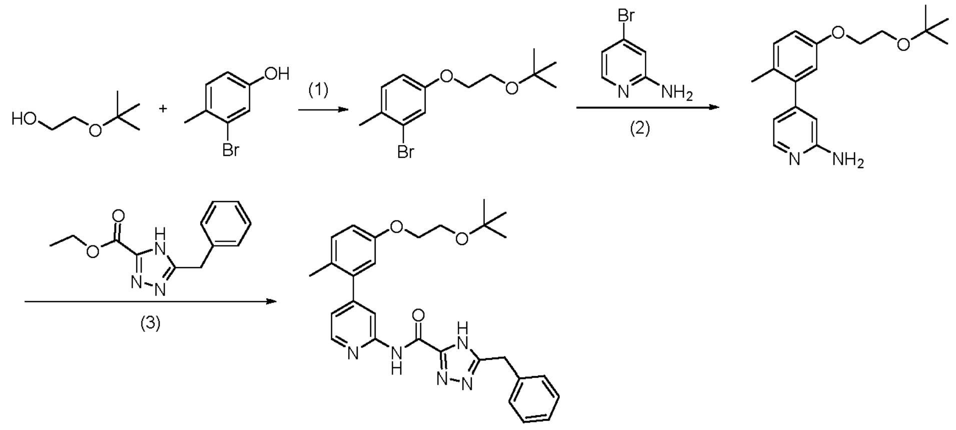


Step 1: Synthesis of 2-bromo-4-(2-(tert-butoxy)ethoxy)-1-methylbenzene

3-Bromo-4-methylphenol (187mg, 1mmol), 2-(tert-butoxy)ethan-1-ol (142mg, 1.2mmol) and triphenylphosphine (393mg, 1.5mmol) were placed in anhydrous tetrahydrofuran (3mL), diisopropyl azodicarboxylate (303mg, 1.5mmol) was added dropwise under argon protection at 0°C, and after the addition, reacted at 25°C for 16 hours. The reaction solution was evaporated to dryness and purified by column chromatography to afford 230 mg of colorless oil. MS: 287 $[M+H]^+$.

Step 2 to Step 3: The preparation was carried out in a similar manner to Example 33, except that in step 1, 2-bromo-4-(2-(tert-butoxy)ethoxy)-1-methylbenzene was used in place of 2-bromo-4-fluoro-1-methylbenzene. $^1$H NMR (400 MHz, DMSO-$d_6$) δ 14.59 (s, 1H), 9.90 (s, 1H), 8.48 - 8.37 (m, 1H), 8.12 (s, 1H), 7.41 - 7.29 (m, 4H), 7.29 - 7.18 (m, 3H), 6.94 (dd, *J* = 8.4, 2.7 Hz, 1H), 6.83 (d, *J* = 2.7 Hz, 1H), 4.18 (s, 2H), 4.12 - 3.99 (m, 2H), 3.69 - 3.58 (m, 2H), 2.19 (s, 3H), 1.15 (s, 9H). MS: 486 $[M+H]^+$.

Example 65: 5-benzyl-N-(4-(2-methyl-5-((tetrahydro-2H-pyran-4-yl)oxy)phenyl)pyridin-2-yl)-4H-1,2,4-triazole-3-carboxamide

The preparation was carried out in a similar manner to Example 64, except that in step 1, tetrahydro-2H-pyran-4-ol was used in place of 2-(tert-butoxy)ethan-1-ol. $^1$H NMR (400 MHz, DMSO-$d_6$) δ 14.58 (s, 1H), 9.89 (s, 1H), 8.42 (d, *J* = 5.1 Hz, 1H), 8.11 (s, 1H), 7.40 - 7.29 (m, 4H), 7.29 - 7.14 (m, 3H), 6.99 (dd, *J* = 8.5, 2.7 Hz, 1H), 6.87 (d, *J* = 2.6 Hz, 1H), 4.65 - 4.54 (m, 1H), 4.20 (s, 2H), 3.89 - 3.76 (m, 2H), 3.52 - 3.41 (m, 2H), 2.18 (s, 3H), 2.02 - 1.88 (m, 2H), 1.66 - 1.50 (m, 2H). MS: 470 $[M+H]^+$.

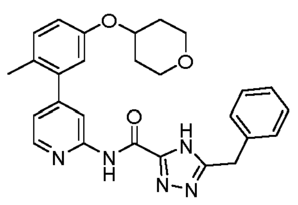
Example 65

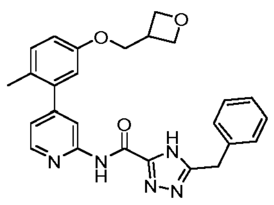
Example 66

Example 66: 5-benzyl-N-(4-(2-methyl-5-(oxetan-3-ylmethoxy)phenyl)pyridin-2-yl)-4H-1,2,4-triazole-3-carboxamide

The preparation was carried out in a similar manner to Example 64, except that in step 1, oxetan-3-yl methanol was used in place of 2-(tert-butoxy)ethan-1-ol. $^1$H NMR (400 MHz, DMSO-$d_6$) δ 14.58 (s, 1H), 9.89 (s, 1H), 8.42 (d, *J* = 5.1 Hz, 1H), 8.12 (s, 1H), 7.45 - 7.14 (m, 7H), 6.97 (dd, *J* = 8.4, 2.8 Hz, 1H), 6.86 (d, *J* = 2.7 Hz, 1H), 4.78 - 4.64 (m, 2H), 4.48 - 4.36 (m, 2H), 4.22 (d, *J* = 6.8 Hz, 2H), 4.18 (s, 2H), 3.46 - 3.34 (m, 1H), 2.20 (s, 3H). MS: 456 $[M+H]^+$.

Example 67: 5-benzyl-N-(4-(2-methyl-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol. $^1$H NMR (400 MHz, DMSO-$d_6$) δ 14.59 (s, 1H), 9.91 (s, 1H), 8.42 (d, *J* = 5.1 Hz, 1H), 8.11 (s, 1H), 7.42 - 7.29 (m, 4H), 7.29 - 7.12 (m, 3H), 6.94 (dd, *J* = 8.4, 2.7 Hz, 1H), 6.82 (d, *J* = 2.7 Hz, 1H), 4.18 (s, 2H), 3.92 - 3.79 (m, 4H), 3.33 - 3.27 (m, 2H), 2.19 (s, 3H), 2.05 - 1.90 (m, 1H), 1.72 - 1.62 (m, 2H), 1.38 - 1.23 (m, 2H). MS: 484 [M+H]$^+$.

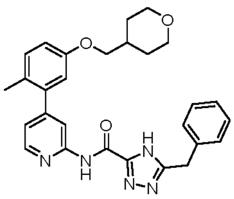

Example 67

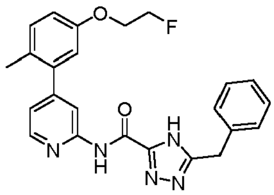

Example 68

Example 68: 5-benzyl-N-(4-(5-(2-fluoroethoxy)-2-methylphenyl)pyridin-2-yl)-4H-1,2,4-triazole-3-carboxamide

The preparation was carried out in a similar manner to Example 64, except that in step 1, 2-fluoro-1-ethanol was used in place of 2-(tert-butoxy)ethan-1-ol. $^1$H NMR (400 MHz, DMSO-$d_6$) δ 14.64 (s, 1H), 9.93 (s, 1H), 8.42 (d, *J* = 5.1 Hz, 1H), 8.12 (d, *J* = 1.5 Hz, 1H), 7.44 - 7.14 (m, 7H), 6.98 (dd, *J* = 8.4, 2.8 Hz, 1H), 6.87 (d, *J* = 2.7 Hz, 1H), 4.84 - 4.75 (m, 1H), 4.72 - 4.61 (m, 1H), 4.33 - 4.26 (m, 1H), 4.26 - 4.20 (m, 1H), 4.18 (s, 2H), 2.20 (s, 3H). MS: 432 [M+H]$^+$.

Example 69: 5-benzyl-N-(4-(5-(2-cyclopropylethoxy)-2-methylphenyl)pyridin-2-yl)-4H-1,2,4-triazole-3-carboxamide

The preparation was carried out in a similar manner to Example 64, except that in step 1, 2-cyclopropyl-1-ethanol was used in place of 2-(tert-butoxy)ethan-1-ol. $^1$H NMR (400 MHz, DMSO-$d_6$) δ 14.60 (s, 1H), 9.90 (s, 1H), 8.42 (d, *J* = 5.0 Hz, 1H), 8.12 (s, 1H), 7.39 -7.30 (m, 4H), 7.30 - 7.19 (m, 3H), 6.94 (dd, *J* = 8.4, 2.7 Hz, 1H), 6.82 (d, *J* = 2.7 Hz, 1H), 4.18 (s, 2H), 4.03 (t, *J* = 6.6 Hz, 2H), 2.19 (s, 3H), 1.69 - 1.55 (m, 2H), 0.89 - 0.75 (m, 1H), 0.50 - 0.37 (m, 2H), 0.20 - 0.06 (m, 2H). MS: 454 [M+H]$^+$.

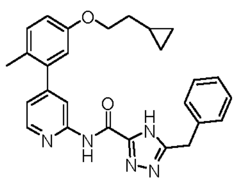

Example 69

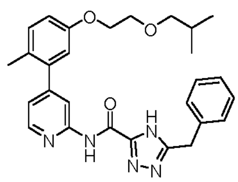

Example 70

Example 70: 5-benzyl-N-(4-(5-(2-isobutoxyethoxy)-2-methylphenyl)pyridin-2-yl)-4H-1,2,4-triazole-3-carboxamide

The preparation was carried out in a similar manner to Example 64, except that in step 1, 2-isobutoxy-1-ethanol was used in place of 2-(tert-butoxy)ethan-1-ol. $^1$H NMR (400 MHz, DMSO-$d_6$) δ 14.57 (s, 1H), 9.89 (s, 1H), 8.42 (d, *J* = 5.1 Hz, 1H), 8.12 (s, 1H), 7.45 - 7.15 (m, 7H), 6.95 (dd, *J* = 8.4, 2.8 Hz, 1H), 6.84 (d, *J* = 2.7 Hz, 1H), 4.18 (s, 2H), 4.15 - 4.06 (m, 2H), 3.74 - 3.61 (m, 2H), 3.21 (d, *J* = 6.6 Hz, 2H), 2.19 (s, 3H), 1.88 - 1.68 (m, 1H), 0.84 (d, *J* = 6.7, 2.5 Hz, 6H). MS: 486 [M+H]$^+$.

Example 71: 5-benzyl-N-(4-(2-methyl-5-(2-(2-oxopyrrolidin-1-yl)ethoxy)phenyl)pyridin-2-yl)-4H-1,2,4-triazole-3-carboxamide

The preparation was carried out in a similar manner to Example 64, except that in step 1, 1-(2-hydroxyethyl)pyrrolidin-2-one was used in place of 2-(tert-butoxy)ethan-1-ol. $^1$H NMR (400 MHz, DMSO-$d_6$) δ 14.54 (s, 1H), 9.91 (s, 1H), 8.42 (d, *J* = 5.1 Hz, 1H), 8.11 (s, 1H), 7.41 - 7.29 (m, 4H), 7.29 - 7.23 (m, 2H), 7.21 (dd, *J* = 5.0, 1.6 Hz, 1H), 6.95 (dd, *J* = 8.4, 2.7 Hz, 1H), 6.84 (d, *J* = 2.7 Hz, 1H), 4.17 (s, 2H), 4.09 (t, *J* = 5.5 Hz, 2H), 3.53 (t, *J* = 5.5 Hz, 2H), 3.45 (t, *J* = 7.0 Hz, 2H), 2.26 - 2.13 (m, 5H), 1.95 - 1.83 (m, 2H). MS: 497 [M+H]$^+$.

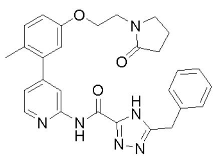
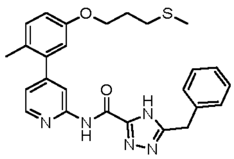

Example 71 Example 72

Example 72: 5-benzyl-N-(4-(2-methyl-5-(3-(methylthio)propoxy)phenyl)pyridin-2-yl)-4H-1,2,4-triazole-3-carboxamide

The preparation was carried out in a similar manner to Example 64, except that in step 1, 3-(methylthio)-1-propanol was used in place of 2-(tert-butoxy)ethan-1-ol. $^1H$ NMR (400 MHz, DMSO-$d_6$) δ 14.58 (s, 1H), 9.90 (s, 1H), 8.42 (d, *J* = 5.0 Hz, 1H), 8.11 (s, 1H), 7.42 - 7.29 (m, 4H), 7.29 - 7.23 (m, 2H), 7.21 (dd, *J* = 5.1, 1.6 Hz, 1H), 6.95 (dd, *J* = 8.4, 2.7 Hz, 1H), 6.83 (d, *J* = 2.7 Hz, 1H), 4.18 (s, 2H), 4.06 (t, *J* = 6.2 Hz, 2H), 2.61 (t, *J* = 7.2 Hz, 2H), 2.19 (s, 3H), 2.05 (s, 3H), 2.02 - 1.90 (m, 2H). MS: 474 $[M+H]^+$.

Example 73: 5-benzyl-N-(4-(2-methyl-5-(3-(methylsulfonyl)propoxy)phenyl)pyridin-2-yl)-4H-1,2,4-triazole-3-carboxamide

The preparation was carried out in a similar manner to Example 64, except that in step 1, 3-(methylsulfonyl)-1-propanol was used in place of 2-(tert-butoxy)ethan-1-ol. $^1H$ NMR (400 MHz, DMSO-$d_6$) δ 14.56 (s, 1H), 9.88 (s, 1H), 8.42 (d, *J* = 5.1 Hz, 1H), 8.12 (s, 1H), 7.41 - 7.15 (m, 7H), 6.96 (dd, *J* = 8.4, 2.7 Hz, 1H), 6.85 (d, *J* = 2.7 Hz, 1H), 4.18 (s, 2H), 4.11 (t, *J* = 6.2 Hz, 2H), 3.31 - 3.23 (m, 2H), 3.01 (s, 3H), 2.20 (s, 3H), 2.17 - 2.05 (m, 2H). MS: 506 $[M+H]^+$.

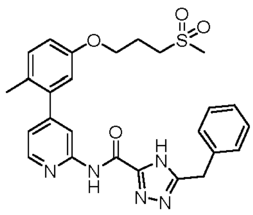
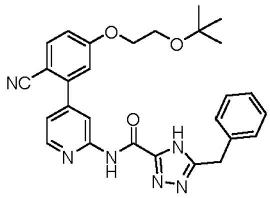

Example 73 Example 74

Example 74: 5-benzyl-N-(4-(5-(2-(tert-butoxy)ethoxy-2-cyanophenyl)pyridin-2-yl)-4H-1,2,4-triazole-3-carboxamide

The preparation was carried out in a similar manner to Example 64, except that in step 1, 2-chloro-4-hydroxybenzonitrile was used in place of 3-bromo-4-methylphenol. $^1H$ NMR (400 MHz, DMSO-$d_6$) δ 14.65 (s, 1H), 10.02 (s, 1H), 8.52 (d, *J* = 5.1 Hz, 1H), 8.34 (s, 1H), 7.94 (d, *J* = 9.2 Hz, 1H), 7.42 (d, *J* = 4.9 Hz, 1H), 7.39 - 7.30 (m, 4H), 7.30 - 7.18 (m, 3H), 4.23 (t, *J* = 4.6 Hz, 2H), 4.18 (s, 2H), 3.67 (t, *J* = 4.7 Hz, 2H), 1.15 (s, 9H). MS: 497 $[M+H]^+$.

Example 75: N-(4-(5-(2-(tert-butoxy)ethoxy)-2-methylphenyl)pyridin-2-yl)-5-(cyclopentylmethyl)-4H-1,2,4-triazole-3-carboxamide

The preparation was carried out in a similar manner to Example 64, except that in step 3, ethyl 5-(cyclopentylmethyl)-4H-1,2,4-triazole-3-carboxylate was used in place of ethyl 5-benzyl-4H-1,2,4-triazol-3-carboxylate. $^1H$ NMR (400 MHz, DMSO-$d_6$) δ 14.40 (s, 1H), 9.86 (s, 1H), 8.42 (d, *J* = 5.1 Hz, 1H), 8.14 (s, 1H), 7.32 - 7.13 (m, 2H), 6.95 (dd, *J* = 8.4, 2.8 Hz, 1H), 6.84 (d, *J* = 2.7 Hz, 1H), 4.09 - 4.00 (m, 2H), 3.67 - 3.57 (m, 2H), 2.78 (d, *J* = 7.5 Hz, 2H), 2.39 - 2.22 (m, 1H), 2.20 (s, 3H), 1.80 - 1.66 (m, 2H), 1.66 - 1.44 (m, 4H), 1.29 - 1.17 (m, 2H), 1.15 (s, 9H). MS: 478 $[M+H]^+$.

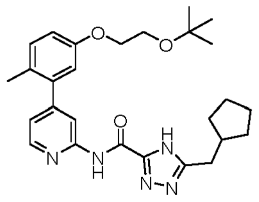

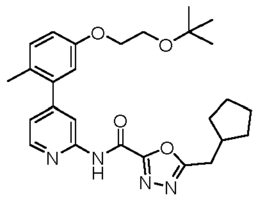

Example 75 Example 76

Example 76: N-(4-(5-(2-(tert-butoxy)ethoxy)-2-methylphenyl)pyridin-2-yl)-5-(cyclohexylmethyl)-1,3,4-oxadiazol-2-carboxamide

The preparation was carried out in a similar manner to Example 64, except that in step 3, ethyl 5-(cyclopentylmethyl)-1,3,4-oxadiazol-2-carboxylate was used in place of ethyl 5-benzyl-4H-1,2,4-triazol-3-carboxylate. $^1$H NMR (400 MHz, DMSO-$d_6$) $\delta$ 11.24 (s, 1H), 8.48 (d, $J$ = 5.0 Hz, 1H), 7.99 (s, 1H), 7.33 - 7.20 (m, 2H), 6.95 (dd, $J$ = 8.4, 2.7 Hz, 1H), 6.83 (d, $J$ = 2.7 Hz, 1H), 4.10 - 3.99 (m, 2H), 3.67 - 3.58 (m, 2H), 2.97 (d, $J$ = 7.4 Hz, 2H), 2.37 - 2.24 (m, 1H), 2.20 (s, 3H), 1.86 - 1.73 (m, 2H), 1.70 - 1.44 (m, 4H), 1.33 - 1.18 (m, 2H), 1.15 (s, 9H). MS: 479 [M+H]$^+$.

Example 77: N-(4-(5-(2-(tert-butoxy)ethoxy)-2-methylphenyl)pyridin-2-yl)-5-(cyclohexylmethyl)-4H-1,2,4-triazole-3-carboxamide

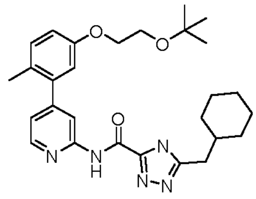

The preparation was carried out in a similar manner to Example 64, except that in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.38 (s, 1H), 9.93 -9.76 (m, 1H), 8.42 (d, $J$ = 5.1 Hz, 1H), 8.14 (s, 1H), 7.32 - 7.15 (m, 2H), 6.94 (dd, $J$ = 8.5, 2.7 Hz, 1H), 6.83 (d, $J$ = 2.7 Hz, 1H), 4.04 (t, $J$ = 4.8 Hz, 2H), 3.63 (t, $J$ = 4.9 Hz, 2H), 2.66 (d, $J$ = 7.0 Hz, 2H), 2.20 (s, 3H), 1.83 - 1.53 (m, 6H), 1.30 - 1.06 (m, 12H), 1.06 - 0.88 (m, 2H). MS: 492 [M+H]$^+$.

Example 78: 5-benzyl-N-(4-(5-(2-(tert-butoxy)ethoxy)-2-vinylphenyl)pyridin-2-yl)-4H-1,2,4-triazole-3-carboxamide

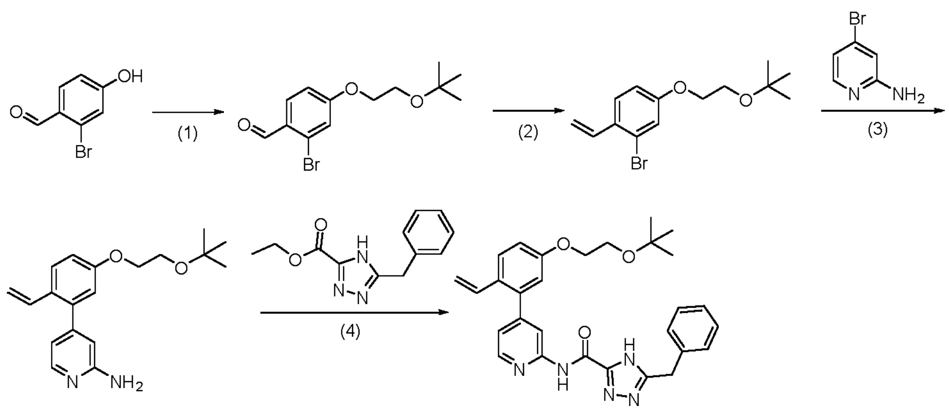

Step 1: Synthesis of 2-bromo-4-(2-(tert-butoxy)ethoxy)benzaldehyde

The operation is the same as step 1 of Example 64, but 2-bromo-4-hydroxybenzaldehyde was used in place of 2-bromo-3-methylphenol in step 1.

Step 2: Synthesis of 2-bromo-4-(2-(tert-butoxy)ethoxy)-1-vinylbenzene

Methyltriphenylphosphonium bromide (1.25g, 3.5mmol) was placed in anhydrous tetrahydrofuran (7mL), and n-butyl lithium was added dropwise at -20°C under argon protection. After reacting at 0°C for 1 hour, 2-bromo-4-(2-(tert-butoxy)ethoxy)benzaldehyde (700mg, 2.3mmol) in anhydrous tetrahydrofuran (2.5mL) solution was added, heated to 25°C and reacted for 5 hours. The reaction solution was quenched with saturated ammonium chloride, extracted with ethyl acetate, dried over anhydrous sodium sulfate, filtered and evaporated to dryness, and the crude product was directly used in the next step.

Step 3 to Step 4: The preparation was carried out in a similar manner to Example 33, except that in step 1, 2-bromo-4-(2-(tert-butoxy)ethoxy)-1-vinylbenzene was used in place of 2-bromo-4-fluoro-1-methylbenzene. $^1$H NMR (400 MHz, DMSO-$d_6$) δ 14.54 (s, 1H), 9.92 (s, 1H), 8.43 (d, $J$ = 5.1 Hz, 1H), 8.11 (s, 1H), 7.69 (d, $J$ = 8.7 Hz, 1H), 7.38 -7.30 (m, 4H), 7.29 - 7.22 (m, 1H), 7.17 (dd, $J$ = 5.0, 1.5 Hz, 1H), 7.06 (dd, $J$ = 8.7, 2.6 Hz, 1H), 6.88 (d, $J$ = 2.6 Hz, 1H), 6.55 (dd, $J$ = 17.4, 11.0 Hz, 1H), 5.71 (d, $J$ = 17.4 Hz, 1H), 5.15 (d, $J$ = 11.1 Hz, 1H), 4.17 (s, 2H), 4.10 (t, $J$ = 4.8 Hz, 2H), 3.64 (dd, $J$ = 5.6, 4.0 Hz, 2H), 1.15 (s, 9H).MS: 498 [M+H]$^+$.

Example 79: 5-(cyclopentylmethyl)-N-(4-(2-methyl-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.41 (s, 1H), 9.86 (s, 1H), 8.42 (d, $J$ = 5.1 Hz, 1H), 8.13 (s, 1H), 7.25 (d, $J$ = 8.5 Hz, 1H), 7.21 (dd, $J$ = 5.1, 1.6 Hz, 1H), 6.95 (dd, $J$ = 8.4, 2.7 Hz, 1H), 6.82 (d, $J$ = 2.7 Hz, 1H), 3.93 - 3.86 (m, 1H), 3.86 - 3.78 (m, 3H), 3.32 - 3.23 (m, 2H), 2.77 (d, $J$ = 7.4 Hz, 2H), 2.35 - 2.22 (m, 1H), 2.19 (s, 3H), 2.06 - 1.92 (m, 1H), 1.79 - 1.44 (m, 8H), 1.40 - 1.12 (m, 4H). MS: 476 [M+H]$^+$.

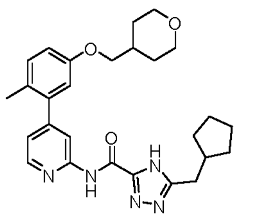

Example 79

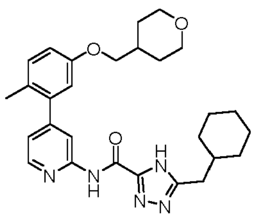

Example 80

Example 80: 5-(cyclohexylmethyl)-N-(4-(2-methyl-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, and in step 3, ethyl 5-was used in place of ethyl 5-benzyl-4H-1,2,4-triazol-3-carboxylate. $^1$H NMR (400 MHz, DMSO-$d_6$) δ 14.36 (s, 1H), 9.84 (s, 1H), 8.42 (d, $J$ = 5.1 Hz, 1H), 8.13 (s, 1H), 7.34 - 7.12 (m, 2H), 6.94 (dd, $J$ = 8.4, 2.7 Hz, 1H), 6.82 (d, $J$ = 2.7 Hz, 1H), 3.93 - 3.79 (m, 4H), 3.40 - 3.31 (m, 3H), 2.72 - 2.62 (m, 2H), 2.19 (s, 3H), 2.07 - 1.92 (m, 1H), 1.84 - 1.70 (m, 1H), 1.70 - 1.53 (m, 6H), 1.41 - 1.05 (m, 5H), 1.05 - 0.90 (m, 2H). MS: 490 [M+H]$^+$.

Example 81: 5-benzyl-N-(4-(5-(3-cyclobutoxypropoxy)-2-methylphenyl)pyridin-2-yl)-4H-1,2,4-triazole-3-carboxamide

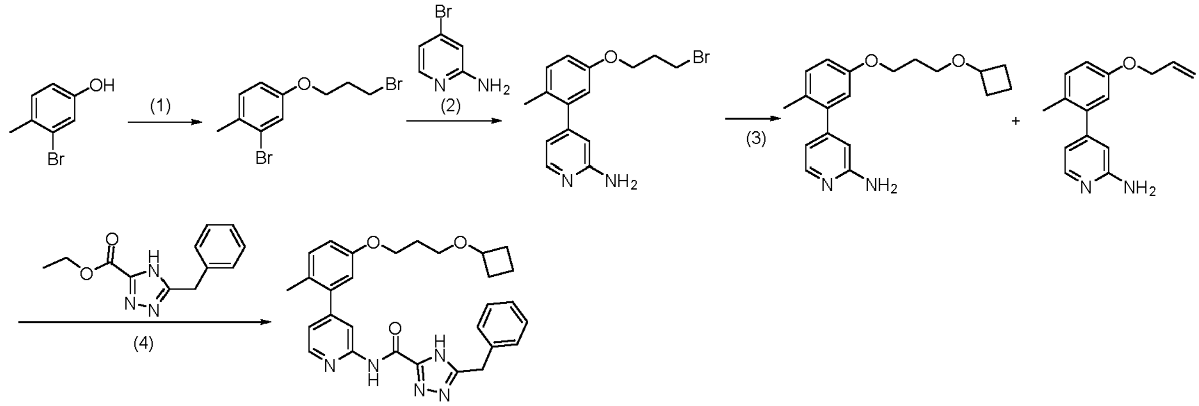


Step 1 to Step 2: Synthesis of 4-(5-(3-bromopropoxy)-2-methylphenyl)pyridin-2-amine

The operation is the same as steps 1 to 2 of Example 64, but 3-bromopropyl-1-ol was used in place of 2-(tert-butoxy)ethan-1-ol in step 1.

Step 3: Synthesis of 4-(5-(3-cyclobutoxypropoxy)-2-methylphenyl)pyridin-2-amine

Cyclobutanol (115mg, 1.6mmol) was dissolved in anhydrous tetrahydrofuran (3mL), and sodium hydride (60%, 65mg, 1.6mmol) was added under argon protection at 0°C. After 30 minutes of reaction, 4-(5-(3-bromopropoxy)-2-methylphenyl)pyridin-2-amine (200mg, 0.6mmol) in tetrahydrofuran was added dropwise, and reacted at 50°C for 4 hours. The reaction was cooled, quenched with saturated ammonium chloride, the reaction solution was evaporated, and purified by column chromatography to afford 40mg of product and 20mg of by-product 4-(5-allyloxy)-2-methylphenyl)pyridin-2-amine. MS: 313 $[M+H]^+$ and MS: 241 $[M+H]^+$

Step 4: Synthesis of 5-benzyl-N-(4-(5-(3-cyclobutoxypropoxy)-2-methylphenyl)pyridin-2-yl)-4H-1,2,4-triazole-3-carboxamide

The operation is the same as step 2 of Example 1, but 4-(5-(3-cyclobutoxypropoxy)-2-methylphenyl)pyridin-2-amine was used in place of 4-phenylpyridin-2-amine. $^1H$ NMR (400 MHz, DMSO-$d_6$) δ 14.58 (s, 1H), 9.88 (s, 1H), 8.42 (d, *J* = 5.1 Hz, 1H), 8.11 (s, 1H), 7.49 - 7.30 (m, 4H), 7.30 - 7.17 (m, 3H), 6.94 (dd, *J* = 8.4, 2.7 Hz, 1H), 6.82 (d, *J* = 2.7 Hz, 1H), 4.18 (s, 2H), 4.03 (t, *J* = 6.3 Hz, 2H), 3.93 - 3.81 (m, 1H), 3.42 - 3.39 (m, 2H), 2.19 (s, 3H), 2.16 - 2.06 (m, 2H), 1.96 - 1.85 (m, 2H), 1.85 - 1.71 (m, 2H), 1.66 - 1.53 (m, 1H), 1.50 - 1.36 (m, 1H). MS: 498 $[M+H]^+$.

Example 82: N-(4-(5-(allyloxy)-2-methylphenyl)pyridin-2-yl)-5-benzyl-4H-l,2,4-triazole-3-carboxamide

The operation is the same as Example 81, but the by-product 4-(5-allyloxy)-2-methylphenyl)pyridin-2-amine obtained in the third step was used for the subsequent reaction. $^1H$ NMR (400 MHz, DMSO-$d_6$) δ 14.38 (s, 1H), 9.92 (s, 1H), 8.42 (d, *J* = 5.1 Hz, 1H), 8.11 (d, *J* = 1.5 Hz, 1H), 7.39 - 7.29 (m, 4H), 7.29 - 7.23 (m, 2H), 7.21 (dd, *J* = 5.1, 1.6 Hz, 1H), 6.96 (dd, *J* = 8.4, 2.8 Hz, 1H), 6.85 (d, *J* = 2.8 Hz, 1H), 6.11 - 5.96 (m, 1H), 5.45 - 5.32 (m, 1H), 5.32 - 5.19 (m, 1H), 4.64 - 4.53 (m, 2H), 4.17 (s, 2H), 2.19 (s, 3H). MS: 426 $[M+H]^+$.

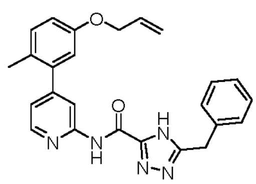

Example 82

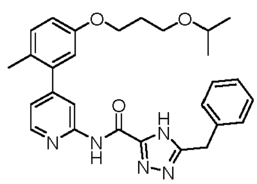

Example 83

Example 83: 5-benzyl-N-(4-(5-(3-isopropoxypropoxy)-2-methylphenyl)pyridin-2-yl)-4H-1,2,4-triazole-3-carboxamide

The operation is the same as Example 81, but isopropanol was used in place of cyclobutanol in the third step. $^1H$ NMR (400 MHz, DMSO-$d_6$) δ 14.57 (s, 1H), 9.89 (s, 1H), 8.41 (d, *J* = 5.1 Hz, 1H), 8.11 (s, 1H), 7.48 - 7.10 (m, 7H),

6.94 (dd, *J* = 8.4, 2.7 Hz, 1H), 6.82 (d, *J* = 2.7 Hz, 1H), 4.18 (s, 2H), 4.03 (t, *J* = 6.3 Hz, 2H), 3.58 - 3.44 (m, 3H), 2.19 (s, 3H), 1.95 - 1.80 (m, 2H), 1.06 (d, *J* = 6.1 Hz, 6H). MS: 486 $[M+H]^+$.

Example 84: 5-benzyl-N-(4-(5-(2-methyl-5-(3-(2-oxopiperidin-1-yl)propoxy)phenyl)pyridin-2-yl)-4H-1,2,4-triazole-3 -carboxamide

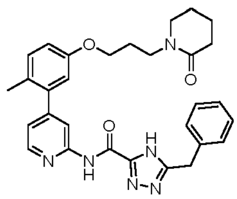

The operation is the same as Example 81, but piperidin-2-one was used in place of cyclobutanol in the third step. $^1$H NMR (400 MHz, DMSO-$d_6$) δ 14.56 (s, 1H), 9.91 (s, 1H), 8.41 (d, *J* = 5.1 Hz, 1H), 8.11 (s, 1H), 7.46 - 7.11 (m, 7H), 6.93 (dd, *J* = 8.4, 2.7 Hz, 1H), 6.81 (d, *J* = 2.7 Hz, 1H), 4.17 (s, 2H), 3.98 (t, *J* = 6.3 Hz, 2H), 3.28 - 3.20 (m, 4H), 2.26 - 2.13 (m, 5H), 1.91 (t, *J* = 6.8 Hz, 2H), 1.77 - 1.61 (m, 4H). MS: 525 $[M+H]^+$.

Example 85: 5-benzyl-N-(4-(5-(2-(2-methoxyethoxy)ethoxy)-2-methylphenyl)pyridin-2-yl)-4H-1,2,4-triazole-3-carboxamide

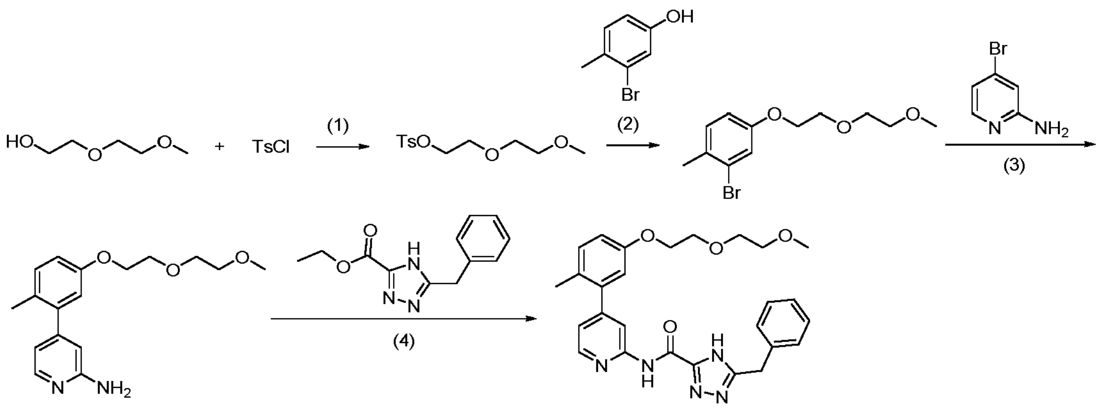


Step 1: Synthesis of 2-(2-methoxyethoxy)ethyl 4-methylbenzenesulfonate

2-(2-methoxyethoxy)ethan-1-ol (1g, 8.3mmol) and triethylamine (2.1g, 20.8mmol) were dissolved in dichloromethane (30mL), p-toluenesulfonyl chloride (1.75g, 9.2mmol) was added at 0°C, and reacted at 25°C for 2 hours. Saturated aqueous solution of sodium bicarbonate was added, extracted with dichloromethane, dried over anhydrous sodium sulfate, filtered and evaporated to dryness to afford 1.8g of yellow oil.

Step 2 to Step 4: 5-benzyl-N-(4-(5-(2-(2-methoxyethoxy)ethoxy)-2-methylphenyl)pyridin-2-yl)-4H-1,2,4-triazole-3-carboxamide

The operation is the same as steps 4 to 6 of Example 55, but 3-bromo-4-methylphenol was used in place of 3-bromo-N,4-dimethylaniline in the fourth step, 2-(2-methoxyethoxy)ethyl 4-methylbenzenesulfonate was used in place of 1-bromo-2-methoxyethane in the fourth step. $^1$H NMR (400 MHz, DMSO-$d_6$) δ 14.59 (s, 1H), 9.91 (s, 1H), 8.42 (d, *J* = 5.1 Hz, 1H), 8.12 (d, *J* = 1.4 Hz, 1H), 7.37 - 7.29 (m, 4H), 7.29 - 7.23 (m, 2H), 7.21 (dd, *J* = 5.1, 1.6 Hz, 1H), 6.95 (dd, *J* = 8.4, 2.8 Hz, 1H), 6.84 (d, *J* = 2.7 Hz, 1H), 4.17 (s, 2H), 4.14 - 4.05 (m, 2H), 3.76 - 3.67 (m, 2H), 3.61 - 3.53 (m, 2H), 3.47 - 3.41 (m, 2H), 3.23 (s, 3H), 2.19 (s, 3H). MS: 488 $[M+H]^+$.

Example 86: 5-benzyl-N-(4-(2-methyl-5-(oxetan-3-yloxy)phenyl)pyridin-2-yl)-4H-1,2,4-triazole-3-carboxamide

The preparation was carried out in a similar manner to Example 85, except that in step 1, oxetan-3-ol was used in place of 2-(2-methoxyethoxy)ethan-1-ol. $^1$H NMR (400 MHz, DMSO-$d_6$) δ 14.61 (s, 1H), 9.92 (s, 1H), 8.42 (d, *J* = 5.1 Hz, 1H), 8.10 (s, 1H), 7.43 - 7.30 (m, 4H), 7.30 - 7.23 (m, 2H), 7.20 (dd, *J* = 5.1, 1.5 Hz, 1H), 6.80 (dd, *J* = 8.4, 2.7 Hz, 1H), 6.69 (d, *J* = 2.7 Hz, 1H), 5.36 - 5.26 (m, 1H), 4.96 - 4.86 (m, 2H), 4.61 - 4.50 (m, 2H), 4.18 (s, 2H), 2.19 (s, 3H). MS: 442 [M+H]$^+$.

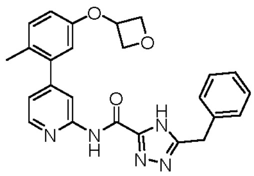

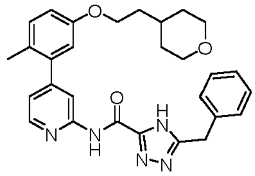

Example 86 Example 87

Example 87: 5-benzyl-N-(4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1,2-( tetrahydro-2H-pyran-4-yl)ethan-1-ol was used in place of 2-(2-methoxyethoxy)ethan-1-ol. $^1$H NMR (400 MHz, DMSO-$d_6$) δ 14.58 (s, 1H), 9.89 (s, 1H), 8.41 (d, *J* = 5.1 Hz, 1H), 8.11 (s, 1H), 7.49 - 7.14 (m, 7H), 6.94 (dd, *J* = 8.4, 2.7 Hz, 1H), 6.82 (d, *J* = 2.7 Hz, 1H), 4.18 (s, 2H), 4.02 (t, *J* = 6.3 Hz, 2H), 3.89 - 3.75 (m, 2H), 3.30 - 3.19 (m, 2H), 2.19 (s, 3H), 1.77 - 1.55 (m, 5H), 1.26 - 1.12 (m, 2H). MS: 498 [M+H]$^+$.

Example 88: 5-(cyclopentylmethyl)-N-(4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.37 (s, 1H), 9.84 (s, 1H), 8.42 (d, *J* = 5.1 Hz, 1H), 8.13 (s, 1H), 7.30 - 7.16 (m, 2H), 6.94 (dd, *J* = 8.4, 2.7 Hz, 1H), 6.83 (d, *J* = 2.7 Hz, 1H), 4.09 - 3.98 (m, 2H), 3.88 - 3.76 (m, 2H), 3.32 - 3.21 (m, 2H), 2.85 - 2.72 (m, 2H), 2.36 - 2.22 (m, 1H), 2.19 (s, 3H), 1.79 - 1.45 (m, 11H), 1.31 - 1.14 (m, 4H). MS: 490 [M+H]$^+$.

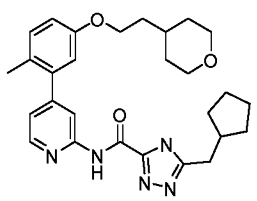

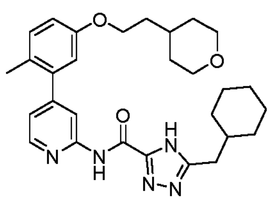

Example 88 Example 89

Example 89: 5-(cyclohexylmethyl)-N-(4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 3, ethyl 5-was used in place of ethyl 5-benzyl-4H-1,2,4-triazol-3-carboxylate. $^1$H NMR (400 MHz, DMSO-$d_6$) δ 14.37 (s, 1H), 9.85 (s, 1H), 8.42 (d, *J* = 5.1 Hz, 1H), 8.13 (s, 1H), 7.31 - 7.15 (m, 2H), 6.94 (dd, *J* = 8.4, 2.7 Hz, 1H), 6.83 (d, *J* = 2.7 Hz, 1H), 4.03 (t, *J* = 6.3 Hz, 2H), 3.87 - 3.75 (m, 2H), 3.32 - 3.21 (m, 2H), 2.67 (d, *J* = 7.0 Hz, 2H), 2.19 (s, 3H), 1.83 - 1.72 (m, 1H), 1.71 - 1.54 (m, 9H), 1.31 - 1.09 (m, 6H), 1.05 - 0.88 (m, 2H). MS: 504 [M+H]$^+$.

Example 90: 5-benzyl-N-(4-(5-((4-cyanopentyl)oxy)-2-methylphenyl)pyridin-2-yl)-4H-1,2,4-triazole-3-carboxamide

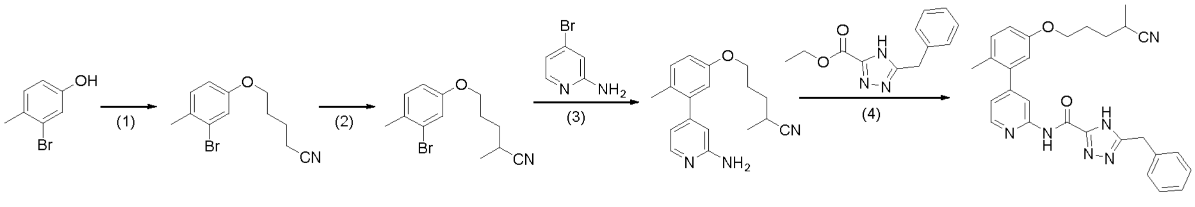


Step 1: Synthesis of 5-(3-bromo-4-methylphenoxy)valeronitrile

The operation is the same as step 4 of Example 55, but 3-bromo-4-methylphenol was used in place of 3-bromo-N,4-dimethylaniline in the fourth step, and 5-chlorovaleronitrile was used in place of 1-bromo-2-methoxyethane in the fourth step.

Step 2: Synthesis of 5-(3-bromo-4-methylphenoxy)-2-methylvaleronitrile

5-(3-Bromo-4-methylphenoxy)valeronitrile (268mg, 1mmol) was dissolved in anhydrous tetrahydrofuran (2mL), and LDA (2M, 0.8mL, 1.6mmol) was added dropwise at -78°C under argon protection. After reacting for 1 hour, iodomethane (215mg, 1.5mmol) was added dropwise at the same temperature, the reaction was gradually warmed to 25°C, and reacted for 16 hours. The reaction was quenched with saturated ammonium chloride, extracted with ethyl acetate, washed with saturated brine, dried over anhydrous sodium sulfate, filtered and dried, and purified by column chromatography to afford 210 mg of product.

Step 3 to Step 4: The preparation was carried out in a similar manner to Example 33, except that in step 1, 5-(3-bromo-4-methylphenoxy)-2-methylvaleronitrile was used in place of 2-bromo-4-fluoro-1-methylbenzene. $^1$H NMR (400 MHz, DMSO-$d_6$) $\delta$ 14.63 (s, 1H), 9.91 (s, 1H), 8.42 (d, *J* = 5.1 Hz, 1H), 8.11 (s, 1H), 7.47 - 7.16 (m, 7H), 6.95 (dd, *J* = 8.4, 2.7 Hz, 1H), 6.83 (d, *J* = 2.7 Hz, 1H), 4.18 (s, 2H), 4.02 (t, *J* = 6.2 Hz, 2H), 2.99 - 2.84 (m, 1H), 2.19 (s, 3H), 1.93 - 1.74 (m, 2H), 1.74 - 1.60 (m, 2H), 1.24 (d, *J* = 7.0 Hz, 3H). MS: 481 [M+H]$^+$.

Example 91: 5-benzyl-N-(4-(5-(4-cyanobutoxy)-2-methylphenyl)pyridin-2-yl)-4H-1,2,4-triazole-3-carboxamide

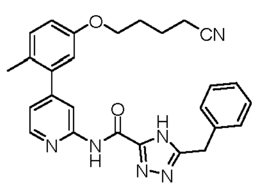

The operation is the same as step of Example 90, but the product of the first step 5-(3-bromo-4-methylphenoxy)valeronitrile was used directly to carry out the third to fourth step reaction. $^1$H NMR (400 MHz, DMSO-$d_6$) $\delta$ 14.47 (s, 1H), 9.91 (s, 1H), 8.42 (d, *J* = 5.0 Hz, 1H), 8.11 (d, *J* = 1.4 Hz, 1H), 7.41 -7.30 (m, 4H), 7.29 - 7.18 (m, 3H), 6.94 (dd, *J* = 8.4, 2.7 Hz, 1H), 6.83 (d, *J* = 2.7 Hz, 1H), 4.17 (s, 2H), 4.02 (t, *J* = 6.1 Hz, 2H), 2.57 (t, *J* = 7.0 Hz, 2H), 2.19 (s, 3H), 1.93 - 1.59 (m, 4H). MS: 467 [M+H]$^+$.

Example 92: 5-benzyl-N-(4-(2-methoxy-6-methylphenyl)pyridin-2-yl)-4H-1,2,4-triazole-3-carboxamide

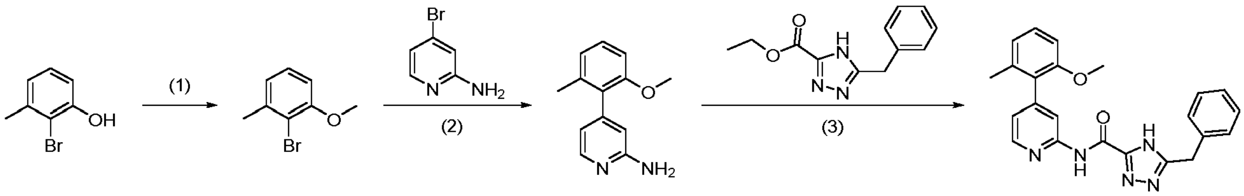


Step 1: Synthesis of 2-bromo-1-methoxy-3-methylbenzene

2-Bromo-3-methylphenol (374mg, 2mmol) was placed in acetonitrile (6mL), potassium carbonate (553mg, 4mmol) was added and stirred at 25°C for 1 hour. Iodomethane (426mg, 3mmol) was added, and reacted at 80°C for 16 hours in sealed tube. The reaction solution was diluted with ethyl acetate, and filtered. The filtrate was evaporated to dryness, and subjected to column chromatography to afford 340 mg of yellow oil. MS: 201 $[M+H]^+$

Step 2 to Step 3: The preparation was carried out in a similar manner to Example 33, except that in step 1, 2-bromo-1-methoxy-3-methylbenzene was used in place of 2-bromo-4-fluoro-1-methylbenzene. $^1H$ NMR (400 MHz, DMSO-$d_6$) δ 14.56 (s, 1H), 9.84 (s, 1H), 8.40 (dd, *J* = 5.0, 0.8 Hz, 1H), 7.96 (s, 1H), 7.46-7.30 (m, 5H), 7.30-7.17 (m, 1H), 7.04 (dd, *J* = 5.1, 1.5 Hz, 1H), 7.01-6.85 (m, 2H), 4.18 (s, 2H), 3.67 (s, 3H), 2.04 (s, 3H). MS: 400 $[M+H]^+$.

Example 93: 5-benzyl-N-(4-(2-ethoxy-6-methylphenyl)pyridin-2-yl)-4H-1,2,4-triazole-3-carboxamide

The preparation was carried out in a similar manner to Example 92, except that in step 1, bromoethane was used in place of iodomethane. $^1H$ NMR (400 MHz, DMSO-$d_6$) δ 14.56 (s, 1H), 9.83 (s, 1H), 8.39 (d, *J* = 5.1 Hz, 1H), 7.99 (s, 1H), 7.43 - 7.29 (m, 4H), 7.29 - 7.16 (m, 2H), 7.05 (dd, *J* = 5.1, 1.5 Hz, 1H), 7.01 - 6.89 (m, 2H), 4.18 (s, 2H), 3.98 (q, *J* = 7.0 Hz, 2H), 2.05 (s, 3H), 1.13 (t, *J* = 7.0 Hz, 3H). MS: 414 $[M+H]^+$.

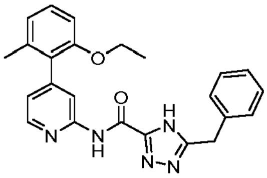

Example 93

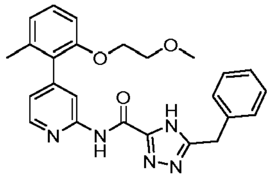

Example 94

Example 94: 5-benzyl-N-(4-(2-(2-methoxyethoxy)-6-methylphenyl)pyridin-2-yl)-4H-1,2,4-triazole-3-carboxamide

The preparation was carried out in a similar manner to Example 92, except that in step 1, 1-bromo-2-methoxyethane was used in place of iodomethane. $^1H$ NMR (400 MHz, DMSO-$d_6$) δ 14.52 (s, 1H), 9.80 (s, 1H), 8.39 (d, *J* = 5.1 Hz, 1H), 8.00 (s, 1H), 7.39-7.30 (m, 4H), 7.30-7.20 (m, 2H), 7.06 (d, *J* = 5.1 Hz, 1H), 7.01-6.87 (m, 2H), 4.19 (s, 2H), 4.09-3.98 (m, 2H), 3.52-3.41 (m, 2H), 3.10 (s, 3H), 2.07 (s, 3H). MS: 444 $[M+H]^+$.

Example 95: 5-benzyl-N-(4-(3-(2-hydroxyethoxy)-2-methylphenyl)pyridin-2-yl)-4H-1,2,4-triazole-3-carboxamide

The preparation was carried out in a similar manner to Example 92, except that in step 1, 2-bromo-1-ethanol was used in place of iodomethane, and 3-bromo-2-methylphenol was used in place of 2-bromo-3-methylphenol. $^1H$ NMR (400 MHz, DMSO-$d_6$) δ 14.60 (s, 1H), 9.93 (s, 1H), 8.41 (d, *J* = 5.0 Hz, 1H), 8.10 (d, *J* = 1.4 Hz, 1H), 7.50 - 7.21 (m, 6H), 7.16 (dd, *J* = 5.1, 1.6 Hz, 1H), 7.05 (d, *J* = 8.2 Hz, 1H), 6.85 (d, *J* = 7.5 Hz, 1H), 5.16 - 4.62 (m, 1H), 4.18 (s, 2H), 4.05 (t, *J* = 5.0 Hz, 2H), 3.76 (t, *J* = 5.0 Hz, 2H), 2.11 (s, 3H). MS: 430 $[M+H]^+$.

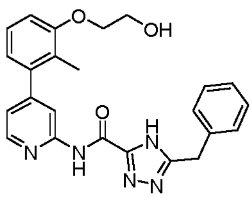 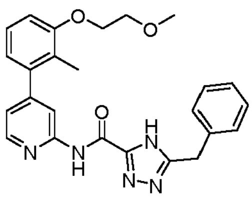

Example 95 Example 96

Example 96: 5-benzyl-N-(4-(3-(2-methoxyethoxy)-2-methylphenyl)pyridin-2-yl)-4H-1,2,4-triazole-3-carboxamide

The preparation was carried out in a similar manner to Example 92, except that in step 1, 1-bromo-2-methoxyethane was used in place of iodomethane, and 3-bromo-2-methylphenol was used in place of 2-bromo-3-methylphenol. $^1$H NMR (400 MHz, DMSO-$d_6$) δ 14.21 (s, 1H), 9.93 (s, 1H), 8.41 (d, *J* = 5.1 Hz, 1H), 8.10 (s, 1H), 7.54 - 7.20 (m, 6H), 7.16 (dd, *J* = 5.0, 1.5 Hz, 1H), 7.05 (d, *J* = 8.2 Hz, 1H), 6.86 (d, *J* = 7.6 Hz, 1H), 4.28 - 4.06 (m, 4H), 3.75 - 3.66 (m, 2H), 3.33 (s, 3H), 2.09 (s, 3H). MS: 444 $[M+H]^+$.

Example 97: 5-benzyl-N-(4-(3-(3-hydroxylpropoxy)-2-methylphenyl)pyridin-2-yl)-4H-1,2,4-triazole-3-carboxamide

The preparation was carried out in a similar manner to Example 92, except that in step 1, 3-bromo-1-propanol was used in place of iodomethane, and 3-bromo-2-methylphenol was used in place of 2-bromo-3-methylphenol. $^1$H NMR (400 MHz, DMSO-$d_6$) δ 14.59 (s, 1H), 9.91 (s, 1H), 8.41 (d, *J* = 5.1 Hz, 1H), 8.08 (d, *J* = 1.4 Hz, 1H), 7.44-7.21 (m, 6H), 7.17 (dd, *J* = 5.1, 1.5 Hz, 1H), 7.05 (d, *J* = 8.2 Hz, 1H), 6.85 (d, *J* = 7.6 Hz, 1H), 4.58 (t, *J* = 5.0 Hz, 1H), 4.17 (s, 2H), 4.09 (t, *J* = 6.2 Hz, 2H), 3.64-3.56 (m, 2H), 2.08 (s, 3H), 1.95-1.84 (m, 2H). MS: 444 $[M+H]^+$.

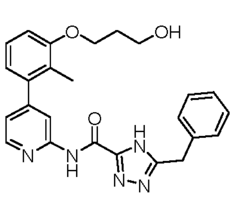 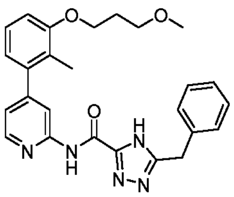

Example 97 Example 98

Example 98: 5-benzyl-N-(4-(3-(3-methoxypropoxy)-2-methylphenyl)pyridin-2-yl)-4H-1,2,4-triazole-3-carboxamide

The preparation was carried out in a similar manner to Example 92, except that in step 1, 1-bromo-3-methoxypropane was used in place of iodomethane, and 3-bromo-2-methylphenol was used in place of 2-bromo-3-methylphenol. $^1$H NMR (400 MHz, DMSO-$d_6$) δ 14.65 (s, 1H), 9.92 (s, 1H), 8.51 - 8.31 (m, 1H), 8.20 - 7.99 (m, 1H), 7.42 -7.30 (m, 4H), 7.30 - 7.20 (m, 2H), 7.16 (dd, *J* = 5.1, 1.6 Hz, 1H), 7.04 (dd, *J* = 8.4, 1.1 Hz, 1H), 6.85 (dd, *J* = 7.7, 1.1 Hz, 1H), 4.17 (s, 2H), 4.07 (t, *J* = 6.2 Hz, 2H), 3.51 (t, *J* = 6.3 Hz, 2H), 3.25 (s, 3H), 2.08 (s, 3H), 2.04 - 1.91 (m, 2H). MS: 458 $[M+H]^+$.

Example 99: 5-benzyl-N-(4-(3-(4-hydroxybutoxy)-2-methylphenyl)pyridin-2-yl)-4H-1,2,4-triazole-3-carboxamide

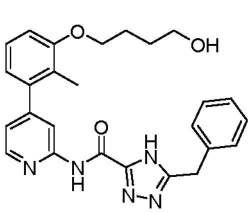

The preparation was carried out in a similar manner to Example 92, except that in step 1, 4-bromo-1-butanol was used in place of iodomethane, and 3-bromo-2-methylphenol was used in place of 2-bromo-3-methylphenol. $^1$H NMR

(400 MHz, DMSO-$d_6$) δ 14.59 (s, 1H), 9.90 (s, 1H), 8.41 (d, *J* = 5.0 Hz, 1H), 8.09 (s, 1H), 7.40-7.21 (m, 6H), 7.17 (d, *J* = 5.1 Hz, 1H), 7.04 (d, *J* = 8.2 Hz, 1H), 6.85 (d, *J* = 7.6 Hz, 1H), 4.53-4.41 (m, 1H), 4.17 (s, 2H), 4.04 (t, *J* = 6.3 Hz, 2H), 3.51-3.44 (m, 2H), 2.09 (s, 3H), 1.87-1.73 (m, 2H), 1.68-1.55 (m, 2H). MS: 458 $[M+H]^+$.

Example 100: 5-benzyl-N-(4-(2-methyl-5-(octyloxy)phenyl)pyridin-2-yl)-4H-1,2,4-triazole-3-carboxamide

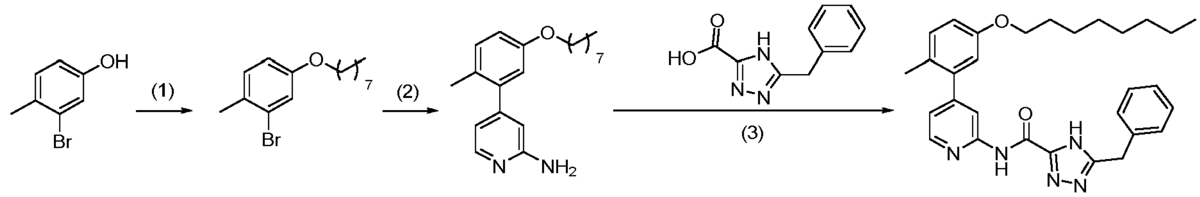


Step 1 to Step 2: Synthesis of 4-(2-methyl-5-(octyloxy)phenyl)pyridin-2-amine

The operation is the same as steps 1 to 2 of Example 92, but 3-bromo-2-methylphenol was used in place of 2-bromo-3-methylphenol in step 1, and 1-bromooctane was used in place of iodomethane in step 1. MS: 313 $[M+H]^+$.

Step 3: Intermediate 2 (98mg, 0.48mmol) was dissolved in DMF (3mL), 1-propanephosphonic acid cyclic anhydride (50%, 305mg, 0.48mmol), diisopropylethylamine (124mg, 0.96mmol) and 4-(2-methyl-5-(octyloxy)phenyl)pyridin-2-amine (100mg, 0.3mmol) were added, and reacted at 25°C for 16 hours. The reaction solution was diluted with water, extracted with dichloromethane, dried over anhydrous sodium sulfate, filtered, evaporated to dryness, and subjected to column chromatography and preparative liquid chromatography to afford 30mg of white solid. $^1$H NMR (400 MHz, DMSO-$d_6$) δ 14.74 (s, 1H), 9.92 (s, 1H), 8.41 (d, *J* = 5.1 Hz, 1H), 8.12 (d, *J* = 1.4 Hz, 1H), 7.41 - 7.29 (m, 4H), 7.29 - 7.13 (m, 3H), 6.91 (dd, *J* = 8.4, 2.7 Hz, 1H), 6.80 (d, *J* = 2.6 Hz, 1H), 4.17 (s, 2H), 3.95 (t, *J* = 6.5 Hz, 2H), 2.18 (s, 3H), 1.76 - 1.57 (m, 2H), 1.45 - 1.33 (m, 2H), 1.33 - 1.14 (m, 8H), 0.91 - 0.76 (m, 3H). MS: 498 $[M+H]^+$.

Example 101: 5-benzyl-N-(4-(5-methoxy-2-methylphenyl)pyridin-2-yl)-4H-1,2,4-triazole-3-carboxamide

The preparation was carried out in a similar manner to Example 92, except that in step 1, 3-bromo-4-methylphenol was used in place of 2-bromo-3-methylphenol. $^1$H NMR (600 MHz, DMSO-$d_6$) δ 14.60 (s, 1H), 9.89 (s, 1H), 8.42 (d, *J* = 5.1 Hz, 1H), 8.12 (s, 1H), 7.42 - 7.31 (m, 4H), 7.31 - 7.23 (m, 2H), 7.21 (dd, *J* = 5.0, 1.5 Hz, 1H), 6.94 (dd, *J* = 8.4, 2.8 Hz, 1H), 6.82 (d, *J* = 2.8 Hz, 1H), 4.18 (s, 2H), 3.77 (s, 3H), 2.19 (s, 3H). MS: 400 $[M+H]^+$.

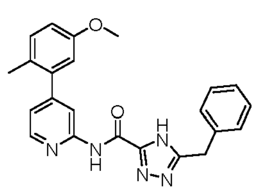
Example 101

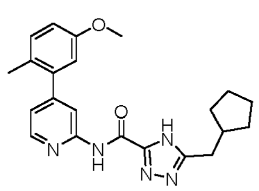
Example 102

Example 102: 5-(cyclopentylmethyl)-N-(4-(5-methoxy-2-methylphenyl)pyridin-2-yl)-4H-1,2,4-triazole-3-carboxamide

The preparation was carried out in a similar manner to Example 92, except that in step 1, 3-bromo-4-methylphenol was used in place of 2-bromo-3-methylphenol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.37 (s, 1H), 9.85 (s, 1H), 8.42 (d, *J* = 5.1 Hz, 1H), 8.14 (s, 1H), 7.27 (d, *J* = 8.4 Hz, 1H), 7.21 (d, *J* = 5.1 Hz, 1H), 6.95 (dd, *J* = 8.5, 2.8 Hz, 1H), 6.83 (d, *J* = 2.8 Hz, 1H), 3.77 (s, 3H), 2.78 (d, *J* = 7.5 Hz, 2H), 2.34 - 2.22 (m, 1H), 2.20 (s, 3H), 1.80 - 1.66 (m, 2H), 1.66 - 1.43 (m, 4H), 1.30 - 1.13 (m, 2H). MS: 392 $[M+H]^+$.

Example 103: 5-(cyclohexylmethyl)-N-(4-(5-methoxy-2-methylphenyl)pyridin-2-yl)-4H-1,2,4-triazole-3-carboxamide

The preparation was carried out in a similar manner to Example 92, except that in step 1, 3-bromo-4-methylphenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.86 (s, 1H), 8.42 (d, $J$ = 5.1 Hz, 1H), 8.14 (s, 1H), 7.34 - 7.16 (m, 2H), 7.03 - 6.88 (m, 1H), 6.83 (d, $J$ = 2.9 Hz, 1H), 3.77 (s, 3H), 2.66 (d, $J$ = 7.0 Hz, 2H), 2.19 (s, 3H), 1.83-1.52 (m, 6H), 1.30-1.06 (m, 3H), 1.06-0.86 (m, 2H). MS: 406 [M+H]$^+$.

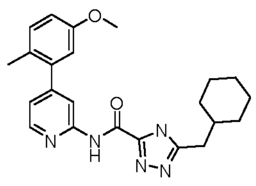
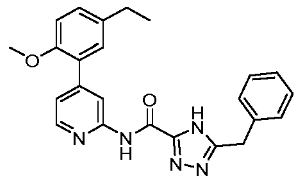

Example 103 Example 104

Example 104: 5-benzyl-N-(4-(5-ethyl-2-methoxyphenyl)pyridin-2-yl)-4H-1,2,4-triazole-3-carboxamide

The preparation was carried out in a similar manner to Example 92, except that in step 1, 2-bromo-4-ethylphenol was used in place of 2-bromo-3-methylphenol. $^1$H NMR (400 MHz, DMSO-$d_6$) δ 14.59 (s, 1H), 9.83 (s, 1H), 8.36 (d, $J$ = 5.2 Hz, 1H), 8.29 (d, $J$ = 1.4 Hz, 1H), 7.54 - 7.16 (m, 8H), 7.09 (d, $J$ = 8.4 Hz, 1H), 4.17 (s, 2H), 3.78 (s, 3H), 2.61 (q, $J$ = 7.6 Hz, 2H), 1.19 (t, $J$ = 7.5 Hz, 3H). MS: 414 [M+H]$^+$.

Example 105: 5-benzyl-N-(5-(2-chloro-5-ethoxyphenyl)pyridin-3-yl)-4H-1,2,4-triazole-3-carboxamide

The preparation was carried out in a similar manner to Example 92, except that in step 1, bromoethane was used in place of iodomethane, 3-bromo-4-chlorophenol was used in place of 2-bromo-3-methylphenol, and in step 2, 5-bromopyridin-3-amine was used in place of 4-bromopyridin-2-amine. $^1$H NMR (400 MHz, DMSO-$d_6$) δ 14.60 (s, 1H), 10.82 (s, 1H), 9.03 (d, $J$ = 2.3 Hz, 1H), 8.44 - 8.29 (m, 2H), 7.49 (d, $J$ = 8.7 Hz, 1H), 7.39 - 7.20 (m, 5H), 7.08 - 6.97 (m, 2H), 4.17 (s, 2H), 4.08 (q, $J$ = 7.0 Hz, 2H), 1.33 (t, $J$ = 6.9 Hz, 3H). MS: 434 [M+H]$^+$.

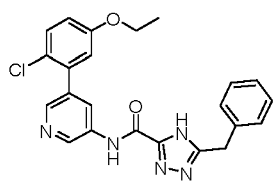
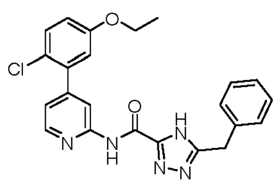

Example 105 Example 106

Example 106: 5-benzyl-N-(4-(2-chloro-5-ethoxyphenyl)pyridin-2-yl)-4H-1,2,4-triazole-3-carboxamide

The preparation was carried out in a similar manner to Example 92, except that in step 1, bromoethane was used in place of iodomethane, and 3-bromo-4-chlorophenol was used in place of 2-bromo-3-methylphenol. $^1$H NMR (400 MHz, DMSO-$d_6$) δ 14.65 (s, 1H), 9.94 (s, 1H), 8.45 (d, $J$ = 5.2 Hz, 1H), 8.23 (d, $J$ = 1.5 Hz, 1H), 7.51 (d, $J$ = 8.8 Hz, 1H), 7.40 - 7.30 (m, 4H), 7.30 - 7.20 (m, 2H), 7.06 (dd, $J$ = 8.8, 3.1 Hz, 1H), 7.01 (d, $J$ = 3.0 Hz, 1H), 4.18 (s, 2H), 4.08 (q, $J$ = 7.0 Hz, 2H), 1.33 (t, $J$ = 7.0 Hz, 3H). MS: 434 [M+H]$^+$.

Example 107: 5-benzyl-N-(4-(5-ethoxy-2-methoxyphenyl)pyridin-2-yl)-4H-1,2,4-triazole-3-carboxamide

The preparation was carried out in a similar manner to Example 92, except that in step 1, bromoethane was used in place of iodomethane, and 3-bromo-4-methoxyphenol was used in place of 2-bromo-3-methylphenol. $^1$H NMR (600 MHz, DMSO-$d_6$) δ 14.54 (s, 1H), 9.79 (s, 1H), 8.43 - 8.24 (m, 2H), 7.47 - 7.29 (m, 5H), 7.27 (d, $J$ = 6.5 Hz, 1H), 7.10 (d, $J$ = 9.0 Hz, 1H), 7.01 (dd, $J$ = 9.0, 3.1 Hz, 1H), 6.94 (d, $J$ = 3.1 Hz, 1H), 4.19 (s, 2H), 4.03 (q, $J$ = 6.9 Hz, 2H), 3.75 (s, 3H), 1.32 (t, $J$ = 6.9 Hz, 3H). MS: 430 [M+H]$^+$.

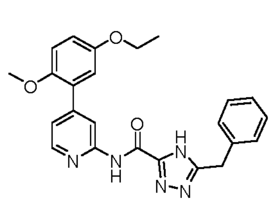

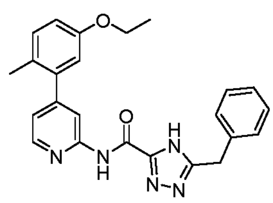

Example 107 Example 108

Example 108: 5-benzyl-N-(4-(5-ethoxy-2-methylphenyl)pyridin-2-yl)-4H-1,2,4-triazole-3-carboxamide

The preparation was carried out in a similar manner to Example 92, except that in step 1, bromoethane was used in place of iodomethane, and 3-bromo-4-methylphenol was used in place of 2-bromo-3-methylphenol. $^1$H NMR (600 MHz, DMSO-$d_6$) δ 14.62 (s, 1H), 9.90 (s, 1H), 8.41 (d, *J* = 5.1 Hz, 1H), 8.11 (s, 1H), 7.41 - 7.30 (m, 4H), 7.30 - 7.22 (m, 2H), 7.20 (dd, *J* = 5.1, 1.5 Hz, 1H), 6.93 (dd, *J* = 8.4, 2.7 Hz, 1H), 6.80 (d, *J* = 2.7 Hz, 1H), 4.17 (s, 2H), 4.04 (q, *J* = 7.0 Hz, 2H), 2.19 (s, 3H), 1.32 (t, *J* = 7.0 Hz, 3H). MS: 414 $[M+H]^+$.

Example 109: 5-benzyl-N-(4-(1-ethyl-3-methyl-1H-pyrazol-5-yl)pyridin-2-yl)-4H-1,2,4-triazole-3-carboxamide

The preparation was carried out in a similar manner to Example 92, except that in step 1, bromoethane was used in place of iodomethane, and 5-bromo-3-methyl-1H-pyrazole was used in place of 2-bromo-3-methylphenol. $^1$H NMR (400 MHz, DMSO-$d_6$) δ 14.63 (s, 1H), 9.98 (s, 1H), 8.46 (d, *J* = 5.1 Hz, 1H), 8.35 - 8.14 (m, 1H), 7.51 - 7.15 (m, 6H), 6.35 (s, 1H), 4.29 - 3.95 (m, 4H), 2.21 (s, 3H), 1.36 (t, *J* = 7.2 Hz, 3H). MS: 388 $[M+H]^+$.

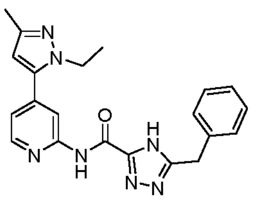

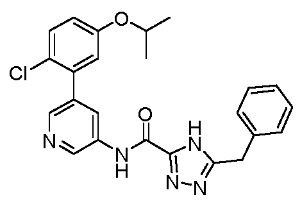

Example 109 Example 110

Example 110: 5-benzyl-N-(5-(2-chloro-5-isopropoxyphenyl)pyridin-3-yl)-4H-1,2,4-triazole-3-carboxamide

The preparation was carried out in a similar manner to Example 92, except that in step 1, 2-bromopropane was used in place of iodomethane, 3-bromo-4-chlorophenol was used in place of 2-bromo-3-methylphenol, and in step 2, 5-bromopyridin-3-amine was used in place of 4-bromopyridin-2-amine. $^1$H NMR (600 MHz, DMSO-$d_6$) δ 14.64 (s, 1H), 10.83 (s, 1H), 9.04 (d, *J* = 2.2 Hz, 1H), 8.49 - 8.20 (m, 2H), 7.48 (dd, *J* = 8.6, 1.9 Hz, 1H), 7.41 - 7.31 (m, 2H), 7.31 - 7.28 (m, 2H), 7.28 - 7.22 (m, 1H), 7.07 - 6.98 (m, 2H), 4.74 - 4.64 (m, 1H), 4.18 (s, 2H), 1.28 (dd, *J* = 6.1, 1.9 Hz, 6H). MS: 448 $[M+H]^+$.

Example 111: 5-benzyl-N-(4-(2-chloro-5-isopropoxyphenyl)pyridin-2-yl)-4H-1,2,4-triazole-3-carboxamide

The preparation was carried out in a similar manner to Example 92, except that in step 1, 2-bromopropane was used in place of iodomethane, and 3-bromo-4-chlorophenol was used in place of 2-bromo-3-methylphenol. $^1$H NMR (400 MHz, DMSO-$d_6$) δ 14.66 (s, 1H), 9.94 (s, 1H), 8.45 (d, *J* = 5.1 Hz, 1H), 8.22 (s, 1H), 7.50 (dd, *J* = 8.8, 2.8 Hz, 1H), 7.41 - 7.30 (m, 4H), 7.30 - 7.22 (m, 2H), 7.09 - 7.02 (m, 1H), 6.99 (d, *J* = 3.0 Hz, 1H), 4.69 (p, *J* = 6.0 Hz, 1H), 4.17 (s, 2H), 1.28 (d, *J* = 6.1, 2.7 Hz, 6H). MS: 448 $[M+H]^+$.

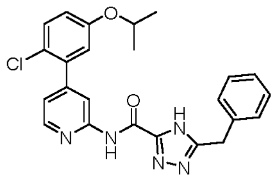 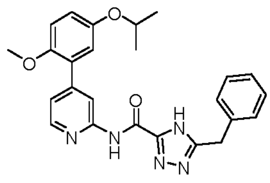

Example 111 Example 112

Example 112: 5-benzyl-N-(4-(5-isopropoxy-2-methoxyphenyl)pyridin-2-yl)-4H-1,2,4-triazole-3-carboxamide

The preparation was carried out in a similar manner to Example 92, except that in step 1, 2-bromopropane was used in place of iodomethane, and 3-bromo-4-methoxyphenol was used in place of 2-bromo-3-methylphenol. $^1$H NMR (600 MHz, DMSO-$d_6$) δ 14.59 (s, 1H), 9.84 (s, 1H), 8.37 (d, *J* = 5.2 Hz, 1H), 8.30 (s, 1H), 7.40 - 7.29 (m, 5H), 7.29 - 7.22 (m, 1H), 7.09 (d, *J* = 9.0 Hz, 1H), 7.00 (dd, *J* = 8.9, 3.0 Hz, 1H), 6.93 (d, *J* = 3.0 Hz, 1H), 4.56 (p, *J* = 6.0 Hz, 1H), 4.18 (s, 2H), 3.75 (s, 3H), 1.25 (d, *J* = 6.0 Hz, 6H). MS: 444 [M+H]$^+$.

Example 113: 5-benzyl-N-(5-(5-isopropoxy-2-methylphenyl)pyridin-3-yl)-4H-1,2,4-triazole-3-carboxamide

The preparation was carried out in a similar manner to Example 92, except that in step 1, 2-bromopropane was used in place of iodomethane, 3-bromo-4-methylphenol was used in place of 2-bromo-3-methylphenol, and in step 2, 5-bromopyridin-3-amine was used in place of 4-bromopyridin-2-amine. $^1$H NMR (400 MHz, DMSO-$d_6$) δ 14.62 (s, 1H), 10.79 (s, 1H), 9.02 (d, *J* = 2.3 Hz, 1H), 8.31 (d, *J* = 2.0 Hz, 1H), 8.25 - 8.08 (m, 1H), 7.42 - 7.16 (m, 6H), 6.88 (dd, *J* = 8.4, 2.7 Hz, 1H), 6.79 (d, *J* = 2.7 Hz, 1H), 4.62 (p, *J* = 6.0 Hz, 1H), 4.17 (s, 2H), 2.17 (s, 3H), 1.26 (d, *J* = 6.0 Hz, 6H). MS: 428 [M+H]$^+$.

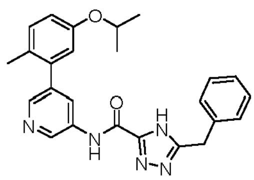 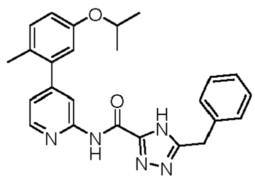

Example 113 Example 114

Example 114: 5-benzyl-N-(4-(5-isopropoxy-2-methylphenyl)pyridin-2-yl)-4H-1,2,4-triazole-3-carboxamide

The preparation was carried out in a similar manner to Example 92, except that in step 1, 2-bromopropane was used in place of iodomethane, and 3-bromo-4-methylphenol was used in place of 2-bromo-3-methylphenol. $^1$H NMR (600 MHz, DMSO-$d_6$) δ 14.57 (s, 1H), 9.90 (s, 1H), 8.58 - 8.29 (m, 1H), 8.11 (s, 1H), 7.63 - 7.29 (m, 4H), 7.29 - 7.06 (m, 3H), 6.92 (d, *J* = 8.0 Hz, 1H), 6.78 (d, *J* = 3.5 Hz, 1H), 4.67 - 4.54 (m, 1H), 4.17 (s, 2H), 2.18 (s, 3H), 1.26 (d, *J* = 6.3, 3.3 Hz, 6H). MS: 428 [M+H]$^+$.

Example 115: 5-benzyl-N-(4-(5-(3,3-dimethylbutoxy)-2-methylphenyl)pyridin-2-yl)-4H-1,2,4-triazole-3-carboxamide

The preparation was carried out in a similar manner to Example 92, except that in step 1, 1-bromo-3,3-dimethylbutane was used in place of iodomethane, and 3-bromo-4-methylphenol was used in place of 2-bromo-3-methylphenol. $^1$H NMR (400 MHz, DMSO-$d_6$) δ 14.62 (s, 1H), 9.91 (s, 1H), 8.41 (d, *J* = 5.1 Hz, 1H), 8.11 (s, 1H), 7.48 - 7.13 (m, 7H), 6.94 (dd, *J* = 8.4, 2.7 Hz, 1H), 6.82 (d, *J* = 2.7 Hz, 1H), 4.17 (s, 2H), 4.02 (t, *J* = 7.2 Hz, 2H), 2.18 (s, 3H), 1.65 (t, *J* = 7.2 Hz, 2H), 0.95 (s, 9H). MS: 470 [M+H]$^+$.

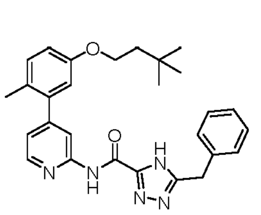

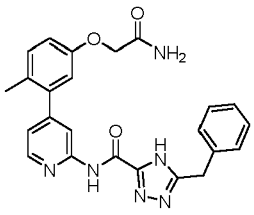

Example 115 Example 116

Example 116: N-(4-(5-(2-amino-2-oxoethoxy)-2-methylphenyl)pyridin-2-yl)-5-benzyl-4H-1,2,4-triazole-3-carboxamide

The preparation was carried out in a similar manner to Example 92, except that in step 1, 2-chloroacetamide was used in place of iodomethane, and 3-bromo-4-methylphenol was used in place of 2-bromo-3-methylphenol. $^1$H NMR (400 MHz, DMSO-$d_6$) δ 14.61 (s, 1H), 9.91 (s, 1H), 8.43 (d, *J* = 5.1 Hz, 1H), 8.12 (s, 1H), 7.54 (s, 1H), 7.42 - 7.30 (m, 5H), 7.30 - 7.22 (m, 2H), 7.20 (dd, *J* = 5.1, 1.6 Hz, 1H), 6.96 (dd, *J* = 8.4, 2.8 Hz, 1H), 6.88 (d, *J* = 2.8 Hz, 1H), 4.44 (s, 2H), 4.18 (s, 2H), 2.20 (s, 3H). MS: 443 $[M+H]^+$.

Example 117: 5-benzyl-N-(4-(5-(2-hydroxyl-2-methylpropoxy)-2-methylphenyl)pyridin-2-yl)-4H-1,2,4-triazole-3-carboxamide

The preparation was carried out in a similar manner to Example 92, except that in step 1, 1-chloro-2-methylpropyl-2-ol was used in place of iodomethane, and 3-bromo-4-methylphenol was used in place of 2-bromo-3-methylphenol. $^1$H NMR (400 MHz, DMSO-$d_6$) δ 14.91 - 14.25 (m, 1H), 9.90 (s, 1H), 8.42 (d, *J* = 5.1 Hz, 1H), 8.12 (s, 1H), 7.45 - 7.13 (m, 7H), 6.95 (dd, *J* = 8.4, 2.7 Hz, 1H), 6.82 (d, *J* = 2.7 Hz, 1H), 4.61 (s, 1H), 4.18 (s, 2H), 3.73 (s, 2H), 2.19 (s, 3H), 1.19 (s, 6H). MS: 458 $[M+H]^+$.

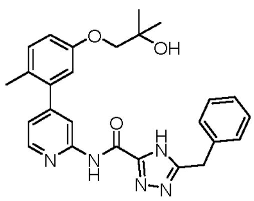

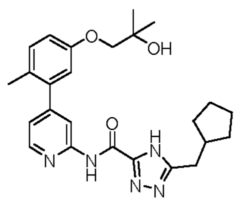

Example 117 Example 118

Example 118: 5-(cyclopentylmethyl)-N-(4-(5-(2-hydroxyl-2-methylpropoxy)-2-methylphenyl)pyridin-2-yl)-4H-1,2,4-triazole-3-carboxamide

The preparation was carried out in a similar manner to Example 92, except that in step 1, 1-chloro-2-methylpropyl-2-ol was used in place of iodomethane, 3-bromo-4-methylphenol was used in place of 2-bromo-3-methylphenol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.42 (s, 1H), 9.87 (s, 1H), 8.42 (d, *J* = 5.1 Hz, 1H), 8.14 (d, *J* = 1.4 Hz, 1H), 7.32 - 7.17 (m, 2H), 6.95 (dd, *J* = 8.4, 2.7 Hz, 1H), 6.83 (d, *J* = 2.7 Hz, 1H), 4.63 (s, 1H), 3.73 (s, 2H), 2.78 (d, *J* = 7.5 Hz, 2H), 2.33 - 2.22 (m, 1H), 2.20 (s, 3H), 1.80 - 1.66 (m, 2H), 1.66 - 1.42 (m, 4H), 1.30 - 1.10 (m, 8H). MS: 450 $[M+H]^+$.

Example 119: 5-(cyclohexylmethyl)-N-(4-(5-(2-hydroxyl-2-methylpropoxy)-2-methylphenyl)pyridin-2-yl)-4H-1,2,4-triazole-3-carboxamide

The preparation was carried out in a similar manner to Example 92, except that in step 1, 1-chloro-2-methylpropyl-2-ol was used in place of iodomethane, 3-bromo-4-methylphenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 9.85 (s, 1H), 8.42 (d, *J* = 5.1 Hz, 1H), 8.14 (s, 1H), 7.34-7.14 (m, 2H), 6.95 (dd, *J* = 8.5, 2.7 Hz, 1H), 6.83 (d, *J* = 2.8 Hz, 1H), 4.63 (s, 1H), 3.73 (s, 2H), 2.67 (d, *J* = 7.0 Hz, 2H), 2.20 (s, 3H), 1.84-1.53 (m, 6H), 1.31-1.05 (m, 9H), 1.05-0.88 (m, 2H). MS: 464 $[M+H]^+$.

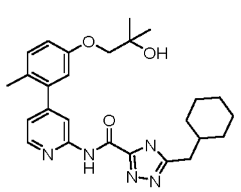

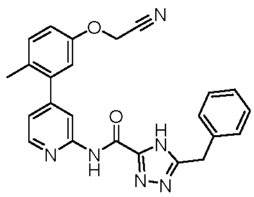

Example 119 Example 120

Example 120: 5-benzyl-N-(4-(5-(cyanomethoxy)-2-methylphenyl)pyridin-2-yl)-4H-1,2,4-triazole-3-carboxamide

The preparation was carried out in a similar manner to Example 92, except that in step 1, bromoacetonitrile was used in place of iodomethane, and 3-bromo-4-methylphenol was used in place of 2-bromo-3-methylphenol. $^{1}H$ NMR (400 MHz, DMSO-$d_6$) δ 14.57 (s, 1H), 9.91 (s, 1H), 8.44 (d, *J* = 5.1 Hz, 1H), 8.13 (s, 1H), 7.41-7.29 (m, 5H), 7.29-7.18 (m, 2H), 7.08 (dd, *J* = 8.4, 2.8 Hz, 1H), 6.99 (d, *J* = 2.8 Hz, 1H), 520 (s, 2H), 4.18 (s, 2H), 2.22 (s, 3H). MS: 425 $[M+H]^+$.

Example 121: 5-benzyl-N-(4-(2-methyl-5-((tetrahydrofuran-2-yl)methoxy)phenyl)pyridin-2-yl)-4H-1,2,4-triazole-3-carboxamide

The preparation was carried out in a similar manner to Example 92, except that in step 1, 2-(bromomethyl)tetrahydrofuran was used in place of iodomethane, and 3-bromo-4-methylphenol was used in place of 2-bromo-3-methylphenol. $^{1}H$ NMR (400 MHz, DMSO-$d_6$) δ 14.60 (s, 1H), 9.91 (s, 1H), 8.42 (d, *J* = 5.1 Hz, 1H), 8.11 (s, 1H), 7.44-7.29 (m, 4H), 7.29-7.12 (m, 3H), 6.94 (dd, *J* = 8.4, 2.7 Hz, 1H), 6.83 (d, *J* = 2.7 Hz, 1H), 4.26-4.07 (m, 3H), 4.02-3.86 (m, 2H), 3.82-3.72 (m, 1H), 3.72-3.61 (m, 1H), 2.19 (s, 3H), 2.06-1.92 (m, 1H), 1.92-1.73 (m, 2H), 1.73 - 1.58 (m, 1H). MS: 470 $[M+H]^+$.

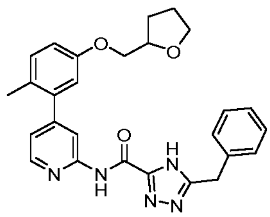

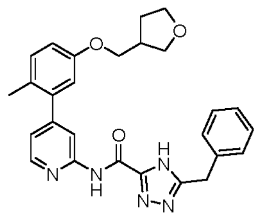

Example 121 Example 122

Example 122: 5-benzyl-N-(4-(2-methyl-5-((tetrahydrofuran-3-yl)methoxy)phenyl)pyridin-2-yl)-4H-1,2,4-triazole-3-carboxamide

The preparation was carried out in a similar manner to Example 92, except that in step 1, 3-(bromomethyl)tetrahydrofuran was used in place of iodomethane, and 3-bromo-4-methylphenol was used in place of 2-bromo-3-methylphenol. $^{1}H$ NMR (400 MHz, DMSO-$d_6$) δ 14.58 (s, 1H), 9.89 (s, 1H), 8.42 (d, *J* = 5.1 Hz, 1H), 8.11 (s, 1H), 7.41 - 7.30 (m, 4H), 7.30 - 7.23 (m, 2H), 7.21 (dd, *J* = 5.1, 1.6 Hz, 1H), 6.95 (dd, *J* = 8.4, 2.7 Hz, 1H), 6.84 (d, *J* = 2.7 Hz, 1H), 4.18 (s, 2H), 4.00 - 3.84 (m, 2H), 3.83 - 3.70 (m, 2H), 3.70 - 3.58 (m, 1H), 3.57 - 3.47 (m, 1H), 2.71 - 2.57 (m, 1H), 2.19 (s, 3H), 2.07 - 1.92 (m, 1H), 1.72 - 1.57 (m, 1H). MS: 470 $[M+H]^+$.

Example 123: 5-benzyl-N-(4-(2-methyl-5-((tetrahydro-2H-pyran-2-yl)methoxy)phenyl)pyridin-2-yl)-4H-1,2,4-triazole-3-carboxamide

The preparation was carried out in a similar manner to Example 92, except that in step 1, 2-(bromomethyl)tetrahydro-2H-pyran was used in place of iodomethane, and 3-bromo-4-methylphenol was used in place of 2-bromo-3-methylphenol. $^{1}H$ NMR (400 MHz, DMSO-$d_6$) δ 14.57 (s, 1H), 9.94 (s, 1H), 8.41 (d, *J* = 5.2 Hz, 1H), 8.11 (s, 1H), 7.41 - 7.29 (m, 4H), 7.29 - 7.15 (m, 3H), 6.93 (dd, *J* = 8.6, 2.7 Hz, 1H), 6.81 (d, *J* = 2.7 Hz, 1H), 4.17 (s, 2H), 3.97 - 3.81 (m, 3H), 3.63 - 3.56 (m, 2H), 2.18 (s, 3H), 1.85 - 1.73 (m, 1H), 1.68 - 1.57 (m, 1H), 1.55 - 1.39 (m, 3H), 1.37 - 1.16 (m, 1H). MS: 484 $[M+H]^+$.

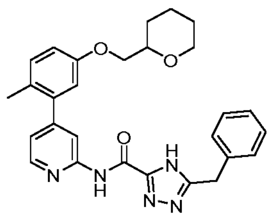 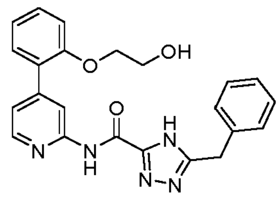

Example 123 Example 124

Example 124: 5-benzyl-N-(4-(2-(2-hydroxyethoxy)phenyl)pyridin-2-yl)-4H-1,2,4-triazole-3-carboxamide

The preparation was carried out in a similar manner to Example 92, except that in step 1, 2-bromo-1-ethanol was used in place of iodomethane, and 2-bromophenol was used in place of 2-bromo-3-methylphenol. $^1$H NMR (600 MHz, DMSO-$d_6$) δ 14.54 (s, 1H), 9.78 (s, 1H), 8.47 - 8.26 (m, 2H), 7.46 - 7.39 (m, 3H), 7.37 - 7.31 (m, 4H), 7.26 (d, *J* = 7.3 Hz, 1H), 7.19 (d, *J* = 8.4 Hz, 1H), 7.12 - 7.07 (m, 1H), 4.76 (t, *J* = 5.5 Hz, 1H), 4.18 (s, 2H), 4.08 (t, *J* = 5.2 Hz, 2H), 3.78 - 3.67 (m, 2H). MS: 416 [M+H]$^+$.

Example 125: 5-benzyl-N-(4-(5-(2-hydroxyethoxy)-2-methylphenyl)pyridin-2-yl)-4H-1,2,4-triazole-3-carboxamide

The preparation was carried out in a similar manner to Example 92, except that in step 1, 2-bromo-1-ethanol was used in place of iodomethane, and 3-bromo-4-methylphenol was used in place of 2-bromo-3-methylphenol. $^1$H NMR (400 MHz, DMSO-$d_6$) δ 14.55 (s, 1H), 9.85 (s, 1H), 8.41 (d, *J* = 5.1 Hz, 1H), 8.13 (s, 1H), 7.44 - 7.14 (m, 7H), 6.95 (dd, *J* = 8.4, 2.7 Hz, 1H), 6.83 (d, *J* = 2.7 Hz, 1H), 4.85 (t, *J* = 5.5 Hz, 1H), 4.19 (s, 2H), 4.00 (t, *J* = 5.0 Hz, 2H), 3.77 - 3.62 (m, 2H), 2.19 (s, 3H). MS: 430 [M+H]$^+$.

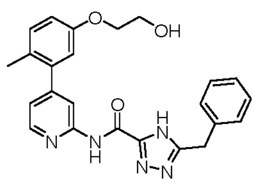 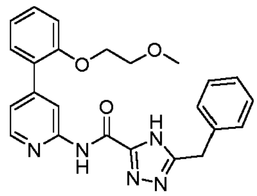

Example 125 Example 126

Example 126: 5-benzyl-N-(4-(2-(2-methoxyethoxy)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, and 2-bromophenol was used in place of 2-bromo-3-methylphenol. $^1$H NMR (600 MHz, DMSO-$d_6$) δ 14.61 (s, 1H), 9.81 (s, 1H), 8.42 - 8.32 (m, 2H), 7.46 - 7.40 (m, 2H), 7.39 (dd, *J* = 5.1, 1.6 Hz, 1H), 7.37 -7.30 (m, 4H), 7.29 - 7.23 (m, 1H), 7.19 (d, *J* = 8.4 Hz, 1H), 7.12 -7.08 (m, 1H), 4.22 - 4.14 (m, 4H), 3.69 - 3.63 (m, 2H), 3.22 (s, 3H). MS: 430 [M+H]$^+$.

Example 127: 5-benzyl-N-(5-(5-(2-methoxyethoxy)-2-methylphenyl)pyridin-3-yl)-4H-1,2,4-triazole-3-carboxamide

The preparation was carried out in a similar manner to Example 92, except that in step 1, 1-bromo-2-methoxyethane was used in place of iodomethane, 3-bromo-4-methylphenol was used in place of 2-bromo-3-methylphenol, and in step 2, 5-bromopyridin-3-amine was used in place of 4-bromopyridin-2-amine. $^1$H NMR (400 MHz, DMSO-$d_6$) δ 14.49 (s, 1H), 10.72 (s, 1H), 9.02 (d, *J* = 2.3 Hz, 1H), 8.31 (d, *J* = 1.9 Hz, 1H), 8.25 - 8.16 (m, 1H), 7.39 - 7.19 (m, 6H), 6.92 (dd, *J* = 8.4, 2.7 Hz, 1H), 6.83 (d, *J* = 2.7 Hz, 1H), 4.17 (s, 2H), 4.13 - 4.05 (m, 2H), 3.69 - 3.61 (m, 2H), 3.30 (s, 3H), 2.18 (s, 3H). MS: 444 [M+H]$^+$.

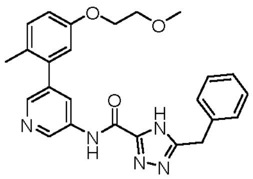 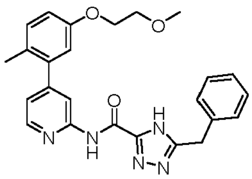

Example 127 Example 128

Example 128: 5-benzyl-N-(4-(5-(2-methoxyethoxy)-2-methylphenyl)pyridin-2-yl)-4H-1,2,4-triazole-3-carboxamide

The preparation was carried out in a similar manner to Example 92, except that in step 1, 1-bromo-2-methoxyethane was used in place of iodomethane, and 3-bromo-4-methylphenol was used in place of 2-bromo-3-methylphenol. $^1$H NMR (400 MHz, DMSO-$d_6$) δ 14.55 (s, 1H), 9.85 (s, 1H), 8.41 (d, *J* = 5.1 Hz, 1H), 8.13 (s, 1H), 7.41 - 7.29 (m, 4H), 7.29 - 7.16 (m, 3H), 6.95 (dd, *J* = 8.4, 2.8 Hz, 1H), 6.83 (d, *J* = 2.8 Hz, 1H), 4.19 (s, 2H), 4.11 (t, *J* = 4.6 Hz, 2H), 3.65 (t, *J* = 4.6 Hz, 2H), 3.30 (s, 3H), 2.19 (s, 3H). MS: 444 [M+H]$^+$.

Example 129: 5-benzyl-N-(5-(2-methoxyethoxy)-2-methyl-[3,4'-bipyridin]-2'-yl)-4H-1,2,4-triazole-3-carboxamide

The preparation was carried out in a similar manner to Example 92, except that in step 1, 1-bromo-2-methoxyethane was used in place of iodomethane, and 5-bromo-6-methylpyridin-3-ol (for the synthetic method refers to Lin, Nan-Homg et al/ U.S., 6437138, 20 Aug 2002) was used in place of 2-bromo-3-methylphenol. $^1$H NMR (400 MHz, DMSO-$d_6$) δ 14.62 (s, 1H), 9.97 (s, 1H), 8.46 (d, *J* = 5.1 Hz, 1H), 8.28 (d, *J* = 2.9 Hz, 1H), 8.16 (s, 1H), 7.42 - 7.20 (m, 7H), 4.28 - 4.11 (m, 4H), 3.66 (s, 2H), 3.30 (s, 3H), 2.39 (s, 3H). MS: 445 [M+H]$^+$.

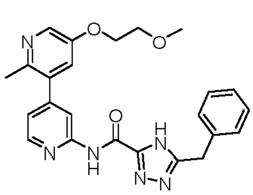 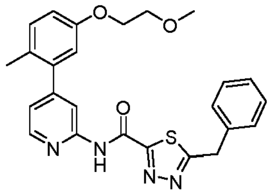

Example 129 Example 130

Example 130: 5-benzyl-N-(4-(5-(2-methoxyethoxy)-2-methylphenyl)pyridin-2-yl)-1,3,4-thiodiazol-2-carboxamide

The preparation was carried out in a similar manner to Example 92, except that in step 1, 1-bromo-2-methoxyethane was used in place of iodomethane, 3-bromo-4-methylphenol was used in place of 2-bromo-3-methylphenol, and in step 3, methyl 5-benzyl-1,3,4-thiodiazol-2-carboxylate was used in place of ethyl 5-benzyl-4H-1,2,4-triazol-3-carboxylate. $^1$H NMR (400 MHz, DMSO-$d_6$) δ 10.84 (s, 1H), 8.47 (dd, *J* = 5.1, 0.7 Hz, 1H), 8.00 (dd, *J* = 1.6, 0.8 Hz, 1H), 7.44 - 7.20 (m, 7H), 6.94 (dd, *J* = 8.4, 2.7 Hz, 1H), 6.83 (d, *J* = 2.8 Hz, 1H), 4.58 (s, 2H), 4.14 - 4.05 (m, 2H), 3.69 - 3.60 (m, 2H), 3.30 (s, 3H), 2.19 (s, 3H). MS: 461 [M+H]$^+$.

Example 131: 5-benzyl-N-(4-(5-(2-ethoxyethoxy)-2-methylphenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, and 3-bromo-4-methylphenol was used in place of 2-bromo-3-methylphenol. $^1$H NMR (400 MHz, DMSO-$d_6$) δ 14.62 (s, 1H), 9.91 (s, 1H), 8.42 (d, *J* = 5.1 Hz, 1H), 8.12 (s, 1H), 7.45 - 7.13 (m, 7H), 6.95 (dd, *J* = 8.4, 2.7 Hz, 1H), 6.84 (d, *J* = 2.7 Hz, 1H), 4.18 (s, 2H), 4.14 - 4.02 (m, 2H), 3.76 - 3.64 (m, 2H), 3.49 (q, *J* = 7.0 Hz, 2H), 2.19 (s, 3H), 1.12 (t, *J* = 7.0 Hz, 3H). MS: 458 [M+H]$^+$.

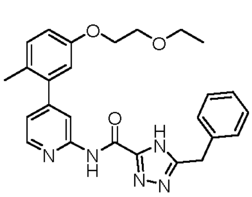

Example 131

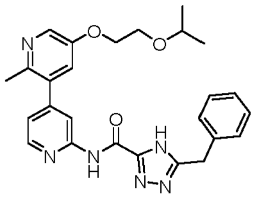

Example 132

Example 132: 5-benzyl-N-(5-(2-isopropoxyethoxy)-2-methyl-[3,4'-dipyridine]-2'-yl)-4H-1,2,4-triazole-3-carboxamide

The preparation was carried out in a similar manner to Example 92, except that in step 1, 1-bromo-2-isopropoxyethane was used in place of iodomethane, and 5-bromo-6-methylpyridin-3-ol (for the synthetic method refers to Lin, Nan-Homg et al/ U.S., 6437138, 20 Aug 2002) was used in place of 2-bromo-3-methylphenol. $^1$H NMR (400 MHz, DMSO-$d_6$) δ 14.64 (s, 1H), 9.98 (s, 1H), 8.46 (d, *J* = 5.1 Hz, 1H), 8.27 (d, *J* = 2.9 Hz, 1H), 8.16 (s, 1H), 7.41 - 7.18 (m, 7H), 4.26 - 4.07 (m, 4H), 3.69 (s, 2H), 3.61 (p, *J* = 6.1 Hz, 1H), 2.39 (s, 3H), 1.09 (d, *J* = 6.0 Hz, 6H). MS: 473 $[M+H]^+$.

Example 133: 5-benzyl-N-(4-(5-(2-isopropoxyethoxy)-2-methylphenyl)pyridin-2-yl)-4H-1,2,4-triazole-3-carboxamide

The preparation was carried out in a similar manner to Example 92, except that in step 1, 1-bromo-2-isopropoxyethane was used in place of iodomethane, and 3-bromo-4-methylphenol was used in place of 2-bromo-3-methylphenol. $^1$H NMR (400 MHz, DMSO-$d_6$) δ 14.62 (s, 1H), 9.90 (s, 1H), 8.42 (dd, *J* = 5.1, 0.8 Hz, 1H), 8.12 (s, 1H), 7.39 - 7.29 (m, 4H), 7.29 - 7.18 (m, 3H), 6.95 (dd, *J* = 8.4, 2.7 Hz, 1H), 6.83 (d, *J* = 2.8 Hz, 1H), 4.17 (s, 2H), 4.12 - 4.01 (m, 2H), 3.74 - 3.65 (m, 2H), 3.61 (p, *J* = 6.1 Hz, 1H), 2.19 (s, 3H), 1.10 (d, *J* = 6.1 Hz, 6H). MS: 472 $[M+H]^+$.

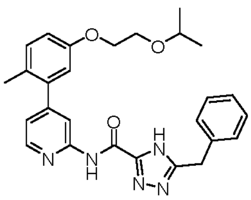

Example 133

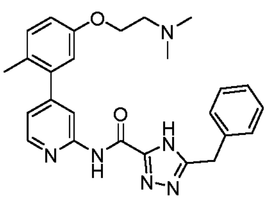

Example 134

Example 134: 5-benzyl-N-(4-(5-(2-(dimethylamino)ethoxy)-2-methylphenyl)pyridin-2-yl)-4H-1,2,4-triazole-3-carboxamide

The preparation was carried out in a similar manner to Example 92, except that in step 1, 2-bromo-N,N-dimethyl-1-ethylamine hydrochloride was used in place of iodomethane, and 3-bromo-4-methylphenol was used in place of 2-bromo-3-methylphenol. $^1$H NMR (400 MHz, DMSO-$d_6$) δ 14.89-14.02 (m, 1H), 9.90 (s, 1H), 8.42 (dd, *J* = 5.1, 0.8 Hz, 1H), 8.15-8.07 (m, 1H), 7.39-7.30 (m, 4H), 7.30-7.17 (m, 3H), 6.95 (dd, *J* = 8.4, 2.7 Hz, 1H), 6.83 (d, *J* = 2.7 Hz, 1H), 4.17 (s, 2H), 4.06 (t, *J* = 5.8 Hz, 2H), 2.62 (t, *J* = 5.8 Hz, 2H), 2.21 (s, 6H), 2.19 (s, 3H). MS: 457 $[M+H]^+$.

Example 135: 5-benzyl-N-(4-(5-(2-(diethylamino)ethoxy)-2-methylphenyl)pyridin-2-yl)-4H-1,2,4-triazole-3-carboxamide

The preparation was carried out in a similar manner to Example 92, except that in step 1, 2-bromo-N,N-dimethyl-1-ethylamine hydrochloride was used in place of iodomethane, and 3-bromo-4-methylphenol was used in place of 2-bromo-3-methylphenol. $^1$H NMR (400 MHz, DMSO-$d_6$) δ 14.20 (s, 1H), 9.91 (s, 1H), 8.42 (d, *J* = 5.1 Hz, 1H), 8.11 (d, *J* = 1.5 Hz, 1H), 7.41-7.29 (m, 4H), 7.29 -7.18 (m, 3H), 6.94 (dd, *J* = 8.4, 2.7 Hz, 1H), 6.83 (d, *J* = 2.7 Hz, 1H), 4.18 (s, 2H), 4.05 (t, *J* = 6.0 Hz, 2H), 2.83 (t, *J* = 6.1 Hz, 2H), 2.60 (q, *J* = 7.1 Hz, 4H), 2.19 (s, 3H), 0.98 (t, *J* = 7.1 Hz, 6H). MS: 485 $[M+H]^+$.

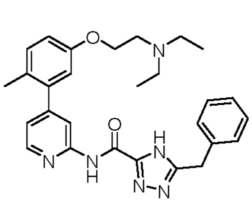

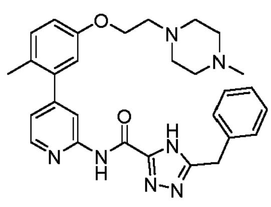

Example 135 Example 136

Example 136: 5-benzyl-N-(4-(2-methyl-5-(2-(4-methylpiperazin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)-4-methylpiperazine dihydrochloride was used in place of iodomethane, and 3-bromo-4-methylphenol was used in place of 2-bromo-3-methylphenol. $^1H$ NMR (400 MHz, DMSO-$d_6$) δ 13.97 (s, 1H), 9.90 (s, 1H), 8.41 (d, $J$ = 5.2 Hz, 1H), 8.12 (s, 1H), 7.40 - 7.15 (m, 7H), 6.94 (dd, $J$ = 8.6, 2.8 Hz, 1H), 6.84 (d, $J$ = 2.8 Hz, 1H), 4.17 (s, 2H), 4.08 (t, $J$ = 5.9 Hz, 2H), 2.67 (t, $J$ = 5.8 Hz, 2H), 2.46 (s, 4H), 2.30 (s, 4H), 2.19 (s, 3H), 2.13 (s, 3H). MS: 512 $[M+H]^+$.

Example 137: N-(4-(5-(2-(1,3-dioxolan-2-yl)ethoxy)-2-methylphenyl)pyridin-2-yl)-5-benzyl-4H-1,2,4-triazole-3-carboxamide

The preparation was carried out in a similar manner to Example 92, except that in step 1, 2-(2-bromoethyl)-1,3-dioxolane was used in place of iodomethane, and 3-bromo-4-methylphenol was used in place of 2-bromo-3-methylphenol. $^1H$ NMR (400 MHz, DMSO-$d_6$) δ 14.60 (s, 1H), 9.91 (s, 1H), 8.42 (d, $J$ = 5.1 Hz, 1H), 8.11 (s, 1H), 7.45 - 7.15 (m, 7H), 6.94 (dd, $J$ = 8.4, 2.7 Hz, 1H), 6.82 (d, $J$ = 2.7 Hz, 1H), 4.98 (t, $J$ = 4.9 Hz, 1H), 4.18 (s, 2H), 4.08 (t, $J$ = 6.6 Hz, 2H), 3.96 - 3.84 (m, 2H), 3.84 - 3.71 (m, 2H), 2.19 (s, 3H), 2.10 - 1.95 (m, 2H). MS: 486 $[M+H]^+$.

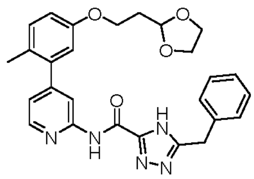

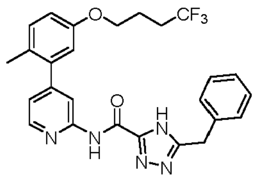

Example 137 Example 138

Example 138: 5-benzyl-N-(4-(2-methyl-5-(4,4,4-trifluorobutoxy)phenyl)pyridin-2-yl)-4H-1,2,4-triazole-3-carboxamide

The preparation was carried out in a similar manner to Example 92, except that in step 1, 4-bromo-1,1,1-trifluorobutane was used in place of iodomethane, and 3-bromo-4-methylphenol was used in place of 2-bromo-3-methylphenol. $^1H$ NMR (400 MHz, DMSO-$d_6$) δ 14.62 (s, 1H), 9.91 (s, 1H), 8.42 (d, $J$ = 5.1 Hz, 1H), 8.12 (d, $J$ = 1.5 Hz, 1H), 7.41 -7.30 (m, 4H), 7.30 - 7.23 (m, 2H), 7.21 (dd, $J$ = 5.1, 1.6 Hz, 1H), 6.95 (dd, $J$ = 8.4, 2.7 Hz, 1H), 6.84 (d, $J$ = 2.7 Hz, 1H), 4.18 (s, 2H), 4.05 (t, $J$ = 6.2 Hz, 2H), 2.47 - 2.31 (m, 2H), 2.19 (s, 3H), 1.99 - 1.85 (m, 2H). MS: 496 $[M+H]^+$.

Example 139: 5-benzyl-N-(4-(5-(3-(dimethylamino)propoxy)-2-methylphenyl)pyridin-2-yl)-4H-1,2,4-triazole-3-carboxamide

The preparation was carried out in a similar manner to Example 92, except that in step 1, 3-bromo-N,N-dimethyl-1-propylamine hydrochloride was used in place of iodomethane, and 3-bromo-4-methylphenol was used in place of 2-bromo-3-methylphenol. $^1H$ NMR (400 MHz, DMSO-$d_6$) δ 13.57 (s, 1H), 9.91 (s, 1H), 8.41 (d, $J$ = 5.1 Hz, 1H), 8.13 (s, 1H), 7.39-7.29 (m, 4H), 7.29-7.14 (m, 3H), 6.92 (dd, $J$ = 8.4, 2.6 Hz, 1H), 6.80 (d, $J$ = 2.7 Hz, 1H), 4.17 (s, 2H), 3.99 (t, $J$ = 6.4 Hz, 2H), 2.36 (t, $J$ = 7.1 Hz, 2H), 2.18 (s, 3H), 2.14 (s, 6H), 1.89-1.77 (m, 2H). MS: 471 $[M+H]^+$.

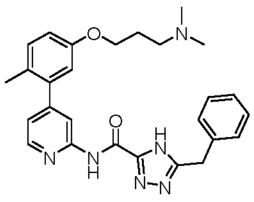
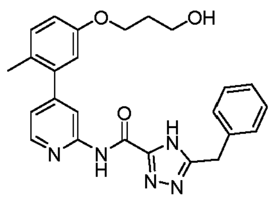

Example 139 Example 140

Example 140: 5-benzyl-N-(4-(5-(3-hydroxylpropoxy)-2-methylphenyl)pyridin-2-yl)-4H-1,2,4-triazole-3-carboxamide

The preparation was carried out in a similar manner to Example 92, except that in step 1, 3-bromo-1-propanol was used in place of iodomethane, and 3-bromo-4-methylphenol was used in place of 2-bromo-3-methylphenol. $^1$H NMR (400 MHz, DMSO-$d_6$) δ 14.80 (s, 1H), 9.90 (s, 1H), 8.41 (d, *J* = 5.1 Hz, 1H), 8.11 (s, 1H), 7.38 -7.30 (m, 4H), 7.26 (dd, *J* = 7.5, 4.8 Hz, 2H), 7.21 (dd, *J* = 5.1, 1.6 Hz, 1H), 6.93 (dd, *J* = 8.4, 2.7 Hz, 1H), 6.81 (d, *J* = 2.7 Hz, 1H), 4.54 (t, *J* = 5.1 Hz, 1H), 4.17 (s, 2H), 4.04 (t, *J* = 6.4 Hz, 2H), 3.57 -3.52 (m, 2H), 2.19 (s, 3H), 1.91 - 1.79 (m, 2H). MS: 444 [M+H]$^+$.

Example 141: 5-benzyl-N-(4-(5-(3-methoxypropoxy)-2-methylphenyl)pyridin-2-yl)-4H-1,2,4-triazole-3-carboxamide

The preparation was carried out in a similar manner to Example 92, except that in step 1, 1-bromo-3-methoxypropane was used in place of iodomethane, and 3-bromo-4-methylphenol was used in place of 2-bromo-3-methylphenol. $^1$H NMR (400 MHz, DMSO-$d_6$) δ 14.40 (s, 1H), 9.90 (s, 1H), 8.41 (dd, *J* = 5.2, 2.1 Hz, 1H), 8.11 (d, *J* = 1.6 Hz, 1H), 7.45-7.15 (m, 7H), 6.94 (dd, *J* = 8.4, 2.7 Hz, 1H), 6.81 (d, *J* = 2.6 Hz, 1H), 4.17 (s, 2H), 4.03 (t, *J* = 6.3 Hz, 2H), 3.48-3.44 (m, 2H), 3.23 (d, *J* = 2.1 Hz, 3H), 2.19 (d, *J* = 2.1 Hz, 3H), 2.02-1.85 (m, 2H). MS: 458 [M+H]$^+$.

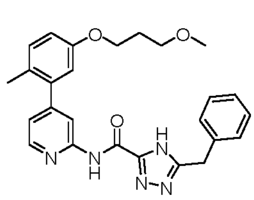
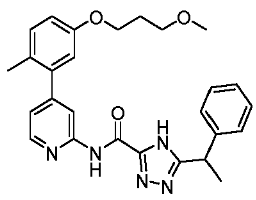

Example 141 Example 142

Example 142: N-(4-(5-(3-methoxypropoxy)-2-methylphenyl)pyridin-2-yl)-5-(1-phenylethyl)-4H-1,2,4-triazole-3-carboxamide

The preparation was carried out in a similar manner to Example 92, except that in step 1, 1-bromo-3-methoxypropane was used in place of iodomethane, 3-bromo-4-methylphenol was used in place of 2-bromo-3-methylphenol, and in step 3, ethyl 5-(1-phenylethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.54 (s, 1H), 9.91 (s, 1H), 8.42 (d, *J* = 5.1 Hz, 1H), 8.12 (s, 1H), 7.44 - 7.28 (m, 4H), 7.29 - 7.17 (m, 3H), 6.94 (dd, *J* = 8.4, 2.7 Hz, 1H), 6.82 (d, *J* = 2.7 Hz, 1H), 4.41 (q, *J* = 7.2 Hz, 1H), 4.03 (t, *J* = 6.4 Hz, 2H), 3.46 (t, *J* = 6.3 Hz, 2H), 3.24 (s, 3H), 2.19 (s, 3H), 1.98 - 1.87 (m, 2H), 1.67 (d, *J* = 7.2 Hz, 3H). MS: 472 [M+H]$^+$.

Example 143: N-(4-(5-(3-methoxypropoxy)-2-methylphenyl)pyridin-2-yl)-5-(1-phenylethyl)-1,3,4-oxadiazol-2-carboxamide

The preparation was carried out in a similar manner to Example 92, except that in step 1, 1-bromo-3-methoxypropane was used in place of iodomethane, and 3-bromo-4-methylphenol was used in place of 2-bromo-3-methylphenol, in step 3, ethyl 5-(1-phenylethyl)-1,3,4-oxadiazol-2-carboxylate was used in place of ethyl 5-benzyl-4H-1,2,4-triazol-3-carboxylate. $^1$H NMR (400 MHz, DMSO-$d_6$) δ 11.29 (s, 1H), 8.46 (d, *J* = 5.1 Hz, 1H), 7.96 (s, 1H), 7.43 - 7.20 (m, 7H), 6.93 (dd, *J* = 8.4, 2.7 Hz, 1H), 6.80 (d, *J* = 2.7 Hz, 1H), 4.63 (q, *J* = 7.2 Hz, 1H), 4.02 (t, *J* = 6.4 Hz, 2H), 3.45 (t, *J* = 6.3 Hz, 2H), 3.34 (s, 3H), 2.18 (s, 3H), 1.98 - 1.87 (m, 2H), 1.71 (d, *J* = 7.2 Hz, 3H). MS: 473 [M+H]$^+$.

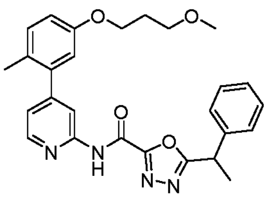

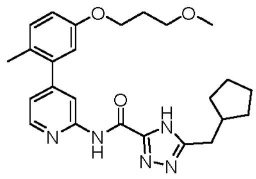

Example 143 Example 144

Example 144: 5-(cyclopentylmethyl)-N-(4-(5-(3-methoxypropoxy)-2-methylphenyl)pyridin-2-yl)-4H-1,2,4-triazole-3-carboxamide

The preparation was carried out in a similar manner to Example 92, except that in step 1, 1-bromo-3-methoxypropane was used in place of iodomethane, 3-bromo-4-methylphenol was used in place of 2-bromo-3-methylphenol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.38 (s, 1H), 9.84 (s, 1H), 8.42 (d, *J* = 5.1 Hz, 1H), 8.14 (s, 1H), 7.33 - 7.14 (m, 2H), 6.94 (dd, *J* = 8.4, 2.7 Hz, 1H), 6.82 (d, *J* = 2.7 Hz, 1H), 4.03 (t, *J* = 6.4 Hz, 2H), 3.46 (t, *J* = 6.3 Hz, 2H), 3.24 (s, 3H), 2.84-2.74 (m, 2H), 2.36-2.22 (m, 1H), 2.20 (s, 3H), 2.00-1.88 (m, 2H), 1.79-1.66 (m, 2H), 1.66-1.56 (m, 2H), 1.56-1.45 (m, 2H), 1.27-1.18 (m, 2H). MS: 450 $[M+H]^+$.

Example 145: 5-(cyclohexylmethyl)-N-(4-(5-(3-methoxypropoxy)-2-methylphenyl)pyridin-2-yl)-4H-1,2,4-triazole-3-carboxamide

The preparation was carried out in a similar manner to Example 92, except that in step 1, 1-bromo-3-methoxypropane was used in place of iodomethane, 3-bromo-4-methylphenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 9.97 - 9.76 (m, 1H), 8.42 (d, *J* = 5.1 Hz, 1H), 8.13 (s, 1H), 7.34 - 7.12 (m, 2H), 6.94 (dd, *J* = 8.4, 2.7 Hz, 1H), 6.82 (d, *J* = 2.8 Hz, 1H), 4.03 (t, *J* = 6.4 Hz, 2H), 3.46 (t, *J* = 6.3 Hz, 2H), 3.24 (s, 3H), 2.67 (d, *J* = 7.0 Hz, 2H), 2.19 (s, 3H), 2.01 - 1.86 (m, 2H), 1.84 - 1.53 (m, 6H), 1.30 - 1.06 (m, 3H), 1.06 - 0.87 (m, 2H). MS: 464 $[M+H]^+$.

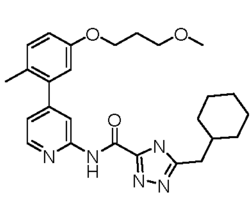

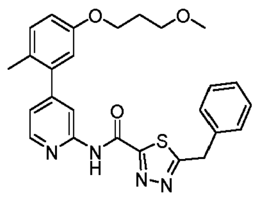

Example 145 Example 146

Example 146: 5-benzyl-N-(4-(5-(3-methoxypropoxy)-2-methylphenyl)pyridin-2-yl)-1,3,4-thiodiazol-2-carboxamide

The preparation was carried out in a similar manner to Example 92, except that in step 1, 1-bromo-3-methoxypropane was used in place of iodomethane, 3-bromo-4-methylphenol was used in place of 2-bromo-3-methylphenol, and in step 3, methyl 5-benzyl-1,3,4-thiodiazol-2-carboxylate was used in place of ethyl 5-benzyl-4H-1,2,4-triazol-3-carboxylate. $^1$H NMR (400 MHz, DMSO-$d_6$) δ 10.84 (s, 1H), 8.47 (dd, *J* = 5.1, 0.8 Hz, 1H), 7.99 (dd, *J* = 1.6, 0.8 Hz, 1H), 7.46 - 7.17 (m, 7H), 6.93 (dd, *J* = 8.4, 2.7 Hz, 1H), 6.81 (d, *J* = 2.7 Hz, 1H), 4.58 (s, 2H), 4.02 (t, *J* = 6.4 Hz, 2H), 3.46 (t, *J* = 6.3 Hz, 2H), 3.23 (s, 3H), 2.18 (s, 3H), 2.00 - 1.87 (m, 2H). MS: 475 $[M+H]^+$.

Example 147: 5-benzyl-N-(4-(5-(3-ethoxypropoxy)-2-methylphenyl)pyridin-2-yl)-4H-1,2,4-triazole-3-carboxamide

The preparation was carried out in a similar manner to Example 92, except that in step 1, 1-bromo-3-ethoxypropane was used in place of iodomethane, and 3-bromo-4-methylphenol was used in place of 2-bromo-3-methylphenol. $^1$H NMR (400 MHz, DMSO-$d_6$) δ 14.63 (s, 1H), 9.92 (s, 1H), 8.41 (d, *J* = 5.1 Hz, 1H), 8.11 (s, 1H), 7.46 - 7.29 (m, 4H), 7.29 - 7.14 (m, 3H), 6.94 (dd, *J* = 8.4, 2.7 Hz, 1H), 6.82 (d, *J* = 2.7 Hz, 1H), 4.18 (s, 2H), 4.03 (t, *J* = 6.4 Hz, 2H), 3.49 (t, *J* = 6.3 Hz, 2H), 3.41 (q, *J* = 7.0 Hz, 2H), 2.19 (s, 3H), 2.00 - 1.86 (m, 2H), 1.08 (t, *J* = 7.0 Hz, 3H). MS: 472 $[M+H]^+$.

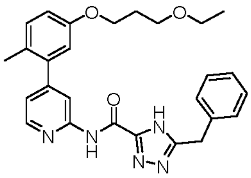
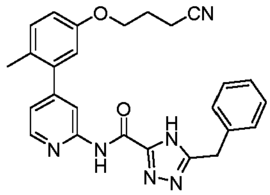

Example 147 Example 148

Example 148: 5-benzyl-N-(4-(5-(3-cyanopropoxy)-2-methylphenyl)pyridin-2-yl)-4H-1,2,4-triazole-3-carboxamide

The preparation was carried out in a similar manner to Example 92, except that in step 1, 4-bromo-1-butanenitrile was used in place of iodomethane, and 3-bromo-4-methylphenol was used in place of 2-bromo-3-methylphenol. $^1$H NMR (400 MHz, DMSO-$d_6$) δ 14.59 (s, 1H), 9.91 (s, 1H), 8.42 (d, *J* = 5.1 Hz, 1H), 8.12 (s, 1H), 7.45 - 7.14 (m, 7H), 6.96 (dd, *J* = 8.4, 2.7 Hz, 1H), 6.85 (d, *J* = 2.7 Hz, 1H), 4.18 (s, 2H), 4.05 (t, *J* = 6.0 Hz, 2H), 2.65 (t, *J* = 7.1 Hz, 2H), 2.19 (s, 3H), 2.06 - 1.96 (m, 2H). MS: 453 $[M+H]^+$.

Example 149: 5-benzyl-N-(4-(2-methyl-5-(pent-4-en-1-yloxy)phenyl)pyridin-2-yl)-4H-1,2,4-triazole-3-carboxamide

The preparation was carried out in a similar manner to Example 92, except that in step 1, 5-bromo-1-pentene was used in place of iodomethane, and 3-bromo-4-methylphenol was used in place of 2-bromo-3-methylphenol. $^1$H NMR (400 MHz, DMSO-$d_6$) δ 14.58 (s, 1H), 9.97 (s, 1H), 8.42 (d, *J* = 5.1 Hz, 1H), 8.11 (s, 1H), 7.44 - 7.16 (m, 7H), 6.94 (dd, *J* = 8.4, 2.7 Hz, 1H), 6.81 (d, *J* = 2.7 Hz, 1H), 5.94 - 5.77 (m, 1H), 5.62 - 5.40 (m, 1H), 5.12 - 4.91 (m, 1H), 4.18 (s, 2H), 4.06 - 3.91 (m, 2H), 2.44 - 2.32 (m, 1H), 2.19 (s, 3H), 1.87 - 1.72 (m, 1H), 1.69 - 1.55 (m, 2H). MS: 454 $[M+H]^+$.

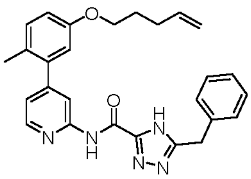
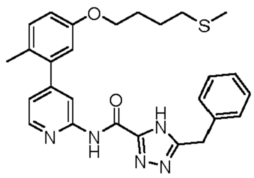

Example 149 Example 150

Example 150: 5-benzyl-N-(4-(2-methyl-5-(4-(methylthio)butoxy)phenyl)pyridin-2-yl)-4H-1,2,4-triazole-3-carboxamide

The preparation was carried out in a similar manner to Example 92, except that in step 1, (4-bromobutyl)(methyl)sulfane was used in place of iodomethane, and 3-bromo-4-methylphenol was used in place of 2-bromo-3-methylphenol. $^1$H NMR (400 MHz, DMSO-$d_6$) δ 14.62 (s, 1H), 9.91 (s, 1H), 8.41 (d, *J* = 5.1 Hz, 1H), 8.11 (s, 1H), 7.41 - 7.29 (m, 4H), 7.29 - 7.23 (m, 2H), 7.21 (dd, *J* = 5.1, 1.6 Hz, 1H), 6.94 (dd, *J* = 8.4, 2.7 Hz, 1H), 6.82 (d, *J* = 2.7 Hz, 1H), 4.17 (s, 2H), 4.00 (t, *J* = 6.4 Hz, 2H), 2.58 - 2.52 (m, 2H), 2.19 (s, 3H), 2.03 (s, 3H), 1.85 - 1.73 (m, 2H), 1.73 - 1.61 (m, 2H). MS: 488 $[M+H]^+$.

Example 151: 5-benzyl-N-(4-(5-(4-hydroxybutoxy)-2-methylphenyl)pyridin-2-yl)-4H-1,2,4-triazole-3-carboxamide

The preparation was carried out in a similar manner to Example 92, except that in step 1, 4-bromo-1-butanol was used in place of iodomethane, and 3-bromo-4-methylphenol was used in place of 2-bromo-3-methylphenol. $^1$H NMR (400 MHz, DMSO-$d_6$) δ 14.60 (s, 1H), 9.90 (s, 1H), 8.41 (d, *J* = 5.1 Hz, 1H), 8.11 (s, 1H), 7.39 - 7.29 (m, 4H), 7.29 - 7.22 (m, 2H), 7.21 (dd, *J* = 5.2, 1.6 Hz, 1H), 6.93 (dd, *J* = 8.4, 2.8 Hz, 1H), 6.81 (d, *J* = 2.7 Hz, 1H), 4.51 - 4.35 (m, 1H), 4.17 (s, 2H), 3.98 (t, *J* = 6.5 Hz, 2H), 3.44 (t, *J* = 6.4 Hz, 2H), 2.19 (s, 3H), 1.80 - 1.67 (m, 2H), 1.62 - 1.49 (m, 2H). MS: 458 $[M+H]^+$.

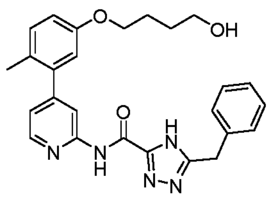
Example 151

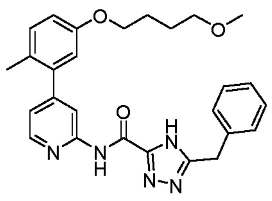
Example 152

Example 152: 5-benzyl-N-(4-(5-(4-methoxybutoxy)-2-methylphenyl)pyridin-2-yl)-4H-1,2,4-triazole-3-carboxamide

The preparation was carried out in a similar manner to Example 92, except that in step 1, 1-bromo-4-methoxybutane was used in place of iodomethane, and 3-bromo-4-methylphenol was used in place of 2-bromo-3-methylphenol. $^1$H NMR (400 MHz, DMSO-$d_6$) δ 14.62 (s, 1H), 9.91 (s, 1H), 8.47 - 8.34 (m, 1H), 8.18 - 8.03 (m, 1H), 7.43 - 7.13 (m, 7H), 6.93 (dd, *J* = 8.4, 2.7 Hz, 1H), 6.81 (d, *J* = 2.7 Hz, 1H), 4.17 (s, 2H), 3.98 (t, *J* = 6.4 Hz, 2H), 3.35 (t, *J* = 6.3 Hz, 2H), 3.22 (s, 3H), 2.19 (s, 3H), 1.81 - 1.68 (m, 2H), 1.68 - 1.56 (m, 2H). MS: 472 $[M+H]^+$.

Example 153: 5-benzyl-N-(4-(5-((5-hydroxylpentyl)oxy)-2-methylphenyl)pyridin-2-yl)-4H-1,2,4-triazole-3-carboxamide

The preparation was carried out in a similar manner to Example 92, except that in step 1, 5-bromo-1-pentanol was used in place of iodomethane, and 3-bromo-4-methylphenol was used in place of 2-bromo-3-methylphenol. $^1$H NMR (400 MHz, DMSO-$d_6$) δ 14.61 (s, 1H), 9.91 (s, 1H), 8.41 (d, *J* = 5.1 Hz, 1H), 8.11 (s, 1H), 7.42-7.30 (m, 4H), 7.30-7.13 (m, 3H), 6.93 (dd, *J* = 8.4, 2.7 Hz, 1H), 6.81 (d, *J* = 2.7 Hz, 1H), 4.45-4.31 (m, 1H), 4.18 (s, 2H), 3.97 (t, *J* = 6.5 Hz, 2H), 3.46-3.36 (m, 2H), 2.19 (s, 3H), 1.70 (t, *J* = 6.9 Hz, 2H), 1.54-1.36 (m, 4H). MS: 472 $[M+H]^+$.

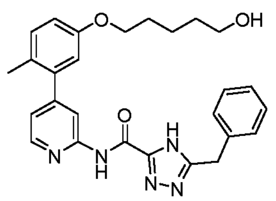
Example 153

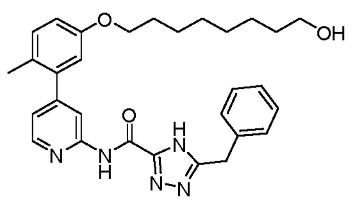
Example 154

Example 154: 5-benzyl-N-(4-(5-((8-hydroxyoctyl)oxy)-2-methylphenyl)pyridin-2-yl)-4H-1,2,4-triazole-3-carboxamide

The preparation was carried out in a similar manner to Example 92, except that in step 1, 8-bromo-1-octanol was used in place of iodomethane, and 3-bromo-4-methylphenol was used in place of 2-bromo-3-methylphenol. $^1$H NMR (400 MHz, DMSO-$d_6$) δ 14.64 (s, 1H), 9.92 (s, 1H), 8.41 (d, *J* = 5.1 Hz, 1H), 8.11 (d, *J* = 1.5 Hz, 1H), 7.42 - 7.11 (m, 7H), 6.92 (dd, *J* = 8.4, 2.7 Hz, 1H), 6.80 (d, *J* = 2.7 Hz, 1H), 4.46 - 4.24 (m, 1H), 4.17 (s, 2H), 3.96 (t, *J* = 6.5 Hz, 2H), 3.37 - 3.34 (m, 2H), 2.18 (s, 3H), 1.78 - 1.60 (m, 2H), 1.50 - 1.34 (m, 4H), 1.34 - 1.13 (m, 6H). MS: 514 $[M+H]^+$.

Example 155: 5-benzyl-N-(4-(5-cyclopropoxy-2-methylphenyl)pyridin-2-yl)-4H-1,2,4-triazole-3-carboxamide

The preparation was carried out in a similar manner to Example 92, except that in step 1, bromocyclopropane was used in place of iodomethane, and 3-bromo-4-methylphenol was used in place of 2-bromo-3-methylphenol. $^1$H NMR (400 MHz, DMSO-$d_6$) δ 14.54 (s, 1H), 9.85 (s, 1H), 8.42 (d, *J* = 5.2 Hz, 1H), 8.13 (s, 1H), 7.39 - 7.30 (m, 4H), 7.30 - 7.23 (m, 2H), 7.23 - 7.18 (m, 1H), 7.05 (dd, *J* = 8.4, 2.7 Hz, 1H), 6.93 (d, *J* = 2.7 Hz, 1H), 4.19 (s, 2H), 3.91 - 3.82 (m, 1H), 2.20 (s, 3H), 0.83 - 0.71 (m, 2H), 0.71 - 0.60 (m, 2H). MS: 426 $[M+H]^+$.

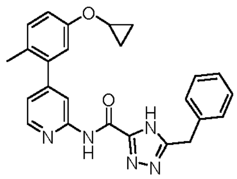
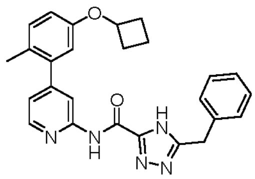

Example 155 Example 156

Example 156: 5-benzyl-N-(4-(5-cyclobutoxy-2-methylphenyl)pyridin-2-yl)-4H-1,2,4-triazole-3-carboxamide

The preparation was carried out in a similar manner to Example 92, except that in step 1, bromocyclobutane was used in place of iodomethane, and 3-bromo-4-methylphenol was used in place of 2-bromo-3-methylphenol. $^1$H NMR (400 MHz, DMSO-$d_6$) δ 14.58 (s, 1H), 9.89 (s, 1H), 8.41 (d, $J$ = 5.1 Hz, 1H), 8.11 (s, 1H), 7.40 - 7.29 (m, 4H), 7.29 - 7.15 (m, 3H), 6.84 (dd, $J$ = 8.4, 2.7 Hz, 1H), 6.72 (d, $J$ = 2.7 Hz, 1H), 4.77 - 4.64 (m, 1H), 4.18 (s, 2H), 2.46 - 2.35 (m, 2H), 2.18 (s, 3H), 2.11-1.94 (m, 2H), 1.83-1.70 (m, 1H), 1.70-1.54 (m, 1H). MS: 440 [M+H]$^+$.

Example 157: N-(4-(5-(4-amino-4-oxobutoxy)-2-methylphenyl)pyridin-2-yl)-5-benzyl-4H-1,2,4-triazole-3-carboxamide

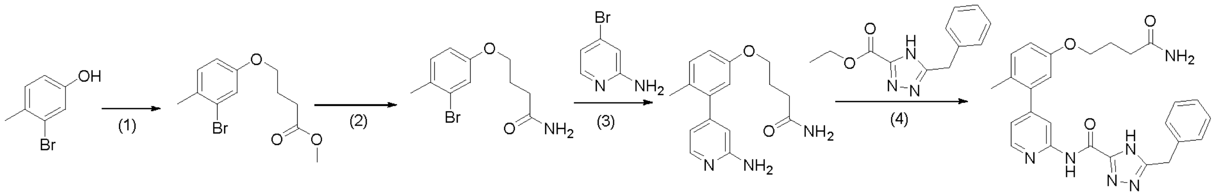


Step 1: Synthesis of methyl 4-(3-bromo-4-methylphenoxy)butanoate

The operation is the same as step 1 of Example 92, but methyl 4-bromobutanoate was used in place of iodomethane in step 1, and 3-bromo-4-methylphenol was used in place of 2-bromo-3-methylphenol in step 1.

Step 2: Synthesis of 4-(3-bromo-4-methylphenoxy)butanamide

Methyl 4-(3-bromo-4-methylphenoxy)butanoate (287mg, 1mmol) was dissolved in 7M ammonia in methanol, and reacted at 80°C for 36 hours in sealed tube. The reaction solution was cooled, evaporated to dryness, and used directly in the next step.

Step 3 to Step 4: The preparation was carried out in a similar manner to Example 33, except that in step 1, 4-(3-bromo-4-methylphenoxy)butanamide was used in place of 2-bromo-4-fluoro-1-methylbenzene. $^1$H NMR (400 MHz, DMSO-$d_6$) δ 14.63 (s, 1H), 9.91 (s, 1H), 8.42 (d, $J$ = 5.1 Hz, 1H), 8.11 (s, 1H), 7.41-7.30 (m, 5H), 7.26 (dd, $J$ = 7.6, 5.2 Hz, 2H), 7.21 (dd, $J$ = 5.1, 1.6 Hz, 1H), 6.93 (dd, $J$ = 8.4, 2.7 Hz, 1H), 6.88-6.67 (m, 2H), 4.18 (s, 2H), 3.97 (t, $J$ = 6.4 Hz, 2H), 2.28-2.12 (m, 5H), 1.97-1.83 (m, 2H). MS: 471 [M+H]$^+$.

Example 158: 5-benzyl-N-(4-(5-carbamoyl-2-methylphenyl)pyridin-2-yl)-4H-1,2,4-triazole-3-carboxamide

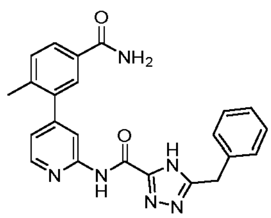

The operation is the same as steps 2 to 4 of Example 157, but methyl 3-bromo-4-methylbenzoate was used

in place of methyl 4-(3-bromo-4-methylphenoxy)butanoate in step 2. $^1H$ NMR (400 MHz, DMSO-$d_6$) δ 14.39 (s, 1H), 9.95 (s, 1H), 8.45 (d, *J* = 5.1 Hz, 1H), 8.15 (d, *J* = 5.3 Hz, 1H), 8.04 (d, *J* = 5.5 Hz, 1H), 7.86 (d, *J* = 7.5 Hz, 1H), 7.81 (d, *J* = 5.4 Hz, 1H), 7.51 - 7.42 (m, 1H), 7.42 - 7.30 (m, 5H), 7.30 - 7.18 (m, 2H), 4.18 (s, 2H), 2.33 (s, 3H). MS: 413 $[M+H]^+$.

Example 159: 5-benzyl-N-(4-(5-((1-hydroxycyclopropyl)methoxy)-2-methylphenyl)pyridin-2-yl)-4H-1,2,4-triazole-3 -carboxamide

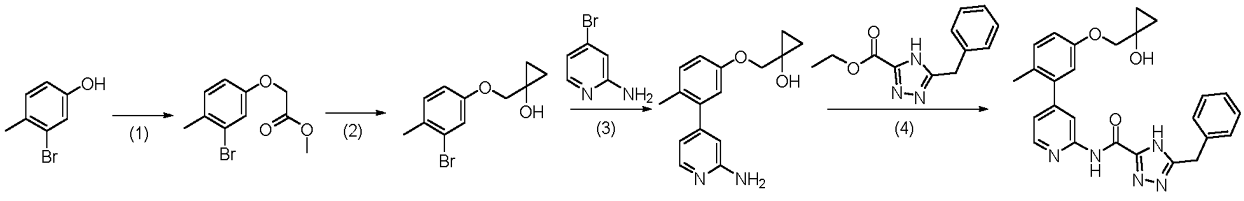


Step 1: Synthesis of methyl 2-(3-bromo-4-methylphenoxy)acetate

The operation is the same as step 1 of Example 92, but methyl bromoacetate was used in place of iodomethane in step 1, and 3-bromo-4-methylphenol was used in place of 2-bromo-3-methylphenol in step 1.

Step 2: Synthesis of 1-((3-bromo-4-methylphenoxy)methyl)cyclopropane-1-ol

Methyl 2-(3-bromo-4-methylphenoxy)acetate (259mg, 1mmol) and tetraisopropyl titanate (57mg, 0.2mmol) were dissolved in anhydrous tetrahydrofuran, and ethyl Grignard reagent (1M in THF, 2.5mL, 2.5mmol) slowly added at 0°C under argon protection. Then the mixture was slowly warmed to 25°C after the addition, and reacted for 4 hours. The reaction solution is quenched with saturated solution of ammonium chloride, extracted with ethyl acetate, washed with saturated brine, dried over anhydrous sodium sulfate, filtered and evaporated to dryness, and used directly in the next step.

Step 3 to Step 4: The preparation was carried out in a similar manner to Example 33, except that in step 1, 1-((3-bromo-4-methylphenoxy)methyl)cyclopropan-1-ol was used in place of 2-bromo-4-fluoro-1-methylbenzene. $^1H$ NMR (400 MHz, DMSO-$d_6$) δ 14.54 (s, 1H), 9.90 (s, 1H), 8.42 (d, *J* = 5.1 Hz, 1H), 8.12 (s, 1H), 7.41 - 7.30 (m, 4H), 7.30 - 7.14 (m, 3H), 6.95 (dd, *J* = 8.4, 2.7 Hz, 1H), 6.84 (d, *J* = 2.8 Hz, 1H), 5.59 (s, 1H), 4.18 (s, 2H), 3.97 (s, 2H), 2.19 (s, 3H), 0.73 - 0.63 (m, 2H), 0.63 - 0.53 (m, 2H). MS: 456 $[M+H]^+$.

Example 160: 5-benzyl-N-(4-(5-((4-hydroxy-4-methylpentyl)oxy)-2-methylphenyl)pyridin-2-yl)-4H-1,2,4-triazole-3-carboxamide

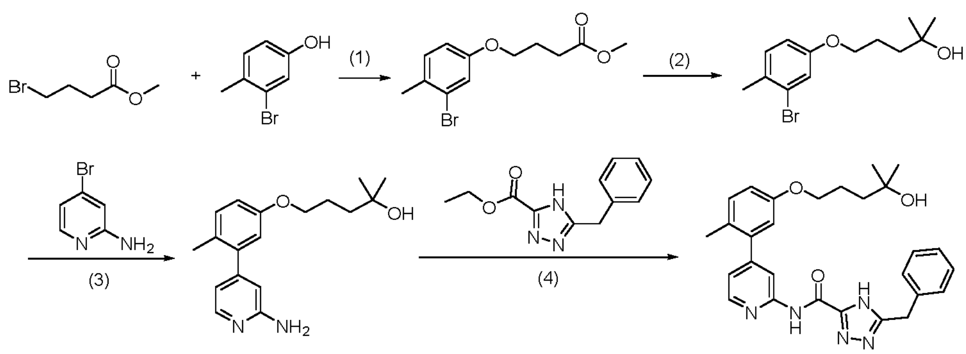


Step 1: Synthesis of methyl 4-(3-bromo-4-methylphenoxy)butanoate

The operation is the same as step 4 of Example 55, but 3-bromo-4-methylphenol was used in place of 3-bromo-N,4-dimethylaniline in the fourth step, and methyl 4-bromobutanoate was used in place of 1-bromo-2-methoxyethane

in the fourth step.

Step 2: Synthesis of 5-(3-bromo-4-methylphenoxy)-2-methylpentan-2-ol

Methyl 4-(3-bromo-4-methylphenoxy)butanoate (287mg, 1mmol) was dissolved in anhydrous tetrahydrofuran (3mL), to which methylmagnesium bromide (3M, 1mL, 3mmol) was added dropwise under argon protection at 0°C, and reacted at 25°C for 2 hours. The reaction was quenched with saturated aqueous solution of ammonium chloride, extracted with dichloromethane, dried over anhydrous sodium sulfate, filtered and evaporated to dryness, and subjected to column chromatography to afford 260mg of product. MS: 287 $[M+H]^+$.

Step 3 to Step 4: The preparation was carried out in a similar manner to Example 33, except that in step 1, 5-(3-bromo-4-methylphenoxy)-2-methylpentan-2-ol was used in place of 2-bromo-4-fluoro-1-methylbenzene. $^1$H NMR (400 MHz, DMSO-$d_6$) δ 14.57 (s, 1H), 9.88 (s, 1H), 8.42 (d, *J* = 5.1 Hz, 1H), 8.11 (s, 1H), 7.41 - 7.30 (m, 4H), 7.30 - 7.16 (m, 3H), 6.93 (dd, *J* = 8.4, 2.7 Hz, 1H), 6.80 (d, *J* = 2.7 Hz, 1H), 4.26 - 4.10 (m, 3H), 3.96 (t, *J* = 6.6 Hz, 2H), 2.19 (s, 3H), 1.82 - 1.68 (m, 2H), 1.54 - 1.40 (m, 2H), 1.09 (s, 6H). MS: 486 $[M+H]^+$.

Example 161: 5-benzyl-N-(4-(4-(2-hydroxypropan-2-yl)-2-methylphenyl)pyridin-2-yl)-4H-1,2,4-triazole-3-carboxamide

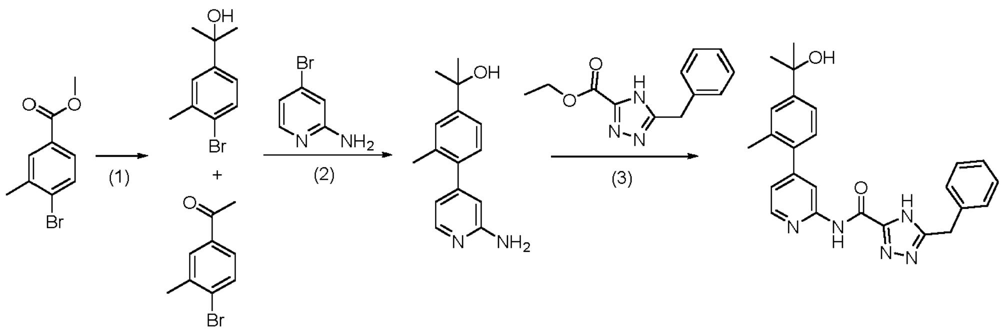


The operation is the same as steps 2 to 4 of Example 160, but methyl 4-bromo-3-methylbenzoate was used in place of methyl 4-(3-bromo-4-methylphenoxy)butanoate in step 2 of Example 160 to carry out the reaction. In this step of reaction, two products of 2-(3-bromo-4-methylphenyl)propan-2-ol and 1-(3-bromo-4-methylphenyl)ethan-1-one were obtained, and 2-(3-bromo-4-methylphenyl)propan-2-ol was used for the subsequent reactions in the present Example. $^1$H NMR (400 MHz, DMSO-$d_6$) δ 14.58 (s, 1H), 9.87 (s, 1H), 8.41 (d, *J* = 5.1 Hz, 1H), 8.12 (s, 1H), 7.51 - 7.14 (m, 9H), 5.07 (s, 1H), 4.18 (s, 2H), 2.29 (s, 3H), 1.45 (s, 6H). MS: 428 $[M+H]^+$.

Example 162: N-(4-(4-acetyl-2-methylphenyl)pyridin-2-yl)-5-benzyl-4H-1,2,4-triazole-3-carboxamide

The operation is the same as Example 161, but the product 1-(3-bromo-4-methylphenyl)ethan-1-one obtained in the first step was used for the subsequent reactions. $^1$H NMR (400 MHz, DMSO-$d_6$) δ 14.59 (s, 1H), 9.95 (s, 1H), 8.46 (d, *J* = 5.1 Hz, 1H), 8.14 (s, 1H), 7.99 - 7.93 (m, 1H), 7.93 - 7.84 (m, 1H), 7.43 (d, *J* = 8.0 Hz, 1H), 7.38 - 7.29 (m, 4H), 7.29 - 7.20 (m, 2H), 4.18 (s, 2H), 2.62 (s, 3H), 2.35 (s, 3H). MS: 412 $[M+H]^+$.

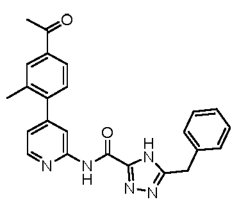

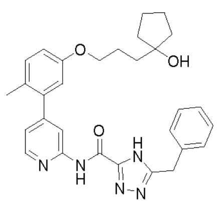

Example 162 Example 163

Example 163: 5-benzyl-N-(4-(5-(3-(1-hydroxycyclopentyl)propoxy)-2-methylphenyl)pyridin-2-yl)-4H-1,2,4-triazole-3-carboxamide

The preparation was carried out in a similar manner to Example 160, except that in step 2, (butane-1,4-diyl)dimagnesium dibromide was used in place of methylmagnesium bromide. $^1$H NMR (400 MHz, DMSO-$d_6$) δ 14.39 (s, 1H), 9.92 (s, 1H), 8.41 (d, *J* = 5.2 Hz, 1H), 8.11 (s, 1H), 7.47 - 7.29 (m, 4H), 7.29 - 7.13 (m, 3H), 6.93 (dd, *J* = 8.4, 2.7 Hz, 1H), 6.80 (d, *J* = 2.7 Hz, 1H), 4.18 (s, 2H), 4.05 (s, 1H), 3.98 (t, *J* = 6.6 Hz, 2H), 2.19 (s, 3H), 1.87 - 1.33 (m, 12H). MS: 512 [M+H]$^+$.

Example 164: 5-benzyl-N-(4-(5-(3-(1-hydroxycyclohexyl)propoxy)-2-methylphenyl)pyridin-2-yl)-4H-1,2,4-triazole-3-carboxamide

The preparation was carried out in a similar manner to Example 160, except that in step 2, (pentane-1,5-diyl)dimagnesium dibromide was used in place of methylmagnesium bromide. $^1$H NMR (400 MHz, DMSO-$d_6$) δ 14.61 (s, 1H), 9.91 (s, 1H), 8.41 (d, *J* = 5.1 Hz, 1H), 8.11 (s, 1H), 7.49 - 7.29 (m, 4H), 7.29 - 7.12 (m, 3H), 6.92 (dd, *J* = 8.4, 2.7 Hz, 1H), 6.80 (d, *J* = 2.7 Hz, 1H), 4.17 (s, 2H), 4.01 - 3.86 (m, 3H), 2.19 (s, 3H), 1.75 (t, *J*= 8.3 Hz, 2H), 1.63 - 1.49 (m, 2H), 1.49 - 1.38 (m, 5H), 1.38 - 1.24 (m, 5H). MS: 526 [M+H]$^+$.

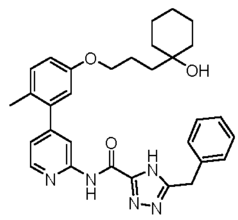

Example 164

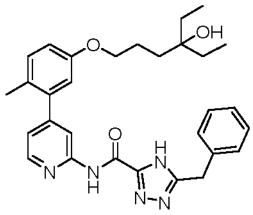

Example 165

Example 165: 5-benzyl-N-(4-(5-((4-ethyl-4-(hydroxyhexyl)oxy)-2-methylphenyl)pyridin-2-yl)-4H-1,2,4-triazole-3-carboxamide

The preparation was carried out in a similar manner to Example 160, except that in step 2, ethylmagnesium bromide was used in place of methylmagnesium bromide. $^1$H NMR (400 MHz, DMSO-$d_6$) δ 14.52 (s, 1H), 9.90 (s, 1H), 8.41 (d, *J* = 5.0 Hz, 1H), 8.11 (s, 1H), 7.40 - 7.28 (m, 4H), 7.28 - 7.22 (m, 2H), 7.21 (dd, *J* = 5.1, 1.5 Hz, 1H), 6.92 (dd, *J* = 8.4, 2.7 Hz, 1H), 6.80 (d, *J* = 2.7 Hz, 1H), 4.17 (s, 2H), 3.96 (t, *J* = 6.5 Hz, 2H), 3.88 (s, 1H), 2.19 (s, 3H), 1.74 - 1.63 (m, 2H), 1.48 - 1.38 (m, 2H), 1.34 (q, *J* = 7.4 Hz, 4H), 0.77 (t, *J* = 7.4 Hz, 6H). MS: 514 [M+H]$^+$.

Example 166: 5-(cyclopentylmethyl)-N-(4-(5-((4-ethyl-4-hydroxyhexyl)oxy)-2-methylphenyl)pyridin-2-yl)-4H-1,2,4-triazole-3-carboxamide

The preparation was carried out in a similar manner to Example 160, except that in step 2, ethylmagnesium bromide was used in place of methylmagnesium bromide, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.38 (s, 1H), 9.85 (s, 1H), 8.42 (d, *J* = 5.1 Hz, 1H), 8.13 (s, 1H), 7.32 - 7.12 (m, 2H), 6.93 (dd, *J* = 8.4, 2.7 Hz, 1H), 6.80 (d, *J* = 2.7 Hz, 1H), 3.96 (t, *J* = 6.5 Hz, 2H), 3.88 (s, 1H), 2.78 (d, *J* = 7.5 Hz, 2H), 2.36 - 2.21 (m, 1H), 2.19 (s, 3H), 1.79 - 1.47 (m, 8H), 1.47 - 1.38 (m, 2H), 1.34 (q, *J* = 7.4 Hz, 4H), 1.28 - 1.11 (m, 2H), 0.77 (t, *J* = 7.4 Hz, 6H). MS: 506 [M+H]$^+$.

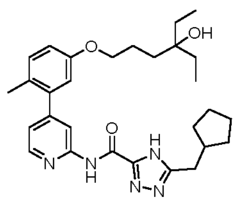

Example 166

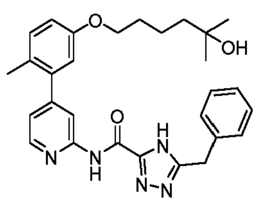

Example 167

Example 167: 5-benzyl-N-(4-(5-((5-hydroxyl-5-methylhexyl)oxy)-2-methylphenyl)pyridin-2-yl)-4H-1,2,4-triazole-3-carboxamide

The preparation was carried out in a similar manner to Example 160, except that in step 1, methyl 5-bromopentanoate was used in place of methyl 4-bromobutanoate. $^1$H NMR (400 MHz, DMSO-$d_6$) δ 14.42 (s, 1H), 9.91 (s, 1H), 8.41 (d, *J* = 5.1 Hz, 1H), 8.11 (s, 1H), 7.41-7.29 (m, 4H), 7.29-7.22 (m, 2H), 7.21 (dd, *J* = 5.1, 1.5 Hz, 1H), 6.93 (dd, *J* = 8.4, 2.7 Hz, 1H), 6.81 (d, *J* = 2.7 Hz, 1H), 4.17 (s, 2H), 4.10 (s, 1H), 3.97 (t, *J* = 6.5 Hz, 2H), 2.19 (s, 3H), 1.68 (t, *J* = 6.9 Hz, 2H), 1.50-1.33 (m, 4H), 1.06 (s, 6H). MS: 500 $[M+H]^+$.

Example 168: 5-benzyl-N-(4-(5-((4-hydroxy-4-(methylpentyl)(methyl)amino)-2-methylphenyl)pyridin-2-yl)-4H-1,2,4-triazole-3-carboxamide

The preparation was carried out in a similar manner to Example 160, except that in step 1, tert-butyl (3-bromo-4-methylphenyl)(methyl)carbamate was used in place of 3-bromo-4-methylphenol. $^1$H NMR (400 MHz, DMSO-$d_6$) δ 14.62 (s, 1H), 9.88 (s, 1H), 8.40 (d, *J* = 5.1 Hz, 1H), 8.11 (s, 1H), 7.46 - 7.29 (m, 4H), 7.29 - 7.22 (m, 1H), 7.18 (dd, *J* = 5.0, 1.5 Hz, 1H), 7.12 (d, *J* = 8.5 Hz, 1H), 6.70 (dd, *J* = 8.5, 2.8 Hz, 1H), 6.52 (d, *J* = 2.7 Hz, 1H), 4.17 (s, 2H), 4.12 (s, 1H), 3.28 (t, *J* = 7.4 Hz, 2H), 2.86 (s, 3H), 2.13 (s, 3H), 1.63 - 1.46 (m, 2H), 1.42 - 1.27 (m, 2H), 1.05 (s, 6H). MS: 499 $[M+H]^+$.

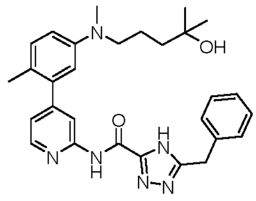
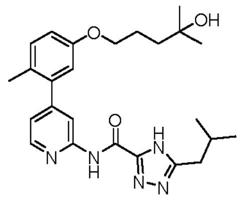

Example 168 Example 169

Example 169: N-(4-(5-((4-hydroxy-4-methylpentyl)oxy)-2-methylphenyl)pyridin-2-yl)-5-isobutyl-4H-1,2,4-triazole-3-carboxamide

The preparation was carried out in a similar manner to Example 160, except that in step 3, ethyl 5-isobutyl-4H-1,2,4-triazol-5-carboxylate was used in place of ethyl 5-benzyl-4H-1,2,4-triazol-3-carboxylate. $^1$H NMR(400 MHz, DMSO-$d_6$) δ 14.35 (s, 1H), 9.86 (s, 1H), 8.42 (d, *J* = 5.1 Hz, 1H), 8.13 (s, 1H), 7.33 - 7.16 (m, 2H), 6.93 (dd, *J* = 8.4, 2.7 Hz, 1H), 6.81 (d, *J* = 2.7 Hz, 1H), 4.19 (s, 1H), 3.97 (t, *J* = 6.6 Hz, 2H), 2.72 - 2.62 (m, 2H), 2.19 (s, 3H), 2.16 - 2.02 (m, 1H), 1.84 - 1.68 (m, 2H), 1.56 - 1.41 (m, 2H), 1.09 (s, 6H), 0.93 (d, *J* = 6.6 Hz, 6H). MS: 452 $[M+H]^+$.

Example 170: N-(4-(5-((4-hydroxy-4-methylpentyl)oxy)-2-methylphenyl)pyridin-2-yl)-5-neopentyl-4H-1,2,4-triazole-3-carboxamide

The preparation was carried out in a similar manner to Example 160, except that in step 3, ethyl 5-neopentyl-4H-1,2,4-triazol-5-carboxylate was used in place of ethyl 5-benzyl-4H-1,2,4-triazol-3-carboxylate. $^1$H NMR (400 MHz, DMSO-$d_6$) δ 14.39 (s, 1H), 9.85 (s, 1H), 8.42 (d, *J* = 5.1 Hz, 1H), 8.15 (s, 1H), 7.31-7.16 (m, 2H), 6.93 (dd, *J* = 8.4, 2.7 Hz, 1H), 6.81 (d, *J* = 2.7 Hz, 1H), 4.18 (s, 1H), 3.97 (t, *J* = 6.6 Hz, 2H), 2.67 (s, 2H), 2.20 (s, 3H), 1.83-1.66 (m, 2H), 1.57-1.39 (m, 2H), 1.09 (s, 6H), 0.97 (s, 9H). MS: 466 $[M+H]^+$.

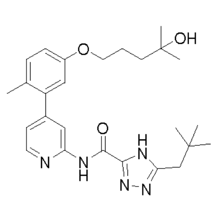
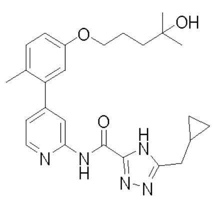

Example 170 Example 171

Example 171: 5-(cyclopropylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(cyclopropylmethyl)-4H-1,2,4-triazol-5-carboxylate was used in place of ethyl 5-benzyl-4H-1,2,4-triazol-3-carboxylate. $^1$H NMR (400 MHz, DMSO-$d_6$) δ 14.47 (s, 1H), 9.88 (s, 1H), 8.42 (d, *J* = 5.1 Hz, 1H), 8.14 (s, 1H), 7.37 - 7.16 (m, 2H), 6.93 (dd, *J* = 8.4, 2.6 Hz, 1H), 6.81 (d, *J* = 2.7 Hz, 1H), 4.18 (s, 1H), 3.97 (t, *J* = 6.5 Hz, 2H), 2.72 (d, *J* = 7.0 Hz, 2H), 2.19 (s, 3H), 1.83 - 1.66 (m, 2H), 1.55 - 1.39 (m, 2H), 1.18 - 1.03 (m, 7H), 0.59 - 0.46 (m, 2H), 0.33 - 0.22 (m, 2H). MS: 450 [M+H]$^+$.

Example 172: 5-(cyclobutylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(cyclobutylmethyl)-4H-1,2,4-triazol-5-carboxylate was used in place of ethyl 5-benzyl-4H-1,2,4-triazol-3-carboxylate. $^1$H NMR (400 MHz, DMSO-$d_6$) δ 14.36 (s, 1H), 9.85 (s, 1H), 8.42 (d, *J* = 5.1 Hz, 1H), 8.13 (s, 1H), 7.34 - 7.16 (m, 2H), 6.93 (dd, *J* = 8.4, 2.7 Hz, 1H), 6.81 (d, *J* = 2.7 Hz, 1H), 4.18 (s, 1H), 3.97 (t, *J* = 6.5 Hz, 2H), 2.88 (d, *J* = 7.5 Hz, 2H), 2.79 - 2.62 (m, 1H), 2.19 (s, 3H), 2.12 - 1.97 (m, 2H), 1.94 - 1.79 (m, 2H), 1.79 - 1.64 (m, 4H), 1.55 - 1.42 (m, 2H), 1.09 (s, 6H). MS: 464 [M+H]$^+$.

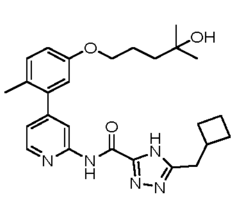

Example 172

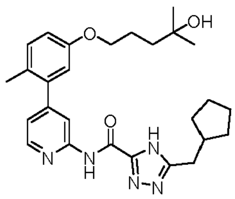

Example 173

Example 173: 5-(cyclopentylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.38 (s, 1H), 9.85 (s, 1H), 8.42 (d, *J* = 5.1 Hz, 1H), 8.14 (s, 1H), 7.33 - 7.16 (m, 2H), 6.93 (dd, *J* = 8.4, 2.7 Hz, 1H), 6.81 (d, *J* = 2.7 Hz, 1H), 4.19 (s, 1H), 3.96 (t, *J* = 6.6 Hz, 2H), 2.78 (d, *J* = 7.5 Hz, 2H), 2.34 - 2.22 (m, 1H), 2.19 (s, 3H), 1.83 - 1.66 (m, 4H), 1.66 - 1.55 (m, 2H), 1.55 - 1.41 (m, 4H), 1.27 - 1.18 (m, 2H), 1.09 (s, 6H). MS: 478 [M+H]$^+$.

Example 174: 5-(cyclopentylmethyl)-N-(4-(5-((4-hydroxy-4-methylpentyl)oxy)-2-methylphenyl)pyridin-2-yl)-4H-1,3,4-oxadiazole-2-carboxamide

The preparation was carried out in a similar manner to Example 160, except that in step 3, ethyl 5-(cyclopentylmethyl)-1,3,4-oxadiazol-2-carboxylate was used in place of ethyl 5-benzyl-4H-1,2,4-triazol-3-carboxylate. $^1$H NMR (400 MHz, DMSO-$d_6$) δ 11.23 (s, 1H), 8.47 (d, *J* = 5.1 Hz, 1H), 7.99 (s, 1H), 7.32 - 7.20 (m, 2H), 6.93 (dd, *J* = 8.4, 2.7 Hz, 1H), 6.81 (d, *J* = 2.7 Hz, 1H), 4.18 (s, 1H), 3.96 (t, *J*= 6.5 Hz, 2H), 2.97 (d, *J* = 7.4 Hz, 2H), 2.38 - 2.24 (m, 1H), 2.19 (s, 3H), 1.87 - 1.69 (m, 4H), 1.69 - 1.39 (m, 6H), 1.32 - 1.20 (m, 2H), 1.09 (s, 6H). MS: 479 [M+H]$^+$.

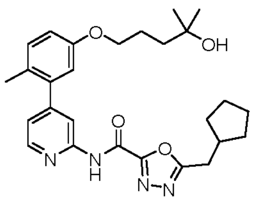

Example 174

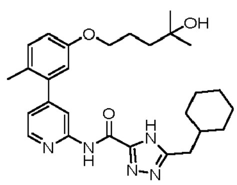

Example 175

Example 175: 5-(cyclohexylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(cyclohexylmethyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.37 (s, 1H), 9.85 (s, 1H), 8.42 (d, *J* = 5.1 Hz, 1H), 8.13 (s, 1H), 7.31 - 7.17 (m, 2H), 6.93 (dd, *J* = 8.4, 2.7 Hz, 1H), 6.81 (d, *J* = 2.7 Hz, 1H), 4.18 (s, 1H), 3.97 (t, *J* = 6.6 Hz, 2H), 2.67 (d, *J* = 7.0 Hz, 2H), 2.19 (s, 3H), 1.84 - 1.70 (m, 3H), 1.70 - 1.56 (m, 5H), 1.54 - 1.41 (m, 2H), 1.29 - 1.15 (m, 3H), 1.09 (s, 6H), 1.05 - 0.89 (m, 2H). MS: 492 $[M+H]^+$.

Example 176: 5-(2-cyclopentylethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(2-cyclopentylethyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.32 (s, 1H), 9.86 (s, 1H), 8.42 (d, *J* = 5.1 Hz, 1H), 8.13 (s, 1H), 7.33 - 7.15 (m, 2H), 6.93 (dd, *J* = 8.4, 2.7 Hz, 1H), 6.81 (d, *J* = 2.7 Hz, 1H), 4.18 (s, 1H), 3.97 (t, *J* = 6.5 Hz, 2H), 2.79 (t, *J* = 7.2 Hz, 2H), 2.19 (s, 3H), 1.84 - 1.66 (m, 7H), 1.66 - 1.39 (m, 6H), 1.18 - 0.98 (m, 8H). MS: 492 $[M+H]^+$.

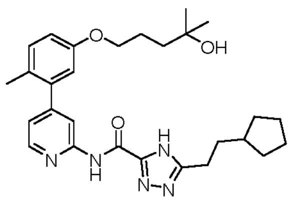

Example 176

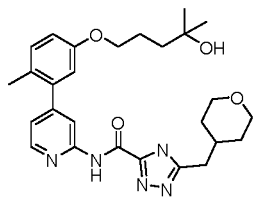

Example 177

Example 177: N-(4-(5-((4-hydroxy-4-methylpentyl)oxy)-2-methylphenyl)pyridin-2-yl)-5-((tetrahydro-2H-pyran-4-yl)methyl)-4H-1,2,4-triazole-3-carboxamide

The preparation was carried out in a similar manner to Example 160, except that in step 3, ethyl 5-((tetrahydro-2H-pyran-4-yl)methyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.41 (s, 1H), 9.87 (s, 1H), 8.42 (d, *J* = 5.1 Hz, 1H), 8.13 (s, 1H), 7.34 - 7.14 (m, 2H), 6.93 (dd, *J* = 8.4, 2.7 Hz, 1H), 6.81 (d, *J* = 2.7 Hz, 1H), 4.18 (s, 1H), 3.97 (t, *J* = 6.5 Hz, 2H), 3.89 - 3.78 (m, 2H), 3.31 - 3.19 (m, 2H), 2.73 (d, *J* = 7.1 Hz, 2H), 2.19 (s, 3H), 2.07 - 1.92 (m, 1H), 1.81 - 1.68 (m, 2H), 1.61 - 1.51 (m, 2H), 1.51 - 1.42 (m, 2H), 1.30 - 1.22 (m, 2H), 1.09 (s, 6H). MS: 494 $[M+H]^+$.

Example 178: 5-((4,4-difluorocyclohexyl)methyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-((4,4-difluorocyclohexyl)methyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.41 (s, 1H), 9.87 (s, 1H), 8.42 (d, *J* = 5.1 Hz, 1H), 8.13 (s, 1H), 7.32 - 7.13 (m, 2H), 6.93 (dd, *J* = 8.5, 2.6 Hz, 1H), 6.81 (d, *J* = 2.7 Hz, 1H), 4.18 (s, 1H), 3.97 (t, *J* = 6.6 Hz, 2H), 2.75 (d, *J* = 7.0 Hz, 2H), 2.19 (s, 3H), 2.09 - 1.64 (m, 9H), 1.55 - 1.40 (m, 2H), 1.35 - 1.23 (m, 2H), 1.09 (s, 6H). MS: 528 $[M+H]^+$.

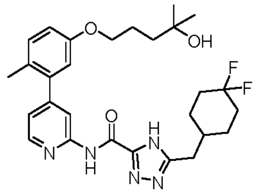

Example 178

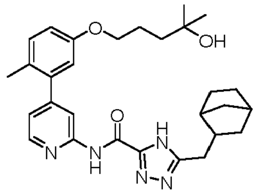

Example 179

Example 179: 5-(bicyclo[2.2.1]heptan-2-ylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(bicyclo[2.2.1]heptan-2-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.32 (s, 1H), 9.86 (s, 1H), 8.42 (d, $J$ = 5.1 Hz, 1H), 8.13 (s, 1H), 7.35 - 7.12 (m, 2H), 6.93 (dd, $J$ = 8.4, 2.7 Hz, 1H), 6.81 (d, $J$ = 2.7 Hz, 1H), 4.18 (s, 1H), 3.97 (t, $J$ = 6.6 Hz, 2H), 3.17 (d, $J$ = 4.1 Hz, 1H), 2.74 (dd, $J$ = 14.6, 8.0 Hz, 1H), 2.59 (dd, $J$ = 14.6, 7.7 Hz, 1H), 2.28 - 2.16 (m, 4H), 1.96 - 1.85 (m, 1H), 1.81 - 1.68 (m, 2H), 1.54 - 1.33 (m, 6H), 1.29 - 1.20 (m, 2H), 1.19 - 1.12 (m, 2H), 1.09 (s, 6H). MS: 504 $[M+H]^+$.

Example 180: 5-(adamantan-1-ylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(adamantan-1-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.32 (s, 1H), 9.83 (s, 1H), 8.39 (d, $J$ = 5.1 Hz, 1H), 8.12 (s, 1H), 7.30 - 7.13 (m, 2H), 6.90 (dd, $J$ = 8.4, 2.7 Hz, 1H), 6.78 (d, $J$ = 2.7 Hz, 1H), 4.17 (s, 1H), 3.94 (t, $J$ = 6.6 Hz, 2H), 2.51 (s, 2H), 2.17 (s, 3H), 1.95 - 1.83 (m, 3H), 1.79 - 1.66 (m, 2H), 1.66 - 1.57 (m, 3H), 1.57 - 1.38 (m, 11H), 1.06 (s, 6H). MS: 544 $[M+H]^+$.

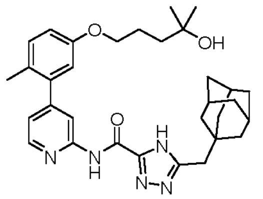
Example 180

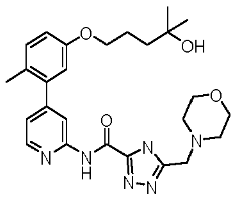
Example 181

Example 181: N-(4-(5-((4-hydroxy-4-methylpentyl)oxy)-2-methylphenyl)pyridin-2-yl)-5-(morpholinomethyl)-4H-1,2,4-triazole-3-carboxamide

The preparation was carried out in a similar manner to Example 160, except that in step 3, ethyl 5-(morpholinomethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.09 (s, 1H), 9.95 (s, 1H), 8.42 (d, $J$ = 5.1 Hz, 1H), 8.12 (s, 1H), 7.32 - 7.16 (m, 2H), 6.93 (dd, $J$ = 8.4, 2.7 Hz, 1H), 6.81 (d, $J$ = 2.7 Hz, 1H), 4.20 (s, 1H), 3.97 (t, $J$ = 6.5 Hz, 2H), 3.71 (s, 2H), 3.59 (t, $J$ = 4.6 Hz, 4H), 2.49 - 2.40 (m, 4H), 2.19 (s, 3H), 1.81 - 1.67 (m, 2H), 1.52 - 1.41 (m, 2H), 1.09 (s, 6H). MS: 495 $[M+H]^+$.

Example 182: N-(4-(5-((4-hydroxy-4-methylpentyl)oxy)-2-methylphenyl)pyridin-2-yl)-5-phenethyl-4H-1,2,4-triazole-3-carboxamide

The preparation was carried out in a similar manner to Example 160, except that in step 3, ethyl 5-(phenethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.43 (s, 1H), 9.89 (s, 1H), 8.43 (d, $J$ = 5.2 Hz, 1H), 8.14 (s, 1H), 7.40-7.10 (m, 7H), 6.93 (dd, $J$ = 8.4, 2.7 Hz, 1H), 6.81 (d, $J$ = 2.7 Hz, 1H), 4.18 (s, 1H), 3.97 (t, $J$ = 6.6 Hz, 2H), 3.22-2.99 (m, 4H), 2.20 (s, 3H), 1.84-1.66 (m, 2H), 1.57-1.37 (m, 2H), 1.09 (s, 6H). MS: 500 $[M+H]^+$.

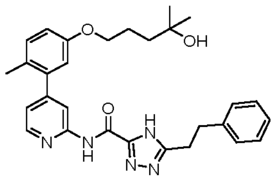
Example 182

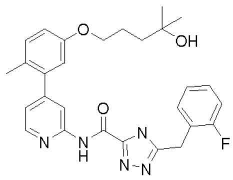
Example 183

Example 183: 5-(2-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 9.90 (s, 1H), 8.41 (d, *J* = 5.1 Hz, 1H), 8.10 (s, 1H), 7.44 - 7.29 (m, 2H), 7.28 - 7.13 (m, 4H), 6.92 (dd, *J* = 8.4, 2.7 Hz, 1H), 6.80 (d, *J* = 2.7 Hz, 1H), 4.29 - 4.09 (m, 3H), 3.96 (t, *J* = 6.5 Hz, 2H), 2.18 (s, 3H), 1.83 - 1.68 (m, 2H), 1.52 - 1.39 (m, 2H), 1.09 (s, 6H). MS: 504 [M+H]$^+$.

Example 184: 5-(3-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 13.74 (s, 1H), 9.93 (s, 1H), 8.41 (d, *J* = 5.1 Hz, 1H), 8.11 (s, 1H), 7.50-7.30 (m, 2H), 7.30-7.06 (m, 4H), 6.92 (d, *J* = 8.6 Hz, 1H), 6.80 (s, 1H), 4.34-4.06 (m, 3H), 3.96 (t, *J* = 6.6 Hz, 2H), 2.19 (s, 3H), 1.84-1.66 (m, 2H), 1.47 (t, *J* = 8.1 Hz, 2H), 1.09 (s, 6H).MS: 504 [M+H]$^+$.

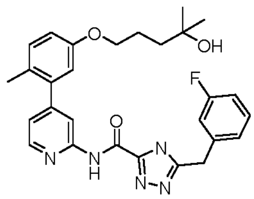
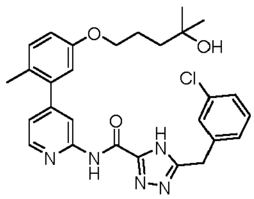

Example 184 Example 185

Example 185: 5-(3-chlorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(3-chlorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.54 (s, 1H), 9.95 (s, 1H), 8.42 (d, *J* = 5.1 Hz, 1H), 8.11 (s, 1H), 7.49 - 7.13 (m, 6H), 6.92 (dd, *J* = 8.4, 2.7 Hz, 1H), 6.80 (d, *J* = 2.7 Hz, 1H), 4.31 - 4.07 (m, 3H), 3.96 (t, *J* = 6.5 Hz, 2H), 2.19 (s, 3H), 1.83 - 1.66 (m, 2H), 1.54 - 1.38 (m, 2H), 1.09 (s, 6H). MS: 520 [M+H]$^+$.

Example 186: 5-(3-methoxybenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(3-methoxybenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 9.92 (s, 1H), 8.41 (d, *J* = 5.1 Hz, 1H), 8.12 (s, 1H), 7.41 - 7.12 (m, 3H), 7.05 - 6.70 (m, 5H), 4.26 - 4.05 (m, 3H), 3.96 (t, *J* = 6.5 Hz, 2H), 3.74 (s, 3H), 2.19 (s, 3H), 1.83 - 1.65 (m, 2H), 1.56 - 1.37 (m, 2H), 1.09 (s, 6H). MS: 516 [M+H]$^+$.

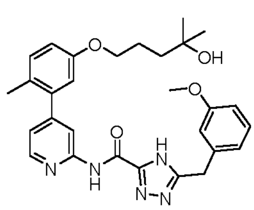
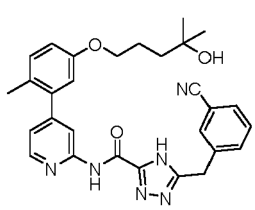

Example 186 Example 187

Example 187: 5-(3-cyanobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(3-cyanobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.69 (s, 1H), 9.98 (s, 1H), 8.42 (d, $J$ = 5.1 Hz, 1H), 8.11 (s, 1H), 7.84 (s, 1H), 7.76 (d, $J$ = 7.7 Hz, 1H), 7.69 (d, $J$ = 7.8 Hz, 1H), 7.62 -7.51 (m, 1H), 7.30 - 7.17 (m, 2H), 6.93 (dd, $J$ = 8.4, 2.7 Hz, 1H), 6.80 (d, $J$ = 2.7 Hz, 1H), 4.27 (s, 2H), 4.19 (s, 1H), 3.96 (t, $J$ = 6.5 Hz, 2H), 2.19 (s, 3H), 1.81 - 1.68 (m, 2H), 1.53 - 1.40 (m, 2H), 1.09 (s, 6H). MS: 511 [M+H]$^+$.

Example 188: 5-(4-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(4-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.54 (s, 1H), 9.93 (s, 1H), 8.42 (d, $J$ = 5.1 Hz, 1H), 8.11 (s, 1H), 7.46 - 7.31 (m, 2H), 7.31 - 7.10 (m, 4H), 6.93 (dd, $J$ = 8.4, 2.7 Hz, 1H), 6.80 (d, $J$ = 2.7 Hz, 1H), 4.29 - 4.08 (m, 3H), 3.96 (t, $J$ = 6.5 Hz, 2H), 2.19 (s, 3H), 1.83 - 1.65 (m, 2H), 1.54 - 1.40 (m, 2H), 1.09 (s, 6H). MS: 504 [M+H]$^+$.

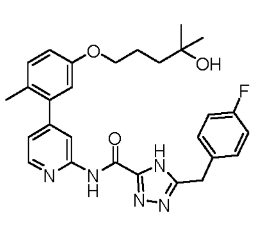 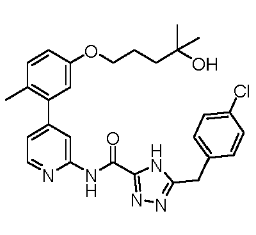

Example 188 Example 189

Example 189: 5-(4-chlorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(4-chlorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.65 (s, 1H), 9.93 (s, 1H), 8.42 (d, $J$ = 5.2 Hz, 1H), 8.11 (s, 1H), 7.51 - 7.29 (m, 4H), 7.29 - 7.13 (m, 2H), 6.93 (dd, $J$ = 8.5, 2.8 Hz, 1H), 6.80 (d, $J$ = 2.8 Hz, 1H), 4.28 - 4.07 (m, 3H), 3.96 (t, $J$ = 6.6 Hz, 2H), 2.19 (s, 3H), 1.75 (t, $J$ = 8.2 Hz, 2H), 1.54 - 1.39 (m, 2H), 1.09 (s, 6H). MS: 520 [M+H]$^+$.

Example 190: 5-(4-methoxybenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(4-methoxybenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.59 (s, 1H), 9.89 (s, 1H), 8.41 (d, $J$ = 5.1 Hz, 1H), 8.11 (s, 1H), 7.40 - 7.09 (m, 4H), 7.04 - 6.70 (m, 4H), 4.18 (s, 1H), 4.10 (s, 2H), 3.96 (t, $J$ = 6.8 Hz, 2H), 2.19 (s, 3H), 1.83 - 1.64 (m, 2H), 1.57 - 1.35 (m, 2H), 1.09 (s, 6H). MS: 516 M+H+.

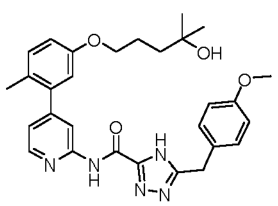 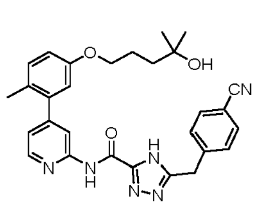

Example 190 Example 191

Example 191: 5-(4-cyanobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(4-cyanobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 9.95 (s, 1H), 8.42 (d, *J* = 5.1 Hz, 1H), 8.11 (s, 1H), 7.91 - 7.76 (m, 2H), 7.62 - 7.47 (m, 2H), 7.32 - 7.13 (m, 2H), 6.93 (dd, *J* = 8.4, 2.7 Hz, 1H), 6.80 (d, *J* = 2.7 Hz, 1H), 4.29 (s, 2H), 4.18 (s, 1H), 3.96 (t, *J* = 6.5 Hz, 2H), 2.19 (s, 3H), 1.81 - 1.67 (m, 2H), 1.53 - 1.41 (m, 2H), 1.09 (s, 6H). MS: 511 [M+H]$^+$.

Example 192: 5-(3,4-difluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, ethyl 5-(3,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 9.96 (s, 1H), 8.42 (d, *J* = 5.1 Hz, 1H), 8.11 (d, *J* = 1.4 Hz, 1H), 7.51 - 7.32 (m, 2H), 7.32 - 7.13 (m, 3H), 6.93 (dd, *J* = 8.4, 2.7 Hz, 1H), 6.80 (d, *J* = 2.7 Hz, 1H), 4.25 - 4.12 (m, 3H), 3.96 (t, *J* = 6.5 Hz, 2H), 2.19 (s, 3H), 1.82 - 1.67 (m, 2H), 1.53 - 1.41 (m, 2H), 1.09 (s, 6H). MS: 522 [M+H]$^+$.

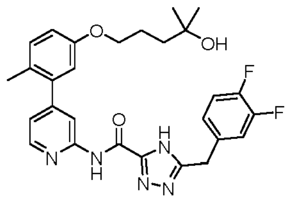
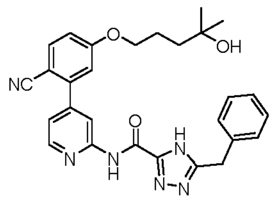

Example 192 Example 193

Example 193: 5-benzyl-N-(4-(2-cyano-5-((4-hydroxy-4-methylpentyl)oxy)phenyl)pyridin-2-yl)-4H-1,2,4-triazole-3-carboxamide

The preparation was carried out in a similar manner to Example 160, except that in step 1, 2-chloro-4-hydroxybenzonitrile was used in place of 3-bromo-4-methylphenol. $^1$H NMR (400 MHz, DMSO-$d_6$) δ 14.54 (s, 1H), 10.04 (s, 1H), 8.52 (d, *J* = 5.1 Hz, 1H), 8.34 (d, *J* = 1.4 Hz, 1H), 7.94 (d, *J* = 8.5 Hz, 1H), 7.41 (dd, *J* = 5.1, 1.6 Hz, 1H), 7.38 - 7.29 (m, 4H), 7.29 - 7.15 (m, 3H), 4.27 - 4.08 (m, 5H), 1.87 - 1.72 (m, 2H), 1.56 - 1.41 (m, 2H), 1.10 (s, 6H). MS: 497 [M+H]$^+$.

Example 194: 5-benzyl-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol. $^1$H NMR (400 MHz, DMSO-$d_6$) δ 14.61 (s, 1H), 9.96 (s, 1H), 8.43 (d, *J* = 5.1 Hz, 1H), 8.16 (s, 1H), 7.79 (d, *J* = 8.9 Hz, 1H), 7.43 - 7.29 (m, 4H), 7.29 - 7.22 (m, 1H), 7.22 - 7.11 (m, 2H), 6.97 (d, *J* = 2.5 Hz, 1H), 4.26 - 4.13 (m, 3H), 4.09 (t, *J* = 6.6 Hz, 2H), 1.86 - 1.69 (m, 2H), 1.54 - 1.39 (m, 2H), 1.09 (s, 6H). MS: 540 [M+H]$^+$.

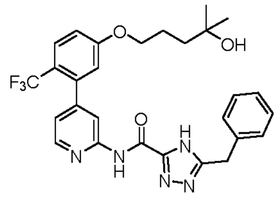
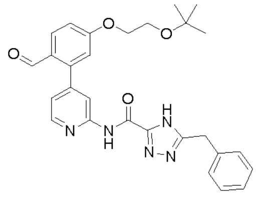

Example 194 Example 195

Example 195: 5-benzyl-N-(4-(5-(2-(tert-butoxy)ethoxy)-2-formylphenyl)pyridin-2-yl)-4H-1,2,4-triazole-3-carboxamide

The preparation was carried out in a similar manner to Example 64, except that in step 1, 2-bromo-4-hydoxylbenzaldehyde was used in place of 3-bromo-4-methylphenol. $^1$H NMR (400 MHz, DMSO-$d_6$) δ 14.60 (s, 1H), 9.99 (s, 1H), 9.80 (s, 1H), 8.46 (d, $J$ = 5.1 Hz, 1H), 8.16 (s, 1H), 7.96 (d, $J$ = 8.7 Hz, 1H), 7.43 - 7.29 (m, 5H), 7.29 - 7.15 (m, 2H), 7.05 (d, $J$ = 2.5 Hz, 1H), 4.28 - 4.20 (m, 2H), 4.18 (s, 2H), 3.73 - 3.61 (m, 2H), 1.15 (s, 9H). MS: 500 [M+H]$^+$.

Example 196: 5-benzyl-N-(5-(5-methoxy-2-methylphenyl)pyridin-3-yl)-4H-1,2,4-triazole-3-carboxamide

The preparation was carried out in a similar manner to Example 92, except that in step 1, 3-bromo-4-methylphenol was used in place of 2-bromo-3-methylphenol, and in step 2, 5-bromopyridin-3-amine was used in place of 4-bromopyridin-2-amine. $^1$H NMR (600 MHz, DMSO-$d_6$) δ 14.54 (s, 1H), 10.75 (s, 1H), 9.02 (s, 1H), 8.32 (s, 1H), 8.21 (s, 1H), 7.43-7.18 (m, 6H), 6.91 (d, $J$ = 8.2 Hz, 1H), 6.83 (s, 1H), 4.18 (s, 2H), 3.76 (s, 3H), 2.18 (s, 3H). MS: 400 [M+H]$^+$.

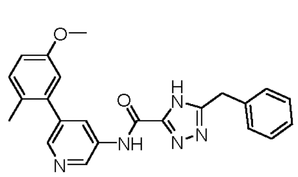

Example 196

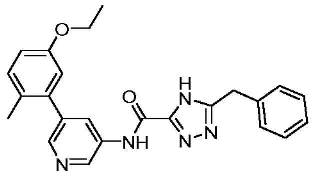

Example 197

Example 197: 5-benzyl-N-(5-(5-ethoxy-2-methylphenyl)pyridin-3-yl)-4H-1,2,4-triazole-3-carboxamide

The preparation was carried out in a similar manner to Example 92, except that in step 1, 3-bromo-4-methylphenol was used in place of 2-bromo-3-methylphenol, bromoethane was used in place of iodomethane, and in step 2, 5-bromopyridin-3-amine was used in place of 4-bromopyridin-2-amine. $^1$H NMR (600 MHz, DMSO-$d_6$) δ 14.49 (s, 1H), 10.70 (s, 1H), 9.02 (d, $J$ = 2.4 Hz, 1H), 8.41 - 8.08 (m, 2H), 7.48 - 7.12 (m, 6H), 6.90 (dd, $J$ = 8.4, 2.7 Hz, 1H), 6.80 (d, $J$ = 2.7 Hz, 1H), 4.18 (s, 2H), 4.03 (q, $J$ = 7.0 Hz, 2H), 2.18 (s, 3H), 1.32 (t, $J$ = 7.0 Hz, 3H). MS: 414 [M+H]$^+$.

Example 198: 5-benzyl-N-(4-(5-butoxy-2-methylphenyl)pyridin-2-yl)-4H-1,2,4-triazole-3-carboxamide

The preparation was carried out in a similar manner to Example 92, except that in step 1, 3-bromo-4-methylphenol was used in place of 2-bromo-3-methylphenol, and 1-bromobutane was used in place of iodomethane. $^1$H NMR (400 MHz, DMSO-$d_6$) δ 14.58 (s, 1H), 9.92 (s, 1H), 8.41 (d, $J$ = 5.1 Hz, 1H), 8.11 *(d, J* = 1.5 Hz, 1H), 7.48 - 7.10 (m, 7H), 6.93 (dd, $J$ = 8.4, 2.7 Hz, 1H), 6.81 (d, $J$ = 2.7 Hz, 1H), 4.17 (s, 2H), 3.97 (t, $J$ = 6.5 Hz, 2H), 2.19 (s, 3H), 1.83 - 1.54 (m, 2H), 1.54 - 1.27 (m, 2H), 0.92 (t, $J$ = 7.4 Hz, 3H). MS: 442 [M+H]$^+$.

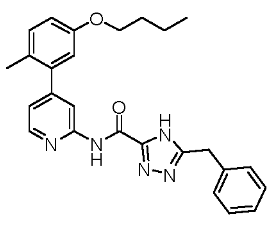

Example 198

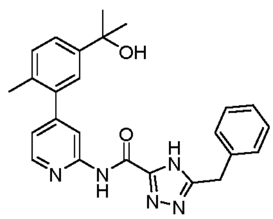

Example 199

Example 199: 5-benzyl-N-(4-(5-(2-hydroxypropan-2-yl)-2-methylphenyl)pyridin-2-yl)-4H-1,2,4-triazole-3-carboxamide

The preparation was carried out in a similar manner to Example 161, except that in step 1, methyl 3-bromo-4-methylbenzoate was used in place of methyl 4-bromo-3-methylbenzoate. $^1$H NMR (400 MHz, DMSO-$d_6$) δ 14.59 (s, 1H), 9.89 (s, 1H), 8.42 (d, $J$ = 5.1 Hz, 1H), 8.12 (d, $J$ = 1.3 Hz, 1H), 7.42 (dd, $J$ = 7.9, 2.0 Hz, 1H), 7.39 - 7.30 (m, 5H), 7.30 - 7.23 (m, 2H), 7.21 (dd, $J$ = 5.1, 1.6 Hz, 1H), 5.05 (s, 1H), 4.17 (s, 2H), 2.25 (s, 3H), 1.43 (s, 6H). MS: 428 [M+H]$^+$.

Example 200: 5-(3,5-difluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.56 (s, 1H), 9.91 (s, 1H), 8.42 (d, *J* = 5.1 Hz, 1H), 8.12 (s, 1H), 7.29-7.03 (m, 5H), 6.93 (dd, *J* = 8.4, 2.7 Hz, 1H), 6.80 (d, *J* = 2.7 Hz, 1H), 4.26 (s, 2H), 4.18 (s, 1H), 3.96 (t, *J* = 6.6 Hz, 2H), 2.19 (s, 3H), 1.81-1.67 (m, 2H), 1.53-1.40 (m, 2H), 1.09 (s, 6H). MS: 522 [M+H]$^+$.

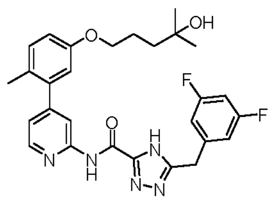

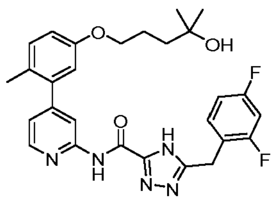

Example 200 Example 201

Example 201: 5-(2,4-difluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.63 (s, 1H), 9.89 (s, 1H), 8.42 (d, *J* = 5.1 Hz, 1H), 8.11 (s, 1H), 7.57 - 7.38 (m, 1H), 7.38 - 7.17 (m, 3H), 7.17 - 7.01 (m, 1H), 6.93 (dd, *J* = 8.4, 2.7 Hz, 1H), 6.81 (d, *J* = 2.7 Hz, 1H), 4.28 - 4.11 (m, 3H), 3.97 (t, *J* = 6.5 Hz, 2H), 2.19 (s, 3H), 1.83 - 1.66 (m, 2H), 1.56 - 1.40 (m, 2H), 1.10 (s, 6H). MS: 522 [M+H]$^+$.

Example 202: 5-(2,3-difluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,3-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.70 (s, 1H), 9.92 (s, 1H), 8.41 (d, *J* = 5.1 Hz, 1H), 8.10 (s, 1H), 7.47 - 7.30 (m, 1H), 7.30 - 7.12 (m, 4H), 6.93 (dd, *J* = 8.4, 2.7 Hz, 1H), 6.80 (d, *J* = 2.7 Hz, 1H), 4.27 (s, 2H), 4.17 (s, 1H), 3.96 (t, *J* = 6.5 Hz, 2H), 2.19 (s, 3H), 1.82 - 1.66 (m, 2H), 1.55 - 1.40 (m, 2H), 1.09 (s, 6H). MS: 522 [M+H]$^+$.

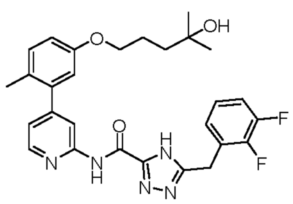

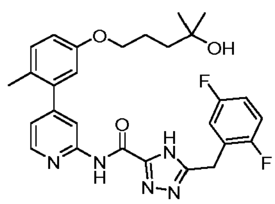

Example 202 Example 203

Example 203: 5-(2,5-difluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.73 (s, 1H), 9.94 (s, 1H), 8.42 (d, *J* = 5.1 Hz, 1H), 8.11 (s, 1H), 7.42-7.11 (m, 5H), 6.93 (dd, *J* = 8.4, 2.7 Hz, 1H), 6.81 (d, *J* = 2.7 Hz, 1H), 4.22 (s, 2H), 4.17 (s, 1H), 3.97 (t, *J* = 6.5 Hz, 2H), 2.19 (s, 3H), 1.80-1.70 (m, 2H), 1.54-1.42 (m, 2H), 1.10 (s, 6H). MS: 522 [M+H]$^+$.

Example 204: 5-(5-chloro-2-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(5-chloro-2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.93 (s, 1H), 8.42 (d, *J* = 5.1 Hz, 1H), 8.10 (s, 1H), 7.53 (dd, *J* = 6.6, 2.7 Hz, 1H), 7.48 - 7.36 (m, 1H), 7.36 - 7.14 (m, 3H), 6.93 (dd, *J* = 8.4, 2.7 Hz, 1H), 6.80 (d, *J* = 2.7 Hz, 1H), 4.22 (s, 2H), 4.17 (s, 1H), 3.96 (t, *J* = 6.5 Hz, 2H), 2.19 (s, 3H), 1.83 - 1.68 (m, 2H), 1.55 - 1.42 (m, 2H), 1.09 (s, 6H). MS: 538 [M+H]$^+$.

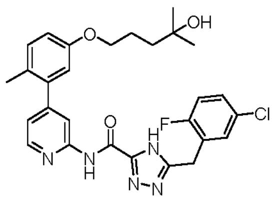

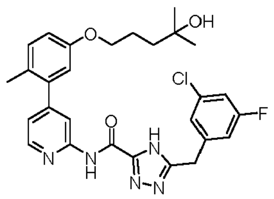

Example 204 Example 205

Example 205: 5-(5-chloro-3-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(5-chloro-3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.56 (s, 1H), 9.91 (s, 1H), 8.42 (d, *J* = 5.1 Hz, 1H), 8.11 (s, 1H), 7.43-7.14 (m, 5H), 6.93 (dd, *J* = 8.4, 2.7 Hz, 1H), 6.80 (d, *J* = 2.7 Hz, 1H), 4.24 (s, 2H), 4.17 (s, 1H), 3.96 (t, *J* = 6.6 Hz, 2H), 2.19 (s, 3H), 1.82-1.68 (m, 2H), 1.52-1.42 (m, 2H), 1.09 (s, 6H). MS: 538 [M+H]$^+$.

Example 206: 5-(3-chloro-4-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3-chloro-4-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 9.95 (s, 1H), 8.42 (d, *J* = 5.1 Hz, 1H), 8.11 (s, 1H), 7.59 (dd, *J* = 7.2, 2.1 Hz, 1H), 7.47 - 7.29 (m, 2H), 7.29 - 7.15 (m, 2H), 6.93 (dd, *J* = 8.4, 2.7 Hz, 1H), 6.80 (d, *J* = 2.7 Hz, 1H), 4.24 - 4.14 (m, 3H), 3.96 (t, *J* = 6.6 Hz, 2H), 2.19 (s, 3H), 1.83 - 1.66 (m, 2H), 1.54 - 1.41 (m, 2H), 1.09 (s, 6H). MS: 538 [M+H]$^+$.

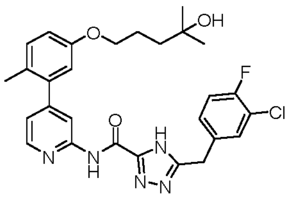

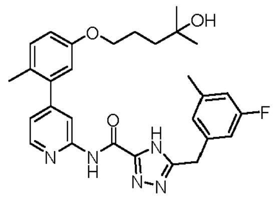

Example 206 Example 207

Example 207: 5-(3-fluoro-5-methylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3-fluoro-5-methylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.55 (s, 1H), 9.90 (s, 1H), 8.42 (d, *J* = 5.1 Hz, 1H), 8.11 (s, 1H), 7.32-7.14 (m, 2H), 7.03-6.86 (m, 4H), 6.80 (d, *J* = 2.7 Hz, 1H), 4.22-4.10 (m, 3H), 3.96 (t, *J* = 6.6 Hz, 2H), 2.30 (s, 3H), 2.19 (s, 3H), 1.82-1.68 (m, 2H), 1.54-1.42 (m, 2H), 1.09 (s, 6H). MS: 518 [M+H]$^+$.

Example 208: 5-(4-fluoro-3-methylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(4-fluoro-3-methylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.53 (s, 1H), 9.89 (s, 1H), 8.42 (d, *J* = 5.1 Hz, 1H), 8.11 (s, 1H), 7.34 - 7.00 (m, 5H), 6.93 (dd, *J* = 8.4, 2.7 Hz, 1H), 6.80 (d, *J* = 2.7 Hz, 1H), 4.24 - 4.04 (m, 3H), 3.96 (t, *J* = 6.5 Hz, 2H), 2.27 - 2.12 (m, 6H), 1.83 - 1.67 (m, 2H), 1.56 - 1.41 (m, 2H), 1.09 (s, 6H). MS: 518 [M+H]$^+$.

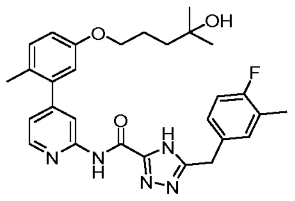
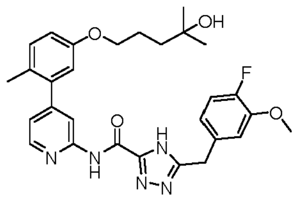

Example 208 Example 209

Example 209: 5-(4-fluoro-3-methoxybenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(4-fluoro-3-methoxybenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.52 (s, 1H), 9.89 (s, 1H), 8.42 (d, *J* = 5.1 Hz, 1H), 8.11 (s, 1H), 7.30 - 7.08 (m, 4H), 6.93 (dd, *J* = 8.4, 2.7 Hz, 1H), 6.90 - 6.83 (m, 1H), 6.80 (d, *J* = 2.7 Hz, 1H), 4.22 - 4.09 (m, 3H), 3.96 (t, *J* = 6.5 Hz, 2H), 3.83 (s, 3H), 2.19 (s, 3H), 1.81 - 1.69 (m, 2H), 1.54 - 1.41 (m, 2H), 1.09 (s, 6H). MS: 534 [M+H]$^+$.

Example 210: 5-(2-fluoro-3-trifluoromethylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2-fluoro-3-trifluoromethylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.91 (s, 1H), 8.41 (d, *J* = 5.1 Hz, 1H), 8.10 (s, 1H), 7.88 - 7.64 (m, 2H), 7.49 - 7.34 (m, 1H), 7.30 - 7.13 (m, 2H), 6.93 (dd, *J* = 8.4, 2.7 Hz, 1H), 6.80 (d, *J* = 2.7 Hz, 1H), 4.32 (s, 2H), 4.16 (s, 1H), 3.96 (t, *J* = 6.5 Hz, 2H), 2.19 (s, 3H), 1.81 - 1.69 (m, 2H), 1.53 - 1.41 (m, 2H), 1.09 (s, 6H). MS: 572 [M+H]$^+$.

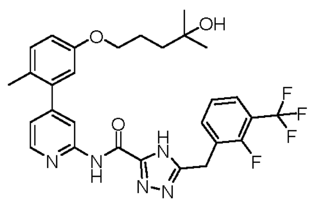
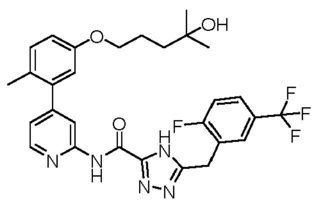

Example 210 Example 211

Example 211: 5-(2-fluoro-5-trifluoromethylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2-fluoro-5-trifluoromethylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.76 (s, 1H), 9.92 (s, 1H), 8.41 (d, *J* = 5.1 Hz, 1H), 8.10 (s, 1H), 7.90 (dd, *J* = 6.7, 2.4 Hz, 1H), 7.83-7.67 (m, 1H), 7.56-7.37 (m, 1H), 7.33-7.12 (m, 2H), 6.92 (dd, *J* = 8.4, 2.7 Hz, 1H), 6.80 (d, *J* = 2.7 Hz, 1H), 4.32 (s, 2H), 4.17 (s, 1H), 3.96 (t, *J* = 6.5 Hz, 2H), 2.18 (s, 3H), 1.80-1.68 (m, 2H), 1.52-1.41 (m, 2H), 1.09 (s, 6H). MS: 572 [M+H]$^+$.

Example 212: 5-(4-fluoro-3-trifluoromethylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(4-fluoro-3-trifluoromethylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.55 (s, 1H), 9.90 (s, 1H), 8.42 (d, *J* = 5.1 Hz, 1H), 8.11 (s, 1H), 7.84 - 7.76 (m, 1H), 7.76 - 7.67 (m, 1H), 7.50 (dd, *J* = 10.6, 8.8 Hz, 1H), 7.29 - 7.16 (m, 2H), 6.93 (dd, *J* = 8.4, 2.7 Hz, 1H), 6.80 (d, *J* = 2.7 Hz, 1H), 4.30 (s, 2H), 4.16 (s, 1H), 3.96 (t, *J* = 6.6 Hz, 2H), 2.19 (s, 3H), 1.82 - 1.66 (m, 2H), 1.52 - 1.42 (m, 2H), 1.09 (s, 6H). MS: 572 [M+H]$^+$.

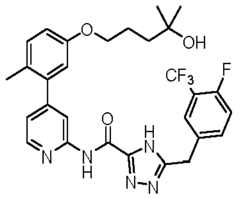
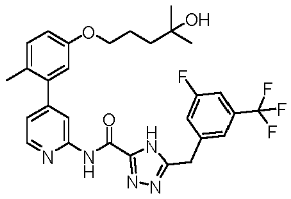

Example 212 Example 213

Example 213: 5-(3-fluoro-5-trifluoromethylbenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3-fluoro-5-trifluoromethylbenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 9.90 (s, 1H), 8.42 (d, *J* = 5.1 Hz, 1H), 8.12 (s, 1H), 7.70 - 7.50 (m, 3H), 7.30 - 7.15 (m, 2H), 6.93 (dd, *J* = 8.4, 2.7 Hz, 1H), 6.80 (d, *J* = 2.7 Hz, 1H), 4.36 (s, 2H), 4.17 (s, 1H), 3.96 (t, *J* = 6.5 Hz, 2H), 2.19 (s, 3H), 1.82 - 1.67 (m, 2H), 1.53 - 1.42 (m, 2H), 1.09 (s, 6H). MS: 572 [M+H]$^+$.

Example 214: 5-(2,3,4-trifluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,3,4-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 9.87 (s, 1H), 8.42 (d, *J* = 5.1 Hz, 1H), 8.10 (s, 1H), 7.40 - 7.15 (m, 4H), 6.93 (dd, *J* = 8.4, 2.7 Hz, 1H), 6.80 (d, *J* = 2.7 Hz, 1H), 4.26 (s, 2H), 4.16 (s, 1H), 3.96 (t, *J* = 6.5 Hz, 2H), 2.19 (s, 3H), 1.80 - 1.66 (m, 2H), 1.53 - 1.41 (m, 2H), 1.09 (s, 6H). MS: 540 [M+H]$^+$.

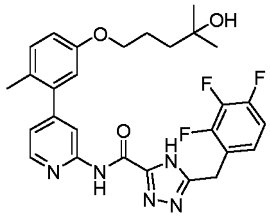
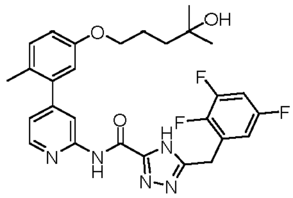

Example 214 Example 215

Example 215: 5-(2,3,5-trifluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,3,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.75 (s, 1H), 9.96 (s, 1H), 8.42 (d, *J* = 5.1 Hz, 1H), 8.10 (s, 1H), 7.58 - 7.41 (m, 1H), 7.33 - 7.13 (m, 3H), 7.01 - 6.87 (m, 1H), 6.80 (d, *J* = 2.7 Hz, 1H), 4.28 (s, 2H), 4.16 (s, 1H), 3.96 (t, *J* = 6.5 Hz, 2H), 2.19 (s, 3H), 1.81 - 1.67 (m, 2H), 1.52 - 1.42 (m, 2H), 1.09 (s, 6H). MS: 540 [M+H]$^+$.

Example 216: 5-(2,3,6-trifluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,3,6-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.75 (s, 1H), 9.89 (s, 1H), 8.41 (d, $J$ = 5.1 Hz, 1H), 8.08 (s, 1H), 7.59 - 7.42 (m, 1H), 7.31 - 7.12 (m, 3H), 6.92 (dd, $J$ = 8.4, 2.7 Hz, 1H), 6.80 (d, $J$ = 2.7 Hz, 1H), 4.25 (s, 2H), 4.17 (s, 1H), 3.96 (t, $J$ = 6.5 Hz, 2H), 2.18 (s, 3H), 1.80 - 1.68 (m, 2H), 1.55 - 1.41 (m, 2H), 1.09 (s, 6H). MS: 540 [M+H]$^+$.

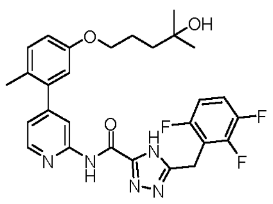
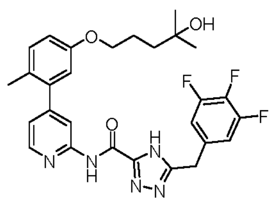

Example 216 Example 217

Example 217: 5-(3,4,5-trifluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3,4,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.00 (s, 1H), 8.42 (d, $J$ = 5.1 Hz, 1H), 8.11 (s, 1H), 7.45 - 7.28 (m, 2H), 7.28 - 7.15 (m, 2H), 6.93 (dd, $J$ = 8.4, 2.7 Hz, 1H), 6.81 (d, $J$ = 2.7 Hz, 1H), 4.26 - 4.13 (m, 3H), 3.96 (t, $J$ = 6.5 Hz, 2H), 2.19 (s, 3H), 1.81 - 1.67 (m, 2H), 1.52 - 1.41 (m, 2H), 1.09 (s, 6H). MS: 540 [M+H]$^+$.

Example 218: 5-(2,4,5-trifluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(2,4,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 9.92 (s, 1H), 8.42 (d, $J$ = 5.1 Hz, 1H), 8.10 (s, 1H), 7.73 - 7.46 (m, 2H), 7.30 - 7.14 (m, 2H), 6.93 (dd, $J$ = 8.4, 2.7 Hz, 1H), 6.80 (d, $J$ = 2.7 Hz, 1H), 4.24 - 4.13 (m, 3H), 3.96 (t, $J$ = 6.5 Hz, 2H), 2.19 (s, 3H), 1.81 - 1.67 (m, 2H), 1.53 - 1.40 (m, 2H), 1.09 (s, 6H). MS: 540 [M+H]$^+$.

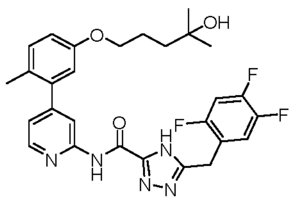
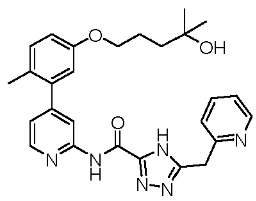

Example 218 Example 219

Example 219: N-(4-(5-((4-hydroxy-4-methylpentyl)oxy)-2-methylphenyl)pyridin-2-yl)-5-(pyridin-2-ylmethyl)-4H-1,2,4-triazole-3-carboxamide

The preparation was carried out in a similar manner to Example 160, except that in step 4, ethyl 5-(pyridin-2-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 9.89 (s, 1H), 8.54 - 8.45 (m, 1H), 8.41 (d, $J$ = 5.1 Hz, 1H), 8.11 (s, 1H), 7.84 - 7.75 (m, 1H), 7.43 (d, $J$ = 7.8 Hz, 1H), 7.34 - 7.17 (m, 3H), 6.93 (dd, $J$ = 8.4, 2.7 Hz, 1H), 6.80 (d, $J$ = 2.7 Hz, 1H), 4.34 (s, 2H), 4.17 (s, 1H), 3.96 (t, $J$ = 6.5 Hz, 2H), 2.19 (s, 3H), 1.82 - 1.68 (m, 2H), 1.54 - 1.40 (m, 2H), 1.09 (s, 6H). MS: 487 [M+H]$^+$.

Example 220: N-(4-(5-((4-hydroxy-4-methylpentyl)oxy)-2-methylphenyl)pyridin-2-yl)-5-(pyridin-3-ylmethyl)-4H-1,2,4-triazole-3-carboxamide

The preparation was carried out in a similar manner to Example 160, except that in step 4, ethyl 5-(pyridin-3-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.66 (s, 1H), 9.95 (s, 1H), 8.58 (d, $J$ = 2.2 Hz, 1H), 8.48 (dd, $J$ = 4.8, 1.7 Hz, 1H), 8.41 (d, $J$ = 5.1 Hz, 1H), 8.13 - 8.09 (m, 1H), 7.82 - 7.70 (m, 1H), 7.43 - 7.32 (m, 1H), 7.28 - 7.17 (m, 2H), 6.93 (dd, $J$ = 8.4, 2.7 Hz, 1H), 6.80 (d, $J$ = 2.8 Hz, 1H), 4.22 (s, 2H), 4.17 (s, 1H), 3.96 (t, $J$ = 6.6 Hz, 2H), 2.19 (s, 3H), 1.81 - 1.69 (m, 2H), 1.54 - 1.42 (m, 2H), 1.09 (s, 6H). MS: 487 [M+H]$^+$.

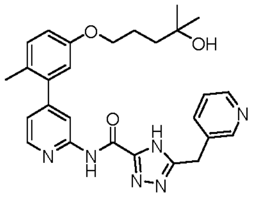
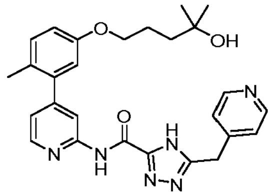

Example 220 Example 221

Example 221: N-(4-(5-((4-hydroxy-4-methylpentyl)oxy)-2-methylphenyl)pyridin-2-yl)-5-(pyridin-4-ylmethyl)-4H-1,2,4-triazole-3-carboxamide

The preparation was carried out in a similar manner to Example 160, except that in step 4, ethyl 5-(pyridin-4-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.63 (s, 1H), 9.90 (s, 1H), 8.53 (d, $J$ = 5.0 Hz, 2H), 8.42 (d, $J$ = 5.2 Hz, 1H), 8.11 (s, 1H), 7.42 - 7.29 (m, 2H), 7.29 - 7.15 (m, 2H), 6.93 (dd, $J$ = 8.4, 2.7 Hz, 1H), 6.81 (d, $J$ = 2.7 Hz, 1H), 4.29 - 4.20 (m, 2H), 4.17 (s, 1H), 3.96 (t, $J$ = 6.6 Hz, 2H), 2.19 (s, 3H), 1.83 - 1.68 (m, 2H), 1.55 - 1.42 (m, 2H), 1.09 (s, 6H). MS: 487 [M+H]$^+$.

Example 222: 5-(cycloheptylmethyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(cycloheptylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 9.85 (s, 1H), 8.42 (dd, $J$ = 5.0, 0.8 Hz, 1H), 8.13 (s, 1H), 7.33 - 7.15 (m, 2H), 6.93 (dd, $J$ = 8.4, 2.7 Hz, 1H), 6.81 (d, $J$ = 2.7 Hz, 1H), 4.17 (s, 1H), 3.97 (t, $J$ = 6.6 Hz, 2H), 2.69 (d, $J$ = 7.3 Hz, 2H), 2.19 (s, 3H), 2.07 - 1.91 (m, 1H), 1.82 - 1.70 (m, 2H), 1.70 - 1.30 (m, 12H), 1.30 - 1.15 (m, 2H), 1.09 (s, 6H). MS: 506 [M+H]$^+$.

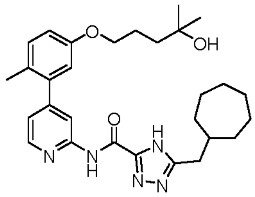
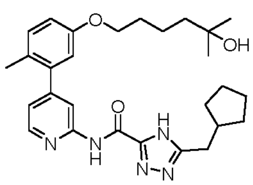

Example 222 Example 223

Example 223: 5-(cyclopentylmethyl)-N-(4-(5-((5-hydroxyl-5-methylhexyl)oxy)-2-methylphenyl)pyridin-2-yl)-4H-1,2,4-triazole-3-carboxamide

The preparation was carried out in a similar manner to Example 160, except that in step 1, methyl 5-bromopentanoate was used in place of methyl 4-bromobutanoate, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 9.83 (s, 1H), 8.42 (d, $J$ = 5.2 Hz, 1H), 8.14 (s, 1H), 7.29 - 7.16 (m, 2H), 6.94 (dd, $J$ = 8.4, 2.7 Hz, 1H), 6.82 (d, $J$ = 2.7 Hz, 1H), 4.09 (s, 1H), 3.98 (t, $J$ = 6.5 Hz, 2H), 2.79 (d, $J$ = 7.5 Hz, 2H), 2.37 - 2.22 (m, 1H), 2.19 (s, 3H), 1.82 - 1.65 (m, 4H), 1.65 - 1.57 (m, 2H), 1.57 - 1.47 (m, 2H), 1.47 - 1.32 (m, 4H), 1.30 - 1.17 (m, 2H), 1.06 (s, 6H). MS: 492 [M+H]$^+$.

Example 224: 5-(cyclopentylmethyl)-N-(4-(5-((5-hydroxypentyl)oxy)-2-methylphenyl)pyridin-2-yl)-4H-1,2,4-triazole-3-carboxamide

The preparation was carried out in a similar manner to Example 92, except that in step 1, 5-bromo-1-pentanol was used in place of iodomethane, 3-bromo-4-methylphenol was used in place of 2-bromo-3-methylphenol, and in step 3, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.36 (s, 1H), 9.83 (s, 1H), 8.42 (d, *J*= 5.1 Hz, 1H), 8.14 (s, 1H), 7.29 - 7.16 (m, 2H), 6.93 (dd, *J*= 8.4, 2.7 Hz, 1H), 6.81 (d, *J* = 2.7 Hz, 1H), 4.37 (t, *J* = 5.1 Hz, 1H), 3.97 (t, *J* = 6.5 Hz, 2H), 3.51 - 3.36 (m, 2H), 2.79 (d, *J*= 7.5 Hz, 2H), 2.37 - 2.22 (m, 1H), 2.19 (s, 3H), 1.79 - 1.66 (m, 4H), 1.66 - 1.56 (m, 2H), 1.56 - 1.36 (m, 6H), 1.30 - 1.15 (m, 2H). MS: 464 [M+H]$^+$.

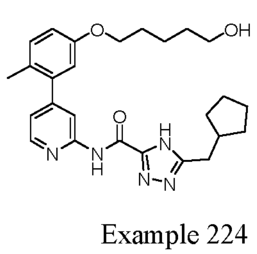

Example 224

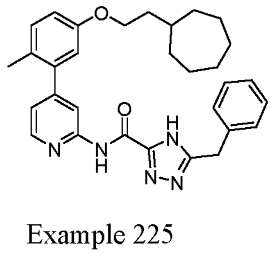

Example 225

Example 225: 5-benzyl-N-(4-(5-(2-cycloheptylethoxy)-2-methylphenyl)pyridin-2-yl)-4H-1,2,4-triazole-3-carboxamide

The preparation was carried out in a similar manner to Example 64, except that in step 1, 2-cycloheptyl-1-ethanol was used in place of 2-(tert-butoxy)ethan-1-ol. $^1$H NMR (400 MHz, DMSO-$d_6$) δ 14.56 (s, 1H), 9.88 (s, 1H), 8.41 (d, *J* = 5.1 Hz, 1H), 8.11 (s, 1H), 7.44-7.30 (m, 4H), 7.30-7.14 (m, 3H), 6.93 (dd, *J* = 8.4, 2.7 Hz, 1H), 6.81 (d, *J* = 2.7 Hz, 1H), 4.18 (s, 2H), 3.99 (t, *J* = 6.3 Hz, 2H), 2.18 (s, 3H), 1.77-1.50 (m, 9H), 1.50 - 1.31 (m, 4H), 1.23 - 1.13 (m, 2H). MS: 510 [M+H]$^+$.

Example 226: 5-(2-fluorobenzyl)-N-(4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 15.08-14.30 (m, 1H), 9.88 (s, 1H), 8.48-8.32 (m, 1H), 8.11 (s, 1H), 7.48-7.29 (m, 2H), 7.29-7.09 (m, 4H), 6.93 (d, *J* = 8.5 Hz, 1H), 6.82 (s, 1H), 4.21 (s, 2H), 4.01 (t, *J* = 6.4 Hz, 2H), 3.92-3.67 (m, 2H), 3.30-3.14 (m, 2H), 2.18 (s, 3H), 1.80-1.48 (m, 5H), 1.33-1.08 (m, 2H). MS: 516 [M+H]$^+$.

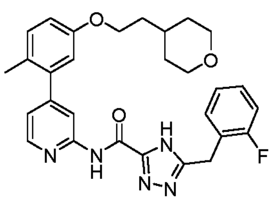

Example 226

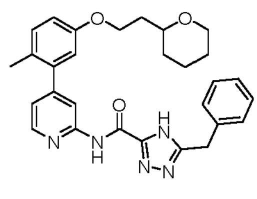

Example 227

Example 227: 5-benzyl-N-(4-(2-methyl-5-(2-(tetrahydro-2H-pyran-2-yl)ethoxy)phenyl)pyridin-2-yl)-4H-1,2,4-triazole-3-carboxamide

The preparation was carried out in a similar manner to Example 92, except that in step 1, 2-(2-bromoethyl)tetrahydro-2H-pyran was used in place of iodomethane, and 3-bromo-4-methylphenol was used in place of 2-bromo-3-methylphenol. $^1$H NMR (400 MHz, DMSO-$d_6$) δ 14.59 (s, 1H), 9.91 (s, 1H), 8.41 (d, *J* = 5.1 Hz, 1H), 8.11 (s, 1H), 7.46 - 7.29 (m, 4H), 7.29 - 7.15 (m, 3H), 6.93 (dd, *J* = 8.4, 2.8 Hz, 1H), 6.81 (d, *J* = 2.8 Hz, 1H), 4.17 (s, 2H), 4.11 - 3.95 (m, 2H), 3.90 - 3.78 (m, 1H), 3.46 - 3.19 (m, 4H), 2.19 (s, 3H), 1.87 - 1.76 (m, 2H), 1.76 - 1.68 (m, 1H), 1.65 - 1.55 (m, 1H), 1.47 - 1.39 (m, 2H). MS: 498 [M+H]$^+$.

Example 228: 5-benzyl-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, and 3-bromo-4-methylphenol was used in place of 2-bromo-3-methylphenol. $^1$H NMR (400 MHz, DMSO-$d_6$) δ 14.59 (s, 1H), 9.91 (s, 1H), 8.41 (d, *J* = 5.2 Hz, 1H), 8.12 (s, 1H), 7.39 - 7.29 (m, 4H), 7.29 - 7.15 (m, 3H), 6.93 (dd, *J* = 8.4, 2.7 Hz, 1H), 6.83 (d, *J* = 2.8 Hz, 1H), 4.17 (s, 2H), 4.07 (t, *J* = 5.9 Hz, 2H), 2.64 (t, *J* = 5.9 Hz, 2H), 2.42 (t, *J* = 5.3 Hz, 4H), 2.19 (s, 3H), 1.55 - 1.41 (m, 4H), 1.41 - 1.27 (m, 2H). MS: 497 [M+H]$^+$.

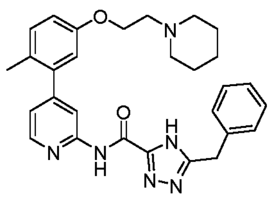
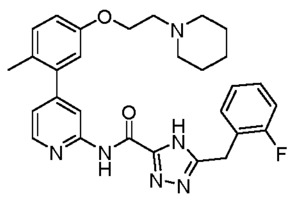

Example 228 Example 229

Example 229: 5-(2-fluorobenzyl)-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.89 (s, 1H), 8.41 (d, *J* = 5.1 Hz, 1H), 8.11 (d, *J* = 1.4 Hz, 1H), 7.36 - 7.11 (m, 6H), 6.95 (dd, *J* = 8.4, 2.7 Hz, 1H), 6.84 (d, *J* = 2.7 Hz, 1H), 4.33 - 4.24 (m, 2H), 4.21 (s, 2H), 4.11 (t, *J* = 5.8 Hz, 2H), 2.75 (t, *J* = 5.8 Hz, 2H), 2.19 (s, 3H), 1.56 - 1.43 (m, 4H), 1.43 - 1.32 (m, 2H), 1.31 - 1.23 (m, 2H). MS: 515 [M+H]$^+$.

Example 230: 5-(2,4-difluorobenzyl)-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, and in step 3, ethyl 5-(2,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.90 (s, 1H), 8.41 (d, *J* = 5.1 Hz, 1H), 8.11 (s, 1H), 7.52 - 7.40 (m, 1H), 7.31 - 7.16 (m, 3H), 7.13 - 7.04 (m, 1H), 6.94 (dd, *J* = 8.4, 2.8 Hz, 1H), 6.83 (d, *J* = 2.8 Hz, 1H), 4.18 (s, 2H), 4.07 (t, *J* = 5.9 Hz, 2H), 2.65 (t, *J* = 5.9 Hz, 2H), 2.47 - 2.35 (m, 4H), 2.19 (s, 3H), 1.55 - 1.41 (m, 4H), 1.41 - 1.28 (m, 2H). MS: 533 [M+H]$^+$.

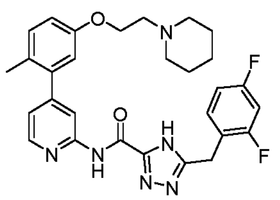
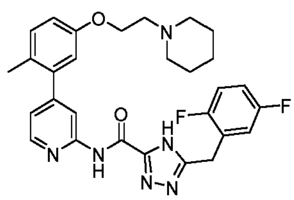

Example 230 Example 231

Example 231: 5-(2,5-difluorobenzyl)-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, and in step 3, ethyl 5-(2,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.93 (s, 1H), 8.40 (d, *J* = 5.1 Hz, 1H), 8.12 (s, 1H), 7.45 - 7.03 (m, 5H), 7.03 - 6.67 (m, 2H), 4.20 (s, 2H), 4.07 (t, *J* = 5.9 Hz, 2H), 2.65 (t, *J* = 5.9 Hz, 2H),

2.50 - 2.33 (m, 4H), 2.18 (s, 3H), 1.62 - 1.41 (m, 4H), 1.41 - 1.21 (m, 2H). MS: 533 $[M+H]^+$.

Example 232: 5-(3,5-difluorobenzyl)-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, and in step 3, ethyl 5-(3,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.54 (s, 1H), 9.99 (s, 1H), 8.42 (d, *J* = 5.1 Hz, 1H), 8.14-8.06 (m, 1H), 7.28-7.05 (m, 5H), 6.94 (dd, *J* = 8.4, 2.7 Hz, 1H), 6.84 (d, *J* = 2.7 Hz, 1H), 4.22 (s, 2H), 4.08 (t, *J* = 5.9 Hz, 2H), 2.66 (t, *J* = 5.9 Hz, 2H), 2.47-2.36 (m, 4H), 2.19 (s, 3H), 1.53-1.42 (m, 4H), 1.42-1.30 (m, 2H). MS: 533 $[M+H]^+$.

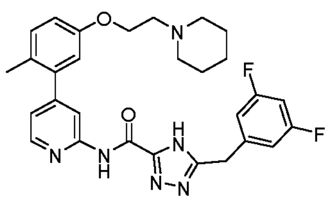

Example 232

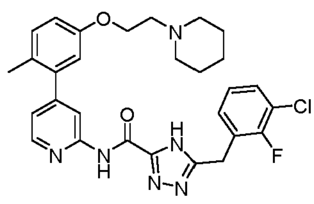

Example 233

Example 233: 5-(2-fluoro-3-chlorobenzyl)-N-(4-(2-methyl-5-(2-(piperidin-1-yl)ethoxy)phenyl)pyridin-2-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, and in step 3, ethyl 5-(2-fluoro-3-chlorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 9.93 (s, 1H), 8.41 (d, *J* = 5.1 Hz, 1H), 8.10 (s, 1H), 7.57 - 7.48 (m, 1H), 7.43 - 7.36 (m, 1H), 7.28 - 7.17 (m, 3H), 6.94 (dd, *J* = 8.4, 2.7 Hz, 1H), 6.83 (d, *J* = 2.7 Hz, 1H), 4.26 (s, 2H), 4.08 (t, *J* = 5.9 Hz, 2H), 2.66 (t, *J* = 5.8 Hz, 2H), 2.46 - 2.38 (m, 4H), 2.19 (s, 3H), 1.53 - 1.43 (m, 4H), 1.37 (t, *J* = 6.2 Hz, 2H). MS: 549 $[M+H]^+$.

Example 234: 5-(2-fluorobenzyl)-N-(4-(2-methyl-5-((1-methylpiperidin-4-yl)methoxy)phenyl)pyridin-2-yl)-4H-1,2,4-triazole-3-carboxamide

The preparation was carried out in a similar manner to Example 92, except that in step 1, 4-(chloromethyl)-1-methylpiperidine hydrochloride was used in place of iodomethane, 3-bromo-4-methylphenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.11 (s, 1H), 9.90 (s, 1H), 8.41 (d, *J* = 5.1 Hz, 1H), 8.10 (s, 1H), 7.44 - 7.30 (m, 2H), 7.28 - 7.14 (m, 4H), 6.93 (dd, *J* = 8.4, 2.7 Hz, 1H), 6.81 (d, *J* = 2.7 Hz, 1H), 4.19 (s, 2H), 3.97 (t, *J* = 6.6 Hz, 1H), 3.86 - 3.79 (m, 1H), 2.82 - 2.66 (m, 2H), 2.27 (s, 1H), 2.18 (s, 3H), 2.16 (s, 3H), 2.01-1.83 (m, 2H), 1.79 - 1.67 (m, 2H), 1.47 - 1.26 (m, 2H). MS: 515 $[M+H]^+$.

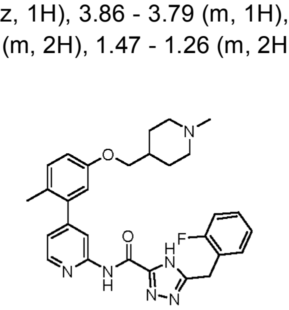

Example 234

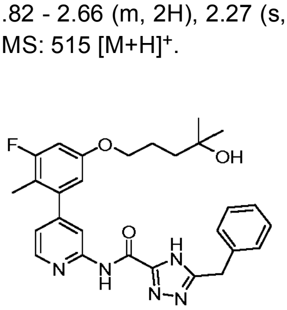

Example 235

Example 235: 5-benzyl-N-(4-(3-fluoro-5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 1, 3-bromo-5-fluoro-4-methylphenol was used in place of 3-bromo-4-methylphenol. $^1$H NMR (400 MHz, DMSO-$d_6$) $\delta$ 14.56 (s, 1H), 9.90 (s, 1H), 8.44 (d, *J* = 5.1 Hz, 1H), 8.11 (s, 1H), 7.39-7.30 (m, 4H), 7.24 (dd, *J* = 20.1, 5.8 Hz, 2H), 6.91 (dd, *J* = 11.7, 2.5 Hz, 1H), 6.70 (d, *J* = 2.1 Hz, 1H), 4.18 (d, *J* = 5.4 Hz, 3H), 3.99 (t, *J* = 6.6 Hz, 2H), 2.12-2.04 (m, 3H), 1.79-1.68 (m, 2H), 1.53-1.39 (m, 2H), 1.09 (s, 6H). MS: 504 [M+H]$^+$.

Example 236: 5-(2-fluorobenzyl)-N-(4-(3-fluoro-5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 1, 3-bromo-5-fluoro-4-methylphenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.67 (s, 1H), 9.92 (s, 1H), 8.43 (d, *J* = 5.1 Hz, 1H), 8.09 (s, 1H), 7.52 - 7.29 (m, 2H), 7.29 - 7.14 (m, 3H), 6.90 (dd, *J* = 11.7, 2.5 Hz, 1H), 6.70 (d, *J* = 2.5 Hz, 1H), 4.21 (s, 2H), 4.17 (s, 1H), 3.99 (t, *J* = 6.5 Hz, 2H), 2.07 (d, *J* = 2.2 Hz, 3H), 1.81 - 1.70 (m, 2H), 1.51 - 1.37 (m, 2H), 1.09 (s, 6H). MS: 522 [M+H]$^+$.

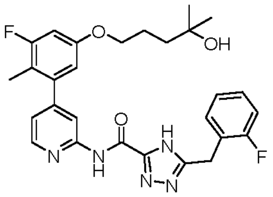
Example 236

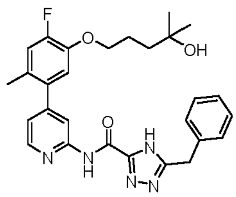
Example 237

Example 237: 5-benzyl-N-(4-(4-fluoro-5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 1, 5-bromo-2-fluoro-4-methylphenol was used in place of 3-bromo-4-methylphenol. $^1$H NMR (400 MHz, DMSO-$d_6$) $\delta$ 14.57 (s, 1H), 9.89 (s, 1H), 8.42 (d, *J* = 5.1 Hz, 1H), 8.10 (s, 1H), 7.40 - 7.29 (m, 4H), 7.29 - 7.15 (m, 3H), 7.03 (d, *J* = 8.7 Hz, 1H), 4.23 - 4.11 (m, 3H), 4.04 (t, *J* = 6.6 Hz, 2H), 2.19 (s, 3H), 1.76 (dd, *J* = 10.3, 6.0 Hz, 2H), 1.53 - 1.41 (m, 2H), 1.09 (s, 6H). MS: 504 [M+H]$^+$.

Example 238: N-(5-chloro-4-(5-((4-hydroxy-4-methylpentyl)oxy)-2-methylphenyl)pyridin-2-yl)-5-(2-fluorobenzyl)-4H-1,2,4-triazole-3-carboxamide

The preparation was carried out in a similar manner to Example 160, except that in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.60 (s, 1H), 10.06 (s, 1H), 8.54 (s, 1H), 8.05 (s, 1H), 7.44 - 7.30 (m, 2H), 7.28 - 7.16 (m, 3H), 6.95 (dd, *J* = 8.4, 2.7 Hz, 1H), 6.74 (d, *J* = 2.7 Hz, 1H), 4.21 (s, 2H), 4.16 (s, 1H), 3.95 (t, *J* = 6.6 Hz, 2H), 2.01 (s, 3H), 1.80 - 1.67 (m, 2H), 1.51 - 1.41 (m, 2H), 1.09 (s, 6H). MS: 538 [M+H]$^+$.

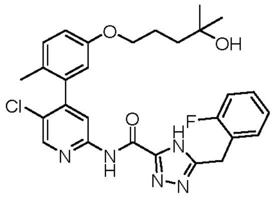
Example 238

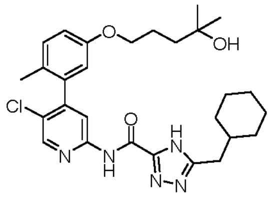
Example 239

Example 239: N-(5-chloro-4-(5-((4-hydroxy-4-methylpentyl)oxy)-2-methylphenyl)pyridin-2-yl)-5-(cyclohexylmethyl)-4H-1,2,4-triazole-3-carboxamide

The preparation was carried out in a similar manner to Example 160, except that in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 10.06 (s, 1H), 8.55 (s, 1H), 8.08 (s, 1H), 7.26 (d, *J* = 8.5 Hz, 1H), 6.95 (dd, *J* = 8.4, 2.7 Hz, 1H), 6.75 (d, *J* = 2.7 Hz, 1H), 4.17 (s, 1H), 3.95 (t, *J* = 6.6 Hz, 2H), 2.66 (d, *J* = 7.1 Hz, 2H), 2.01 (s, 3H), 1.84 - 1.56 (m, 9H), 1.52 - 1.41 (m, 2H), 1.20 - 1.12 (m, 2H), 1.09 (s, 6H), 1.05 - 0.90 (m, 2H). MS: 526 [M+H]$^+$.

Example 240: 5-(2-fluorobenzyl)-N-(4-(5-((4-hydroxy-4-methylpentyl)oxy)-2-methylphenyl)-5-methylpyridin-2-yl)-4H-1,2,4-triazole-3-carboxamide

The preparation was carried out in a similar manner to Example 160, except that in step 3, 4-bromo-5-methylpyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 9.75 (s, 1H), 8.30 (s, 1H), 7.87 (s, 1H), 7.44 - 7.29 (m, 2H), 7.29 - 7.14 (m, 3H), 6.90 (dd, *J* = 8.4, 2.7 Hz, 1H), 6.66 (d, *J* = 2.7 Hz, 1H), 4.20 (s, 2H), 4.17 (s, 1H), 3.94 (t, *J* = 6.6 Hz, 2H), 1.99 (s, 3H), 1.94 (s, 3H), 1.79 - 1.68 (m, 2H), 1.52 - 1.39 (m, 2H), 1.08 (s, 6H). MS: 518 [M+H]$^+$.

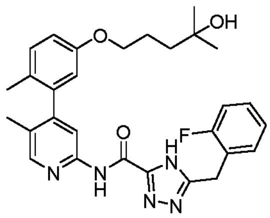

Example 240

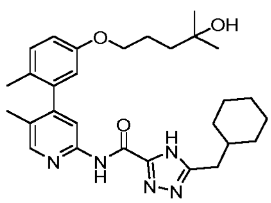

Example 241

Example 241: 5-(cyclohexylmethyl)-N-(4-(5-((4-hydroxy-4-methylpentyl)oxy)-2-methylphenyl)-5-methylpyridin-2-yl)-4H-1,2,4-triazole-3-carboxamide

The preparation was carried out in a similar manner to Example 160, except that in step 3, 4-bromo-5-methylpyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.34 (s, 1H), 9.74 (s, 1H), 8.31 (s, 1H), 7.90 (s, 1H), 7.24 (d, *J* = 8.4 Hz, 1H), 6.91 (dd, *J* = 8.4, 2.7 Hz, 1H), 6.66 (d, *J* = 2.7 Hz, 1H), 4.18 (s, 1H), 3.94 (t, *J* = 6.6 Hz, 2H), 2.66 (d, *J* = 7.2 Hz, 2H), 2.00 (s, 3H), 1.95 (s, 3H), 1.82 - 1.57 (m, 8H), 1.52 - 1.40 (m, 2H), 1.24 - 1.13 (m, 3H), 1.08 (s, 6H), 1.05 - 0.90 (m, 2H). MS: 506 [M+H]$^+$.

Example 242: N-(4-(5-((4-ethyl-4-hydroxyhexyl)oxy)-2-methylphenyl)-5-fluoropyridin-2-yl)-5-(2-fluorobenzyl)-4H-1,2,4-triazole-3-carboxamide

The preparation was carried out in a similar manner to Example 160, except that in step 2, ethylmagnesium bromide was used in place of methylmagnesium bromide, in step 3, 5-fluoro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 10.08 (s, 1H), 8.47 (s, 1H), 8.06 (d, *J*= 5.6 Hz, 1H), 7.45 - 7.30 (m, 2H), 7.30 - 7.14 (m, 3H), 6.97 (dd, *J* = 8.5, 2.7 Hz, 1H), 6.83 (d, *J* = 2.7 Hz, 1H), 4.20 (s, 2H), 3.95 (t, *J* = 6.5 Hz, 2H), 3.87 (s, 1H), 2.09 (s, 3H), 1.73 - 1.59 (m, 2H), 1.46 - 1.38 (m, 2H), 1.34 (q, *J* = 7.4 Hz, 4H), 0.77 (t, *J* = 7.4 Hz, 6H). MS: 550 [M+H]$^+$.

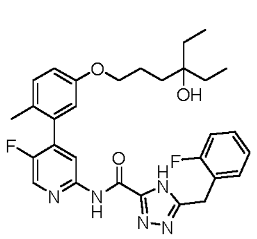 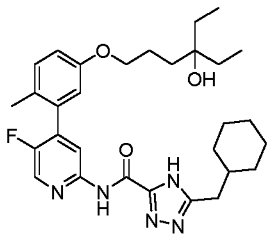

Example 242 Example 243

Example 243: 5-(cyclohexylmethyl)-N-(4-(5-((4-ethyl-4-hydroxyhexyl)oxy)-2-methylphenyl)-5-fluoropyridin-2-yl)-4H-1,2,4-triazole-3-carboxamide

The preparation was carried out in a similar manner to Example 160, except that in step 2, ethylmagnesium bromide was used in place of methylmagnesium bromide, in step 3, 5-fluoro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.02 (s, 1H), 8.48 (d, $J$ = 1.2 Hz, 1H), 8.09 (d, $J$ = 5.6 Hz, 1H), 7.27 (d, $J$ = 8.5 Hz, 1H), 6.97 (dd, $J$ = 8.4, 2.7 Hz, 1H), 6.84 (d, $J$ = 2.7 Hz, 1H), 3.96 (t, $J$ = 6.5 Hz, 2H), 3.87 (s, 1H), 2.66 (d, $J$ = 7.0 Hz, 2H), 2.10 (s, 3H), 1.81 - 1.55 (m, 8H), 1.46 - 1.38 (m, 2H), 1.34 (q, $J$ = 7.4 Hz, 4H), 1.26 - 1.06 (m, 3H), 1.03 - 0.90 (m, 2H), 0.77 (t, $J$ = 7.4 Hz, 6H). MS: 538 $[M+H]^+$.

Example 244: N-(5-chloro-4-(5-((4-ethyl-4-hydroxyhexyl)oxy)-2-methylphenyl)pyridin-2-yl)-5-(2-fluorobenzyl)-4H-1,2,4-triazole-3-carboxamide

The preparation was carried out in a similar manner to Example 160, except that in step 2, ethylmagnesium bromide was used in place of methylmagnesium bromide, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 10.10 (s, 1H), 8.54 (s, 1H), 8.05 (s, 1H), 7.45 - 7.30 (m, 2H), 7.30 - 7.14 (m, 3H), 6.94 (dd, $J$ = 8.4, 2.7 Hz, 1H), 6.74 (d, $J$ = 2.7 Hz, 1H), 4.20 (s, 2H), 3.95 (t, $J$ = 6.5 Hz, 2H), 3.86 (s, 1H), 2.00 (s, 3H), 1.73 - 1.60 (m, 2H), 1.46 - 1.38 (m, 2H), 1.34 (q, $J$ = 7.4 Hz, 4H), 0.77 (t, $J$ = 7.4 Hz, 6H). MS: 566 $[M+H]^+$.

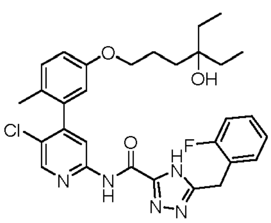 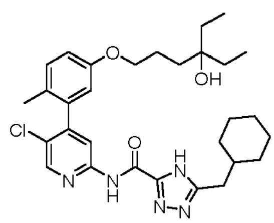

Example 244 Example 245

Example 245: N-(5-chloro-4-(5-((4-ethyl-4-hydroxyhexyl)oxy)-2-methylphenyl)pyridin-2-yl)-5-(cyclohexylmethyl)-4H-1,2,4-triazole-3-carboxamide

The preparation was carried out in a similar manner to Example 160, except that in step 2, ethylmagnesium bromide was used in place of methylmagnesium bromide, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 10.03 (s, 1H), 8.55 (s, 1H), 8.08 (s, 1H), 7.26 (d, $J$ = 8.5 Hz, 1H), 6.95 (dd, $J$ = 8.5, 2.7 Hz, 1H), 6.74 (d, $J$ = 2.7 Hz, 1H), 3.95 (t, $J$ = 6.6 Hz, 2H), 3.87 (s, 1H), 2.71 - 2.62 (m, 2H), 2.01 (s, 3H), 1.81 - 1.71 (m, 1H), 1.71 - 1.56 (m, 7H), 1.46 - 1.38 (m, 2H), 1.34 (q, $J$ = 7.4 Hz, 4H), 1.29 - 1.14 (m, 3H), 1.03 - 0.90 (m, 2H), 0.77 (t, $J$ = 7.5 Hz, 6H). MS: 554 $[M+H]^+$.

Example 246: 5-(cyclohexylmethyl)-N-(4-(5-((4-ethyl-4-hydroxyhexyl)oxy)-2-methylphenyl)-5-methylpyridin-2-yl)-4H-1,2,4-triazole-3-carboxamide

The preparation was carried out in a similar manner to Example 160, except that in step 2, ethylmagnesium bromide was used in place of methylmagnesium bromide, in step 3, 5-methyl-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.33 (s, 1H), 9.74 (s, 1H), 8.31 (s, 1H), 7.90 (s, 1H), 7.25 (d, $J$ = 8.5 Hz, 1H), 6.91 (dd, $J$ = 8.4, 2.7 Hz, 1H), 6.66 (d, $J$ = 2.7 Hz, 1H), 3.94 (t, $J$ = 6.5 Hz, 2H), 3.87 (s, 1H), 2.71 - 2.61 (m, 2H), 2.00 (s, 3H), 1.95 (s, 3H), 1.83 - 1.55 (m, 8H), 1.44 - 1.37 (m, 2H), 1.34 (q, $J$ = 7.4 Hz, 4H), 1.23 - 1.09 (m, 3H), 1.04 - 0.91 (m, 2H), 0.77 (t, $J$ = 7.4 Hz, 6H). MS: 534 [M+H]$^+$.

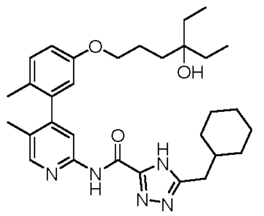

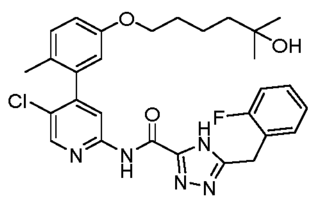

Example 246 Example 247

Example 247: N-(5-chloro-4-(5-((5-hydroxyl-5-methylhexyl)oxy)-2-methylphenyl)pyridin-2-yl)-5-(2-fluorobenzyl)-4H-1,2,4-triazole-3-carboxamide

The preparation was carried out in a similar manner to Example 160, except that in step 1, methyl 5-bromopentanoate was used in place of methyl 4-bromobutanoate, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.59 (s, 1H), 10.11 (s, 1H), 8.53 (s, 1H), 8.05 (s, 1H), 7.45 - 7.29 (m, 2H), 7.29 - 7.12 (m, 3H), 6.95 (d, $J$ = 9.0 Hz, 1H), 6.75 (d, $J$ = 2.7 Hz, 1H), 4.19 (s, 2H), 4.09 (s, 1H), 3.95 (t, $J$ = 6.5 Hz, 2H), 2.00 (s, 3H), 1.74 - 1.59 (m, 2H), 1.51 - 1.37 (m, 4H), 1.06 (s, 6H). MS: 552 [M+H]$^+$.

Example 248: 5-(cyclohexylmethyl)-N-(5-chloro-4-(5-((5-hydroxyl-5-methylhexyl)oxy)-2-methylphenyl)pyridin-2-yl)-4H-1,2,4-triazole-3-carboxamide

The preparation was carried out in a similar manner to Example 160, except that in step 1, methyl 5-bromopentanoate was used in place of methyl 4-bromobutanoate, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 10.04 (s, 1H), 8.55 (s, 1H), 8.08 (s, 1H), 7.26 (d, $J$ = 8.5 Hz, 1H), 6.96 (dd, $J$ = 8.4, 2.7 Hz, 1H), 6.76 (d, $J$ = 2.7 Hz, 1H), 4.09 (s, 1H), 3.96 (t, $J$ = 6.5 Hz, 2H), 2.66 (d, $J$ = 6.9 Hz, 2H), 2.01 (s, 3H), 1.65 (dd, $J$ = 13.9, 9.1 Hz, 8H), 1.49 - 1.35 (m, 4H), 1.22 - 1.10 (m, 3H), 1.06 (s, 6H), 1.04 - 0.94 (m, 2H). MS: 540 [M+H]$^+$.

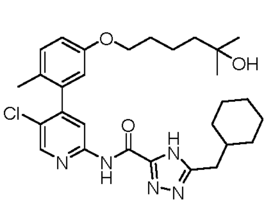

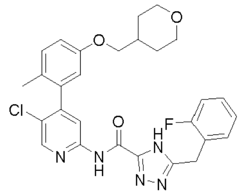

Example 248 Example 249

Example 249: N-(5-chloro-4-(2-methyl-5-((tetrahydro-2H-pyran-4-yl)methoxy)phenyl)pyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 5-chloro-4-bromopyridin-2-amine was used in

place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 10.05 (s, 1H), 8.53 (s, 1H), 8.06 (s, 1H), 7.47 - 7.29 (m, 2H), 7.29 - 7.11 (m, 3H), 6.96 (dd, *J* = 8.5, 2.8 Hz, 1H), 6.76 (d, *J* = 2.7 Hz, 1H), 4.23 (s, 2H), 3.92 - 3.78 (m, 4H), 3.32 - 3.26 (m, 2H), 2.01 (s, 3H), 1.99 - 1.92 (m, 1H), 1.73 - 1.59 (m, 2H), 1.37 - 1.26 (m, 2H). MS: 536 $[M+H]^+$.

Example 250: N-(5-chloro-4-(2-methyl-5-((tetrahydro-2H-pyran-4-yl)methoxy)phenyl)pyridin-2-yl)-5-(cyclohexylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 5-chloro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 10.07 (s, 1H), 8.55 (s, 1H), 8.08 (s, 1H), 7.26 (d, *J* = 8.5 Hz, 1H), 6.97 (dd, *J* = 8.5, 2.7 Hz, 1H), 6.77 (d, *J* = 2.7 Hz, 1H), 3.92 - 3.77 (m, 4H), 2.66 (d, *J* = 7.1 Hz, 2H), 2.02 (s, 3H), 1.97 (dd, *J* = 9.5, 5.2 Hz, 1H), 1.82 - 1.56 (m, 8H), 1.39 - 1.13 (m, 7H), 1.04 - 0.91 (m, 2H). MS: 524 $[M+H]^+$.

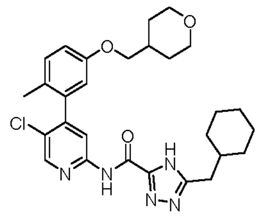

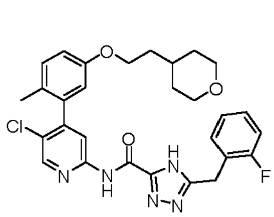

Example 250 Example 251

Example 251: N-(5-chloro-4-(2-methyl-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.12 (s, 1H), 8.54 (s, 1H), 8.05 (s, 1H), 7.44 - 7.29 (m, 2H), 7.28 - 7.15 (m, 3H), 6.96 (dd, *J* = 8.4, 2.7 Hz, 1H), 6.77 (d, *J* = 2.7 Hz, 1H), 4.20 (s, 2H), 4.00 (t, *J* = 6.3 Hz, 2H), 3.87 - 3.75 (m, 2H), 3.31 - 3.21 (m, 2H), 2.00 (s, 3H), 1.76 - 1.55 (m, 5H), 1.27 - 1.11 (m, 2H). MS: 550 $[M+H]^+$.

Example 252: N-(5-chloro-4-(2-methyl-5-(2-(tetrahydro-2H-pyran-4-yl)ethoxy)phenyl)pyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 10.06 (s, 1H), 8.55 (s, 1H), 8.08 (s, 1H), 7.26 (d, *J* = 8.5 Hz, 1H), 6.96 (dd, *J* = 8.4, 2.7 Hz, 1H), 6.77 (d, *J* = 2.7 Hz, 1H), 4.01 (t, *J* = 6.3 Hz, 2H), 3.86 - 3.76 (m, 2H), 3.30 - 3.20 (m, 2H), 2.71 - 2.61 (m, 2H), 2.01 (s, 3H), 1.82 - 1.55 (m, 11H), 1.29 - 1.08 (m, 5H), 1.05 - 0.89 (m, 2H). MS: 538 $[M+H]^+$.

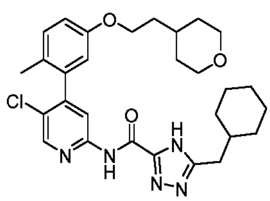

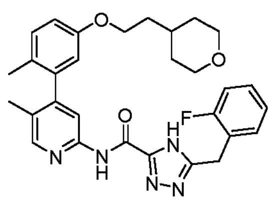

Example 252 Example 253

Example 253: 5-(2-fluorobenzyl)-N-(5-methyl-4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-methyl-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.76 (s, 1H), 8.30 (s, 1H), 7.87 (s, 1H), 7.46 - 7.28 (m, 2H), 7.28 - 7.12 (m, 3H), 6.91 (dd, *J* = 8.4, 2.7 Hz, 1H), 6.68 (d, *J* = 2.7 Hz, 1H), 4.20 (s, 2H), 3.99 (t, *J* = 6.3 Hz, 2H), 3.86 - 3.75 (m, 2H), 3.30 - 3.19 (m, 2H), 1.99 (s, 3H), 1.94 (s, 3H), 1.72 - 1.54 (m, 5H), 1.25 - 1.17 (m, 2H). MS: 530 [M+H]$^+$.

Example 254: 5-(cyclohexylmethyl)-N-(5-methyl-4-(2-methyl-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-methyl-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.34 (s, 1H), 9.74 (s, 1H), 8.31 (s, 1H), 7.91 (s, 1H), 7.24 (d, *J* = 8.5 Hz, 1H), 6.92 (dd, *J* = 8.5, 2.7 Hz, 1H), 6.69 (d, *J* = 2.7 Hz, 1H), 4.00 (t, *J* = 6.3 Hz, 2H), 3.81 (dd, *J* = 11.8, 4.3 Hz, 2H), 3.26 (t, *J* = 11.6 Hz, 2H), 2.66 (d, *J* = 7.1 Hz, 2H), 2.00 (s, 3H), 1.95 (s, 3H), 1.81 - 1.52 (m, 11H), 1.25 - 1.10 (m, 5H), 1.05 - 0.87 (m, 2H). MS: 518 [M+H]$^+$.

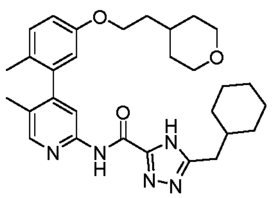
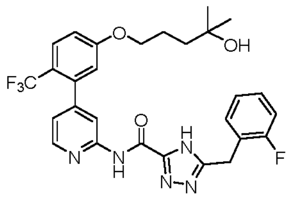

Example 254 Example 255

Example 255: 5-(2-fluorobenzyl)-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 9.94 (s, 1H), 8.43 (d, *J* = 5.1 Hz, 1H), 8.15 (s, 1H), 7.79 (d, *J* = 8.9 Hz, 1H), 7.45 - 7.29 (m, 2H), 7.26 - 7.12 (m, 4H), 6.96 (d, *J* = 2.6 Hz, 1H), 4.31 - 4.14 (m, 3H), 4.09 (t, *J* = 6.6 Hz, 2H), 1.86 - 1.67 (m, 2H), 1.53 - 1.39 (m, 2H), 1.09 (s, 6H). MS: 558 [M+H]$^+$.

Example 256: 5-(3-fluorobenzyl)-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 4, ethyl 5-(3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 10.00 (s, 1H), 8.44 (d, *J* = 5.1 Hz, 1H), 8.16 (s, 1H), 7.79 (d, *J* = 8.8 Hz, 1H), 7.44 - 7.33 (m, 1H), 7.24 - 7.05 (m, 5H), 6.97 (d, *J* = 2.5 Hz, 1H), 4.20 (d, *J* = 6.3 Hz, 3H), 4.09 (t, *J* = 6.6 Hz, 2H), 1.84 - 1.69 (m, 2H), 1.55 - 1.42 (m, 2H), 1.09 (s, 6H). MS: 558 [M+H]$^+$.

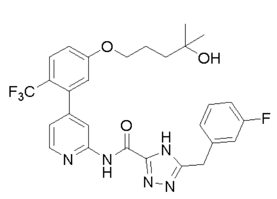

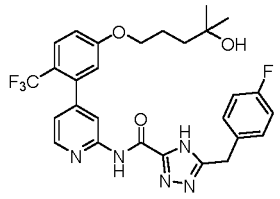

Example 256 Example 257

Example 257: 5-(4-fluorobenzyl)-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 4, ethyl 5-(4-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 9.97 (s, 1H), 8.43 (dd, *J* = 5.1, 0.8 Hz, 1H), 8.16 (s, 1H), 7.79 (d, *J* = 8.9 Hz, 1H), 7.43 - 7.32 (m, 2H), 7.23 - 7.11 (m, 4H), 6.97 (d, *J* = 2.5 Hz, 1H), 4.31 - 4.13 (m, 3H), 4.09 (t, *J* = 6.5 Hz, 2H), 1.86 - 1.69 (m, 2H), 1.54 - 1.41 (m, 2H), 1.09 (s, 6H). MS: 558 $[M+H]^+$.

Example 258: 5-(cyclopentylmethyl)-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.39 (s, 1H), 9.90 (s, 1H), 8.44 (d, *J* = 5.1 Hz, 1H), 8.17 (s, 1H), 7.79 (d, *J* = 8.9 Hz, 1H), 7.26 - 7.13 (m, 2H), 6.97 (d, *J* = 2.6 Hz, 1H), 4.20 (s, 1H), 4.09 (t, *J* = 6.5 Hz, 2H), 2.78 (d, *J* = 7.4 Hz, 2H), 2.37 - 2.19 (m, 1H), 1.85 - 1.66 (m, 4H), 1.66 - 1.56 (m, 2H), 1.56 - 1.41 (m, 4H), 1.26 - 1.20 (m, 2H), 1.09 (s, 6H). MS: 532 $[M+H]^+$.

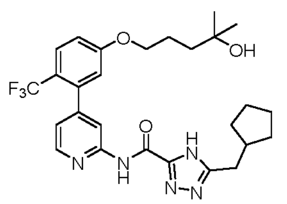

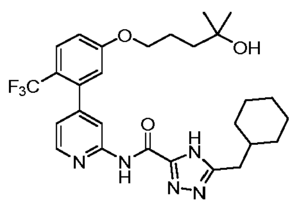

Example 258 Example 259

Example 259: 5-(cyclohexylmethyl)-N-(4-(5-((4-hydroxy-4-methylpentyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 9.89 (s, 1H), 8.44 (d, *J* = 5.1 Hz, 1H), 8.17 (s, 1H), 7.79 (d, *J* = 8.9 Hz, 1H), 7.28 - 7.12 (m, 2H), 6.97 (d, *J* = 2.6 Hz, 1H), 4.19 (s, 1H), 4.09 (t, *J* = 6.6 Hz, 2H), 2.67 (d, *J* = 7.0 Hz, 2H), 1.82 - 1.55 (m, 8H), 1.52 - 1.43 (m, 2H), 1.27 - 1.13 (m, 3H), 1.10 (s, 6H), 1.05 - 0.88 (m, 2H). MS: 546 $[M+H]^+$.

Example 260: 5-benzyl-N-(4-(5-((4-ethyl-4-hydroxyhexyl)oxy)-2-(trifluoromethyl)phenyl)pyridin-2-yl)-4H-1,2,4-triazole-3-carboxamide

The preparation was carried out in a similar manner to Example 160, except that in step 1, 3-chloro-4-(trifluoromethyl)phenol was used in place of 3-bromo-4-methylphenol, and in step 2, ethylmagnesium bromide was used in place of methylmagnesium bromide. $^1$H NMR (400 MHz, DMSO-$d_6$) δ 14.61 (s, 1H), 9.95 (s, 1H), 8.51 - 8.36 (m, 1H), 8.15 (s, 1H), 7.79 (d, *J* = 8.9 Hz, 1H), 7.43 - 7.29 (m, 4H), 7.29 - 7.22 (m, 1H), 7.22 - 7.11 (m, 2H), 6.96 (d, *J* = 2.5 Hz, 1H), 4.17 (s, 2H), 4.08 (t, *J* = 6.5 Hz, 2H), 3.89 (s, 1H), 1.78 - 1.63 (m, 2H), 1.49 - 1.38 (m, 2H), 1.34 (q, *J* = 7.5 Hz, 4H),

0.77 (t, *J* = 7.5 Hz, 6H). MS: 568 [M+H]+.

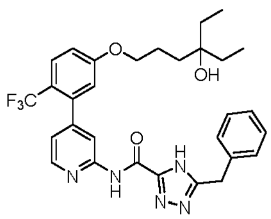

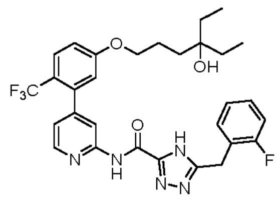

Example 260 Example 261

Example 261: 5-(2-fluorobenzyl)-N-(4-(5-((4-ethyl-4-hydroxyhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, in step 2, ethylmagnesium bromide was used in place of methylmagnesium bromide, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 9.88 (d, *J* = 46.2 Hz, 1H), 8.42 (dd, *J* = 12.1, 5.2 Hz, 1H), 8.15 (s, 1H), 7.80 (dd, *J* = 8.7, 4.7 Hz, 1H), 7.48 - 7.32 (m, 3H), 7.22 - 7.15 (m, 3H), 6.96 (d, *J* = 2.6 Hz, 1H), 4.21 (s, 2H), 4.16 - 4.04 (m, 2H), 3.89 (s, 1H), 1.77 - 1.60 (m, 2H), 1.48 - 1.39 (m, 2H), 1.35 (q, *J* = 7.5 Hz, 4H), 0.78 (t, *J* = 7.4 Hz, 6H). MS: 586 [M+H]+.

Example 262: 5-(cyclopentylmethyl)-N-(4-(5-((4-ethyl-4-hydroxyhexyl)oxy)-2-(trifluoromethyl)phenyl)pyridin-2-yl)-4H-1,2,4-triazole-3-carboxamide

The preparation was carried out in a similar manner to Example 160, except that in step 1, 3-chloro-4-(trifluoromethyl)phenol was used in place of 3-bromo-4-methylphenol, in step 2, ethylmagnesium bromide was used in place of methylmagnesium bromide, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.38 (s, 1H), 9.89 (s, 1H), 8.44 (d, *J* = 5.1 Hz, 1H), 8.17 (s, 1H), 7.79 (d, *J* = 8.9 Hz, 1H), 7.26 - 7.11 (m, 2H), 6.97 (d, *J* = 2.5 Hz, 1H), 4.09 (t, *J* = 6.5 Hz, 2H), 3.89 (s, 1H), 2.78 (d, *J* = 7.5 Hz, 2H), 2.36 - 2.18 (m, 1H), 1.79 - 1.66 (m, 4H), 1.66 - 1.47 (m, 4H), 1.47 - 1.39 (m, 2H), 1.35 (q, *J* = 7.5 Hz, 4H), 1.29 - 1.21 (m, 2H), 0.77 (t, *J* = 7.4 Hz, 6H). MS: 560 [M+H]+.

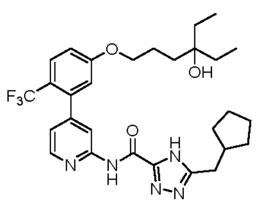

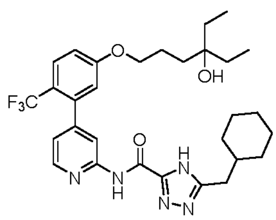

Example 262 Example 263

Example 263: 5-(cyclohexylmethyl)-N-(4-(5-((4-ethyl-4-hydroxyhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, in step 2, ethylmagnesium bromide was used in place of methylmagnesium bromide, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 9.89 (s, 1H), 8.44 (d, *J* = 5.1 Hz, 1H), 8.17 (s, 1H), 7.79 (d, *J* = 8.9 Hz, 1H), 7.28 - 7.13 (m, 2H), 6.97 (d, *J* = 2.6 Hz, 1H), 4.09 (t, *J* = 6.5 Hz, 2H), 3.89 (s, 1H), 2.67 (d, *J* = 7.0 Hz, 2H), 1.83 - 1.56 (m, 8H), 1.51 - 1.39 (m, 2H), 1.35 (q, *J* = 7.5 Hz, 4H), 1.27 - 1.09 (m, 3H), 1.06 - 0.89 (m, 2H), 0.77 (t, *J* = 7.5 Hz, 6H). MS: 574 [M+H]+.

Example 264: 5-benzyl-N-(4-(5-(2-hydroxyl-2-methylpropoxy)-2-(trifluoromethyl)phenyl)pyridin-2-yl)-4H-1,2,4-triazole-3-carboxamide

The preparation was carried out in a similar manner to Example 160, except that in step 1, 3-chloro-4-(trifluoromethyl)phenol was used in place of 3-bromo-4-methylphenol, and methyl bromoacetate was used in place of methyl 4-bromobutanoate. $^1$H NMR (400 MHz, DMSO-$d_6$) δ 14.66 (s, 1H), 9.96 (s, 1H), 8.43 (d, *J* = 5.1 Hz, 1H), 8.16 (s, 1H), 7.79 (d, *J* = 8.8 Hz, 1H), 7.41-7.29 (m, 4H), 7.29 - 7.13 (m, 3H), 6.99 (d, *J* = 2.6 Hz, 1H), 4.69 (s, 1H), 4.17 (s, 2H), 3.85 (s, 2H), 1.20 (s, 6H). MS: 512 $[M+H]^+$.

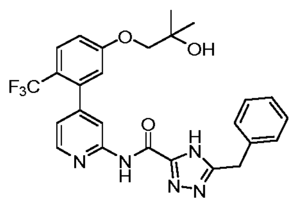
Example 264

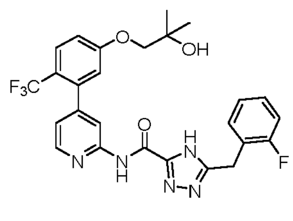
Example 265

Example 265: 5-(2-fluorobenzyl)-N-(4-(5-(2-hydroxyl-2-methylpropoxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl bromoacetate was used in place of methyl 4-bromobutanoate, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.95 (s, 1H), 8.43 (d, *J* = 5.1 Hz, 1H), 8.15 (s, 1H), 7.79 (d, *J* = 8.9 Hz, 1H), 7.48 - 7.29 (m, 2H), 7.29 - 7.13 (m, 4H), 6.99 (d, *J* = 2.6 Hz, 1H), 4.68 (s, 1H), 4.20 (s, 2H), 3.85 (s, 2H), 1.20 (s, 6H). MS: 530 $[M+H]^+$.

Example 266: 5-(cyclopentylmethyl)-N-(4-(5-(2-hydroxyl-2-methylpropoxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl bromoacetate was used in place of methyl 4-bromobutanoate, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.40 (s, 1H), 9.91 (s, 1H), 8.44 (d, *J* = 5.1 Hz, 1H), 8.18 (s, 1H), 7.79 (d, *J* = 8.9 Hz, 1H), 7.27 - 7.14 (m, 2H), 7.00 (d, *J* = 2.6 Hz, 1H), 4.69 (s, 1H), 3.85 (s, 2H), 2.77 (d, *J* = 7.5 Hz, 2H), 2.34 - 2.20 (m, 1H), 1.79 - 1.66 (m, 2H), 1.66 - 1.56 (m, 2H), 1.56 - 1.43 (m, 2H), 1.23 - 1.14 (m, 8H). MS: 504 $[M+H]^+$.

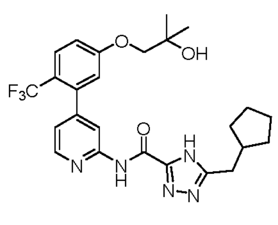
Example 266

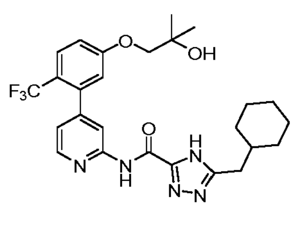
Example 267

Example 267: 5-(cyclohexylmethyl)-N-(4-(5-(2-hydroxyl-2-methylpropoxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl bromoacetate was used in place of methyl 4-bromobutanoate, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 9.91 (s, 1H), 8.49-8.38 (m, 1H), 8.17 (d, *J* = 1.4 Hz, 1H), 7.79 (d, *J* = 8.9 Hz, 1H), 7.29-7.11 (m, 2H), 7.00 (d, *J* = 2.6 Hz, 1H), 4.69 (s, 1H), 3.85 (s, 2H),

2.66 (d, *J* = 7.1 Hz, 2H), 1.85-1.52 (m, 6H), 1.30-1.07 (m, 9H), 1.07-0.90 (m, 2H). MS: 518 $[M+H]^+$.

Example 268: 5-benzyl-N-(4-(5-((5-hydroxyl-5-methylhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and methyl 5-bromopentanoate was used in place of methyl 4-bromobutanoate. $^1$H NMR (400 MHz, DMSO-$d_6$) δ 14.62 (s, 1H), 9.95 (s, 1H), 8.43 (dd, *J* = 5.1, 0.8 Hz, 1H), 8.16 (s, 1H), 7.78 (d, *J* = 8.9 Hz, 1H), 7.44 - 7.29 (m, 4H), 7.29 - 7.22 (m, 1H), 7.22 - 7.10 (m, 2H), 6.98 (d, *J* = 2.6 Hz, 1H), 4.17 (s, 2H), 4.14 - 4.00 (m, 3H), 1.71 (t, *J* = 6.9 Hz, 2H), 1.51 - 1.32 (m, 4H), 1.06 (s, 6H). MS: 554 $[M+H]^+$.

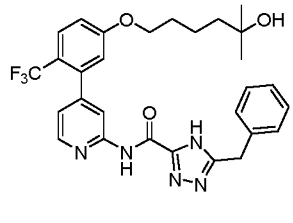
Example 268

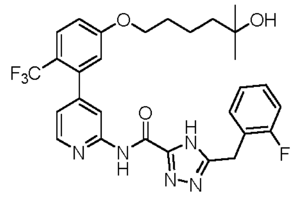
Example 269

Example 269: 5-(2-fluorobenzyl)-N-(4-(5-((5-hydroxyl-5-methylhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl 5-bromopentanoate was used in place of methyl 4-bromobutanoate, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 9.93 (s, 1H), 8.43 (d, *J* = 5.1 Hz, 1H), 8.15 (s, 1H), 7.78 (d, *J* = 8.9 Hz, 1H), 7.46 - 7.28 (m, 2H), 7.28 - 7.12 (m, 4H), 6.97 (d, *J* = 2.5 Hz, 1H), 4.21 (s, 2H), 4.15 - 4.02 (m, 3H), 1.71 (t, *J* = 7.0 Hz, 2H), 1.52 - 1.34 (m, 4H), 1.06 (s, 6H). MS: 572 $[M+H]^+$.

Example 270: 5-(cyclopentylmethyl)-N-(4-(5-((5-hydroxyl-5-methylhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl 5-bromopentanoate was used in place of methyl 4-bromobutanoate, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.41 (s, 1H), 9.90 (s, 1H), 8.44 (dd, *J* = 5.1, 0.8 Hz, 1H), 8.17 (s, 1H), 7.79 (d, *J* = 8.9 Hz, 1H), 7.27 - 7.13 (m, 2H), 6.98 (d, *J* = 2.5 Hz, 1H), 4.17 - 4.01 (m, 3H), 2.78 (d, *J* = 7.4 Hz, 2H), 2.35 - 2.19 (m, 1H), 1.81 - 1.66 (m, 4H), 1.66 - 1.33 (m, 8H), 1.29 - 1.15 (m, 2H), 1.06 (s, 6H). MS: 546 $[M+H]^+$.

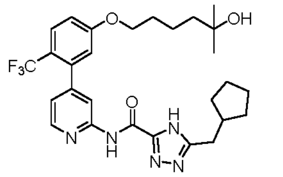
Example 270

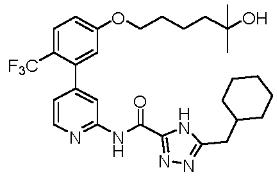
Example 271

Example 271: 5-(cyclohexylmethyl)-N-(4-(5-((5-hydroxyl-5-methylhexyl)oxy)-2-(trifluoromethyl)phenyl)pyridin-2-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, methyl 5-bromopentanoate was used in place of methyl 4-bromobutanoate, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.90 (s, 1H), 8.43 (dd, *J* = 5.1,

0.8 Hz, 1H), 8.17 (s, 1H), 7.79 (d, *J* = 8.9 Hz, 1H), 7.27 - 7.12 (m, 2H), 6.98 (d, *J* = 2.5 Hz, 1H), 4.17 - 4.01 (m, 3H), 2.66 (d, *J* = 7.1 Hz, 2H), 1.86 - 1.55 (m, 8H), 1.51 - 1.33 (m, 4H), 1.28 - 1.09 (m, 3H), 1.06 (s, 6H), 1.04 - 0.89 (m, 2H). MS: 560 $[M+H]^+$.

Example 272: 5-benzyl-N-(4-(5-(4-hydroxybutoxy)-2-(trifluoromethyl)phenyl)pyridin-2-yl)-4H-1,2,4-triazole-3-carboxamide

The preparation was carried out in a similar manner to Example 92, except that in step 1, 4-bromo-1-butanol was used in place of iodomethane, and 3-chloro-4-(trifluoromethyl)phenol was used in place of 2-bromo-3-methylphenol. $^1$H NMR (400 MHz, DMSO-$d_6$) δ 14.59 (s, 1H), 9.95 (s, 1H), 8.43 (d, *J* = 5.1 Hz, 1H), 8.15 (s, 1H), 7.79 (d, *J* = 8.9 Hz, 1H), 7.39 - 7.29 (m, 4H), 7.29 - 7.22 (m, 1H), 7.22 - 7.14 (m, 2H), 6.98 (d, *J* = 2.6 Hz, 1H), 4.45 (t, *J* = 5.1 Hz, 1H), 4.17 (s, 2H), 4.11 (t, *J* = 6.5 Hz, 2H), 3.48 - 3.41 (m, 2H), 1.82 - 1.69 (m, 2H), 1.61 - 1.49 (m, 2H). MS: 512 $[M+H]^+$.

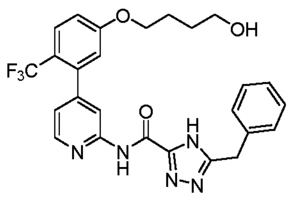

Example 272

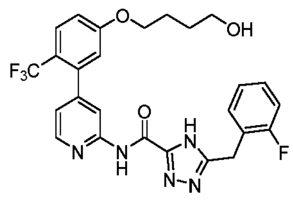

Example 273

Example 273: 5-(2-fluorobenzyl)-N-(4-(5-(4-hydroxybutoxy)-2-(trifluoromethyl)phenyl)pyridin-2-yl)-4H-1,2,4-triazole-3-carboxamide

The preparation was carried out in a similar manner to Example 92, except that in step 1, 4-bromo-1-butanol was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.63 (s, 1H), 9.93 (s, 1H), 8.43 (d, *J* = 5.1 Hz, 1H), 8.15 (s, 1H), 7.79 (d, *J* = 8.9 Hz, 1H), 7.44-7.29 (m, 2H), 7.26-7.14 (m, 4H), 6.97 (d, *J* = 2.5 Hz, 1H), 4.45 (t, *J* = 5.1 Hz, 1H), 4.21 (s, 2H), 4.10 (t, *J* = 6.5 Hz, 2H), 3.48-3.40 (m, 2H), 1.81-1.70 (m, 2H), 1.61-1.50 (m, 2H). MS: 530 $[M+H]^+$.

Example 274: 5-(cyclopentylmethyl)-N-(4-(5-(4-hydroxybutoxy)-2-(trifhioromethyl)phenyl)pyridin-2-yl)-4H-1,2,4-triazole-3-carboxamide

The preparation was carried out in a similar manner to Example 92, except that in step 1, 4-bromo-1-butanol was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 9.90 (s, 1H), 8.44 (d, *J* = 5.1 Hz, 1H), 8.17 (s, 1H), 7.79 (d, *J* = 8.9 Hz, 1H), 7.23 - 7.15 (m, 2H), 6.98 (d, *J* = 2.6 Hz, 1H), 4.45 (t, *J* = 5.2 Hz, 1H), 4.11 (t, *J* = 6.6 Hz, 2H), 3.49 - 3.39 (m, 2H), 2.77 (d, *J* = 7.5 Hz, 2H), 2.37 - 2.19 (m, 1H), 1.83 - 1.67 (m, 4H), 1.67 - 1.45 (m, 6H), 1.30 - 1.14 (m, 2H). MS: 504 $[M+H]^+$.

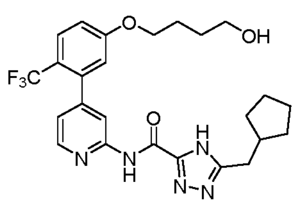

Example 274

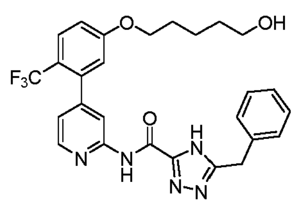

Example 275

Example 275: 5-benzyl-N-(4-(5-((5-hydroxypentyl)oxy)-2-(trifluoromethyl)phenyl)pyridin-2-yl)-4H-1,2,4-triazole-3-carboxamide

The preparation was carried out in a similar manner to Example 92, except that in step 1, 5-bromo-1-pentanol

was used in place of iodomethane, and 3-chloro-4-(trifluoromethyl)phenol was used in place of 2-bromo-3-methylphenol. $^1$H NMR (400 MHz, DMSO-$d_6$) δ 14.61 (s, 1H), 9.95 (s, 1H), 8.43 (d, $J$ = 5.1 Hz, 1H), 8.15 (s, 1H), 7.79 (d, $J$ = 8.9 Hz, 1H), 7.44 - 7.30 (m, 4H), 7.30 - 7.22 (m, 1H), 7.22 - 7.10 (m, 2H), 6.98 (d, $J$ = 2.6 Hz, 1H), 4.37 (t, $J$ = 5.1 Hz, 1H), 4.17 (s, 2H), 4.09 (t, $J$ = 6.5 Hz, 2H), 3.42 - 3.38 (m, 2H), 1.80 - 1.68 (m, 2H), 1.54 - 1.36 (m, 4H). MS: 526 [M+H]$^+$.

Example 276: 5-(2-fluorobenzyl)-N-(4-(5-((5-hydroxypentyl)oxy)-2-(trifluoromethyl)phenyl)pyridin-2-yl)-4H-1,2,4-triazole-3 -carboxamide

The preparation was carried out in a similar manner to Example 92, except that in step 1, 5-bromo-1-pentanol was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.67 (s, 1H), 9.94 (s, 1H), 8.43 (d, $J$ = 5.0 Hz, 1H), 8.15 (s, 1H), 7.78 (d, $J$ = 8.8 Hz, 1H), 7.47-7.29 (m, 2H), 7.29-7.12 (m, 4H), 6.97 (d, $J$ = 2.6 Hz, 1H), 4.38 (d, $J$ = 5.5 Hz, 1H), 4.20 (s, 2H), 4.09 (t, $J$ = 6.5 Hz, 2H), 3.42-3.38 (m, 2H), 1.81-1.65 (m, 2H), 1.55-1.34 (m, 4H). MS: 544 [M+H]$^+$.

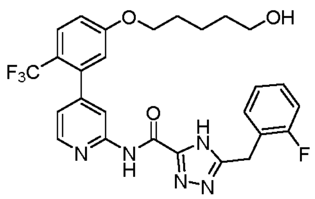

Example 276

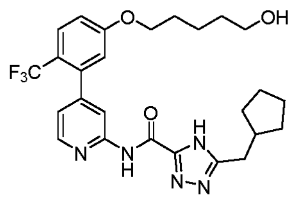

Example 277

Example 277: 5-(cyclopentylmethyl)-N-(4-(5-((5-hydroxypentyl)oxy)-2-(trifluoromethyl)phenyl)pyridin-2-yl)-4H-1,2,4-triazole-3 -carboxamide

The preparation was carried out in a similar manner to Example 92, except that in step 1, 5-bromo-1-pentanol was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 9.90 (s, 1H), 8.44 (d, $J$ = 5.1 Hz, 1H), 8.17 (s, 1H), 7.79 (d, $J$ = 8.9 Hz, 1H), 7.28 - 7.12 (m, 2H), 6.98 (d, $J$ = 2.6 Hz, 1H), 4.37 (t, $J$ = 5.1 Hz, 1H), 4.09 (t, $J$ = 6.5 Hz, 2H), 3.45 - 3.36 (m, 2H), 2.78 (d, $J$ = 7.5 Hz, 2H), 2.36 - 2.21 (m, 1H), 1.81 - 1.66 (m, 4H), 1.66 - 1.38 (m, 8H), 1.28 - 1.15 (m, 2H). MS: 518 [M+H]$^+$.

Example 278: 5-(cyclohexylmethyl)-N-(4-(5-((5-hydroxypentyl)oxy)-2-(trifluoromethyl)phenyl)pyridin-2-yl)-4H-1,2,4-triazole-3 -carboxamide

The preparation was carried out in a similar manner to Example 92, except that in step 1, 5-bromo-1-pentanol was used in place of iodomethane, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.36 (s, 1H), 9.89 (s, 1H), 8.44 (dd, $J$ = 5.1, 0.8 Hz, 1H), 8.17 (s, 1H), 7.79 (d, $J$ = 8.9 Hz, 1H), 7.28 - 7.12 (m, 2H), 6.98 (d, $J$ = 2.6 Hz, 1H), 4.37 (t, $J$ = 5.1 Hz, 1H), 4.09 (t, $J$ = 6.5 Hz, 2H), 3.45 - 3.36 (m, 2H), 2.67 (d, $J$ = 7.0 Hz, 2H), 1.83 - 1.53 (m, 8H), 1.53 - 1.36 (m, 4H), 1.26 - 1.15 (m, 3H), 1.03 - 0.90 (m, 2H). MS: 532 [M+H]$^+$.

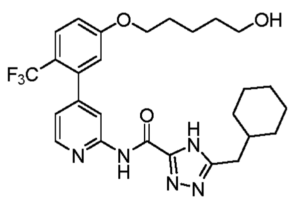

Example 278

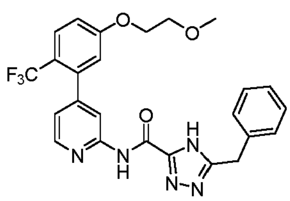

Example 279

Example 279: 5-benzyl-N-(4-(5-(2-methoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, and 3-chloro-4-(trifluoromethyl)phenol was used in place of 2-bromo-3-methylphenol. $^1$H NMR (400 MHz, DMSO-$d_6$) δ 14.62 (s, 1H), 9.96 (s, 1H), 8.44 (dd, *J* = 5.1, 0.8 Hz, 1H), 8.16 (d, *J* = 1.4 Hz, 1H), 7.79 (d, *J* = 8.9 Hz, 1H), 7.48 - 7.29 (m, 4H), 7.29 - 7.13 (m, 3H), 7.01 (d, *J* = 2.6 Hz, 1H), 4.28 - 4.20 (m, 2H), 4.17 (s, 2H), 3.73 - 3.62 (m, 2H), 3.30 (s, 3H). MS: 498 [M+H]$^+$.

Example 280: 5-(2-fluorobenzyl)-N-(4-(5-(2-methoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.95 (s, 1H), 8.43 (dd, *J* = 5.1, 0.8 Hz, 1H), 8.15 (s, 1H), 7.79 (d, *J* = 8.9 Hz, 1H), 7.46 - 7.28 (m, 2H), 7.28 - 7.07 (m, 4H), 7.00 (d, *J* = 2.5 Hz, 1H), 4.29 - 4.15 (m, 4H), 3.70 - 3.62 (m, 2H), 3.30 (s, 3H). MS: 516 [M+H]$^+$.

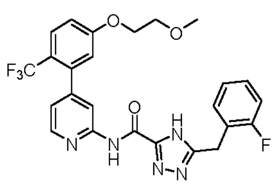

Example 280

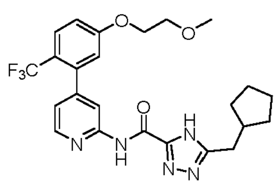

Example 281

Example 281: 5-(cyclopentylmethyl)-N-(4-(5-(2-methoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.91 (s, 1H), 8.44 (d, *J* = 5.1 Hz, 1H), 8.18 (s, 1H), 7.80 (d, *J* = 8.9 Hz, 1H), 7.32 - 7.12 (m, 2H), 7.01 (d, *J* = 2.6 Hz, 1H), 4.31 - 4.17 (m, 2H), 3.72 - 3.62 (m, 2H), 3.30 (s, 3H), 2.77 (d, *J* = 7.4 Hz, 2H), 2.37 - 2.18 (m, 1H), 1.79 - 1.66 (m, 2H), 1.66 - 1.44 (m, 4H), 1.29 - 1.12 (m, 2H). MS: 490 [M+H]$^+$.

Example 282: 5-(cyclohexylmethyl)-N-(4-(5-(2-methoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.90 (s, 1H), 8.52 - 8.39 (m, 1H), 8.18 (s, 1H), 7.80 (d, *J* = 8.9 Hz, 1H), 7.30-7.14 (m, 2H), 7.01 (d, *J* = 2.6 Hz, 1H), 4.31 - 4.17 (m, 2H), 3.73 - 3.60 (m, 2H), 3.30 (s, 3H), 2.67 (d, *J* = 7.0 Hz, 2H), 1.83 - 1.54 (m, 6H), 1.27 - 1.12 (m, 3H), 1.04 - 0.91 (m, 2H). MS: 504 [M+H]$^+$.

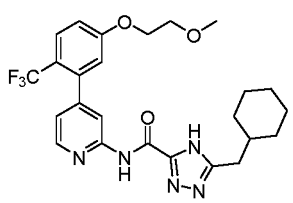

Example 282

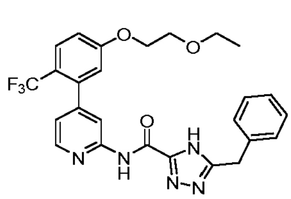

Example 283

Example 283: 5-benzyl-N-(4-(5-(2-ethoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, and 3-chloro-4-(trifluoromethyl)phenol was used in place of 2-bromo-3-methylphenol. $^1$H NMR (400 MHz, DMSO-$d_6$) δ 14.61 (s, 1H), 9.96 (s, 1H), 8.43 (d, *J* = 5.1 Hz, 1H), 8.16 (s, 1H), 7.79 (d, *J* = 8.9 Hz, 1H), 7.43 - 7.09 (m, 7H), 7.01 (d, *J* = 2.6 Hz, 1H), 4.28 - 4.20 (m, 2H), 4.17 (s, 2H), 3.75 - 3.66 (m, 2H), 3.49 (q, *J* = 7.0 Hz, 2H), 1.11 (t, *J* = 7.0 Hz, 3H). MS: 512 $[M+H]^+$.

Example 284: 5-(2-fluorobenzyl)-N-(4-(5-(2-ethoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.67 (s, 1H), 9.94 (s, 1H), 8.48-8.37 (m, 1H), 8.15 (s, 1H), 7.79 (d, *J* = 8.8 Hz, 1H), 7.45-7.29 (m, 2H), 7.26-7.14 (m, 4H), 7.01 (d, *J* = 2.6 Hz, 1H), 4.30 - 4.14 (m, 4H), 3.74-3.64 (m, 2H), 3.49 (q, *J* = 7.0 Hz, 2H), 1.11 (t, *J* = 7.0 Hz, 3H). MS: 530 $[M+H]^+$.

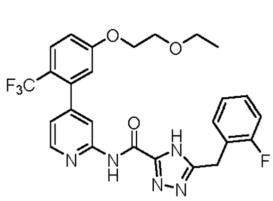

Example 284

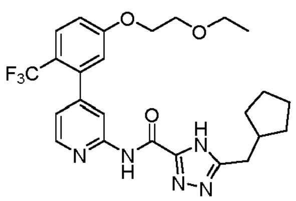

Example 285

Example 285: 5-(cyclopentylmethyl)-N-(4-(5-(2-ethoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 9.90 (s, 1H), 8.44 (d, *J* = 5.1 Hz, 1H), 8.18 (s, 1H), 7.80 (d, *J* = 8.9 Hz, 1H), 7.29 - 7.13 (m, 2H), 7.02 (d, *J* = 2.6 Hz, 1H), 4.29 - 4.17 (m, 2H), 3.76 - 3.65 (m, 2H), 3.49 (q, *J* = 7.0 Hz, 2H), 2.78 (d, *J* = 7.5 Hz, 2H), 2.35 - 2.18 (m, 1H), 1.79 - 1.67 (m, 2H), 1.67 - 1.44 (m, 4H), 1.30 - 1.16 (m, 2H), 1.11 (t, *J* = 7.0 Hz, 3H). MS: 504 $[M+H]^+$.

Example 286: 5-(cyclohexylmethyl)-N-(4-(5-(2-ethoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.44 (d, *J* = 5.1 Hz, 1H), 8.18 (s, 1H), 7.80 (d, *J* = 8.9 Hz, 1H), 7.28 - 7.13 (m, 2H), 7.02 (d, *J* = 2.6 Hz, 1H), 4.28 - 4.19 (m, 2H), 3.76 - 3.66 (m, 2H), 3.49 (q, *J* = 7.0 Hz, 2H), 2.66 (d, *J* = 7.0 Hz, 2H), 1.80 - 1.57 (m, 6H), 1.25 - 1.14 (m, 3H), 1.11 (t, *J* = 7.0 Hz, 3H), 1.06 - 0.91 (m, 2H). MS: 518 $[M+H]^+$.

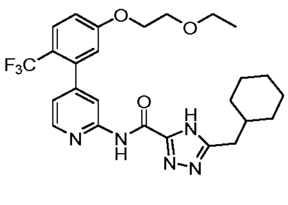 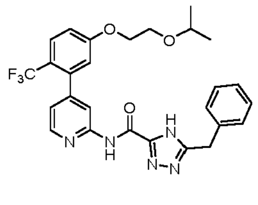

Example 286 Example 287

Example 287: 5-benzyl-N-(4-(5-(2-isopropoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-isopropoxyethane was used in place of iodomethane, and 3-chloro-4-(trifluoromethyl)phenol was used in place of 2-bromo-3-methylphenol. $^1$H NMR (400 MHz, DMSO-$d_6$) δ 14.58 (s, 1H), 9.94 (s, 1H), 8.43 (dd, $J$ = 5.0, 0.8 Hz, 1H), 8.16 (s, 1H), 7.79 (d, $J$ = 8.9 Hz, 1H), 7.41-7.11 (m, 7H), 7.01 (d, $J$ = 2.6 Hz, 1H), 4.25-4.12 (m, 4H), 3.74-3.67 (m, 2H), 3.62 (p, $J$ = 6.1 Hz, 1H), 1.09 (d, $J$ = 6.1 Hz, 6H). MS: 526 [M+H]$^+$.

Example 288: 5-(2-fluorobenzyl)-N-(4-(5-(2-isopropoxyethoxy)-2-(trifluoromethyl)phenyl)pyridin-2-yl)-4H-1,2,4-triazole-3-carboxamide

The preparation was carried out in a similar manner to Example 92, except that in step 1, 1-bromo-2-isopropoxyethane was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.94 (s, 1H), 8.43 (d, $J$ = 5.1 Hz, 1H), 8.15 (s, 1H), 7.79 (d, $J$ = 8.9 Hz, 1H), 7.48 - 7.28 (m, 2H), 7.28 - 7.11 (m, 4H), 7.01 (d, $J$ = 2.6 Hz, 1H), 4.29 - 4.13 (m, 4H), 3.76 - 3.66 (m, 2H), 3.61 (p, $J$ = 6.1 Hz, 1H), 1.09 (d, $J$ = 6.1 Hz, 6H). MS: 544 [M+H]$^+$.

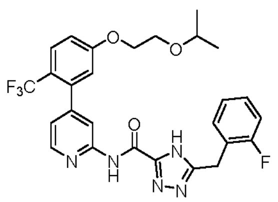 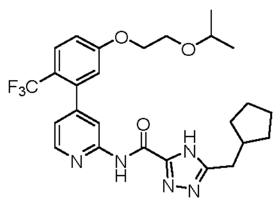

Example 288 Example 289

Example 289: 5-(cyclopentylmethyl)-N-(4-(5-(2-isopropoxyethoxy)-2-(trifluoromethyl)phenyl)pyridin-2-yl)-4H-1,2,4-triazole-3 -carboxamide

The preparation was carried out in a similar manner to Example 92, except that in step 1, 1-bromo-2-isopropoxyethane was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.33 (s, 1H), 9.90 (s, 1H), 8.44 (d, $J$ = 5.1 Hz, 1H), 8.18 (s, 1H), 7.79 (d, $J$ = 8.9 Hz, 1H), 7.28 - 7.11 (m, 2H), 7.01 (d, $J$ = 2.5 Hz, 1H), 4.26 - 4.15 (m, 2H), 3.75 - 3.67 (m, 2H), 3.62 (p, $J$ = 6.1 Hz, 1H), 2.78 (d, $J$ = 7.5 Hz, 2H), 2.36 - 2.21 (m, 1H), 1.78 - 1.66 (m, 2H), 1.66 - 1.44 (m, 4H), 1.24 - 1.16 (m, 2H), 1.09 (d, $J$ = 6.1 Hz, 6H). MS: 518 [M+H]$^+$.

Example 290: 5-(cyclohexylmethyl)-N-(4-(5-(2-isopropoxyethoxy)-2-(trifluoromethyl)phenyl)pyridin-2-yl)-4H-1,2,4-triazole-3 -carboxamide

The preparation was carried out in a similar manner to Example 92, except that in step 1, 1-bromo-2-isopropoxyethane was used in place of iodomethane, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.36 (s, 1H), 9.88 (s, 1H), 8.44 (d, $J$ = 5.1 Hz, 1H), 8.17 (s, 1H), 7.79 (d, $J$ = 8.9 Hz, 1H), 7.28 - 7.14 (m, 2H), 7.01 (d, $J$ = 2.6 Hz, 1H), 4.25 - 4.16 (m, 2H), 3.75 - 3.67 (m, 2H), 3.62 (p, $J$ = 6.1 Hz, 1H), 2.67 (d, $J$ = 6.9 Hz, 2H), 1.84 - 1.54 (m, 6H), 1.24 - 1.13 (m, 3H), 1.09 (d, $J$ = 6.1 Hz,

6H), 1.05 - 0.91 (m, 2H). MS: 532 $[M+H]^+$.

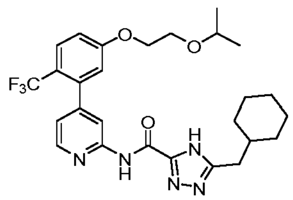

Example 290

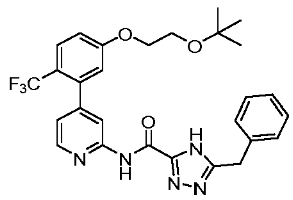

Example 291

Example 291: 5-benzyl-N-(4-(5-(2-(tert-butoxy)ethoxy)-2-(trifluoromethyl)phenyl)pyridin-2-yl)-4H-1,2,4-triazole-3-carboxamide

The preparation was carried out in a similar manner to Example 64, except that in step 1, 3-chloro-4-(trifluoromethyl)phenol was used in place of 2-bromo-3-methylphenol. $^1$H NMR (400 MHz, DMSO-$d_6$) δ 14.53 (s, 1H), 9.95 (s, 1H), 8.43 (d, *J* = 5.0 Hz, 1H), 8.16 (s, 1H), 7.79 (d, *J* = 8.9 Hz, 1H), 7.40 - 7.11 (m, 7H), 7.00 (d, *J* = 2.5 Hz, 1H), 4.22 - 4.10 (m, 4H), 3.69 - 3.59 (m, 2H), 1.14 (s, 9H). MS: 540 $[M+H]^+$.

Example 292: 5-(2-fluorobenzyl)-N-(4-(5-(2-(tert-butoxy)ethoxy)-2-(trifluoromethyl)phenyl)pyridin-2-yl)-4H-1,2,4-triazole-3-carboxamide

The preparation was carried out in a similar manner to Example 64, except that in step 1, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.67 (s, 1H), 9.94 (s, 1H), 8.43 (d, *J* = 5.2 Hz, 1H), 8.15 (s, 1H), 7.78 (d, *J* = 8.8 Hz, 1H), 7.51 - 7.27 (m, 2H), 7.27 -7.10 (m, 4H), 7.00 (d, *J* = 2.6 Hz, 1H), 4.28 - 4.09 (m, 4H), 3.65 (t, *J* = 4.8 Hz, 2H), 1.14 (s, 9H). MS: 558 $[M+H]^+$.

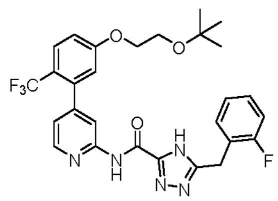

Example 292

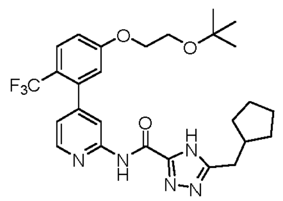

Example 293

Example 293: 5-(cyclopentylmethyl)-N-(4-(5-(2-(tert-butoxy)ethoxy)-2-(trifluoromethyl)phenyl)pyridin-2-yl)-4H-1,2,4-triazole-3 -carboxamide

The preparation was carried out in a similar manner to Example 64, except that in step 1, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 9.88 (s, 1H), 8.44 (d, *J* = 5.1 Hz, 1H), 8.18 (s, 1H), 7.79 (d, *J* = 8.9 Hz, 1H), 7.28-7.11 (m, 2H), 7.01 (d, *J* = 2.6 Hz, 1H), 4.24-4.11 (m, 2H), 3.71-3.60 (m, 2H), 2.78 (d, *J* = 7.5 Hz, 2H), 2.28 (p, *J* = 7.6 Hz, 1H), 1.79-1.66 (m, 2H), 1.66-1.43 (m, 4H), 1.25-1.20 (m, 2H), 1.15 (s, 9H). MS: 532 $[M+H]^+$.

Example 294: 5-(cyclohexylmethyl)-N-(4-(5-(2-(tert-butoxy)ethoxy)-2-(trifluoromethyl)phenyl)pyridin-2-yl)-4H-1,2,4-triazole-3 -carboxamide

The preparation was carried out in a similar manner to Example 64, except that in step 1, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.32 (s, 1H), 9.89 (s, 1H), 8.44 (d, *J* = 5.1 Hz, 1H), 8.17 (s, 1H), 7.79 (d, *J* = 8.9 Hz, 1H), 7.29 - 7.12 (m, 2H), 7.01 (d, *J* = 2.6 Hz, 1H), 4.24 - 4.13 (m, 2H), 3.66 (dd, *J* = 5.5, 4.0 Hz, 2H), 2.67 (d, *J* = 7.0 Hz, 2H), 1.83 - 1.56 (m, 6H), 1.23 - 1.16 (m, 3H), 1.15 (s, 9H), 1.05 - 0.93 (m, 2H). MS: 546 $[M+H]^+$.

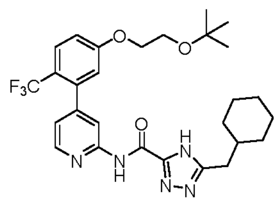 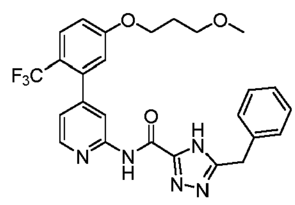

Example 294 Example 295

Example 295: 5-benzyl-N-(4-(5-(3-methoxypropoxy)-2-(trifluoromethyl)phenyl)pyridin-2-yl)-4H-1,2,4-triazole-3-carboxamide

The preparation was carried out in a similar manner to Example 92, except that in step 1, 1-bromo-3-methoxypropane was used in place of iodomethane, and 3-chloro-4-(trifluoromethyl)phenol was used in place of 2-bromo-3-methylphenol. $^1$H NMR (400 MHz, DMSO-$d_6$) δ 14.61 (s, 1H), 9.96 (s, 1H), 8.43 (d, *J* = 5.1 Hz, 1H), 8.16 (s, 1H), 7.79 (d, *J* = 8.9 Hz, 1H), 7.42 - 7.29 (m, 4H), 7.29 - 7.23 (m, 1H), 7.23 - 7.12 (m, 2H), 6.98 (d, *J* = 2.6 Hz, 1H), 4.22 - 4.08 (m, 4H), 3.46 (t, *J* = 6.2 Hz, 2H), 3.23 (s, 3H), 2.02 - 1.90 (m, 2H). MS: 512 $[M+H]^+$.

Example 296: 5-(2-fluorobenzyl)-N-(4-(5-(3-methoxypropoxy)-2-(trifluoromethyl)phenyl)pyridin-2-yl)-4H-1,2,4-triazole-3-carboxamide

The preparation was carried out in a similar manner to Example 92, except that in step 1, 1-bromo-3-methoxypropane was used in place of iodomethane, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.67 (s, 1H), 9.94 (s, 1H), 8.43 (dd, *J* = 5.1, 0.8 Hz, 1H), 8.15 (s, 1H), 7.79 (d, *J* = 8.9 Hz, 1H), 7.46 - 7.28 (m, 2H), 7.28 - 7.12 (m, 4H), 6.98 (d, *J* = 2.6 Hz, 1H), 4.21 (s, 2H), 4.14 (t, *J* = 6.4 Hz, 2H), 3.46 (t, *J* = 6.2 Hz, 2H), 3.23 (s, 3H), 2.01 - 1.91 (m, 2H). MS: 530 $[M+H]^+$.

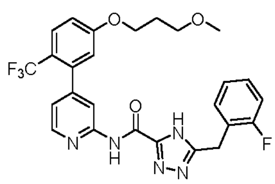 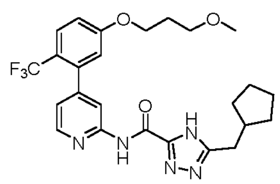

Example 296 Example 297

Example 297: 5-(cyclopentylmethyl)-N-(4-(5-(3-methoxypropoxy)-2-(trifluoromethyl)phenyl)pyridin-2-yl)-4H-1,2,4-triazole-3 -carboxamide

The preparation was carried out in a similar manner to Example 92, except that in step 1, 1-bromo-3-methoxypropane was used in place of iodomethane, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.91 (s, 1H), 8.44 (dd, *J* = 5.1, 0.8 Hz, 1H), 8.23 - 8.13 (m, 1H), 7.79 (d, *J* = 8.9 Hz, 1H), 7.28 - 7.12 (m, 2H), 6.99 (d, *J* = 2.5 Hz, 1H), 4.15 (t, *J* = 6.4 Hz, 2H), 3.46 (t, *J* = 6.2 Hz, 2H), 3.24 (s, 3H), 2.77 (d, *J* = 7.5 Hz, 2H), 2.36 - 2.19 (m, 1H), 2.04 - 1.91 (m, 2H), 1.78 - 1.66 (m, 2H), 1.66 - 1.44 (m, 4H), 1.29 - 1.14 (m, 2H). MS: 504 $[M+H]^+$.

Example 298: 5-(cyclohexylmethyl)-N-(4-(5-(3-methoxypropoxy)-2-(trifluoromethyl)phenyl)pyridin-2-yl)-4H-1,2,4-triazole-3 -carboxamide

The preparation was carried out in a similar manner to Example 92, except that in step 1, 1-bromo-3-methoxypropane was used in place of iodomethane, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.90 (s, 1H), 8.49 - 8.39 (m, 1H), 8.17 (s, 1H), 7.79 (d, *J* = 8.8 Hz, 1H), 7.29 - 7.13 (m, 2H), 6.99 (d, *J* = 2.6 Hz, 1H), 4.15 (t, *J* = 6.4 Hz, 2H), 3.47 (t, *J* = 6.2 Hz, 2H), 3.24 (s, 3H), 2.67 (d, *J* = 7.0 Hz, 2H), 2.03 - 1.90 (m, 2H), 1.81 - 1.57 (m, 6H), 1.24 - 1.10 (m, 3H), 1.03 - 0.89 (m,

2H). MS: 518 [M+H]+.

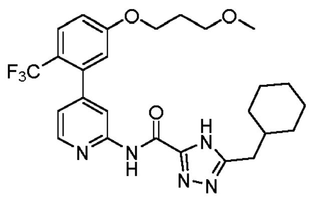

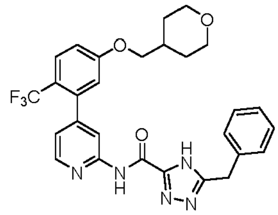

Example 298 Example 299

Example 299: 5-benzyl-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, and 3-chloro-4-(trifluoromethyl)phenol was used in place of 2-bromo-3-methylphenol. $^1$H NMR (400 MHz, DMSO-$d_6$) δ 14.56 (s, 1H), 9.95 (s, 1H), 8.43 (d, *J* = 5.1 Hz, 1H), 8.16 (s, 1H), 7.79 (d, *J* = 8.9 Hz, 1H), 7.41 - 7.29 (m, 4H), 7.29 - 7.13 (m, 3H), 6.99 (d, *J* = 2.6 Hz, 1H), 4.17 (s, 2H), 3.97 (d, *J* = 6.5 Hz, 2H), 3.92 - 3.79 (m, 2H), 2.10 - 1.93 (m, 1H), 1.72 - 1.60 (m, 2H), 1.42 - 1.18 (m, 4H). MS: 538 [M+H]+.

Example 300: 5-(2-fluorobenzyl)-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 9.94 (s, 1H), 8.43 (d, *J* = 5.1 Hz, 1H), 8.15 (s, 1H), 7.79 (d, *J* = 8.9 Hz, 1H), 7.45 - 7.29 (m, 2H), 7.26 - 7.13 (m, 4H), 6.99 (d, *J* = 2.6 Hz, 1H), 4.21 (s, 2H), 3.96 (d, *J* = 6.5 Hz, 2H), 3.92 - 3.80 (m, 2H), 2.09 - 1.91 (m, 1H), 1.72 - 1.62 (m, 2H), 1.41 - 1.16 (m, 4H). MS: 556 [M+H]+.

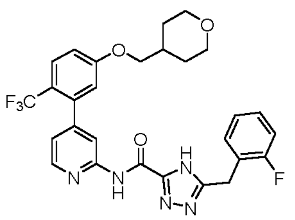

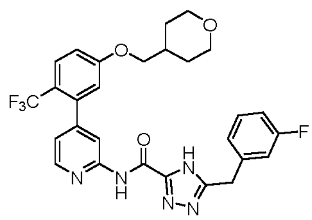

Example 300 Example 301

Example 301: 5-(3-fluorobenzyl)-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 2-bromo-3-methylphenol, and in step 3, ethyl 5-(3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 9.99 (s, 1H), 8.43 (d, *J* = 5.1 Hz, 1H), 8.16 (s, 1H), 7.79 (d, *J* = 8.9 Hz, 1H), 7.44 - 7.32 (m, 1H), 7.26 - 7.13 (m, 4H), 7.13 - 7.04 (m, 1H), 6.99 (d, *J* = 2.5 Hz, 1H), 4.21 (s, 2H), 3.96 (d, *J* = 6.5 Hz, 2H), 3.91 - 3.80 (m, 2H), 3.35 - 3.29 (m, 2H), 2.07 - 1.95 (m, 1H), 1.73 - 1.60 (m, 2H), 1.41 - 1.25 (m, 2H). MS: 556 [M+H]+.

Example 302: 5-(4-fluorobenzyl)-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 2-bromo-3-methylphenol, and in step 3, ethyl 5-(4-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.53 (s, 1H), 9.91 (s, 1H), 8.43 (d, $J$ = 5.1 Hz, 1H), 8.15 (s, 1H), 7.79 (d, $J$ = 8.9 Hz, 1H), 7.42 - 7.31 (m, 2H), 7.26 - 7.09 (m, 4H), 6.99 (d, $J$ = 2.5 Hz, 1H), 4.18 (s, 2H), 3.97 (d, $J$ = 6.5 Hz, 2H), 3.92 - 3.79 (m, 2H), 3.33 - 3.31 (m, 2H), 2.13 - 1.92 (m, 1H), 1.74 - 1.60 (m, 2H), 1.42 - 1.23 (m, 2H). MS: 556 [M+H]$^+$.

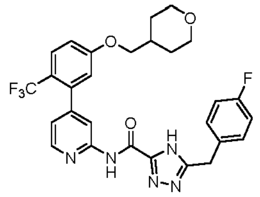

Example 302

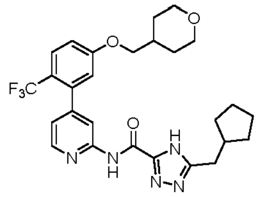

Example 303

Example 303: 5-(cyclopentylmethyl)-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro2H-pyran-4-yl)methanol was used in place of 2-(tert-butoxy)ethan-1-ol, 3-chloro-4-(trifluoromethyl)phenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 9.89 (s, 1H), 8.44 (d, $J$ = 5.0 Hz, 1H), 8.17 (s, 1H), 7.79 (d, $J$ = 8.9 Hz, 1H), 7.25 - 7.14 (m, 2H), 6.99 (d, $J$ = 2.5 Hz, 1H), 3.97 (d, $J$ = 6.5 Hz, 2H), 3.91 - 3.81 (m, 2H), 3.33 - 3.26 (m, 2H), 2.78 (d, $J$ = 7.5 Hz, 2H), 2.35 - 2.18 (m, 1H), 2.11 - 1.93 (m, 1H), 1.79 - 1.42 (m, 8H), 1.41 - 1.27 (m, 2H), 1.27-1.18 (m, 2H). MS: 530 [M+H]$^+$.

Example 304: 5-(cyclohexylmethyl)-N-(4-(5-((tetrahydro-2H-pyran-4-yl)methoxy)-2-(trifluoromethyl)phenyl)pyridin-2-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate.

$^1$H NMR (400 MHz, DMSO-$d_6$) $\delta$ 14.38 (s, 1H), 9.90 (s, 1H), 8.44 (d, $J$ = 5.1 Hz, 1H), 8.17 (s, 1H), 7.79 (d, $J$ = 8.9 Hz, 1H), 7.29 - 7.13 (m, 2H), 7.00 (d, $J$ = 2.6 Hz, 1H), 3.97 (d, $J$ = 6.4 Hz, 2H), 3.92 - 3.79 (m, 2H), 3.37 - 3.33 (m, 2H), 2.67 (d, $J$ = 7.0 Hz, 2H), 2.10 - 1.92 (m, 1H), 1.81 - 1.72 (m, 1H), 1.70 - 1.62 (m, 6H), 1.40 - 1.11 (m, 6H), 1.05 - 0.89 (m, 2H). MS: 544 [M+H]$^+$.

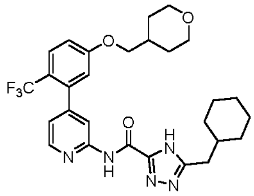

Example 304

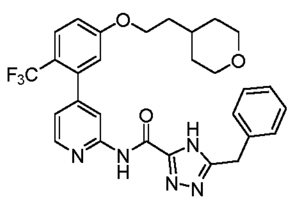

Example 305

Example 305: 5-benzyl-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 2, 3-chloro-4-(trifluoromethyl)phenol was used in place of 2-bromo-3-methylphenol. $^1$H NMR (400 MHz, DMSO-$d_6$) δ 14.59 (s, 1H), 9.95 (s, 1H), 8.43 (d, $J$ = 5.1 Hz, 1H), 8.16 (s, 1H), 7.78 (d, $J$ = 8.9 Hz, 1H), 7.42 - 7.29 (m, 4H), 7.29 - 7.23 (m, 1H), 7.23 - 7.13 (m, 2H), 6.99 (d, $J$ = 2.6 Hz, 1H), 4.25 - 4.06 (m, 4H), 3.89 - 3.71 (m, 2H), 3.29 - 3.16 (m, 2H), 1.77 - 1.65 (m, 3H), 1.65 - 1.53 (m, 2H), 1.29 - 1.18 (m, 2H). MS: 552 [M+H]$^+$.

Example 306: 5-(2-fluorobenzyl)-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 2-bromo-3-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 9.94 (s, 1H), 8.43 (d, $J$ = 5.1 Hz, 1H), 8.15 (s, 1H), 7.78 (d, $J$ = 8.9 Hz, 1H), 7.48 - 7.28 (m, 2H), 7.28 - 7.12 (m, 4H), 6.99 (d, $J$ = 2.6 Hz, 1H), 4.21 (s, 2H), 4.14 (t, $J$ = 6.1 Hz, 2H), 3.89 - 3.76 (m, 2H), 3.31 - 3.20 (m, 2H), 1.77 - 1.55 (m, 5H), 1.31 - 1.12 (m, 2H). MS: 570 [M+H]$^+$.

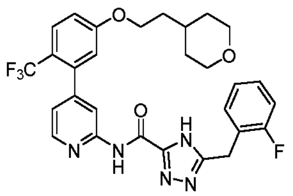

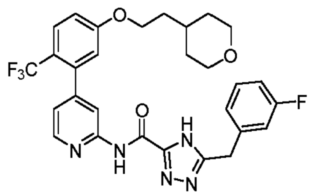

Example 306 Example 307

Example 307: 5-(3-fluorobenzyl)-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 2-bromo-3-methylphenol, and in step 4, ethyl 5-(3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 9.97 (s, 1H), 8.44 (d, $J$ = 5.1 Hz, 1H), 8.15 (s, 1H), 7.79 (d, $J$ = 8.9 Hz, 1H), 7.45 - 7.32 (m, 1H), 7.23 - 7.13 (m, 4H), 7.13 - 7.05 (m, 1H), 7.00 (d, $J$ = 2.6 Hz, 1H), 4.21 (s, 2H), 4.14 (t, $J$ = 6.1 Hz, 2H), 3.93 - 3.72 (m, 2H), 3.32 - 3.21 (m, 2H), 1.75 - 1.57 (m, 5H), 1.29 - 1.13 (m, 2H). MS: 570 [M+H]$^+$.

Example 308: 5-(4-fluorobenzyl)-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 2-bromo-3-methylphenol, and in step 4, ethyl 5-(4-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 9.96 (s, 1H), 8.44 (d, $J$ = 5.1 Hz, 1H), 8.16 (s, 1H), 7.79 (d, $J$ = 8.9 Hz, 1H), 7.44 - 7.31 (m, 2H), 7.27 - 7.10 (m, 4H), 7.00 (d, $J$ = 2.6 Hz, 1H), 4.23 - 4.10 (m, 4H), 3.87 - 3.77 (m, 2H), 3.31 - 3.20 (m, 2H), 1.79 - 1.57 (m, 5H), 1.30 - 1.14 (m, 2H). MS: 570 [M+H]$^+$.

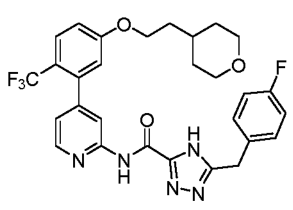

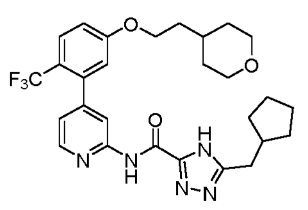

Example 308 Example 309

Example 309: 5-(cyclopentylmethyl)-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 2-bromo-3-methylphenol, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.41 (s, 1H), 9.90 (s, 1H), 8.44 (d, $J$ = 5.1 Hz, 1H), 8.17 (d, $J$ = 1.4 Hz, 1H), 7.79 (d, $J$ = 8.9 Hz, 1H), 7.25 - 7.14 (m, 2H), 7.00 (d, $J$ = 2.5 Hz, 1H), 4.14 (t, $J$ = 6.1 Hz, 2H), 3.93 - 3.69 (m, 2H), 3.30 - 3.23 (m, 2H), 2.77 (d, $J$ = 7.5 Hz, 2H), 2.36 - 2.19 (m, 1H), 1.78 - 1.42 (m, 11H), 1.31 - 1.13 (m, 4H). MS: 544 [M+H]$^+$.

Example 310: 5-(cyclohexylmethyl)-N-(4-(5-(2-(tetrahydro-2H-pyran-4-yl)ethoxy)-2-(trifluoromethyl)phenyl)pyridin-2-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 2-bromo-3-methylphenol, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.90 (s, 1H), 8.44 (d, $J$ = 5.0 Hz, 1H), 8.17 (s, 1H), 7.79 (d, $J$ = 8.9 Hz, 1H), 7.25 - 7.14 (m, 2H), 7.00 (d, $J$ = 2.5 Hz, 1H), 4.15 (t, $J$ = 6.1 Hz, 2H), 3.88 - 3.76 (m, 2H), 3.30 - 3.21 (m, 2H), 2.67 (d, $J$ = 7.0 Hz, 2H), 1.82 - 1.52 (m, 10H), 1.31 - 1.07 (m, 6H), 1.07 - 0.91 (m, 2H). MS: 558 [M+H]$^+$.

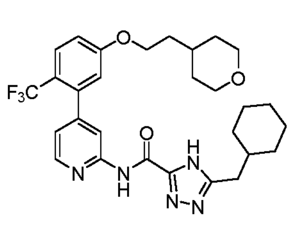

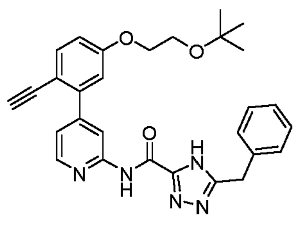

Example 310 Example 311

Example 311: 5-benzyl-N-(4-(5-(2-(tert-butoxy)ethoxy)-2-ethynylphenyl)pyridin-2-yl)-4H-1,2,4-triazole-3-carboxamide

The preparation was carried out in a similar manner to Example 64, except that in step 1, 3-bromo-4-ethynylphenol was used in place of 3-bromo-4-methylphenol. $^1$H NMR (400 MHz, DMSO-$d_6$) δ 14.56 (s, 1H), 9.87 (s, 1H), 8.43 (d, $J$ = 5.3 Hz, 1H), 8.37 (s, 1H), 7.57 (d, $J$ = 8.5 Hz, 1H), 7.42 - 7.29 (m, 5H), 7.26 (s, 1H), 7.10 - 6.97 (m, 2H), 4.18 (s, 2H), 4.16 - 4.09 (m, 2H), 4.05 (s, 1H), 3.65 (t, $J$ = 4.8 Hz, 2H), 1.15 (s, 9H). MS: 496 [M+H]$^+$.

Example 312: 5-benzyl-N-(4-(2-fluoro-5-((4-hydroxy-4-methylpentyl)oxy)phenyl)pyridin-2-yl)-4H-1,2,4-triazole-3-carboxamide

The preparation was carried out in a similar manner to Example 160, except that in step 1, 3-bromo-4-fluorophenol was used in place of 3-bromo-4-methylphenol. $^1$H NMR (400 MHz, DMSO-$d_6$) δ 14.57 (s, 1H), 9.90 (s, 1H), 8.46 (d, $J$ = 5.2 Hz, 1H), 8.35 (s, 1H), 7.40 (d, $J$ = 5.2 Hz, 1H), 7.38 - 7.29 (m, 5H), 7.29 - 7.19 (m, 1H), 7.16 - 6.99 (m, 2H), 4.24 - 4.11 (m, 3H), 4.01 (t, $J$ = 6.5 Hz, 2H), 1.84 - 1.69 (m, 2H), 1.55 - 1.43 (m, 2H), 1.10 (s, 6H). MS: 490 [M+H]$^+$.

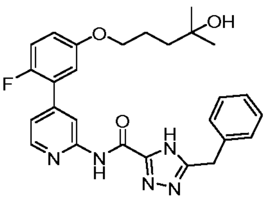

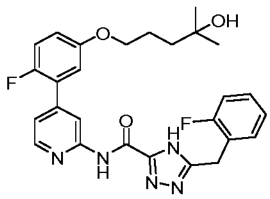

Example 312 Example 313

Example 313: 5-(2-fluorobenzyl)-N-(4-(2-fluoro-5-((4-hydroxy-4-methylpentyl)oxy)phenyl)pyridin-2-yl)-4H-1,2,4-triazole-3-carboxamide

The preparation was carried out in a similar manner to Example 160, except that in step 1, 3-bromo-4-fluorophenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.70 (s, 1H), 9.92 (s, 1H), 8.45 (d, *J* = 5.2 Hz, 1H), 8.34 (s, 1H), 7.48 - 7.26 (m, 4H), 7.26 - 7.15 (m, 2H), 7.15 - 6.99 (m, 2H), 4.26 - 4.14 (m, 3H), 4.01 (t, *J* = 6.5 Hz, 2H), 1.84 - 1.68 (m, 2H), 1.56 - 1.41 (m, 2H), 1.10 (s, 6H). MS: 508 [M+H]$^+$.

Example 314: N-(4-(5-((4-ethyl-4-hydroxyhexyl)oxy)-2-fluorophenyl)pyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 2, ethylmagnesium bromide was used in place of methylmagnesium bromide, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.21 (s, 1H), 9.91 (s, 1H), 8.45 (d, *J* = 5.2 Hz, 1H), 8.34 (s, 1H), 7.48 - 7.26 (m, 4H), 7.26 - 7.14 (m, 2H), 7.14 - 6.97 (m, 2H), 4.21 (s, 2H), 4.01 (t, *J* = 6.5 Hz, 2H), 3.91 - 3.88 (m, 1H), 1.77 - 1.57 (m, 2H), 1.48 - 1.39 (m, 2H), 1.35 (q, *J* = 7.5 Hz, 4H), 0.78 (t, *J* = 7.6 Hz, 6H). MS: 536 [M+H]$^+$.

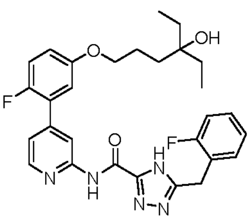

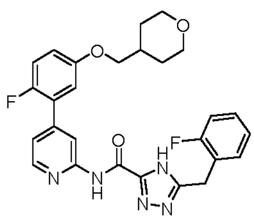

Example 314 Example 315

Example 315: 5-(2-fluorobenzyl)-N-(4-(2-fluoro-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.63 (s, 1H), 9.89 (s, 1H), 8.45 (d, *J* = 5.2 Hz, 1H), 8.33 (s, 1H), 7.48 - 7.26 (m, 4H), 7.26 - 7.15 (m, 2H), 7.15 - 7.02 (m, 2H), 4.21 (s, 2H), 3.95 - 3.81 (m, 4H), 3.40 - 3.33 (m, 2H), 2.07 - 1.93 (m, 1H), 1.75 - 1.60 (m, 2H), 1.41 - 1.25 (m, 2H). MS: 506 [M+H]$^+$.

Example 316: N-(4-(2-fluoro-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 0H), 9.89 (s, 1H), 8.45 (d, *J* = 5.2 Hz, 1H), 8.33 (s, 1H), 7.46 - 7.26 (m, 4H), 7.26 - 7.16 (m, 2H), 7.13 (dd, *J* = 6.3, 3.1 Hz, 1H), 7.11 - 7.04 (m,

1H), 4.21 (s, 2H), 4.07 (t, *J* = 6.2 Hz, 2H), 3.87 - 3.77 (m, 2H), 3.27 (dd, *J* = 11.6, 2.1 Hz, 2H), 2.09 - 1.90 (m, 1H), 1.70 - 1.59 (m, 4H), 1.23 - 1.12 (m, 2H). MS: 520 $[M+H]^+$.

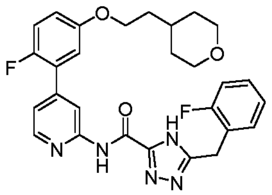
Example 316

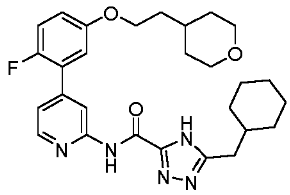
Example 317

Example 317: 5-(cyclohexylmethyl)-N-(4-(2-fluoro-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, and in step 4, ethyl 5-(cyclohexylmethyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.36 (s, 1H), 9.85 (s, 1H), 8.46 (d, *J* = 5.2 Hz, 1H), 8.37 (s, 1H), 7.41 (d, *J* = 5.2 Hz, 1H), 7.32 (dd, *J* = 10.4, 9.0 Hz, 1H), 7.13 (dd, *J* = 6.3, 3.1 Hz, 1H), 7.11-7.04 (m, 1H), 4.08 (t, *J* = 6.3 Hz, 2H), 3.88-3.76 (m, 2H), 3.30-3.20 (m, 2H), 2.68 (d, *J* = 7.1 Hz, 2H), 1.77-1.60 (m, 10H), 1.29-1.14 (m, 6H), 1.06-0.90 (m, 2H). MS: 508 $[M+H]^+$.

Example 318: N-(5-fluoro-4-(2-fluoro-5-((4-hydroxy-4-methylpentyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 3, 4-bromo-5-fluoropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.59 (s, 1H), 10.07 (s, 1H), 8.51 (d, *J* = 1.4 Hz, 1H), 8.20 (d, *J* = 5.4 Hz, 1H), 7.44 - 7.29 (m, 3H), 7.26 - 7.16 (m, 2H), 7.15 - 7.04 (m, 2H), 4.21 (s, 2H), 4.17 (s, 1H), 4.00 (t, *J* = 6.5 Hz, 2H), 1.82 - 1.68 (m, 2H), 1.54 - 1.42 (m, 2H), 1.10 (s, 6H). MS: 526 $[M+H]^+$.

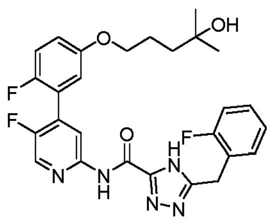
Example 318

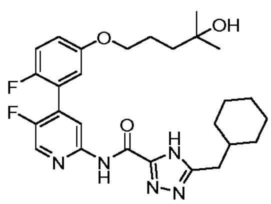
Example 319

Example 319: 5-(cyclohexylmethyl)-N-(5-fluoro-4-(2-fluoro-5-((4-hydroxy-4-methylpentyl)oxy)phenyl)pyridin-2-yl)-4H-1,2,4-triazole-3 -carboxamide

The preparation was carried out in a similar manner to Example 160, except that in step 1, 3-bromo-4-fluorophenol was used in place of 3-bromo-4-methylphenol, in step 3, 4-bromo-5-fluoropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.35 (s, 1H), 10.04 (s, 1H), 8.52 (d, *J* = 1.4 Hz, 1H), 8.23 (d, *J* = 5.5 Hz, 1H), 7.40 - 7.26 (m, 1H), 7.17 - 7.03 (m, 2H), 4.18 (s, 1H), 4.00 (t, *J* = 6.5 Hz, 2H), 2.67 (d, *J* = 7.0 Hz, 2H), 1.84 - 1.71 (m, 3H), 1.71 - 1.61 (m, 4H), 1.55 - 1.44 (m, 2H), 1.29 - 1.12 (m, 4H), 1.10 (s, 6H), 1.05 - 0.92 (m, 2H). MS: 514 $[M+H]^+$.

Example 320: N-(5-chloro-4-(2-fluoro-5-((4-hydroxy-4-methylpentyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 3, 4-bromo-5-chloropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 10.10 (s, 1H), 8.56 (s, 1H), 8.18 (s, 1H), 7.51 - 7.14 (m, 5H), 7.14 - 7.04 (m, 1H), 6.99 (dd, *J* = 5.9, 3.1 Hz, 1H), 4.26 - 4.14 (m, 3H), 3.98 (t, *J* = 6.6 Hz, 2H), 1.83 - 1.67 (m, 2H), 1.54 - 1.39 (m, 2H), 1.09 (s, 6H). MS: 542 [M+H]$^+$.

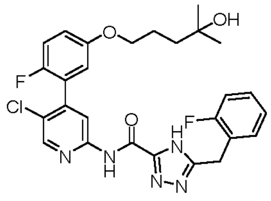

Example 320

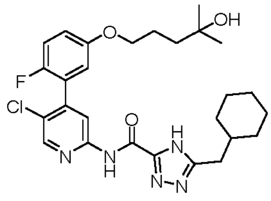

Example 321

Example 321: N-(5-chloro-4-(2-fluoro-5-((4-hydroxy-4-methylpentyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 3, 4-bromo-5-chloropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.08 (s, 1H), 8.58 (s, 1H), 8.20 (s, 1H), 7.37 - 7.26 (m, 1H), 7.14 - 7.06 (m, 1H), 7.00 (dd, *J* = 5.9, 3.1 Hz, 1H), 4.18 (s, 1H), 3.99 (t, *J* = 6.5 Hz, 2H), 2.72 - 2.61 (m, 2H), 1.83 - 1.71 (m, 3H), 1.71 - 1.62 (m, 4H), 1.51 - 1.43 (m, 2H), 1.28 - 1.11 (m, 4H), 1.10 (s, 6H), 1.06 - 0.90 (m, 2H). MS: 530 [M+H]$^+$.

Example 322: N-(4-(2-fluoro-5-((4-hydroxy-4-methylpentyl)oxy)phenyl)-5-methylpyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 3, 5-methyl-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 9.83 (s, 1H), 8.33 (s, 1H), 8.00 (s, 1H), 7.49 - 7.12 (m, 5H), 7.12 - 6.97 (m, 1H), 6.90 (dd, *J* = 6.0, 3.1 Hz, 1H), 4.27 - 4.13 (m, 3H), 3.98 (t, *J* = 6.6 Hz, 2H), 2.12 (s, 3H), 1.83 - 1.67 (m, 2H), 1.53 - 1.42 (m, 2H), 1.09 (s, 6H). MS: 522 [M+H]$^+$.

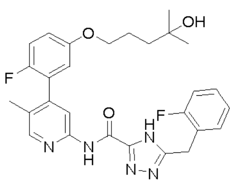

Example 322

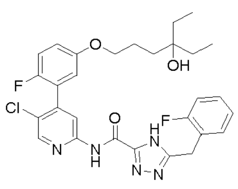

Example 323

Example 323: N-(5-chloro-4-(5-((4-ethyl-4-hydroxyhexyl)oxy)-2-fluorophenyl)pyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, in step 2, ethylmagnesium bromide was used in place of methylmagnesium bromide, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 10.14 (s, 1H), 8.57 (s, 1H), 8.17 (s, 1H), 7.46 - 7.25 (m,

3H), 7.25 - 7.15 (m, 2H), 7.14 - 7.05 (m, 1H), 6.99 (dd, *J* = 5.9, 3.1 Hz, 1H), 4.21 (s, 2H), 3.98 (t, *J* = 6.5 Hz, 2H), 3.88 (s, 1H), 1.75 - 1.61 (m, 2H), 1.48 - 1.38 (m, 2H), 1.34 (q, *J* = 7.4 Hz, 4H), 0.77 (t, *J* = 7.4 Hz, 6H). MS: 570 $[M+H]^+$.

Example 324: 5-(cyclohexylmethyl)-N-(5-chloro-4-(5-((4-ethyl-4-hydroxyhexyl)oxy)-2-fluorophenyl)pyridin-2-yl)-4H-1,2,4-triazole-3-carboxamide

The preparation was carried out in a similar manner to Example 160, except that in step 1, 3-bromo-4-fluorophenol was used in place of 3-bromo-4-methylphenol, in step 2, ethylmagnesium bromide was used in place of methylmagnesium bromide, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.38 (s, 1H), 10.08 (s, 1H), 8.58 (s, 1H), 8.20 (s, 1H), 7.39 - 7.23 (m, 1H), 7.15 - 7.04 (m, 1H), 6.99 (dd, *J* = 5.8, 3.1 Hz, 1H), 3.99 (t, *J* = 6.5 Hz, 2H), 3.88 (s, 1H), 2.67 (d, *J* = 6.9 Hz, 2H), 1.82 - 1.57 (m, 8H), 1.49 - 1.38 (m, 2H), 1.34 (q, *J* = 7.4 Hz, 4H), 1.28 - 1.09 (m, 3H), 1.06 - 0.91 (m, 2H), 0.77 (t, *J* = 7.5 Hz, 6H). MS: 558 $[M+H]^+$.

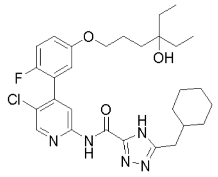

Example 324

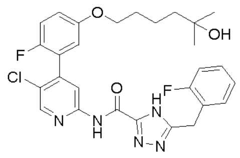

Example 325

Example 325: N-(5-chloro-4-(2-fhioro-5-((5-hydroxyl-5-methylhexyl)oxy)phenyl)pyridin-2-yl)-5-(2-fluorobenzyl)-4H-1,2,4-triazole-3-carboxamide

The preparation was carried out in a similar manner to Example 160, except that in step 1, methyl 5-bromopentanoate was used in place of methyl 4-bromobutanoate, 3-bromo-4-fluorophenol was used in place of 3-bromo-4-methylphenol, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.66 (s, 1H), 10.16 (s, 1H), 8.57 (s, 1H), 8.17 (s, 1H), 7.45 - 7.26 (m, 3H), 7.26 - 7.15 (m, 2H), 7.15 - 7.04 (m, 1H), 7.00 (dd, *J* = 5.8, 3.1 Hz, 1H), 4.21 (s, 2H), 4.10 (s, 1H), 3.99 (t, *J* = 6.4 Hz, 2H), 1.74 - 1.63 (m, 2H), 1.51 - 1.34 (m, 4H), 1.06 (s, 6H). MS: 556 $[M+H]^+$.

Example 326: 5-(cyclohexylmethyl)-N-(5-chloro-4-(2-fluoro-5-((5-hydroxyl-5-methylhexyl)oxy)phenyl)pyridin-2-yl)-4H-1,2,4-triazole-3-carboxamide

The preparation was carried out in a similar manner to Example 160, except that in step 1, methyl 5-bromopentanoate was used in place of methyl 4-bromobutanoate, 3-bromo-4-fluorophenol was used in place of 3-bromo-4-methylphenol, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.39 (s, 1H), 10.10 (s, 1H), 8.58 (s, 1H), 8.20 (s, 1H), 7.37 - 7.26 (m, 1H), 7.16 - 7.06 (m, 1H), 7.01 (dd, *J* = 5.8, 3.1 Hz, 1H), 4.10 (s, 1H), 4.00 (t, *J* = 6.5 Hz, 2H), 2.70 - 2.61 (m, 2H), 1.81 - 1.56 (m, 8H), 1.51 - 1.34 (m, 4H), 1.28 - 1.14 (m, 3H), 1.07 (s, 6H), 1.04 - 0.89 (m, 2H). MS: 544 $[M+H]^+$.

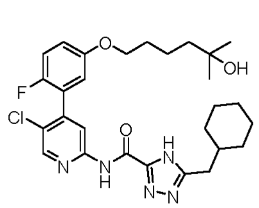

Example 326

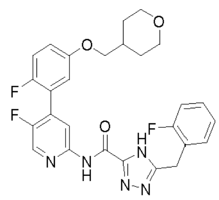

Example 327

Example 327: N-(5-fluoro-4-(2-fluoro-5-((tetrahydro-2H-pyran-4-yl)methoxy)phenyl)pyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-fluoro-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 10.05 (s, 1H), 8.51 (s, 1H), 8.25 - 8.09 (m, 1H), 7.45 - 7.07 (m, 6H), 6.53 (s, 1H), 4.23 (s, 2H), 3.93 - 3.82 (m, 4H), 3.35 (d, *J* = 2.1 Hz, 2H), 2.05 - 1.92 (m, 1H), 1.72 - 1.61 (m, 2H), 1.39 - 1.24 (m, 2H). MS: 524 $[M+H]^+$.

Example 328: 5-(cyclohexylmethyl)-N-(5-fluoro-4-(2-fluoro-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-fluoro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 10.03 (s, 1H), 8.55 - 8.49 (m, 1H), 8.24 (d, *J* = 5.4 Hz, 1H), 7.37 - 7.30 (m, 1H), 7.18 - 7.06 (m, 2H), 3.93 - 3.82 (m, 4H), 3.41 - 3.33 (m, 2H), 2.67 (d, *J* = 7.0 Hz, 2H), 2.05 - 1.90 (m, 1H), 1.83 - 1.55 (m, 8H), 1.40 - 1.26 (m, 2H), 1.26 - 1.06 (m, 3H), 1.06 - 0.91 (m, 2H). MS: 512 $[M+H]^+$.

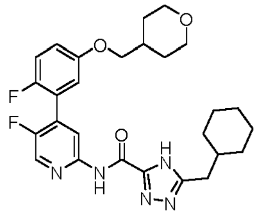

Example 328

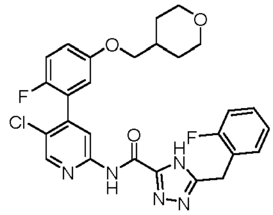

Example 329

Example 329: N-(5-chloro-4-(2-fluoro-5-((tetrahydro-2H-pyran-4-yl)methoxy)phenyl)pyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-chloro-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 10.12 (s, 1H), 8.57 (s, 1H), 8.17 (s, 1H), 7.46 - 7.26 (m, 3H), 7.26 - 7.15 (m, 2H), 7.15 - 7.06 (m, 1H), 7.01 (dd, *J* = 5.9, 3.1 Hz, 1H), 4.21 (s, 2H), 3.92 - 3.76 (m, 4H), 3.33 - 3.25 (m, 2H), 2.04 - 1.91 (m, 1H), 1.72 - 1.59 (m, 2H), 1.41 - 1.25 (m, 2H). MS: 540 $[M+H]^+$.

Example 330: 5-(cyclohexylmethyl)-N-(5-chloro-4-(2-fluoro-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-chloro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.09 (s, 1H), 8.58 (s, 1H), 8.20 (s, 1H), 7.37 - 7.26 (m, 1H), 7.16 - 7.07 (m, 1H), 7.02 (dd, *J* = 5.9, 3.1 Hz, 1H), 3.93 - 3.82 (m, 4H), 3.34 - 3.27 (m, 2H), 2.71 - 2.63 (m, 2H), 2.06 - 1.92 (m, 1H), 1.85 - 1.52 (m, 8H), 1.39 - 1.14 (m, 5H), 1.06 - 0.90 (m, 2H). MS: 528 $[M+H]^+$.

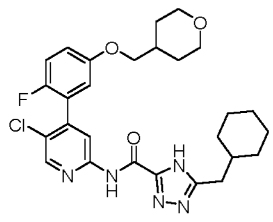

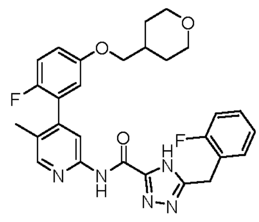

Example 330 Example 331

Example 331: N-(4-(2-fluoro-5-((tetrahydro-2H-pyran-4-yl)methoxy)phenyl)-5-methylpyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-methyl-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 9.83 (s, 1H), 8.33 (s, 1H), 8.00 (s, 1H), 7.46 - 7.14 (m, 5H), 7.10 - 7.01 (m, 1H), 6.92 (dd, *J* = 5.9, 3.1 Hz, 1H), 4.20 (s, 2H), 3.95 - 3.76 (m, 4H), 3.32 - 3.24 (m, 2H), 2.12 (s, 3H), 2.05 - 1.92 (m, 1H), 1.74 - 1.61 (m, 2H), 1.39 - 1.24 (m, 2H). MS: 520 $[M+H]^+$.

Example 332: 5-(cyclohexylmethyl)-N-(4-(2-fluoro-5-((tetrahydro-2H-pyran-4-yl)methoxy)phenyl)-5-methylpyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-fluorophenol was used in place of 3-bromo-4-methylphenol, in step 2, 5-methyl-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 9.80 (s, 1H), 8.34 (s, 1H), 8.03 (s, 1H), 7.34-7.24 (m, 1H), 7.11-7.02 (m, 1H), 6.92 (dd, *J* = 6.0, 3.1 Hz, 1H), 3.93-3.79 (m, 4H), 3.33-3.27 (m, 2H), 2.70-2.62 (m, 2H), 2.13 (s, 3H), 2.05-1.93 (m, 1H), 1.82-1.55 (m, 8H), 1.40-1.14 (m, 5H), 1.05-0.90 (m, 2H). MS: 508 $[M+H]^+$.

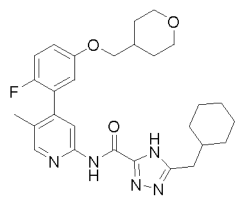

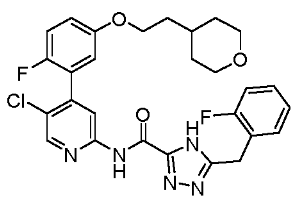

Example 332 Example 333

Example 333: N-(5-chloro-4-(2-fluoro-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 10.11 (s, 1H), 8.57 (s, 1H), 8.18 (s, 1H), 7.50-7.27 (m, 3H), 7.27-7.15 (m, 2H), 7.15-7.06 (m, 1H), 7.02 (dd, *J* = 5.9, 3.1 Hz, 1H), 4.22 (s, 2H), 4.04 (t, *J* = 6.3 Hz, 2H), 3.86-3.78 (m, 2H), 3.32-3.23 (m, 2H), 1.78-1.56 (m, 5H), 1.29-1.12 (m, 2H). MS: 554 $[M+H]^+$.

Example 334: N-(5-chloro-4-(2-fluoro-5-(2-(tetrahydro-2H-pyran-4-yl)ethoxy)phenyl)pyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.08 (s, 1H), 8.58 (s, 1H), 8.20 (s, 1H), 7.36-7.25 (m, 1H), 7.16-7.06 (m, 1H), 7.02 (dd, $J$ = 5.9, 3.1 Hz, 1H), 4.05 (t, $J$ = 6.2 Hz, 2H), 3.87-3.78 (m, 2H), 3.31-3.24 (m, 2H), 2.67 (d, $J$ = 7.0 Hz, 2H), 1.81-1.56 (m, 11H), 1.28-1.13 (m, 5H), 1.04-0.90 (m, 2H). MS: 542 [M+H]$^+$.

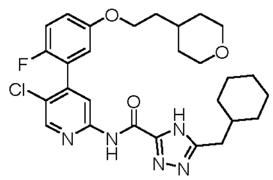
Example 334

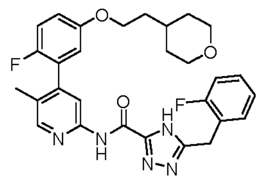
Example 335

Example 335: N-(4-(2-fluoro-5-(2-(tetrahydro-2H-pyran-4-yl)ethoxy)phenyl)-5-methylpyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, in step 3, 5-methyl-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.86 (s, 1H), 8.33 (s, 1H), 8.00 (s, 1H), 7.45 - 7.14 (m, 5H), 7.11 - 7.01 (m, 1H), 6.92 (dd, $J$ = 6.0, 3.1 Hz, 1H), 4.20 (s, 2H), 4.03 (t, $J$ = 6.3 Hz, 2H), 3.86 - 3.78 (m, 2H), 3.30 - 3.24 (m, 2H), 2.12 (s, 3H), 1.77 - 1.54 (m, 5H), 1.30 - 1.08 (m, 2H). MS: 534 [M+H]$^+$.

Example 336: 5-(cyclohexylmethyl)-N-(4-(2-fluoro-5-(2-(tetrahydro-2H-pyran-4-yl)ethoxy)phenyl)-5-methylpyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-fluorophenol was used in place of 3-bromo-4-methylphenol, in step 3, 5-methyl-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 9.79 (s, 1H), 8.34 (s, 1H), 8.03 (s, 1H), 7.35 - 7.22 (m, 1H), 7.11 - 7.02 (m, 1H), 6.93 (dd, $J$ = 6.0, 3.1 Hz, 1H), 4.04 (t, $J$ = 6.3 Hz, 2H), 3.87 - 3.77 (m, 2H), 3.32 - 3.22 (m, 2H), 2.66 (d, $J$ = 7.1 Hz, 2H), 2.13 (s, 3H), 1.81 - 1.55 (m, 11H), 1.30 - 1.11 (m, 5H), 1.05 - 0.89 (m, 2H). MS: 522 [M+H]$^+$.

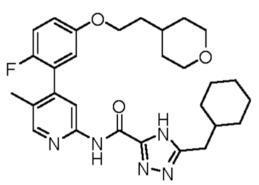
Example 336

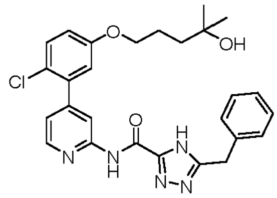
Example 337

Example 337: 5-benzyl-N-(4-(5-((4-hydroxy-4-methylpentyl)oxy)-2-chlorophenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol. $^1$H NMR (400 MHz, DMSO-$d_6$) δ 14.58 (s, 1H), 9.93 (s, 1H), 8.45 (d, $J$ = 5.1 Hz, 1H), 8.23 (s, 1H), 7.51 (d, $J$ = 8.8 Hz, 1H), 7.43 - 7.18 (m, 6H), 7.13 - 6.93 (m, 2H), 4.26 - 4.09 (m, 3H), 4.01 (t, $J$ = 6.6 Hz, 2H), 1.83 - 1.69 (m, 2H), 1.53 - 1.41 (m, 2H), 1.09 (s, 6H). MS: 506 [M+H]$^+$.

Example 338: N-(4-(2-chloro-5-((4-hydroxy-4-methylpentyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 9.95 (s, 1H), 8.45 (d, *J* = 5.2 Hz, 1H), 8.21 (s, 1H), 7.51 (d, *J* = 8.8 Hz, 1H), 7.45 - 7.14 (m, 5H), 7.06 (dd, *J* = 8.7, 3.0 Hz, 1H), 7.01 (d, *J* = 3.0 Hz, 1H), 4.27 - 4.16 (m, 3H), 4.01 (t, *J* = 6.6 Hz, 2H), 1.83 - 1.69 (m, 2H), 1.53 - 1.39 (m, 2H), 1.09 (s, 6H). MS: 524 [M+H]$^+$.

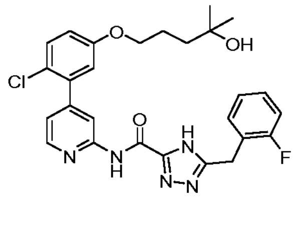

Example 338

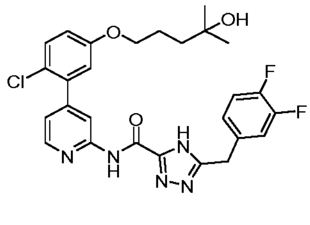

Example 339

Example 339: N-(4-(2-chloro-5-((4-hydroxy-4-methylpentyl)oxy)phenyl)pyridin-2-yl)-5-(3,4-difluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(3,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 9.99 (s, 1H), 8.46 (d, *J* = 5.1 Hz, 1H), 8.22 (s, 1H), 7.51 (d, *J* = 8.8 Hz, 1H), 7.48 - 7.36 (m, 2H), 7.29 (dd, *J* = 5.1, 1.6 Hz, 1H), 7.22 - 7.14 (m, 1H), 7.06 (dd, *J* = 8.8, 3.0 Hz, 1H), 7.01 (d, *J* = 3.0 Hz, 1H), 4.26 - 4.13 (m, 3H), 4.01 (t, *J* = 6.6 Hz, 2H), 1.83 - 1.69 (m, 2H), 1.52 - 1.41 (m, 2H), 1.09 (s, 6H). MS: 542 [M+H]$^+$.

Example 340: 5-(5-chloro-2-fluorobenzyl)-N-(4-(2-chloro-5-((4-hydroxy-4-methylpentyl)oxy)phenyl)pyridin-2-yl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl 5-(5-chloro-2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 9.94 (s, 1H), 8.46 (d, *J* = 5.1 Hz, 1H), 8.21 (s, 1H), 7.60 - 7.46 (m, 2H), 7.46 - 7.36 (m, 1H), 7.36 - 7.22 (m, 2H), 7.13 - 6.95 (m, 2H), 4.27 - 4.15 (m, 3H), 4.01 (t, *J* = 6.6 Hz, 2H), 1.82 - 1.69 (m, 2H), 1.52 - 1.41 (m, 2H), 1.09 (s, 6H). MS: 558 [M+H]$^+$.

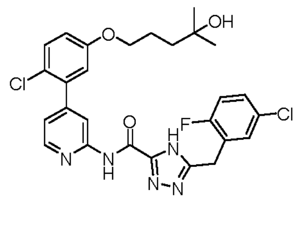

Example 340

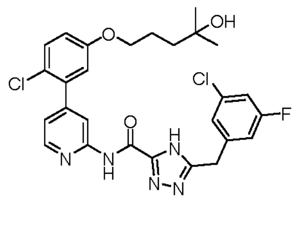

Example 341

Example 341: N-(4-(2-chloro-5-((4-hydroxy-4-methylpentyl)oxy)phenyl)pyridin-2-yl)-5-(3-chloro-5-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(3-chloro-5-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.02 (s, 1H), 8.46 (d, *J* = 5.1 Hz, 1H), 8.22 (s, 1H), 7.51 (d, *J* = 8.8 Hz, 1H), 7.43 - 7.33 (m, 1H), 7.33 - 7.26 (m, 2H), 7.26 - 7.16 (m, 1H), 7.06 (dd, *J* = 8.8, 3.0 Hz, 1H), 7.01 (d, *J* = 3.0 Hz, 1H), 4.23 (s, 2H), 4.19 (s, 1H), 4.01 (t,

*J* = 6.6 Hz, 2H), 1.82 - 1.69 (m, 2H), 1.53 - 1.42 (m, 2H), 1.09 (s, 6H). MS: 558 $[M+H]^+$.

Example 342: 5-(3-chloro-4-fluorobenzyl)-N-(4-(2-chloro-5-((4-hydroxy-4-methylpentyl)oxy)phenyl)pyridin-2-yl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl 5-(3-chloro-4-fluorobenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.58 (s, 1H), 9.98 (s, 1H), 8.46 (d, *J* = 5.1 Hz, 1H), 8.22 (s, 1H), 7.59 (dd, *J* = 7.2, 2.0 Hz, 1H), 7.51 (d, *J* = 8.8 Hz, 1H), 7.43 - 7.32 (m, 2H), 7.29 (dd, *J* = 5.1, 1.6 Hz, 1H), 7.06 (dd, *J* = 8.8, 3.0 Hz, 1H), 7.01 (d, *J* = 3.0 Hz, 1H), 4.28 - 4.14 (m, 3H), 4.01 (t, *J* = 6.5 Hz, 2H), 1.82 - 1.70 (m, 2H), 1.52 - 1.42 (m, 2H), 1.09 (s, 6H). MS: 558 $[M+H]^+$.

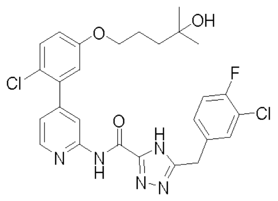

Example 342

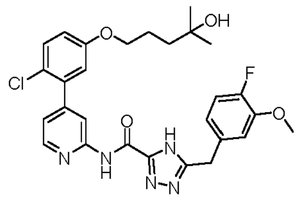

Example 343

Example 343: N-(4-(2-chloro-5-((4-hydroxy-4-methylpentyl)oxy)phenyl)pyridin-2-yl)-5-(4-fluoro-3-methoxybenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(4-fluoro-3-methoxybenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.52 (s, 1H), 9.92 (s, 1H), 8.46 (d, *J* = 5.1 Hz, 1H), 8.23 (s, 1H), 7.51 (d, *J* = 8.8 Hz, 1H), 7.31 - 7.25 (m, 1H), 7.20 - 7.12 (m, 2H), 7.06 (dd, *J* = 8.8, 3.0 Hz, 1H), 7.01 (d, *J* = 3.0 Hz, 1H), 6.89 - 6.83 (m, 1H), 4.24 - 4.11 (m, 3H), 4.01 (t, *J* = 6.5 Hz, 2H), 3.83 (s, 3H), 1.83 - 1.69 (m, 2H), 1.53 - 1.41 (m, 2H), 1.09 (s, 6H). MS: 554 $[M+H]^+$.

Example 344: N-(4-(2-chloro-5-((4-hydroxy-4-methylpentyl)oxy)phenyl)pyridin-2-yl)-5-(3-fluoro-5-methylbenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(3-fluoro-5-methylbenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.58 (s, 1H), 9.96 (s, 1H), 8.46 (d, *J* = 5.1 Hz, 1H), 8.22 (s, 1H), 7.51 (d, *J* = 8.8 Hz, 1H), 7.28 (dd, *J* = 5.1, 1.6 Hz, 1H), 7.11 - 6.85 (m, 5H), 4.23 - 4.09 (m, 3H), 4.01 (t, *J* = 6.6 Hz, 2H), 2.30 (s, 3H), 1.82 - 1.70 (m, 2H), 1.55 - 1.39 (m, 2H), 1.09 (s, 6H). MS: 538 $[M+H]^+$.

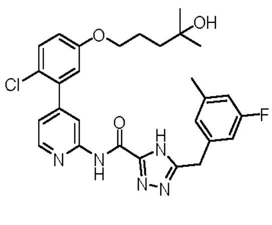

Example 344

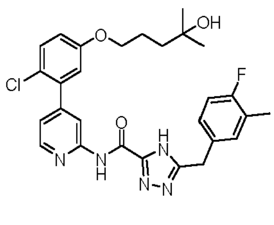

Example 345

Example 345: N-(4-(2-chloro-5-((4-hydroxy-4-methylpentyl)oxy)phenyl)pyridin-2-yl)-5-(4-fluoro-3-methylbenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(4-fluoro-3-methylbenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.52

(s, 1H), 9.91 (s, 1H), 8.46 (d, *J* = 5.1 Hz, 1H), 8.22 (s, 1H), 7.51 (d, *J* = 8.8 Hz, 1H), 7.36 - 6.94 (m, 6H), 4.18 (s, 1H), 4.13 (s, 2H), 4.01 (t, *J* = 6.6 Hz, 2H), 2.21 (d, *J* = 2.0 Hz, 3H), 1.83 - 1.69 (m, 2H), 1.54 - 1.41 (m, 2H), 1.09 (s, 6H). MS: 538 $[M+H]^+$.

Example 346: N-(4-(2-chloro-5-((4-hydroxy-4-methylpentyl)oxy)phenyl)pyridin-2-yl)-5-(2-fluoro-3-(trifluoromethyl)benzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2-fluoro-3-(trifluoromethyl)benzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.76 (s, 1H), 9.98 (s, 1H), 8.45 (d, *J* = 5.2 Hz, 1H), 8.21 (s, 1H), 7.86 - 7.65 (m, 2H), 7.57 - 7.34 (m, 2H), 7.28 (d, *J* = 5.1 Hz, 1H), 7.11 - 6.91 (m, 2H), 4.32 (s, 2H), 4.18 (s, 1H), 4.01 (t, *J* = 6.5 Hz, 2H), 1.83 - 1.66 (m, 2H), 1.55 - 1.39 (m, 2H), 1.09 (s, 6H). MS: 592 $[M+H]^+$.

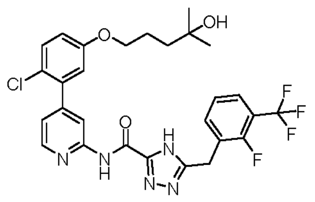

Example 346

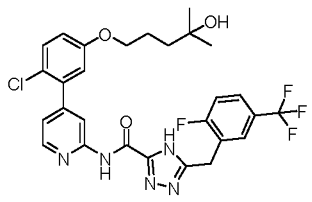

Example 347

Example 347: N-(4-(2-chloro-5-((4-hydroxy-4-methylpentyl)oxy)phenyl)pyridin-2-yl)-5-(2-fluoro-5-(trifluoromethyl)benzyl)-4H- 1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl 5-(2-fluoro-5-(trifluoromethyl)benzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 9.92 (s, 1H), 8.45 (d, *J* = 5.1 Hz, 1H), 8.21 (s, 1H), 7.90 (d, *J* = 6.4 Hz, 1H), 7.78 (d, *J* = 8.8 Hz, 1H), 7.56-7.41 (m, 2H), 7.28 (dd, *J* = 5.2, 1.6 Hz, 1H), 7.11-6.93 (m, 2H), 4.32 (s, 2H), 4.18 (s, 1H), 4.01 (t, *J* = 6.6 Hz, 2H), 1.83-1.68 (m, 2H), 1.54-1.40 (m, 2H), 1.09 (s, 6H). MS: 592 $[M+H]^+$.

Example 348: N-(4-(2-chloro-5-((4-hydroxy-4-methylpentyl)oxy)phenyl)pyridin-2-yl)-5-(3-fluoro-5-(trifluoromethyl)benzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 4, ethyl 5-(3-fluoro-5-(trifluoromethyl)benzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.02 (s, 1H), 8.46 (d, *J* = 5.1 Hz, 1H), 8.22 (s, 1H), 7.68 - 7.45 (m, 4H), 7.29 (dd, *J* = 5.1, 1.6 Hz, 1H), 7.12 - 6.94 (m, 2H), 4.34 (s, 2H), 4.19 (s, 1H), 4.01 (t, *J*= 6.6 Hz, 2H), 1.84 - 1.69 (m, 2H), 1.53 - 1.41 (m, 2H), 1.09 (s, 6H). MS: 592 $[M+H]^+$.

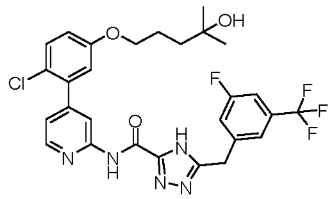

Example 348

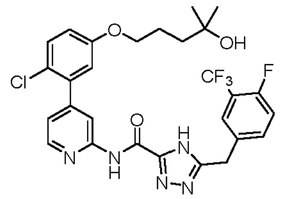

Example 349

Example 349: N-(4-(2-chloro-5-((4-hydroxy-4-methylpentyl)oxy)phenyl)pyridin-2-yl)-5-(4-fluoro-3-(trifluoromethyl)benzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(4-fluoro-3-(trifluoromethyl)benzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.53 (s, 1H), 9.91 (s, 1H), 8.45 (d, *J* = 5.2 Hz, 1H), 8.20 (d, *J* = 20.8 Hz, 1H), 7.88-7.64 (m, 2H), 7.57-7.44 (m, 2H), 7.33-7.21 (m, 1H), 7.06 (dd, *J* = 8.9, 3.0 Hz, 1H), 7.01 (d, *J* = 3.0 Hz, 1H), 4.32 (s, 2H), 4.17 (s, 1H), 4.01 (t, *J* = 6.6 Hz, 2H), 1.82-1.70 (m, 2H), 1.52-1.41 (m, 2H), 1.09 (s, 6H). MS: 592 $[M+H]^+$.

Example 350: N-(4-(2-chloro-5-((4-hydroxy-4-methylpentyl)oxy)phenyl)pyridin-2-yl)-5-(5-ethyl-2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(5-ethyl-2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 9.88 (s, 1H), 8.45 (d, *J* = 5.2 Hz, 1H), 8.22 (s, 1H), 7.50 (d, *J* = 8.8 Hz, 1H), 7.35 - 6.93 (m, 6H), 4.25 - 4.09 (m, 3H), 4.09 - 3.93 (m, 2H), 2.58 (q, *J* = 7.6 Hz, 2H), 1.82 - 1.68 (m, 2H), 1.55 - 1.38 (m, 2H), 1.16 (t, *J* = 7.6 Hz 3H), 1.09 (s, 6H). MS: 552 $[M+H]^+$.

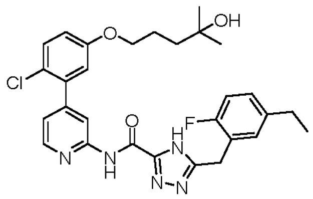

Example 350

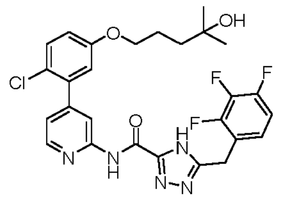

Example 351

Example 351: N-(4-(2-chloro-5-((4-hydroxy-4-methylpentyl)oxy)phenyl)pyridin-2-yl)-5-(2,3,4-trifluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2,3,4-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.89 (s, 1H), 8.46 (d, *J* = 5.2 Hz, 1H), 8.22 (s, 1H), 7.51 (d, *J* = 8.8 Hz, 1H), 7.41 - 7.21 (m, 3H), 7.12 - 6.95 (m, 2H), 4.28 (s, 2H), 4.17 (s, 1H), 4.01 (t, *J* = 6.5 Hz, 2H), 1.84 - 1.69 (m, 2H), 1.53 - 1.39 (m, 2H), 1.09 (s, 6H). MS: 560 $[M+H]^+$.

Example 352: N-(4-(2-chloro-5-((4-hydroxy-4-methylpentyl)oxy)phenyl)pyridin-2-yl)-5-(2,3,5-trifluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2,3,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.75 (s, 1H), 10.01 (s, 1H), 8.46 (d, *J* = 5.1 Hz, 1H), 8.28 - 8.13 (m, 1H), 7.56 - 7.41 (m, 2H), 7.29 (dd, *J* = 5.1, 1.6 Hz, 1H), 7.25 - 7.14 (m, 1H), 7.10 - 6.95 (m, 2H), 4.28 (s, 2H), 4.17 (s, 1H), 4.01 (t, *J* = 6.6 Hz, 2H), 1.82 - 1.69 (m, 2H), 1.54 - 1.39 (m, 2H), 1.09 (s, 6H). MS: 560 $[M+H]^+$.

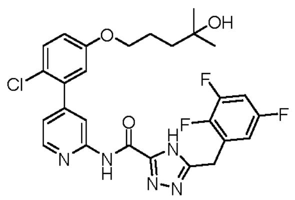

Example 352

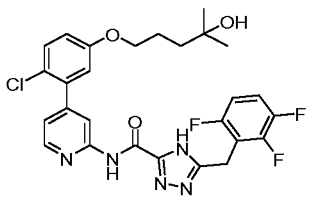

Example 353

Example 353: N-(4-(2-chloro-5-((4-hydroxy-4-methylpentyl)oxy)phenyl)pyridin-2-yl)-5-(2,3,6-trifluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2,3,6-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.78 (s, 1H), 9.95 (s, 1H), 8.46 (d, *J* = 5.1 Hz, 1H), 8.20 (s, 1H), 7.58 - 7.42 (m, 2H), 7.29 (dd, *J* = 5.1, 1.6 Hz, 1H), 7.25 - 7.14 (m, 1H), 7.10 - 6.93 (m, 2H), 4.26 (s, 2H), 4.19 (s, 1H), 4.01 (t, *J* = 6.6 Hz, 2H), 1.84 - 1.69 (m, 2H), 1.53 - 1.39 (m, 2H), 1.10 (s, 6H). MS: 560 [M+H]$^+$.

Example 354: N-(4-(2-chloro-5-((4-hydroxy-4-methylpentyl)oxy)phenyl)pyridin-2-yl)-5-(3,4,5-trifluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(3,4,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.61 (s, 1H), 10.04 (s, 1H), 8.47 (d, *J* = 5.1 Hz, 1H), 8.23 (s, 1H), 7.51 (d, *J* = 8.8 Hz, 1H), 7.41 - 7.23 (m, 3H), 7.08 - 6.97 (m, 2H), 4.26 - 4.15 (m, 3H), 4.01 (t, *J* = 6.6 Hz, 2H), 1.82 - 1.70 (m, 2H), 1.53 - 1.42 (m, 2H), 1.09 (s, 6H). MS: 560 [M+H]$^+$.

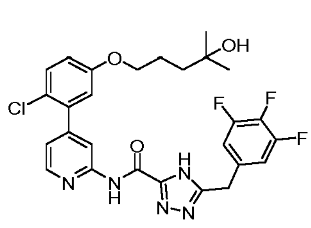

Example 354

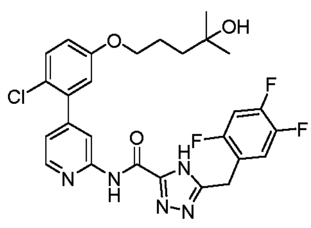

Example 355

Example 355: N-(4-(2-chloro-5-((4-hydroxy-4-methylpentyl)oxy)phenyl)pyridin-2-yl)-5-(2,4,5-trifluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(2,4,5-trifluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 9.92 (s, 1H), 8.46 (d, *J* = 5.1 Hz, 1H), 8.22 (s, 1H), 7.72 - 7.44 (m, 3H), 7.29 (d, *J* = 4.8 Hz, 1H), 7.12 - 6.94 (m, 2H), 4.28 - 4.07 (m, 3H), 4.01 (t, *J* = 6.5 Hz, 2H), 1.83 - 1.66 (m, 2H), 1.55 - 1.39 (m, 2H), 1.09 (s, 6H). MS: 560 [M+H]$^+$.

Example 356: N-(4-(2-chloro-5-((4-hydroxy-4-methylpentyl)oxy)phenyl)pyridin-2-yl)-5-(cyclopentylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.39 (s, 1H), 9.90 (s, 1H), 8.46 (d, *J* = 5.2 Hz, 1H), 8.24 (s, 1H), 7.51 (d, *J* = 8.8 Hz, 1H), 7.28 (dd, *J* = 5.1, 1.6 Hz, 1H), 7.12 - 6.96 (m, 2H), 4.19 (s, 1H), 4.02 (t, *J* = 6.5 Hz, 2H), 2.78 (d, *J* = 7.5 Hz, 2H), 2.36 - 2.20 (m, 1H), 1.82 - 1.66 (m, 4H), 1.66 - 1.57 (m, 2H), 1.57 - 1.43 (m, 4H), 1.24 - 1.20 (m, 2H), 1.09 (s, 6H). MS: 498 [M+H]$^+$.

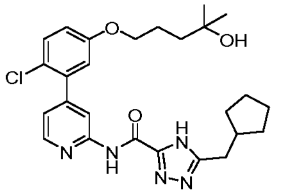

Example 356

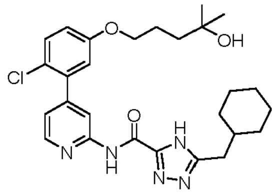

Example 357

Example 357: N-(4-(2-chloro-5-((4-hydroxy-4-methylpentyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 9.92 (s, 1H), 8.46 (d, *J* = 5.1 Hz, 1H), 8.30 - 8.18 (m, 1H), 7.51 (d, *J* = 8.8 Hz, 1H), 7.28 (dd, *J* = 5.1, 1.6 Hz, 1H), 7.13 - 6.94 (m, 2H), 4.21 (s, 1H), 4.01 (t, *J* = 6.5 Hz, 2H), 2.67 (d, *J* = 7.0 Hz, 2H), 1.87 - 1.55 (m, 8H), 1.51 - 1.41 (m, 2H), 1.21 - 1.12 (m, 3H), 1.09 (s, 6H), 1.05 - 0.89 (m, 2H). MS: 512 $[M+H]^+$.

Example 358: N-(4-(2-chloro-5-((4-hydroxy-4-methylpentyl)oxy)phenyl)pyridin-2-yl)-5-(pyrrolidin-1-ylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(pyrrolidin-1-ylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 13.83 (s, 1H), 9.95 (s, 1H), 8.52 - 8.40 (m, 1H), 8.29 - 8.19 (m, 1H), 7.51 (d, *J* = 8.8 Hz, 1H), 7.27 (dd, *J* = 5.1, 1.6 Hz, 1H), 7.13 - 6.94 (m, 2H), 4.18 (s, 1H), 4.01 (t, *J* = 6.6 Hz, 2H), 3.82 (s, 2H), 2.60 - 2.53 (m, 4H), 1.85 - 1.63 (m, 6H), 1.55 - 1.42 (m, 2H), 1.09 (s, 6H). MS: 499 $[M+H]^+$.

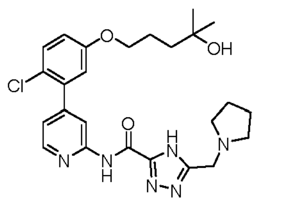
Example 358

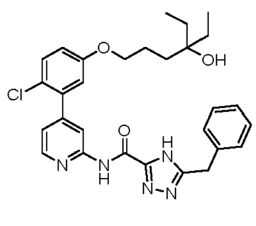
Example 359

Example 359: 5-benzyl-N-(4-(2-chloro-5-((4-ethyl-4-hydroxyhexyl)oxy)phenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 2, ethylmagnesium bromide was used in place of methylmagnesium bromide. $^1$H NMR (400 MHz, DMSO-$d_6$) δ 14.64 (s, 1H), 9.96 (s, 1H), 8.45 (d, *J* = 5.1 Hz, 1H), 8.30 - 8.17 (m, 1H), 7.50 (d, *J* = 8.8 Hz, 1H), 7.44 - 7.18 (m, 6H), 7.13 - 6.92 (m, 2H), 4.18 (s, 2H), 4.00 (t, *J* = 6.5 Hz, 2H), 3.89 (s, 1H), 1.78 - 1.59 (m, 2H), 1.51 - 1.38 (m, 2H), 1.34 (q, *J* = 7.4 Hz, 4H), 0.77 (t, *J* = 7.4 Hz, 6H). MS: 534 $[M+H]^+$.

Example 360: N-(4-(2-chloro-5-((4-ethyl-4-hydroxyhexyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.94 (s, 1H), 8.45 (d, *J* = 5.1 Hz, 1H), 8.21 (d, *J* = 1.6 Hz, 1H), 7.50 (d, *J* = 8.8 Hz, 1H), 7.47 - 7.30 (m, 2H), 7.28 (dd, *J* = 5.1, 1.6 Hz, 1H), 7.25 - 7.16 (m, 2H), 7.05 (dd, *J* = 8.8, 3.0 Hz, 1H), 7.00 (d, *J* = 3.0 Hz, 1H), 4.21 (s, 2H), 4.01 (t, *J* = 6.5 Hz, 2H), 3.89 (s, 1H), 1.76 - 1.62 (m, 2H), 1.47 - 1.38 (m, 2H), 1.34 (q, *J* = 7.4 Hz, 4H), 0.77 (t, *J* = 7.5 Hz, 6H). MS: 552 $[M+H]^+$.

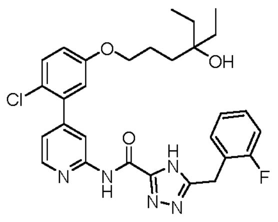

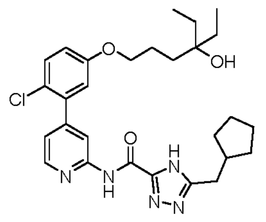

Example 360 Example 361

Example 361: N-(4-(2-chloro-5-((4-ethyl-4-hydroxyhexyl)oxy)phenyl)pyridin-2-yl)-5-(cyclopentylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.42 (s, 1H), 9.90 (s, 1H), 8.46 (dd, *J* = 5.1, 0.8 Hz, 1H), 8.32-8.18 (m, 1H), 7.51 (d, *J* = 8.8 Hz, 1H), 7.28 (dd, *J* = 5.1, 1.6 Hz, 1H), 7.12-6.94 (m, 2H), 4.01 (t, *J* = 6.5 Hz, 2H), 3.89 (s, 1H), 2.78 (d, *J* = 7.5 Hz, 2H), 2.36 - 2.19 (m, 1H), 1.80-1.64 (m, 4H), 1.64 - 1.47 (m, 4H), 1.47 - 1.29 (m, 6H), 1.29-1.14 (m, 2H), 0.77 (t, *J* = 7.4 Hz, 6H). MS: 526 [M+H]$^+$.

Example 362: N-(4-(2-chloro-5-((4-ethyl-4-hydroxyhexyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.46 (d, *J* = 5.1 Hz, 1H), 8.24 (d, *J* = 1.4 Hz, 1H), 7.51 (d, *J* = 8.8 Hz, 1H), 7.28 (dd, *J* = 5.1, 1.6 Hz, 1H), 7.16-6.95 (m, 2H), 4.01 (t, *J* = 6.5 Hz, 2H), 3.89 (s, 1H), 2.67 (d, *J* = 7.1 Hz, 2H), 1.86-1.56 (m, 8H), 1.48-1.39 (m, 2H), 1.35 (q, *J* = 7.5 Hz, 4H), 1.25-1.12 (m, 3H), 1.05-0.92 (m, 2H), 0.78 (t, *J* = 7.5 Hz, 6H). MS: 540 [M+H]$^+$.

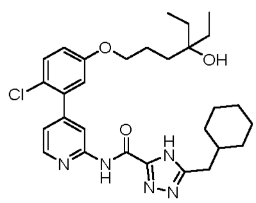

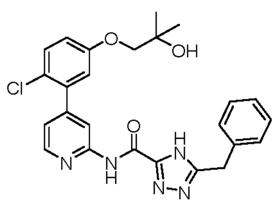

Example 362 Example 363

Example 363: 5-benzyl-N-(4-(2-chloro-5-(2-hydroxyl-2-methylpropoxy)phenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol, and methyl bromoacetate was used in place of methyl 4-bromobutanoate. $^1$H NMR (400 MHz, DMSO-$d_6$) δ 14.63 (s, 1H), 9.96 (s, 1H), 8.55-8.39 (m, 1H), 8.29-8.16 (m, 1H), 7.51 (d, *J* = 8.8 Hz, 1H), 7.41-7.19 (m, 6H), 7.13-6.99 (m, 2H), 4.66 (s, 1H), 4.18 (s, 2H), 3.77 (s, 2H), 1.19 (s, 6H). MS: 478 [M+H]$^+$.

Example 364: 5-(2-fluorobenzyl)-N-(4-(2-chloro-5-(2-hydroxyl-2-methylpropoxy)phenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol, methyl bromoacetate was used in place of methyl 4-bromobutanoate, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.70 (s, 1H), 9.94 (s, 1H), 8.45 (d, $J$ = 5.1 Hz, 1H), 8.31 - 8.12 (m, 1H), 7.51 (d, $J$ = 8.8 Hz, 1H), 7.47 - 7.26 (m, 3H), 7.26 - 7.13 (m, 2H), 7.13 - 6.96 (m, 2H), 4.66 (s, 1H), 4.21 (s, 2H), 3.77 (s, 2H), 1.19 (s, 6H). MS: 496 [M+H]$^+$.

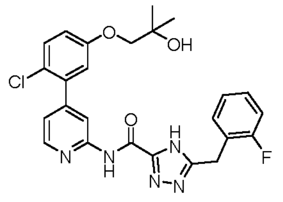

Example 364

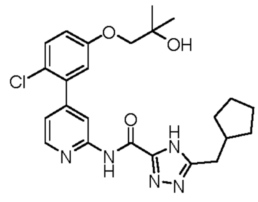

Example 365

Example 365: N-(4-(2-chloro-5-(2-hydroxyl-2-methylpropoxy)phenyl)pyridin-2-yl)-5-(cyclopentylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, methyl bromoacetate was used in place of methyl 4-bromobutanoate, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) $\delta$ 14.40 (s, 1H), 9.91 (s, 1H), 8.46 (dd, $J$ = 5.1, 0.8 Hz, 1H), 8.34 - 8.19 (m, 1H), 7.52 (d, $J$ = 8.8 Hz, 1H), 7.29 (dd, $J$ = 5.1, 1.6 Hz, 1H), 7.15 - 6.93 (m, 2H), 4.66 (s, 1H), 3.78 (s, 2H), 2.78 (d, $J$ = 7.5 Hz, 2H), 2.37 - 2.19 (m, 1H), 1.82 - 1.66 (m, 2H), 1.66 - 1.44 (m, 4H), 1.30 - 1.12 (m, 8H). MS: 470 [M+H]$^+$.

Example 366: N-(4-(2-chloro-5-(2-hydroxyl-2-methylpropoxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, methyl bromoacetate was used in place of methyl 4-bromobutanoate, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.39 (s, 1H), 9.90 (s, 1H), 8.46 (d, $J$ = 5.1 Hz, 1H), 8.24 (d, $J$ = 1.4 Hz, 1H), 7.52 (d, $J$ = 8.8 Hz, 1H), 7.29 (dd, $J$ = 5.1, 1.6 Hz, 1H), 7.16 - 6.95 (m, 2H), 4.66 (s, 1H), 3.78 (s, 2H), 2.67 (d, $J$ = 7.0 Hz, 2H), 1.83 - 1.71 (m, 1H), 1.71 - 1.53 (m, 5H), 1.29 - 1.06 (m, 9H), 1.06 - 0.89 (m, 2H). MS: 484 [M+H]$^+$.

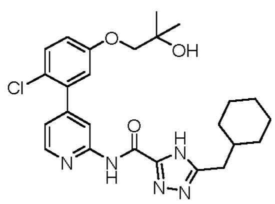

Example 366

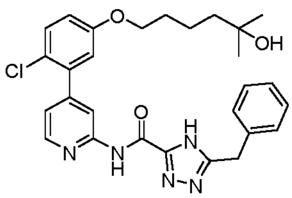

Example 367

Example 367: 5-benzyl-N-(4-(2-chloro-5-((5-hydroxyl-5-methylhexyl)oxy)phenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol, and methyl 5-bromopentanoate was used in place of methyl 4-bromobutanoate. $^1$H NMR (400 MHz, DMSO-$d_6$) $\delta$ 14.65 (s, 1H), 9.96 (s, 1H), 8.45 (dd, $J$ = 5.1, 0.8 Hz, 1H), 8.23 (dd, $J$ = 1.5, 0.8 Hz, 1H), 7.50 (d, $J$ = 8.8 Hz, 1H), 7.45 - 7.20 (m, 6H), 7.13 - 6.97 (m, 2H), 4.18 (s, 2H), 4.10 (s, 1H), 4.02 (t, $J$ = 6.5 Hz, 2H), 1.75 - 1.62 (m, 2H), 1.51 - 1.32 (m, 4H), 1.06 (s, 6H). MS: 520 [M+H]$^+$.

Example 368: 5-(2-fluorobenzyl)-N-(4-(2-chloro-5-((5-hydroxyl-5-methylhexyl)oxy)phenyl)pyridin-2-yl)-4H-1,2,4-triazole-3-carboxamide

The preparation was carried out in a similar manner to Example 160, except that in step 1, 3-bromo-4-chlorophenol was used in place of 3-bromo-4-methylphenol, methyl 5-bromopentanoate was used in place of methyl 4-bromobutanoate, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.71 (s, 1H), 9.94 (s, 1H), 8.44 (d, *J* = 5.1 Hz, 1H), 8.31 - 8.13 (m, 1H), 7.49 (d, *J* = 8.8 Hz, 1H), 7.45 - 7.30 (m, 2H), 7.27 (dd, *J* = 5.1, 1.6 Hz, 1H), 7.25 - 7.15 (m, 2H), 7.09 - 6.98 (m, 2H), 4.21 (s, 2H), 4.11 (s, 1H), 4.01 (t, *J* = 6.4 Hz, 2H), 1.69 (t, *J* = 7.0 Hz, 2H), 1.49 - 1.33 (m, 4H), 1.06 (s, 6H). MS: 538 $[M+H]^+$.

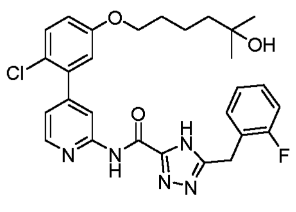

Example 368

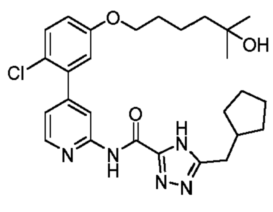

Example 369

Example 369: N-(4-(2-chloro-5-((5-hydroxyl-5-methylhexyl)oxy)phenyl)pyridin-2-yl)-5-(cyclopentylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, methyl 5-bromopentanoate was used in place of methyl 4-bromobutanoate, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.42 (s, 1H), 9.90 (s, 1H), 8.46 (dd, *J* = 5.1, 0.8 Hz, 1H), 8.25 (dd, *J* = 1.6, 0.8 Hz, 1H), 7.51 (d, *J* = 8.8 Hz, 1H), 7.28 (dd, *J* = 5.1, 1.6 Hz, 1H), 7.11 - 6.96 (m, 2H), 4.10 (s, 1H), 4.02 (t, *J* = 6.5 Hz, 2H), 2.78 (d, *J* = 7.5 Hz, 2H), 2.36 - 2.19 (m, 1H), 1.79 - 1.65 (m, 4H), 1.65 - 1.33 (m, 8H), 1.30 - 1.15 (m, 2H), 1.06 (s, 6H). MS: 512 $[M+H]^+$.

Example 370: N-(4-(2-chloro-5-((5-hydroxyl-5-methylhexyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, methyl 5-bromopentanoate was used in place of methyl 4-bromobutanoate, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.90 (s, 1H), 8.46 (d, *J* = 5.1 Hz, 1H), 8.24 (s, 1H), 7.51 (d, *J* = 8.8 Hz, 1H), 7.28 (dd, *J* = 5.1, 1.6 Hz, 1H), 7.13 - 6.97 (m, 2H), 4.10 (s, 1H), 4.03 (t, *J* = 6.4 Hz, 2H), 2.67 (d, *J* = 7.0 Hz, 2H), 1.84 - 1.58 (m, 7H), 1.52 - 1.35 (m, 4H), 1.31 - 1.10 (m, 4H), 1.06 (s, 6H), 1.02 - 0.92 (m, 2H). MS: 526 $[M+H]^+$.

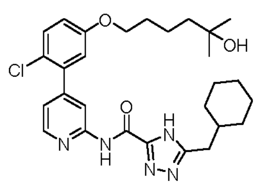

Example 370

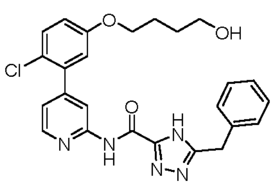

Example 371

Example 371: 5-benzyl-N-(4-(2-chloro-5-(4-hydroxybutoxy)phenyl)pyridin-2-yl)-4H-1,2,4-triazole-3-carboxamide

The preparation was carried out in a similar manner to Example 92, except that in step 1, 4-bromo-1-butanol was used in place of iodomethane, and 3-bromo-4-chlorophenol was used in place of 2-bromo-3-methylphenol. $^1$H NMR (400 MHz, DMSO-$d_6$) δ 14.56 (s, 1H), 9.92 (s, 1H), 8.45 (d, *J* = 5.1 Hz, 1H), 8.22 (s, 1H), 7.51 (d, *J* = 8.8 Hz, 1H), 7.40 - 7.30 (m, 4H), 7.30 - 7.21 (m, 2H), 7.06 (dd, *J* = 8.8, 3.0 Hz, 1H), 7.02 (d, *J* = 3.0 Hz, 1H), 4.45 (t, *J* = 5.1 Hz, 1H), 4.18 (s, 2H), 4.03 (t, *J* = 6.5 Hz, 2H), 3.47 - 3.41 (m, 2H), 1.81 - 1.69 (m, 2H), 1.61 - 1.50 (m, 2H). MS: 478 $[M+H]^+$.

Example 372: 5-(2-fluorobenzyl)-N-(4-(2-chloro-5-(4-hydroxybutoxy)phenyl)pyridin-2-yl)-4H-1,2,4-triazole-3-carboxamide

The preparation was carried out in a similar manner to Example 92, except that in step 1, 4-bromo-1-butanol was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.54 (s, 1H), 9.91 (s, 1H), 8.45 (d, *J* = 5.1 Hz, 1H), 8.21 (s, 1H), 7.51 (d, *J* = 8.8 Hz, 1H), 7.46 - 7.31 (m, 2H), 7.28 (d, *J* = 5.2 Hz, 1H), 7.26 - 7.15 (m, 2H), 7.11 - 6.98 (m, 2H), 4.45 (t, *J* = 5.2 Hz, 1H), 4.21 (s, 2H), 4.03 (t, *J* = 6.5 Hz, 2H), 3.46 - 3.42 (m, 2H), 1.80 - 1.67 (m, 2H), 1.62 - 1.49 (m, 2H). MS: 496 [M+H]$^+$.

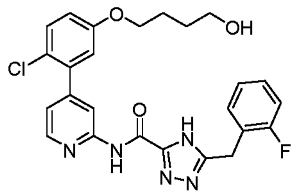

Example 372

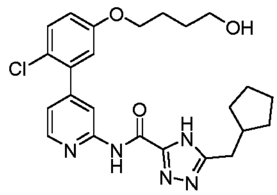

Example 373

Example 373: N-(4-(2-chloro-5-(4-hydroxybutoxy)phenyl)pyridin-2-yl)-5-(cyclopentylmethyl)-4H-1,2,4-triazole-3-carboxamide

The preparation was carried out in a similar manner to Example 92, except that in step 1, 4-bromo-1-butanol was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.88 (s, 1H), 8.46 (d, *J* = 5.2 Hz, 1H), 8.24 (s, 1H), 7.51 (d, *J* = 8.8 Hz, 1H), 7.29 (d, *J* = 5.0 Hz, 1H), 7.12 - 6.99 (m, 2H), 4.45 (t, *J* = 5.2 Hz, 1H), 4.04 (t, *J* = 6.5 Hz, 2H), 3.46 - 3.43 (m, 2H), 2.78 (d, *J* = 7.5 Hz, 2H), 2.31 - 2.23 (m, 1H), 1.78 - 1.68 (m, 4H), 1.65 - 1.48 (m, 6H), 1.25 - 1.22 (m, 2H). MS: 470 [M+H]$^+$.

Example 374: N-(4-(2-chloro-5-(4-hydroxybutoxy)phenyl)pyridin-2-yl)-5-(cyclohexylmethyl)-4H-1,2,4-triazole-3-carboxamide

The preparation was carried out in a similar manner to Example 92, except that in step 1, 4-bromo-1-butanol was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 9.89 (s, 1H), 8.46 (dd, *J* = 5.1, 0.8 Hz, 1H), 8.24 (d, *J* = 1.1 Hz, 1H), 7.51 (d, *J* = 8.8 Hz, 1H), 7.28 (dd, *J* = 5.1, 1.6 Hz, 1H), 7.13 - 6.98 (m, 2H), 4.45 (t, *J* = 5.2 Hz, 1H), 4.04 (t, *J* = 6.5 Hz, 2H), 3.47 - 3.41 (m, 2H), 2.67 (d, *J* = 7.0 Hz, 2H), 1.81 - 1.52 (m, 10H), 1.22 - 0.90 (m, 5H). MS: 484 [M+H]$^+$.

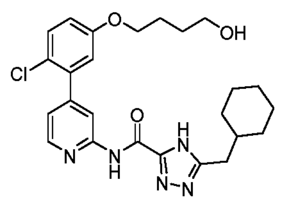

Example 374

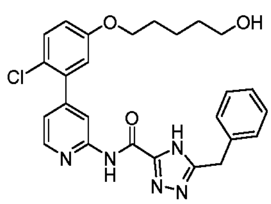

Example 375

Example 375: 5-benzyl-N-(4-(2-chloro-5-((5-hydroxypentyl)oxy)phenyl)pyridin-2-yl)-4H-1,2,4-triazole-3-carboxamide

The preparation was carried out in a similar manner to Example 92, except that in step 1, 5-bromo-1-pentanol was used in place of iodomethane, and 3-bromo-4-chlorophenol was used in place of 2-bromo-3-methylphenol. $^1$H NMR (400 MHz, DMSO-$d_6$) δ 14.61 (s, 1H), 9.95 (s, 1H), 8.45 (d, *J* = 5.0 Hz, 1H), 8.22 (d, *J* = 1.6 Hz, 1H), 7.51 (d, *J* = 8.8 Hz, 1H), 7.39 - 7.30 (m, 4H), 7.30 - 7.19 (m, 2H), 7.06 (dd, *J* = 8.8, 3.0 Hz, 1H), 7.02 (d, *J* = 3.0 Hz, 1H), 4.44 - 4.30 (m, 1H), 4.18 (s, 2H), 4.02 (t, *J* = 6.5 Hz, 2H), 3.44 - 3.37 (m, 2H), 1.78 - 1.64 (m, 2H), 1.53 - 1.35 (m, 4H). MS: 492 [M+H]$^+$.

Example 376: 5-(2-fluorobenzyl)-N-(4-(2-chloro-5-((5-hydroxypentyl)oxy)phenyl)pyridin-2-yl)-4H-1,2,4-triazole-3-carboxamide

The preparation was carried out in a similar manner to Example 92, except that in step 1, 5-bromo-1-pentanol was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.94 (s, 1H), 8.45 (d, *J* = 5.2 Hz, 1H), 8.21 (d, *J* = 1.6 Hz, 1H), 7.50 (d, *J* = 8.8 Hz, 1H), 7.45 - 7.31 (m, 2H), 7.28 (dd, *J* = 5.1, 1.6 Hz, 1H), 7.26 - 7.13 (m, 2H), 7.11 - 6.99 (m, 2H), 4.38 (s, 1H), 4.21 (s, 2H), 4.01 (t, *J* = 6.5 Hz, 2H), 3.45 - 3.37 (m, 2H), 1.79 - 1.66 (m, 2H), 1.52 - 1.37 (m, 4H). MS: 510 [M+H]$^+$.

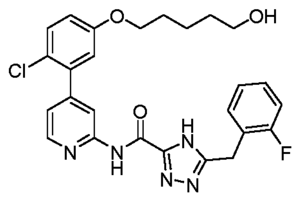

Example 376

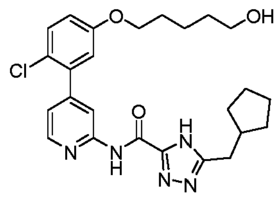

Example 377

Example 377: N-(4-(2-chloro-5-((5-hydroxypentyl)oxy)phenyl)pyridin-2-yl)-5-(cyclopentylmethyl)-4H-1,2,4-triazole-3-carboxamide

The preparation was carried out in a similar manner to Example 92, except that in step 1, 5-bromo-1-pentanol was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.89 (s, 1H), 8.46 (d, *J* = 5.1 Hz, 1H), 8.24 (s, 1H), 7.51 (d, *J* = 8.8 Hz, 1H), 7.28 (dd, *J* = 5.1, 1.6 Hz, 1H), 7.14 - 6.96 (m, 2H), 4.38 (t, *J* = 5.2 Hz, 1H), 4.02 (t, *J* = 6.5 Hz, 2H), 3.46 - 3.35 (m, 2H), 2.78 (d, *J* = 7.5 Hz, 2H), 2.36 - 2.20 (m, 1H), 1.80 - 1.67 (m, 4H), 1.67 - 1.38 (m, 8H), 1.30 - 1.15 (m, 2H). MS: 484 [M+H]$^+$.

Example 378: N-(4-(2-chloro-5-((5-hydroxypentyl)oxy)phenyl)pyridin-2-yl)-5-(cyclohexylmethyl)-4H-1,2,4-triazole-3-carboxamide

The preparation was carried out in a similar manner to Example 92, except that in step 1, 5-bromo-1-pentanol was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 9.89 (s, 1H), 8.46 (d, *J* = 5.1 Hz, 1H), 8.24 (d, *J* = 1.5 Hz, 1H), 7.51 (d, *J* = 8.8 Hz, 1H), 7.28 (dd, *J* = 5.1, 1.6 Hz, 1H), 7.07 (dd, *J* = 8.8, 3.1 Hz, 1H), 7.02 (d, *J* = 3.0 Hz, 1H), 4.38 (t, *J* = 5.2 Hz, 1H), 4.02 (t, *J* = 6.5 Hz, 2H), 3.45-3.37 (m, 2H), 2.67 (d, *J* = 7.0 Hz, 2H), 1.84-1.54 (m, 8H), 1.52-1.36 (m, 4H), 1.27-1.11 (m, 3H), 1.04-0.91 (m, 2H). MS: 498 [M+H]$^+$.

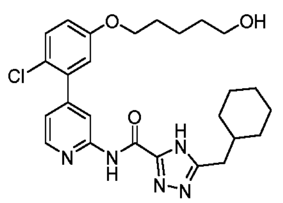

Example 378

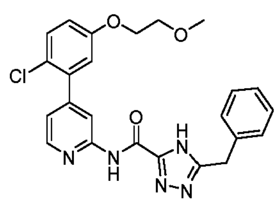

Example 379

Example 379: 5-benzyl-N-(4-(2-chloro-5-(2-methoxyethoxy)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, and 3-bromo-4-chlorophenol was used in place of 2-bromo-3-methylphenol. $^1$H NMR (400 MHz, DMSO-$d_6$) δ 14.63 (s, 1H), 9.96 (s, 1H), 8.45 (dd, *J* = 5.1, 0.8 Hz, 1H), 8.24 (dd, *J* = 1.6, 0.8 Hz, 1H), 7.51 (d, *J* = 8.7 Hz, 1H), 7.43 - 7.30 (m, 4H), 7.30 - 7.20 (m, 2H), 7.15 - 6.98 (m, 2H), 4.25 - 4.10 (m, 4H), 3.71 -

3.59 (m, 2H), 3.30 (s, 3H). MS: 464 [M+H]+.

Example 380: 5-(2-fluorobenzyl)-N-(4-(2-chloro-5-(2-methoxyethoxy)phenyl)pyridin-2-yl)-4H-1,2,4-triazole-3-carboxamide

The preparation was carried out in a similar manner to Example 92, except that in step 1, 1-bromo-2-methoxyethane was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.94 (s, 1H), 8.56 - 8.38 (m, 1H), 8.31 - 8.14 (m, 1H), 7.51 (d, $J$ = 8.8 Hz, 1H), 7.46 - 7.14 (m, 5H), 7.14 - 6.94 (m, 2H), 4.21 (s, 2H), 4.18 - 4.11 (m, 2H), 3.73 - 3.60 (m, 2H), 3.30 (s, 3H). MS: 482 [M+H]+.

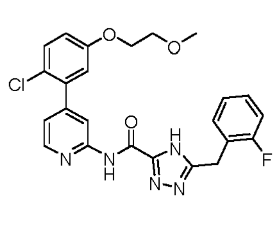
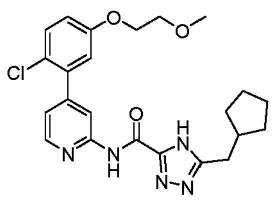

Example 380 Example 381

Example 381: N-(4-(2-chloro-5-(2-methoxyethoxy)phenyl)pyridin-2-yl)-5-(cyclopentylmethyl)-4H-1,2,4-triazole-3-carboxamide

The preparation was carried out in a similar manner to Example 92, except that in step 1, 1-bromo-2-methoxyethane was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.46 (d, $J$ = 5.1 Hz, 1H), 8.25 (t, $J$ = 1.1 Hz, 1H), 7.52 (d, $J$ = 8.8 Hz, 1H), 7.29 (dd, $J$ = 5.1, 1.6 Hz, 1H), 7.16 - 6.98 (m, 2H), 4.25 - 4.09 (m, 2H), 3.75 - 3.61 (m, 2H), 3.30 (s, 3H), 2.78 (d, $J$ = 7.5 Hz, 2H), 2.37 - 2.18 (m, 1H), 1.81 - 1.67 (m, 2H), 1.67 - 1.44 (m, 4H), 1.32 - 1.13 (m, 2H). MS: 456 [M+H]+.

Example 382: N-(4-(2-chloro-5-(2-methoxyethoxy)phenyl)pyridin-2-yl)-5-(cyclohexylmethyl)-4H-1,2,4-triazole-3-carboxamide

The preparation was carried out in a similar manner to Example 92, except that in step 1, 1-bromo-2-methoxyethane was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.46 (d, $J$ = 5.1 Hz, 1H), 8.25 (d, $J$ = 1.5 Hz, 1H), 7.52 (d, $J$ = 8.8 Hz, 1H), 7.29 (dd, $J$ = 5.1, 1.6 Hz, 1H), 7.16 - 7.00 (m, 2H), 4.23 - 4.10 (m, 2H), 3.72 - 3.61 (m, 2H), 3.30 (s, 3H), 2.67 (d, $J$ = 7.1 Hz, 2H), 1.85 - 1.53 (m, 6H), 1.28 - 1.11 (m, 3H), 1.03 - 0.90 (m, 2H). MS: 470 [M+H]+.

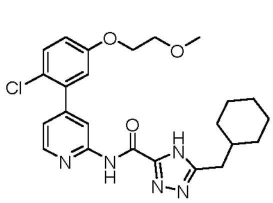
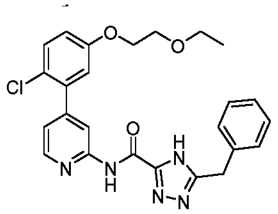

Example 382 Example 383

Example 383: 5-benzyl-N-(4-(2-chloro-5-(2-ethoxyethoxy)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, and 3-bromo-4-chlorophenol was used in place of 2-bromo-3-methylphenol. $^1$H NMR (400 MHz, DMSO-$d_6$) δ 14.60 (s, 1H), 9.94 (s, 1H), 8.46 (d, $J$ = 5.2 Hz, 1H), 8.23 (s, 1H), 7.52 (d, $J$ = 8.8 Hz,

1H), 7.43-7.30 (m, 4H), 7.30-7.22 (m, 2H), 7.18-6.97 (m, 2H), 4.22 - 4.11 (m, 4H), 3.73 - 3.66 (m, 2H), 3.49 (q, *J* = 7.0 Hz, 2H), 1.12 (t, *J* = 7.0 Hz, 3H). MS: 478 $[M+H]^+$.

Example 384: 5-(2-fluorobenzyl)-N-(4-(2-chloro-5-(2-ethoxyethoxy)phenyl)pyridin-2-yl)-4H-1,2,4-triazole-3-carboxamide

The preparation was carried out in a similar manner to Example 92, except that in step 1, 1-bromo-2-ethoxyethane was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.94 (s, 1H), 8.45 (d, *J* = 5.2 Hz, 1H), 8.28 - 8.16 (m, 1H), 7.51 (d, *J* = 8.8 Hz, 1H), 7.46 - 7.13 (m, 5H), 7.13 - 6.97 (m, 2H), 4.21 (s, 2H), 4.18 - 4.09 (m, 2H), 3.75 - 3.63 (m, 2H), 3.49 (q, *J* = 7.0 Hz, 2H), 1.11 (t, *J* = 7.0 Hz, 3H). MS: 496 $[M+H]^+$.

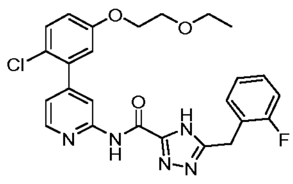
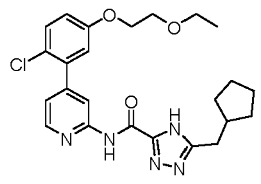

Example 384 Example 385

Example 385: N-(4-(2-chloro-5-(2-ethoxyethoxy)phenyl)pyridin-2-yl)-5-(cyclopentylmethyl)-4H-1,2,4-triazole-3-carboxamide

The preparation was carried out in a similar manner to Example 92, except that in step 1, 1-bromo-2-ethoxyethane was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.90 (s, 1H), 8.46 (dd, *J* = 5.1, 0.8 Hz, 1H), 8.25 (t, *J* = 1.1 Hz, 1H), 7.52 (d, *J* = 8.8 Hz, 1H), 7.29 (dd, *J* = 5.1, 1.6 Hz, 1H), 7.17-6.96 (m, 2H), 4.23-4.09 (m, 2H), 3.75-3.65 (m, 2H), 3.49 (q, *J* = 7.0 Hz, 2H), 2.78 (d, *J* = 7.5 Hz, 2H), 2.36-2.18 (m, 1H), 1.80-1.67 (m, 2H), 1.67-1.57 (m, 2H), 1.57-1.44 (m, 2H), 1.25-1.16 (m, 2H), 1.12 (t, *J* = 7.0 Hz, 3H). MS: 470 $[M+H]^+$.

Example 386: N-(4-(2-chloro-5-(2-ethoxyethoxy)phenyl)pyridin-2-yl)-5-(cyclohexylmethyl)-4H-1,2,4-triazole-3-carboxamide

The preparation was carried out in a similar manner to Example 92, except that in step 1, 1-bromo-2-ethoxyethane was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 9.90 (s, 1H), 8.46 (d, *J* = 5.1 Hz, 1H), 8.25 (s, 1H), 7.52 (d, *J* = 8.8 Hz, 1H), 7.29 (dd, *J* = 5.1, 1.7 Hz, 1H), 7.20 - 7.00 (m, 2H), 4.16 (dd, *J* = 5.6, 3.5 Hz, 2H), 3.70 (dd, *J* = 5.6, 3.4 Hz, 2H), 3.50 (q, *J* = 7.0 Hz, 2H), 2.67 (d, *J* = 7.0 Hz, 2H), 1.83 - 1.53 (m, 6H), 1.25 - 1.14 (m, 3H), 1.12 (t, *J* = 7.0 Hz, 3H), 1.06 - 0.88 (m, 2H). MS: 484 $[M+H]^+$.

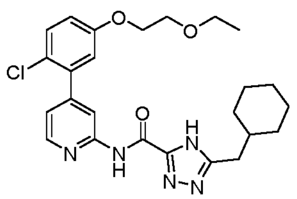
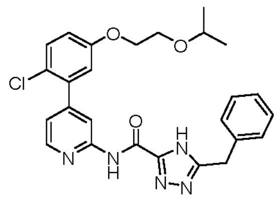

Example 386 Example 387

Example 387: 5-benzyl-N-(4-(2-chloro-5-(2-isopropoxyethoxy)phenyl)pyridin-2-yl)-4H-1,2,4-triazole-3-carboxamide

The preparation was carried out in a similar manner to Example 92, except that in step 1, 1-bromo-2-isopropoxyethane was used in place of iodomethane, and 3-bromo-4-chlorophenol was used in place of 2-bromo-3-methyl-

phenol. $^1$H NMR (400 MHz, DMSO-$d_6$) δ 14.63 (s, 1H), 9.96 (s, 1H), 8.46 (dd, *J* = 5.1, 0.8 Hz, 1H), 8.29 - 8.16 (m, 1H), 7.51 (d, *J* = 8.7 Hz, 1H), 7.44 - 7.18 (m, 6H), 7.18 - 6.96 (m, 2H), 4.18 (s, 2H), 4.15 - 4.05 (m, 2H), 3.75 - 3.66 (m, 2H), 3.61 (p, *J* = 6.1 Hz, 1H), 1.09 (d, *J* = 6.0 Hz, 6H). MS: 492 $[M+H]^+$.

Example 388: 5-(2-fluorobenzyl)-N-(4-(2-chloro-5-(2-isopropoxyethoxy)phenyl)pyridin-2-yl)-4H-1,2,4-triazole-3-carboxamide

The preparation was carried out in a similar manner to Example 92, except that in step 1, 1-bromo-2-isopropoxyethane was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 9.94 (s, 1H), 8.45 (dd, *J* = 5.1, 0.8 Hz, 1H), 8.30 - 8.14 (m, 1H), 7.51 (d, *J* = 8.8 Hz, 1H), 7.46 - 7.25 (m, 3H), 7.25 - 7.13 (m, 2H), 7.13 - 6.97 (m, 2H), 4.21 (s, 2H), 4.17 - 4.06 (m, 2H), 3.74 - 3.65 (m, 2H), 3.61 (p, *J* = 6.1 Hz, 1H), 1.09 (d, *J* = 6.1 Hz, 6H). MS: 510 $[M+H]^+$.

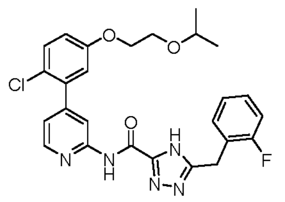
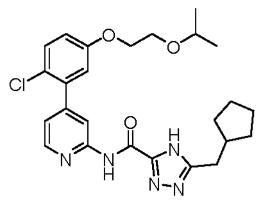

Example 388 Example 389

Example 389: N-(4-(2-chloro-5-(2-isopropoxyethoxy)phenyl)pyridin-2-yl)-5-(cyclopentylmethyl)-4H-1,2,4-triazole-3-carboxamide

The preparation was carried out in a similar manner to Example 92, except that in step 1, 1-bromo-2-isopropoxyethane was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.90 (s, 1H), 8.46 (d, *J* = 5.1 Hz, 1H), 8.25 (d, *J* = 1.4 Hz, 1H), 7.52 (d, *J* = 8.7 Hz, 1H), 7.29 (dd, *J* = 5.1, 1.6 Hz, 1H), 7.19 - 6.99 (m, 2H), 4.22 - 4.05 (m, 2H), 3.75 - 3.66 (m, 2H), 3.62 (p, *J* = 6.1 Hz, 1H), 2.78 (d, *J* = 7.5 Hz, 2H), 2.36 - 2.20 (m, 1H), 1.84 - 1.66 (m, 2H), 1.66 - 1.46 (m, 4H), 1.26 - 1.18 (m, 2H), 1.10 (d, *J*= 6.1 Hz, 6H). MS: 484 $[M+H]^+$.

Example 390: N-(4-(2-chloro-5-(2-isopropoxyethoxy)phenyl)pyridin-2-yl)-5-(cyclohexylmethyl)-4H-1,2,4-triazole-3-carboxamide

The preparation was carried out in a similar manner to Example 92, except that in step 1, 1-bromo-2-isopropoxyethane was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.47 (d, *J* = 5.2 Hz, 1H), 8.25 (s, 1H), 7.52 (d, *J* = 8.8 Hz, 1H), 7.29 (dd, *J* = 5.1, 1.6 Hz, 1H), 7.15 - 6.96 (m, 2H), 4.18 - 4.09 (m, 2H), 3.74 - 3.65 (m, 2H), 3.62 (p, *J* = 6.1 Hz, 1H), 2.67 (d, *J* = 7.1 Hz, 2H), 1.85 - 1.56 (m, 6H), 1.26 - 1.17 (m, 3H), 1.10 (d, *J* = 6.1 Hz, 6H), 1.05 - 0.91 (m, 2H). MS: 498 $[M+H]^+$.

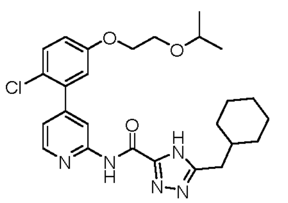
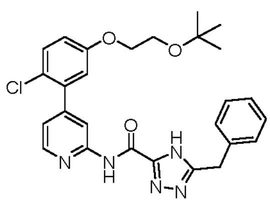

Example 390 Example 391

Example 391: 5-benzyl-N-(4-(5-(2-(tert-butoxy)ethoxy)-2-chlorophenyl)pyridin-2-yl)-4H-1,2,4-triazole-3-carboxamide

The preparation was carried out in a similar manner to Example 64, except that in step 1, 3-bromo-4-chlorophenol was used in place of 3-bromo-4-methylphenol. $^1$H NMR (400 MHz, DMSO-$d_6$) $\delta$ 14.58 (s, 1H), 9.93 (s, 1H), 8.45 (d, $J$ = 5.2 Hz, 1H), 8.23 (s, 1H), 7.51 (d, $J$ = 8.7 Hz, 1H), 7.43 - 7.18 (m, 6H), 7.14 - 6.97 (m, 2H), 4.18 (s, 2H), 4.13 - 4.00 (m, 2H), 3.64 (t, $J$ = 4.8 Hz, 2H), 1.15 (s, 9H). MS: 506 $[M+H]^+$.

Example 392: 5-(2-fluorobenzyl)-N-(4-(5-(2-(tert-butoxy)ethoxy)-2-chlorophenyl)pyridin-2-yl)-4H-1,2,4-triazole-3-carboxamide

The preparation was carried out in a similar manner to Example 64, except that in step 1, 3-bromo-4-chlorophenol was used in place of 3-bromo-4-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.61 (s, 1H), 9.90 (s, 1H), 8.45 (d, $J$ = 5.1 Hz, 1H), 8.22 (s, 1H), 7.51 (d, $J$ = 8.8 Hz, 1H), 7.45 - 7.31 (m, 2H), 7.28 (dd, $J$ = 5.2, 1.6 Hz, 1H), 7.26 - 7.15 (m, 2H), 7.12 - 7.01 (m, 2H), 4.21 (s, 2H), 4.14 - 4.03 (m, 2H), 3.69 - 3.58 (m, 2H), 1.15 (s, 9H). MS: 524 $[M+H]^+$.

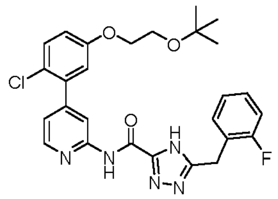

Example 392

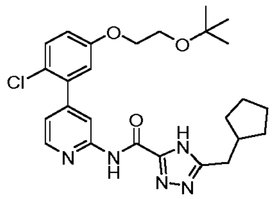

Example 393

Example 393: N-(4-(5-(2-(tert-butoxy)ethoxy)-2-chlorophenyl)pyridin-2-yl)-5-(cyclopentylmethyl)-4H-1,2,4-triazole-3-carboxamide

The preparation was carried out in a similar manner to Example 64, except that in step 1, 3-bromo-4-chlorophenol was used in place of 3-bromo-4-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 9.87 (s, 1H), 8.46 (d, $J$ = 5.2 Hz, 1H), 8.25 (s, 1H), 7.51 (d, $J$ = 8.7 Hz, 1H), 7.34 - 7.24 (m, 1H), 7.16 - 6.96 (m, 2H), 4.16 - 4.03 (m, 2H), 3.70 - 3.56 (m, 2H), 2.79 (d, $J$ = 7.5 Hz, 2H), 2.37 - 2.22 (m, 1H), 1.80 - 1.66 (m, 2H), 1.66 - 1.57 (m, 2H), 1.57 - 1.41 (m, 2H), 1.29 - 1.18 (m, 2H), 1.15 (s, 9H). MS: 498 $[M+H]^+$.

Example 394: N-(4-(5-(2-(tert-butoxy)ethoxy)-2-chlorophenyl)pyridin-2-yl)-5-(cyclohexylmethyl)-4H-1,2,4-triazole-3-carboxamide

The preparation was carried out in a similar manner to Example 64, except that in step 1, 3-bromo-4-chlorophenol was used in place of 3-bromo-4-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.37 (s, 1H), 9.88 (s, 1H), 8.46 (d, $J$ = 5.1 Hz, 1H), 8.25 (s, 1H), 7.52 (d, $J$ = 8.7 Hz, 1H), 7.29 (dd, $J$ = 5.1, 1.6 Hz, 1H), 7.13 - 7.00 (m, 2H), 4.17 - 4.03 (m, 2H), 3.70 - 3.60 (m, 2H), 2.67 (d, $J$ = 7.0 Hz, 2H), 1.84 - 1.57 (m, 6H), 1.28 - 1.13 (m, 12H), 1.03 - 0.91 (m, 2H). MS: 512 $[M+H]^+$.

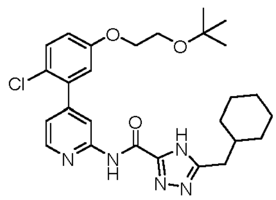

Example 394

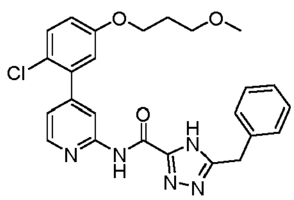

Example 395

Example 395: 5-benzyl-N-(4-(2-chloro-5-(3-methoxypropoxy)phenyl)pyridin-2-yl)-4H-1,2,4-triazole-3-carboxamide

The preparation was carried out in a similar manner to Example 92, except that in step 1, 1-bromo-3-methoxypropane was used in place of iodomethane, and 3-bromo-4-chlorophenol was used in place of 2-bromo-3-methylphenol. $^1$H NMR (400 MHz, DMSO-$d_6$) δ 14.63 (s, 1H), 9.96 (s, 1H), 8.45 (dd, *J* = 5.1, 0.8 Hz, 1H), 8.22 (d, *J* = 1.2 Hz, 1H), 7.51 (d, *J* = 8.8 Hz, 1H), 7.40-7.23 (m, 6H), 7.13-6.95 (m, 2H), 4.18 (s, 2H), 4.07 (t, *J* = 6.4 Hz, 2H), 3.46 (t, *J* = 6.2 Hz, 2H), 3.24 (s, 3H), 2.00-1.89 (m, 2H). MS: 478 [M+H]$^+$.

Example 396: 5-(2-fluorobenzyl)-N-(4-(2-chloro-5-(3-methoxypropoxy)phenyl)pyridin-2-yl)-4H-1,2,4-triazole-3-carboxamide

The preparation was carried out in a similar manner to Example 92, except that in step 1, 1-bromo-3-methoxypropane was used in place of iodomethane, 3-bromo-4-chlorophenol was used in place of 2-bromo-3-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.69 (s, 1H), 9.94 (s, 1H), 8.45 (dd, *J* = 5.1, 0.8 Hz, 1H), 8.28 - 8.13 (m, 1H), 7.51 (d, *J* = 8.8 Hz, 1H), 7.44 - 7.31 (m, 2H), 7.28 (dd, *J* = 5.1, 1.6 Hz, 1H), 7.26 - 7.16 (m, 2H), 7.10 - 6.95 (m, 2H), 4.21 (s, 2H), 4.07 (t, *J* = 6.4 Hz, 2H), 3.46 (t, *J* = 6.2 Hz, 2H), 3.23 (s, 3H), 2.03 - 1.89 (m, 2H). MS: 496 [M+H]$^+$.

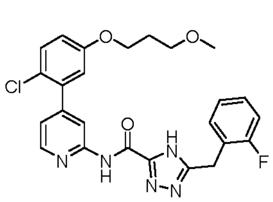

Example 396

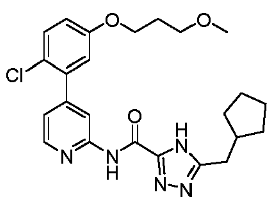

Example 397

Example 397: N-(4-(2-chloro-5-(3-methoxypropoxy)phenyl)pyridin-2-yl)-5-(cyclopentylmethyl)-4H-1,2,4-triazole-3-carboxamide

The preparation was carried out in a similar manner to Example 92, except that in step 1, 1-bromo-3-methoxypropane was used in place of iodomethane, 3-bromo-4-chlorophenol was used in place of 2-bromo-3-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.91 (s, 1H), 8.46 (dd, *J* = 5.1, 0.8 Hz, 1H), 8.24 (dd, *J* = 1.5, 0.8 Hz, 1H), 7.51 (d, *J* = 8.8 Hz, 1H), 7.28 (dd, *J* = 5.1, 1.6 Hz, 1H), 7.13 - 6.99 (m, 2H), 4.08 (t, *J* = 6.4 Hz, 2H), 3.46 (t, *J* = 6.3 Hz, 2H), 3.24 (s, 3H), 2.78 (d, *J* = 7.5 Hz, 2H), 2.36 - 2.18 (m, 1H), 2.00 - 1.89 (m, 2H), 1.79 - 1.67 (m, 2H), 1.67 - 1.44 (m, 4H), 1.30 - 1.15 (m, 2H). MS: 470 [M+H]$^+$.

Example 398: N-(4-(2-chloro-5-(3-methoxypropoxy)phenyl)pyridin-2-yl)-5-(cyclohexylmethyl)-4H-1,2,4-triazole-3-carboxamide

The preparation was carried out in a similar manner to Example 92, except that in step 1, 1-bromo-3-methoxypropane was used in place of iodomethane, 3-bromo-4-chlorophenol was used in place of 2-bromo-3-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.40 (s, 1H), 9.90 (s, 1H), 8.52 - 8.40 (m, 1H), 8.24 (d, *J* = 1.2 Hz, 1H), 7.51 (d, *J* = 8.8 Hz, 1H), 7.28 (dd, *J* = 5.1, 1.6 Hz, 1H), 7.14 - 6.96 (m, 2H), 4.07 (t, *J* = 6.4 Hz, 2H), 3.46 (t, *J* = 6.3 Hz, 2H), 3.24 (s, 3H), 2.67 (d, *J* = 7.1 Hz, 2H), 2.02 - 1.87 (m, 2H), 1.82 - 1.58 (m, 6H), 1.28 - 1.11 (m, 3H), 1.05 - 0.89 (m, 2H). MS: 484 [M+H]$^+$.

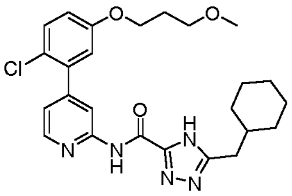 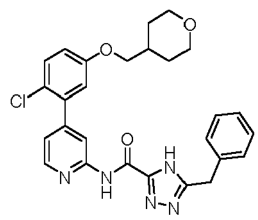

Example 398 Example 399

Example 399: 5-benzyl-N-(4-(2-chloro-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, and 3-bromo-4-chlorophenol was used in place of 3-bromo-4-methylphenol. $^{1}$H NMR (400 MHz, DMSO-$d_6$) δ 14.57 (s, 1H), 9.95 (s, 1H), 8.45 (d, *J* = 5.1 Hz, 1H), 8.22 (d, *J* = 1.5 Hz, 1H), 7.51 (d, *J* = 8.8 Hz, 1H), 7.42 -7.30 (m, 4H), 7.30 - 7.19 (m, 2H), 7.14 - 6.97 (m, 2H), 4.18 (s, 2H), 3.96 - 3.80 (m, 4H), 3.35 - 3.26 (m, 2H), 2.05 - 1.94 (m, 1H), 1.73 - 1.61 (m, 2H), 1.40 - 1.24 (m, 2H). MS: 504 [M+H]$^+$.

Example 400: 5-(2-fluorobenzyl)-N-(4-(2-chloro-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, and in step 3, ethyl 5-(2-fluorobenzyl)-4H-1,2,4-triazol-3-carboxylate was used in place of ethyl 5-benzyl-4H-1,2,4-triazol-3-carboxylate. $^{1}$H NMR (400 MHz, DMSO-$d_6$) δ 14.66 (s, 1H), 9.94 (s, 1H), 8.45 *(d, J*= 5.1 Hz, 1H), 8.21 (d, *J* = 1.5 Hz, 1H), 7.51 *(d, J*= 8.8 Hz, 1H), 7.47 - 7.12 (m, 5H), 7.12 - 6.95 (m, 2H), 4.21 (s, 2H), 3.95 - 3.79 (m, 4H), 3.33 - 3.26 (m, 2H), 2.06 - 1.92 (m, 1H), 1.75 - 1.62 (m, 2H), 1.41 - 1.19 (m, 2H). MS: 522 [M+H]$^+$.

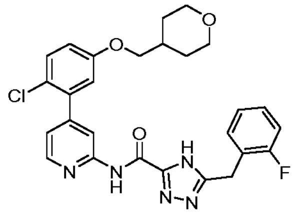 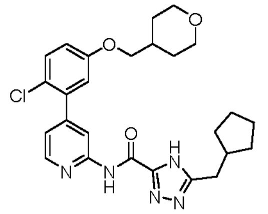

Example 400 Example 401

Example 401: N-(4-(2-chloro-5-((tetrahydro-2H-pyran-4-yl)methoxy)phenyl)pyridin-2-yl)-5-(cyclopentylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, and in step 3, ethyl 5-(cyclopentylmethyl)-4H-1,2,4-triazol-3-carboxylate was used in place of ethyl 5-benzyl-4H-1,2,4-triazol-3-carboxylate. $^{1}$H NMR (400 MHz, DMSO-$d_6$) δ 14.41 (s, 1H), 9.91 (s, 1H), 8.46 (d, *J* = 5.1 Hz, 1H), 8.30 - 8.19 (m, 1H), 7.51 (d, *J* = 8.8 Hz, 1H), 7.28 (dd, *J* = 5.1, 1.6 Hz, 1H), 7.15 - 6.97 (m, 2H), 3.94 - 3.81 (m, 4H), 3.32 - 3.22 (m, 2H), 2.78 (d, *J* = 7.5 Hz, 2H), 2.36 - 2.17 (m, 1H), 2.06 - 1.93 (m, 1H), 1.79 - 1.46 (m, 8H), 1.39 - 1.14 (m, 4H). MS: 496 [M+H]$^+$.

Example 402: N-(4-(2-chloro-5-((tetrahydro-2H-pyran-4-yl)methoxy)phenyl)pyridin-2-yl)-5-(cyclohexylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 9.91 (s, 1H), 8.46 (d, $J$ = 5.1 Hz, 1H), 8.24 (d, $J$ = 1.6 Hz, 1H), 7.61 - 7.44 (m, 1H), 7.28 (dd, $J$ = 5.1, 1.6 Hz, 1H), 7.18 - 6.96 (m, 2H), 4.00 - 3.80 (m, 4H), 3.33 - 3.23 (m, 2H), 2.67 (d, $J$ = 7.0 Hz, 2H), 2.10 - 1.91 (m, 1H), 1.83 - 1.54 (m, 8H), 1.38 - 1.09 (m, 5H), 1.04 - 0.90 (m, 2H). MS: 510 [M+H]$^+$.

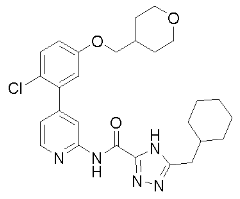
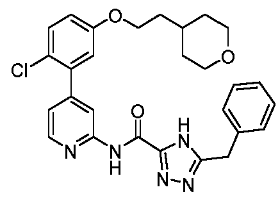

Example 402 Example 403

Example 403: 5-benzyl-N-(4-(2-chloro-5-(2-(tetrahydro-2H-pyran-4-yl)ethoxy)phenyl)pyridin-2-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 2, 3-bromo-4-chlorophenol was used in place of 3-bromo-4-methylphenol. $^1$H NMR (400 MHz, DMSO-$d_6$) δ 14.63 (s, 1H), 9.96 (s, 1H), 8.46 (d, $J$ = 5.1 Hz, 1H), 8.23 (d, $J$ = 1.5 Hz, 1H), 7.51 (d, $J$ = 8.8 Hz, 1H), 7.41 - 7.21 (m, 6H), 7.12 - 7.00 (m, 2H), 4.18 (s, 2H), 4.07 (t, $J$ = 6.2 Hz, 2H), 3.88 - 3.76 (m, 2H), 3.31 - 3.22 (m, 2H), 1.76 - 1.55 (m, 5H), 1.23 - 1.14 (m, 2H). MS: 518 [M+H]$^+$.

Example 404: N-(4-(2-chloro-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 9.95 (s, 1H), 8.45 (d, $J$ = 5.1 Hz, 1H), 8.28 - 8.14 (m, 1H), 7.51 (d, $J$ = 8.7 Hz, 1H), 7.46 - 7.30 (m, 2H), 7.28 (dd, $J$ = 5.1, 1.6 Hz, 1H), 7.26 - 7.12 (m, 2H), 7.12 - 7.00 (m, 2H), 4.20 (s, 2H), 4.07 (t, $J$ = 6.2 Hz, 2H), 3.88 - 3.76 (m, 2H), 3.30 - 3.24 (m, 2H), 1.79 - 1.56 (m, 5H), 1.22 - 1.13 (m, 2H). MS: 536 [M+H]$^+$.

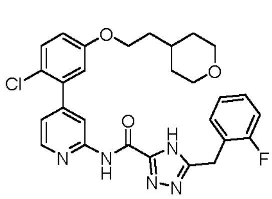
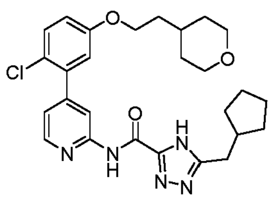

Example 404 Example 405

Example 405: N-(4-(2-chloro-5-(2-(tetrahydro-2H-pyran-4-yl)ethoxy)phenyl)pyridin-2-yl)-5-(cyclopentylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.42 (s, 1H), 9.91 (s, 1H), 8.46 (d, $J$ = 5.1 Hz, 1H), 8.24 (d, $J$ = 1.5 Hz, 1H), 7.51 (d, $J$ = 8.7 Hz, 1H), 7.28 (dd, $J$ = 5.1, 1.6 Hz, 1H), 7.14 - 6.99 (m, 2H), 4.07 (t, $J$ = 6.2 Hz, 2H), 3.88 - 3.75 (m, 2H), 3.31 - 3.21 (m, 2H), 2.78 (d, $J$ = 7.5 Hz, 2H), 2.36 - 2.19 (m, 1H), 1.78 - 1.55 (m, 9H), 1.55 - 1.46 (m, 2H), 1.26 - 1.19 (m, 4H). MS: 510 [M+H]$^+$.

Example 406: N-(4-(2-chloro-5-(2-(tetrahydro-2H-pyran-4-yl)ethoxy)phenyl)pyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.91 (s, 1H), 8.46 (d, *J* = 5.1 Hz, 1H), 8.33 - 8.17 (m, 1H), 7.51 (d, *J* = 8.7 Hz, 1H), 7.28 (dd, *J* = 5.1, 1.6 Hz, 1H), 7.14 - 6.98 (m, 2H), 4.07 (t, *J* = 6.2 Hz, 2H), 3.87 - 3.76 (m, 2H), 3.30 - 3.21 (m, 2H), 2.67 (d, *J* = 7.1 Hz, 2H), 1.83 - 1.51 (m, 11H), 1.27 - 1.12 (m, 5H), 1.05 - 0.89 (m, 2H). MS: 524 [M+H]$^+$.

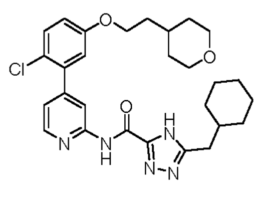

Example 406

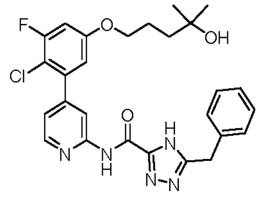

Example 407

Example 407: 5-benzyl-N-(4-(2-chloro-3-fluoro-5-((4-hydroxy-4-methylpentyl)oxy)phenyl)pyridin-2-yl)-4H-1,2,4-triazole-3-carboxamide

The preparation was carried out in a similar manner to Example 160, except that in step 1, 3-bromo-4-chloro-5-fluorophenol was used in place of 3-bromo-4-methylphenol. $^1$H NMR (400 MHz, DMSO-$d_6$) δ 14.65 (s, 1H), 9.91 (s, 1H), 8.45 (d, *J* = 5.2 Hz, 1H), 8.34 (s, 1H), 7.46 - 7.26 (m, 4H), 7.26 - 7.15 (m, 2H), 7.15 - 7.02 (m, 2H), 4.21 (s, 2H), 4.10 (s, 1H), 4.02 (t, *J* = 6.4 Hz, 2H), 1.77 - 1.64 (m, 2H), 1.52 - 1.42 (m, 2H), 1.07 (s, 6H). MS: 524 [M+H]$^+$.

Example 408: N-(4-(2-chloro-3-fluoro-5-((4-hydroxy-4-methylpentyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-chloro-5-fluorophenol was used in place of 3-bromo-4-methylphenol, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.62 (s, 1H), 9.98 (s, 1H), 8.47 (d, *J* = 5.2 Hz, 1H), 8.40 (d, *J* = 1.7 Hz, 1H), 7.58 (dd, *J* = 5.2, 1.7 Hz, 1H), 7.41 (dd, *J* = 9.8, 1.8 Hz, 1H), 7.35 - 7.31 (m, 4H), 7.29 - 7.23 (m, 1H), 4.29 - 4.20 (m, 3H), 4.18 (s, 2H), 1.89 - 1.77 (m, 2H), 1.58 - 1.47 (m, 2H), 1.12 (s, 6H). MS: 542 [M+H]$^+$.

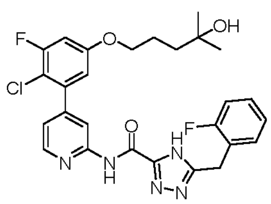

Example 408

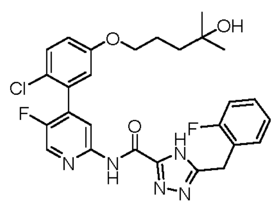

Example 409

Example 409: N-(4-(2-chloro-5-((4-hydroxy-4-methylpentyl)oxy)phenyl)-5-fluoropyridin-2-yl)-5-(2-fluorophenyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-fluoropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.68 (s, 1H), 10.13 (s, 1H), 8.51 (d, *J* = 1.1 Hz, 1H), 8.12 (d, *J* = 5.5 Hz, 1H), 7.53 (d, *J* = 8.7 Hz, 1H), 7.43 - 7.29 (m, 2H), 7.26 - 7.14 (m, 2H), 7.14 - 7.03 (m, 2H), 4.20 (s, 2H), 4.17 (s, 1H), 4.01 (t, *J* = 6.5 Hz, 2H), 1.83 - 1.69 (m, 2H), 1.52 - 1.42 (m, 2H), 1.09 (s, 6H). MS: 542 [M+H]$^+$.

Example 410: N-(4-(2-chloro-5-((4-hydroxy-4-methylpentyl)oxy)phenyl)-5-fluoropyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-fluoropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.37 (s, 1H), 10.06 (s, 1H), 8.52 (d, $J$ = 1.2 Hz, 1H), 8.15 (d, $J$ = 5.5 Hz, 1H), 7.54 (d, $J$ = 8.7 Hz, 1H), 7.15 - 7.04 (m, 2H), 4.17 (s, 1H), 4.01 (t, $J$ = 6.6 Hz, 2H), 2.67 (d, $J$ = 7.0 Hz, 2H), 1.82 - 1.56 (m, 8H), 1.52 - 1.41 (m, 2H), 1.28 - 1.12 (m, 3H), 1.09 (s, 6H), 1.05 - 0.90 (m, 2H). MS: 530 $[M+H]^+$.

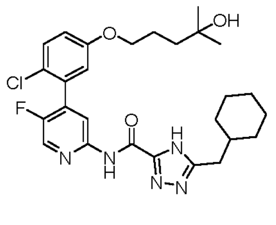
Example 410

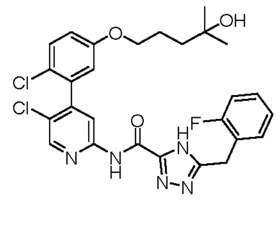
Example 411

Example 411: N-(5-chloro-4-(2-chloro-5-((4-hydroxy-4-methylpentyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-chloropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.63 (s, 1H), 10.14 (s, 1H), 8.57 (s, 1H), 8.11 (s, 1H), 7.51 (d, $J$ = 8.9 Hz, 1H), 7.44 - 7.29 (m, 2H), 7.26 - 7.15 (m, 2H), 7.08 (dd, $J$ = 8.9, 3.0 Hz, 1H), 7.00 (d, $J$ = 3.0 Hz, 1H), 4.25 - 4.14 (m, 3H), 3.99 (t, $J$ = 6.6 Hz, 2H), 1.81 - 1.69 (m, 2H), 1.52 - 1.40 (m, 2H), 1.09 (s, 6H). MS: 558 $[M+H]^+$.

Example 412: N-(5-chloro-4-(2-chloro-5-((4-hydroxy-4-methylpentyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-chloropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.09 (s, 1H), 8.58 (s, 1H), 8.13 (s, 1H), 7.52 (d, $J$ = 8.9 Hz, 1H), 7.09 (dd, $J$ = 8.9, 3.0 Hz, 1H), 7.00 (d, $J$ = 3.0 Hz, 1H), 4.18 (s, 1H), 4.00 (t, $J$ = 6.6 Hz, 2H), 2.67 (d, $J$ = 6.9 Hz, 2H), 1.82 - 1.55 (m, 8H), 1.52 - 1.42 (m, 2H), 1.28 - 1.12 (m, 3H), 1.09 (s, 6H), 1.05 - 0.87 (m, 2H). MS: 546 $[M+H]^+$.

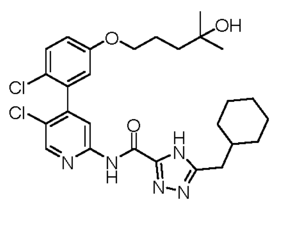
Example 412

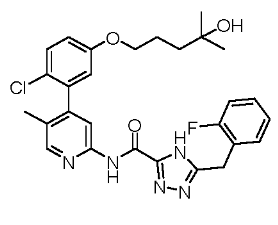
Example 413

Example 413: N-(4-(2-chloro-5-((4-hydroxy-4-methylpentyl)oxy)phenyl)-5-methylpyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-methylpyridin-2-amine was used in place

of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 9.80 (s, 1H), 8.32 (s, 1H), 7.93 (s, 1H), 7.50 (d, *J* = 8.9 Hz, 1H), 7.45 - 7.28 (m, 2H), 7.28 - 7.14 (m, 2H), 7.05 (dd, *J* = 8.9, 3.0 Hz, 1H), 6.90 (d, *J* = 3.0 Hz, 1H), 4.26 - 4.12 (m, 3H), 3.99 (t, *J* = 6.6 Hz, 2H), 2.05 (s, 3H), 1.82 - 1.66 (m, 2H), 1.51 - 1.41 (m, 2H), 1.08 (s, 6H). MS: 538 $[M+H]^+$.

Example 414: N-(4-(2-chloro-5-((4-hydroxy-4-methylpentyl)oxy)phenyl)-5-methylpyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 4-bromo-5-methylpyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.34 (s, 1H), 9.79 (s, 1H), 8.33 (s, 1H), 7.96 (s, 1H), 7.50 (d, *J* = 8.9 Hz, 1H), 7.05 (dd, *J* = 8.9, 3.0 Hz, 1H), 6.91 (d, *J* = 3.0 Hz, 1H), 4.18 (s, 1H), 3.99 (t, *J* = 6.6 Hz, 2H), 2.66 (d, *J* = 7.1 Hz, 2H), 2.05 (s, 3H), 1.82 - 1.56 (m, 8H), 1.52 - 1.41 (m, 2H), 1.30 - 1.12 (m, 3H), 1.09 (s, 6H), 1.04 - 0.90 (m, 2H). MS: 526 $[M+H]^+$.

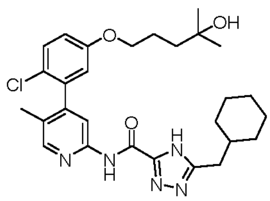

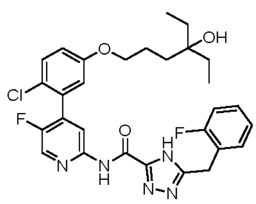

Example 414 Example 415

Example 415: N-(4-(2-chloro-5-((4-ethyl-4-hydroxyhexyl)oxy)phenyl)-5-fluoropyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, in step 3, 4-bromo-5-fluoropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 10.13 (s, 1H), 8.51 (d, *J* = 1.1 Hz, 1H), 8.12 (d, *J* = 5.5 Hz, 1H), 7.53 (d, *J* = 8.7 Hz, 1H), 7.45 - 7.29 (m, 2H), 7.26 - 7.15 (m, 2H), 7.14 - 7.04 (m, 2H), 4.20 (s, 2H), 4.00 (t, *J* = 6.5 Hz, 2H), 3.87 (s, 1H), 1.76 - 1.61 (m, 2H), 1.47 - 1.38 (m, 2H), 1.34 (q, *J* = 7.5 Hz, 4H), 0.77 (t, *J* = 7.4 Hz, 6H). MS: 570 $[M+H]^+$.

Example 416: N-(4-(2-chloro-5-((4-ethyl-4-hydroxyhexyl)oxy)phenyl)-5-fluoropyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, in step 3, 4-bromo-5-fluoropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 10.07 (s, 1H), 8.52 (d, *J* = 1.1 Hz, 1H), 8.15 (d, *J* = 5.5 Hz, 1H), 7.53 (d, *J* = 8.7 Hz, 1H), 7.13 - 7.04 (m, 2H), 4.01 (t, *J* = 6.5 Hz, 2H), 3.88 (s, 1H), 2.66 (d, *J* = 7.1 Hz, 2H), 1.82 - 1.55 (m, 8H), 1.46 - 1.39 (m, 2H), 1.34 (q, *J* = 7.5 Hz, 4H), 1.28 - 1.09 (m, 3H), 1.05 - 0.91 (m, 2H), 0.77 (t, *J* = 7.5 Hz, 6H). MS: 558 $[M+H]^+$.

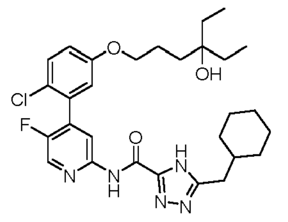

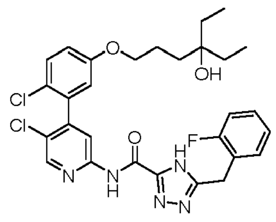

Example 416 Example 417

Example 417: N-(5-chloro-4-(2-chloro-5-((4-ethyl-4-hydroxyhexyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, in step 3, 4-bromo-5-chloropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^{1}$H NMR (400 MHz, DMSO-$d_6$) δ 14.62 (s, 1H), 10.13 (s, 1H), 8.57 (s, 1H), 8.11 (s, 1H), 7.51 (d, $J$ = 8.8 Hz, 1H), 7.46 - 7.29 (m, 2H), 7.26 - 7.15 (m, 2H), 7.08 (dd, $J$ = 8.9, 3.0 Hz, 1H), 6.99 (d, $J$ = 3.0 Hz, 1H), 4.21 (s, 2H), 3.99 (t, $J$ = 6.5 Hz, 2H), 3.88 (s, 1H), 1.74 - 1.61 (m, 2H), 1.45 - 1.37 (m, 2H), 1.34 (q, $J$ = 7.5 Hz, 4H), 0.77 (t, $J$ = 7.5 Hz, 6H). MS: 586 [M+H]$^+$.

Example 418: N-(5-chloro-4-(2-chloro-5-((4-ethyl-4-hydroxyhexyl)oxy)phenyl)pyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, in step 2, ethylmagnesium bromide was used in place of methylmagnesium bromide, in step 3, 4-bromo-5-chloropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^{1}$H NMR (400 MHz, DMSO-$d_6$) δ 14.38 (s, 1H), 10.09 (s, 1H), 8.58 (s, 1H), 8.13 (s, 1H), 7.52 (d, $J$ = 8.9 Hz, 1H), 7.09 (dd, $J$ = 8.9, 3.0 Hz, 1H), 7.00 (d, $J$ = 3.0 Hz, 1H), 4.00 (t, $J$ = 6.5 Hz, 2H), 3.88 (s, 1H), 2.67 (d, $J$ = 6.8 Hz, 2H), 1.82 - 1.56 (m, 8H), 1.48 - 1.38 (m, 2H), 1.34 (q, $J$ = 7.4 Hz, 4H), 1.27 - 1.09 (m, 3H), 1.05 - 0.89 (m, 2H), 0.77 (t, $J$ = 7.4 Hz, 6H). MS: 574 [M+H]$^+$.

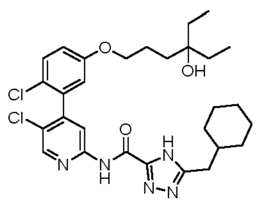

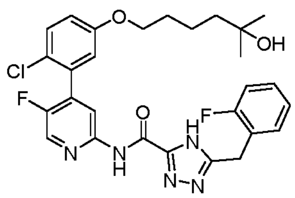

Example 418 Example 419

Example 419: N-(4-(2-chloro-5-((5-hydroxyl-5-methylhexyl)oxy)phenyl)-5-fluoropyridin-2-yl)-5-(2-fluorobenzyl)-4H-1,2,4-triazole-3-carboxamide

The preparation was carried out in a similar manner to Example 160, except that in step 1, methyl 5-bromopentanoate was used in place of methyl 4-bromobutanoate, 3-bromo-4-chlorophenol was used in place of 3-bromo-4-methylphenol, in step 3, 5-fluoro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^{1}$H NMR (400 MHz, DMSO-$d_6$) δ 14.58 (s, 1H), 10.05 (s, 1H), 8.52 (d, $J$ = 15.7 Hz, 1H), 8.14 (d, $J$ = 5.5 Hz, 1H), 7.53 (d, $J$ = 8.6 Hz, 1H), 7.44 - 7.07 (m, 6H), 4.23 (s, 2H), 4.08 (s, 1H), 4.01 (t, $J$ = 6.4 Hz, 2H), 1.76 - 1.63 (m, 2H), 1.50 - 1.33 (m, 4H), 1.06 (s, 6H). MS: 556 [M+H]$^+$.

Example 420: N-(5-chloro-4-(2-chloro-5-((5-hydroxyl-5-methylhexyl)oxy)phenyl)pyridin-2-yl)-5-(2-fluorobenzyl)-4H-1,2,4-triazole-3-carboxamide

The preparation was carried out in a similar manner to Example 160, except that in step 1, methyl 5-bromopentanoate was used in place of methyl 4-bromobutanoate, 3-bromo-4-chlorophenol was used in place of 3-bromo-4-methylphenol, in step 3, 5-chloro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 10.11 (s, 1H), 8.56 (s, 1H), 8.12 (s, 1H), 7.51 (d, *J* = 8.9 Hz, 1H), 7.46 - 7.27 (m, 2H), 7.27 - 7.13 (m, 2H), 7.09 (dd, *J* = 8.9, 3.0 Hz, 1H), 7.01 (d, *J* = 3.0 Hz, 1H), 4.23 (s, 2H), 4.11 (s, 1H), 4.00 (t, *J* = 6.7 Hz, 2H), 1.74 - 1.62 (m, 2H), 1.48 - 1.29 (m, 4H), 1.06 (s, 6H). MS: 572 $[M+H]^+$.

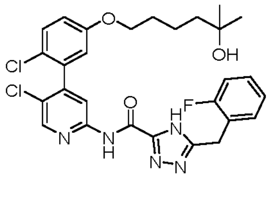

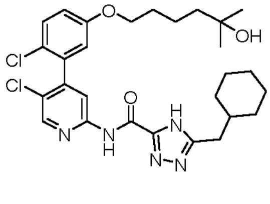

Example 420 Example 421

Example 421: N-(5-chloro-4-(2-chloro-5-((5-hydroxyl-5-methylhexyl)oxy)phenyl)pyridin-2-yl)-5-(cyclohexylmethyl)-4H-1,2,4-triazole-3-carboxamide

The preparation was carried out in a similar manner to Example 160, except that in step 1, methyl 5-bromopentanoate was used in place of methyl 4-bromobutanoate, 3-bromo-4-chlorophenol was used in place of 3-bromo-4-methylphenol, in step 3, 5-chloro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 10.12 (s, 1H), 8.58 (s, 1H), 8.13 (s, 1H), 7.52 (d, *J* = 8.9 Hz, 1H), 7.10 (dd, *J* = 8.9, 3.0 Hz, 1H), 7.02 (d, *J* = 3.0 Hz, 1H), 4.10 (s, 1H), 4.01 (t, *J* = 6.7 Hz, 2H), 2.66 (d, *J* = 7.0 Hz, 2H), 1.83 - 1.40 (m, 11H), 1.30 - 1.08 (m, 4H), 1.06 (s, 6H), 1.03 - 0.88 (m, 2H). MS: 560 $[M+H]^+$.

Example 422: N-(4-(2-chloro-5-((tetrahydro-2H-pyran-4-yl)methoxy)phenyl)-5-fluoropyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 5-fluoro-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 10.13 (s, 1H), 8.51 (d, *J* = 1.2 Hz, 1H), 8.12 (d, *J* = 5.5 Hz, 1H), 7.53 (d, *J* = 8.5 Hz, 1H), 7.43-7.29 (m, 2H), 7.25-7.16 (m, 2H), 7.14-7.09 (m, 2H), 4.21 (s, 2H), 3.92-3.80 (m, 4H), 3.38 - 3.32 (m, 2H), 2.05 - 1.92 (m, 1H), 1.72 - 1.58 (m, 2H), 1.41 - 1.23 (m, 2H). MS: 540 $[M+H]^+$.

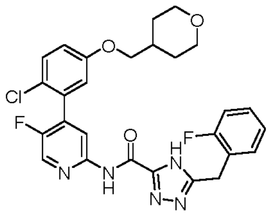

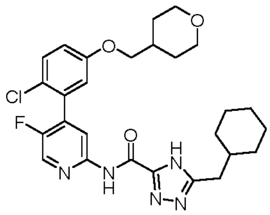

Example 422 Example 423

Example 423: N-(4-(2-chloro-5-((tetrahydro-2H-pyran-4-yl)methoxy)phenyl)-5-fluoropyridin-2-yl)-5-(cyclohexylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 5-fluoro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 10.07 (s, 1H), 8.52 (s, 1H), 8.15 (d, $J$ = 5.5 Hz, 1H), 7.54 (d, $J$ = 8.5 Hz, 1H), 7.17 - 7.07 (m, 2H), 3.94 - 3.82 (m, 4H), 3.37 - 3.30 (m, 2H), 2.66 (d, $J$ = 7.1 Hz, 2H), 2.06 - 1.92 (m, 1H), 1.82 - 1.56 (m, 8H), 1.39 - 1.25 (m, 2H), 1.25 - 1.10 (m, 3H), 1.05 - 0.90 (m, 2H). MS: 528 $[M+H]^+$.

Example 424: N-(5-chloro-4-(2-chloro-5-((tetrahydro-2H-pyran-4-yl)methoxy)phenyl)pyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 5-chloro-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.63 (s, 1H), 10.14 (s, 1H), 8.57 (s, 1H), 8.11 (s, 1H), 7.52 (d, $J$ = 8.9 Hz, 1H), 7.45-7.29 (m, 2H), 7.26-7.14 (m, 2H), 7.10 (dd, $J$ = 8.9, 3.0 Hz, 1H), 7.02 (d, $J$ = 3.0 Hz, 1H), 4.21 (s, 2H), 3.96-3.75 (m, 4H), 3.34-3.23 (m, 2H), 2.06-1.90 (m, 1H), 1.73-1.60 (m, 2H), 1.40-1.24 (m, 2H). MS: 556 $[M+H]^+$.

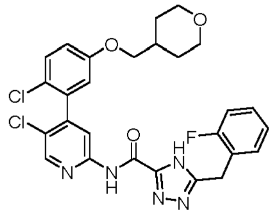

Example 424

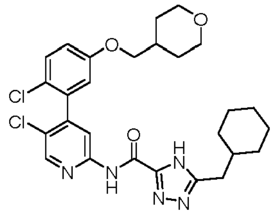

Example 425

Example 425: N-(5-chloro-4-(2-chloro-5-((tetrahydro-2H-pyran-4-yl)methoxy)phenyl)pyridin-2-yl)-5-(cyclohexylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 5-chloro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.10 (s, 1H), 8.58 (s, 1H), 8.13 (s, 1H), 7.52 (d, $J$ = 8.8 Hz, 1H), 7.11 (dd, $J$ = 8.9, 3.0 Hz, 1H), 7.03 (d, $J$ = 3.0 Hz, 1H), 3.95 - 3.81 (m, 4H), 3.34 - 3.26 (m, 2H), 2.66 (d, $J$ = 7.0 Hz, 2H), 2.07 - 1.92 (m, 1H), 1.83 - 1.55 (m, 8H), 1.39 - 1.07 (m, 5H), 1.07 - 0.89 (m, 2H). MS: 544 $[M+H]^+$.

Example 426: N-(4-(2-chloro-5-((tetrahydro-2H-pyran-4-yl)methoxy)phenyl)-5-methylpyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 4-bromo-5-methylpyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 9.81 (s, 1H), 8.32 (s, 1H), 7.93 (s, 1H), 7.50 (d, $J$ = 8.8 Hz, 1H), 7.46 - 7.29 (m, 2H), 7.26 - 7.15 (m, 2H), 7.06 (dd, $J$ = 8.9, 3.0 Hz, 1H), 6.92 (d, $J$ = 3.0 Hz, 1H), 4.20 (s, 2H), 3.95 - 3.78 (m, 4H), 3.38 - 3.30 (m, 2H), 2.05 (s, 3H), 2.02 - 1.92 (m, 1H), 1.66 (dd, $J$ = 12.8, 3.1 Hz, 2H), 1.39 - 1.24 (m, 2H). MS: 536 $[M+H]^+$.

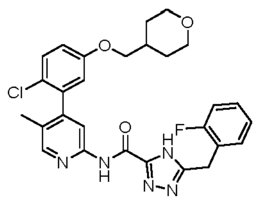
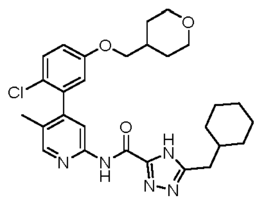

Example 426 Example 427

Example 427: N-(4-(2-chloro-5-((tetrahydro-2H-pyran-4-yl)methoxy)phenyl)-5-methylpyridin-2-yl)-5-(cyclohexylmethyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 4-bromo-5-methylpyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 9.79 (s, 1H), 8.33 (s, 1H), 7.96 (s, 1H), 7.51 (d, *J* = 8.9 Hz, 1H), 7.07 (dd, *J* = 8.9, 3.0 Hz, 1H), 6.93 (d, *J* = 3.0 Hz, 1H), 3.92 - 3.81 (m, 4H), 3.38 - 3.33 (m, 2H), 2.70 - 2.62 (m, 2H), 2.05 (s, 3H), 2.04 - 1.92 (m, 1H), 1.82 - 1.58 (m, 8H), 1.38 - 1.12 (m, 5H), 1.05 - 0.89 (m, 2H). MS: 524 $[M+H]^+$.

Example 428: N-(4-(2-chloro-5-(2-(tetrahydro-2H-pyran-4-yl)ethoxy)phenyl)-5-fluoropyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, in step 3, 4-bromo-5-fluoropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.65 (s, 1H), 10.14 (s, 1H), 8.51 (d, *J* = 1.1 Hz, 1H), 8.12 (d, *J* = 5.5 Hz, 1H), 7.56 - 7.49 (m, 1H), 7.45 - 7.29 (m, 2H), 7.25 - 7.15 (m, 2H), 7.15 - 7.06 (m, 2H), 4.20 (s, 2H), 4.06 (t, *J* = 6.2 Hz, 2H), 3.87 - 3.76 (m, 2H), 3.35 - 3.28 (m, 2H), 1.77 - 1.55 (m, 5H), 1.29 - 1.12 (m, 2H). MS: 554 $[M+H]^+$.

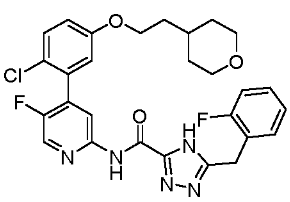
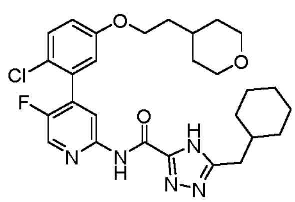

Example 428 Example 429

Example 429: N-(4-(2-chloro-5-(2-(tetrahydro-2H-pyran-4-yl)ethoxy)phenyl)-5-fluoropyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, 3-bromo-4-chlorophenol was used in place of 3-bromo-4-methylphenol, in step 3, 4-bromo-5-fluoropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.07 (s, 1H), 8.52 (d, *J* = 1.2 Hz, 1H), 8.15 (d, *J* = 5.5 Hz, 1H), 7.57 - 7.48 (m, 1H), 7.17 - 7.08 (m, 2H), 4.07 (t, *J* = 6.2 Hz, 2H), 3.88 - 3.75 (m, 2H), 3.31 - 3.21 (m, 2H), 2.66 (d, *J* = 7.0 Hz, 2H), 1.83 - 1.55 (m, 12H), 1.30 - 1.12 (m, 4H), 1.05 - 0.90 (m, 2H). MS: 542 $[M+H]^+$.

Example 430: N-(5-chloro-4-(2-chloro-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, 3-bromo-4-chlorophenol was used in place

of 3-bromo-4-methylphenol, in step 3, 4-bromo-5-chloropyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.64 (s, 1H), 10.15 (s, 1H), 8.57 (s, 1H), 8.10 (s, 1H), 7.51 (d, $J$ = 8.9 Hz, 1H), 7.45 - 7.29 (m, 2H), 7.26 - 7.14 (m, 2H), 7.10 (dd, $J$ = 8.9, 3.0 Hz, 1H), 7.03 (d, $J$ = 3.0 Hz, 1H), 4.21 (s, 2H), 4.05 (t, $J$ = 6.3 Hz, 2H), 3.86 - 3.74 (m, 2H), 3.31 - 3.19 (m, 2H), 1.76 - 1.54 (m, 5H), 1.27 - 1.13 (m, 2H). MS: 570 [M+H]$^+$.

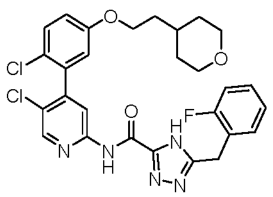

Example 430

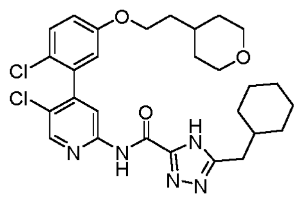

Example 431

Example 431: N-(5-chloro-4-(2-chloro-5-(2-(tetrahydro-2H-pyran-4-yl)ethoxy)phenyl)pyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, 3-bromo-4-chlorophenol was used in place of 3-bromo-4-methylphenol, in step 3, 4-bromo-5-chloropyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.38 (s, 1H), 10.09 (s, 1H), 8.58 (s, 1H), 8.13 (s, 1H), 7.52 (d, $J$ = 8.9 Hz, 1H), 7.10 (dd, $J$ = 8.9, 3.0 Hz, 1H), 7.03 (d, $J$ = 3.0 Hz, 1H), 4.05 (t, $J$ = 6.4 Hz, 2H), 3.86 - 3.75 (m, 2H), 3.31 - 3.20 (m, 2H), 2.67 (d, $J$ = 6.8 Hz, 2H), 1.82 - 1.54 (m, 11H), 1.28 - 1.13 (m, 5H), 1.05 - 0.89 (m, 2H). MS: 558 [M+H]$^+$.

Example 432: 5-benzyl-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine. $^1$H NMR (400 MHz, DMSO-$d_6$) δ 14.68 (s, 1H), 10.35 (s, 1H), 9.01 (d, $J$ = 1.3 Hz, 1H), 8.24 (d, $J$ = 1.3 Hz, 1H), 7.48 - 7.16 (m, 6H), 7.09 - 6.90 (m, 2H), 4.26 - 4.12 (m, 3H), 3.98 (t, $J$ = 6.6 Hz, 2H), 2.31 (s, 3H), 1.86 - 1.66 (m, 2H), 1.56 - 1.40 (m, 2H), 1.09 (s, 6H). MS: 487 [M+H]$^+$.

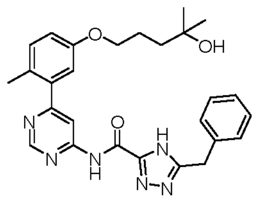

Example 432

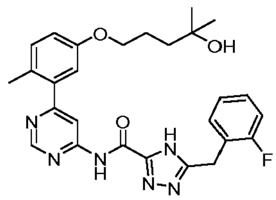

Example 433

Example 433: 5-(2-fluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.73 (s, 1H), 10.33 (s, 1H), 9.01 (d, $J$ = 1.2 Hz, 1H), 8.23 (d, $J$ = 1.3 Hz, 1H), 7.51 - 7.29 (m, 2H), 7.29 - 7.13 (m, 3H), 7.08 - 6.91 (m, 2H), 4.22 (s, 2H), 4.18 (s, 1H), 3.97 (t, $J$ = 6.6 Hz, 2H), 2.31 (s, 3H), 1.83 - 1.68 (m, 2H), 1.54 - 1.42 (m, 2H), 1.09 (s, 6H). MS: 505 [M+H]$^+$.

Example 434: 5-(3-fluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(3-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.70 (s, 1H), 10.39 (s, 1H), 9.02 (d, *J* = 1.2 Hz, 1H), 8.24 (d, *J* = 1.2 Hz, 1H), 7.46 - 7.31 (m, 1H), 7.26 (d, *J* = 8.4 Hz, 1H), 7.23 - 7.14 (m, 2H), 7.14 - 7.06 (m, 1H), 7.03 (d, *J* = 2.7 Hz, 1H), 6.98 (dd, *J* = 8.3, 2.8 Hz, 1H), 4.22 (s, 2H), 4.18 (s, 1H), 3.98 (t, *J* = 6.6 Hz, 2H), 2.31 (s, 3H), 1.83 - 1.69 (m, 2H), 1.54 - 1.43 (m, 2H), 1.09 (s, 6H). MS: 505 [M+H]$^+$.

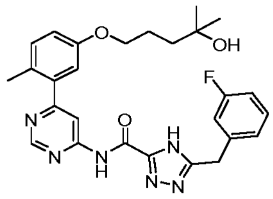

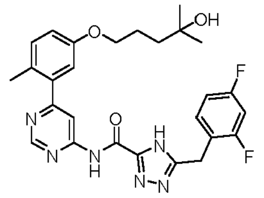

Example 434 Example 435

Example 435: 5-(2,4-difluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(2,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.72 (s, 1H), 10.35 (s, 1H), 9.01 (d, *J* = 1.2 Hz, 1H), 8.23 (d, *J* = 1.2 Hz, 1H), 7.56-7.40 (m, 1H), 7.27 (dd, *J* = 10.8, 7.8 Hz, 2H), 7.16-7.06 (m, 1H), 7.06-6.91 (m, 2H), 4.25 - 4.13 (m, 3H), 3.97 (t, *J* = 6.5 Hz, 2H), 2.31 (s, 3H), 1.82 - 1.67 (m, 2H), 1.53 - 1.42 (m, 2H), 1.09 (s, 6H). MS: 523 [M+H]$^+$.

Example 436: 5-(2,5-difluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(2,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.51 (s, 1H), 10.33 (s, 1H), 9.00 (d, *J* = 1.2 Hz, 1H), 8.24 (d, *J* = 1.3 Hz, 1H), 7.39 - 7.11 (m, 4H), 7.08 - 6.90 (m, 2H), 4.24 - 4.12 (m, 3H), 3.98 (t, *J* = 6.5 Hz, 2H), 2.31 (s, 3H), 1.82 - 1.69 (m, 2H), 1.53 - 1.42 (m, 2H), 1.10 (s, 6H). MS: 523 [M+H]$^+$.

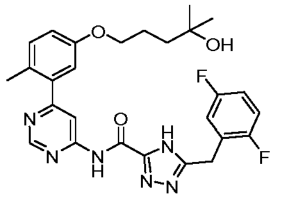

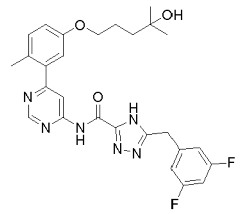

Example 436 Example 437

Example 437: 5-(3,5-difluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(3,5-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.73 (s, 1H), 10.44 (s, 1H), 9.02 (d, *J* = 1.2 Hz, 1H), 8.24 (d, *J* = 1.2 Hz, 1H), 7.26 (d, *J* = 8.4 Hz, 1H), 7.22 - 7.06 (m, 3H), 7.03 (d, *J* = 2.7 Hz, 1H), 6.98 (dd, *J* = 8.4, 2.8 Hz, 1H), 4.24 (s, 2H), 4.18 (s, 1H), 3.98 (t, *J* = 6.5 Hz, 2H), 2.31 (s, 3H), 1.82 - 1.68 (m, 2H), 1.53 - 1.43 (m, 2H), 1.09 (s, 6H). MS: 523 [M+H]$^+$.

Example 438: 5-(3-chloro-2-fluorobenzyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(3-chloro-2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.71 (s, 1H), 10.31 (s, 1H), 9.01 (d, *J* = 1.2 Hz, 1H), 8.23 (d, *J* = 1.3 Hz, 1H), 7.60 - 7.45 (m, 1H), 7.45 - 7.33 (m, 1H), 7.33 - 7.15 (m, 2H), 7.08 - 6.91 (m, 2H), 4.27 (s, 2H), 4.18 (s, 1H), 3.97 (t, *J* = 6.5 Hz, 2H), 2.31 (s, 3H), 1.83 - 1.69 (m, 2H), 1.54 - 1.40 (m, 2H), 1.09 (s, 6H). MS: 539 [M+H]$^+$.

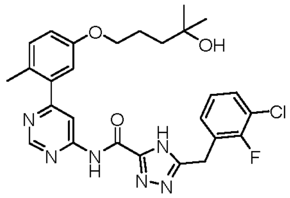

Example 438

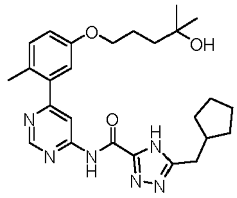

Example 439

Example 439: 5-(cyclopentylmethyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.47 (s, 1H), 10.27 (s, 1H), 9.02 (d, *J* = 1.2 Hz, 1H), 8.26 (d, *J* = 1.3 Hz, 1H), 7.26 (d, *J* = 8.4 Hz, 1H), 7.09 - 6.90 (m, 2H), 4.18 (s, 1H), 3.98 (t, *J* = 6.5 Hz, 2H), 2.79 (d, *J* = 7.5 Hz, 2H), 2.32 (s, 3H), 2.30 - 2.18 (m, 1H), 1.82 - 1.67 (m, 4H), 1.67 - 1.56 (m, 2H), 1.56 - 1.43 (m, 4H), 1.25 - 1.21 (m, 2H), 1.10 (s, 6H). MS: 479 [M+H]$^+$.

Example 440: 5-(cyclohexylmethyl)-N-(6-(5-((4-hydroxy-4-methylpentyl)oxy)-2-methylphenyl)pyrimidin-4-yl)-4H-1,2,4-triazole-3-carboxamide

The preparation was carried out in a similar manner to Example 160, except that in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 10.28 (s, 1H), 9.02 (s, 1H), 8.26 (s, 1H), 7.26 (d, *J* = 8.4 Hz, 1H), 7.03 (d, *J* = 2.7 Hz, 1H), 6.99 (dd, *J* = 8.3, 2.8 Hz, 1H), 4.18 (s, 1H), 3.98 (t, *J* = 6.6 Hz, 2H), 2.67 (d, *J* = 7.0 Hz, 2H), 2.32 (s, 3H), 1.83 - 1.55 (m, 8H), 1.52 - 1.42 (m, 2H), 1.25 - 1.14 (m, 3H), 1.10 (s, 6H), 1.04 - 0.89 (m, 2H). MS: 493 [M+H]$^+$.

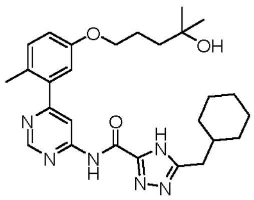

Example 440

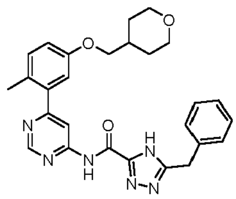

Example 441

Example 441: 5-benzyl-N-(6-(2-methyl-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, and in step 2, 6-chloropyrimidin-4-amine was used in place of 4-bromopyridin-2-amine. $^1$H NMR (400 MHz, DMSO-$d_6$) δ 14.68 (s, 1H), 10.34 (s, 1H), 9.02 (d, *J* = 1.2 Hz, 1H), 8.24 (d, *J* = 1.3 Hz, 1H), 7.39 - 7.29 (m, 4H), 7.29 - 7.22 (m, 2H), 7.04 (d, *J* = 2.7 Hz, 1H), 7.00 (dd, *J* = 8.3, 2.8 Hz, 1H), 4.19 (s, 2H), 3.92 - 3.80 (m, 4H), 3.33 - 3.28 (m, 2H), 2.31 (s, 3H), 2.05 - 1.93 (m, 1H), 1.76 - 1.62 (m, 2H), 1.39 - 1.26 (m,

2H). MS: 485 [M+H]+.

Example 442: 5-(2-fluorobenzyl)-N-(6-(2-methyl-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 6-chloropyrimidin-4-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.75 (s, 1H), 10.34 (s, 1H), 9.01 (d, $J$ = 1.3 Hz, 1H), 8.23 (d, $J$ = 1.3 Hz, 1H), 7.46 - 7.30 (m, 2H), 7.30 - 7.16 (m, 3H), 7.04 (d, $J$ = 2.8 Hz, 1H), 6.99 (dd, $J$ = 8.4, 2.8 Hz, 1H), 4.22 (s, 2H), 3.92 - 3.79 (m, 4H), 3.32 - 3.25 (m, 2H), 2.31 (s, 3H), 2.05 - 1.92 (m, 1H), 1.73 - 1.62 (m, 2H), 1.40 - 1.26 (m, 2H). MS: 503 [M+H]+.

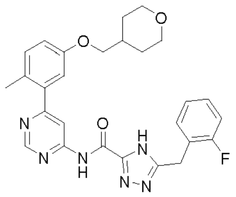

Example 442

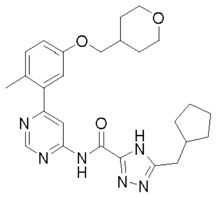

Example 443

Example 443: 5-(cyclopentylmethyl)-N-(6-(2-methyl-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 6-chloropyrimidin-4-amine was used in place of 4-bromopyridin-2-amine, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.48 (s, 1H), 10.27 (s, 1H), 9.03 (d, $J$ = 1.3 Hz, 1H), 8.26 (d, $J$ = 1.3 Hz, 1H), 7.27 (d, $J$ = 8.4 Hz, 1H), 7.05 (d, $J$ = 2.8 Hz, 1H), 7.00 (dd, $J$ = 8.3, 2.8 Hz, 1H), 3.93-3.81 (m, 4H), 3.33-3.29 (m, 2H), 2.79 (d, $J$ = 7.5 Hz, 2H), 2.32 (s, 3H), 2.31-2.21 (m, 1H), 2.08-1.93 (m, 1H), 1.79-1.45 (m, 8H), 1.38-1.27 (m, 2H), 1.27-1.17 (m, 2H). MS: 477 [M+H]+.

Example 444: 5-(cyclohexylmethyl)-N-(6-(2-methyl-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 6-chloropyrimidin-4-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.47 (s, 1H), 10.26 (s, 1H), 9.02 (d, $J$ = 1.3 Hz, 1H), 8.25 (d, $J$ = 1.3 Hz, 1H), 7.26 (d, $J$ = 8.4 Hz, 1H), 7.05 (d, $J$ = 2.7 Hz, 1H), 7.00 (dd, $J$ = 8.4, 2.8 Hz, 1H), 3.92-3.81 (m, 4H), 3.37-3.32 (m, 2H), 2.67 (d, $J$ = 7.1 Hz, 2H), 2.31 (s, 3H), 2.06-1.93 (m, 1H), 1.82-1.53 (m, 8H), 1.42-1.25 (m, 2H), 1.25-1.06 (m, 3H), 1.06-0.91 (m, 2H). MS: 491 [M+H]+.

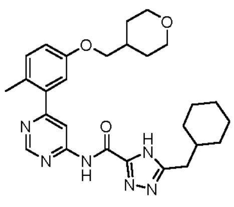

Example 444

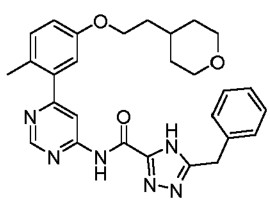

Example 445

Example 445: 5-benzyl-N-(6-(2-methyl-5-(2-(tetrahydro-2H-pyran-4-yl)ethoxy)phenyl)pyrimidin-4-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, and in step 2, 6-chloropyrimidin-4-amine was used in place of 4-bromopyridin-2-amine. $^1$H NMR (400 MHz, DMSO-$d_6$) δ 14.68 (s, 1H), 10.35 (s, 1H), 9.02 (d, *J* = 1.2 Hz, 1H), 8.24 (d, *J* = 1.3 Hz, 1H), 7.45 - 7.17 (m, 6H), 7.17 - 6.86 (m, 2H), 4.19 (s, 2H), 4.04 (t, *J* = 6.3 Hz, 2H), 3.93 - 3.66 (m, 2H), 3.30 - 3.20 (m, 2H), 2.31 (s, 3H), 1.82 - 1.56 (m, 5H), 1.28 - 1.17 (m, 2H). MS: 499 $[M+H]^+$.

Example 446: 5-(2-fluorobenzyl)-N-(6-(2-methyl-5-(2-(tetrahydro-2H-pyran-4-yl)ethoxy)phenyl)pyrimidin-4-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.71 (s, 1H), 10.34 (s, 1H), 9.01 (d, *J* = 1.2 Hz, 1H), 8.23 (d, *J* = 1.3 Hz, 1H), 7.47 - 7.29 (m, 2H), 7.29 - 7.15 (m, 3H), 7.08 - 6.93 (m, 2H), 4.22 (s, 2H), 4.03 (t, *J* = 6.3 Hz, 2H), 3.91 - 3.74 (m, 2H), 3.30 - 3.18 (m, 2H), 2.30 (s, 3H), 1.83 - 1.53 (m, 5H), 1.24 - 1.13 (m, 2H). MS: 517 $[M+H]^+$.

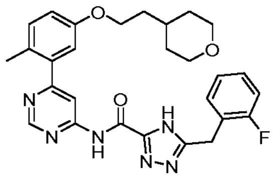
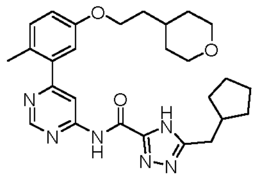

Example 446 Example 447

Example 447: 5-(cyclopentylmethyl)-N-(6-(2-methyl-5-(2-(tetrahydro-2H-pyran-4-yl)ethoxy)phenyl)pyrimidin-4-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.49 (s, 1H), 10.27 (s, 1H), 9.02 (d, *J* = 1.3 Hz, 1H), 8.25 (d, *J* = 1.3 Hz, 1H), 7.26 (d, *J* = 8.4 Hz, 1H), 7.12 - 6.83 (m, 2H), 4.04 (t, *J* = 6.3 Hz, 2H), 3.93 - 3.66 (m, 2H), 3.30 - 3.17 (m, 2H), 2.78 (d, *J* = 7.5 Hz, 2H), 2.31 (s, 3H), 2.29 - 2.14 (m, 1H), 1.80 - 1.43 (m, 11H), 1.26 - 1.14 (m, 4H). MS: 491 $[M+H]^+$.

Example 448: 5-(cyclohexylmethyl)-N-(6-(2-methyl-5-(2-(tetrahydro-2H-pyran-4-yl)ethoxy)phenyl)pyrimidin-4-yl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.39 (s, 1H), 10.26 (s, 1H), 9.01 (s, 1H), 8.25 (s, 1H), 7.26 (d, *J* = 8.4 Hz, 1H), 7.05 (d, *J* = 2.8 Hz, 1H), 7.00 (dd, *J* = 8.4, 2.8 Hz, 1H), 4.04 (t, *J* = 6.3 Hz, 2H), 3.82 (dd, *J* = 11.2, 4.3 Hz, 2H), 3.28-3.22 (m, 2H), 2.67 (d, *J* = 7.0 Hz, 2H), 2.31 (s, 3H), 1.83-1.55 (m, 12H), 1.25-1.17 (m, 4H), 1.04-0.89 (m, 2H). MS: 505 $[M+H]^+$.

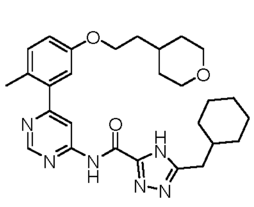 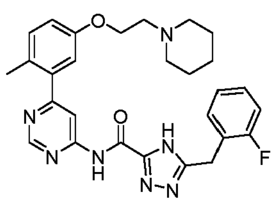

Example 448 Example 449

Example 449: 5-(2-fluorobenzyl)-N-(6-(2-methyl-5-(2-(piperidin-1-yl)ethoxy)phenyl)pyrimidin-4-yl)-4H-1,2,4-triazole-3-carboxamide

The preparation was carried out in a similar manner to Example 92, except that in step 1, 1-(2-chloroethyl)piperidine hydrochloride was used in place of iodomethane, 3-bromo-4-methylphenol was used in place of 2-bromo-3-methylphenol, in step 2, 6-chloropyrimidin-4-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.57 (s, 1H), 10.33 (s, 1H), 9.01 (d, *J* = 1.2 Hz, 1H), 8.23 (d, *J* = 1.3 Hz, 1H), 7.46-7.29 (m, 2H), 7.29-7.16 (m, 3H), 7.05 (d, *J* = 2.7 Hz, 1H), 7.00 (dd, *J* = 8.4, 2.8 Hz, 1H), 4.21 (s, 2H), 4.09 (t, *J* = 5.9 Hz, 2H), 2.67 (t, *J* = 5.9 Hz, 2H), 2.47-2.39 (m, 4H), 2.31 (s, 3H), 1.54-1.44 (m, 4H), 1.41-1.31 (m, 2H). MS: 516 $[M+H]^+$.

Example 450: 5-benzyl-N-(6-(5-((4-hydroxy-4-methylpentyl)oxy)-2-(trifluoromethyl)phenyl)pyrimidin-4-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, and in step 3, 6-chloropyrimidin-4-amine was used in place of 4-bromopyridin-2-amine. $^1$H NMR (400 MHz, DMSO-$d_6$) δ 14.68 (s, 1H), 10.45 (s, 1H), 9.02 (d, *J* = 1.2 Hz, 1H), 8.25 (d, *J* = 1.2 Hz, 1H), 7.81 (d, *J* = 8.9 Hz, 1H), 7.38 - 7.29 (m, 4H), 7.29 - 7.20 (m, 2H), 7.11 (d, *J* = 2.6 Hz, 1H), 4.24 - 4.15 (m, 3H), 4.10 (t, *J* = 6.6 Hz, 2H), 1.84 - 1.69 (m, 2H), 1.54 - 1.41 (m, 2H), 1.10 (s, 6H). MS: 541 $[M+H]^+$.

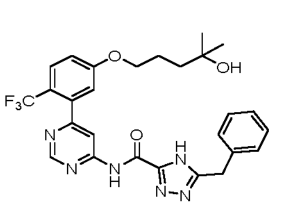 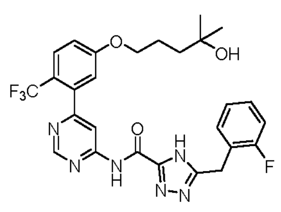

Example 450 Example 451

Example 451: 5-(2-fluorobenzyl)-N-(6-(5-((4-hydroxy-4-methylpentyl)oxy)-2-(trifluoromethyl)phenyl)pyrimidin-4-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.75 (s, 1H), 10.46 (s, 1H), 9.02 (d, *J* = 1.3 Hz, 1H), 8.24 (d, *J* = 1.3 Hz, 1H), 7.81 (d, *J* = 8.9 Hz, 1H), 7.45 - 7.30 (m, 2H), 7.27 - 7.16 (m, 3H), 7.11 (d, *J* = 2.6 Hz, 1H), 4.27 - 4.16 (m, 3H), 4.10 (t, *J* = 6.6 Hz, 2H), 1.86 - 1.71 (m, 2H), 1.55 - 1.42 (m, 2H), 1.10 (s, 6H). MS: 559 $[M+H]^+$.

Example 452: 5-(cyclopentylmethyl)-N-(6-(5-((4-hydroxy-4-methylpentyl)oxy)-2-(trifluoromethyl)phenyl)pyrimidin-4-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.54 (s, 1H), 10.39 (s, 1H), 9.03 (d,

$J$ = 1.3 Hz, 1H), 8.26 (d, $J$ = 1.3 Hz, 1H), 7.81 (d, $J$ = 8.9 Hz, 1H), 7.24 (dd, $J$ = 8.7, 2.6 Hz, 1H), 7.12 (d, $J$ = 2.6 Hz, 1H), 4.20 (s, 1H), 4.10 (t, $J$ = 6.5 Hz, 2H), 2.78 (d, $J$ = 7.5 Hz, 2H), 2.36 - 2.17 (m, 1H), 1.86 - 1.66 (m, 4H), 1.66 - 1.43 (m, 6H), 1.25 - 1.18 (m, 2H), 1.10 (s, 6H). MS: 533 $[M+H]^+$.

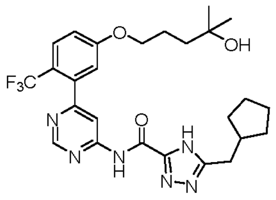

Example 452

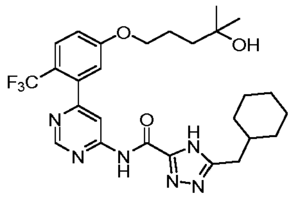

Example 453

Example 453: 5-(cyclohexylmethyl)-N-(6-(5-((4-hydroxy-4-methylpentyl)oxy)-2-(trifluoromethyl)phenyl)pyrimidin-4-yl)-4H-1,2,4-triazole-3 -carboxamide

The preparation was carried out in a similar manner to Example 160, except that in step 1, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.47 (s, 1H), 10.37 (s, 1H), 9.03 (d, $J$ = 1.2 Hz, 1H), 8.26 (d, $J$ = 1.3 Hz, 1H), 7.81 (d, $J$ = 8.9 Hz, 1H), 7.24 (dd, $J$ = 8.9, 2.6 Hz, 1H), 7.12 (d, $J$ = 2.6 Hz, 1H), 4.20 (s, 1H), 4.10 (t, $J$ = 6.6 Hz, 2H), 2.67 (d, $J$ = 7.1 Hz, 2H), 1.85 - 1.54 (m, 8H), 1.54 - 1.41 (m, 2H), 1.23 - 1.16 (m, 3H), 1.10 (s, 6H), 1.05 - 0.89 (m, 2H). MS: 547 $[M+H]^+$.

Example 454: 5-benzyl-N-(6-(5-((tetrahydro-2H-pyran-4-yl)methoxy)-2-(trifluoromethyl)phenyl)pyrimidin-4-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 3-bromo-4-methylphenol, and in step 2, 6-chloropyrimidin-4-amine was used in place of 4-bromopyridin-2-amine. $^1$H NMR (400 MHz, DMSO-$d_6$) $\delta$ 14.65 (s, 1H), 10.45 (s, 1H), 9.02 (d, $J$ = 1.2 Hz, 1H), 8.25 (d, $J$ = 1.2 Hz, 1H), 7.81 (d, $J$ = 8.9 Hz, 1H), 7.39 - 7.29 (m, 4H), 7.29 - 7.19 (m, 2H), 7.13 (d, $J$ = 2.6 Hz, 1H), 4.19 (s, 2H), 3.98 (d, $J$ = 6.5 Hz, 2H), 3.92-3.81 (m, 2H), 3.37-3.32 (m, 2H), 2.07 - 1.94 (m, 1H), 1.74 - 1.60 (m, 2H), 1.40 - 1.26 (m, 2H). MS: 539 $[M+H]^+$.

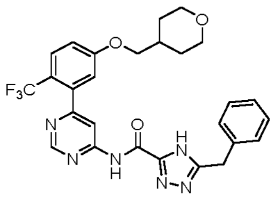

Example 454

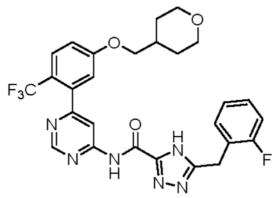

Example 455

Example 455: 5-(2-fluorobenzyl)-N-(6-(5-((tetrahydro-2H-pyran-4-yl)methoxy)-2-(trifluoromethyl)phenyl)pyrimidin-4-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 3-bromo-4-methylphenol, in step 2, 6-chloropyrimidin-4-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) $\delta$ 14.73 (s, 1H), 10.46 (s, 1H), 9.02 (d, $J$ = 1.3 Hz, 1H), 8.24 (d, $J$ = 1.3 Hz, 1H), 7.81 (d, $J$ = 8.9 Hz, 1H), 7.49 - 7.28 (m, 2H), 7.28 - 7.07 (m, 4H), 4.22 (s, 2H), 3.97 (d, $J$ = 6.5 Hz, 2H), 3.93 - 3.82 (m, 2H), 3.36 - 3.30 (m, 2H), 2.09 - 1.95 (m, 1H), 1.74 - 1.62 (m, 2H), 1.42 - 1.24 (m, 2H). MS: 557 $[M+H]^+$.

Example 456: 5-(cyclopentylmethyl)-N-(6-(5-((tetrahydro-2H-pyran-4-yl)methoxy)-2-(trifluoromethyl)phenyl)pyrimidin-4-yl)-4H- 1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 3-bromo-4-methylphenol, in step 2, 6-chloropyrimidin-4-amine was used in place of 4-bromopyridin-2-amine, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.47 (s, 1H), 10.38 (s, 1H), 9.03 (d, *J* = 1.2 Hz, 1H), 8.26 (d, *J* = 1.2 Hz, 1H), 7.81 (d, *J* = 8.9 Hz, 1H), 7.26 (dd, *J* = 8.8, 2.6 Hz, 1H), 7.14 (d, *J* = 2.6 Hz, 1H), 3.98 (d, *J* = 6.4 Hz, 2H), 3.93 - 3.83 (m, 2H), 3.43 - 3.36 (m, 2H), 2.78 (d, *J* = 7.5 Hz, 2H), 2.36 - 2.21 (m, 1H), 2.08 - 1.95 (m, 1H), 1.75 - 1.64 (m, 4H), 1.64 - 1.56 (m, 2H), 1.56 - 1.46 (m, 2H), 1.39 - 1.28 (m, 2H), 1.23 - 1.14 (m, 2H). MS: 531 $[M+H]^+$.

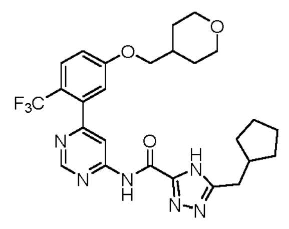
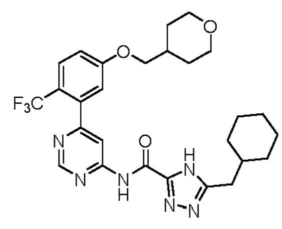

Example 456 Example 457

Example 457: 5-(cyclohexylmethyl)-N-(6-(5-((tetrahydro-2H-pyran-4-yl)methoxy)-2-(trifluoromethyl)phenyl)pyrimidin-4-yl)-4H-1,2,4-triazole-3 -carboxamide

The preparation was carried out in a similar manner to Example 64, except that in step 1, (tetrahydro-2H-pyran-4-yl)methanol was used in place of 2-(tert-butoxy)ethan-1-ol, 3-chloro-4-(trifluoromethyl)phenol was used in place of 3-bromo-4-methylphenol, in step 2, 6-chloropyrimidin-4-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.47 (s, 1H), 10.38 (s, 1H), 9.03 (d, *J* = 1.3 Hz, 1H), 8.26 (d, *J* = 1.3 Hz, 1H), 7.81 (d, *J* = 8.9 Hz, 1H), 7.26 (dd, *J* = 8.7, 2.6 Hz, 1H), 7.14 (d, *J* = 2.6 Hz, 1H), 3.98 (d, *J* = 6.5 Hz, 2H), 3.93 - 3.81 (m, 2H), 3.35 - 3.30 (m, 2H), 2.67 (d, *J* = 7.1 Hz, 2H), 2.11-1.96 (m, 1H), 1.83 - 1.57 (m, 8H), 1.42 -1.26 (m, 2H), 1.26 - 1.07 (m, 3H), 1.07 - 0.91 (m, 2H). MS: 545 $[M+H]^+$.

Example 458: 5-benzyl-N-(6-(5-(2-(tetrahydro-2H-pyran-4-yl)ethoxy)-2-(trifluoromethyl)phenyl)pyrimidin-4-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 3-bromo-4-methylphenol, and in step 3, 6-chloropyrimidin-4-amine was used in place of 4-bromopyridin-2-amine. $^1$H NMR (400 MHz, DMSO-$d_6$) δ 14.59 (s, 1H), 10.47 (s, 1H), 9.02 (d, *J* = 1.2 Hz, 1H), 8.25 (d, *J* = 1.2 Hz, 1H), 7.80 (d, *J* = 8.9 Hz, 1H), 7.39 - 7.29 (m, 4H), 7.29 - 7.18 (m, 2H), 7.13 (d, *J* = 2.6 Hz, 1H), 4.25 - 4.10 (m, 4H), 3.88 - 3.76 (m, 2H), 3.27 - 3.23 (m, 2H), 1.77 - 1.65 (m, 3H), 1.65 - 1.56 (m, 2H), 1.26 - 1.19 (m, 2H). MS: 553 $[M+H]^+$.

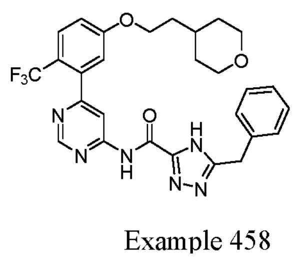
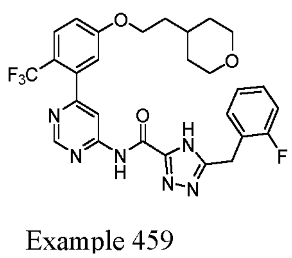

Example 458 Example 459

Example 459: 5-(2-fluorobenzyl)-N-(6-(5-(2-(tetrahydro-2H-pyran-4-yl)ethoxy)-2-(trifluoromethyl)phenyl)pyrimidin-4-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.70 (s, 1H), 10.47 (s, 1H), 9.02 (d, *J* = 1.2 Hz, 1H), 8.24 (d, *J* = 1.2 Hz, 1H), 7.80 (d, *J* = 8.9 Hz, 1H), 7.50-7.30 (m, 2H), 7.30-7.06 (m, 4H), 4.21 (s, 2H), 4.15 (t, *J* = 6.1 Hz, 2H), 3.88-3.76 (m, 2H), 3.29-3.18 (m, 2H), 1.71-1.56 (m, 4H), 1.30-1.13 (m, 3H). MS: 571 $[M+H]^+$.

Example 460: 5-(cyclopentylmethyl)-N-(6-(5-(2-(tetrahydro-2H-pyran-4-yl)ethoxy)-2-(trifluoromethyl)phenyl)pyrimidin-4-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.47 (s, 1H), 10.40 (s, 1H), 9.03 (s, 1H), 8.26 (s, 1H), 7.81 (d, *J* = 8.9 Hz, 1H), 7.26 (dd, *J* = 8.8, 2.6 Hz, 1H), 7.14 (d, *J* = 2.6 Hz, 1H), 4.16 (t, *J* = 6.0 Hz, 2H), 3.82 (dd, *J* = 10.8, 4.3 Hz, 2H), 3.29 - 3.21 (m, 2H), 2.78 (d, *J* = 7.5 Hz, 2H), 2.37 - 2.18 (m, 1H), 1.82 - 1.66 (m, 5H), 1.66 - 1.57 (m, 4H), 1.57 - 1.46 (m, 2H), 1.25 - 1.14 (m, 4H). MS: 545 $[M+H]^+$.

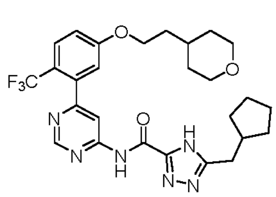

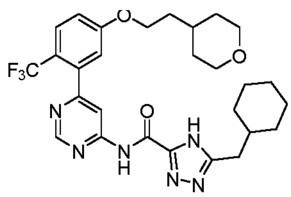

Example 460 Example 461

Example 461: 5-(cyclohexylmethyl)-N-(6-(5-(2-(tetrahydro-2H-pyran-4-yl)ethoxy)-2-(trifluoromethyl)phenyl)pyrimidin-4-yl)-4H-1,2,4-triazole-3 -carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-chloro-4-(trifluoromethyl)phenol was used in place of 3-bromo-4-methylphenol, in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-*$d_6$*) δ 14.47 (s, 1H), 10.40 (s, 1H), 9.03 (d, *J* = 1.2 Hz, 1H), 8.26 (d, *J* = 1.2 Hz, 1H), 7.81 (d, *J* = 8.9 Hz, 1H), 7.26 (dd, *J* = 8.7, 2.6 Hz, 1H), 7.14 (d, *J* = 2.6 Hz, 1H), 4.16 (t, *J* = 6.1 Hz, 2H), 3.90 -3.73 (m, 2H), 3.31-3.20 (m, 2H), 2.67 (d, *J* = 7.1 Hz, 2H), 1.83 - 1.54 (m, 11H), 1.24 - 1.06 (m, 5H), 1.06 - 0.89 (m, 2H). MS: 559 $[M+H]^+$.

Example 462: 5-benzyl-N-(6-(2-chloro-5-((4-hydroxy-4-methylpentyl)oxy)phenyl)pyrimidin-4-yl)-4H -1,2,4-triazole-3-carboxamide

The preparation was carried out in a similar manner to Example 160, except that in step 1, 3-bromo-4-chlorophenol was used in place of 3-bromo-4-methylphenol, and in step 3, 6-chloropyrimidin-4-amine was used in place of 4-bromopyridin-2-amine. $^1$H NMR (400 MHz, DMSO-*$d_6$*) δ 14.61 (s, 1H), 10.42 (s, 1H), 9.06 (d, *J* = 1.3 Hz, 1H), 8.45 (d, *J* = 1.3 Hz, 1H), 7.52 (d, *J* = 8.8 Hz, 1H), 7.38 - 7.30 (m, 4H), 7.30 - 7.22 (m, 1H), 7.20 (d, *J* = 3.0 Hz, 1H), 7.11 (dd, *J* = 8.8, 3.1 Hz, 1H), 4.27 - 4.13 (m, 3H), 4.01 (t, *J* = 6.5 Hz, 2H), 1.84 - 1.70 (m, 2H), 1.53 - 1.43 (m, 2H), 1.09 (s, 6H). MS: 507 $[M+H]^+$.

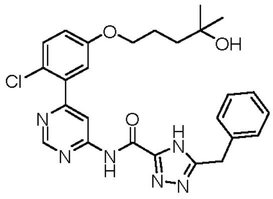 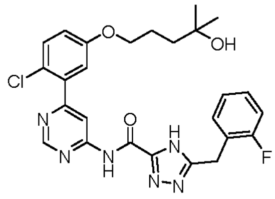

Example 462 Example 463

Example 463: 5-(2-fluorobenzyl)-N-(6-(2-chloro-5-((4-hydroxy-4-methylpentyl)oxy)phenyl)pyrimidin-4-yl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.71 (s, 1H), 10.42 (s, 1H), 9.05 (d, *J* = 1.2 Hz, 1H), 8.44 (d, *J* = 1.2 Hz, 1H), 7.52 (d, *J* = 8.8 Hz, 1H), 7.46 - 7.30 (m, 2H), 7.27 - 7.15 (m, 3H), 7.11 (dd, *J* = 8.8, 3.1 Hz, 1H), 4.31-4.14 (m, 3H), 4.01 (t, *J* = 6.6 Hz, 2H), 1.84 - 1.68 (m, 2H), 1.55 - 1.41 (m, 2H), 1.09 (s, 6H). MS: 525 [M+H]$^+$.

Example 464: 5-(cyclopentylmethyl)-N-(6-(2-chloro-5-((4-hydroxy-4-methylpentyl)oxy)phenyl)pyrimidin-4-yl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.32 (s, 1H), 10.34 (s, 1H), 9.06 (d, *J* = 1.3 Hz, 1H), 8.46 (d, *J* = 1.2 Hz, 1H), 7.52 (d, *J* = 8.8 Hz, 1H), 7.21 (d, *J* = 3.1 Hz, 1H), 7.11 (dd, *J* = 8.8, 3.1 Hz, 1H), 4.19 (s, 1H), 4.02 (t, *J* = 6.5 Hz, 2H), 2.78 (d, *J* = 7.5 Hz, 2H), 2.37 - 2.20 (m, 1H), 1.85 - 1.66 (m, 4H), 1.66 - 1.42 (m, 6H), 1.25 - 1.16 (m, 2H), 1.10 (s, 6H). MS: 499 [M+H]$^+$.

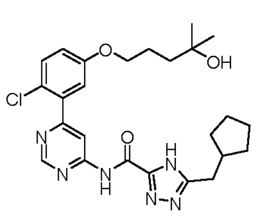 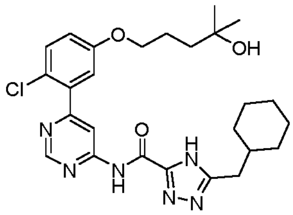

Example 464 Example 465

Example 465: 5-(cyclohexylmethyl)-N-(6-(2-chloro-5-((4-hydroxy-4-methylpentyl)oxy)phenyl)pyrimidin-4-yl)-4H-1,2,4-triazole-3-carboxamide

The preparation was carried out in a similar manner to Example 160, except that in step 1, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 10.35 (s, 1H), 9.06 (s, 1H), 8.46 (s, 1H), 7.52 (d, *J* = 8.8 Hz, 1H), 7.21 (d, *J* = 3.0 Hz, 1H), 7.11 (dd, *J* = 8.9, 3.1 Hz, 1H), 4.19 (s, 1H), 4.02 (t, *J* = 6.5 Hz, 2H), 2.67 (d, *J* = 7.0 Hz, 2H), 1.84 - 1.55 (m, 8H), 1.55 - 1.41 (m, 2H), 1.26 - 1.14 (m, 3H), 1.10 (s, 6H), 1.05 - 0.91 (m, 2H). MS: 513 [M+H]$^+$.

Example 466: 5-benzyl-N-(6-(2-chloro-5-((tetrahydro-2H-pyran-4-yl)methoxy)phenyl)pyrimidin-4-yl)-4 H -1 ,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, and in step 2, 6-chloropyrimidin-4-amine was used in place of 4-bromopyridin-2-amine. $^1$H NMR (400 MHz, DMSO-$d_6$) δ 14.59 (s, 1H), 10.42 (s, 1H), 9.06 (d, $J$ = 1.3 Hz, 1H), 8.45 (d, $J$ = 1.3 Hz, 1H), 7.52 (d, $J$ = 8.9 Hz, 1H), 7.41 - 7.29 (m, 4H), 7.29 - 7.19 (m, 2H), 7.12 (dd, $J$ = 8.9, 3.1 Hz, 1H), 4.18 (s, 2H), 3.97 - 3.82 (m, 4H), 3.30 - 3.28 (m, 2H), 2.05 - 1.93 (m, 1H), 1.75 - 1.60 (m, 2H), 1.40 - 1.25 (m, 2H). MS: 505 [M+H]$^+$.

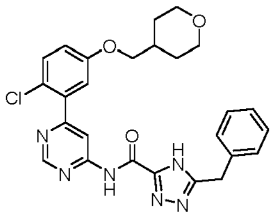
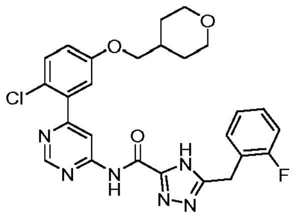

Example 466 Example 467

Example 467: 5-(2-fluorobenzyl)-N-(6-(2-chloro-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 6-chloropyrimidin-4-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.41 (s, 1H), 9.05 (d, $J$ = 1.2 Hz, 1H), 8.44 (d, $J$ = 1.3 Hz, 1H), 7.52 (d, $J$ = 8.8 Hz, 1H), 7.46 - 7.30 (m, 2H), 7.28 - 7.16 (m, 3H), 7.12 (dd, $J$ = 8.9, 3.1 Hz, 1H), 4.21 (s, 2H), 3.94 - 3.81 (m, 4H), 3.31 - 3.29 (m, 2H), 2.09 - 1.92 (m, 1H), 1.73 - 1.60 (m, 2H), 1.40 - 1.25 (m, 2H). MS: 523 [M+H]$^+$.

Example 468: 5-(cyclopentylmethyl)-N-(6-(2-chloro-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 6-chloropyrimidin-4-amine was used in place of 4-bromopyridin-2-amine, and in step 3, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.50 (s, 1H), 10.35 (s, 1H), 9.06 (d, $J$ = 1.3 Hz, 1H), 8.46 (d, $J$ = 1.3 Hz, 1H), 7.53 (d, $J$ = 8.9 Hz, 1H), 7.23 (d, $J$ = 3.1 Hz, 1H), 7.13 (dd, $J$ = 8.8, 3.1 Hz, 1H), 3.95 - 3.81 (m, 4H), 3.31 - 3.28 (m, 2H), 2.78 (d, $J$ = 7.5 Hz, 2H), 2.37 - 2.19 (m, 1H), 2.09 - 1.94 (m, 1H), 1.80 - 1.44 (m, 8H), 1.39 - 1.27 (m, 2H), 1.27 - 1.22 (m, 2H). MS: 497 [M+H]$^+$.

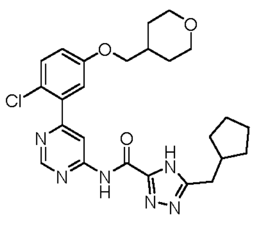
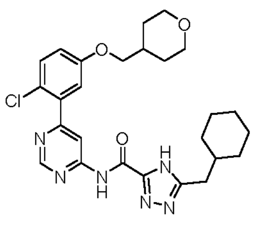

Example 468 Example 469

Example 469: 5-(cyclohexylmethyl)-N-(6-(2-chloro-5-((tetrahydro-2H-pyran-4-yl)methoxy)phenyl)pyrimidin-4-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, 3-bromo-4-chlorophenol was used in place of 3-bromo-4-methylphenol, in step 2, 6-chloropyrimidin-4-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.54 (s, 1H), 10.35 (s, 1H), 9.06 (d, $J$ = 1.3 Hz, 1H), 8.46 (d, $J$ = 1.2 Hz, 1H), 7.53 (d,

$J$ = 8.8 Hz, 1H), 7.23 (d, $J$ = 3.1 Hz, 1H), 7.13 (dd, $J$ = 8.9, 3.1 Hz, 1H), 3.98 - 3.82 (m, 4H), 3.31 - 3.28 (m, 2H), 2.67 (d, $J$ = 7.0 Hz, 2H), 2.11 - 1.92 (m, 1H), 1.83 - 1.56 (m, 8H), 1.41 - 1.26 (m, 2H), 1.26 - 1.10 (m, 3H), 1.07 - 0.92 (m, 2H). MS: 511 $[M+H]^+$.

Example 470: 5-benzyl-N-(6-(2-chloro-5-(2-(tetrahydro-2H-pyran-4-yl)ethoxy)phenyl)pyrimidin-4-yl)-4 H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, and in step 3, 6-chloropyrimidin-4-amine was used in place of 4-bromopyridin-2-amine. $^1$H NMR (400 MHz, DMSO-$d_6$) δ 14.71 (s, 1H), 10.43 (s, 1H), 9.06 (d, $J$ = 1.3 Hz, 1H), 8.44 (d, $J$ = 1.3 Hz, 1H), 7.52 (d, $J$ = 8.9 Hz, 1H), 7.37 - 7.30 (m, 4H), 7.29 - 7.21 (m, 2H), 7.12 (dd, $J$ = 8.9, 3.1 Hz, 1H), 4.18 (s, 2H), 4.07 (t, $J$ = 6.2 Hz, 2H), 3.90 - 3.73 (m, 2H), 3.28 - 3.20 (m, 2H), 1.77-1.56 (m, 5H), 1.30 - 1.15 (m, 2H). MS: 519 $[M+H]^+$.

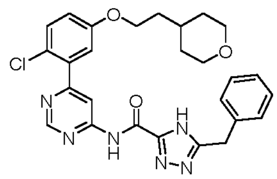

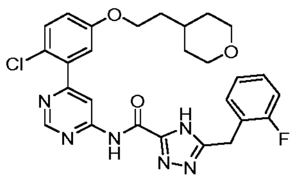

Example 470 Example 471

Example 471: N-(6-(2-chloro-5-(2-(tetrahydro-2H-pyran-4-yl)ethoxy)phenyl)pyrimidin-4-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.34 (s, 1H), 10.58 (s, 1H), 9.04 (d, $J$ = 1.3 Hz, 1H), 8.44 (d, $J$ = 1.3 Hz, 1H), 7.51 (d, $J$ = 8.9 Hz, 1H), 7.43 - 7.28 (m, 2H), 7.25 - 7.15 (m, 3H), 7.12 (dd, $J$ = 8.8, 3.1 Hz, 1H), 4.19 (s, 2H), 4.07 (t, $J$ = 6.2 Hz, 2H), 3.86 - 3.78 (m, 2H), 3.29 - 3.27 (m, 2H), 1.74 - 1.57 (m, 5H), 1.28 - 1.19 (m, 2H). MS: 537 $[M+H]^+$.

Example 472: N-(6-(2-chloro-5-(2-(tetrahydro-2H-pyran-4-yl)ethoxy)phenyl)pyrimidin-4-yl)-5-(cyclopentylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(cyclopentylmethyl)-4H-1,2,4-triazole-3-carboxylate was used in place of ethyl 5-benzyl-4H-1,2,4-triazol-3-carboxylate. $^1$H NMR (400 MHz, DMSO-$d_6$) δ 14.39 (s, 1H), 10.33 (s, 1H), 9.06 (d, $J$ = 1.3 Hz, 1H), 8.46 (d, $J$ = 1.3 Hz, 1H), 7.52 (d, $J$ = 8.8 Hz, 1H), 7.23 (d, $J$ = 3.1 Hz, 1H), 7.13 (dd, $J$ = 8.9, 3.1 Hz, 1H), 4.08 (t, $J$ = 6.2 Hz, 2H), 3.89 - 3.77 (m, 2H), 3.28 - 3.23 (m, 2H), 2.82 - 2.75 (m, 2H), 2.36 - 2.21 (m, 1H), 1.77 - 1.56 (m, 10H), 1.56 - 1.46 (m, 2H), 1.24 - 1.16 (m, 3H). MS: 511 $[M+H]^+$.

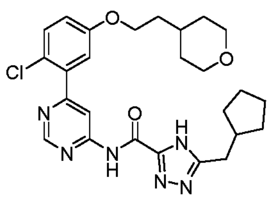

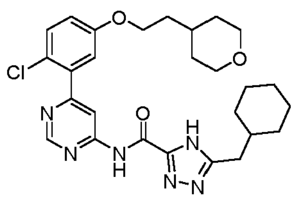

Example 472 Example 473

Example 473: N-(6-(2-chloro-5-(2-(tetrahydro-2H-pyran-4-yl)ethoxy)phenyl)pyrimidin-4-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 2, 3-bromo-4-chlorophenol was used in place of 3-bromo-4-methylphenol, in step 3, 6-chloropyrimidin-4-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 10.36 (s, 1H), 9.06 (s, 1H), 8.46 (s, 1H), 7.52 (d, *J* = 8.8 Hz, 1H), 7.23 (d, *J* = 3.1 Hz, 1H), 7.12 (dd, *J* = 8.8, 3.1 Hz, 1H), 4.08 (t, *J* = 6.2 Hz, 2H), 3.86 - 3.78 (m, 2H), 3.28 - 3.23 (m, 2H), 2.67 (d, *J* = 7.1 Hz, 2H), 1.79 - 1.72 (m, 1H), 1.72 - 1.55 (m, 10H), 1.24 - 1.08 (m, 5H), 1.05 - 0.92 (m, 2H). MS: 525 [M+H]$^+$.

Example 474: 5-benzyl-N-(4-(5-((4-hydroxy-4-methylpentyl)sulfonyl)-2-methylphenyl)pyridin-2-yl)-4 H-1,2,4-triazole-3 -carboxamide

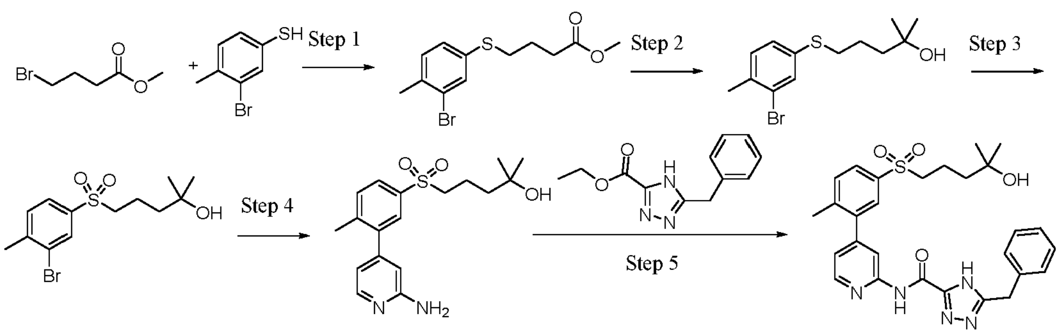


Steps 1 to 2 were carried out in a similar manner to Steps 1 to 2 of Example 160, except that 3-bromo-4-methylbenzenethio was used in place of 3-bromo-4-methylphenol.

Step 3: 5-((3-bromo-4-methylphenyl)thio)-2-methylpentan-2-ol (606mg, 2mmol) was dissolved in dichloromethane (6mL), m-chloroperoxybenzoic acid (520mg, 3mmol) was added in batches at 0°C, and reacted at 25°C for 1.5 hours. The reaction solution was diluted with dichloromethane, washed with saturated aqueous solution of sodium carbonate and saturated brine, dried over anhydrous sodium sulfate, filtered and evaporated to dryness, purified by column chromatography to afford 600mg of 5-((3-bromo-4-methylphenyl)sulfonyl)-2-methylpentan-2-ol.

Steps 4 to 5 were carried out in a similar manner to Steps 3 to 4 of Example 160, except that 5-((3-bromo-4-methylphenyl)sulfonyl)-2-methylpentan-2-ol was used in place of 5-(3-bromo-4-methylphenoxy)-2-methylpentan-2-ol.

$^1$H NMR (400 MHz, DMSO-$d_6$) δ 14.59 (s, 1H), 10.00 (s, 1H), 8.48 (d, *J* = 5.1 Hz, 1H), 8.15 (d, *J* = 1.5 Hz, 1H), 7.88 (dd, *J* = 8.1, 2.0 Hz, 1H), 7.74 (d, *J* = 2.0 Hz, 1H), 7.67 (d, *J* = 8.1 Hz, 1H), 7.40 - 7.20 (m, 6H), 4.20 (s, 1H), 4.18 (s, 2H), 3.32 - 3.23 (m, 2H), 2.38 (s, 3H), 1.71-1.55 (m, 2H), 1.44 - 1.32 (m, 2H), 1.02 (s, 6H). MS: 534 [M+H]$^+$.

Example 475: 5-(cyclopentylmethyl)-N-(4-(5-((4-hydroxy-4-methylpentyl)sulfonyl)-2-methylphenyl)pyridin-2-yl)-4H-1,2,4-triazole-3-carboxamide

The preparation was carried out in a similar manner to Example 474, except that in step 5, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.41 (s, 1H), 9.94 (s, 1H), 8.49 (d, *J* = 5.1 Hz, 1H), 8.25 - 8.10 (m, 1H), 7.88 (dd, *J* = 8.0, 2.0 Hz, 1H), 7.75 (d, *J* = 2.0 Hz, 1H), 7.67 (d, *J* = 8.1 Hz, 1H), 7.29 (dd, *J* = 5.1, 1.6 Hz, 1H), 4.20 (s, 1H), 3.39 - 3.34 (m, 2H), 2.78 (d, *J* = 7.5 Hz, 2H), 2.39 (s, 3H), 2.35 - 2.18 (m, 1H), 1.81 - 1.67 (m, 2H), 1.67 - 1.56 (m, 4H), 1.56 - 1.45 (m, 2H), 1.44 - 1.33 (m, 2H), 1.26 - 1.18 (m, 2H), 1.03 (s, 6H). MS: 526 [M+H]$^+$.

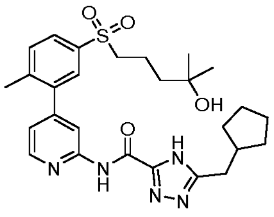
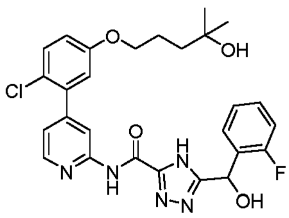

Example 475 Example 476

Example 476: N-(4-(2-chloro-5-((4-hydroxy-4-methylpentyl)oxy)phenyl)pyridin-2-yl)-5-((2-fluorophenyl)(hydroxyl)methyl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl 5-((2-fluorophenyl)(hydroxyl)methyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.89 (s, 1H), 9.87 (s, 1H), 8.44 (d, *J* = 5.1 Hz, 1H), 8.20 (d, *J* = 1.5 Hz, 1H), 7.58 (s, 1H), 7.50 (d, *J* = 8.8 Hz, 1H), 7.43 - 7.34 (m, 1H), 7.32 - 7.16 (m, 3H), 7.05 (dd, *J* = 8.8, 3.0 Hz, 1H), 7.00 (d, *J* = 3.0 Hz, 1H), 6.77 (s, 1H), 6.15 (d, *J* = 4.9 Hz, 1H), 4.19 (s, 1H), 4.00 (t, *J* = 6.6 Hz, 2H), 1.82 - 1.70 (m, 2H), 1.52 - 1.41 (m, 2H), 1.09 (s, 6H). MS: 540 $[M+H]^+$.

Example 477: (R)-N-(4-(2-chloro-5-((4-hydroxy-4-methylpentyl)oxy)phenyl)pyridin-2-yl)-5-(hydroxyl(phenyl)methyl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl (R)-5-(hydroxyl(phenyl)methyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.78 (s, 1H), 9.86 (s, 1H), 8.44 (d, *J* = 5.2 Hz, 1H), 8.22 (s, 1H), 7.54 - 7.44 (m, 3H), 7.44 - 7.34 (m, 2H), 7.34 - 7.23 (m, 2H), 7.06 (dd, *J* = 8.8, 3.0 Hz, 1H), 7.00 (d, *J* = 3.0 Hz, 1H), 6.71 (d, *J* = 4.3 Hz, 1H), 5.96 (d, *J* = 4.3 Hz, 1H), 4.18 (s, 1H), 4.01 (t, *J* = 6.5 Hz, 2H), 1.82 -1.68 (m, 2H), 1.53 - 1.41 (m, 2H), 1.09 (s, 6H). MS: 522 $[M+H]^+$.

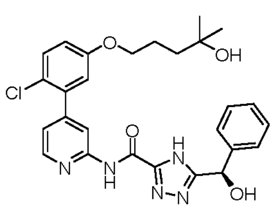
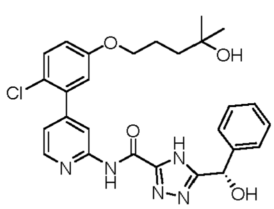

Example 477 Example 478

Example 478: (S)-N-(4-(2-chloro-5-((4-hydroxy-4-methylpentyl)oxy)phenyl)pyridin-2-yl)-5-(hydroxyl(phenyl)methyl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl (S)-5-(hydroxyl(phenyl)methyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.78 (s, 1H), 9.88 (s, 1H), 8.45 (d, *J* = 5.1 Hz, 1H), 8.21 (s, 1H), 7.54 - 7.44 (m, 3H), 7.38 (t, *J* = 7.5 Hz, 2H), 7.34 - 7.24 (m, 2H), 7.06 (dd, *J* = 8.8, 3.0 Hz, 1H), 7.00 (d, *J* = 3.1 Hz, 1H), 6.71 (s, 1H), 5.95 (s, 1H), 4.19 (s, 1H), 4.01 (t, *J* = 6.6 Hz, 2H), 1.82 - 1.69 (m, 2H), 1.51 - 1.40 (m, 2H), 1.09 (s, 6H). MS: 522 $[M+H]^+$.

Example 479: 5-benzyl-N-(4-(2-methyl-5-(tetrahydro-2H-pyran-4-yl)phenyl)pyridin-2-yl)-4H-1,2,4-triazole-3-carboxamide

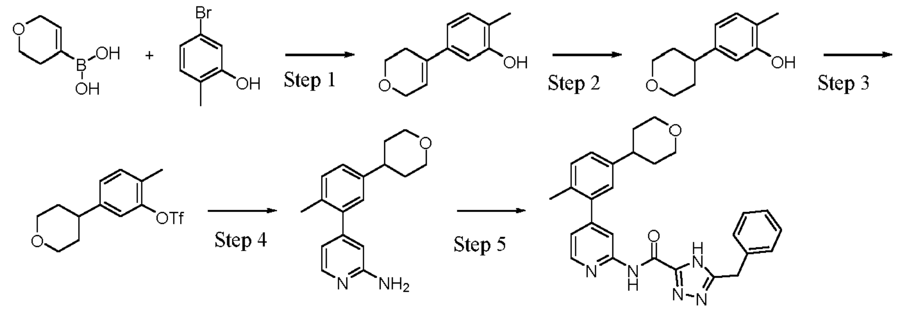


Steps 1 to 2 were carried out in a similar manner to Steps 1 to 2 of Example 47, except that (3,6-dihydro-2H-pyran-4-yl)boronic acid was used in place of cyclopent-1-en-1-ylboronic acid, and 5-bromo-2-methylphenol was used in place of 4-bromopyridin-2-amine.

Steps 3 to 5 were carried out in a similar manner to Example 43, except that 2-methyl-5-(tetrahydro-2H-pyran)-4-yl)phenol was used in place of 5,6,7,8-tetrahydronaphthalen-1-ol. $^1$H NMR (400 MHz, DMSO-$d_6$) $\delta$ 14.59 (s, 1H), 9.90 (s, 1H), 8.41 (d, *J* = 5.1 Hz, 1H), 8.11 (d, *J* = 1.5 Hz, 1H), 7.45 - 7.29 (m, 4H), 7.29 - 7.13 (m, 5H), 4.18 (s, 2H), 4.01 - 3.91 (m, 2H), 3.50 - 3.41 (m, 2H), 2.85 - 2.71 (m, 1H), 2.28 (s, 3H), 1.81 - 1.64 (m, 4H). MS: 454 [M+H]$^+$.

Example 480: Methyl 3-(3-(2-(5-benzyl-4H-1,2,4-triazol-3-carboxamido)pyridin-4-yl)-4-methylphenyl)acrylate

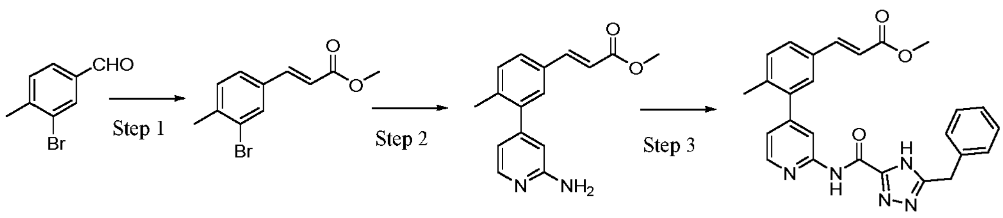


Step 1 was carried out in a similar manner to Step 2 of Example 78, except that 3-bromo-4-methylbenzaldehyde was used in place of 2-bromo-4-(2-(tert-butoxy)ethoxy)benzaldehyde, and trimethyl phosphonoacetate was used in place of methyltriphenylphosphonium bromide.

Steps 2 to 3 were carried out in a similar manner to Example 33, except that methyl 3-(3-bromo-4-methylphenyl)acrylate was used in place of 2-bromo-4-fluoro-1-methylbenzene. $^1$H NMR (400 MHz, DMSO-$d_6$) $\delta$ 14.59 (s, 1H), 9.93 (s, 1H), 8.44 (d, *J* = 5.1 Hz, 1H), 8.13 (s, 1H), 7.77 - 7.62 (m, 3H), 7.42 (d, *J* = 8.0 Hz, 1H), 7.38 - 7.30 (m, 4H), 7.30 - 7.18 (m, 2H), 6.69 (d, *J* = 16.0 Hz, 1H), 4.18 (s, 2H), 3.72 (s, 3H), 2.29 (s, 3H). MS: 454 [M+H]$^+$.

Example 481: Methyl 3-(3-(2-(5-(cyclopentylmethyl)-4H-1,2,4-triazol-3-carboxamido)pyridin-4-yl)-4-methylphenyl)propionate

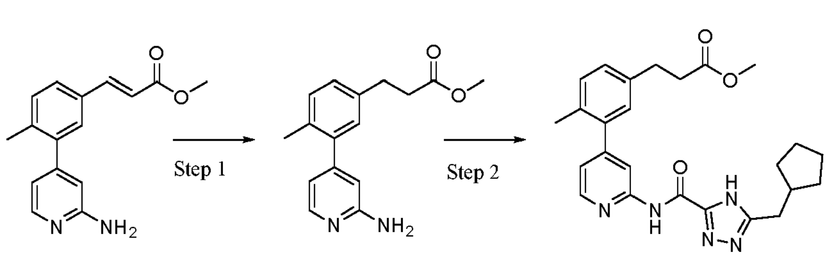


It was carried out in a similar manner to steps 2 to 3 of Example 47, except that methyl (E)-3-(3-(2-aminopyridin-4-yl)-4-methylphenyl)acrylate (see Example 480 for the synthetic method) was used in place of 4-(cyclopent-1-en-1-yl)pyridin-2-amine, and ethyl 5-(cyclopentylmethyl)-4H-1,2,4-triazol-3-carboxylate was used in place of ethyl 5-benzyl-4H-1,2,4-triazol-3-carboxylate.

$^1$H NMR (400 MHz, DMSO-$d_6$) $\delta$ 14.43 (s, 1H), 9.86 (s, 1H), 8.42 (d, *J* = 5.1 Hz, 1H), 8.13 (d, *J* = 1.4 Hz, 1H),

7.35 - 7.17 (m, 3H), 7.14 (d, *J* = 1.8 Hz, 1H), 3.58 (s, 3H), 2.87 (t, *J* = 7.6 Hz, 2H), 2.82 - 2.72 (m, 2H), 2.66 (t, *J* = 7.6 Hz, 2H), 2.35 - 2.25 (m, 1H), 2.24 (s, 3H), 1.79 - 1.67 (m, 2H), 1.67 - 1.56 (m, 2H), 1.56 - 1.48 (m, 2H), 1.26 - 1.21 (m, 2H). MS: 448 $[M+H]^+$.

Example 482: 5-benzyl-N-(4-(5-(3-hydroxyl-3-methylbutyl)-2-methylphenyl)pyridin-2-yl)-4H-1,2,4-triazole-3-carboxamide

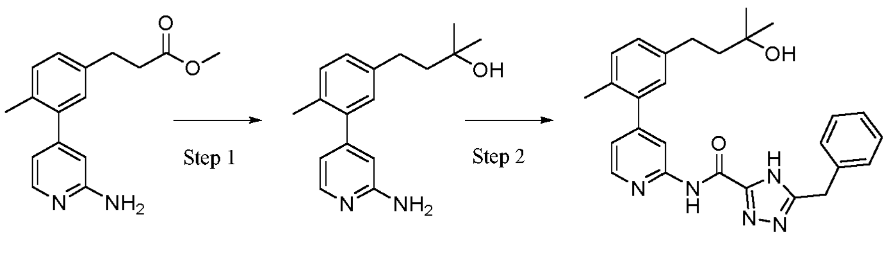


Step 1: The preparation was carried out in a similar manner to step 2 of Example 160, except that methyl 3-(3-(2-aminopyridin-4-yl)-4-methylphenyl)propionate was used in place of methyl 4-(3-bromo-4-methylphenoxy)butanoate.

Step 2: The preparation was carried out in a similar manner to step 3 of Example 100, except that 4-(3-(2-aminopyridin-4-yl)-4-methylphenyl)-2-methylbutan-2-ol was used in place of 4-(2-methyl-5-(octyloxy)phenyl)pyridin-2-amine.

$^1H$ NMR (400 MHz, DMSO-$d_6$) δ 14.58 (s, 1H), 9.90 (s, 1H), 8.41 (d, *J* = 5.1 Hz, 1H), 8.11 (d, *J* = 1.4 Hz, 1H), 7.39 - 7.30 (m, 4H), 7.29 - 7.23 (m, 2H), 7.23 - 7.16 (m, 2H), 7.09 (d, *J* = 1.8 Hz, 1H), 4.24 (s, 1H), 4.18 (s, 2H), 2.70 - 2.59 (m, 2H), 2.24 (s, 3H), 1.71 - 1.60 (m, 2H), 1.13 (s, 6H). MS: 456 $[M+H]^+$.

Example 483: 5-benzyl-N-(4-(5-(3-hydoxylpropyl)-2-methylphenyl)pyridin-2-yl)-4H-1,2,4-triazole-3-carboxamide

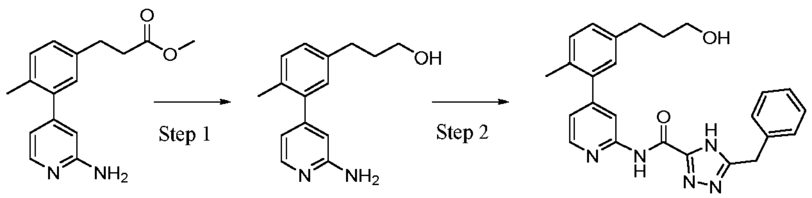


Step 1: Synthesis of 3-(3-(2-aminopyridin-4-yl)-4-methylphenyl)propanol

Methyl 3-(3-(2-aminopyridin-4-yl)-4-methylphenyl)propionate (270mg, 1mmol) was placed in anhydrous tetrahydrofuran (3mL), and lithium aluminum hydride (115mg, 3mmol) was added in batches under argon protection at 0°C. After reacting at 50°C for 2 hours, the reaction solution was quenched with saturated ammonium chloride, diluted with dichloromethane, washed with saturated brine, dried over anhydrous sodium sulfate, filtered and evaporated to dryness, and purified by column chromatography to afford 150 mg of product.

Step 2: It was carried out in a similar manner to step 2 of Example 1, except that 3-(3-(2-aminopyridin-4-yl)-4-methylphenyl)propanol was used in place of 4-phenylpyridin-2-amine.

$^1H$ NMR (400 MHz, DMSO-$d_6$) δ 14.47 (s, 1H), 9.90 (s, 1H), 8.41 (d, *J* = 5.1 Hz, 1H), 8.15 - 8.08 (m, 1H), 7.40 - 7.30 (m, 4H), 7.30 - 7.23 (m, 2H), 7.23 - 7.13 (m, 2H), 7.09 (d, *J* = 1.8 Hz, 1H), 4.67 - 4.34 (m, 1H), 4.17 (s, 2H), 3.41 (t, *J* = 6.4 Hz, 2H), 2.63 (t, *J* = 7.7 Hz, 2H), 2.24 (s, 3H), 1.80 - 1.66 (m, 2H). MS: 428 $[M+H]^+$.

Example 484: 5-benzyl-N-(4-(5-(1-hydroxyl-4-methoxybutyl)-2-methylphenyl)pyridin-2-yl)-4H-1,2,4-triazole-3-carboxamide

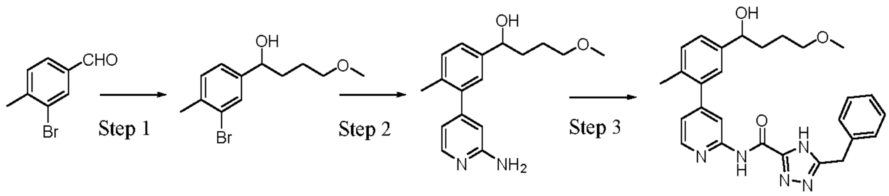


It was carried out in a similar manner to steps 2 to 4 of Example 160, except that 3-bromo-4-methylbenzaldehyde was used in place of methyl 4-(3-bromo-4-methylphenoxy)butanoate, and (3-methoxypropyl)magnesium bromide was used in place of methylmagnesium bromide. $^1$H NMR (400 MHz, DMSO-$d_6$) δ 14.48 (s, 1H), 9.90 (s, 1H), 8.42 (d, *J* = 5.1 Hz, 1H), 8.12 (s, 1H), 7.38-7.22 (m, 7H), 7.22-7.17 (m, 2H), 5.19 (d, *J* = 4.5 Hz, 1H), 4.67-4.43 (m, 1H), 4.18 (s, 2H), 3.29 (t, *J* = 6.3 Hz, 2H), 3.18 (s, 3H), 2.26 (s, 3H), 1.69-1.52 (m, 3H), 1.52-1.39 (m, 1H). MS: 472 [M+H]$^+$.

Example 485: 5-benzyl-N-(4-(2-methyl-5-(2-(tetrahydro-2H-pyran-4-yl)acetyl)phenyl)pyridin-2-yl)-4H-1,2,4-triazole-3-carboxamide

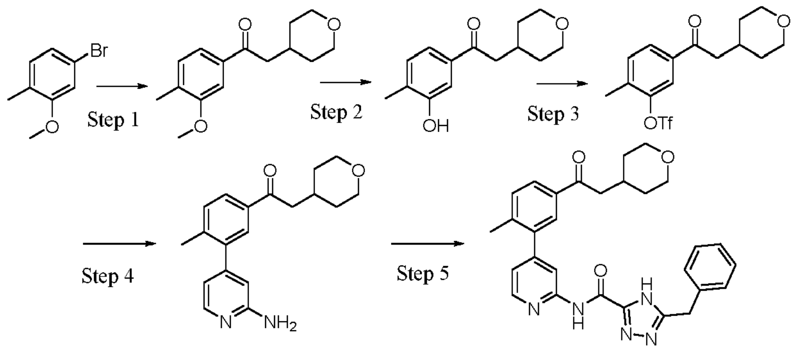


Step 1: Synthesis of 1-(3-methoxy-4-methylphenyl)-2-(tetrahydro-2H-pyran-4-yl)ethan-1-one

4-Bromo-2-methoxy-1-methylbenzene (500mg, 2.5mmol) was dissolved in anhydrous tetrahydrofuran (5mL), and n-butyl lithium (2.5M, 1mL, 2.5mmol) was added dropwise at -78°C under argon protection. After reacting at this temperature for 30 minutes, N-methoxy-N-methyl-2-(tetrahydro-2H-pyran-4-yl)acetamide (505mg, 2.7mmol) in anhydrous tetrahydrofuran (1mL) was added, warmed slowly, and reacted at room temperature for 2 hours. The reaction solution was quenched with saturated aqueous solution of ammonium chloride, extracted with ethyl acetate, the organic phase was washed with saturated brine, dried over anhydrous sodium sulfate, filtered and evaporated to dryness, and purified by column chromatography to afford 500 mg of product.

Step 2: Synthesis of 1-(3-hydroxyl-4-methylphenyl)-2-(tetrahydro-2H-pyran-4-yl)ethan-1-one

1-(3-Methoxy-4-methylphenyl)-2-(tetrahydro-2H-pyran-4-yl)ethan-1-one (500mg, 2.0mmol) was dissolved in anhydrous dichloromethane (5mL), a solution of boron tribromide in dichloromethane (1M, 3mL, 3mmol) was added dropwise at 0°C under argon protection, and the reaction was slowly warmed to room temperature and reacted for 2 hours. The reaction solution was quenched with saturated aqueous solution of ammonium chloride, extracted with dichloromethane, and the organic phase was washed with saturated brine, dried over anhydrous sodium sulfate, filtered and evaporated to dryness, and purified by column chromatography to afford 350mg of product.

Steps 3 to 5 were carried out in a similar manner to Example 43, except that 1-(3-hydroxyl-4-methylphenyl)-2-(tetrahydro-2H-pyran-4-yl)ethan-1-one was used in place of 5,6,7,8-tetrahydronaphthalen-1-ol.

$^1$H NMR (400 MHz, DMSO-$d_6$) δ 14.60 (s, 1H), 9.96 (s, 1H), 8.46 (d, *J* = 5.1 Hz, 1H), 8.14 (s, 1H), 7.96 (d, *J* = 1.7 Hz, 1H), 7.91 (dd, *J* = 7.9, 1.8 Hz, 1H), 7.43 (d, *J* = 7.9 Hz, 1H), 7.39-7.29 (m, 4H), 7.29-7.21 (m, 2H), 4.18 (s, 2H), 3.88-3.75 (m, 2H), 3.32-3.25 (m, 2H), 3.00 (d, *J* = 6.7 Hz, 2H), 2.35 (s, 3H), 2.20-2.05 (m, 1H), 1.66-1.54 (m, 2H), 1.34-1.22 (m, 2H). MS: 496 [M+H]$^+$.

Example 486: 5-(cyclopentylmethyl)-N-(4-(2-methyl-5-(2-(tetrahydro-2H-pyran-4-yl)acetyl)phenyl)pyridin-2-yl)-4H-1,2,4-triazole-3-carboxamide

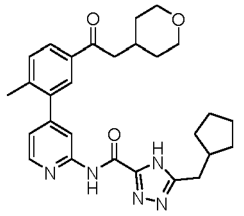

It was carried out in a similar manner to Example 485, except that in step 5, ethyl 5-(cyclopentylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.42 (s, 1H), 9.91 (s, 1H), 8.47 (d, *J* = 5.1 Hz, 1H), 8.16 (d, *J* = 1.4 Hz, 1H), 7.97 (d, *J* = 1.7 Hz, 1H), 7.92 (dd, *J* = 8.0, 1.8 Hz, 1H), 7.43 (d, *J* = 7.9 Hz, 1H), 7.25 (dd, *J* = 5.1, 1.6 Hz, 1H), 3.89 - 3.76 (m, 2H), 3.32 - 3.24 (m, 2H), 3.00 (d, *J* = 6.7 Hz, 2H), 2.78 (d, *J* = 7.5 Hz, 2H), 2.35 (s, 3H), 2.31 - 2.22 (m, 1H), 2.18 - 2.05 (m, 1H), 1.80 - 1.67 (m, 2H), 1.67 - 1.56 (m, 4H), 1.56 - 1.44 (m, 2H), 1.32 - 1.21 (m, 4H). MS: 488 [M+H]$^+$.

Example 487: 5-benzyl-N-(4-(5-((3-hydroxyl-3-methylbutyl)carbamoyl)-2-methylphenyl)pyridin-2-yl)-4 H-1,2,4-triazole-3 -carboxamide

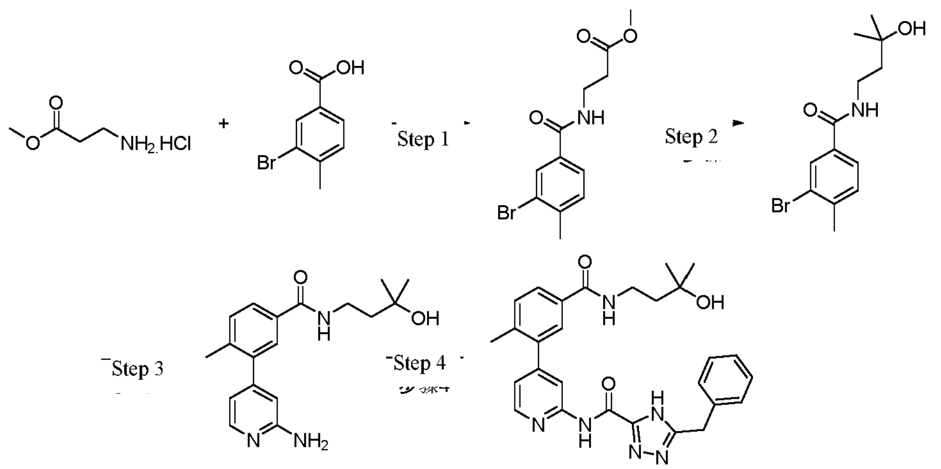


Step 1: Synthesis of methyl 3-(3-bromo-4-methylbenzamido)propionate

3-Bromo-4-methylbenzoic acid (430mg, 2mmol), methyl 3-aminopropionate hydrochloride (280mg, 2mmol), T3P (2,4,6-tripropyl-l,3,5,2,4,6-trioxatriphosphorinane-2,4,6-trioxide) (950mg, 50%, 3mmol) and DIEA (780mg, 6mmol) were dissolved in dichloromethane (6mL), and reacted at 25°C for 2 hours. The reaction solution was diluted with dichloromethane, washed with saturated brine, dried over anhydrous sodium sulfate, filtered and evaporated to dryness, purified by column chromatography to afford 540mg of product.

Steps 2 to 4 were carried out in a similar manner to steps 2 to 4 of Example 160, except that methyl 3-(3-bromo-4-methylbenzamido)propionate was used in place of methyl 4-(3-bromo-4-methylphenoxy)butanoate.

$^1$H NMR (400 MHz, DMSO-$d_6$) δ 14.54 (s, 1H), 9.94 (s, 1H), 8.50 - 8.38 (m, 2H), 8.14 (s, 1H), 7.82 (dd, *J* = 8.0, 1.9 Hz, 1H), 7.75 (d, *J* = 2.0 Hz, 1H), 7.45 (d, *J* = 8.0 Hz, 1H), 7.39 - 7.29 (m, 4H), 7.29 - 7.17 (m, 2H), 4.33 (s, 1H), 4.18 (s, 2H), 3.38 - 3.34 (m, 2H), 2.32 (s, 3H), 1.71 - 1.58 (m, 2H), 1.12 (s, 6H). MS: 499 [M+H]$^+$.

Example 488: 5-(cyclopentylmethyl)-N-(4-(5-((3-hydroxyl-3-methylbutyl)carbamoyl)-2-methylphenyl)pyridin-2-yl)-4H-1,2,4-triazole-3-carboxamide

The preparation was carried out in a similar manner to Example 487, except that in step 4, ethyl 5-(cyclopentylmethyl)-4H-1,2,4-triazole-3-carboxylate was used in place of ethyl 5-benzyl-4H-1,2,4-triazol-3-carboxylate. $^1H$ NMR (400 MHz, DMSO-$d_6$) δ 14.34 (s, 1H), 9.89 (s, 1H), 8.55 - 8.34 (m, 2H), 8.16 (s, 1H), 7.82 (dd, *J* = 8.0, 1.9 Hz, 1H), 7.75 (d, *J* = 1.9 Hz, 1H), 7.45 (d, *J* = 8.0 Hz, 1H), 7.26 (dd, *J* = 5.1, 1.6 Hz, 1H), 4.33 (s, 1H), 3.38 - 3.35 (m, 2H), 2.78 (d, *J* = 7.5 Hz, 2H), 2.32 (s, 3H), 2.30 - 2.19 (m, 1H), 1.83 - 1.68 (m, 2H), 1.68 - 1.56 (m, 4H), 1.56 - 1.41 (m, 2H), 1.26 - 1.20 (m, 2H), 1.13 (s, 6H). MS: 491 $[M+H]^+$.

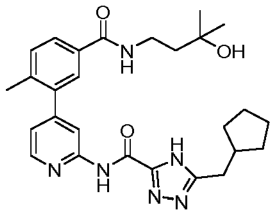

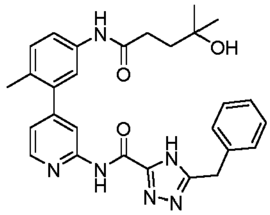

Example 488 Example 489

Example 489: 5-benzyl-N-(4-(5-(4-hydroxy-4-methylvaleramido)-2-methylphenyl)pyridin-2-yl)-4H-1,2,4-triazole-3-carboxamide

The preparation was carried out in a similar manner to Example 487, except that in step 1, monomethyl succinate was used in place of 3-bromo-4-methylbenzoic acid, and 3-bromo-4-methylaniline was used in place of methyl 3-aminopropionate hydrochloride. $^1H$ NMR (400 MHz, DMSO-$d_6$) δ 14.56 (s, 1H), 9.97 (s, 1H), 9.86 (s, 1H), 8.42 (d, *J* = 5.3 Hz, 1H), 8.12 (s, 1H), 7.56 (d, *J* = 8.1 Hz, 2H), 7.40 - 7.30 (m, 4H), 7.30 - 7.22 (m, 2H), 7.22 - 7.15 (m, 1H), 4.25 (s, 1H), 4.19 (s, 2H), 2.41 - 2.30 (m, 2H), 2.21 (s, 3H), 1.73 - 1.61 (m, 2H), 1.10 (s, 6H). MS: 499 $[M+H]^+$.

Example 490: 5-(cyclopentylmethyl)-N-(4-(5-(4-hydroxy-4-methylvaleramido)-2-methylphenyl)pyridin-2-yl)-4H-1,2,4-triazole-3-carboxamide

The preparation was carried out in a similar manner to Example 487, except that in step 1, monomethyl succinate was used in place of 3-bromo-4-methyl benzoic acid, 3-bromo-4-methylaniline was used in place of methyl 3-aminopropionate hydrochloride, and in step 4, ethyl 5-(cyclopentylmethyl)-4H-1,2,4-triazole-3-carboxylate was used in place of ethyl 5-benzyl-4H-1,2,4-triazol-3-carboxylate. $^1H$ NMR (400 MHz, DMSO-$d_6$) δ 14.37 (s, 1H), 9.98 (s, 1H), 9.83 (s, 1H), 8.42 (d, *J* = 5.2 Hz, 1H), 8.14 (s, 1H), 7.63 - 7.51 (m, 2H), 7.26 (d, J = 8.2 Hz, 1H), 7.19 (d, *J* = 5.2 Hz, 1H), 4.25 (s, 1H), 2.79 (d, *J* = 7.5 Hz, 2H), 2.41 - 2.32 (m, 2H), 2.32 - 2.24 (m, 1H), 2.22 (s, 3H), 1.79 - 1.44 (m, 8H), 1.25 - 1.19 (m, 2H), 1.10 (s, 6H). MS: 491 $[M+H]^+$.

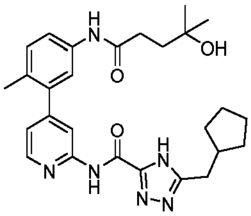

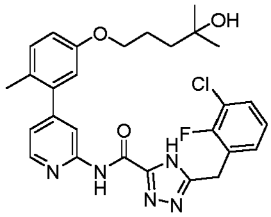

Example 490 Example 491

Example 491: 5-(3-chloro-2-fluorobenzyl)-N-(4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 4, ethyl 5-(3-chloro-2-fluorobenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.73 (s, 1H), 9.92 (s, 1H), 8.41 (d, *J* = 5.0 Hz, 1H), 8.10 (s, 1H), 7.57 - 7.48 (m, 1H), 7.43 - 7.35 (m, 1H), 7.28 - 7.18 (m, 3H), 6.93 (dd, *J* = 8.4, 2.7 Hz, 1H), 6.80 (d, *J* = 2.7 Hz, 1H), 4.26 (s, 2H), 4.17 (s, 1H), 3.96 (t, *J* = 6.6 Hz, 2H), 2.19 (s, 3H), 1.82 - 1.65 (m, 2H), 1.54 - 1.42 (m, 2H), 1.09 (s, 6H). MS: 538 $[M+H]^+$.

Example 492: N-(5-fluoro-4-(5-((4-hydroxy-4-methylpentyl)oxy)-2-methylphenyl)pyridin-2-yl)-5-(2-fluorobenzyl)-4H-1,2,4-triazole-3-carboxamide

The preparation was carried out in a similar manner to Example 160, except that in step 3, 5-fluoro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.70 (s, 1H), 10.09 (s, 1H), 8.47 (s, 1H), 8.06 (d, *J* = 5.6 Hz, 1H), 7.46 - 7.30 (m, 2H), 7.30 - 7.14 (m, 3H), 6.97 (dd, *J* = 8.4, 2.7 Hz, 1H), 6.84 (d, *J* = 2.7 Hz, 1H), 4.20 (s, 2H), 4.17 (s, 1H), 3.95 (t, *J* = 6.6 Hz, 2H), 2.09 (s, 3H), 1.81 - 1.67 (m, 2H), 1.52 - 1.41 (m, 2H), 1.09 (s, 6H). MS: 522 [M+H]$^+$.

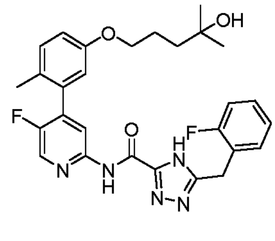

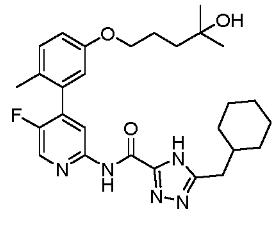

Example 492 Example 493

Example 493: 5-(cyclohexylmethyl)-N-(5-fluoro-4-(5-((4-hydroxy-4-methylpentyl)oxy)-2-methylphenyl)pyridin-2-yl)-4H-1,2,4-triazole-3-carboxamide

The preparation was carried out in a similar manner to Example 160, except that in step 3, 5-fluoro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.36 (s, 1H), 10.00 (s, 1H), 8.48 (s, 1H), 8.09 (d, *J* = 5.5 Hz, 1H), 7.27 (d, *J* = 8.4 Hz, 1H), 6.97 (dd, *J* = 8.4, 2.7 Hz, 1H), 6.84 (d, *J* = 2.7 Hz, 1H), 4.17 (s, 1H), 3.96 (t, *J* = 6.6 Hz, 2H), 2.66 *(d, J* = 7.0 Hz, 2H), 2.10 (s, 3H), 1.82 - 1.57 (m, 8H), 1.52 - 1.41 (m, 2H), 1.24 - 1.13 (m, 3H), 1.09 (s, 6H), 1.04 - 0.91 (m, 2H). MS: 510 [M+H]$^+$.

Example 494: N-(5-fluoro-4-(5-((5-hydroxyl-5-methylhexyl)oxy)-2-methylphenyl)pyridin-2-yl)-5-(2-fluorobenzyl)-4H-1,2,4-triazole-3-carboxamide

The preparation was carried out in a similar manner to Example 160, except that in step 1, methyl 5-bromopentanoate was used in place of methyl 4-bromobutanoate, in step 3, 5-fluoro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.58 (s, 1H), 10.02 (s, 1H), 8.47 (s, 1H), 8.06 (d, *J*= 5.5 Hz, 1H), 7.46 - 7.30 (m, 2H), 7.30 - 7.15 (m, 3H), 6.97 (dd, *J* = 8.5, 2.7 Hz, 1H), 6.85 (d, *J* = 2.7 Hz, 1H), 4.21 (s, 2H), 4.09 (s, 1H), 3.96 (t, *J* = 6.5 Hz, 2H), 2.09 (s, 3H), 1.68 (t, *J* = 6.8 Hz, 2H), 1.52 - 1.30 (m, 4H), 1.06 (s, 6H). MS: 536 [M+H]$^+$.

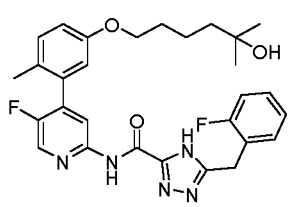

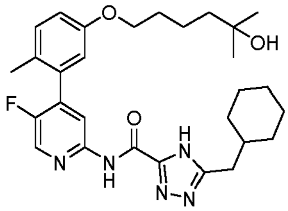

Example 494 Example 495

Example 495: 5-(cyclohexylmethyl)-N-(5-fluoro-4-(5-((5-hydroxyl-5-methylhexyl)oxy)-2-methylphenyl)pyridin-2-yl)-4H-1,2,4-triazole-3-carboxamide

The preparation was carried out in a similar manner to Example 160, except that in step 1, methyl 5-bromopentanoate was used in place of methyl 4-bromobutanoate, in step 3, 5-fluoro-4-bromopyridin-2-amine was used in

place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.34 (s, 1H), 9.99 (s, 1H), 8.48 (s, 1H), 8.09 (d, $J$ = 5.5 Hz, 1H), 7.27 (d, $J$ = 8.5 Hz, 1H), 6.98 (dd, $J$ = 8.4, 2.7 Hz, 1H), 6.85 (d, $J$ = 2.7 Hz, 1H), 4.09 (s, 1H), 3.97 (t, $J$ = 6.5 Hz, 2H), 2.67 (d, $J$ = 6.9 Hz, 2H), 2.10 (s, 3H), 1.79 - 1.57 (m, 8H), 1.47 - 1.39 (m, 3H), 1.24 - 1.10 (m, 4H), 1.06 (s, 6H), 1.02 - 0.90 (m, 2H). MS: 524 [M+H]$^+$.

Example 496: N-(5-fluoro-4-(2-methyl-5-((tetrahydro-2H-pyran-4-yl)methoxy)phenyl)pyridin-2-yl)-5-(2-fluorobenzyl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 5-fluoro-4-bromopyridin-2-amine was used in place of 4-bromopyridin-2-amine, and in step 3, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.59 (s, 1H), 10.02 (s, 1H), 8.47 (s, 1H), 8.06 (d, $J$ = 5.4 Hz, 1H), 7.44 - 7.29 (m, 2H), 7.29 - 7.14 (m, 3H), 6.98 (dd, $J$ = 8.4, 2.7 Hz, 1H), 6.86 (d, $J$ = 2.7 Hz, 1H), 4.21 (s, 2H), 3.91 - 3.79 (m, 4H), 3.33 - 3.23 (m, 2H), 2.09 (s, 3H), 2.04 - 1.90 (m, 1H), 1.72 - 1.61 (m, 2H), 1.39 - 1.24 (m, 2H). MS: 520 [M+H]$^+$.

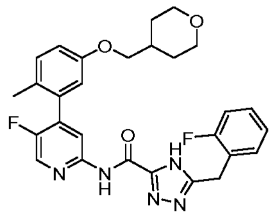
Example 496

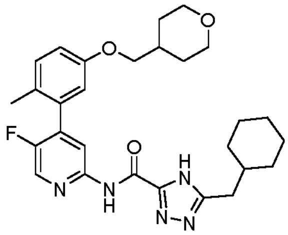
Example 497

Example 497: 5-(cyclohexylmethyl)-N-(5-fluoro-4-(2-methyl-5-((tetrahydro-2H-pyran-4-yl)methoxy)phenyl)pyridin-2-yl)-4H-1,2,4-triazole-3-carboxamide

The preparation was carried out in a similar manner to Example 64, except that in step 1, (tetrahydro-2H-pyran-4-yl)methanol was used in place of 2-(tert-butoxy)ethan-1-ol, in step 2, 5-fluoro-4-bromopyridin-2-amine was used in place of 4-bromopyridin-2-amine, and in step 3, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 10.00 (s, 1H), 8.48 (s, 1H), 8.09 (d, $J$ = 5.5 Hz, 1H), 7.28 (d, $J$ = 8.5 Hz, 1H), 6.99 (dd, $J$ = 8.4, 2.7 Hz, 1H), 6.86 (d, $J$ = 2.7 Hz, 1H), 3.92 - 3.78 (m, 4H), 3.32 - 3.24 (m, 2H), 2.66 (d, $J$ = 7.1 Hz, 2H), 2.10 (s, 3H), 2.04 - 1.91 (m, 1H), 1.81 - 1.55 (m, 8H), 1.36 - 1.15 (m, 5H), 1.04 - 0.93 (m, 2H). MS: 508 [M+H]$^+$.

Example 498: N-(5-fluoro-4-(2-methyl-5-(2-(tetrahydro-2H-pyran-4-yl)ethoxy)phenyl)pyridin-2-yl)-5-(2-fluorobenzyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-fluoro-4-bromopyridin-2-amine was used in place of 4-bromopyridin-2-amine, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.66 (s, 1H), 10.09 (s, 1H), 8.47 (s, 1H), 8.06 (d, $J$ = 5.6 Hz, 1H), 7.46 - 7.30 (m, 2H), 7.30 - 7.14 (m, 3H), 6.98 (dd, $J$ = 8.5, 2.7 Hz, 1H), 6.86 (d, $J$ = 2.7 Hz, 1H), 4.20 (s, 2H), 4.01 (t, $J$ = 6.3 Hz, 2H), 3.81 (dd, $J$ = 11.6, 4.2 Hz, 2H), 3.29 - 3.21 (m, 2H), 2.09 (s, 3H), 1.76 - 1.52 (m, 5H), 1.25 - 1.12 (m, 2H). MS: 534 [M+H]$^+$.

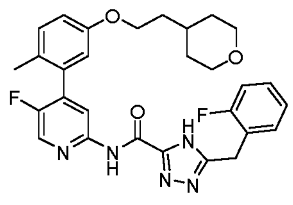 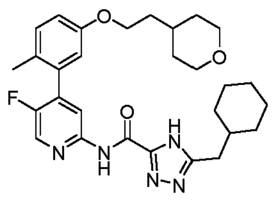

Example 498 Example 499

Example 499: N-(5-fluoro-4-(2-methyl-5-(2-(tetrahydro-2H-pyran-4-yl)ethoxy)phenyl)pyridin-2-yl)-5-(cyclohexylmethyl)-4H-1,2,4-triazole-3-carboxamide

The preparation was carried out in a similar manner to Example 85, except that in step 1, 2-(tetrahydro-2H-pyran-4-yl)ethan-1-ol was used in place of 2-(2-methoxyethoxy)ethan-1-ol, in step 3, 5-fluoro-4-bromopyridin-2-amine was used in place of 4-bromopyridin-2-amine, and in step 4, ethyl 5-(cyclohexylmethyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.35 (s, 1H), 9.99 (s, 1H), 8.47 (s, 1H), 8.09 (d, *J* = 5.6 Hz, 1H), 7.27 (d, *J* = 8.5 Hz, 1H), 6.98 (dd, *J* = 8.4, 2.7 Hz, 1H), 6.87 (d, *J* = 2.7 Hz, 1H), 4.02 (t, *J* = 6.3 Hz, 2H), 3.89 - 3.76 (m, 2H), 3.30 - 3.20 (m, 2H), 2.66 (d, *J* = 7.1 Hz, 2H), 2.10 (s, 3H), 1.80 - 1.55 (m, 11H), 1.30 - 1.13 (m, 5H), 1.04 - 0.92 (m, 2H). MS: 522 $[M+H]^+$.

Example 500: N-(4-(2-fhioro-5-((5-hydroxyl-5-methylhexyl)oxy)phenyl)pyridin-2-yl)-5-(2-fluorobenzyl)-4H-1,2,4-triazole-3-carboxamide

The preparation was carried out in a similar manner to Example 160, except that in step 1, 3-bromo-4-fluorophenol was used in place of 3-bromo-4-methylphenol, methyl 5-bromopentanoate was used in place of methyl 4-bromobutanoate, and in step 4, ethyl 5-(2-fluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.60 (s, 1H), 9.85 (s, 1H), 8.45 (d, *J* = 5.2 Hz, 1H), 8.35 (s, 1H), 7.51 - 7.15 (m, 6H), 7.15 - 7.00 (m, 2H), 4.23 (s, 2H), 4.11 (s, 1H), 4.02 (t, *J* = 6.4 Hz, 2H), 1.75 - 1.64 (m, 2H), 1.52 - 1.37 (m, 4H), 1.07 (s, 6H). MS: 522 $[M+H]^+$.

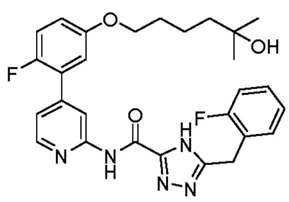 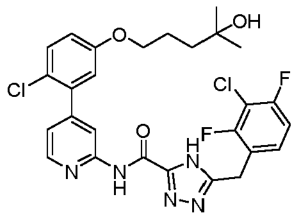

Example 500 Example 501

Example 501: 5-(3-chloro-2,4-difluorobenzyl)-N-(4-(2-chloro-5-((4-hydroxy-4-methylpentyl)oxy)phenyl)pyridin-2-yl)-4H-1,2,4-triazole-3 -carboxamide

The preparation was carried out in a similar manner to Example 160, except that in step 1, 3-bromo-4-chlorophenol was used in place of 3-bromo-4-methylphenol, and in step 4, ethyl 5-(3-chloro-2,4-difluorobenzyl)-4H-1,2,4-triazol-3-carboxylate was used in place of ethyl 5-benzyl-4H-1,2,4-triazol-3-carboxylate. $^1$H NMR (400 MHz, DMSO-$d_6$) δ 14.72 (s, 1H), 9.98 (s, 1H), 8.45 (d, *J* = 5.1 Hz, 1H), 8.21 (s, 1H), 7.57 - 7.44 (m, 2H), 7.38 - 7.25 (m, 2H), 7.06 (dd, *J* = 8.9, 3.0 Hz, 1H), 7.01 (d, *J* = 3.0 Hz, 1H), 4.26 (s, 2H), 4.18 (s, 1H), 4.01 (t, *J* = 6.5 Hz, 2H), 1.82 - 1.69 (m, 2H), 1.53 - 1.40 (m, 2H), 1.09 (s, 6H). MS: 576 $[M+H]^+$.

Example 502: N-(4-(2-chloro-5-((5-hydroxyl-5-methylhexyl)oxy)phenyl)-5-fluoropyridin-2-yl)-5-(cyclohexylmethyl)-4H-1,2,4-triazole-3-carboxamide

The preparation was carried out in a similar manner to Example 160, except that in step 1, 3-bromo-4-chlorophenol was used in place of 3-bromo-4-methylphenol, methyl 5-bromopentanoate was used in place of methyl 4-bromobutanoate, in step 3, 4-bromo-5-fluoropyridin-2-amine was used in place of 4-bromopyridin-2-amine, and in step

4, ethyl 5-(cyclohexylmethyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.35 (s, 1H), 10.04 (s, 1H), 8.52 (d, $J$ = 1.2 Hz, 1H), 8.16 (d, $J$ = 5.5 Hz, 1H), 7.53 (d, $J$ = 8.6 Hz, 1H), 7.17 - 7.05 (m, 2H), 4.09 (s, 1H), 4.02 (t, $J$ = 6.5 Hz, 2H), 2.67 (d, $J$ = 7.1 Hz, 2H), 1.82 - 1.57 (m, 8H), 1.51 - 1.33 (m, 4H), 1.28 - 1.10 (m, 3H), 1.06 (s, 6H), 1.04 - 0.88 (m, 2H). Ms: 544 [M+H]$^+$.

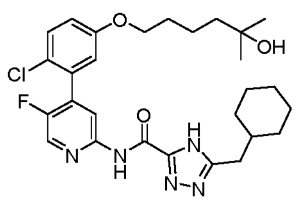

Example 502

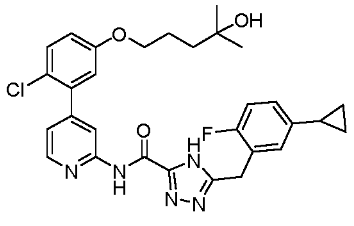

Example 503

Example 503: N-(4-(2-chloro-5-((4-hydroxy-4-methylpentyl)oxy)phenyl)pyridin-2-yl)-5-(5-cyclopropyl-2-fluorobenzyl)-4H-1,2,4-triazole-3-carboxamide

The preparation was carried out in a similar manner to Example 160, except that in step 1, 3-bromo-4-chlorophenol was used in place of 3-bromo-4-methylphenol, and in step 4, ethyl 5-(5-cyclopropyl-2-fluorobenzyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.56 (s, 1H), 9.89 (s, 1H), 8.45 (d, $J$ = 5.2 Hz, 1H), 8.22 (s, 1H), 7.51 (d, $J$ = 8.8 Hz, 1H), 7.28 (d, $J$ = 5.2 Hz, 1H), 7.17-6.95 (m, 5H), 4.22-4.11 (m, 3H), 4.01 (t, $J$ = 6.6 Hz, 2H), 1.95-1.85 (m, 1H), 1.82-1.69 (m, 2H), 1.53-1.41 (m, 2H), 1.09 (s, 6H), 0.97-0.88 (m, 2H), 0.68-0.57 (m, 2H). MS: 564 [M+H]$^+$.

Example 504: N-(4-(2-chloro-5-((4-hydroxy-4-methylpentyl)oxy)phenyl)pyridin-2-yl)-5-((4-fluoro-[1,1'-biphenyl]-3-yl)methyl)-4H-1,2,4-triazole-3-carboxamide

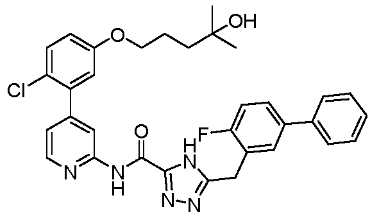

The preparation was carried out in a similar manner to Example 160, except that in step 1, 3-bromo-4-chlorophenol was used in place of 3-bromo-4-methylphenol, and in step 4, ethyl 5-((4-fluoro-[1,1'-biphenyl]-3-yl)methyl)-4H-1,2,4-triazol-3-carboxylate was used in place of ethyl 5-benzyl-4H-1,2,4-triazol-3-carboxylate. $^1H$ NMR (400 MHz, DMSO-$d_6$) δ 14.70 (s, 1H), 9.94 (s, 1H), 8.44 (d, $J$ = 5.2 Hz, 1H), 8.21 (s, 1H), 7.82 - 7.68 (m, 1H), 7.68 - 7.56 (m, 3H), 7.56 - 7.41 (m, 3H), 7.41 - 7.22 (m, 3H), 7.14 - 6.89 (m, 2H), 4.29 (s, 2H), 4.17 (s, 1H), 4.00 (t, $J$ = 6.6 Hz, 2H), 1.82 - 1.68 (m, 2H), 1.54 - 1.39 (m, 2H), 1.09 (s, 6H). MS: 600 [M+H]$^+$.

**Assay example 1. Western Blot Determination of Inhibition of Compounds on RIPK1 Phosphorylation in HT29 Cells**

Western blot was used to determine the effect of the test compounds on the phosphorylation of intracellular RIPK1 during the induction of programmed necrosis of HT29 cells, verifying the effect of compounds on RIPK1 phosphorylation, and initially estimating the $IC_{50}$ range of compounds for RIPK1 phosphorylation.

**1. Main assay reagents/instruments**

Table 2. Main reagents and instruments used in assay example 1

| Name | Item No. | Brand |
|---|---|---|
| McCoy's 5A | SH30200.01 | Hyclone |
| BCA Protein concentration determination kit | P0012 | Beyotime |
| HT29 cells | HTB-38 | ATCC |
| Phospho-RIP (Ser166) (D1L3S) Rabbit mAb | 65746s | CST |
| RIP ($D94C_{12}$) XP Rabbit mAb | 3493s | CST |
| Anti-rabbit IgG (H+L) (DyLight 680 Conjugate) | 5366s | CST |
| Anti-rabbit IgG (H+L) (DyLight 800 4X PEG Conjugate) | 5151s | CST |
| β-Actin(8H10D10)Mouse mAb | 3700s | CST |
| Precision Plus ProteinTM Dual Color Standards | 1610374 | Bio-Rad |
| 10%Mini-PROTEAN® TGX Stain-Free™ Protein Gels | 4568034 | Bio-Rad |
| AT-406 (SM-406) | S2754 | Selleck chem |
| Z-VAD-FMK | S7023 | Selleck chem |
| Human TNF-α | 300-01A-10 | Peprotech |
| Electrophoresis power supply PowerPacTM basic electrophoresis power supply | 1645050 | Bio-Rad |
| Mini-PROTEAN® Tetra electrophoresis tank | 1658004 | Bio-Rad |
| Trans-Blot® TurboTM Trans-Blot Turbo Transfer System | 1704155 | Bio-Rad |
| Odyssey CLXInfrared fluorescence scanning imaging system | CLX-1688 | LI-COR |

**II. Experiment procedure**

1. Cell collection and protein sample extraction

a) HT29 cells in good condition were collected, resuspended in McCoy's 5A complete medium and counted, the cells were planted in a 12-well plate at a cell density of $2\times10^6$/1 mL/well, and placed in a 37 °C cell culture incubator overnight.
b) Z-VAD-FMK (at final concentration of 20μM) was added to the above 12-well plate, after 30 minutes, the inducer (where the final concentration of TNF-α is 20ng/mL, and the final concentration of AT-406 is 100nM), and test compounds of different concentrations were then added, and incubated in a cell incubator at 37°C for 7 hours.
c) Preparation of complete lysate: A tube of aliquoted RIPA Lysis Buffer was taken, PMSF, Phosphatase Inhibitor, and Roche Protease Inhibitor were added after thawing (volume ratio: PMSF: RIPA=1:100; phosphatase inhibitor: RIPA=1:100; Roche protease inhibitor: RIPA=4:100), and placed on ice for later use.
d) The cell culture medium supernatant was discarded, washed twice with PBS, 100 μL of the prepared complete cell lysate was added to each well, the cells were lysed on ice for 15 minutes, and shaked intermittently during the lysis;
e) After the lysis is complete, the lysate was transferred to a 1.5 mL centrifuge tube labeled in advance and centrifuged at 4°C and 14000 rpm for 20 minutes;
f) The supernatant was taken, transferred to a labeled 600 μL centrifuge tube, and placed on ice.
g) The BCA kit was used to detect and the protein concentration was calculated;

2. The protein sample was mixed well with 4× loading buffer and heated in a metal bath at 100°C for 5 min to fully denature the protein and placed on ice to cool down.
3. 40-50 ug of protein sample to be determined or pre-stained two-color protein molecular weight standard were added to each well.
4. Electrophoresis was carried out at a voltage of 100 V under constant voltage conditions. After 30 minutes of electrophoresis, the voltage was increased to 120V, and the electrophoresis time was set to 120 minutes or appropriately extended. The electrophoresis could be stopped when the pre-stained two-color protein molecular weight standard band on the gel run out of the lower edge of the lane.
5. After the electrophoresis was over, the gel holder was removed, the short glass plate was gently pried up with

the peeling spatula, the upper layer of concentrated gel was dicarded, the separation gel was gently peeled off and placed in the transfer buffer;

6. A moderately sized NC membrane was cut according to the size of the gel, soaked with pure water, and then immersed in the pre-cooled transfer buffer; at the same time the filter paper was soaked in the transfer buffer solution.

7. The transfer cartridge was assembled in the order of filter paper, NC membrane, gel, and filter paper, then the transfer cartridge was put into the Trans-Blot® Turbo, and the "Standard SD" program that comes with the instrument was called to complete the transfer.

8. The protein-loaden NC membrane was rinsed with deionized water, blocking solution was added to immerse the membrane, and blocked with slow shaking on a shaker at room temperature for 1 hour.

9. After the blocking was completed, the NC membrane was put into the antibody incubation box, the primary antibody was diluted at a ratio of 1:1000, after adding to the antibody incubation box, the box was incubated on a shaker at room temperature for 1 hour.

10. After the incubation was completed, the membrane was washed with TBST for 3 times, 10 minutes each time.

11. The secondary antibody was selected according to the source of the primary antibody, the secondary antibody was diluted with the diluent of the secondary antibody at a ratio of 1:10000~1:15000, and added into the antibody incubation box and the membrane was immersed therein, and incubated on a shaker at room temperature for 1 hour.

12. After the incubation was completed, the membrane was washed with TBST for 3 times, with 10 minutes each time.

13. The membrane was rinsed once with pure water, Odyssey CLX infrared fluorescence scanning imaging system was used to scan the membrane to afford an image, and the fluorescence intensity value was read at the same time.

**III. Typical assay results and analysis**

The inhibition rate of compounds on protein phosphorylation was calculated according to the fluorescence intensity value, and the calculation method is as follows:

$$\text{Inhibiton rate (\%)} = (1\text{-}A/B) \times 100\%$$

A: Fluorescence intensity value after treatment with IL-2 and compound

B: Fluorescence intensity value after treatment with IL-2

The compound of Example 2 was tested by the above method, and the results were shown in Figure 1 and Figure 2. Figure 1 is a Western Blot picture of the inhibition of RIPK1 phosphorylation by the compound of different concentrations. Figure 2 is a quantitative calculation of the inhibition rate of RIPK1 phosphorylation based on the gray value of the band in the above picture. Therefore, it can be shown that the compound of Example 2 directly inhibit the phosphorylation activation of RIPK1.

**Assay example 2. Assay method for compounds inhibiting cell necrosis (CCK8 method)**

In this assay example 2, under the condition of inducing programmed cell necrosis, different concentrations of the test compounds were added to test the IC50 of the rescue effect of the test compounds on the programmed necrosis.

**I. Main assay reagents, instruments and materials**

Table 3. Main reagents and instruments used in Assay Example 2

| Name | Brand | Item No./Name |
|---|---|---|
| DMSO | Sigma | D2650 (100 mL) |
| Trypan blue | GENIA Stock | G8003 |
| McCOY's 5A | Hylcone | SH30200.01 |
| CCK-8 | Tongren Chemistry | CK04 |
| GSK2982772 | Selleckchem | S8484 |
| AT-406 | Selleckchem | S2754 |
| Z-VAD-FMK | Selleckchem | S7023 |

(continued)

| Name | Brand | Item No./Name |
|---|---|---|
| TNF-α | PeproTech | 300-01A |
| HT29 | ATCC | HTB-38 |
| Multi-function plate reader | PerkinElmer | Envision |

**Compounds:** Dissolved in DMSO to make a 10 mM solution;
**TNF-α:** The initial concentration was 100 μg/mL;
**Z-VAD-FMK:** Dissolved in DMSO to make a 10 mM solution;
**AT-406:** Dissolved in DMSO to make a 10 mM solution.

**II. Experimental steps**

1) Compound dilution: All test compounds were dissolved in DMSO to prepare a 10 mM stock solution, and the first concentration gradient dilution of the test compounds was completed in DMSO. The gradient dilution factor was 4 times and 9 concentration gradients were made; A 40-fold overall dilution of all compounds was completed in the cell culture medium, and the resulting compound was 10× compound, which will be added to the wells of the 96-well plate containing cells, so that the final cell culture medium was 1 × the designed final concentration.
The general drug design is 9 concentration gradients, of which the highest final concentration is 25000 nM, the lowest concentration after 4-fold dilution of 9 concentrations is 0.38 nM, and the final concentration of DMSO in all wells is 0.25%. The compound dilution process is shown in Figure 3.

2) HT 29 cells in good growth condition were selected, trypsinized, the cells was collected by centrifugation, resuspended in McCOY's 5A complete medium (containing 10% FBS) and counted, and the number of cells and survival rate were recorded.

3) Cells were inoculated to the 60 wells in the middle of the 96-well culture plate at a density of 12000 cells/ 80 μL/well, surrounding wells were filled with sterile water, and incubated overnight.

4) 10 μL of the 10× compound diluted with the medium was added to the assay wells, 10 μL of McCOY's 5A complete medium containing 2.5% DMSO was added to the inducer-free wells and the control wells, mixed and shaked, and incubated for 30 minutes.

5) Then 10 μL of inducer mixture (TNF-α, AT-406, Z-VAD-FMK with the final concentration of 20 ng/mL, 1 μM, 20 μM) was added into the assay wells and control wells. 10 μL McCOY's 5A complete medium was added to wells without inducer. Cells were cultured in an incubator with 5% $CO_2$, at 37°C.

6) After culturing for 16-20 hours, 10 μL of CCK-8 reagent was added to each well, and incubated for about 2 hours;

7) The OD value was read at 450 nm on the multi-function plate reader.

8) Data processing:

8) Data processing: Inhibition rate of necrosis (%) =100* (X-B) /(A-B)

A: The OD values of the wells without inducer (containing cells, CCK-8, without compound and inducer);
X: OD values of assay wells (containing cells, CCK-8, compound, inducer);
B: OD values of control wells (containing cells, CCK-8, inducer, without compound).

Then the value were imported into Graphpad PrismS software for curve fitting and the IC50 values were calculated.
Table 4 lists the determination results of some compounds of the present disclosure in inhibiting HT29 cell necrosis, wherein A represents $IC_{50}$ is less than or equal to 100 nM, B represents $IC_{50}$ is greater than 100 nM but less

than or equal to 1000 nM, C represents $IC_{50}$ is greater than 1000 nM but less than or equal to 10000 nM, D represents $IC_{50}$ is greater than 10000 nM.

Table 4. the determination results of some compounds of the present disclosure in inhibiting necrosis of HT29 cells

| Example No. | HT29 $IC_{50}$ (nM) | Example No. | HT29 $IC_{50}$ (nM) |
|---|---|---|---|
| 1 | C | 101 | B |
| 2 | B | 102 | B |
| 3 | C | 103 | B |
| 4 | C | 104 | C |
| 5 | D | 105 | D |
| 6 | C | 106 | C |
| 7 | B | 107 | C |
| 8 | C | 108 | C |
| 9 | D | 109 | C |
| 10 | C | 110 | D |
| 11 | C | 111 | A |
| 12 | B | 112 | C |
| 13 | C | 113 | D |
| 14 | C | 114 | B |
| 15 | C | 115 | B |
| 16 | C | 116 | B |
| 17 | D | 117 | A |
| 18 | C | 118 | A |
| 19 | C | 119 | A |
| 20 | B | 120 | B |
| 21 | C | 121 | A |
| 22 | C | 122 | A |
| 23 | D | 123 | A |
| 24 | B | 124 | C |
| 25 | C | 125 | B |
| 26 | B | 126 | C |
| 27 | C | 127 | NT |
| 28 | C | 128 | A |
| 29 | D | 129 | B |
| 30 | D | 130 | D |
| 31 | D | 131 | A |
| 32 | D | 132 | B |
| 33 | B | 133 | A |
| 34 | B | 134 | B |
| 35 | A | 135 | A |
| 36 | B | 136 | A |
| 37 | B | 137 | A |
| 38 | C | 138 | A |
| 39 | C | 139 | D |
| 40 | C | 140 | A |
| 41 | D | 141 | A |
| 42 | C | 142 | B |
| 43 | C | 143 | B |
| 44 | NT | 144 | A |
| 45 | B | 145 | A |
| 46 | C | 146 | C |
| 47 | D | 147 | A |

(continued)

| Example No. | HT29 $IC_{50}$ (nM) | Example No. | HT29 $IC_{50}$ (nM) |
|---|---|---|---|
| 48 | D | 148 | A |
| 49 | C | 149 | A |
| 50 | C | 150 | A |
| 51 | C | 151 | A |
| 52 | D | 152 | A |
| 53 | D | 153 | A |
| 54 | D | 154 | A |
| 55 | B | 155 | B |
| 56 | B | 156 | B |
| 57 | B | 157 | B |
| 58 | B | 158 | C |
| 59 | A | 159 | A |
| 60 | D | 160 | A |
| 61 | C | 161 | B |
| 62 | D | 162 | B |
| 63 | C | 163 | A |
| 64 | A | 164 | A |
| 65 | A | 165 | A |
| 66 | A | 166 | A |
| 67 | A | 167 | A |
| 68 | B | 168 | B |
| 69 | A | 169 | A |
| 70 | A | 170 | B |
| 71 | A | 171 | B |
| 72 | A | 172 | A |
| 73 | A | 173 | A |
| 74 | B | 174 | B |
| 75 | A | 175 | A |
| 76 | D | 176 | A |
| 77 | A | 177 | A |
| 78 | A | 178 | A |
| 79 | A | 179 | A |
| 80 | A | 180 | A |
| 81 | A | 181 | C |
| 82 | B | 182 | B |
| 83 | A | 183 | A |
| 84 | A | 184 | A |
| 85 | A | 185 | A |
| 86 | A | 186 | B |
| 87 | A | 187 | A |
| 88 | A | 188 | A |
| 89 | A | 189 | A |
| 90 | A | 190 | B |
| 91 | A | 191 | B |
| 92 | B | 192 | A |
| 93 | C | 193 | A |
| 94 | C | 194 | A |
| 95 | B | 195 | A |
| 96 | B | 196 | D |

(continued)

| Example No. | HT29 $IC_{50}$ (nM) | Example No. | HT29 $IC_{50}$ (nM) |
|---|---|---|---|
| 97 | B | 197 | D |
| 98 | B | 198 | B |
| 99 | B | 199 | C |
| 100 | B | 200 | A |
| 201 | A | 353 | A |
| 202 | A | 354 | B |
| 203 | A | 355 | B |
| 204 | A | 356 | A |
| 205 | B | 357 | A |
| 206 | B | 358 | D |
| 207 | B | 359 | A |
| 208 | B | 360 | A |
| 209 | B | 361 | A |
| 210 | B | 362 | A |
| 211 | B | 363 | A |
| 212 | B | 364 | A |
| 213 | C | 365 | A |
| 214 | A | 366 | A |
| 215 | A | 367 | A |
| 216 | A | 368 | A |
| 217 | B | 369 | A |
| 218 | A | 370 | A |
| 219 | A | 371 | A |
| 220 | A | 372 | A |
| 221 | B | 373 | A |
| 222 | A | 374 | A |
| 223 | A | 375 | A |
| 224 | A | 376 | A |
| 225 | A | 377 | A |
| 226 | A | 378 | A |
| 227 | A | 379 | B |
| 228 | B | 380 | A |
| 229 | B | 381 | A |
| 230 | B | 382 | A |
| 231 | B | 383 | A |
| 232 | B | 384 | A |
| 233 | C | 385 | A |
| 234 | A | 386 | A |
| 235 | A | 387 | A |
| 236 | A | 388 | A |
| 237 | A | 389 | A |
| 238 | A | 390 | A |
| 239 | A | 391 | A |
| 240 | B | 392 | A |
| 241 | B | 393 | A |
| 242 | A | 394 | A |
| 243 | A | 395 | A |
| 244 | A | 396 | A |
| 245 | A | 397 | A |

(continued)

| Example No. | HT29 $IC_{50}$ (nM) | Example No. | HT29 $IC_{50}$ (nM) |
|---|---|---|---|
| 246 | B | 398 | A |
| 247 | B | 399 | A |
| 248 | B | 400 | A |
| 249 | B | 401 | A |
| 250 | A | 402 | A |
| 251 | A | 403 | A |
| 252 | A | 404 | A |
| 253 | B | 405 | A |
| 254 | B | 406 | A |
| 255 | A | 407 | D |
| 256 | A | 408 | D |
| 257 | A | 409 | A |
| 258 | A | 410 | A |
| 259 | A | 411 | B |
| 260 | A | 412 | B |
| 261 | A | 413 | B |
| 262 | A | 414 | B |
| 263 | A | 415 | A |
| 264 | A | 416 | A |
| 265 | B | 417 | B |
| 266 | A | 418 | B |
| 267 | A | 419 | A |
| 268 | A | 420 | B |
| 269 | A | 421 | B |
| 270 | A | 422 | A |
| 271 | A | 423 | A |
| 272 | A | 424 | B |
| 273 | A | 425 | B |
| 274 | A | 426 | B |
| 275 | A | 427 | B |
| 276 | A | 428 | A |
| 277 | A | 429 | A |
| 278 | A | 430 | B |
| 279 | A | 431 | B |
| 280 | A | 432 | A |
| 281 | A | 433 | A |
| 282 | A | 434 | A |
| 283 | A | 435 | A |
| 284 | A | 436 | A |
| 285 | A | 437 | B |
| 286 | A | 438 | B |
| 287 | A | 439 | A |
| 288 | A | 440 | A |
| 289 | A | 441 | A |
| 290 | A | 442 | A |
| 291 | A | 443 | A |
| 292 | A | 444 | A |
| 293 | A | 445 | A |
| 294 | A | 446 | A |

(continued)

| Example No. | HT29 $IC_{50}$ (nM) | Example No. | HT29 $IC_{50}$ (nM) |
|---|---|---|---|
| 295 | A | 447 | A |
| 296 | A | 448 | A |
| 297 | A | 449 | B |
| 298 | A | 450 | A |
| 299 | A | 451 | A |
| 300 | A | 452 | A |
| 301 | A | 453 | A |
| 302 | A | 454 | A |
| 303 | A | 455 | A |
| 304 | A | 456 | A |
| 305 | A | 457 | A |
| 306 | A | 458 | A |
| 307 | A | 459 | A |
| 308 | A | 460 | A |
| 309 | A | 461 | A |
| 310 | A | 462 | A |
| 311 | B | 463 | A |
| 312 | B | 464 | A |
| 313 | B | 465 | A |
| 314 | B | 466 | A |
| 315 | B | 467 | A |
| 316 | B | 468 | A |
| 317 | A | 469 | A |
| 318 | A | 470 | A |
| 319 | A | 471 | A |
| 320 | B | 472 | A |
| 321 | B | 473 | A |
| 322 | B | 474 | B |
| 323 | B | 475 | A |
| 324 | B | 476 | B |
| 325 | B | 477 | C |
| 326 | B | 478 | B |
| 327 | A | 479 | B |
| 328 | A | 480 | A |
| 329 | B | 481 | B |
| 330 | B | 482 | A |
| 331 | C | 483 | B |
| 332 | C | 484 | A |
| 333 | B | 485 | B |
| 334 | B | 486 | A |
| 335 | C | 487 | B |
| 336 | C | 488 | A |
| 337 | A | 489 | B |
| 338 | A | 490 | A |
| 339 | B | 491 | B |
| 340 | B | 492 | A |
| 341 | B | 493 | A |
| 342 | B | 494 | A |
| 343 | C | 495 | A |

(continued)

| Example No. | HT29 $IC_{50}$ (nM) | Example No. | HT29 $IC_{50}$ (nM) |
|---|---|---|---|
| 344 | B | 496 | A |
| 345 | B | 497 | A |
| 346 | B | 498 | A |
| 347 | B | 499 | A |
| 348 | C | 500 | B |
| 349 | D | 501 | B |
| 350 | B | 502 | A |
| 351 | B | 503 | C |
| 352 | B | 504 | B |

The biological data provided by the present disclosure shows that the compounds of the present disclosure are beneficial for the treatment or prevention of diseases caused by abnormal RIP1 kinase. Therefore, the compounds of the present disclosure are beneficial to the treatment of diseases related to RIP1 including ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic myeloid leukemia, acute myeloid leukemia, non-Hodgkin's lymphoma, nasopharyngeal cancer, esophageal cancer, brain tumor, B-cell and T-cell lymphoma, lymphoma, multiple myeloma, biliary cancer and sarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behget's disease, interleukin-1 converzyme-related fever syndrome, chronic obstructive pulmonary disease, tumor necrosis factor receptor related periodic syndrome and periodontitis. The compounds of the present disclosure can be used as monotherapy or combination therapy, and can be used in combination with multiple compounds of the present disclosure or in combination with other drugs that are not included in the present disclosure.

## Claims

1. A compound of formula (I), or the pharmaceutically acceptable salts, the diastereomers, the enantiomers, or the solvates thereof,

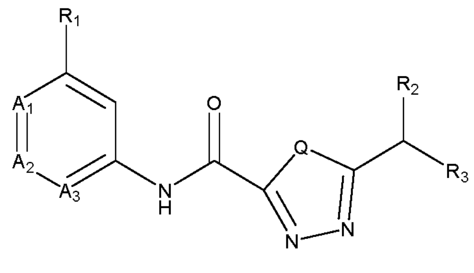

formula (I)

in formula (I),

Q is NH, O or S;

$A_1$, $A_2$, and $A_3$ are each independently selected from N or $CR_4$ and at least one of $A_1$, $A_2$, and $A_3$ is N, $R_4$ is H,

F, Cl or methyl;

$R_1$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,

or aryl or heteroaryl, which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$, -SO-$R_5$, -CO-$R_5$, -CONH-$R_5$, -NHCO-$R_5$, -R'-COO-R", -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl and/or $C_1$-$C_6$ alkoxy, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms, the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is substituted with 1 to 3 susbtituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

R' is $C_2$-$C_6$ alkenylene, or $C_1$-$C_6$ alkylene,

R" is hydrogen, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_2$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_7$-$C_{12}$ bridged cyclyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,

or $C_1$-$C_{10}$ alkyl, which is unsubstituted or substituted with 1 to 3 substituents selected from the group consisting of $C_1$-$C_6$ alkoxy, $C_1$-$C_6$ alkylthio, $C_1$-$C_3$ acyl, hydroxyl, halogen, cyano, -$CONH_2$, $C_3$-$C_8$ cycloalkyl, and -$NR^cR^d$,

or -$(CH_2)$n-$R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, $C_3$-$C_6$ cycloalkyl, phenyl, naphthyl, $C_2$-$C_6$ alkynyl, $C_2$-$C_6$ alkenyl, and -$NR^cR^d$, wherein n is an integer from 0 to 3,

or -$(CH_2)$m-$R^f$, wherein $R^f$ is 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$, m is an integer from 0 to 3,

the 4- to 8-membered heteroalicyclic group is a 4- to 8-membered heteroalicyclic group containing 1 to 2 atoms selected from N, O, or S as ring atoms,

the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms,

$R^c$ and $R^d$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono-

or di-$C_1$-$C_3$ alkyl;
$R_3$ is hydrogen, $C_1$-$C_3$ alkyl, hydroxyl, halogen, trifluoromethyl, or cyano.

**2.** The compound, the pharmaceutically acceptable salts, diastereomers, enantiomers, or solvates thereof according to claim 1, wherein, Q is NH, O or S;

$A_1$, $A_2$, and $A_3$ are each independently selected from N or $CR_4$ and at least one of $A_1$, $A_2$, and $A_3$ is N, $R_4$ is H, F, Cl or methyl;
$R_1$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,
or 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^aR^b$,
or aryl or heteroaryl, which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$, -SO-$R_5$, -CO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,
the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms, the 4- to 8-membered heteroalicyclic group is a 4- to 8-membered heteroalicyclic group containing 1 to 2 atoms selected from N, O, or S as ring atoms,
$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,
$R_6$ is $C_1$-$C_{10}$ alkyl, $C_3$-$C_8$ cycloalkyl, 4- to 8-membered heteroalicyclic group, or $C_1$-$C_{10}$ alkyl which is substituted with 1 to 3 susbtituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,
$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;
$R_2$ is $C_3$-$C_8$ cycloalkyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,
or $C_7$-$C_{12}$ bridged cyclyl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$,
or $C_1$-$C_{10}$ alkyl, which is unsubstituted or substituted with 1 to 3 substituents selected from the group consisting of $C_1$-$C_6$ alkoxy, $C_1$-$C_6$ alkylthio, $C_1$-$C_3$ acyl, hydroxyl, halogen, cyano, -$CONH_2$, $C_3$-$C_8$ cycloalkyl, and -$NR^cR^d$,
or -$(CH_2)$n-$R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, $C_2$-$C_6$ alkynyl, $C_2$-$C_6$ alkenyl, and -$NR^cR^d$, wherein n is an integer from 0 to 3,
or -$(CH_2)$m-$R^f$, wherein $R^f$ is 4- to 8-membered heteroalicyclic group, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, oxo (=O) and -$NR^cR^d$, m is an integer from 0 to 3,
the 4- to 8-membered heteroalicyclic group is a 4- to 8-membered heteroalicyclic group containing 1 to 2 atoms selected from N, O, or S as ring atoms,
the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms,
$R^c$ and $R^d$ are each independently selected from hydrogen, $C_1$-$C_6$ alkyl, $C_3$-$C_8$ cycloalkyl, $C_1$-$C_6$ alkyl substituted

with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;
$R_3$ is hydrogen, $C_1$-$C_3$ alkyl, hydroxyl, halogen, trifluoromethyl, or cyano.

**3.** The compound, the pharmaceutically acceptable salts, diastereomers, enantiomers, or solvates thereof according to claim 1 or 2, wherein, $A_3$ is N, $A_1$, and $A_2$ are each independently selected from N or $CR_4$, $R_4$ is H, F, Cl or methyl.

**4.** The compound, the pharmaceutically acceptable salts, diastereomers, enantiomers, or solvates thereof according to any one of claims 1-3, wherein, Q is NH.

**5.** The compound, the pharmaceutically acceptable salts, diastereomers, enantiomers, or solvates thereof according to any one of claims 1 to 4, wherein, $R_1$ is $C_3$-$C_8$ cycloalkyl, 4- to 8-membered heteroalicyclic group, or aryl or heteroaryl which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and -O-$R_6$,

the aryl group is phenyl, naphthyl,

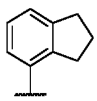 、 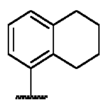 ,

the heteroaryl group is pyrrolyl, furyl, pyridyl, thienyl, imidazolyl, thiazolyl, isothiazolyl, indazolyl, indolyl, isoindolyl, indolinyl, isoindolinyl, isoquinolinyl, indolizinyl, isoxazolyl, 1,5-naphthyridinyl, 1,6-naphthyridinonyl, oxadiazolyl, oxazolyl, 1-phenyl-1H-pyrrolyl, phenazinyl, phenothiazinyl, phenoxazinyl, phthalazinyl, pteridinyl, purinyl, pyranyl, pyrazolyl, pyrazolo[3,4-d]pyrimidinyl, pyrido[3,2-d]pyrimidinyl, pyrido[3,4-d]pyrimidinyl, pyrazinyl, pyrimidinyl, pyridazinyl, quinazolinyl, quinoxalinyl, quinolinyl,

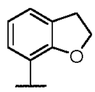 、 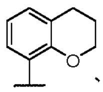 、 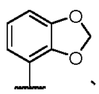 、 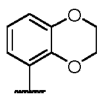 、 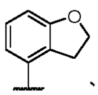 、 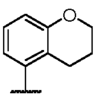 ,

the 4- to 8-membered heteroalicyclic group is a 4- to 8-membered heteroalicyclic group containing 1 to 2 atoms selected from N, O, or S as ring atoms,
$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,
$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -CO-$R_5$, -$SO_2$-$R_5$, -SO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, or $C_1$-$C_6$ alkylthio,
$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;
preferably, wherein, $R_1$ is cyclopropyl, cyclobutyl, cyclopentyl, cyclohexyl, oxetan-3-yl, tetrahydrofuran-3-yl, tetrahydropyran-4-yl, tetrahydropyran-3-yl, pyrrolidinyl, piperidinyl, piperazinyl, morpholinyl, thiomorpholinyl, or aryl or heteroaryl which is unsubstituted or substituted with 1 to 3 substituents selected from the group consisting of $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -$SO_2$-$R_5$,

$-SO-R_5$, $-CO-R_5$, $-CONH-R_5$, $-NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, and $-O-R_6$,

the aryl group is phenyl,

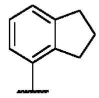, 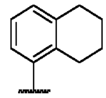,

, the heteroaryl group is pyrazolyl, pyridyl, pyrimidinyl, thiazolyl, oxazolyl,

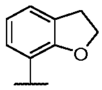, 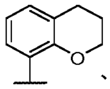, 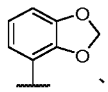, 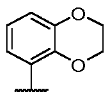, 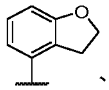, 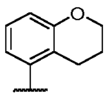,

the 4- to 8-membered heteroalicyclic group is a 4- to 8-membered heteroalicyclic group containing 1 to 2 atoms selected from N, O, or S as ring atoms,

$R_5$ is hydrogen, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, $-NR^aR^b$, $C_3$-$C_8$ cycloalkyloxy, $-CONH-R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, $-SO_2-R_5$, $-SO-R_5$, $-CO-R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

preferably, wherein $R_1$ is

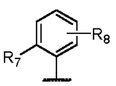, 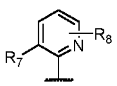, 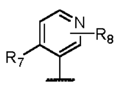, 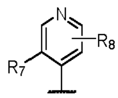, 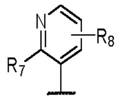, 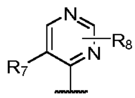,

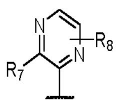, or 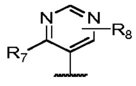,

$R_7$ is hydrogen, $C_1$-$C_4$ alkyl, $C_1$-$C_3$ alkoxy, halogen, hydroxyl, trifluoromethyl, trifluoromethoxy, cyano, $C_2$-$C_4$ alkynyl, $C_2$-$C_4$ alkenyl, $C_3$-$C_4$ cycloalkyl, or $C_1$-$C_3$ acyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, $-CO-R_5$, $-SO_2-R_5$, $-SO-R_5$, $-CONH-R_5$, $-NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or $-O-R_6$,

the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

preferably, wherein, $R_1$ is

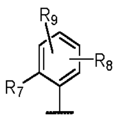, 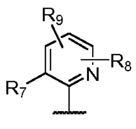, 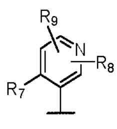, 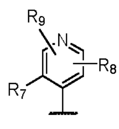, 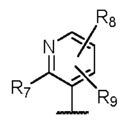, 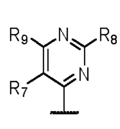,

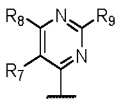, 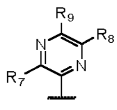, 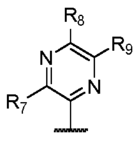, 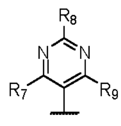 or 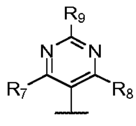,

$R_7$ is hydrogen, $C_1$-$C_4$ alkyl, $C_1$-$C_3$ alkoxy, halogen, hydroxyl, trifluoromethyl, trifluoromethoxy, cyano, $C_2$-$C_4$ alkynyl, $C_2$-$C_4$ alkenyl, $C_3$-$C_4$ cycloalkyl, or $C_1$-$C_3$ acyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or -O-$R_6$,

the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, or $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl which is unsubstituted or substituted with 1 to 3 substituents selected from the group consisting of hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;

$R_9$ is hydrogen, $C_1$-$C_4$ alkyl, $C_1$-$C_3$ alkoxy, halogen, hydroxy, trifluoromethyl, trifluoromethoxy, cyano, $C_2$-$C_4$ alkynyl, $C_2$-$C_4$ alkenyl, $C_3$-$C_4$ Cycloalkyl, or $C_1$-$C_3$ acyl;

preferably, wherein, $R_1$ is:

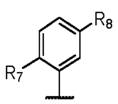, 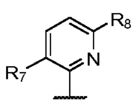, 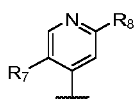, 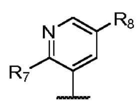, 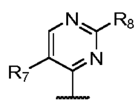,

or

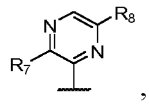

,

$R_7$ is hydrogen, methyl, ethyl, propyl, isopropyl, methoxy, ethoxy, propoxy, isopropoxy, fluorine, chlorine, hydroxyl, trifluoromethyl, trifluoromethoxy, cyano, ethynyl, propynyl, vinyl, propenyl, cyclopropyl, cyclobutyl, formyl, or acetyl;

$R_8$ is hydrogen, $C_1$-$C_{10}$ alkyl, halogen, $C_3$-$C_8$ cycloalkyl, halogenated $C_1$-$C_{10}$ alkyl, cyano, hydroxyl, $C_1$-$C_6$ alkylthio, -CO-$R_5$, -$SO_2$-$R_5$, -SO-$R_5$, -CONH-$R_5$, -$NR^aR^b$, 4- to 8-membered heteroalicyclic group, $C_2$-$C_6$ alkynyl, $C_2$-$C_6$ alkenyl, $C_1$-$C_3$ alkoxy $C_1$-$C_6$ alkylthio, $C_1$-$C_{10}$ alkyl substituted with hydroxyl, $C_1$-$C_6$ alkoxy $C_1$-$C_{10}$ alkyl, $C_3$-$C_8$ cycloalkyl $C_1$-$C_6$ alkyl, ($C_3$-$C_8$ cycloalkyl)-O-($C_1$-$C_6$ alkyl), $C_1$-$C_6$ alkyl substituted with 4- to 8-membered heteroalicyclic group, or -O-$R_6$,

the 4- to 8-membered heteroalicyclic group is a 4- to 8-membered heteroalicyclic group containing 1 to 2 atoms selected from N, O, or S as ring atoms,

$R_5$ is hydrogen, hydroxyl, $C_1$-$C_6$ alkyl, $C_1$-$C_6$ alkoxy $C_1$-$C_6$ alkyl, $C_1$-$C_6$ alkyl substituted with hydroxyl, $C_3$-$C_8$ cycloalkyl, $C_1$-$C_6$ alkyl substituted with 4- to 8-membered heteroalicyclic group,

$R_6$ is $C_1$-$C_{10}$ alkyl, $C_3$-$C_8$ cycloalkyl, 4- to 8-membered heteroalicyclic group, or $C_1$-$C_{10}$ alkyl substituted with 1 to 3 susbtituents selected from hydroxyl, $C_1$-$C_6$ alkoxy, cyano, -$NR^aR^b$, $C_3$-$C_8$ cycloalkyloxy, -CONH-$R_5$, $C_3$-$C_8$ cycloalkyl, $C_3$-$C_8$ cycloalkyl substituted with hydroxyl and/or $C_1$-$C_4$ alkyl, carboxylic, halogen, halogenated $C_1$-$C_6$ alkoxy, -$SO_2$-$R_5$, -SO-$R_5$, -CO-$R_5$, $C_2$-$C_6$ alkynyl, $C_2$-$C_6$ alkenyl, $C_1$-$C_4$ alkoxy $C_1$-$C_6$ alkoxy, 4- to 8-membered heteroalicyclic group, 4- to 8-membered heteroalicyclic group substituted with oxo, 4- to 8-membered heteroalicyclic group substituted with hydroxyl and/or $C_1$-$C_4$ alkyl, and $C_1$-$C_6$ alkylthio,

$R^a$ and $R^b$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl.

**6.** The compound, the pharmaceutically acceptable salts, diastereomers, enantiomers, or solvates thereof according to any one of claims 1 to 5, wherein, $R_2$ is $C_3$-$C_8$ cycloalkyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino,

or $C_7$-$C_{10}$ bridged cyclyl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino,

or $C_1$-$C_{10}$ alkyl, which is unsubstituted or substituted with 1 to 3 substituents selected from the group consisting of methoxy, ethoxy, propoxy, isopropoxy, methylthio, ethylthio, propylthio, formyl, acetyl, hydroxyl, fluorine, chlorine, cyano, -$CONH_2$, cyclopropyl, cyclobutyl, cyclopentyl, cyclohexyl, amino, dimethylamino, and diethylamino,

or -$(CH_2)$n-$R^e$, wherein $R^e$ is aryl or heteroaryl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, ethynyl, vinyl, amino, dimethylamino, and diethylamino, n is an integer from 0 to 3,

or -$(CH_2)$m-$R^f$, wherein $R^f$ is 4- to 8-membered heteroalicyclic group, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, oxo (=O), amino, dimethylamino, and diethylamino, m is an integer from 0 to 3,

the 4- to 8-membered heteroalicyclic group contains 1 to 2 atoms selected from N, O, or S as ring atoms, the aryl group is phenyl, and the heteroaryl group is pyridyl, pyrimidinyl, pyrazolyl, oxazolyl, isoxazolyl, thiazolyl.

**7.** The compound, the pharmaceutically acceptable salts, diastereomers, enantiomers, or solvates thereof according to claim 6, wherein, $R_2$ is cyclopropyl, cyclobutyl, cyclopentyl, cyclohexyl, cycloheptyl, cyclooctyl, 4,4-difluorocyclohexyl, bicyclo[2.2.1]heptyl, adamantyl, methyl, ethyl, propyl, isopropyl, butyl, isobutyl, sec-butyl, tert-butyl, pentyl, neopentyl, hexyl, 2-hydroxy-2-methylpropyl, 3,3-dimethylbutyl, 3-hydroxy-3-methylbutyl, cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, benzyl, phenethyl, phenyl, 2-fluorophenyl, 2-chlorophenyl, 2-methoxyphenyl, 2-cyanophenyl, 2-ethynylphenyl, 3-fluorophenyl, 3-chlorophenyl, 3-methoxyphenyl, 3-cyanophenyl, 3-ethynylphenyl, 4-fluorophenyl, 4-chlorophenyl, 4-methoxyphenyl, 4-cyanophenyl, 4-ethynylphenyl, 3,4-difluorophenyl, 3-cyano-4-methylphenyl, pyridin-2-yl, pyridin-3-yl, pyridine-4-yl, pyrimidin-2-yl, pyrimidin-4-yl, pyrimidin-5-yl, pyrazinyl, pyrazolyl, oxazolyl, isoxazolyl, thiazolyl, oxetan-3-yl, tetrahydrofuran-3-yl, tetrahydro-2H-pyran-4-yl, tetrahydropyrrolyl, piperidin-1-yl, piperazin-1-yl, morpholin-4-yl, methylpiperazin-4-yl, 1-methylpiperidin-4-yl, 1-acetylpiperidin-4-yl, 4-hydroxypiperidin-1-yl, 4-methyl-4-hydroxypiperidin-1-yl,

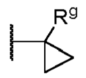, 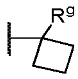,

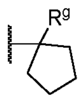, 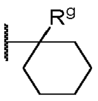, 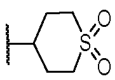, 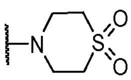, 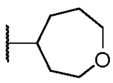, 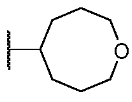, 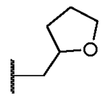, 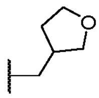, 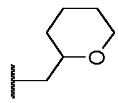,

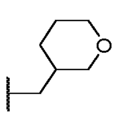, or 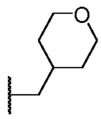,

$R^g$ is $-CH_3$ or -OH.

**8.** The compound, the pharmaceutically acceptable salts, diastereomers, enantiomers, or solvates thereof according to claim 1, wherein, $R_2$ is 2,3-difluorophenyl, 3,5-difluorophenyl, 2,5-difluorophenyl, 2,4-difluorophenyl, 2,3,4-trifluorophenyl, 2,3,5-trifluorophenyl, 2,3,6-trifluorophenyl, 3,4,5-trifluorophenyl, 2,4,5-trifluorophenyl, 3-chloro-2-fluorophenyl, 2-chloro-3-fluorophenyl, 5-chloro-2-fluorophenyl, 2-chloro-5-fluorophenyl, 5-chloro-3-fluorophenyl, 3-chloro-5-fluorophenyl, 3-chloro-4-fluorophenyl, 4-chloro-3-fluorophenyl, 2-chloro-4-fluorophenyl, 4-chloro-2-fluorophenyl, 3-chloro-2,4-difluorophenyl, 5-chloro-2,4-difluorophenyl, 3-chloro-2,5-difluorophenyl, 3-chloro-2,6-difluorophenyl, 3-chloro-4,5-difluorophenyl, 2-chloro-4,5-difluorophenyl, 2-chloro-3,4-difluorophenyl, 2-chloro-3,5-difluorophenyl, 2-chloro-3,6-difluorophenyl, 4-chloro-2,3-difluorophenyl, 4-chloro-3,5-difluorophenyl, 4-chloro-2,5-difluorophenyl, 5-chloro-2,3 -difluorophenyl, 5 -chloro-3,4-difluorophenyl, 6-chloro-2,3 -difluorophenyl, 3 -fluoro-5 -methylphenyl, 4-fluoro-3-methylphenyl, 2-fluoro-3-methylphenyl, 2-fluoro-4-methylphenyl, 2-fluoro-5-methylphenyl, 3-fluoro-4-methylphenyl, 3-fluoro-2-methylphenyl, 3-fluoro-5-methoxyphenyl, 4-fluoro-3-methoxyphenyl, 2-fluoro-3-methoxyphenyl, 2-fluoro-4-methoxyphenyl, 2-fluoro-5-methoxyphenyl, 3-fluoro-4-methoxyphenyl, 3-fluoro-2-methoxyphenyl, 2-fluoro-3-trifluoromethylphenyl, 2-fluoro-4-trifluoromethylphenyl, 2-fluoro-5-trifluoromethylphenyl, 3-fluoro-2-trifluoromethylphenyl, 4-fluoro-2-trifluoromethylphenyl, 5-fluoro-2-trifluoromethylphenyl, 4-fluoro-3-trifluoromethylphenyl, 3-fluoro-4-trifluoromethylphenyl, 3-fluoro-5-trifluoromethylphenyl, 2-fluoro-5-ethylphenyl, 2-fluoro-5-cyclopropylphenyl, or 2-fluoro-5-phenylphenyl.

**9.** The compound, the pharmaceutically acceptable salts, diastereomers, enantiomers, or solvates thereof according to claim 1, wherein, $R_3$ is hydrogen, methyl, ethyl, hydroxyl, cyano, trifluoromethyl, fluorine, or chlorine.

**10.** The compound, the pharmaceutically acceptable salts, diastereomers, enantiomers, or solvates thereof according to claim 1,

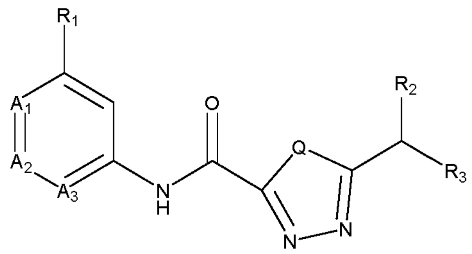

formula (I)

in formula (I),

Q is NH;
$A_1$, and $A_2$ are each CH;
$A_3$ is N;
$R_1$ is

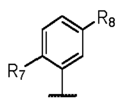 , 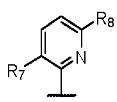 , 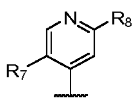 , 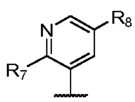 , 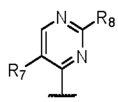 , or 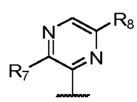 ;

$R_7$ is methyl, ethyl, propyl, isopropyl, fluorine, chlorine, trifluoromethyl, vinyl, or propenyl;
$R_8$ is cyano, $C_1$-$C_3$ alkoxy $C_1$-$C_6$ alkylthio, or -O-$R_6$;
$R_5$ is $C_1$-$C_6$ alkyl;
$R_6$ is 4- to 8-membered heteroalicyclic group, or $C_1$-$C_{10}$ alkyl which is substituted with 1 to 3 substituents selected from the group consisting of hydroxyl, $C_1$-$C_6$ alkoxy, cyano, $C_3$-$C_8$ cycloalkyloxy, $C_3$-$C_8$ cycloalkyl, - $SO_2$-$R_5$, 4- to 8-membered heteroalicyclic group, and $C_1$-$C_6$ alkylthio;
$R_2$ is -$(CH_2)$n-$R^e$, wherein $R^e$ is aryl or heteroaryl, which is unsubstituted or substituted with 1 to 3 substituents selected from the group consisting of $C_1$-$C_6$ alkyl, $C_1$-$C_6$ alkoxy, $C_1$-$C_6$ alkylthio, $C_1$-$C_3$ acyl, hydroxyl, halogen, trifluoromethyl, cyano, -$CONH_2$, $C_2$-$C_6$ alkynyl, $C_2$-$C_6$ alkenyl, and -$NR^cR^d$, wherein n is an integer from 0 to 3;
$R^c$ and $R^d$ are each independently selected from hydrogen, $C_1$-$C_6$ alkyl, $C_3$-$C_8$ cycloalkyl, $C_1$-$C_6$ alkyl substituted with $C_1$-$C_6$ alkoxy, $C_1$-$C_6$ alkyl substituted with hydroxyl, $C_3$-$C_8$ cycloalkyl $C_1$-$C_6$ alkyl, $C_1$-$C_6$ alkyl substituted with 4- to 8-membered heteroalicyclic group, $C_1$-$C_6$ alkyl substituted with $C_1$-$C_3$ alkylthio, or $C_1$-$C_6$ alkyl substituted with substituted amino or unsubstituted amino, wherein the substituted amino is substituted with mono- or di-$C_1$-$C_3$ alkyl;
the 4- to 8-membered heteroalicyclic group is a 4- to 8-membered heteroalicyclic group containing 1 to 2 atoms selected from N, O, or S as ring atoms;
the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms;
$R_3$ is hydrogen.

**11.** The compound, the pharmaceutically acceptable salts, diastereomers, enantiomers, or solvates thereof according to claim 10, wherein

Q is NH;
$A_1$, and $A_2$ are each CH;
$A_3$ is N;
$R_1$ is

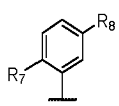 , 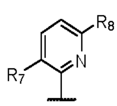 , 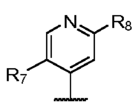 , 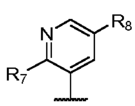 , 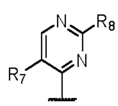 , or 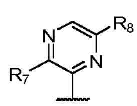 ;

$R_7$ is methyl, chlorine, or trifluoromethyl;
$R_8$ is cyano, $C_1$-$C_3$ alkoxy $C_1$-$C_6$ alkylthio, or -O-$R_6$;
$R_6$ is $C_1$-$C_{10}$ alkyl which is substituted with 1 to 3 substituents selected from hydroxyl;
$R_2$ is -($CH_2$)n-$R^e$, wherein $R^e$ is aryl or heteroaryl, which is unsubstituted or substituted with 1 to 3 substituents selected from the group consisting of methyl, ethyl, propyl, isopropyl, methoxy, ethoxy, propoxy, isopropoxy, methylthio, ethylthio, propylthio, formyl, acetyl, hydroxyl, fluorine, chlorine, trifluoromethyl, cyano, -$CONH_2$, ethynyl, vinyl, amino, dimethylamino, and diethylamino, n is an integer from 0 to 3,
the aryl group is a monocyclic or bicyclic group containing 6 to 12 carbon ring atoms and having at least one aromatic ring, the heteroaryl is a monocyclic or bicyclic group having 5 to 10 ring atoms and containing 1 to 3 heteroatoms selected from N, O, or S as ring atoms;
$R_3$ is hydrogen.

**12.** The compound, the pharmaceutically acceptable salts, diastereomers, enantiomers, or solvates thereof according to claim 1 wherein the compound is selected from the following structures:

| | | | |
|---|---|---|---|
| 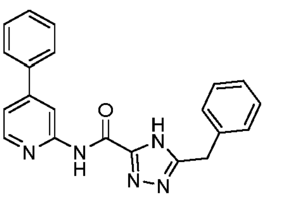 | 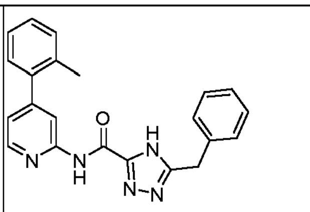 | 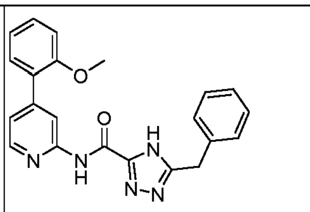 | 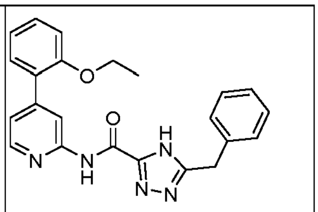 |
| 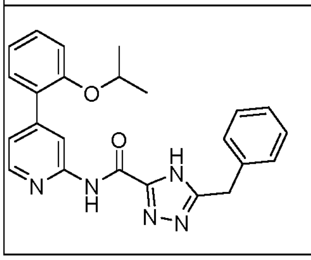 | 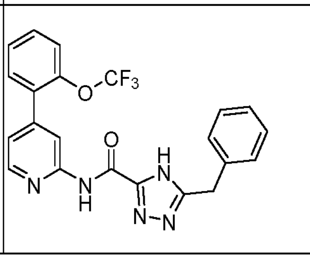 | 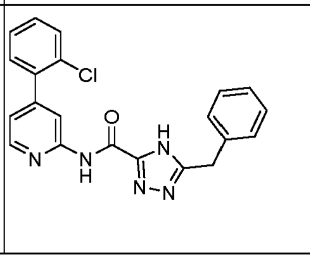 | 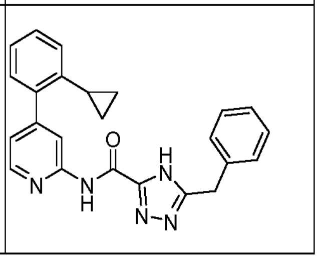 |

| | | | |
|---|---|---|---|
| 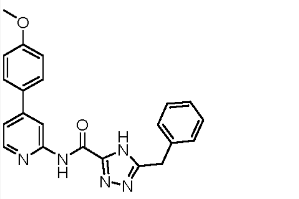 | 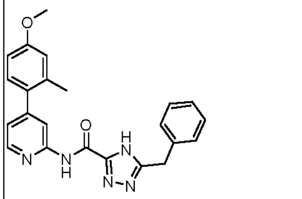 | 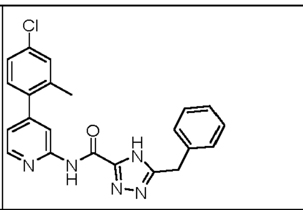 | 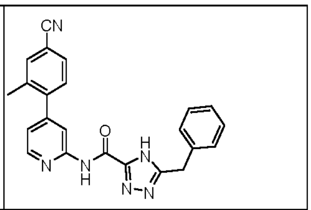 |
| 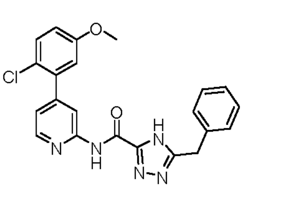 | 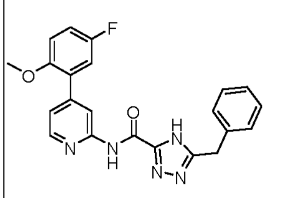 | 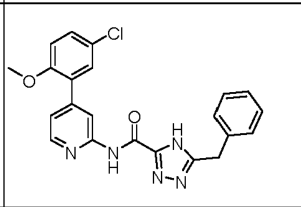 | 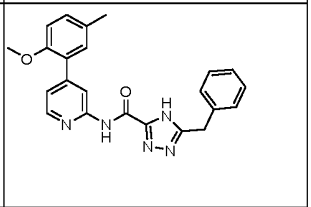 |
| 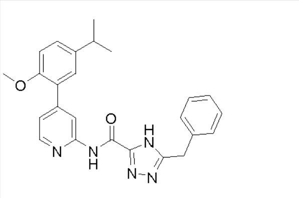 | 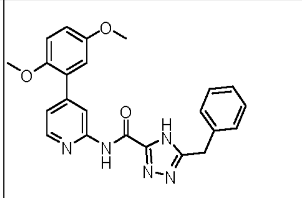 | 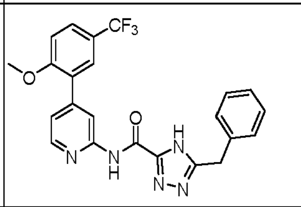 | 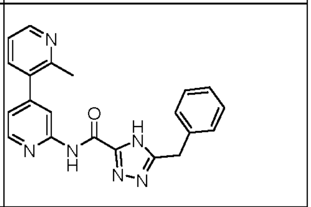 |
| 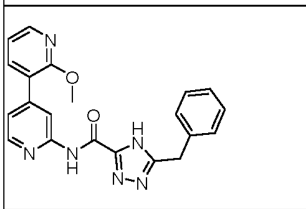 | 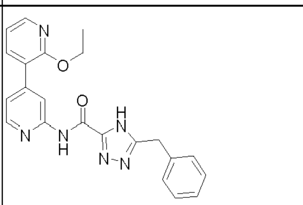 | 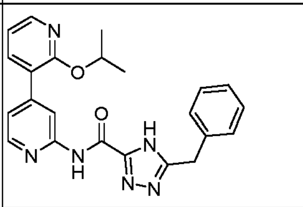 | 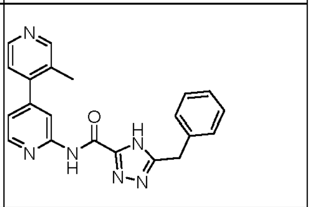 |
| 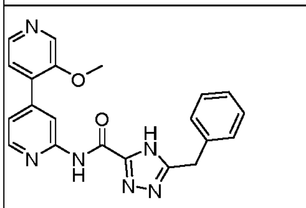 | 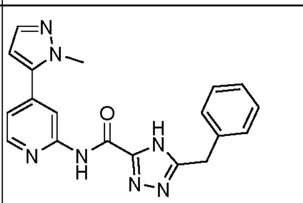 | 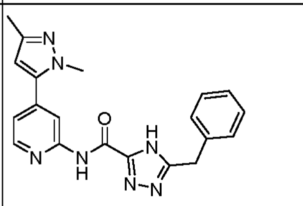 | 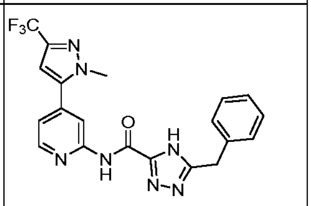 |
| 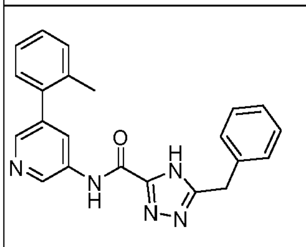 | 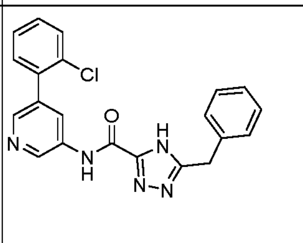 | 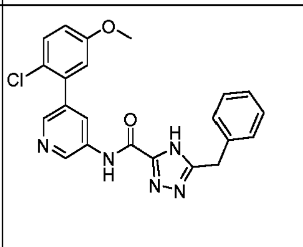 | 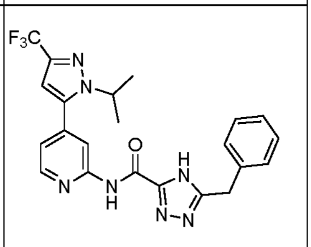 |
| 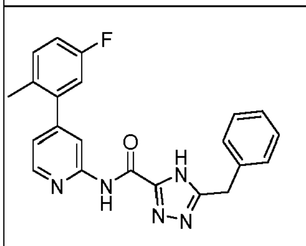 | 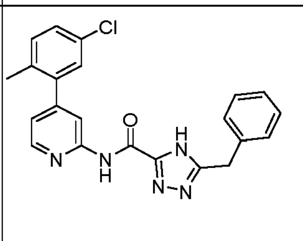 | 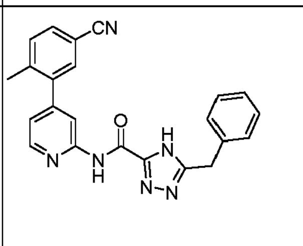 | 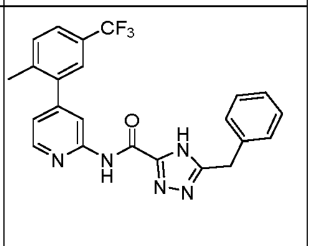 |
| 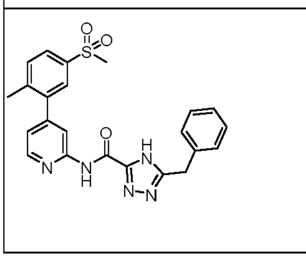 | 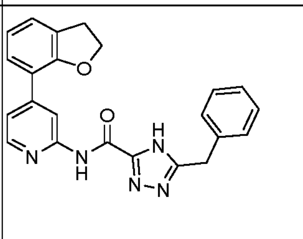 | 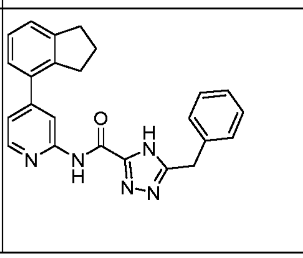 | 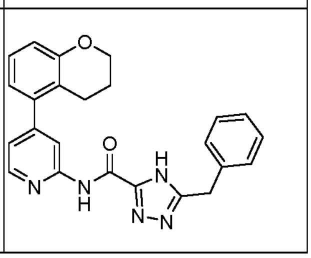 |

| | | | |
|---|---|---|---|
| 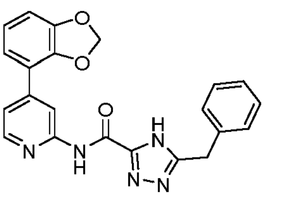 | 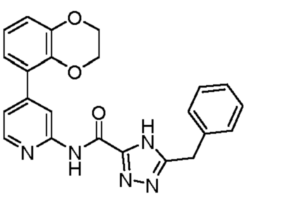 | 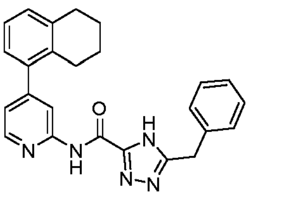 | 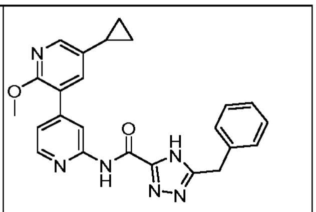 |
| 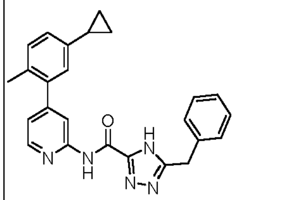 | 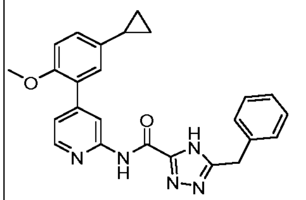 | 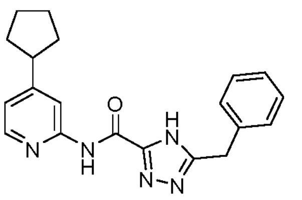 | 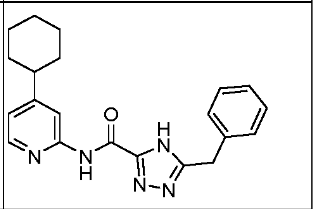 |
| 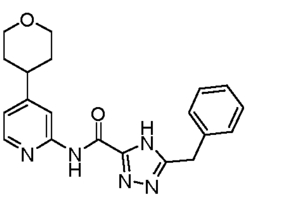 | 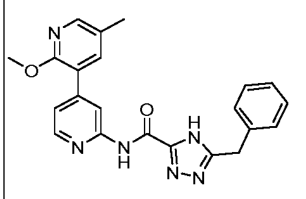 | 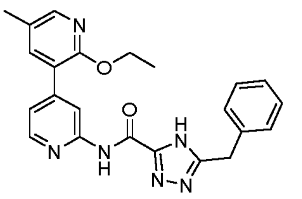 | 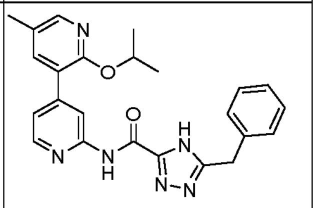 |
| 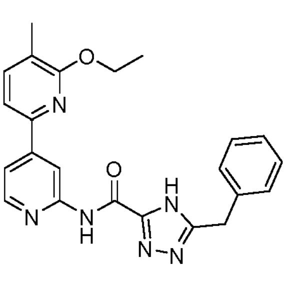 | 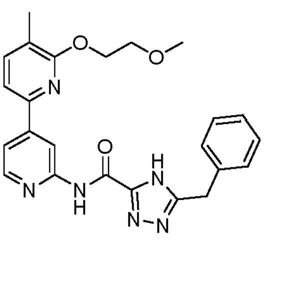 | 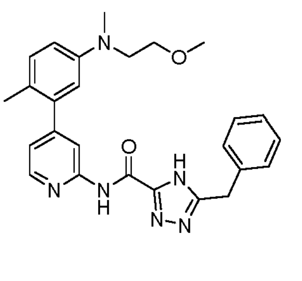 | 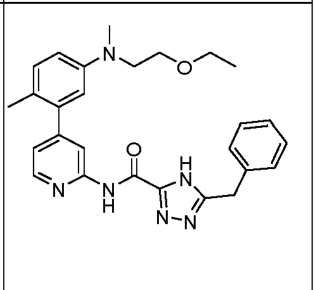 |
| 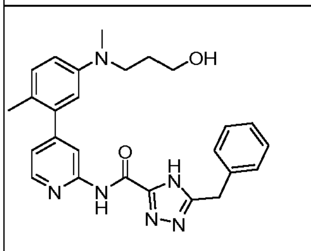 | 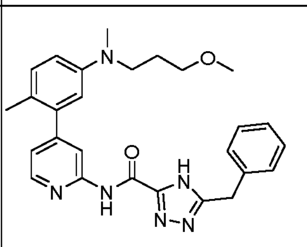 | 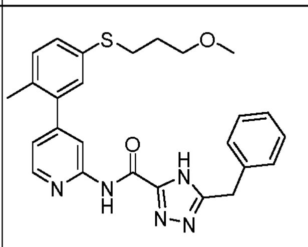 | 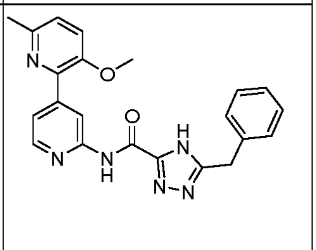 |
| 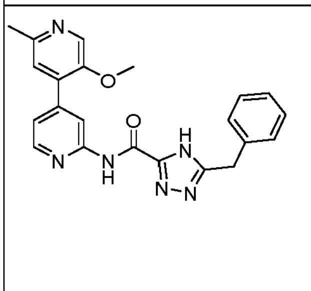 | 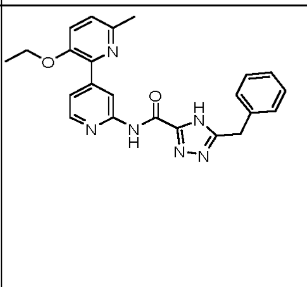 | 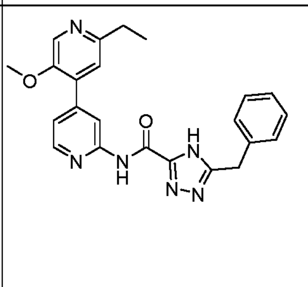 | 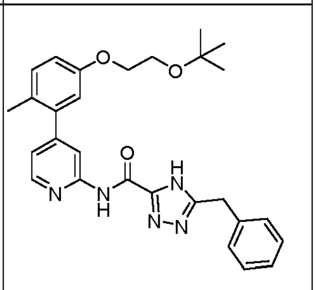 |
| 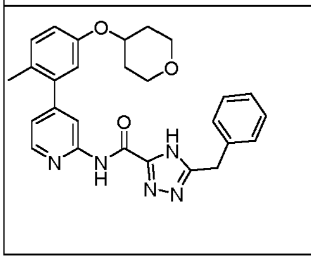 | 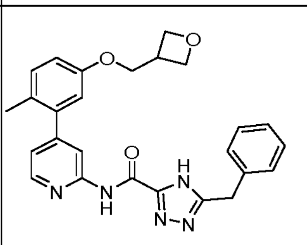 | 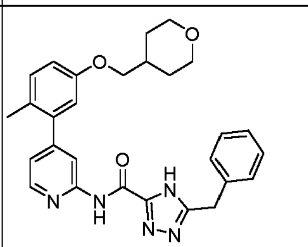 | 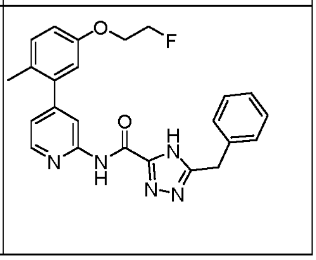 |

| | | | |
|---|---|---|---|
| 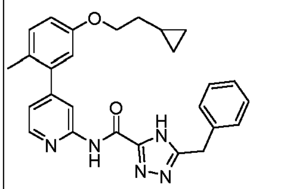 | 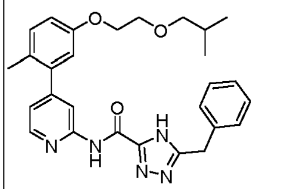 | 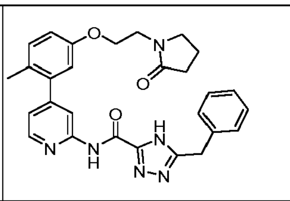 | 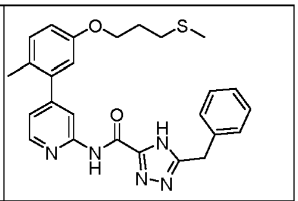 |
| 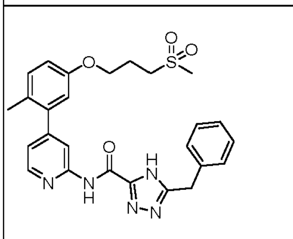 | 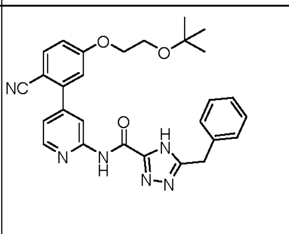 | 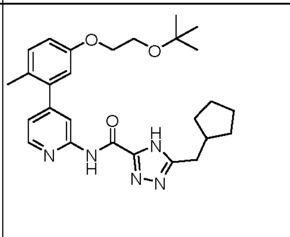 | 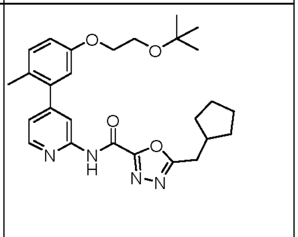 |
| 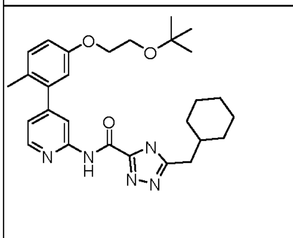 | 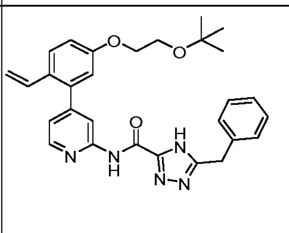 | 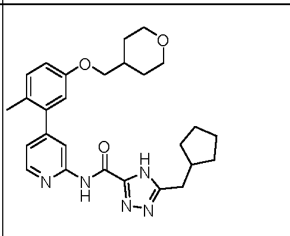 | 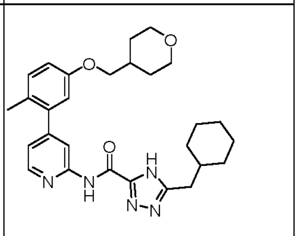 |
| 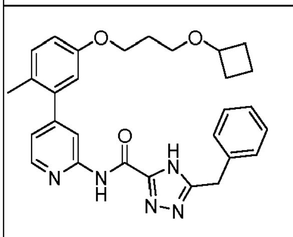 | 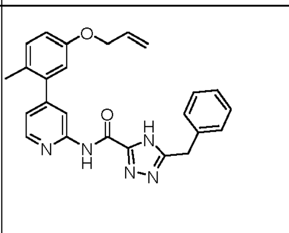 | 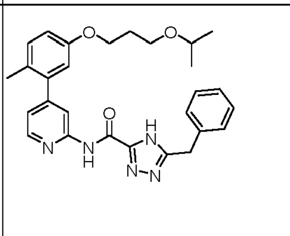 | 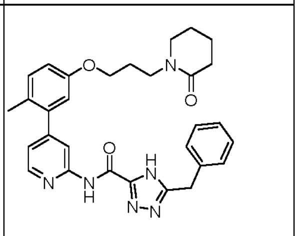 |
| 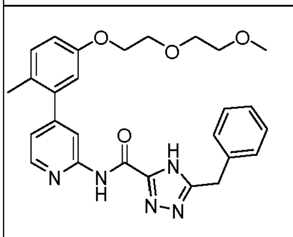 | 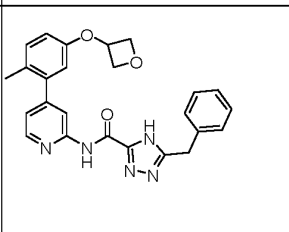 | 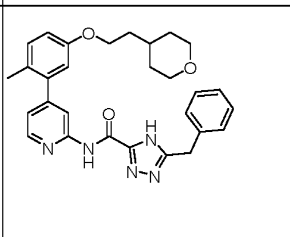 | 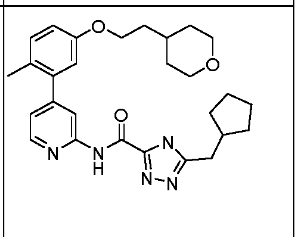 |
| 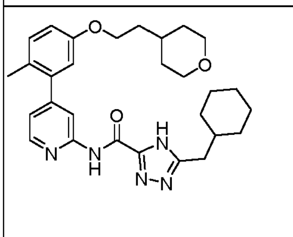 | 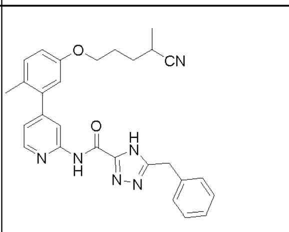 | 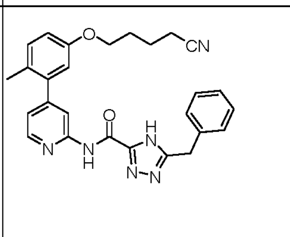 | 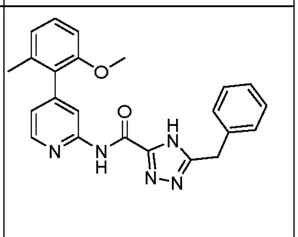 |
| 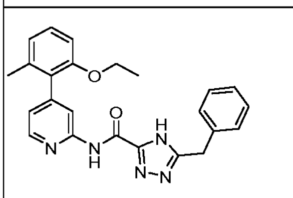 | 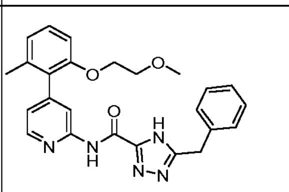 | 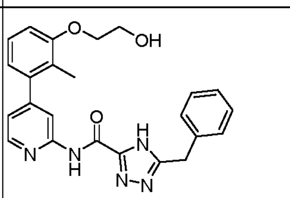 | 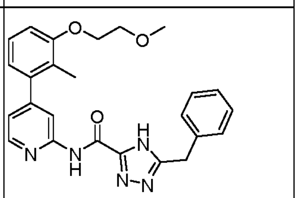 |
| 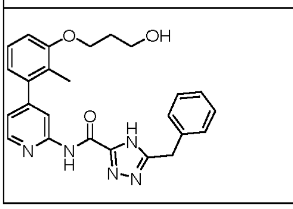 | 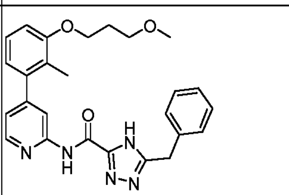 | 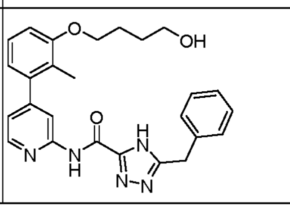 | 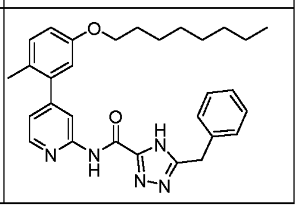 |

| | | | |
|---|---|---|---|
| 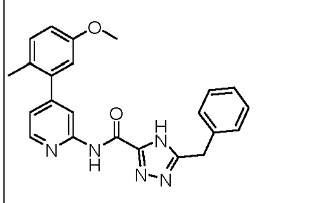 | 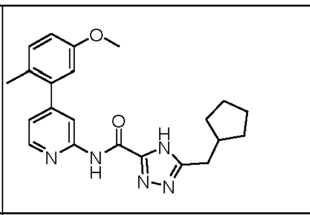 | 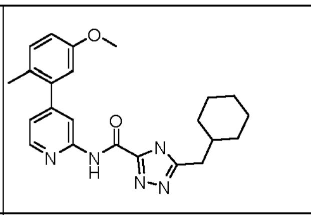 | 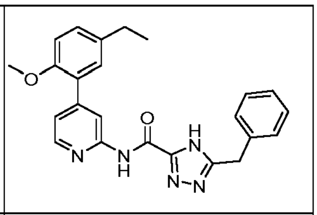 |
| 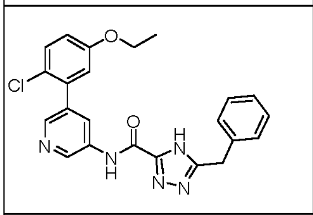 | 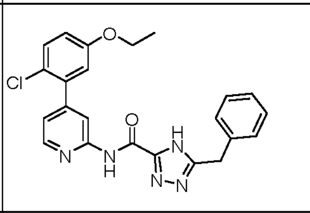 | 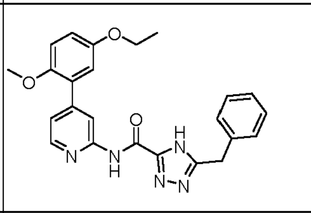 | 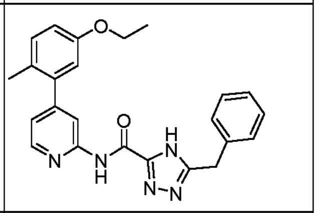 |
| 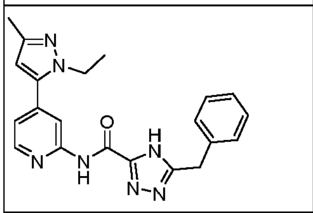 | 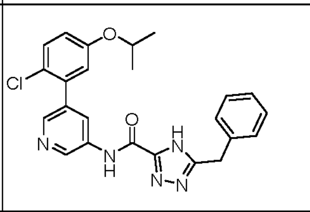 | 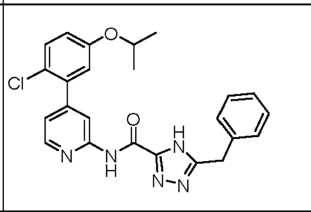 | 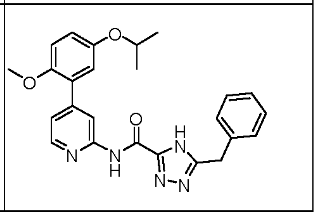 |
| 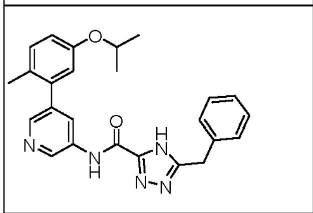 | 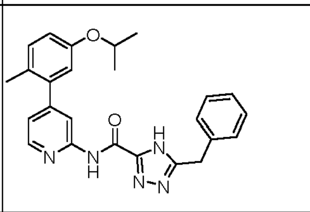 | 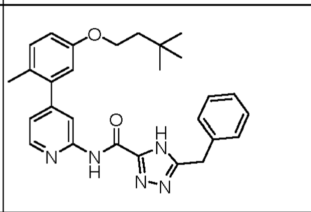 | 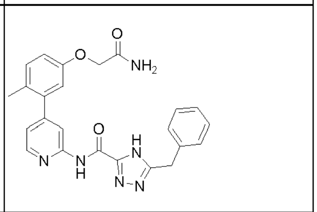 |
| 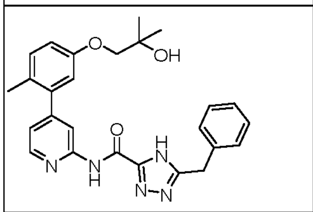 | 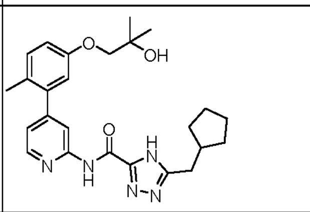 | 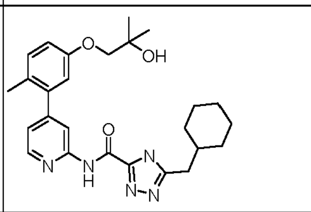 | 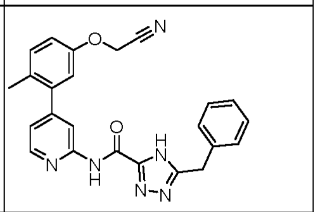 |
| 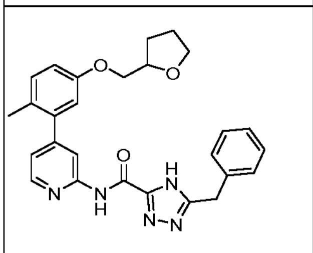 | 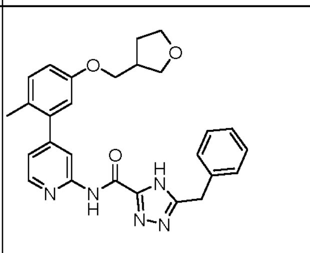 | 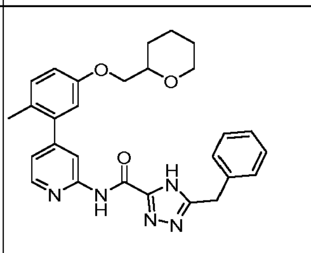 | 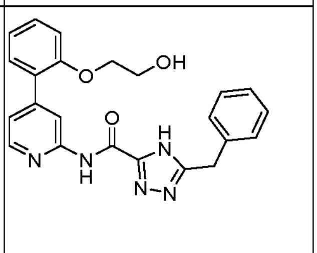 |
| 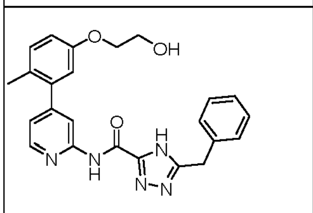 | 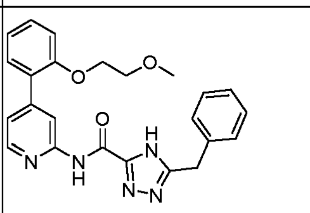 | 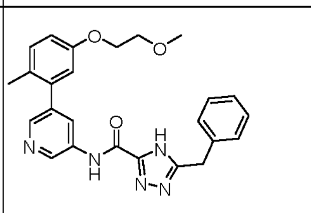 | 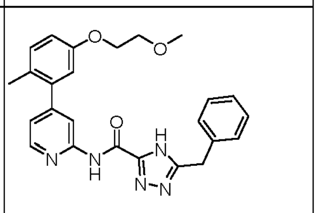 |
| 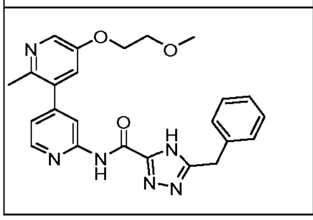 | 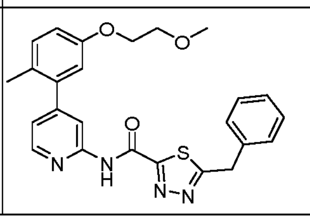 | 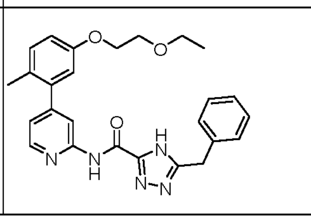 | 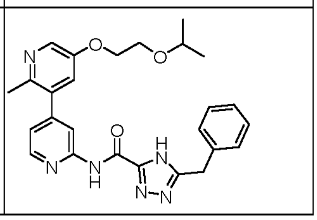 |

| | | | |
|---|---|---|---|
| 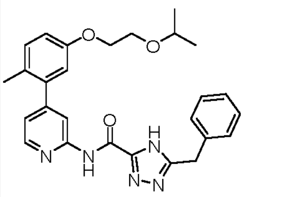 | 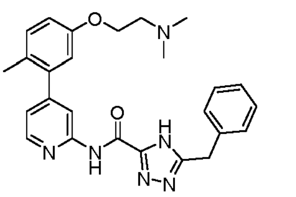 | 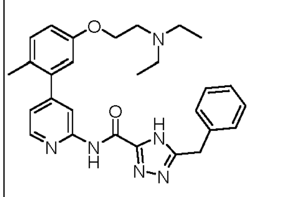 | 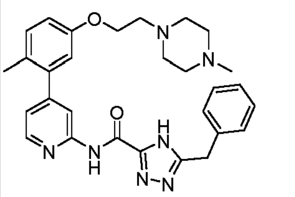 |
| 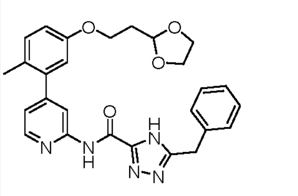 | 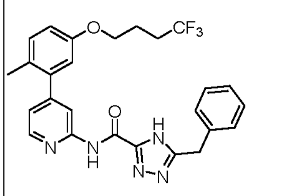 | 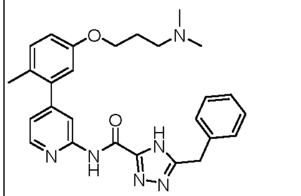 | 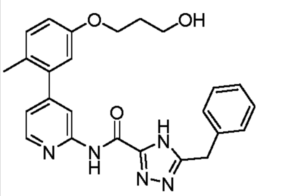 |
| 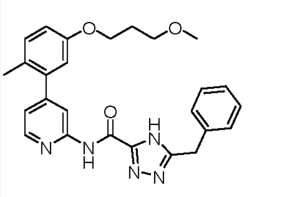 | 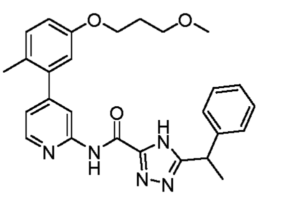 | 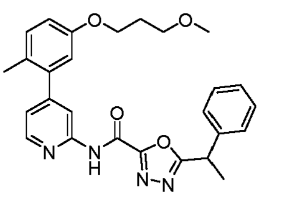 | 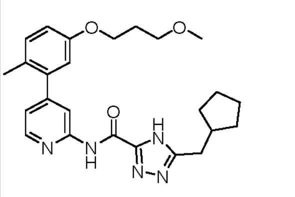 |
| 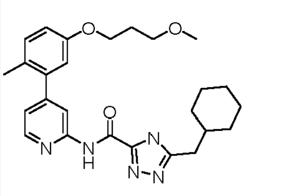 | 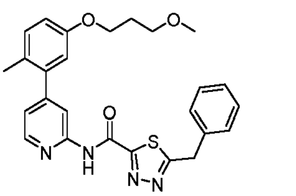 | 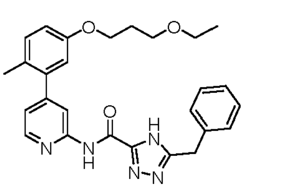 | 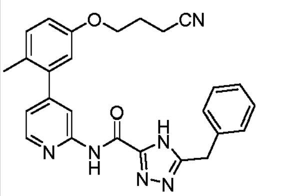 |
| 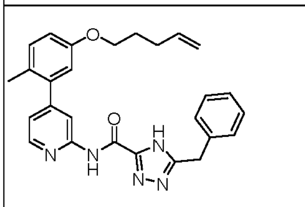 | 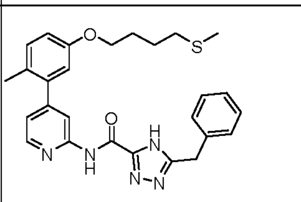 | 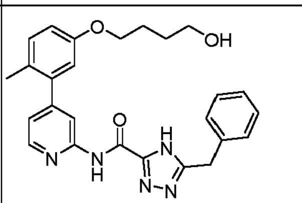 | 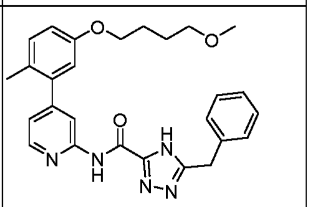 |
| 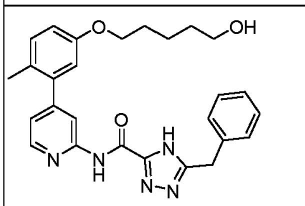 | 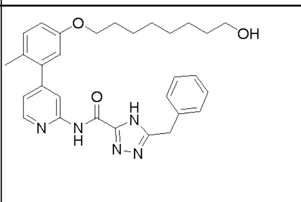 | 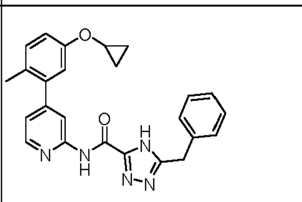 | 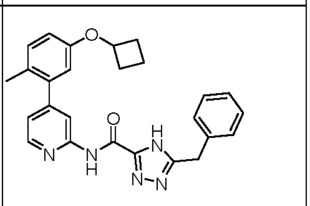 |
| 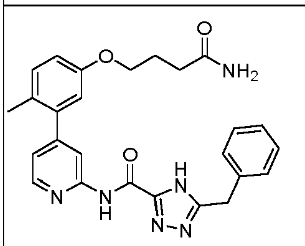 | 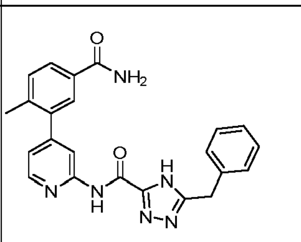 | 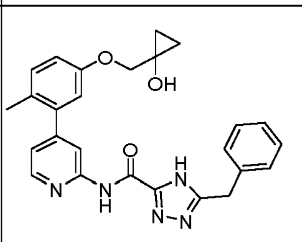 | 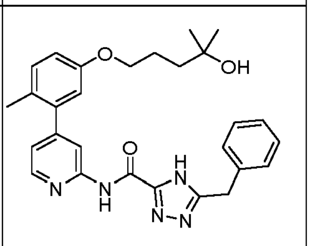 |
| 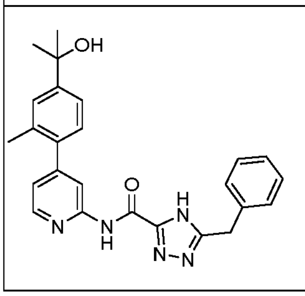 | 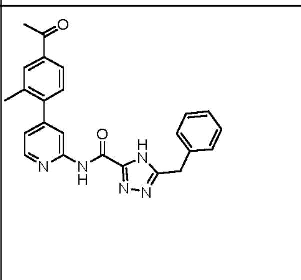 | 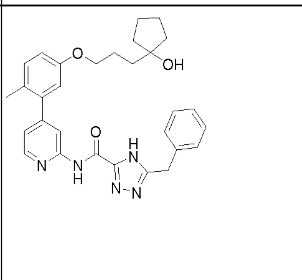 | 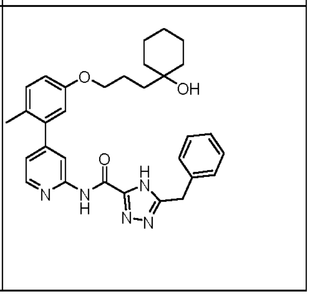 |

| | | | |
|---|---|---|---|
| 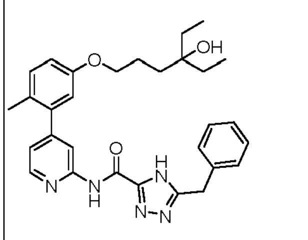 | 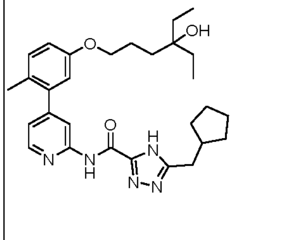 | 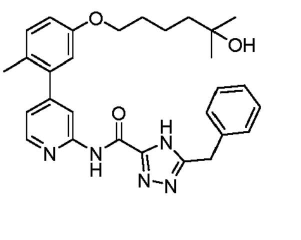 | 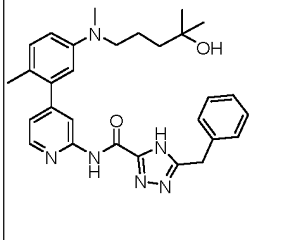 |
| 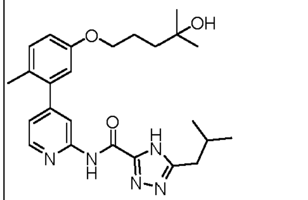 | 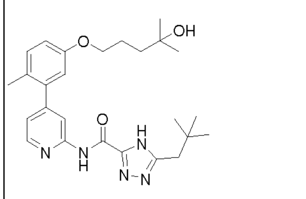 | 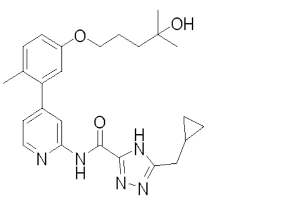 | 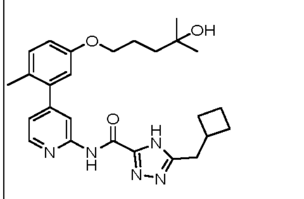 |
| 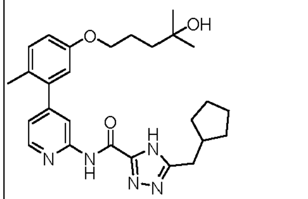 | 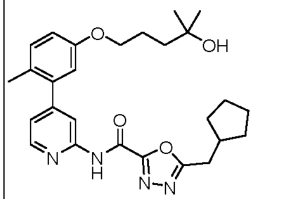 | 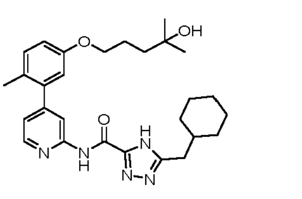 | 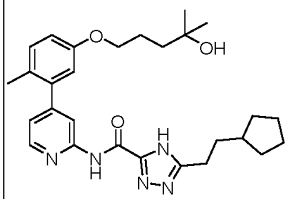 |
| 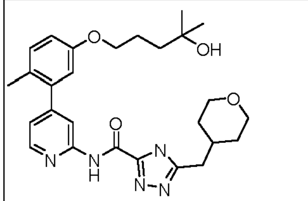 | 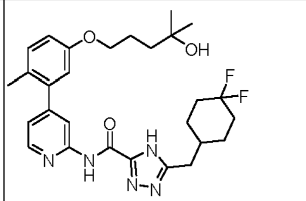 | 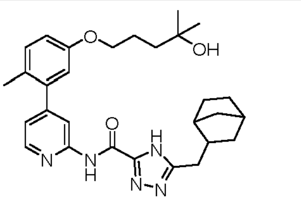 | 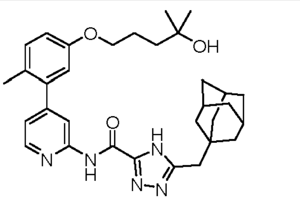 |
| 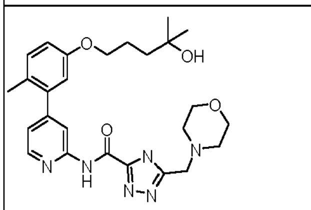 | 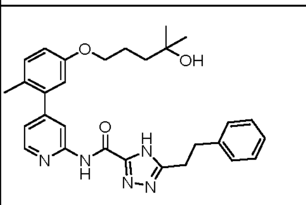 | 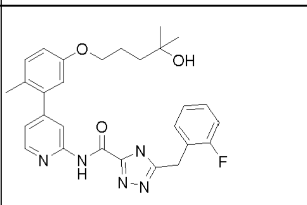 | 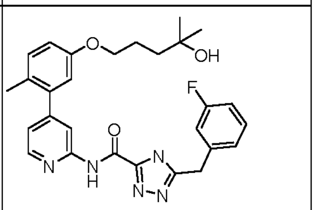 |
| 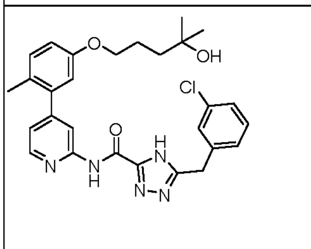 | 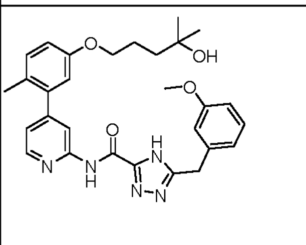 | 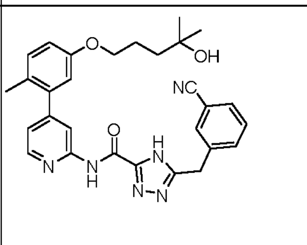 | 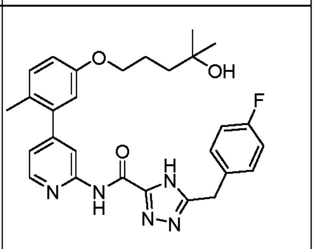 |
| 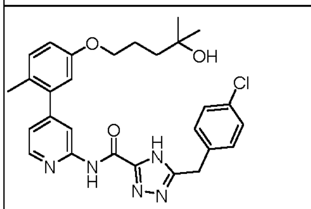 | 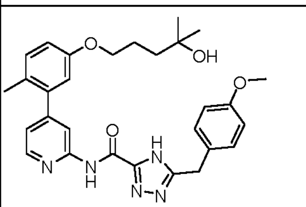 | 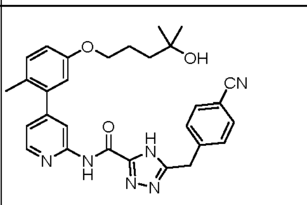 | 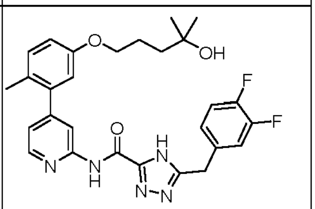 |
| 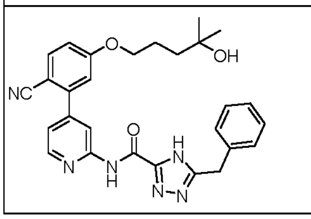 | 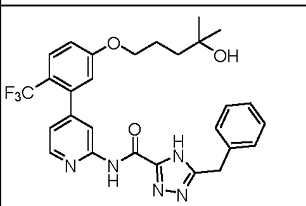 | 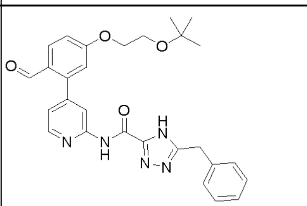 | 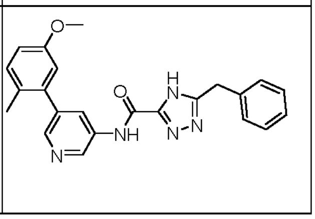 |

| | | | |
|---|---|---|---|
| 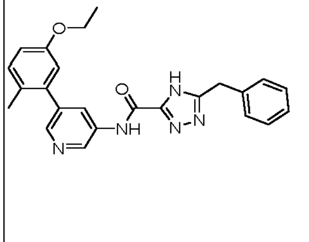 | 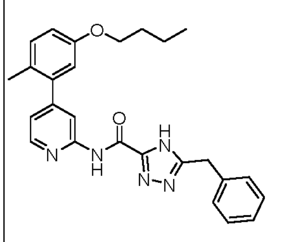 | 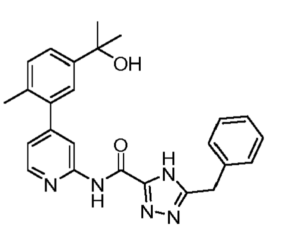 | 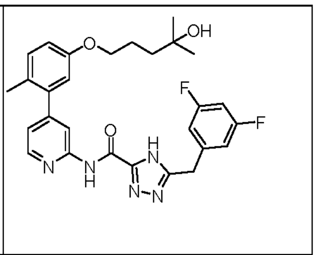 |
| 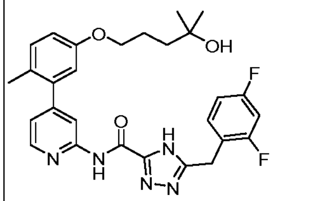 | 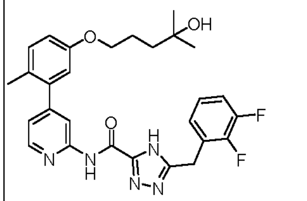 | 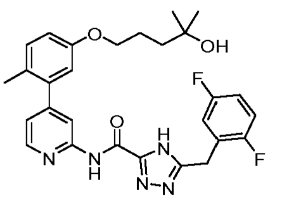 | 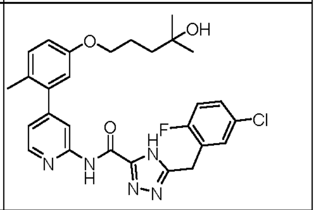 |
| 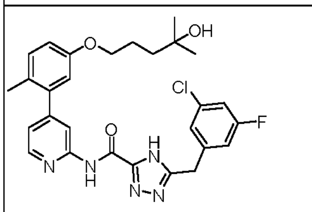 | 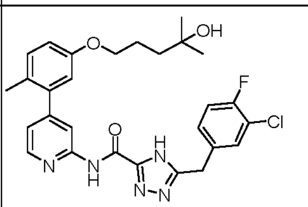 | 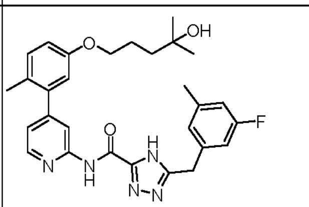 | 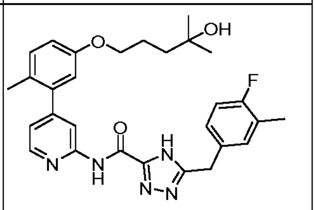 |
| 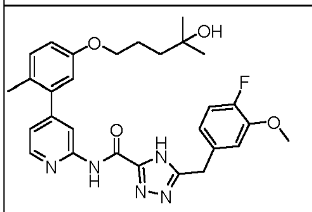 | 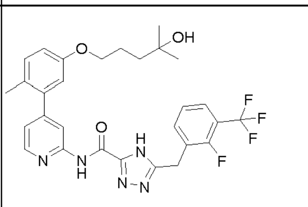 | 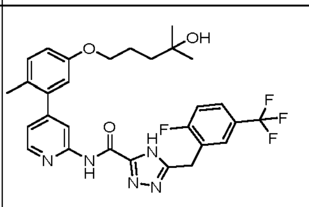 | 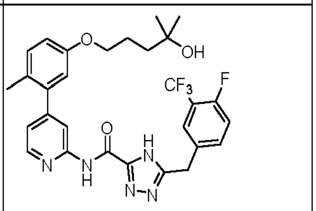 |
| 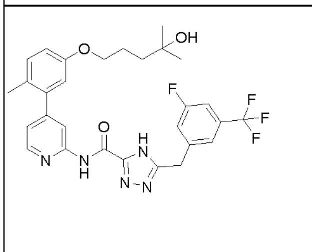 | 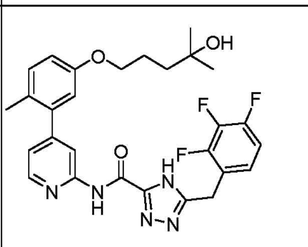 | 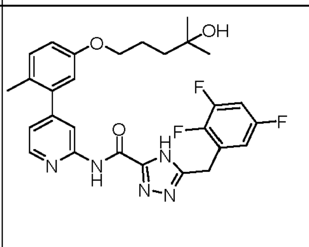 | 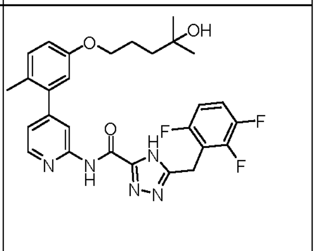 |
| 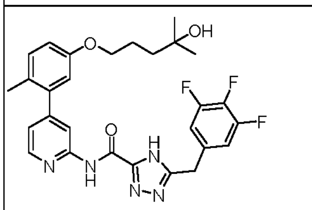 | 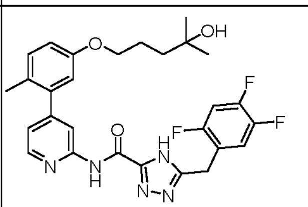 | 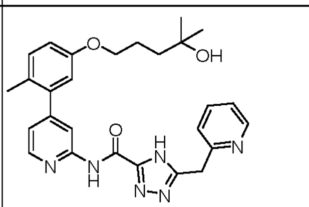 | 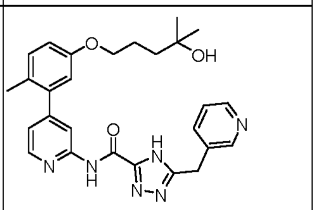 |
| 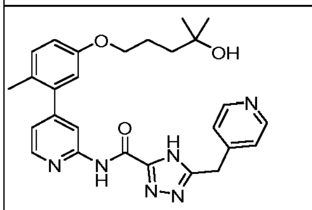 | 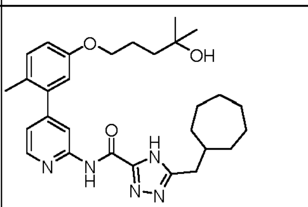 | 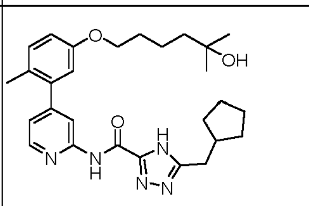 | 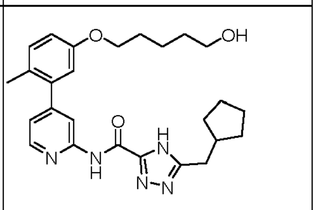 |
| 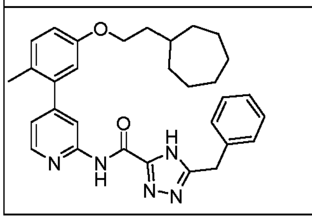 | 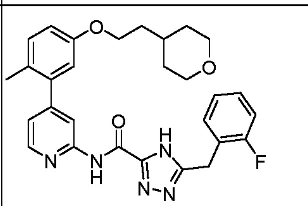 | 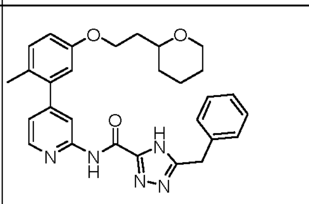 | 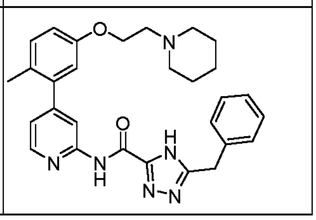 |

| | | | |
|---|---|---|---|
| 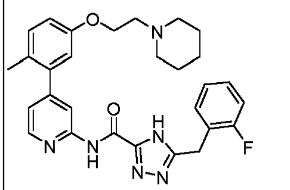 | 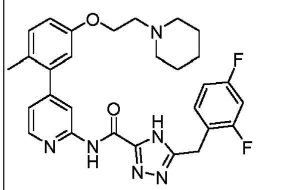 | 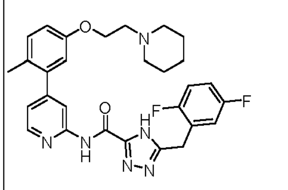 | 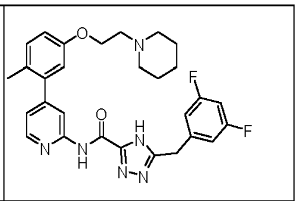 |
| 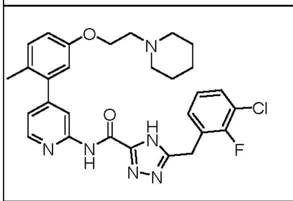 | 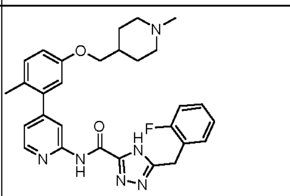 | 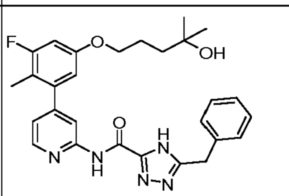 | 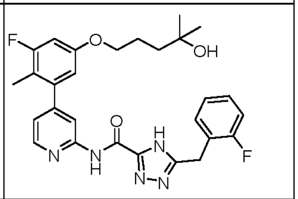 |
| 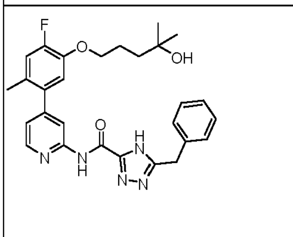 | 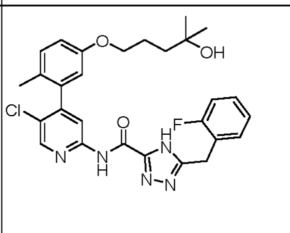 | 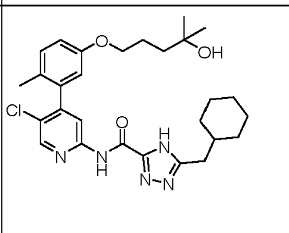 | 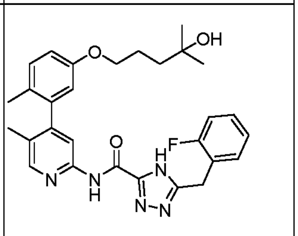 |
| 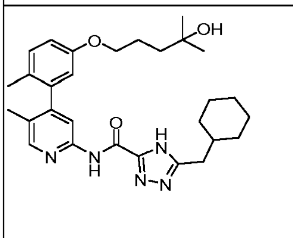 | 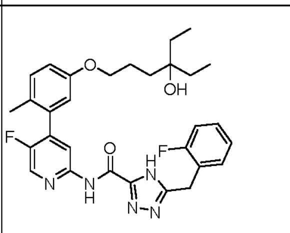 | 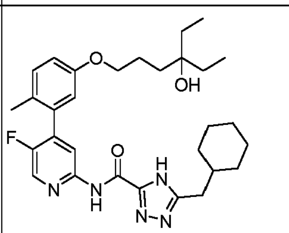 | 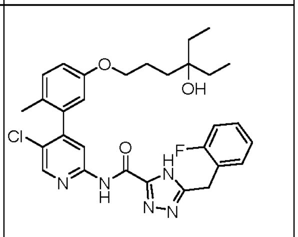 |
| 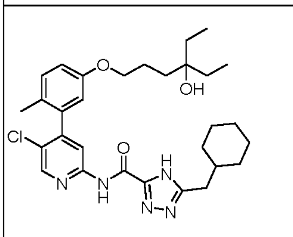 | 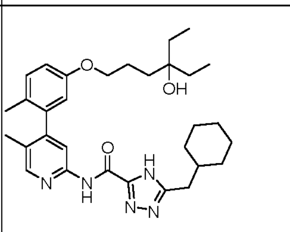 | 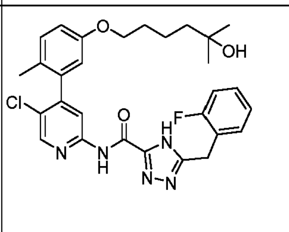 | 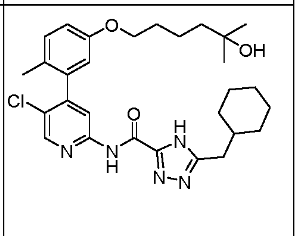 |
| 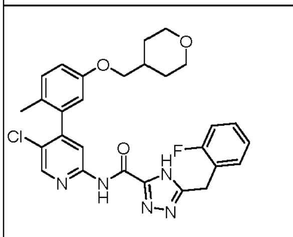 | 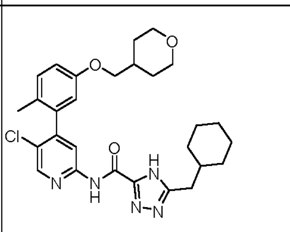 | 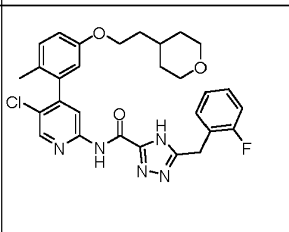 | 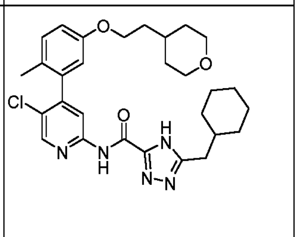 |
| 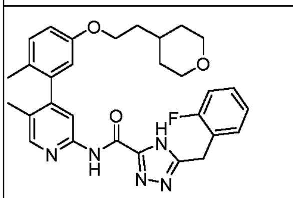 | 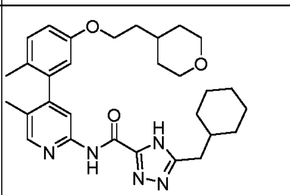 | 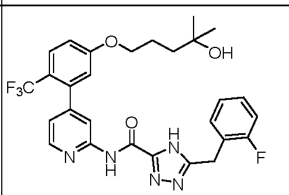 | 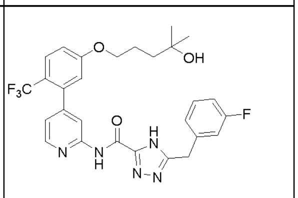 |
| 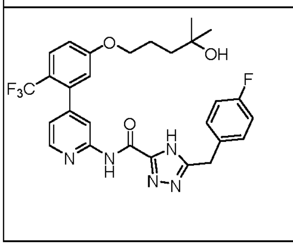 | 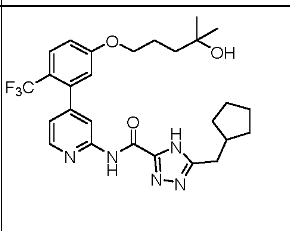 | 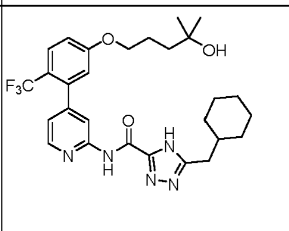 | 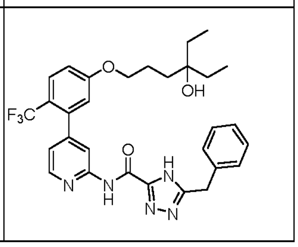 |

| | | | |
|---|---|---|---|
| 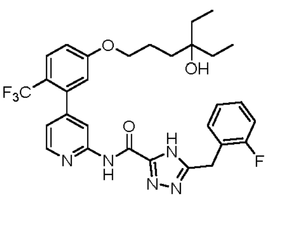 | 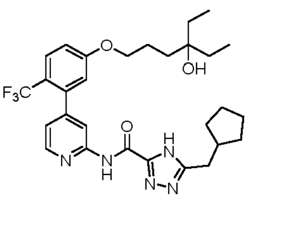 | 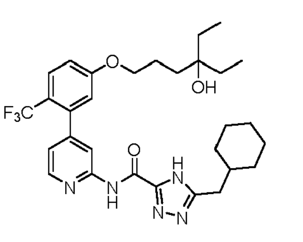 | 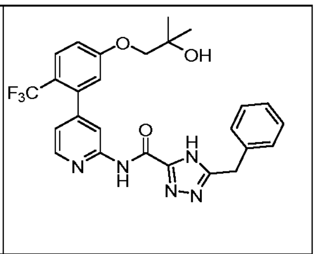 |
| 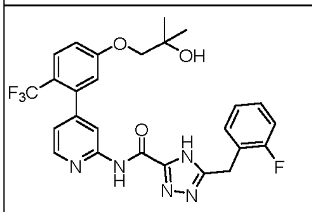 | 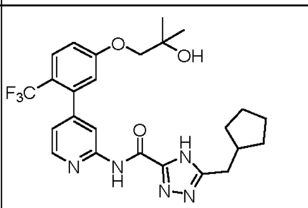 | 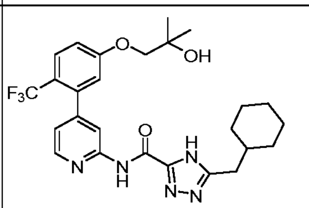 | 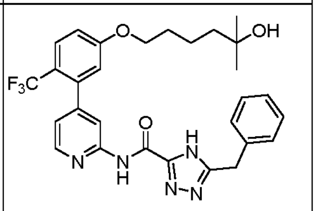 |
| 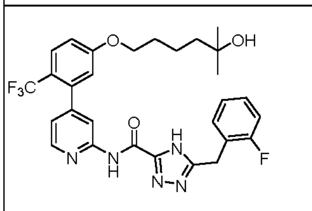 | 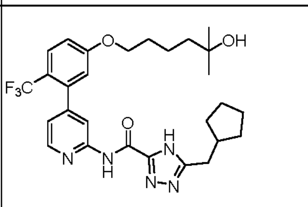 | 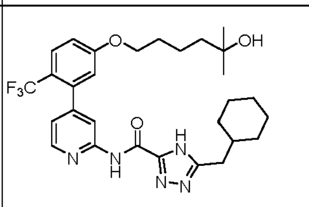 | 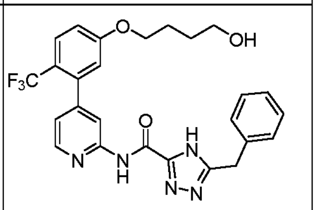 |
| 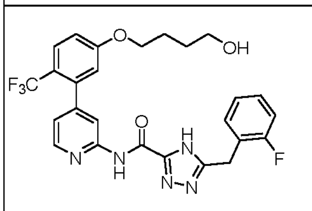 | 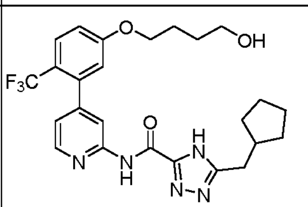 | 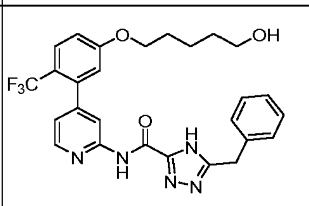 | 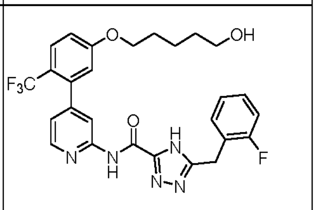 |
| 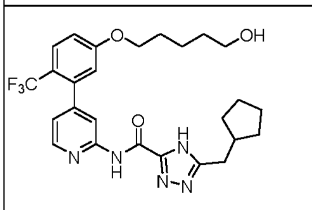 | 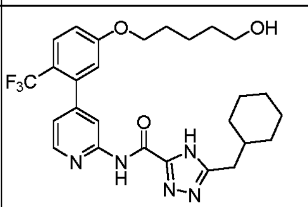 | 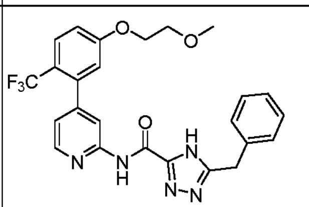 | 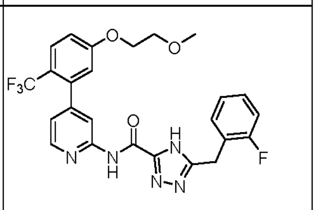 |
| 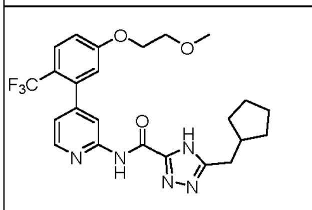 | 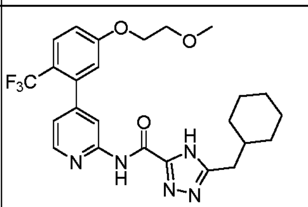 | 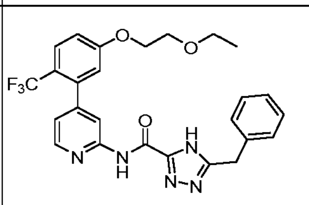 | 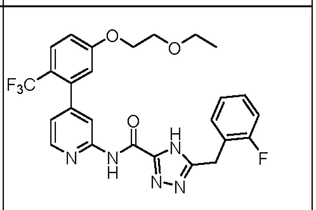 |
| 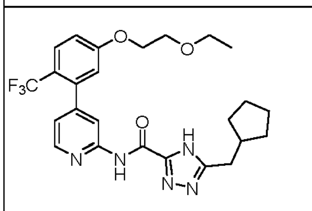 | 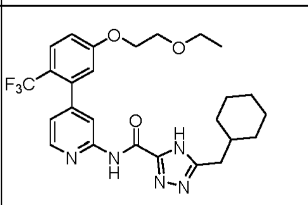 | 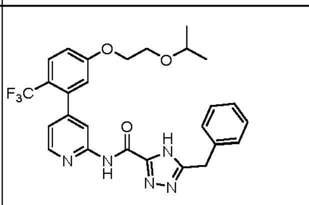 | 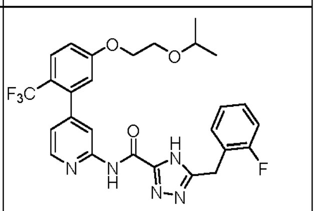 |
| 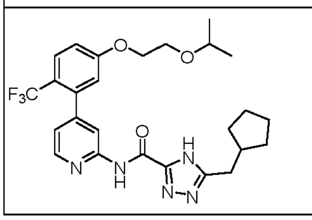 | 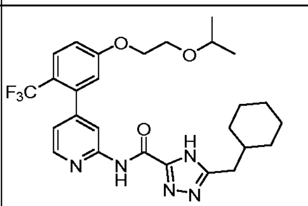 | 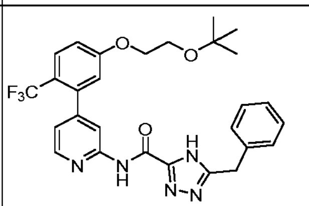 | 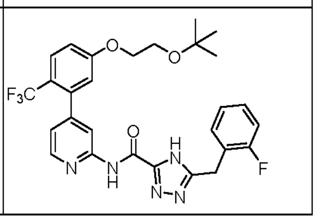 |

| | | | |
|---|---|---|---|
| 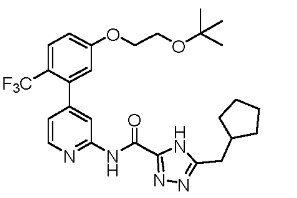 | 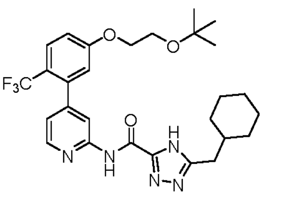 | 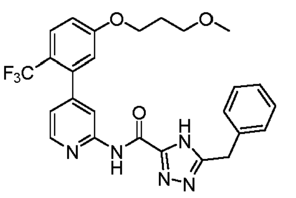 | 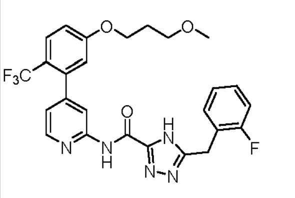 |
| 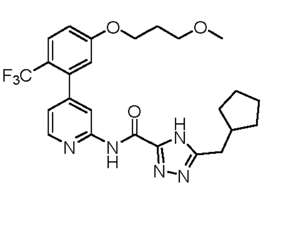 | 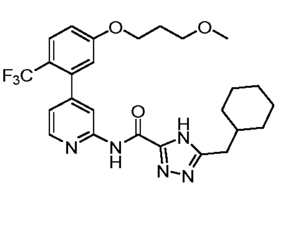 | 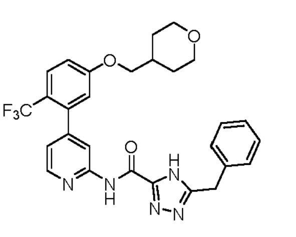 | 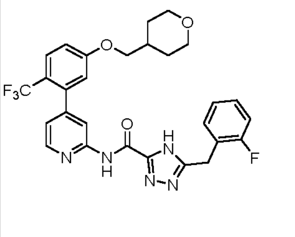 |
| 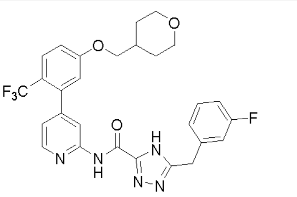 | 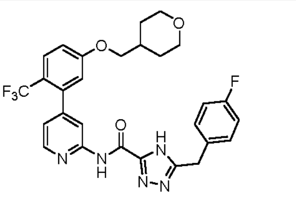 | 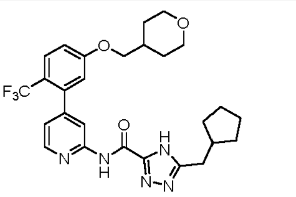 | 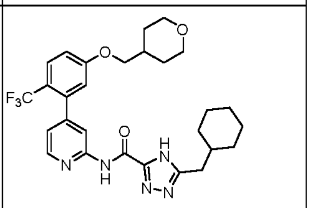 |
| 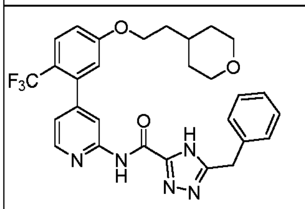 | 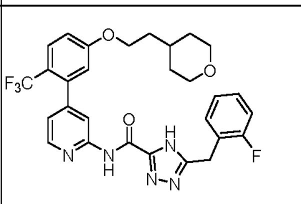 | 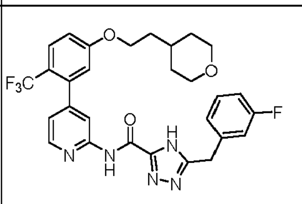 | 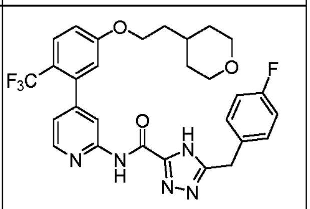 |
| 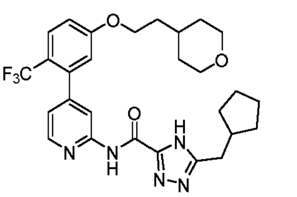 | 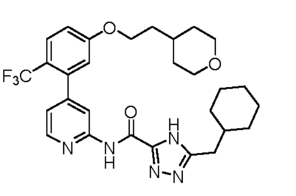 | 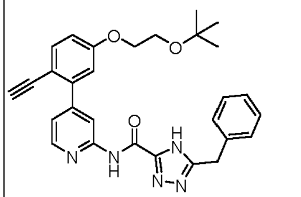 | 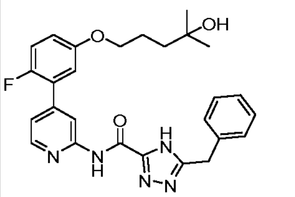 |
| 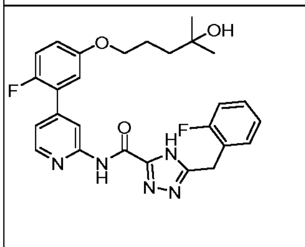 | 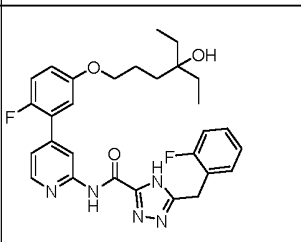 | 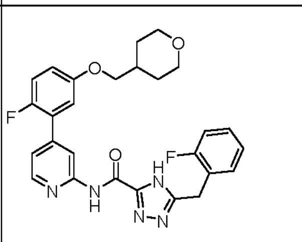 | 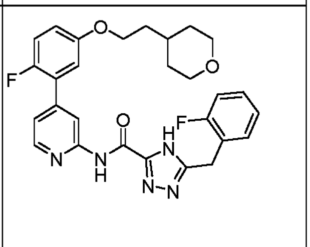 |
| 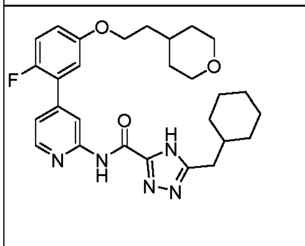 | 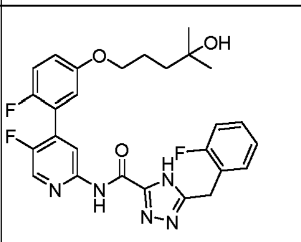 | 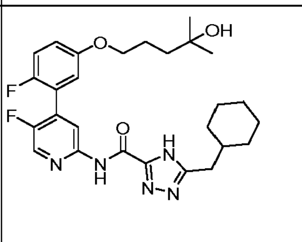 | 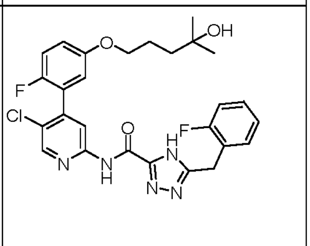 |
| 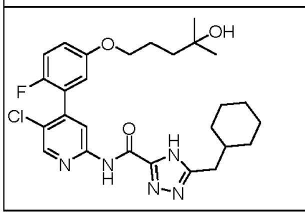 | 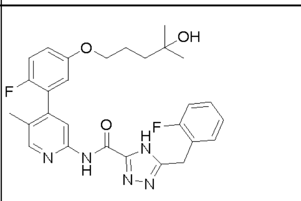 | 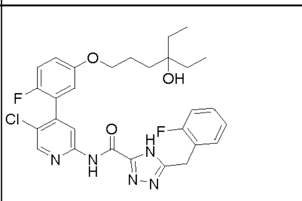 | 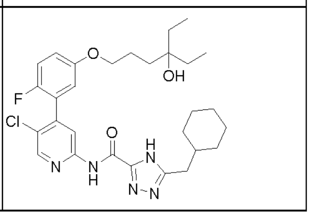 |

| | | | |
|---|---|---|---|
| 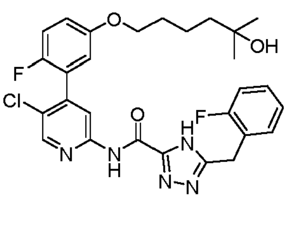 | 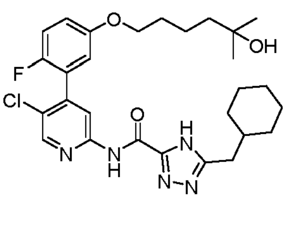 | 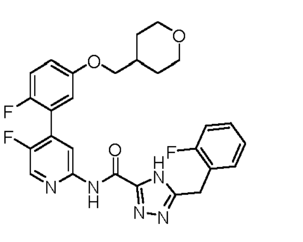 | 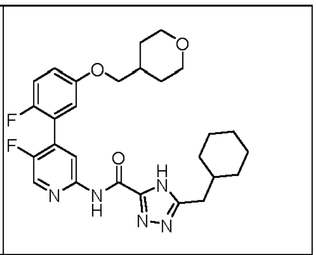 |
| 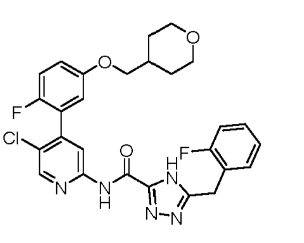 | 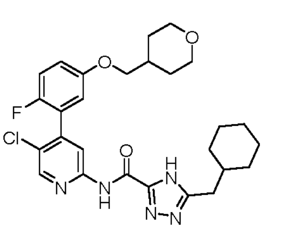 | 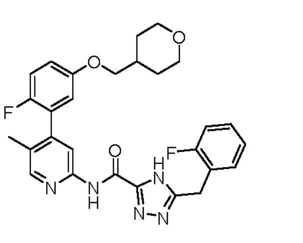 | 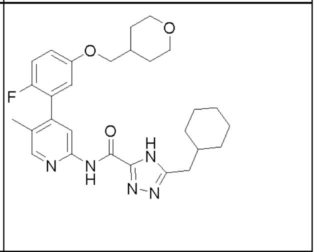 |
| 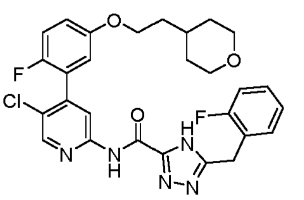 | 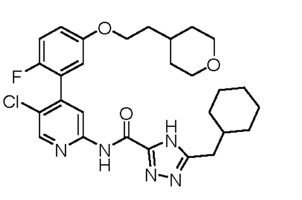 | 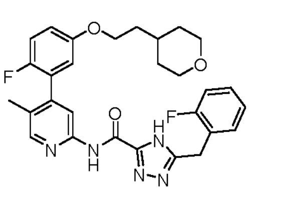 | 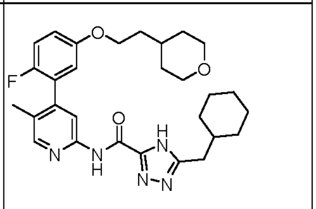 |
| 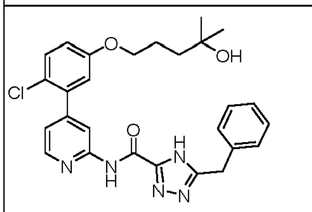 | 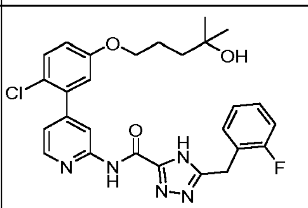 | 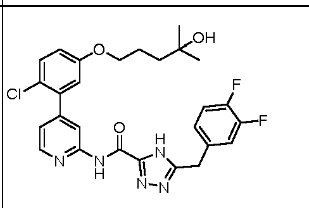 | 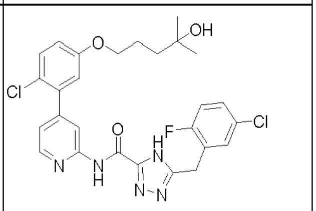 |
| 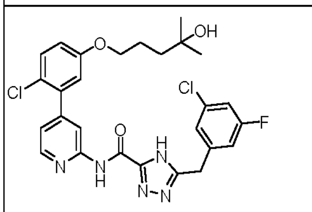 | 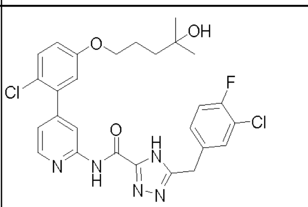 | 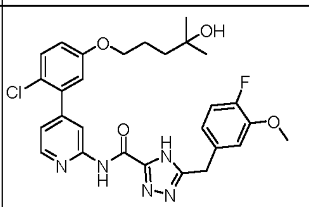 | 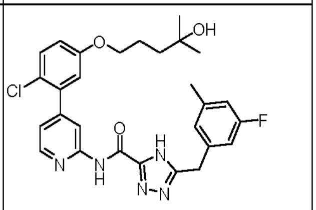 |
| 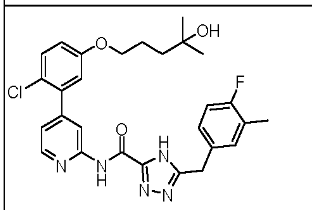 | 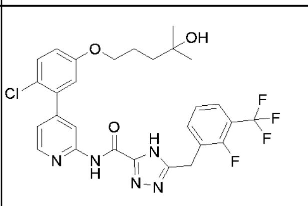 | 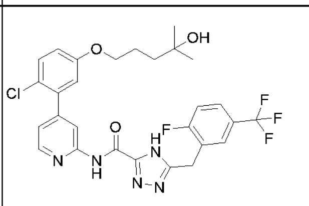 | 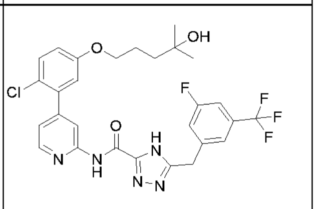 |
| 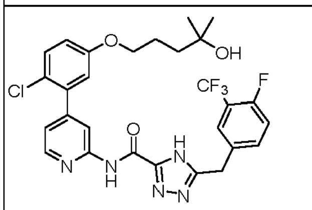 | 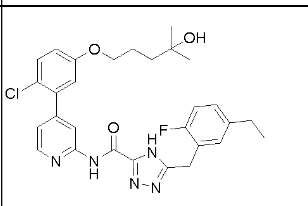 | 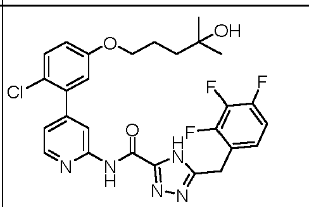 | 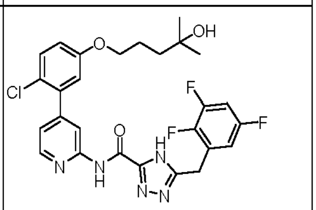 |
| 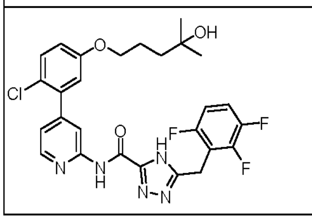 | 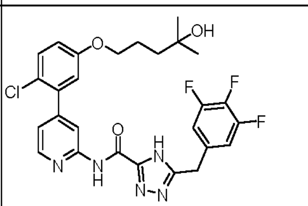 | 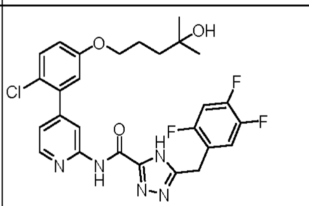 | 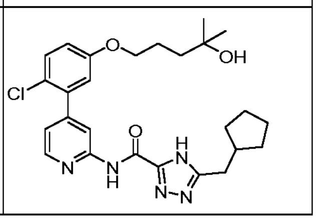 |

| | | | |
|---|---|---|---|
| 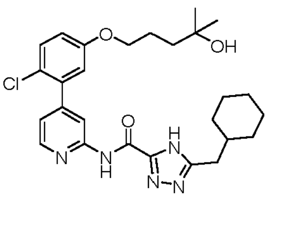 | 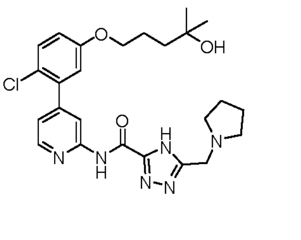 | 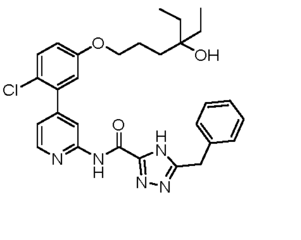 | 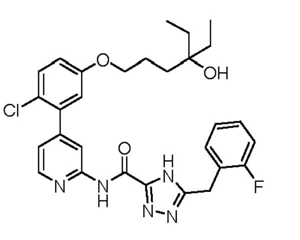 |
| 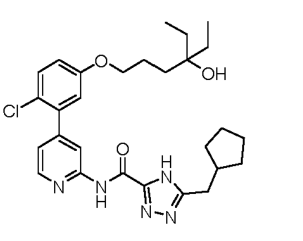 | 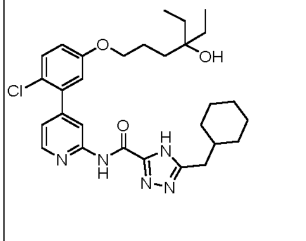 | 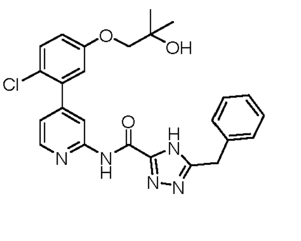 | 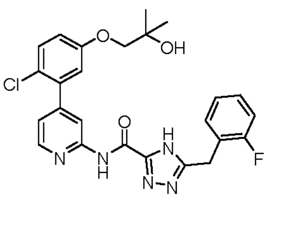 |
| 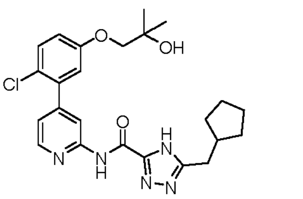 | 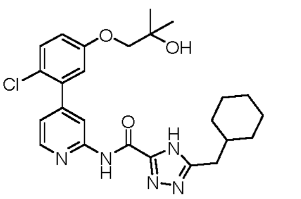 | 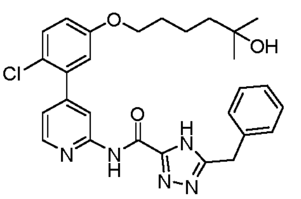 | 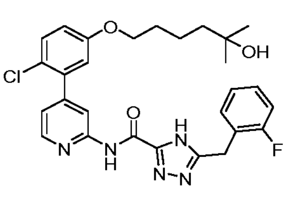 |
| 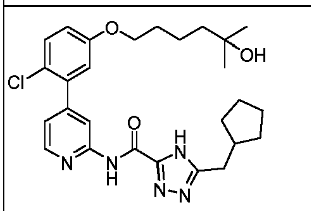 | 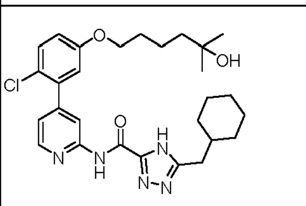 | 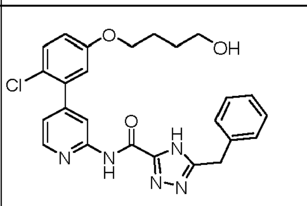 | 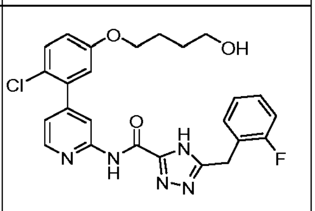 |
| 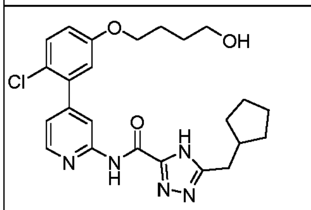 | 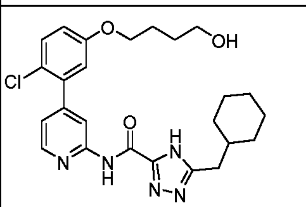 | 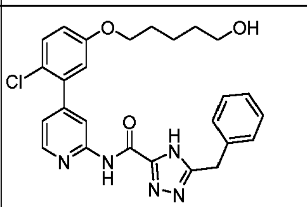 | 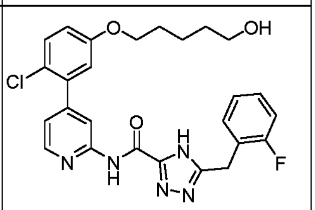 |
| 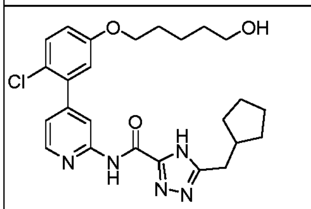 | 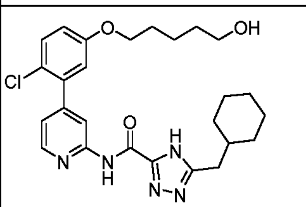 | 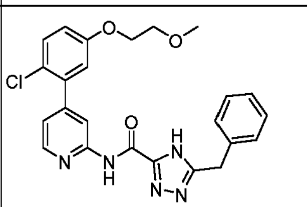 | 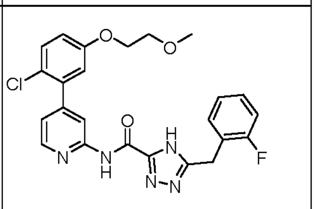 |
| 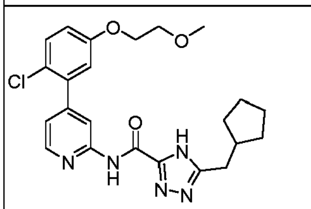 | 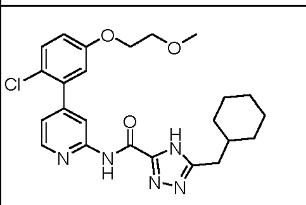 | 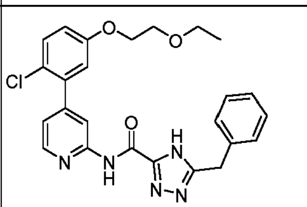 | 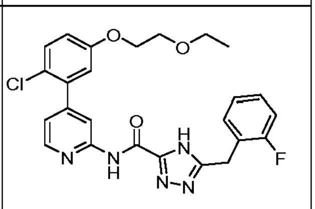 |
| 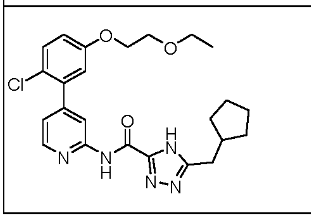 | 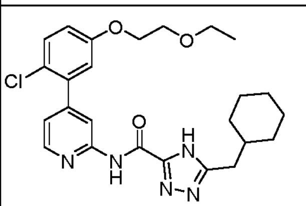 | 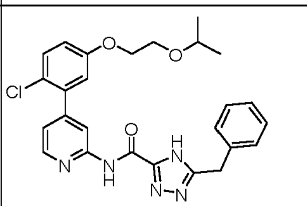 | 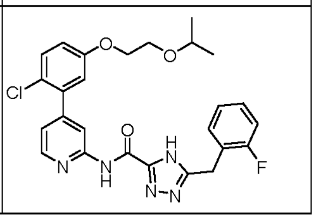 |

| | | | |
|---|---|---|---|
| 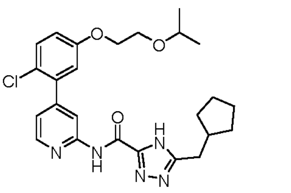 | 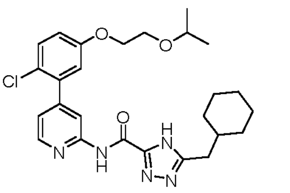 | 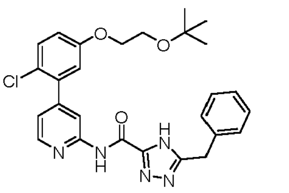 | 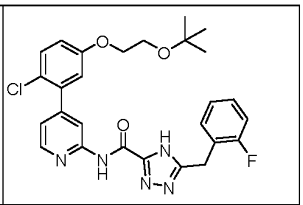 |
| 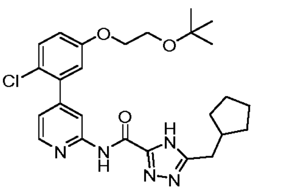 | 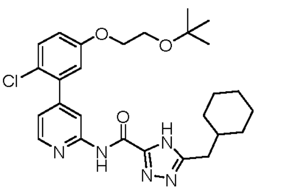 | 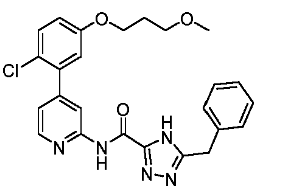 | 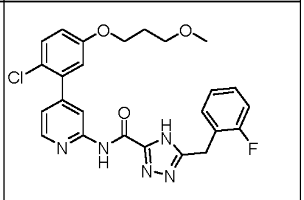 |
| 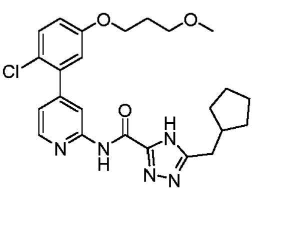 | 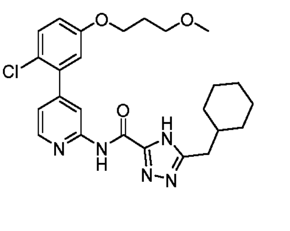 | 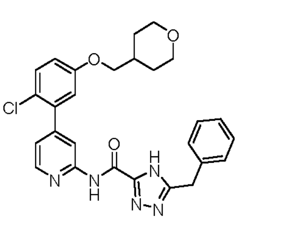 | 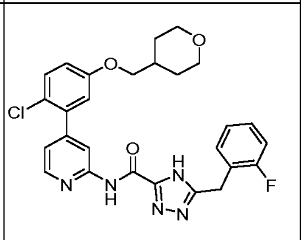 |
| 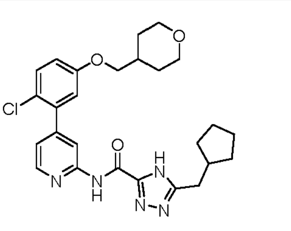 | 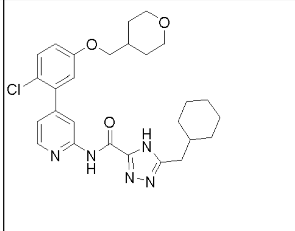 | 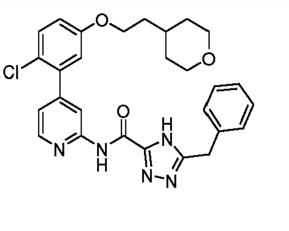 | 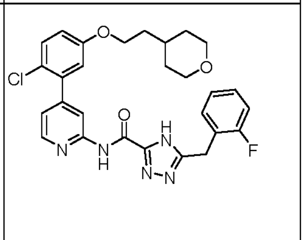 |
| 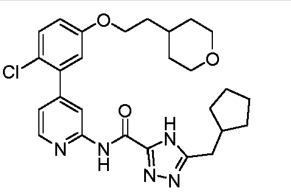 | 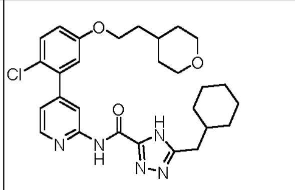 | 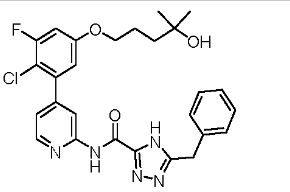 | 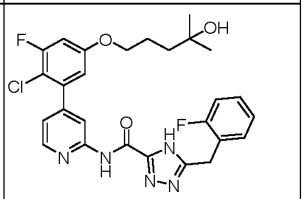 |
| 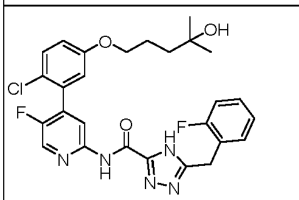 | 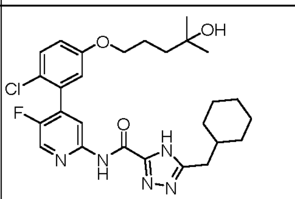 | 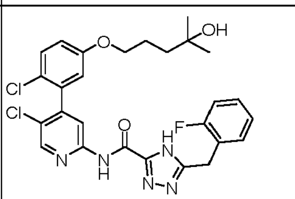 | 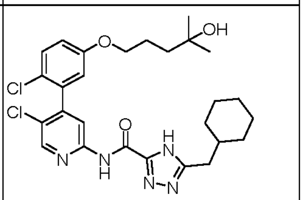 |
| 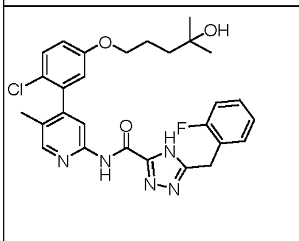 | 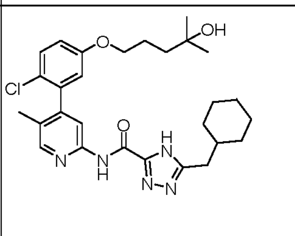 | 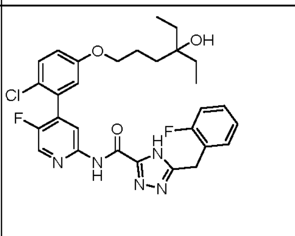 | 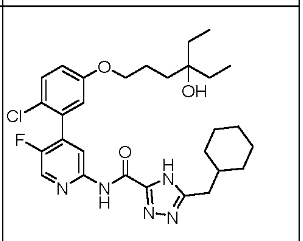 |
| 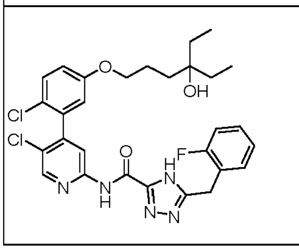 | 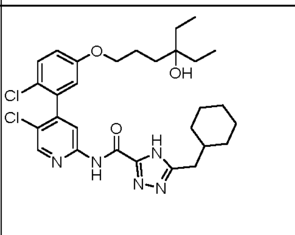 | 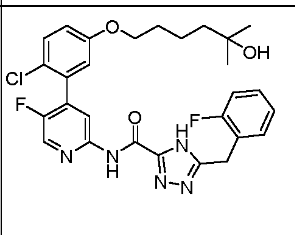 | 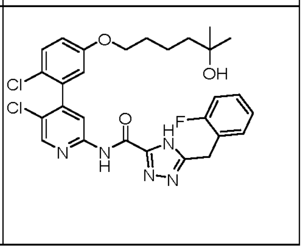 |

| | | | |
|---|---|---|---|
| 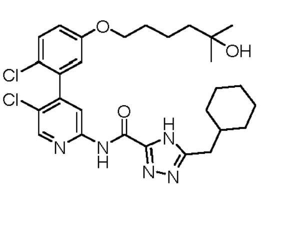 | 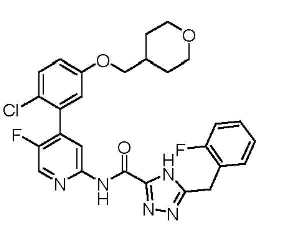 | 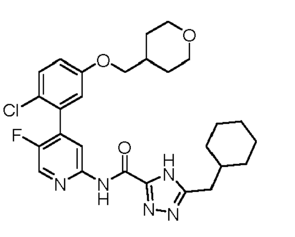 | 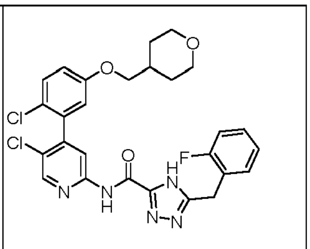 |
| 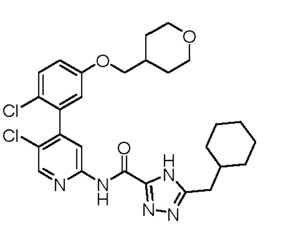 | 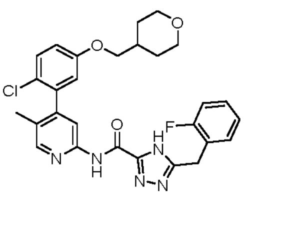 | 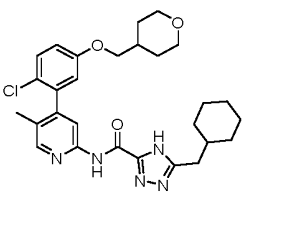 | 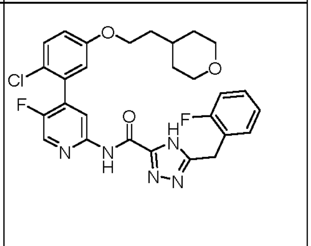 |
| 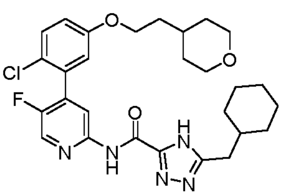 | 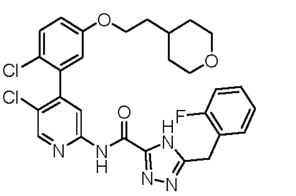 | 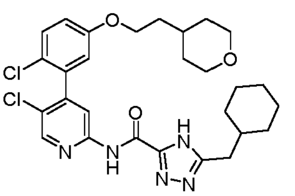 | 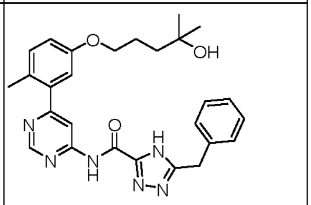 |
| 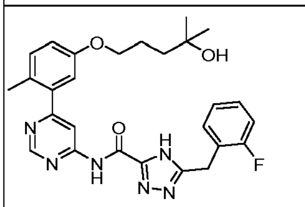 | 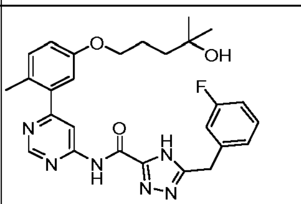 | 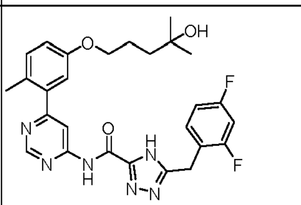 | 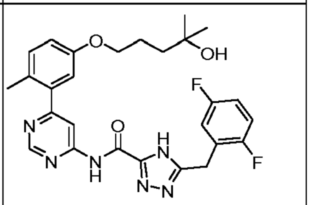 |
| 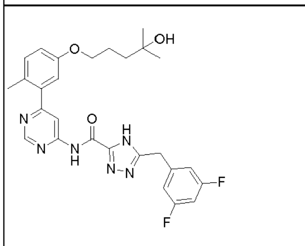 | 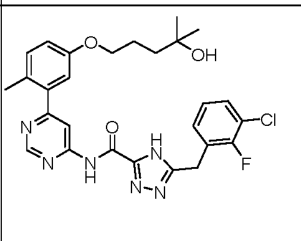 | 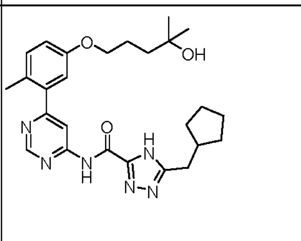 | 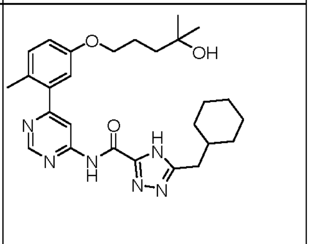 |
| 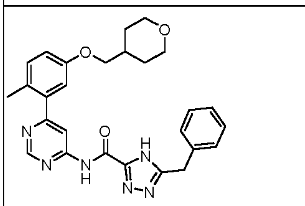 | 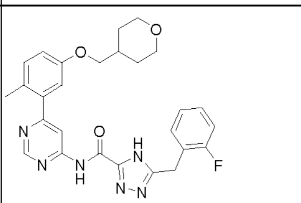 | 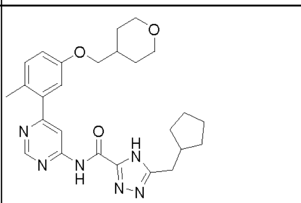 | 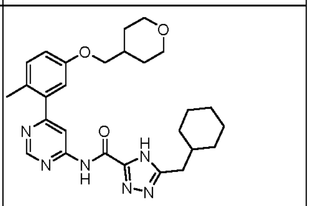 |
| 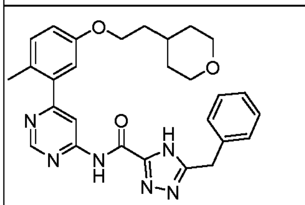 | 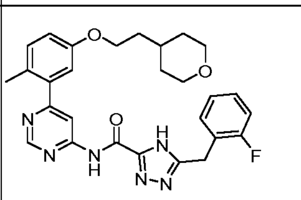 | 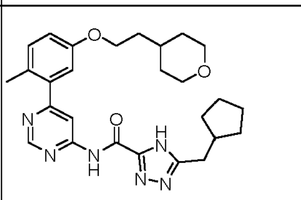 | 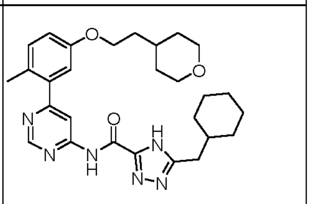 |
| 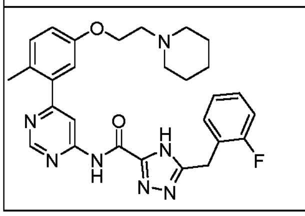 | 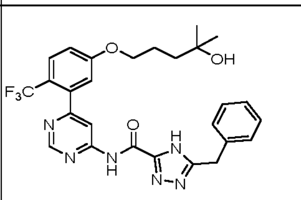 | 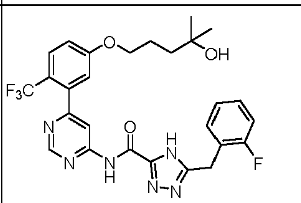 | 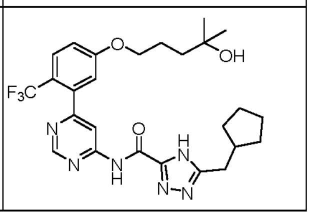 |

| | | | |
|---|---|---|---|
| 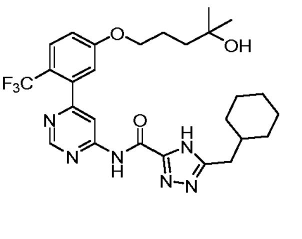 | 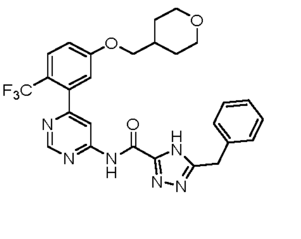 | 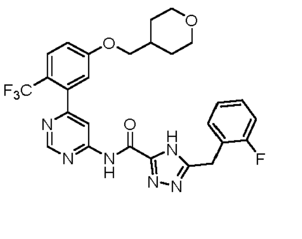 | 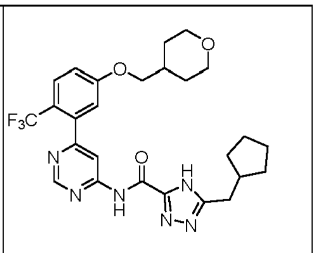 |
| 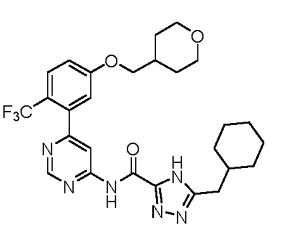 | 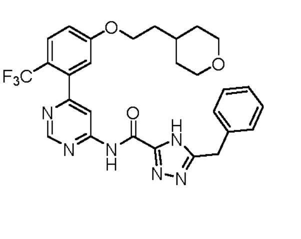 | 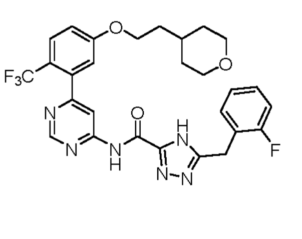 | 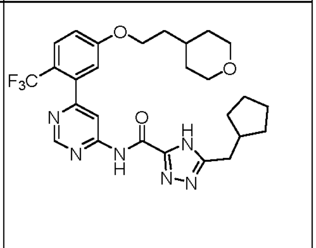 |
| 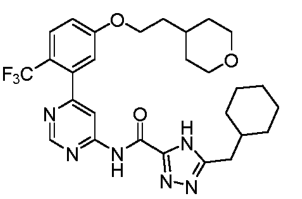 | 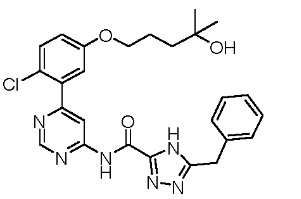 | 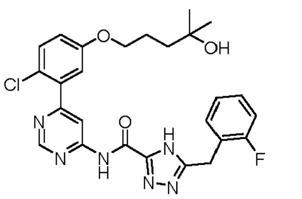 | 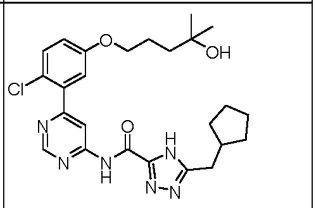 |
| 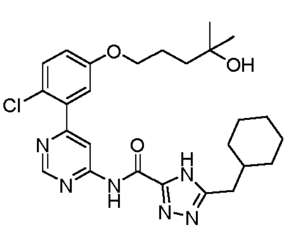 | 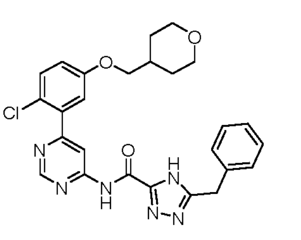 | 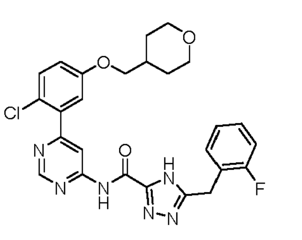 | 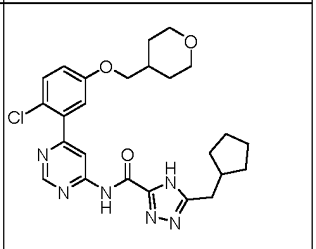 |
| 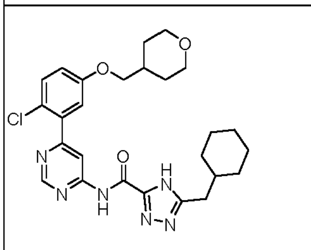 | 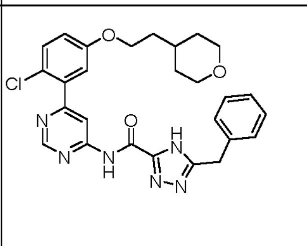 | 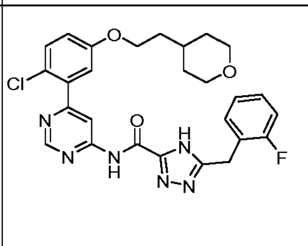 | 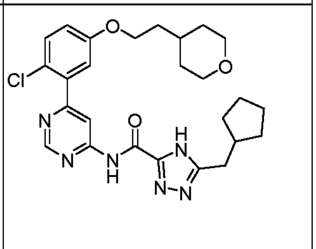 |
| 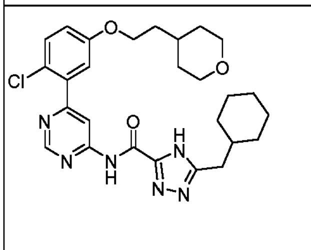 | 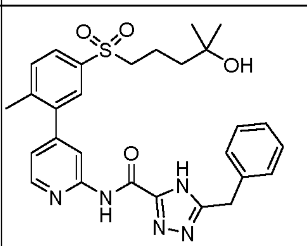 | 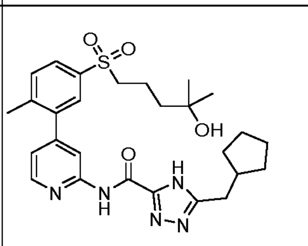 | 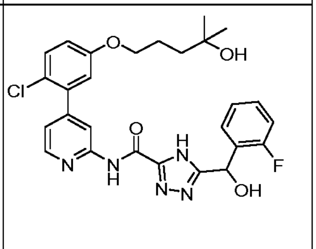 |
| 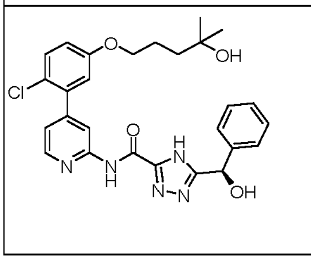 | 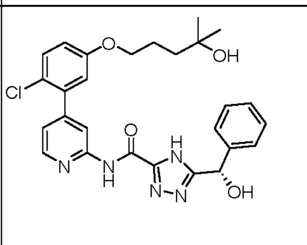 | 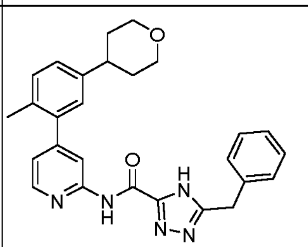 | 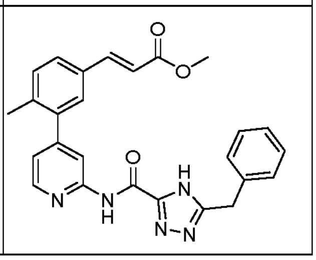 |

| | | | |
|---|---|---|---|
| 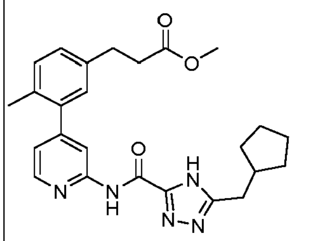 | 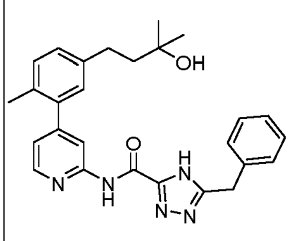 | 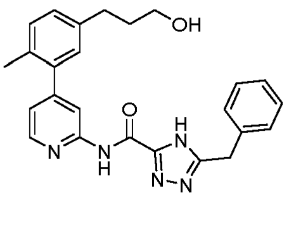 | 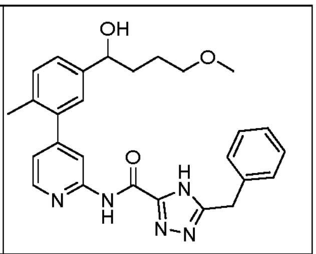 |
| 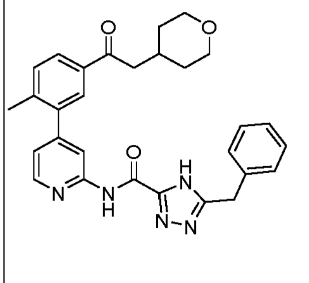 | 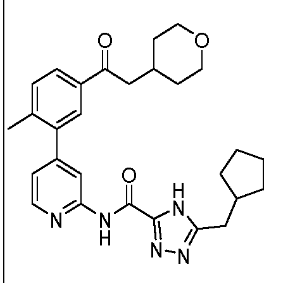 | 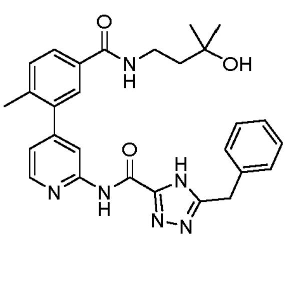 | 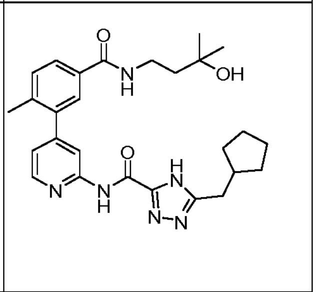 |
| 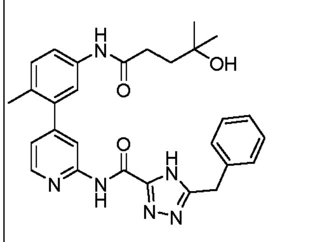 | 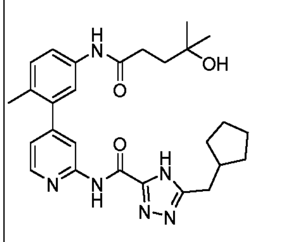 | 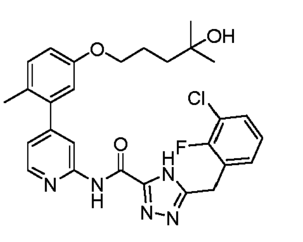 | 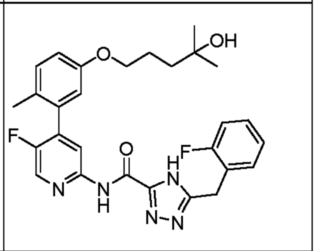 |
| 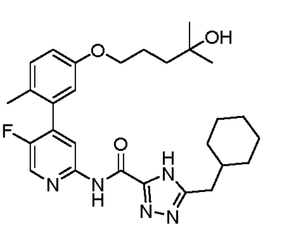 | 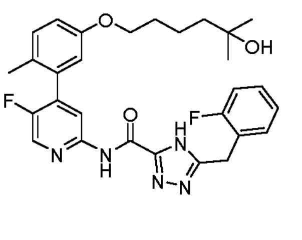 | 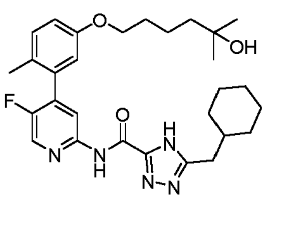 | 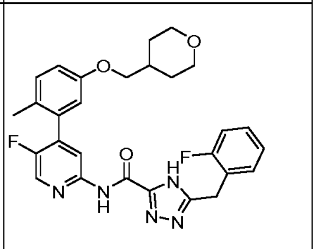 |
| 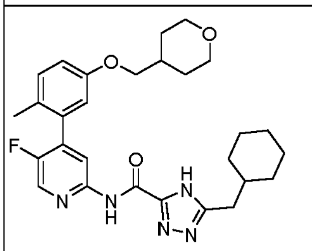 | 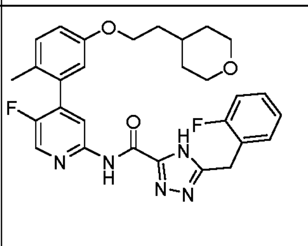 | 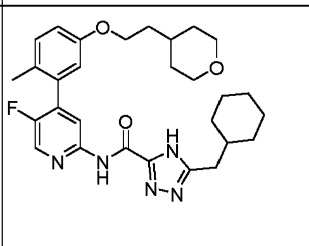 | 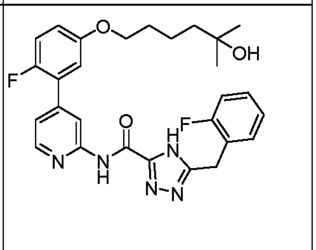 |
| 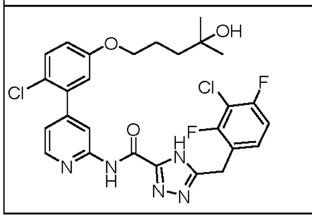 | 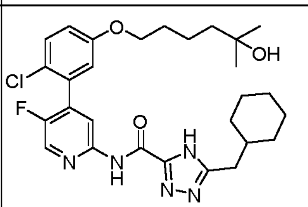 | 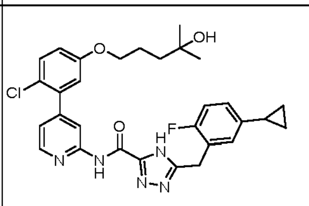 | 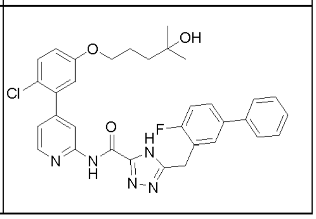 |

**13.** The compound, the pharmaceutically acceptable salts, diastereomers, enantiomers, or solvates thereof according to any one of claims 1-12, for use in the treatment for RIP1-related diseases, wherein, the RIP1-related disease includes ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic myeloid leukemia, acute myeloid leukemia, non-Hodgkin's lymphoma, nasopharyngeal cancer, esophageal cancer, brain tumor, B-cell and T-cell lymphoma, lymphoma, multiple myeloma, biliary cancer and sarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemiareperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behget's disease, interleukin-1 converzyme-related fever syndrome, chronic obstructive pulmonary disease, tumor necrosis factor receptor related periodic syndrome and periodontitis.

**14.** A pharmaceutical composition, comprising a compound, the pharmaceutically acceptable salts, diastereomers, enantiomers, or solvates thereof according to any one of claims 1-12, and one or more pharmaceutically acceptable carriers or excipients.

## Patentansprüche

**1.** Verbindung der Formel (I) oder die pharmazeutisch verträglichen Salze, die Diastereomere, die Enantiomere oder die Solvate davon,

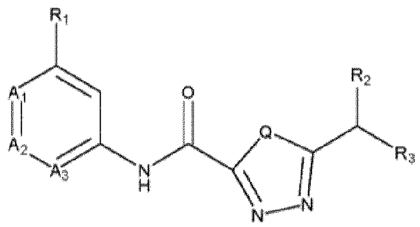

Formel (I)

in Formel (I),

Q ist NH, O oder S;
$A_1$, $A_2$ und $A_3$ sind jeweils unabhängig aus N oder $CR_4$ ausgewählt und mindestens einer von $A_1$, $A_2$ und $A_3$ ist N, $R_4$ ist H, F, Cl oder Methyl;
$R_1$ ist $C_3$-$C_8$-Cycloalkyl, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^aR^b$,
oder 4- bis 8-gliedrige heteroalicyclische Gruppe, die unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^aR^b$,
oder Aryl oder Heteroaryl, das unsubstituiert oder mit 1 bis 3 Substituenten substituiert ist, ausgewählt aus der Gruppe, bestehend aus $C_1$-$C_{10}$-Alkyl, Halogen, $C_3$-$C_8$-Cycloalkyl, halogeniertem $C_1$-$C_{10}$-Alkyl, Cyano, Hydroxyl, $C_1$-$C_6$-Alkylthio, -$SO_2$-$R_5$, -SO-$R_5$, -CO-$R_5$, -CONH-$R_5$, -NHCO-$R_5$, -R'-COO-R", - $NR^aR^b$, 4- bis 8-gliedriger heteroalicyclischer Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl und/oder $C_1$-$C_6$-Alkoxy, $C_3$-$C_8$-Cycloalkyl-$C_1$-$C_6$-alkyl, ($C_3$-$C_8$-Cycloalkyl)-O-($C_1$-$C_6$-alkyl), $C_1$-$C_6$-Alkyl, substituiert mit 4- bis 8-gliedriger heteroalicyclischer Gruppe, und -O-$R_6$,
die Arylgruppe ist eine monocyclische oder bicyclische Gruppe, die 6 bis 12 Kohlenstoffringatome enthält und mindestens einen aromatischen Ring aufweist, das Heteroaryl ist eine monocyclische oder bicyclische Gruppe mit 5 bis 10 Ringatomen und enthält 1 bis 3 Heteroatome, ausgewählt aus N, O oder S, als Ringatome, die 4- bis 8-gliedrige heteroalicyclische Gruppe ist eine 4- bis 8-gliedrige heteroalicyclische Gruppe, die 1 bis 2 Atome, ausgewählt aus N, O oder S, als Ringatome enthält,
$R_5$ ist Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe,
$R_6$ ist $C_1$-$C_{10}$-Alkyl, $C_3$-$C_8$-Cycloalkyl, 4- bis 8-gliedrige heteroalicyclische Gruppe oder $C_1$-$C_{10}$-Alkyl, das mit 1 bis 3 Substituenten substituiert ist, ausgewählt aus der Gruppe, bestehend aus Hydroxyl, $C_1$-$C_6$-Alkoxy, Cyano, -$NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl

und/oder $C_1$-$C_4$-Alkyl, Carboxyl, Halogen, halogeniertem $C_1$-$C_6$-Alkoxy, -$SO_2$-$R_5$, -SO-$R_5$, -CO-$R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, und $C_1$-$C_6$-Alkylthio,

R' ist $C_2$-$C_6$-Alkenylen oder $C_1$-$C_6$-Alkylen,

R" ist Wasserstoff, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe,

$R^a$ und $R^b$ sind jeweils unabhängig ausgewählt aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist;

$R_2$ ist $C_3$-$C_8$-Cycloalkyl, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$,

oder verbrücktes $C_7$-$C_{12}$-Cyclyl, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$,

oder $C_1$-$C_{10}$-Alkyl, das unsubstituiert oder mit 1 bis 3 Substituenten substituiert ist, ausgewählt aus der Gruppe, bestehend aus $C_1$-$C_6$-Alkoxy, $C_1$-$C_6$-Alkylthio, $C_1$-$C_3$-Acyl, Hydroxyl, Halogen, Cyano, -$CONH_2$, $C_3$-$C_8$-Cycloalkyl und -$NR^cR^d$, oder -$(CH_2)$n-$R^e$, wobei $R^e$ Aryl oder Heteroaryl ist, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, $C_3$-$C_6$-Cycloalkyl, Phenyl, Naphthyl, $C_2$-$C_6$-Alkinyl, $C_2$-$C_6$-Alkenyl und -$NR^cR^d$, wobei n eine ganze Zahl von 0 bis 3 ist,

oder -$(CH_2)$m-$R^f$, wobei $R^f$ 4- bis 8-gliedrige heteroalicyclische Gruppe ist, die unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$, m ist eine ganze Zahl von 0 bis 3,

die 4- bis 8-gliedrige heteroalicyclische Gruppe ist eine 4-bis 8-gliedrige heteroalicyclische Gruppe, die 1 bis 2 Atome, ausgewählt aus N, O oder S, als Ringatome enthält,

die Arylgruppe ist eine monocyclische oder bicyclische Gruppe, die 6 bis 12 Kohlenstoffringatome enthält und mindestens einen aromatischen Ring aufweist, das Heteroaryl ist eine monocyclische oder bicyclische Gruppe mit 5 bis 10 Ringatomen und enthält 1 bis 3 Heteroatome, ausgewählt aus N, O oder S, als Ringatome,

$R^c$ und $R^d$ sind jeweils unabhängig ausgewählt aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist; $R_3$ ist Wasserstoff, $C_1$-$C_3$-Alkyl, Hydroxyl, Halogen, Trifluormethyl oder Cyano.

**2.** Verbindung, die pharmazeutisch verträglichen Salze, Diastereomere, Enantiomere oder Solvate davon nach Anspruch 1, wobei Q NH, O oder S ist;

$A_1$, $A_2$ und $A_3$ jeweils unabhängig aus N oder $CR_4$ ausgewählt sind und mindestens einer von $A_1$, $A_2$ und $A_3$ N ist, $R_4$ H, F, Cl oder Methyl ist;

$R_1$ $C_3$-$C_8$-Cycloalkyl ist, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^aR^b$,

oder 4- bis 8-gliedrige heteroalicyclische Gruppe, die unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^aR^b$,

oder Aryl oder Heteroaryl, das unsubstituiert oder mit 1 bis 3 Substituenten substituiert ist, ausgewählt aus der Gruppe, bestehend aus $C_1$-$C_{10}$-Alkyl, Halogen, $C_3$-$C_8$-Cycloalkyl, halogeniertem $C_1$-$C_{10}$-Alkyl, Cyano, Hydroxyl, $C_1$-$C_6$-Alkylthio, -$SO_2$-$R_5$, -SO-$R_5$, -CO-$R_5$, -CONH-$R_5$, -$NR^aR^b$, 4- bis 8-gliedriger heteroalicyclischer Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl) -O-($C_1$-$C_6$-alkyl), $C_1$-$C_6$-Alkyl, substituiert mit 4- bis 8-gliedriger heteroalicyclischer Gruppe, und -O-$R_6$,

die Arylgruppe eine monocyclische oder bicyclische Gruppe ist, die 6 bis 12 Kohlenstoffringatome enthält und

mindestens einen aromatischen Ring aufweist, das Heteroaryl eine monocyclische oder bicyclische Gruppe mit 5 bis 10 Ringatomen aufweist und 1 bis 3 Heteroatome, ausgewählt aus N, O oder S, als Ringatome enthält, die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

$R_5$ Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe, ist,

$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl ist, das mit 1 bis 3 Substituenten substituiert ist, ausgewählt aus der Gruppe, bestehend aus Hydroxyl, $C_1$-$C_6$-Alkoxy, Cyano, -$NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl und/oder $C_1$-$C_4$-Alkyl Carboxyl, Halogen, halogeniertem $C_1$-$C_6$-Alkoxy, -$SO_2$-$R_5$, -SO-$R_5$, -CO-$R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, und $C_1$-$C_6$-Alkylthio,

$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind;

$R_2$ $C_3$-$C_8$-Cycloalkyl ist, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$,

oder verbrücktes $C_7$-$C_{12}$-Cyclyl, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$,

oder $C_1$-$C_{10}$-Alkyl, das unsubstituiert oder mit 1 bis 3 Substituenten substituiert ist, ausgewählt aus der Gruppe, bestehend aus $C_1$-$C_6$-Alkoxy, $C_1$-$C_6$-Alkylthio, $C_1$-$C_3$-Acyl, Hydroxyl, Halogen, Cyano, -$CONH_2$, $C_3$-$C_8$-Cycloalkyl und -$NR^cR^d$, oder -$(CH_2)$n-$R^e$, wobei $R^e$ Aryl oder Heteroaryl ist, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, $C_2$-$C_6$-Alkinyl, $C_2$-$C_6$-Alkenyl und -$NR^cR^d$, wobei n eine ganze Zahl von 0 bis 3 ist,

oder -$(CH_2)$m-$R^f$, wobei $R^f$ 4- bis 8-gliedrige heteroalicyclische Gruppe ist, die unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, Oxo (=O) und -$NR^cR^d$, m eine ganze Zahl von 0 bis 3 ist,

die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

die Arylgruppe eine monocyclische oder bicyclische Gruppe ist, die 6 bis 12 Kohlenstoffringatome enthält und mindestens einen aromatischen Ring aufweist, die Heteroarylgruppe eine monocyclische oder bicyclische Gruppe ist, die 5 bis 10 Ringatome aufweist und 1 bis 3 Heteroatome, ausgewählt aus N, O oder S, als Ringatome enthält,

$R^c$ und $R^d$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit einer 4- bis 8-gliedrigen heteroalicyclischen Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind;

$R_3$ Wasserstoff, $C_1$-$C_3$-Alkyl, Hydroxyl, Halogen, Trifluormethyl oder Cyano ist.

3. Verbindung, die pharmazeutisch verträglichen Salze, Diastereomere, Enantiomere oder Solvate davon nach Anspruch 1 oder 2, wobei $A_3$ N ist, $A_1$ und $A_2$ jeweils unabhängig aus N oder $CR_4$ ausgewählt sind, $R_4$ H, F, Cl oder Methyl ist.

4. Verbindung, die pharmazeutisch verträglichen Salze, Diastereomere, Enantiomere oder Solvate davon nach einem der Ansprüche 1-3, wobei Q NH ist.

5. Verbindung, die pharmazeutisch verträglichen Salze, Diastereomere, Enantiomere oder Solvate davon nach einem der Ansprüche 1 bis 4, wobei $R_1$ $C_3$-$C_8$-Cycloalkyl, 4- bis 8-gliedrige heteroalicyclische Gruppe oder Aryl oder Heteroaryl ist, das unsubstituiert oder mit 1 bis 3 Substituenten substituiert ist, ausgewählt aus der Gruppe, bestehend aus $C_1$-$C_{10}$-Alkyl, Halogen, $C_3$-$C_8$-Cycloalkyl, halogeniertem $C_1$-$C_{10}$-Alkyl, Cyano, Hydroxyl, $C_1$-$C_6$-Alkylthio, -CO-

$R_5$, $-SO_2-R_5$, $-SO-R_5$, $-CONH-R_5$, $-NR^aR^b$, 4- bis 8-gliedrige heteroalicyclische Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl)-O-($C_1$-$C_6$-alkyl), $C_1$-$C_6$-Alkyl, substituiert mit einer 4- bis 8-gliedrigen heteroalicyclischen Gruppe, und $-O-R_6$, die Arylgruppe Phenyl, Naphthyl,

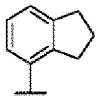 , 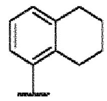

ist, die Heteroarylgruppe Pyrrolyl, Furyl, Pyridyl, Thienyl, Imidazolyl, Thiazolyl, Isothiazolyl, Indazolyl, Indolyl, Isoindolyl, Indolinyl, Isoindolinyl, Isochinolinyl, Indolizinyl, Isoxazolyl, 1,5-Naphthyridinyl, 1,6-Naphthyridinonyl, Oxadiazolyl, Oxazolyl, 1-Phenyl-1H-pyrrolyl, Phenazinyl, Phenothiazinyl, Phenoxazinyl, Phthalazinyl, Pteridinyl, Purinyl, Pyranyl, Pyrazolyl, Pyrazolo[3,4-d]pyrimidinyl, Pyrido[3,2-d]pyrimidinyl, Pyrido[3,4-d]pyrimidinyl, Pyrazinyl, Pyrimidinyl, Pyridazinyl, Chinazolinyl, Chinoxalinyl, Chinolinyl,

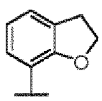 , 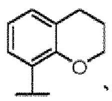 , 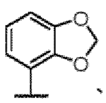 , 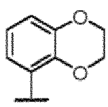 , 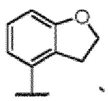 , 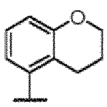

ist, die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

$R_5$ Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe, ist,
$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl, das unsubstituiert oder mit 1 bis 3 Substituenten substituiert ist, ausgewählt aus der Gruppe, bestehend aus Hydroxyl, $C_1$-$C_6$-Alkoxy, Cyano, $-NR^aR^b$, $C_3$-$C_8$-Cycloalkyloxy, $-CONH-R_5$, $C_3$-$C_8$-Cycloalkyl, $C_3$-$C_8$-Cycloalkyl, substituiert mit Hydroxyl und/oder $C_1$-$C_4$-Alkyl, Carboxyl, Halogen, halogeniertem $C_1$-$C_6$-Alkoxy, $-CO-R_5$, $-SO_2-R_5$, $-SO-R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, oder $C_1$-$C_6$-Alkylthio, ist,
$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-Alkyl substituiert ist, ausgewählt sind;
vorzugsweise, wobei $R_1$ Cyclopropyl, Cyclobutyl, Cyclopentyl, Cyclohexyl, Oxetan-3-yl, Tetrahydrofuran-3-yl, Tetrahydropyran-4-yl, Tetrahydropyran-3-yl, Pyrrolidinyl, Piperidinyl, Piperazinyl, Morpholinyl, Thiomorpholinyl, oder Aryl oder Heteroaryl ist, das unsubstituiert oder mit 1 bis 3 Substituenten substituiert ist, ausgewählt aus der Gruppe, bestehend aus $C_1$-$C_{10}$-Alkyl, Halogen, $C_3$-$C_8$-Cycloalkyl, halogeniertem $C_1$-$C_{10}$-Alkyl, Cyano, Hydroxyl, $C_1$-$C_6$-Alkylthio, $-SO_2-R_5$, $-SO-R_5$, $-CO-R_5$, $-CONH-R_5$, $-NR^aR^b$, 4- bis 8-gliedriger heteroalicyclischer Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl) -O-($C_1$-$C_6$-alkyl), $C_1$-$C_6$-Alkyl, substituiert mit 4- bis 8-gliedriger heteroalicyclischer Gruppe und $-O-R_5$,
die Arylgruppe Phenyl,

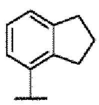 , 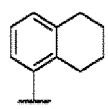

ist, die Heteroarylgruppe Pyrazolyl, Pyridyl, Pyrimidinyl, Thiazolyl, Oxazolyl,

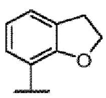, 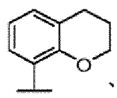, 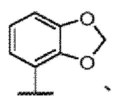, 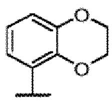, 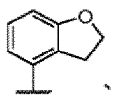, 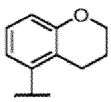

ist, die 4- bis 8-gliedrige heteroalicyclische Gruppe eine 4- bis 8-gliedrige heteroalicyclische Gruppe, die 1 bis 2 Atome, ausgewählt aus N, O oder S, als Ringatome enthält, ist,

$R_5$ Wasserstoff, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe, ist,

$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl, das unsubstituiert oder mit 1 bis 3 Substituenten substituiert ist, ausgewählt aus der Gruppe, bestehend aus Hydroxyl, $C_1$-$C_6$-Alkoxy, Cyano, -$NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl und/oder $C_1$-$C_4$-Alkyl, Carboxyl, Halogen, halogeniertem $C_1$-$C_6$-Alkoxy, -$SO_2$-$R_5$, -SO-$R_5$, -CO-$R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, und $C_1$-$C_6$-Alkylthio, ist,

$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind;

vorzugsweise, wobei $R_1$

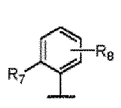, 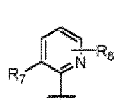, 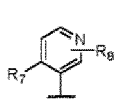, 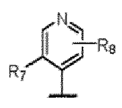, 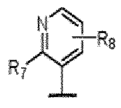, 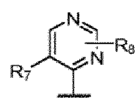, 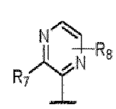

oder

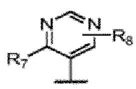

ist,

$R_7$ Wasserstoff, $C_1$-$C_4$-Alkyl, $C_1$-$C_3$-Alkoxy, Halogen, Hydroxyl, Trifluormethyl, Trifluormethoxy, Cyano, $C_2$-$C_4$-Alkinyl, $C_2$-$C_4$-Alkenyl, $C_3$-$C_4$-Cycloalkyl oder $C_1$-$C_3$-Acyl ist;

$R_8$ Wasserstoff, $C_1$-$C_{10}$-Alkyl, Halogen, $C_3$-$C_8$-Cycloalkyl, halogeniertes $C_1$-$C_{10}$-Alkyl, Cyano, Hydroxyl, $C_1$-$C_6$-Alkylthio, -CO-$R_5$, -$SO_2$-$R_5$, -SO-$R_5$, -CONH-$R_5$, -$NR^aR^b$, 4- bis 8-gliedrige heteroalicyclische Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl)-O-($C_1$-$C_6$-alkyl), $C_1$-$C_6$-Alkyl, substituiert mit 4- bis 8-gliedriger heteroalicyclischer Gruppe, oder -O-$R_6$ ist,

die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

$R_5$ Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe, ist,

$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl ist, das unsubstituiert oder mit 1 bis 3 Substituenten substituiert ist, ausgewählt aus der Gruppe, bestehend aus Hydroxyl, $C_1$-$C_6$-Alkoxy, Cyano, -$NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl und/oder $C_1$-$C_4$-Alkyl, Carboxyl, Halogen, halogeniertem $C_1$-$C_6$-Alkoxy, -$SO_2$-$R_5$, -SO-$R_5$, -CO-$R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, $C_1$-$C_6$-Alkylthio, ist,

$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind;

vorzugsweise, wobei $R_1$

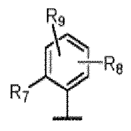, 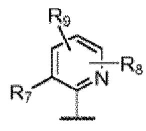, 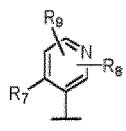, 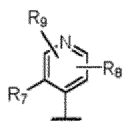, 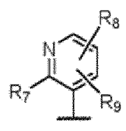, 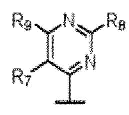,

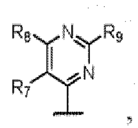, 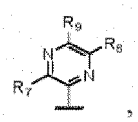, 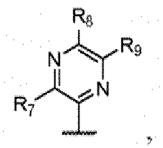, 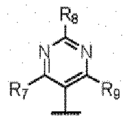 oder 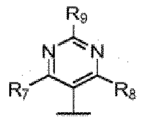 ist,

$R_7$ Wasserstoff, $C_1$-$C_4$-Alkyl, $C_1$-$C_3$-Alkoxy, Halogen, Hydroxyl, Trifluormethyl, Trifluormethoxy, Cyano, $C_2$-$C_4$-Alkinyl, $C_2$-$C_4$-Alkenyl, $C_3$-$C_4$-Cycloalkyl oder $C_1$-$C_3$-Acyl ist;

$R_8$ Wasserstoff, $C_1$-$C_{10}$-Alkyl, Halogen, $C_3$-$C_8$-Cycloalkyl, halogeniertes $C_1$-$C_{10}$-Alkyl, Cyano, Hydroxyl, $C_1$-$C_6$-Alkylthio, -CO-$R_5$, -$SO_2$-$R_5$, -SO-$R_5$, -CONH-$R_5$, -$NR^aR^b$, 4- bis 8-gliedrige heteroalicyclische Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl)-O-($C_1$-$C_6$-alkyl), $C_1$-$C_6$-Alkyl, substituiert mit 4- bis 8-gliedriger heteroalicyclischer Gruppe, oder -O-$R_6$ ist,

die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

$R_5$ Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl, $C_3$-$C_8$-Cycloalkyl oder $C_1$-$C_6$-Alkyl, substituiert mit 4- bis 8-gliedriger heteroalicyclischer Gruppe, ist,

$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl, das unsubstituiert oder mit 1 bis 3 Substituenten substituiert ist, ausgewählt aus der Gruppe, bestehend aus Hydroxyl, $C_1$-$C_6$-Alkoxy, Cyano, -$NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl und/oder $C_1$-$C_4$-Alkyl, Carboxyl, Halogen, halogeniertem $C_1$-$C_6$-Alkoxy, -$SO_2$-$R_5$, -SO-$R_5$, -CO-$R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4- bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, und $C_1$-$C_6$-Alkylthio ist,

$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind;

$R_9$ Wasserstoff, $C_1$-$C_4$-Alkyl, $C_1$-$C_3$-Alkoxy, Halogen, Hydroxy, Trifluormethyl, Trifluormethoxy, Cyano, $C_2$-$C_4$-Alkinyl, $C_2$-$C_4$-Alkenyl, $C_3$-$C_4$-Cycloalkyl oder $C_1$-$C_3$-Acyl ist;

vorzugsweise, wobei $R_1$:

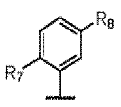, 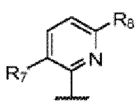, 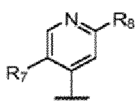, 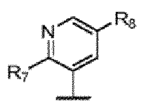, 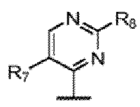 oder 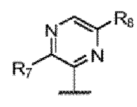

ist,

$R_7$ Wasserstoff, Methyl, Ethyl, Propyl, Isopropyl, Methoxy, Ethoxy, Propoxy, Isopropoxy, Fluor, Chlor, Hydroxyl, Trifluormethyl, Trifluormethoxy, Cyano, Ethinyl, Propinyl, Vinyl, Propenyl, Cyclopropyl, Cyclobutyl, Formyl oder Acetyl ist;

$R_8$ Wasserstoff, $C_1$-$C_{10}$-Alkyl, Halogen, $C_3$-$C_8$-Cycloalkyl, halogeniertes $C_1$-$C_{10}$-Alkyl, Cyano, Hydroxyl, $C_1$-$C_6$-Alkylthio, -CO-$R_5$, -$SO_2$-$R_5$, -SO-$R_5$, -CONH-$R_5$, -$NR^aR^b$, 4- bis 8-gliedrige heteroalicyclische Gruppe, $C_2$-$C_6$-Alkinyl, $C_2$-$C_6$-Alkenyl, $C_1$-$C_3$-Alkoxy-$C_1$-$C_6$-alkylthio, $C_1$-$C_{10}$-Alkyl, substituiert mit Hydroxyl, $C_1$-$C_6$-Alkoxy-$C_1$-$C_{10}$-alkyl, $C_3$-$C_8$-Cycloalkyl-$C_1$-$C_6$-alkyl, ($C_3$-$C_8$-Cycloalkyl) -O-($C_1$-$C_6$-alkyl), $C_1$-$C_6$-Alkyl, substituiert mit 4- bis 8-gliedriger heteroalicyclischer Gruppe, oder -O-$R_6$ ist,

die 4- bis 8-gliedrige heteroalicyclische Gruppe eine 4- bis 8-gliedrige heteroalicyclische Gruppe ist, die 1 bis 2 Atome, ausgewählt aus N, O oder S, als Ringatome enthält,

$R_5$ Wasserstoff, Hydroxyl, $C_1$-$C_6$-Alkyl, $C_1$-$C_6$-Alkoxy-$C_1$-$C_6$-alkyl, $C_1$-$C_6$-Alkyl, substituiert mit Hydroxyl,

$C_3$-$C_8$-Cycloalkyl, $C_1$-$C_6$-Alkyl, substituiert mit 4- bis 8-gliedriger heteroalicyclischer Gruppe, ist,

$R_6$ $C_1$-$C_{10}$-Alkyl, $C_3$-$C_8$-Cycloalkyl, 4- bis 8-gliedrige heteroalicyclische Gruppe oder $C_1$-$C_{10}$-Alkyl, substituiert mit 1 bis 3 Substituenten, ausgewählt aus Hydroxyl, $C_1$-$C_6$-Alkoxy, Cyano, $-NR^aR^b$, $C_3$-$C_8$-Cycloalkyloxy, -CONH-$R_5$, $C_3$-$C_8$-Cycloalkyl, $C_3$-$C_8$-Cycloalkyl, substituiert mit Hydroxyl und/oder $C_1$-$C_4$-Alkyl, Carboxyl, Halogen, halogeniertem $C_1$-$C_6$-Alkoxy, $-SO_2-R_5$, $-SO-R_5$, $-CO-R_5$, $C_2$-$C_6$-Alkinyl, $C_2$-$C_6$-Alkenyl, $C_1$-$C_4$-Alkoxy-$C_1$-$C_6$-alkoxy, 4- bis 8-gliedriger heteroalicyclischer Gruppe, 4-bis 8-gliedriger heteroalicyclischer Gruppe, substituiert mit Oxo, 4- bis 8-gliedriger heteroalicyclischer Gruppe, substituiert mit Hydroxyl und/oder $C_1$-$C_4$-Alkyl, und $C_1$-$C_6$-Alkylthio ist,

$R^a$ und $R^b$ jeweils unabhängig aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist, ausgewählt sind.

**6.** Verbindung, die pharmazeutisch verträglichen Salze, Diastereomere, Enantiomere oder Solvate davon nach einem der Ansprüche 1 bis 5, wobei $R_2$ $C_3$-$C_8$-Cycloalkyl ist, das unsubstituiert oder mit 1 bis 3 Substituenten substituiert ist, ausgewählt aus der Gruppe, bestehend aus Methyl, Ethyl, Propyl, Isopropyl, Methoxy, Ethoxy, Propoxy, Isopropoxy, Methylthio, Ethylthio, Propylthio, Formyl, Acetyl, Hydroxyl, Fluor, Chlor, Trifluormethyl, Cyano, $-CONH_2$, Oxo (=O), Amino, Dimethylamino und Diethylamino,

oder verbrücktes $C_7$-$C_{10}$-Cyclyl, das unsubstituiert oder mit 1 bis 3 Substituenten substituiert ist, ausgewählt aus der Gruppe, bestehend aus Methyl, Ethyl, Propyl, Isopropyl, Methoxy, Ethoxy, Propoxy, Isopropoxy, Methylthio, Ethylthio, Propylthio, Formyl, Acetyl, Hydroxyl, Fluor, Chlor, Trifluormethyl, Cyano, $-CONH_2$, Oxo (=O), Amino, Dimethylamino und Diethylamino,

oder $C_1$-$C_{10}$-Alkyl, das unsubstituiert oder mit 1 bis 3 Substituenten substituiert ist, ausgewählt aus der Gruppe, bestehend aus Methoxy, Ethoxy, Propoxy, Isopropoxy, Methylthio, Ethylthio, Propylthio, Formyl, Acetyl, Hydroxyl, Fluor, Chlor, Cyano, $-CONH_2$, Cyclopropyl, Cyclobutyl, Cyclopentyl, Cyclohexyl, Amino, Dimethylamino und Diethylamino,

oder $-(CH_2)n-R^e$, wobei $R^e$ Aryl oder Heteroaryl ist, das unsubstituiert oder mit 1 bis 3 Substituenten substituiert ist, ausgewählt aus der Gruppe, bestehend aus Methyl, Ethyl, Propyl, Isopropyl, Methoxy, Ethoxy, Propoxy, Isopropoxy, Methylthio, Ethylthio, Propylthio, Formyl, Acetyl, Hydroxyl, Fluor, Chlor, Trifluormethyl, Cyano, $-CONH_2$, Ethinyl, Vinyl, Amino, Dimethylamino und Diethylamino, n eine ganze Zahl von 0 bis 3 ist,

oder $-(CH_2)m-R^f$, wobei $R^f$ 4- bis 8-gliedrige heteroalicyclische Gruppe ist, die unsubstituiert oder mit 1 bis 3 Substituenten substituiert ist, ausgewählt aus der Gruppe, bestehend aus Methyl, Ethyl, Propyl, Isopropyl, Methoxy, Ethoxy, Propoxy, Isopropoxy, Methylthio, Ethylthio, Propylthio, Formyl, Acetyl, Hydroxyl, Fluor, Chlor, Trifluormethyl, Cyano, $-CONH_2$, Oxo (=O), Amino, Dimethylamino und Diethylamino, m eine ganze Zahl von 0 bis 3 ist,

die 4- bis 8-gliedrige heteroalicyclische Gruppe 1 bis 2 Atome, ausgewählt aus N, O oder S, als Ringatome enthält, die Arylgruppe Phenyl ist und die Heteroarylgruppe Pyridyl, Pyrimidinyl, Pyrazolyl, Oxazolyl, Isoxazolyl, Thiazolyl ist.

**7.** Verbindung, die pharmazeutisch verträglichen Salze, Diastereomere, Enantiomere oder Solvate davon nach Anspruch 6, wobei $R_2$ Cyclopropyl, Cyclobutyl, Cyclopentyl, Cyclohexyl, Cycloheptyl, Cyclooctyl, 4,4-Difluorcyclohexyl, Bicyclo[2.2.1]heptyl, Adamantyl, Methyl, Ethyl, Propyl, Isopropyl, Butyl, Isobutyl, sec-Butyl, tert-Butyl, Pentyl, Neopentyl, Hexyl, 2-Hydroxy-2-methylpropyl, 3,3-Dimethylbutyl, 3-Hydroxy-3-methylbutyl, Cyclopropylmethyl, Cyclopropylethyl, Cyclopropylpropyl, Cyclobutylmethyl, Cyclobutylethyl, Cyclobutylpropyl, Cyclopentylmethyl, Cyclopentylethyl, Cyclopentylpropyl, Cyclohexylmethyl, Cyclohexylethyl, Cyclohexylpropyl, Benzyl, Phenethyl, Phenyl, 2-Fluorphenyl, 2-Chlorphenyl, 2-Methoxyphenyl, 2-Cyanophenyl, 2-Ethinylphenyl, 3-Fluorphenyl, 3-Chlorphenyl, 3-Methoxyphenyl, 3-Cyanophenyl, 3-Ethinylphenyl, 4-Fluorphenyl, 4-Chlorphenyl, 4-Methoxyphenyl, 4-Cyanophenyl, 4-Ethinylphenyl, 3,4-Difluorphenyl, 3-Cyano-4-methylphenyl, Pyridin-2-yl, Pyridin-3-yl, Pyridin-4-yl, Pyrimidin-2-yl, Pyrimidin-4-yl, Pyrimidin-5-yl, Pyrazinyl, Pyrazolyl, Oxazolyl, Isoxazolyl, Thiazolyl, Oxetan-3-yl, Tetrahydrofuran-3-yl, Tetrahydro-2H-pyran-4-yl, Tetrahydropyrrolyl, Piperidin-1-yl, Piperazin-1-yl, Morpholin-4-yl, Methylpiperazin-4-yl, 1-Methylpiperidin-4-yl, 1-Acetylpiperidin-4-yl, 4-Hydroxypiperidin-1-yl, 4-Methyl-4-hydroxypiperidin-1-yl,

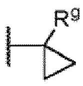 , 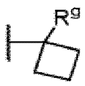 ,

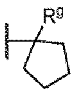, 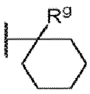, 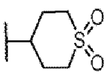, 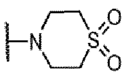, 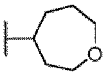, 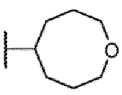, 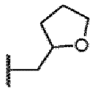, 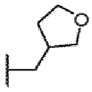, 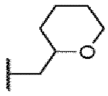,

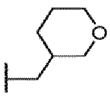 oder 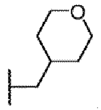

ist, $R^g$ -$CH_3$ oder -OH ist.

8. Verbindung, die pharmazeutisch verträglichen Salze, Diastereomere, Enantiomere oder Solvate davon nach Anspruch 1, wobei $R_2$ 2,3-Difluorphenyl, 3,5-Difluorphenyl, 2,5-Difluorphenyl, 2,4-Difluorphenyl, 2,3,4-Trifluorphenyl, 2,3,5-Trifluorphenyl, 2,3,6-Trifluorphenyl, 3,4,5-Trifluorphenyl, 2,4,5-Trifluorphenyl, 3-Chlor-2-fluorphenyl, 2-Chlor-3-fluorphenyl, 5-Chlor-2-fluorphenyl, 2-Chlor-5-fluorphenyl, 5-Chlor-3-fluorphenyl, 3-Chlor-5-fluorphenyl, 3-Chlor-4-fluorphenyl, 4-Chlor-3-fluorphenyl, 2-Chlor-4-fluorphenyl, 4-Chlor-2-fluorphenyl, 3-Chlor-2,4-difluorphenyl, 5-Chlor-2,4-difluorphenyl, 3-Chlor-2,5-difluorphenyl, 3-Chlor-2,6-difluorphenyl, 3-Chlor-4,5-difluorphenyl, 2-Chlor-4,5-difluorphenyl, 2-Chlor-3,4-difluorphenyl, 2-Chlor-3,5-difluorphenyl, 2-Chlor-3,6-difluorphenyl, 4-Chlor-2,3-difluorphenyl, 4-Chlor-3,5-difluorphenyl, 4-Chlor-2,5-difluorphenyl, 5-Chlor-2,3-difluorphenyl, 5-Chlor-3,4-difluorphenyl, 6-Chlor-2,3-difluorphenyl, 3-Fluor-5-methylphenyl, 4-Fluor-3-methylphenyl, 2-Fluor-3-methylphenyl, 2-Fluor-4-methylphenyl, 2-Fluor-5-methylphenyl, 3-Fluor-4-methylphenyl, 3-Fluor-2-methylphenyl, 3-Fluor-5-methoxyphenyl, 4-Fluor-3-methoxyphenyl, 2-Fluor-3-methoxyphenyl, 2-Fluor-4-methoxyphenyl, 2-Fluor-5-methoxyphenyl, 3-Fluor-4-methoxyphenyl, 3-Fluor-2-methoxyphenyl, 2-Fluor-3-trifluormethylphenyl, 2-Fluor-4-trifluormethylphenyl, 2-Fluor-5-trifluormethylphenyl, 3-Fluor-2-trifluormethylphenyl, 4-Fluor-2-trifluormethylphenyl, 5-Fluor-2-trifluormethylphenyl, 4-Fluor-3-trifluormethylphenyl, 3-Fluor-4-trifluormethylphenyl, 3-Fluor-5-trifluormethylphenyl, 2-Fluor-5-ethylphenyl, 2-Fluor-5-cyclopropylphenyl oder 2-Fluor-5-phenyl ist.

9. Verbindung, die pharmazeutisch verträglichen Salze, Diastereomere, Enantiomere oder Solvate davon nach Anspruch 1, wobei $R_3$ Wasserstoff, Methyl, Ethyl, Hydroxyl, Cyano, Trifluormethyl, Fluor oder Chlor ist.

10. Verbindung, die pharmazeutisch verträglichen Salze, Diastereomere, Enantiomere oder Solvate davon nach Anspruch 1,

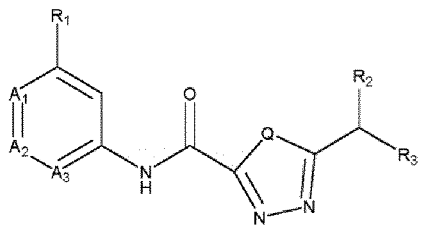

Formel (I)

in Formel (I),

Q ist NH;
$A_1$ und $A_2$ sind jeweils CH;
$A_3$ ist N;
$R_1$ ist

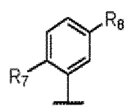, 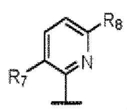, 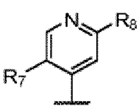, 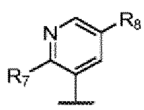, 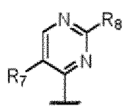 oder 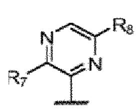;

$R_7$ ist Methyl, Ethyl, Propyl, Isopropyl, Fluor, Chlor, Trifluormethyl, Vinyl oder Propenyl;
$R_8$ ist Cyano, $C_1$-$C_3$-Alkoxy, $C_1$-$C_6$-Alkylthio oder -O-$R_6$;

$R_5$ ist $C_1$-$C_6$-Alkyl;

$R_6$ ist 4- bis 8-gliedrige heteroalicyclische Gruppe oder $C_1$-$C_{10}$-Alkyl, das mit 1 bis 3 Substituenten substituiert ist, ausgewählt aus der Gruppe, bestehend aus Hydroxyl, $C_1$-$C_6$-Alkoxy, Cyano, $C_3$-$C_8$-Cycloalkyloxy, $C_3$-$C_8$-Cycloalkyl, -$SO_2$-$R_5$, 4- bis 8-gliedriger heteroalicyclischer Gruppe und $C_1$-$C_6$-Alkylthio;

$R_2$ ist -$(CH_2)$n-$R^e$, wobei $R^e$ Aryl oder Heteroaryl ist, das unsubstituiert oder mit 1 bis 3 Substituenten substituiert ist, ausgewählt aus der Gruppe, bestehend aus $C_1$-$C_6$-Alkyl, $C_1$-$C_6$-Alkoxy, $C_1$-$C_6$-Alkylthio, $C_1$-$C_3$-Acyl, Hydroxyl, Halogen, Trifluormethyl, Cyano, -$CONH_2$, $C_2$-$C_6$-Alkinyl, $C_2$-$C_6$-Alkenyl und -$NR^cR^d$, wobei n eine ganze Zahl von 0 bis 3 ist;

$R^c$ und $R^d$ sind jeweils unabhängig ausgewählt aus Wasserstoff, $C_1$-$C_6$-Alkyl, $C_3$-$C_8$-Cycloalkyl, $C_1$-$C_6$-Alkyl, substituiert mit $C_1$-$C_6$-Alkoxy, $C_1$-$C_6$-Alkyl, substituiert mit Hydroxyl, $C_3$-$C_8$-Cycloalkyl-$C_1$-$C_6$-alkyl, $C_1$-$C_6$-Alkyl, substituiert mit 4- bis 8-gliedriger heteroalicyclischer Gruppe, $C_1$-$C_6$-Alkyl, substituiert mit $C_1$-$C_3$-Alkylthio, oder $C_1$-$C_6$-Alkyl, substituiert mit substituiertem Amino oder unsubstituiertem Amino, wobei das substituierte Amino mit Mono- oder Di-$C_1$-$C_3$-alkyl substituiert ist;

die 4- bis 8-gliedrige heteroalicyclische Gruppe ist eine 4-bis 8-gliedrige heteroalicyclische Gruppe, die 1 bis 2 Atome, ausgewählt aus N, O oder S, als Ringatome enthält;

die Arylgruppe ist eine monocyclische oder bicyclische Gruppe, die 6 bis 12 Kohlenstoffringatome enthält und mindestens einen aromatischen Ring aufweist, das Heteroaryl ist eine monocyclische oder bicyclische Gruppe, die 5 bis 10 Ringatome aufweist und 1 bis 3 Heteroatome, ausgewählt aus N, O oder S, als Ringatome enthält;

$R_3$ ist Wasserstoff.

**11.** Verbindung, die pharmazeutisch verträglichen Salze, Diastereomere, Enantiomere oder Solvate davon nach Anspruch 10, wobei

Q NH ist;

$A_1$ und $A_2$ jeweils CH sind;

$A_3$ N ist;

$R_1$

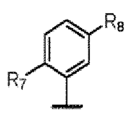, 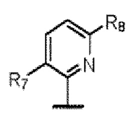, 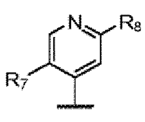, 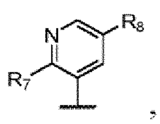, 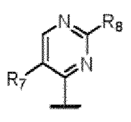 oder 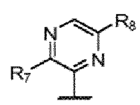

ist;

$R_7$ Methyl, Chlor oder Trifluormethyl ist;

$R_8$ Cyano, $C_1$-$C_3$-Alkoxy-$C_1$-$C_6$-alkylthio oder -O-$R_6$ ist;

$R_6$ $C_1$-$C_{10}$-Alkyl, das mit 1 bis 3 Substituenten substituiert ist, ausgewählt aus Hydroxyl, ist;

$R_2$ -$(CH_2)_n$-$R^e$ ist, wobei $R^e$ Aryl oder Heteroaryl ist, das unsubstituiert oder mit 1 bis 3 Substituenten substituiert ist, ausgewählt aus der Gruppe, bestehend aus Methyl, Ethyl, Propyl, Isopropyl, Methoxy, Ethoxy, Propoxy, Isopropoxy, Methylthio, Ethylthio, Propylthio, Formyl, Acetyl, Hydroxyl, Fluor, Chlor, Trifluormethyl, Cyano, -$CONH_2$, Ethinyl, Vinyl, Amino, Dimethylamino und Diethylamino, n eine ganze Zahl von 0 bis 3 ist,

die Arylgruppe eine monocyclische oder bicyclische Gruppe ist, die 6 bis 12 Kohlenstoffringatome enthält und mindestens einen aromatischen Ring aufweist, das Heteroaryl eine monocyclische oder bicyclische Gruppe ist, die 5 bis 10 Ringatome aufweist und 1 bis 3 Heteroatome, ausgewählt aus N, O oder S, als Ringatome enthält;

$R_3$ Wasserstoff ist.

**12.** Verbindung, die pharmazeutisch verträglichen Salze, Diastereomere, Enantiomere oder Solvate davon nach Anspruch 1, wobei die Verbindung aus den folgenden Strukturen ausgewählt ist:

| | | | |
|---|---|---|---|
| 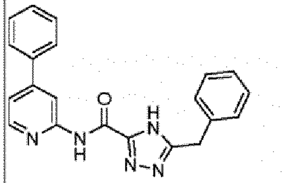 | 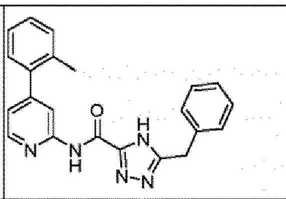 | 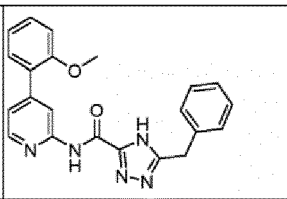 | 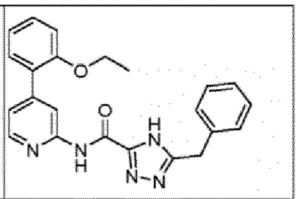 |
| 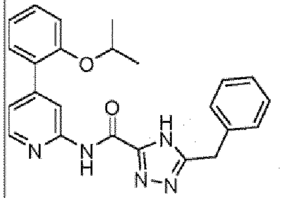 | 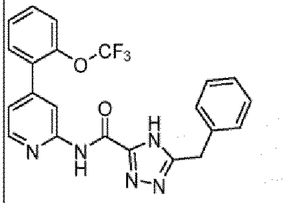 | 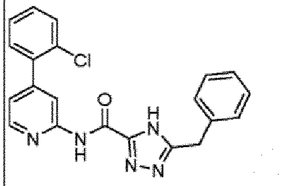 | 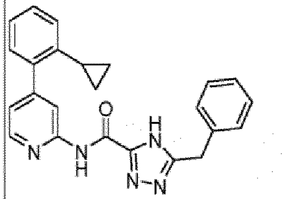 |

| | | | |
|---|---|---|---|
| 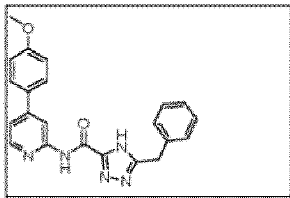 | 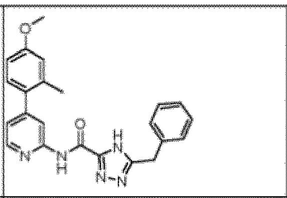 | 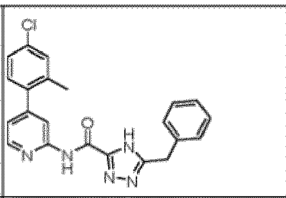 | 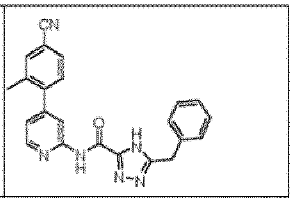 |
| 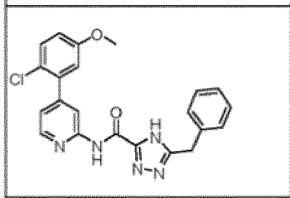 | 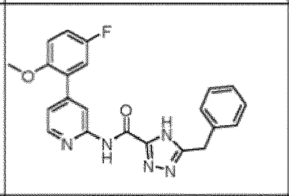 | 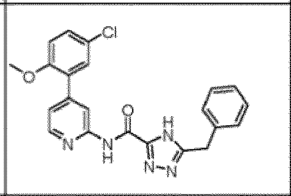 | 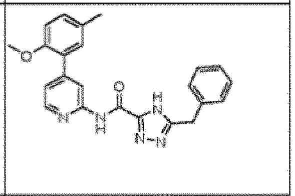 |
| 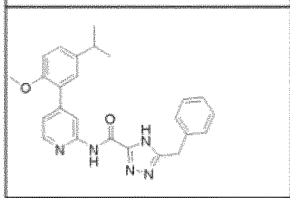 | 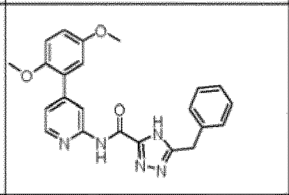 | 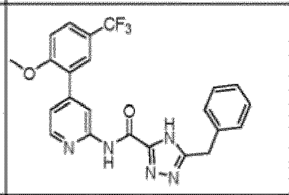 | 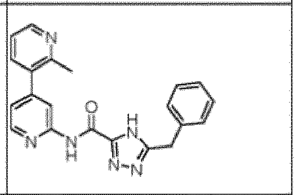 |
| 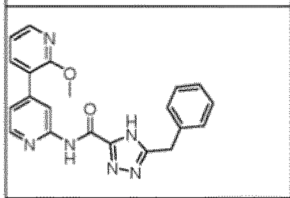 | 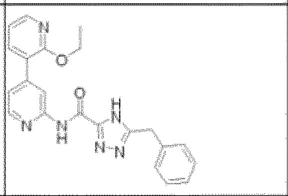 | 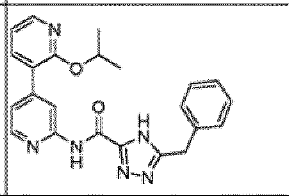 | 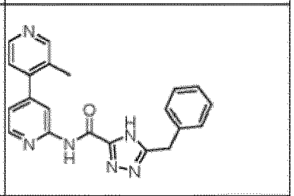 |
| 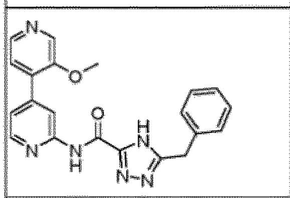 | 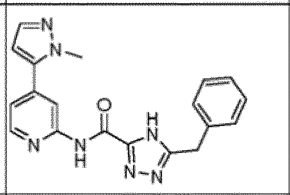 | 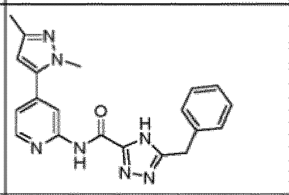 | 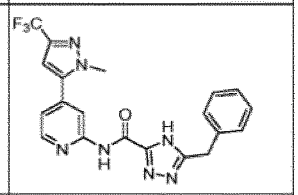 |
| 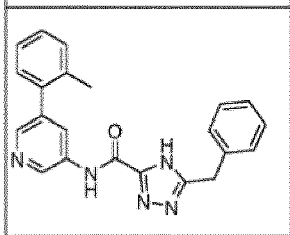 | 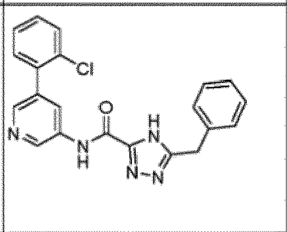 | 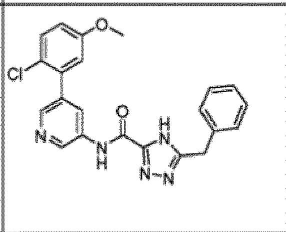 | 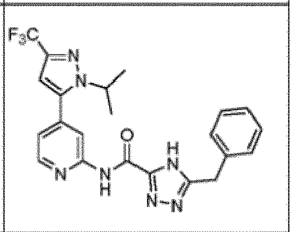 |
| 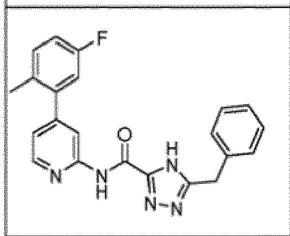 | 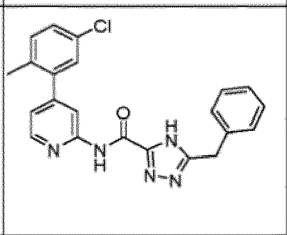 | 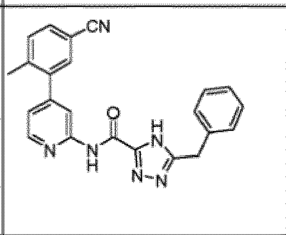 | 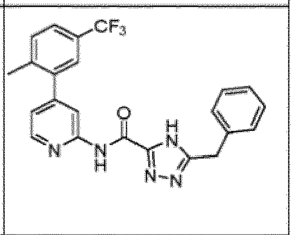 |
| 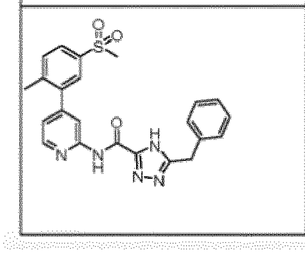 | 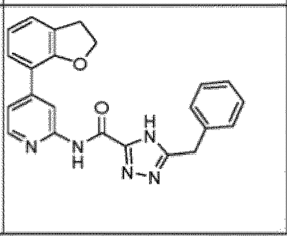 | 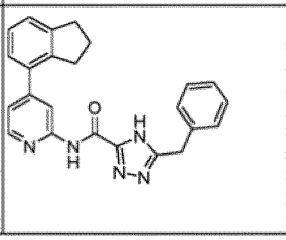 | 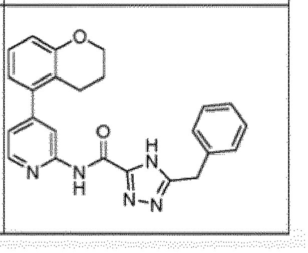 |

| | | | |
|---|---|---|---|
| 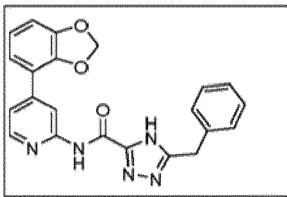 | 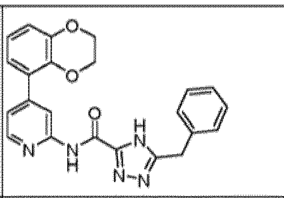 | 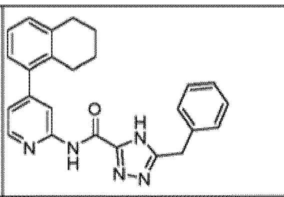 | 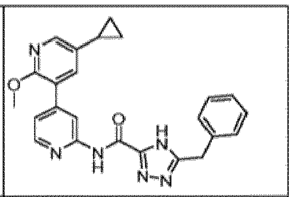 |
| 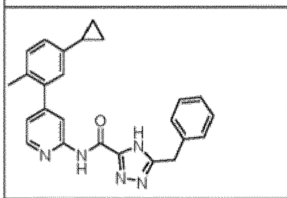 | 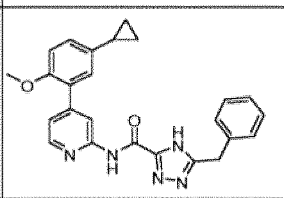 | 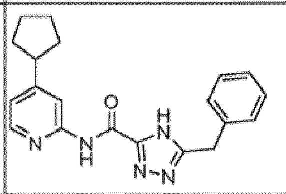 | 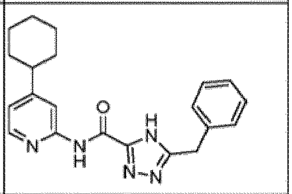 |
| 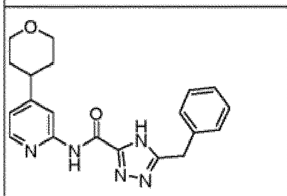 | 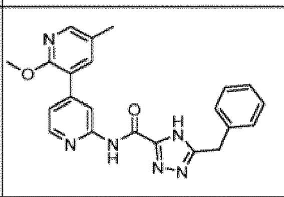 | 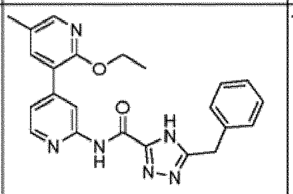 | 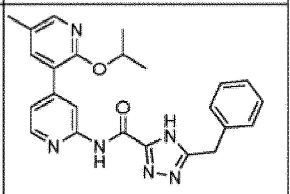 |
| 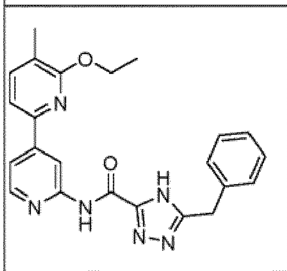 | 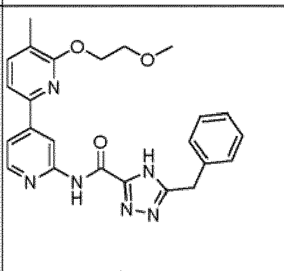 | 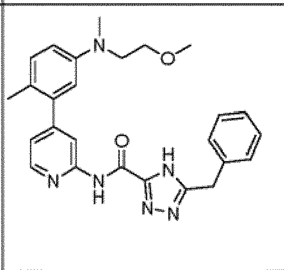 | 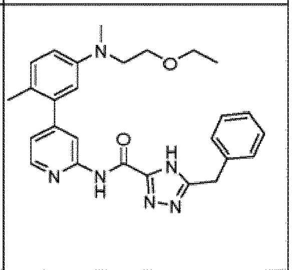 |
| 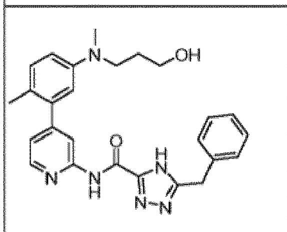 | 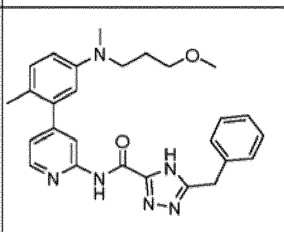 | 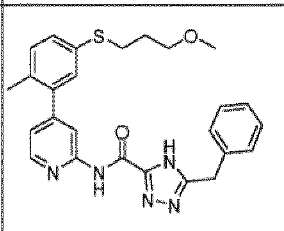 | 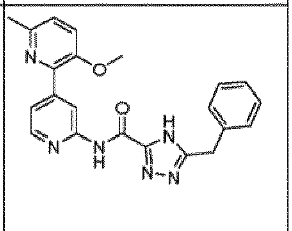 |
| 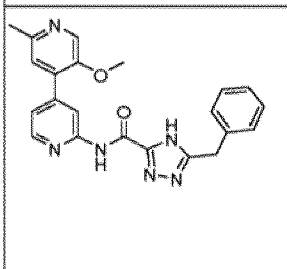 | 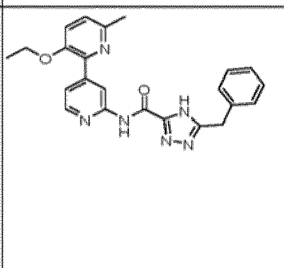 | 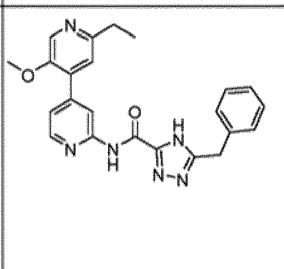 | 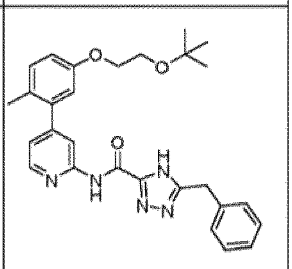 |
| 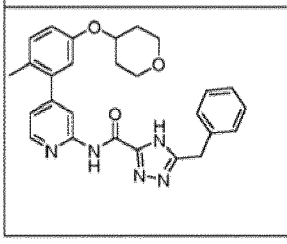 | 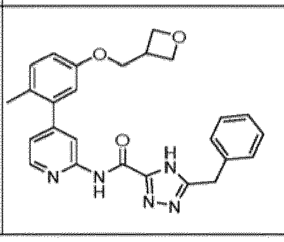 | 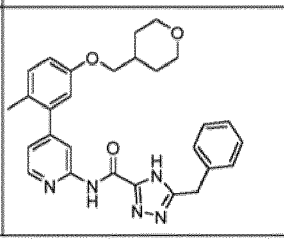 | 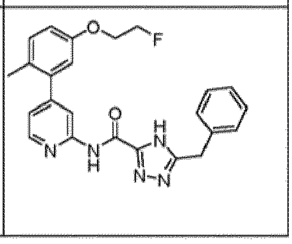 |

| | | | |
|---|---|---|---|
| 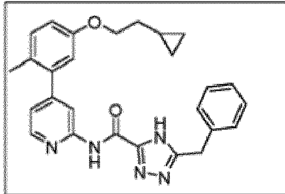 | 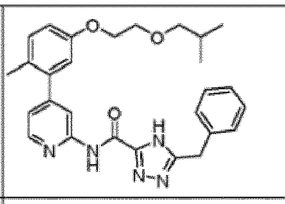 | 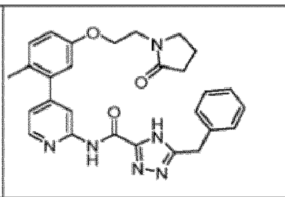 | 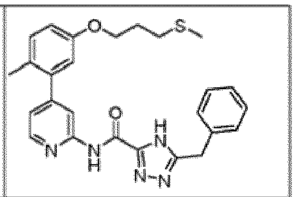 |
| 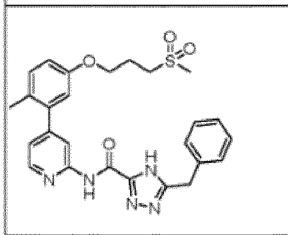 | 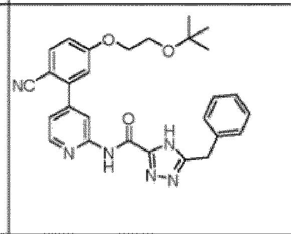 | 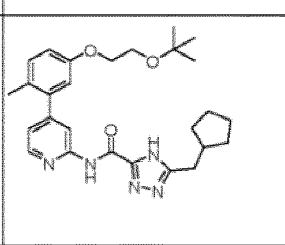 | 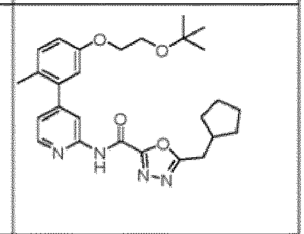 |
| 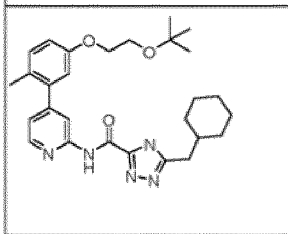 | 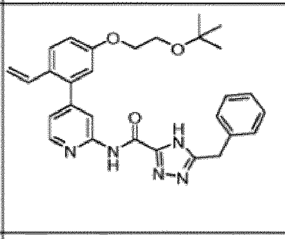 | 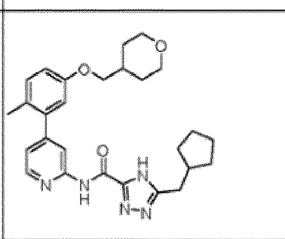 | 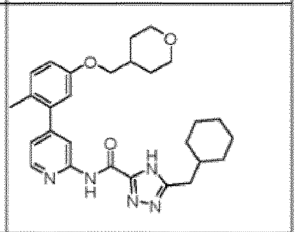 |
| 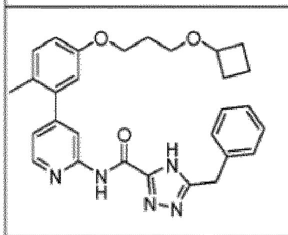 | 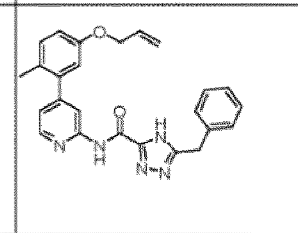 | 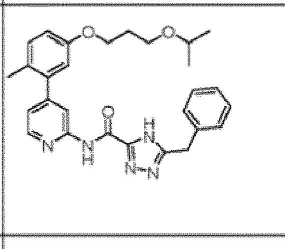 | 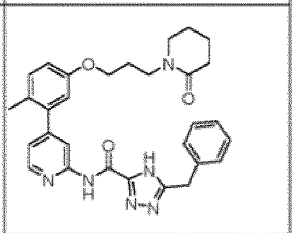 |
| 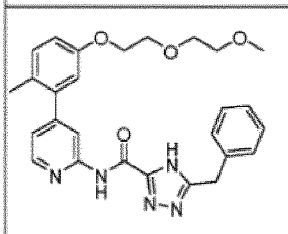 | 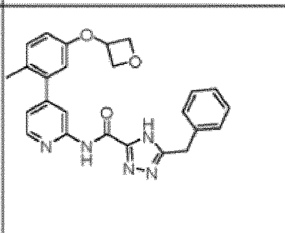 | 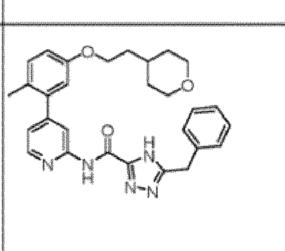 | 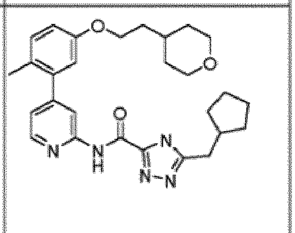 |
| 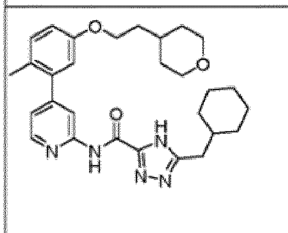 | 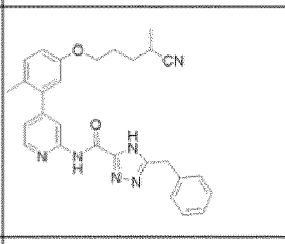 | 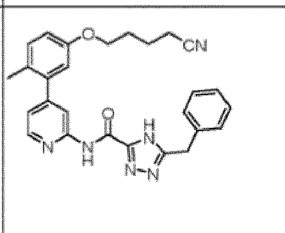 | 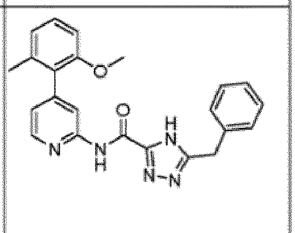 |
| 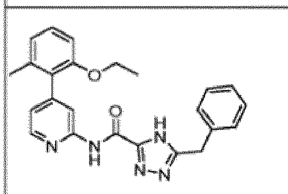 | 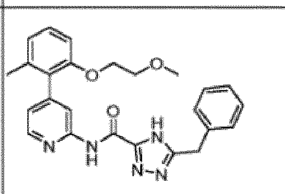 | 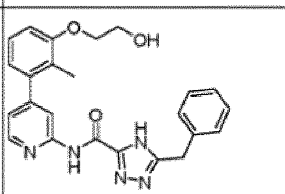 | 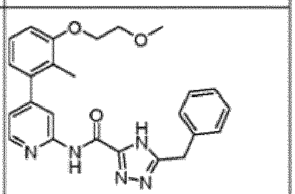 |
| 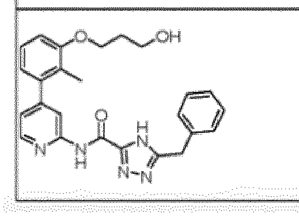 | 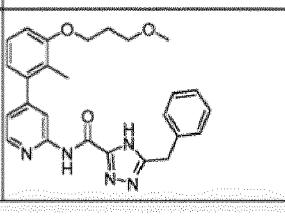 | 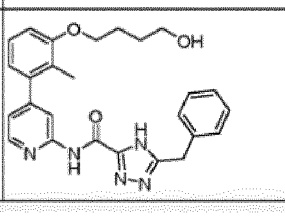 | 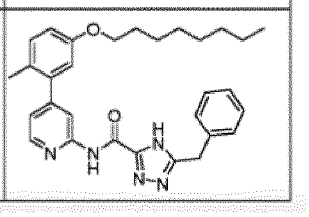 |

| | | | |
|---|---|---|---|
| 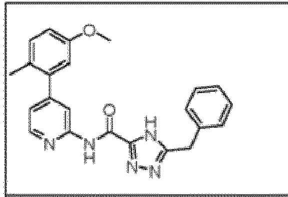 | 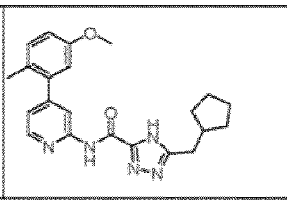 | 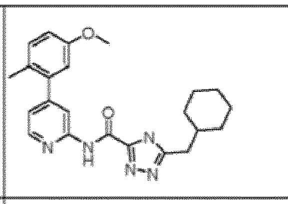 | 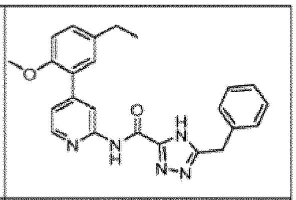 |
| 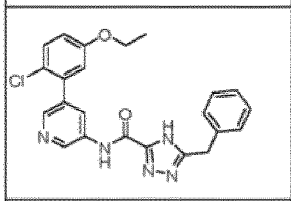 | 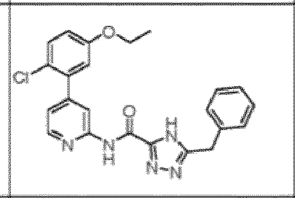 | 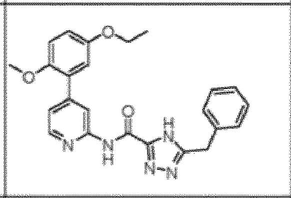 | 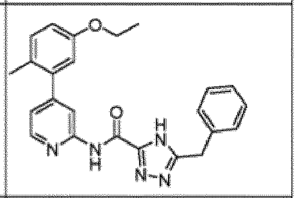 |
| 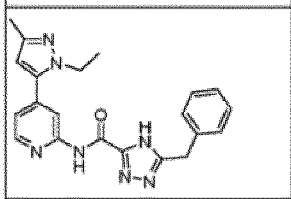 | 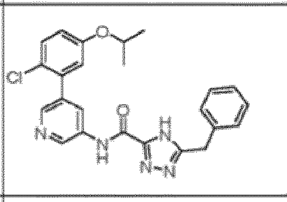 | 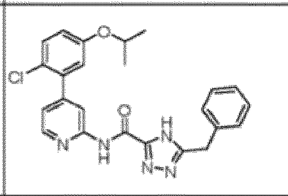 | 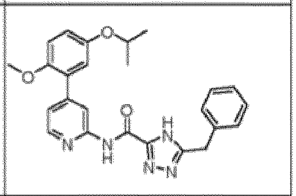 |
| 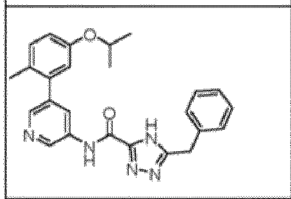 | 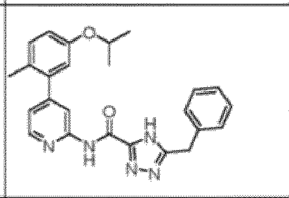 | 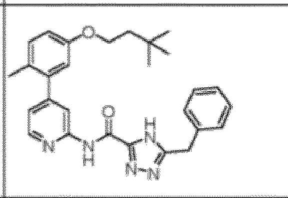 | 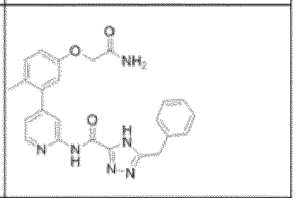 |
| 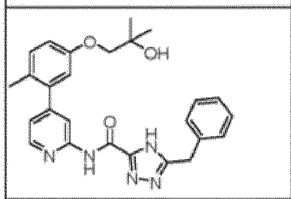 | 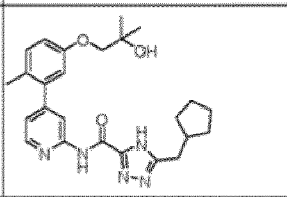 | 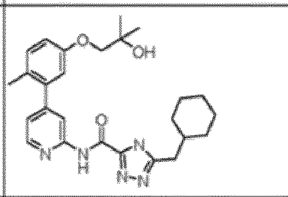 | 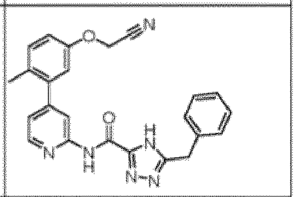 |
| 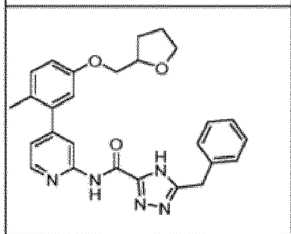 | 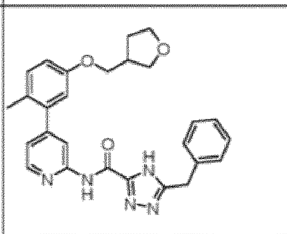 | 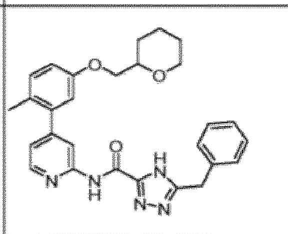 | 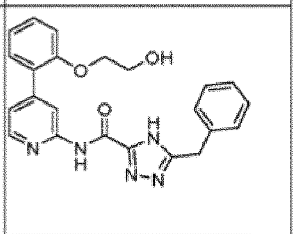 |
| 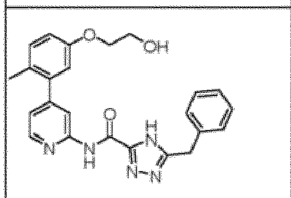 | 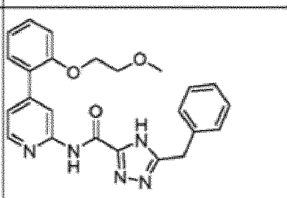 | 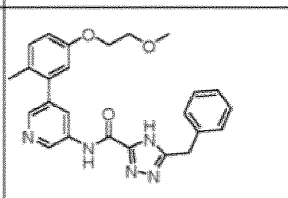 | 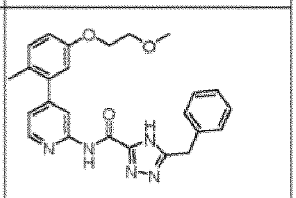 |
| 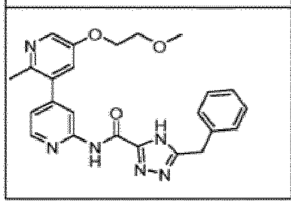 | 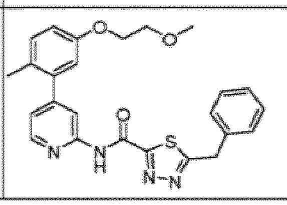 | 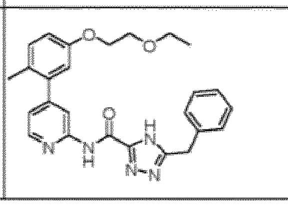 | 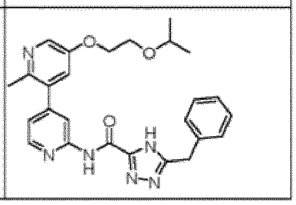 |

| | | | |
|---|---|---|---|
| 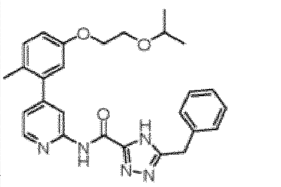 | 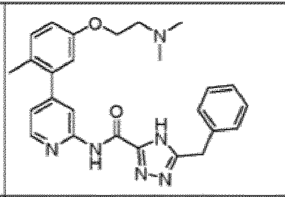 | 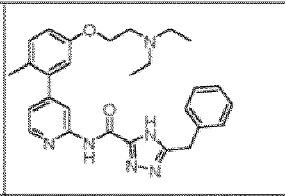 | 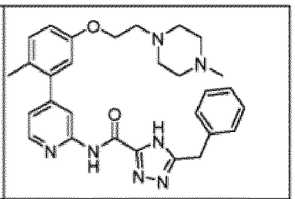 |
| 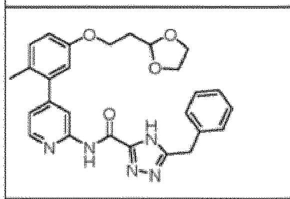 | 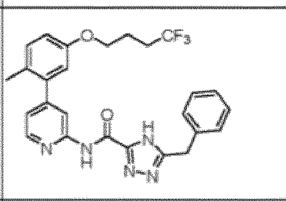 | 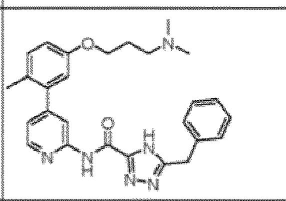 | 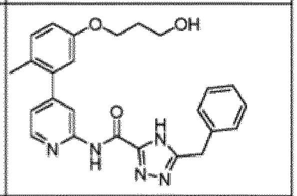 |
| 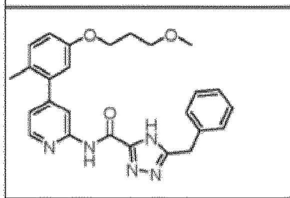 | 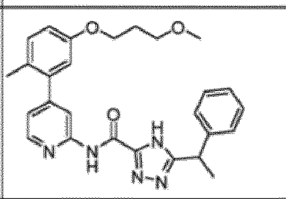 | 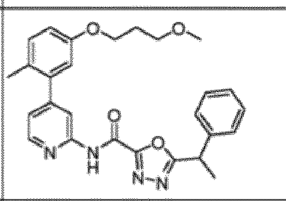 | 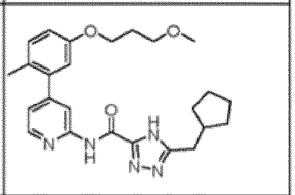 |
| 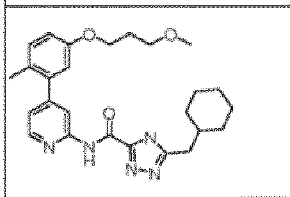 | 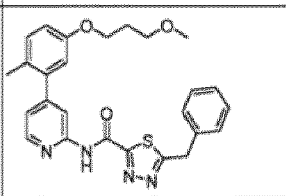 | 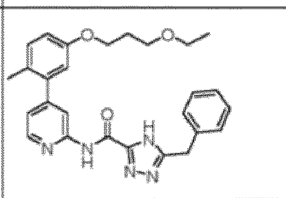 | 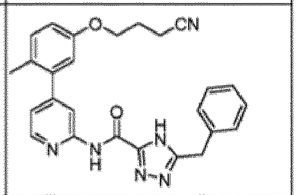 |
| 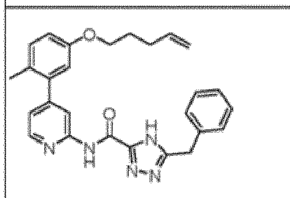 | 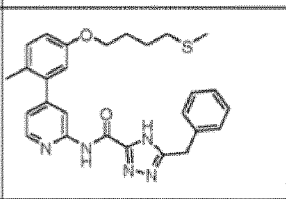 | 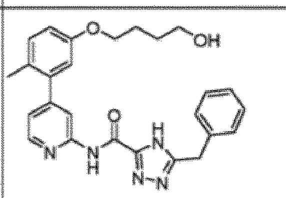 | 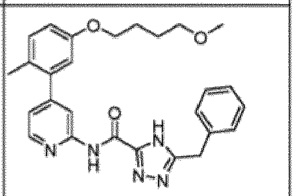 |
| 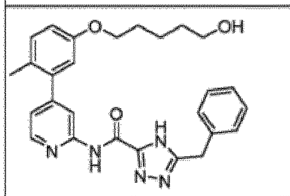 | 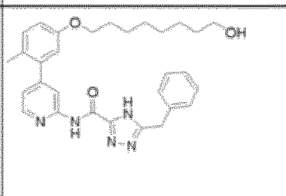 | 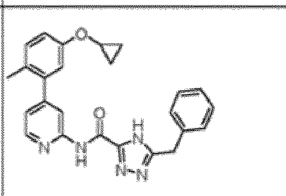 | 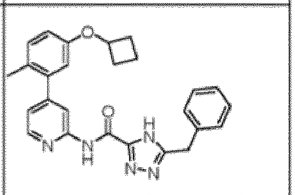 |
| 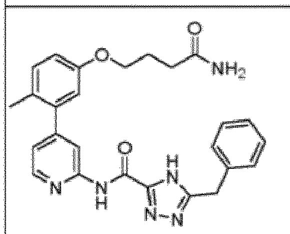 | 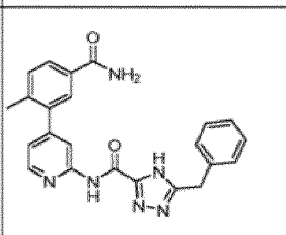 | 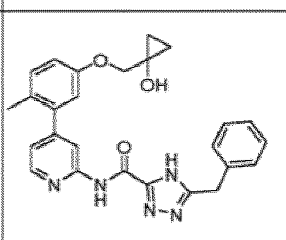 | 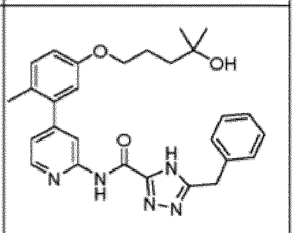 |
| 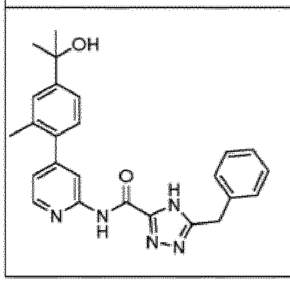 | 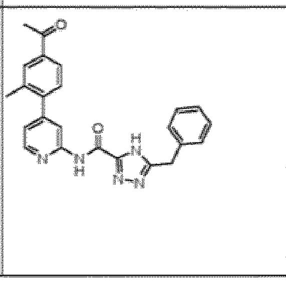 | 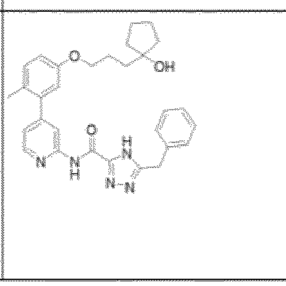 | 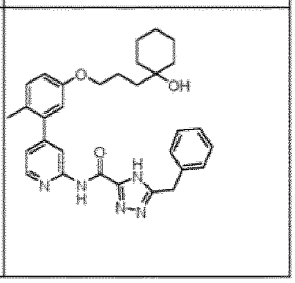 |

| | | | |
|---|---|---|---|
| 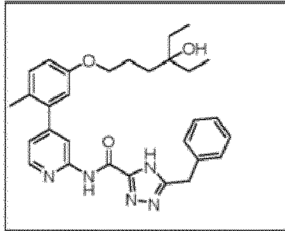 | 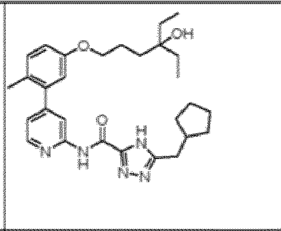 | 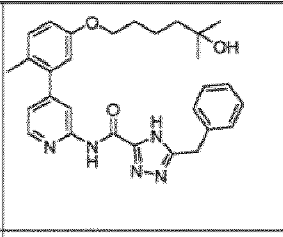 | 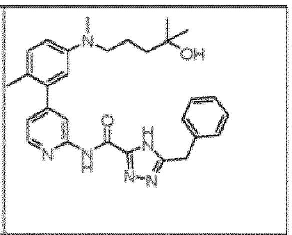 |
| 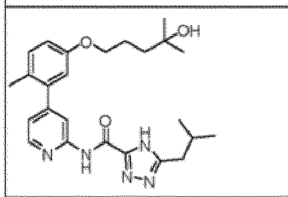 | 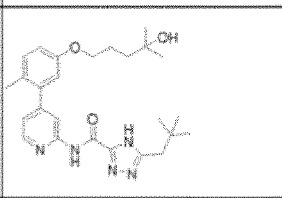 | 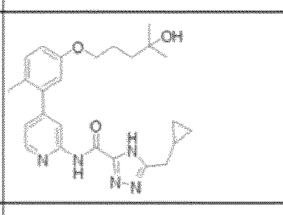 | 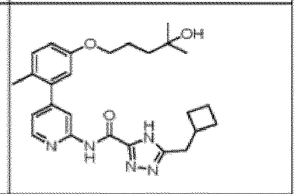 |
| 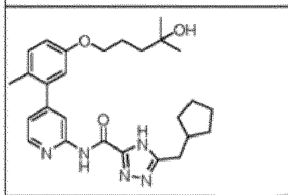 | 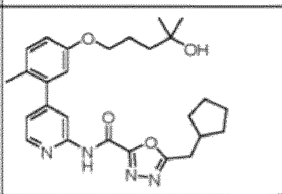 | 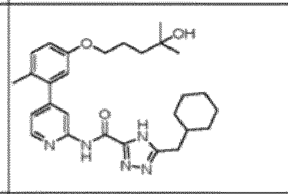 | 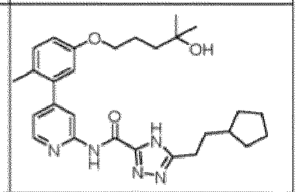 |
| 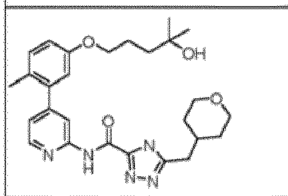 | 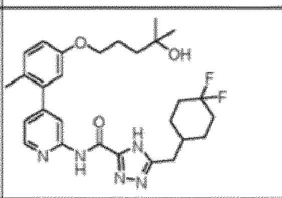 | 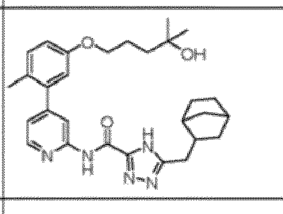 | 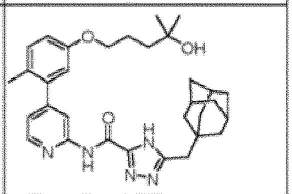 |
| 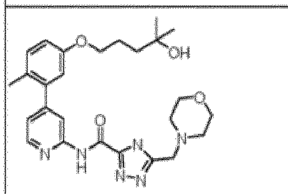 | 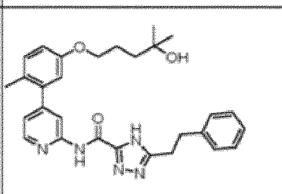 | 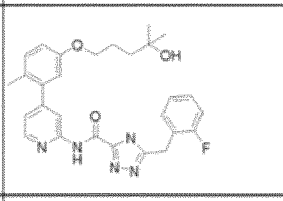 | 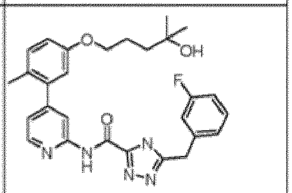 |
| 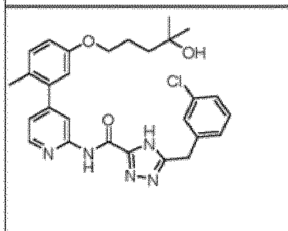 | 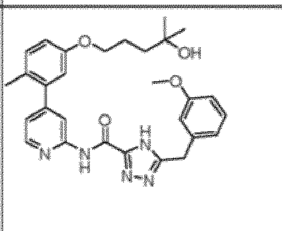 | 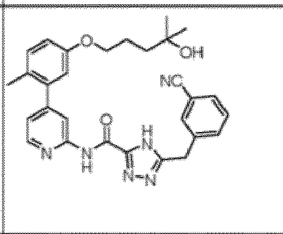 | 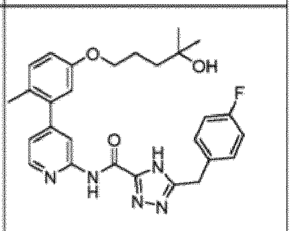 |
| 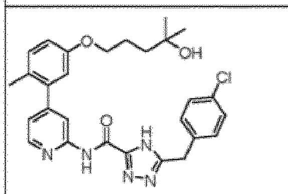 | 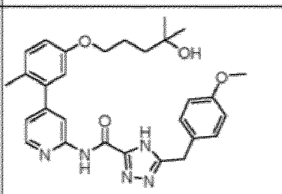 | 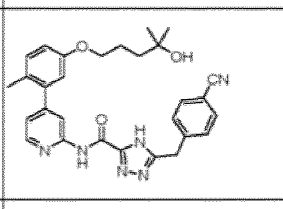 | 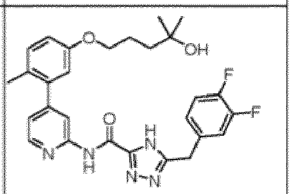 |
| 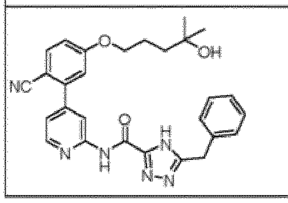 | 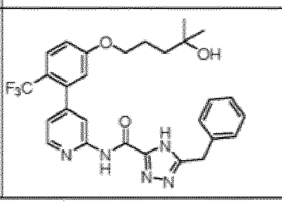 | 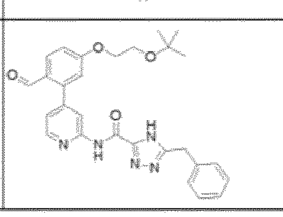 | 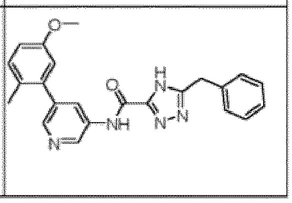 |

| | | | |
|---|---|---|---|
| 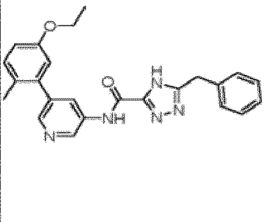 | 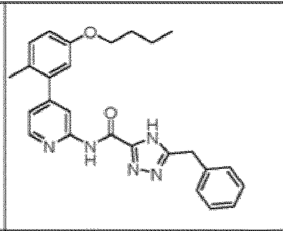 | 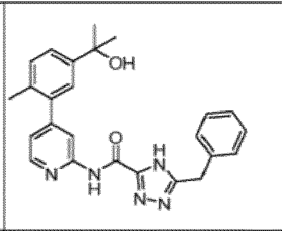 | 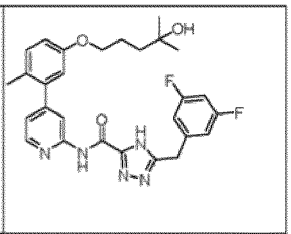 |
| 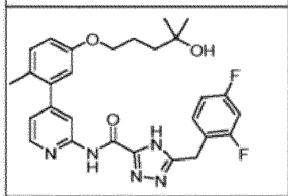 | 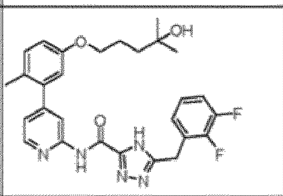 | 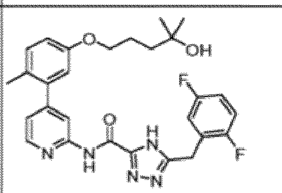 | 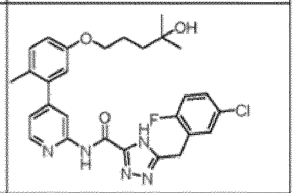 |
| 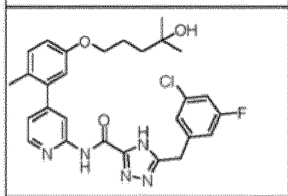 | 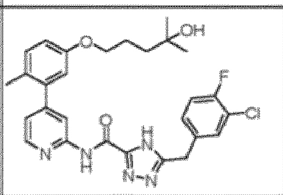 | 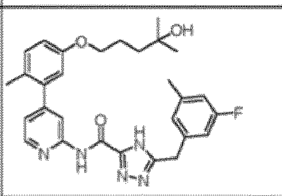 | 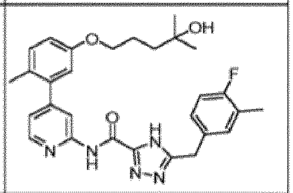 |
| 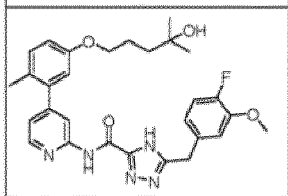 | 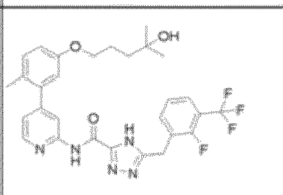 | 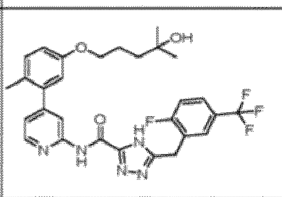 | 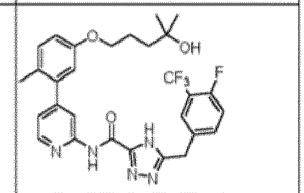 |
| 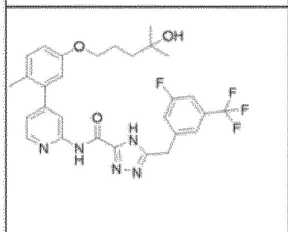 | 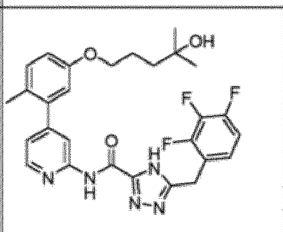 | 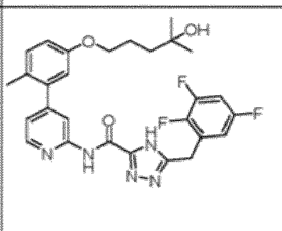 | 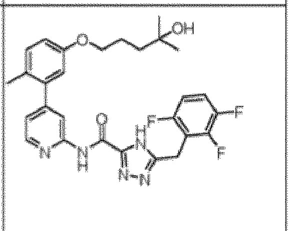 |
| 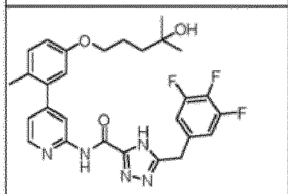 | 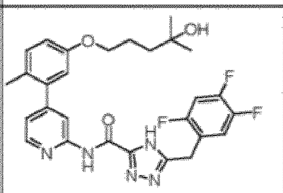 | 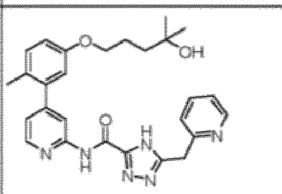 | 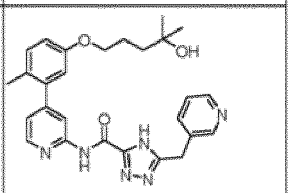 |
| 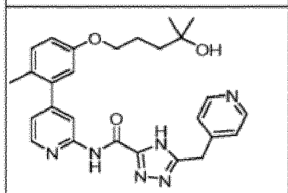 | 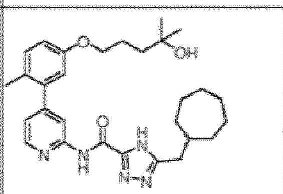 | 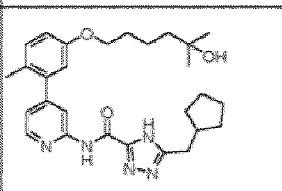 | 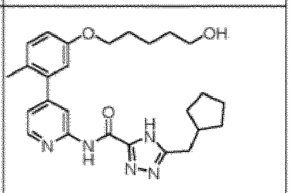 |
| 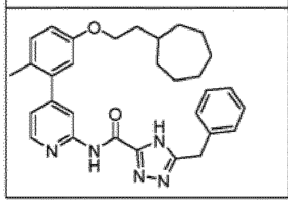 | 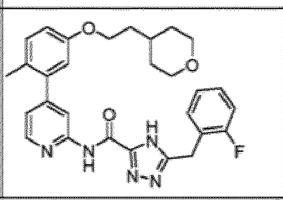 | 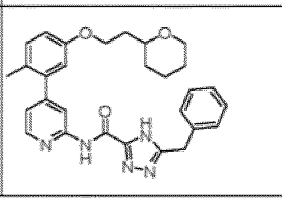 | 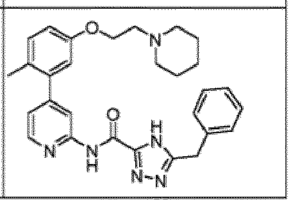 |

| | | | |
|---|---|---|---|
| 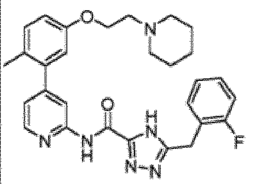 | 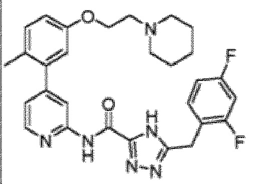 | 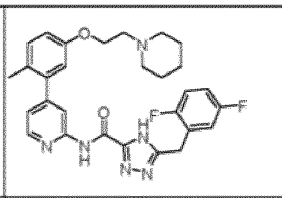 | 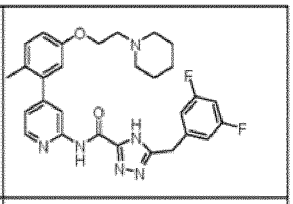 |
| 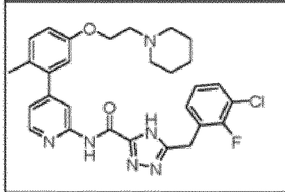 | 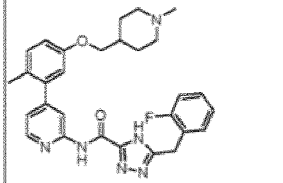 | 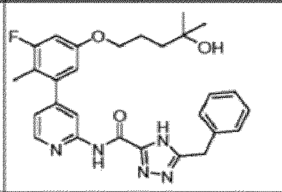 | 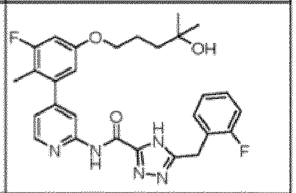 |
| 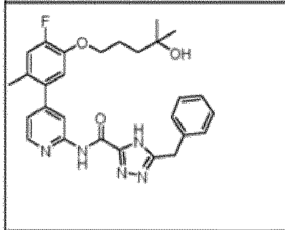 | 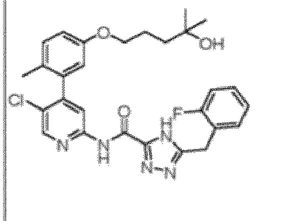 | 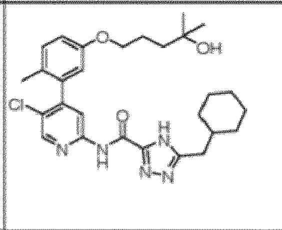 | 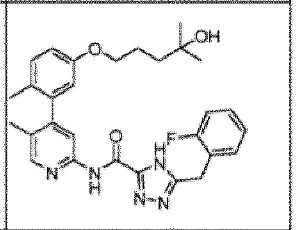 |
| 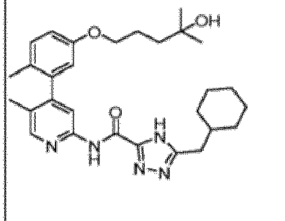 | 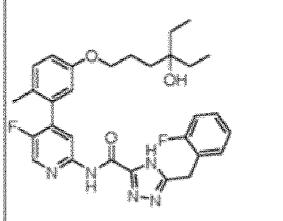 | 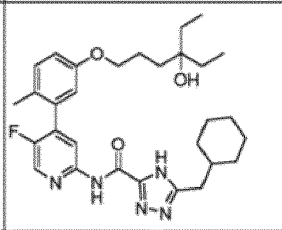 | 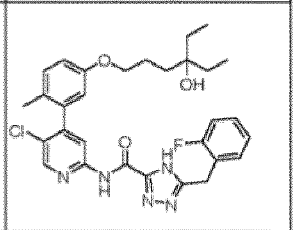 |
| 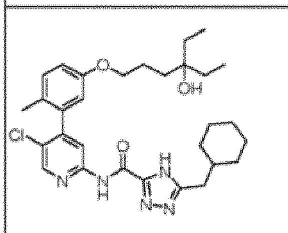 | 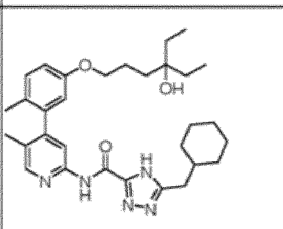 | 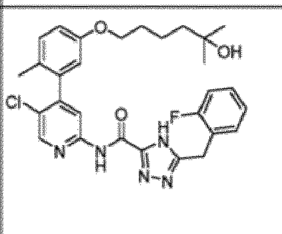 | 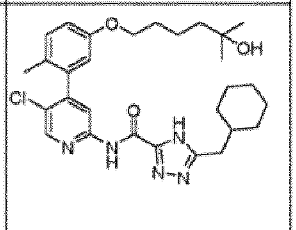 |
| 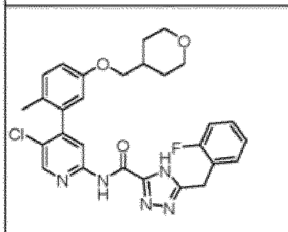 | 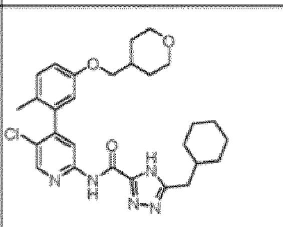 | 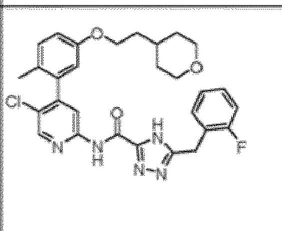 | 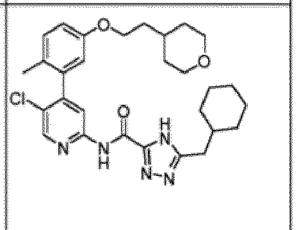 |
| 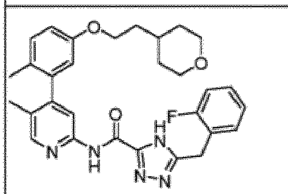 | 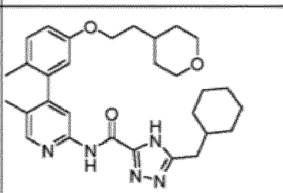 | 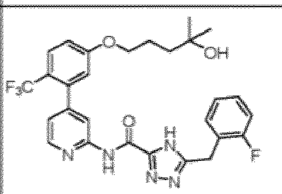 | 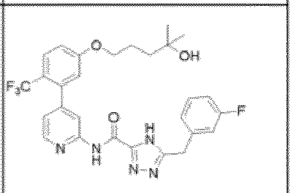 |
| 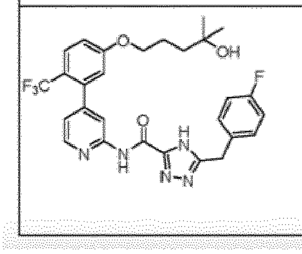 | 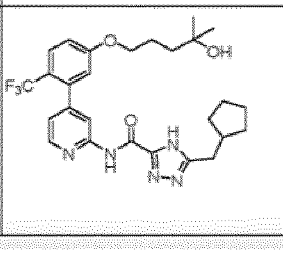 | 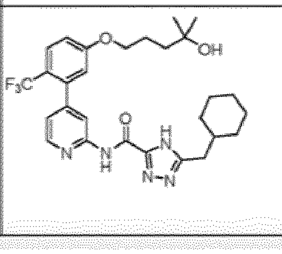 | 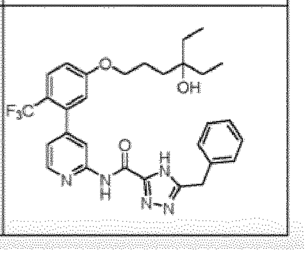 |

| | | | |
|---|---|---|---|
| 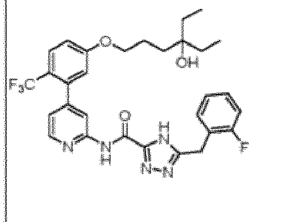 | 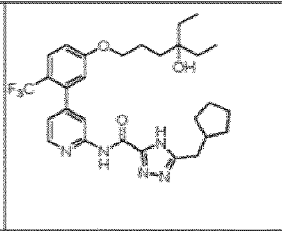 | 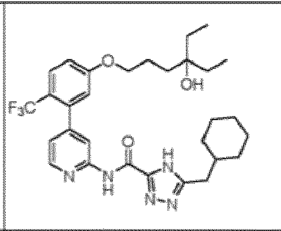 | 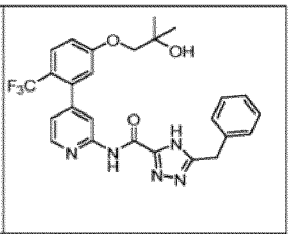 |
| 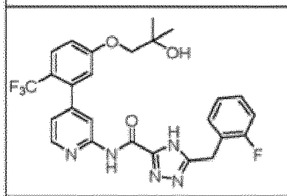 | 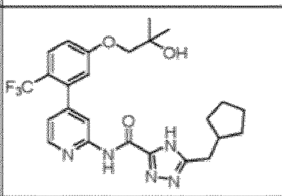 | 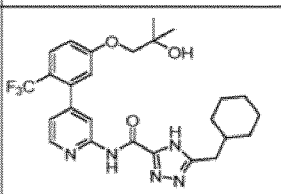 | 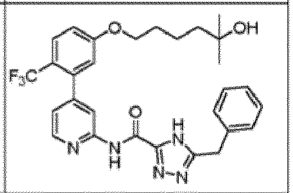 |
| 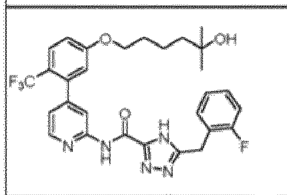 | 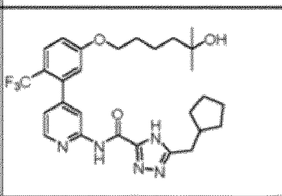 | 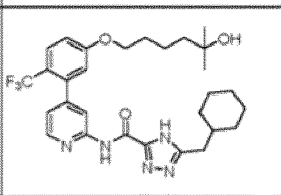 | 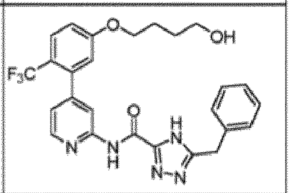 |
| 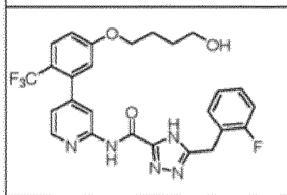 | 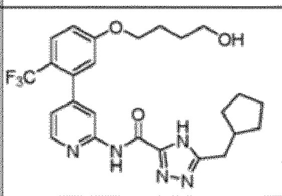 | 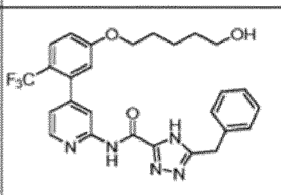 | 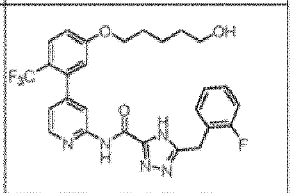 |
| 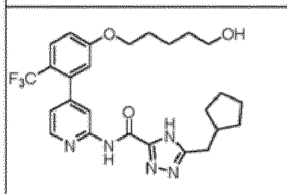 | 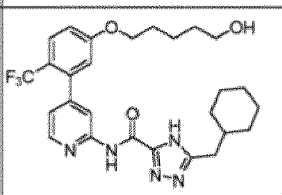 | 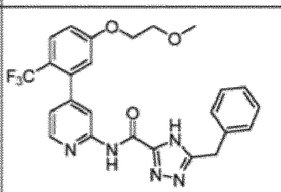 | 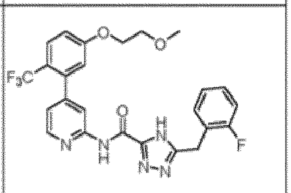 |
| 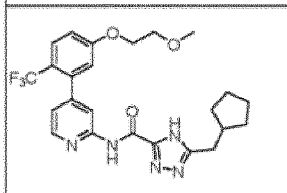 | 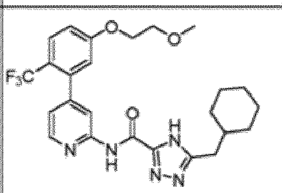 | 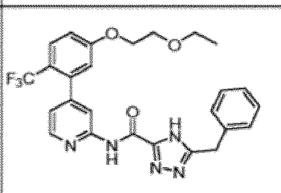 | 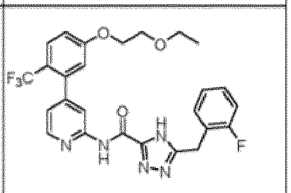 |
| 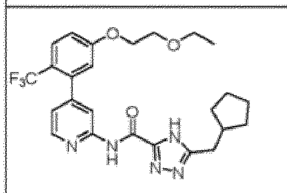 | 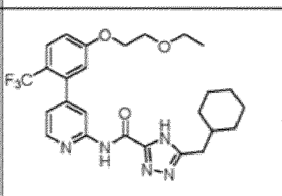 | 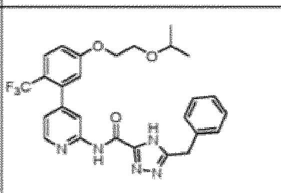 | 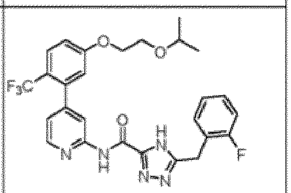 |
| 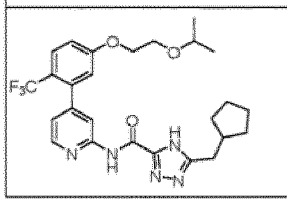 | 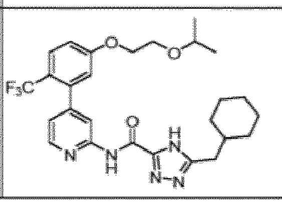 | 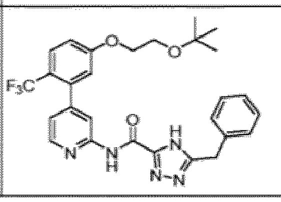 | 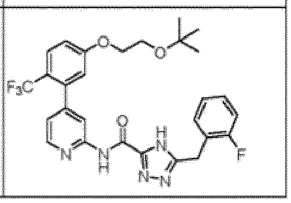 |

| | | | |
|---|---|---|---|
| 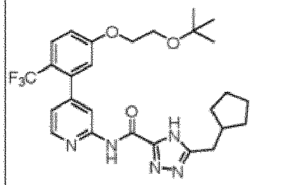 | 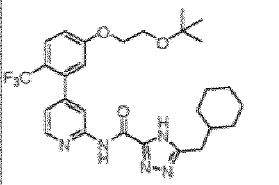 | 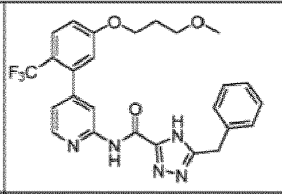 | 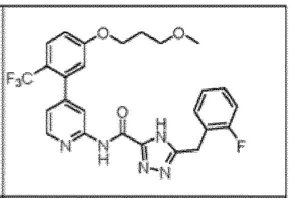 |
| 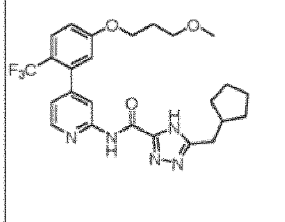 | 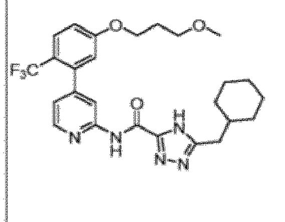 | 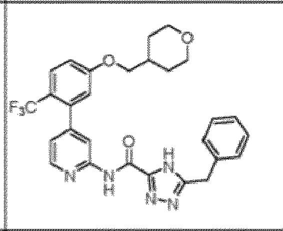 | 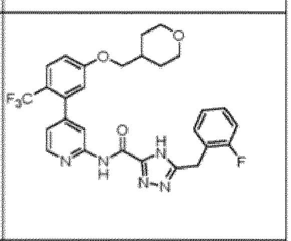 |
| 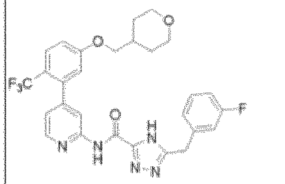 | 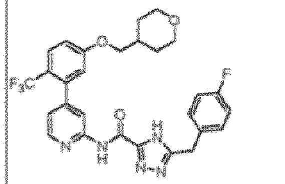 | 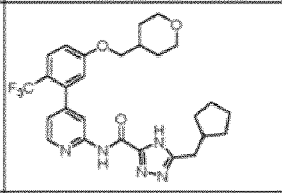 | 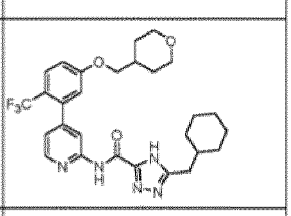 |
| 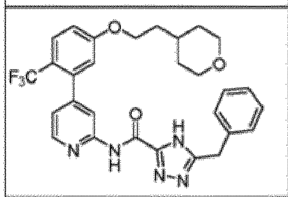 | 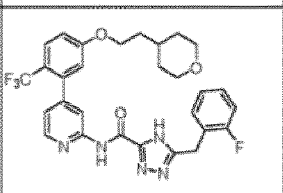 | 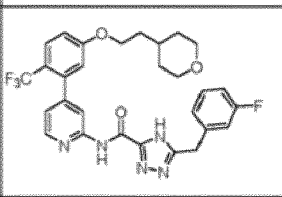 | 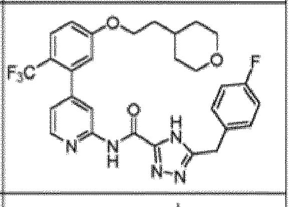 |
| 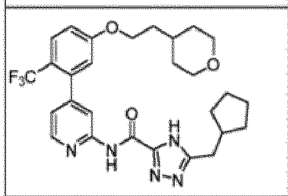 | 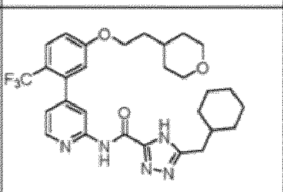 | 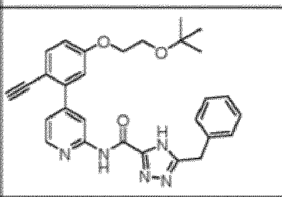 | 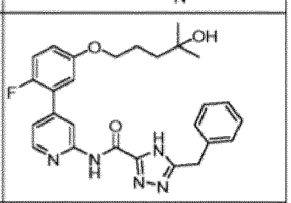 |
| 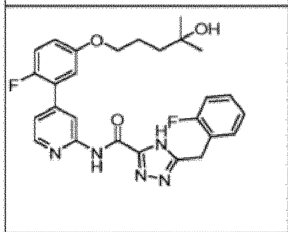 | 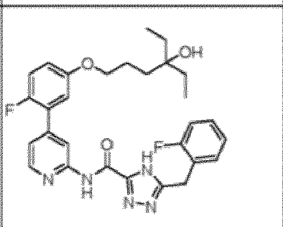 | 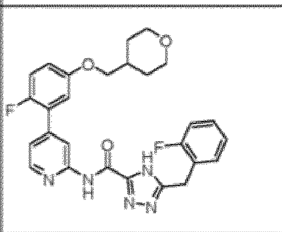 | 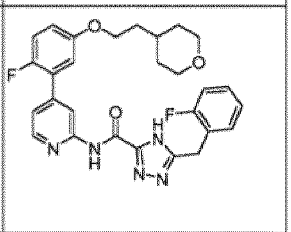 |
| 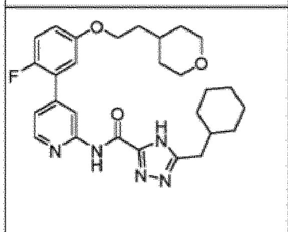 | 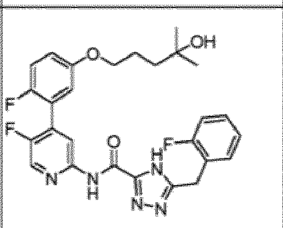 | 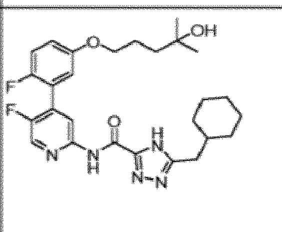 | 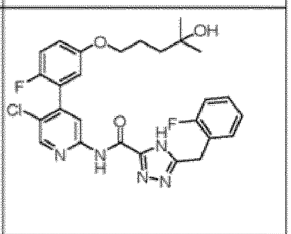 |
| 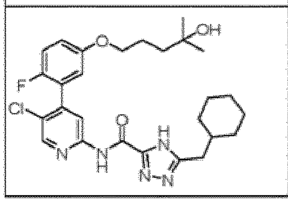 | 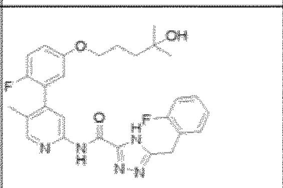 | 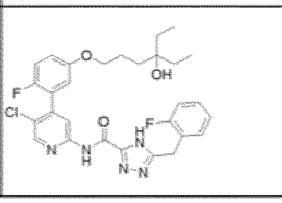 | 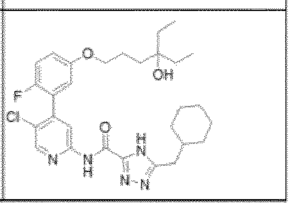 |

| | | | |
|---|---|---|---|
| 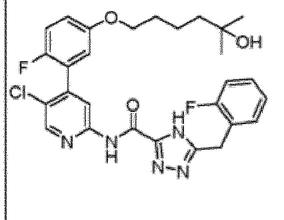 | 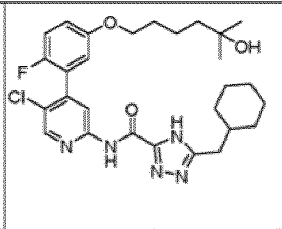 | 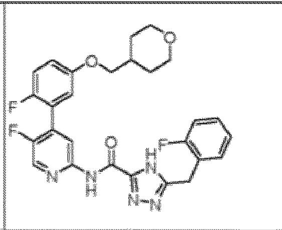 | 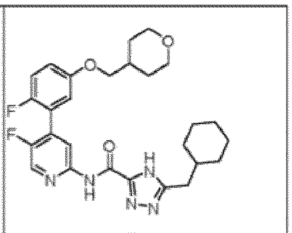 |
| 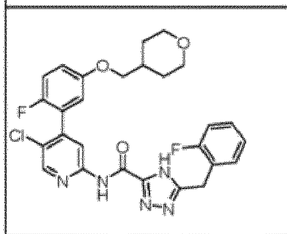 | 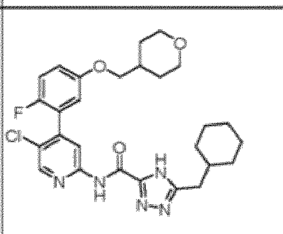 | 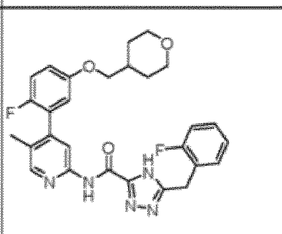 | 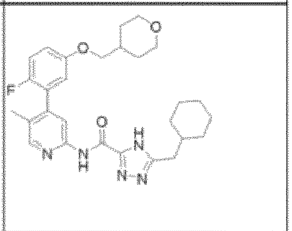 |
| 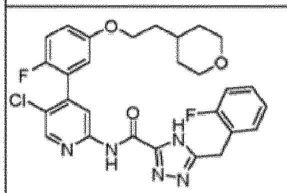 | 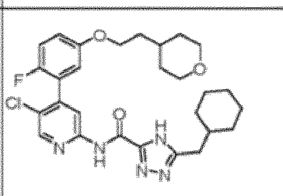 | 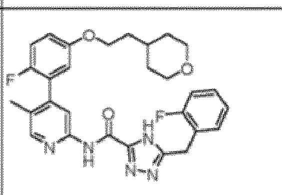 | 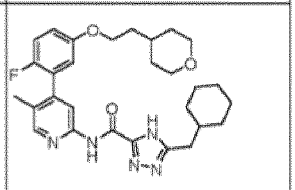 |
| 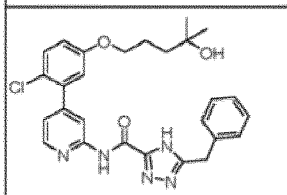 | 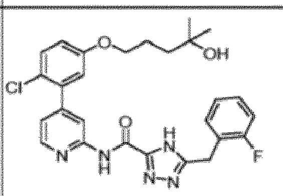 | 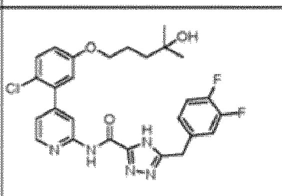 | 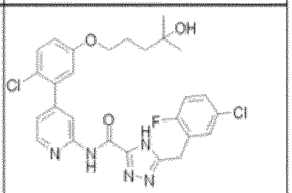 |
| 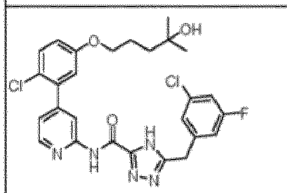 | 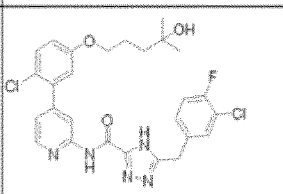 | 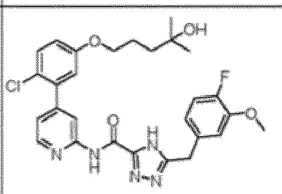 | 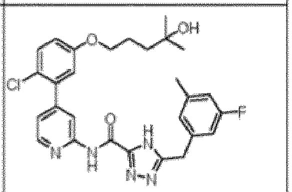 |
| 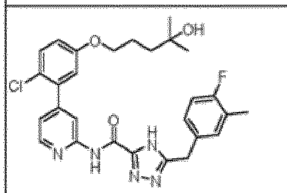 | 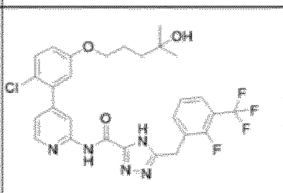 | 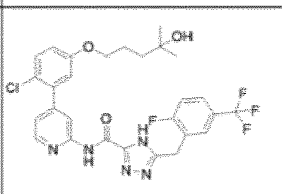 | 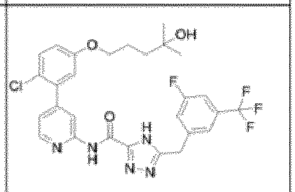 |
| 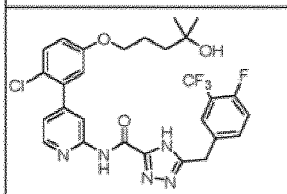 | 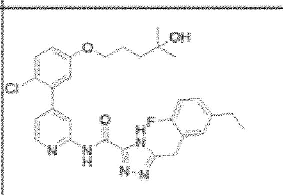 | 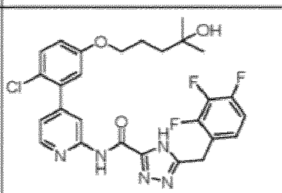 | 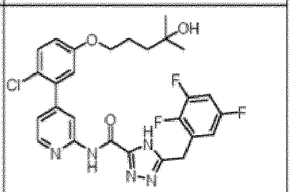 |
| 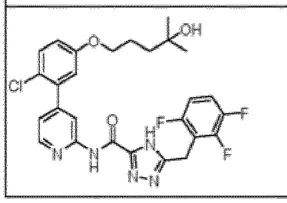 | 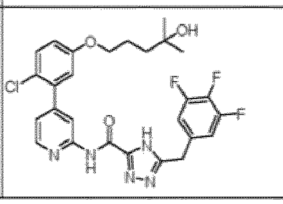 | 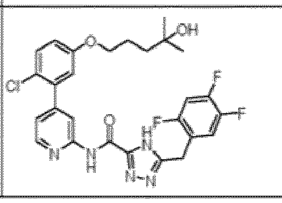 | 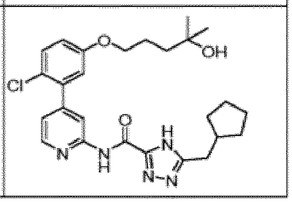 |

| | | | |
|---|---|---|---|
| 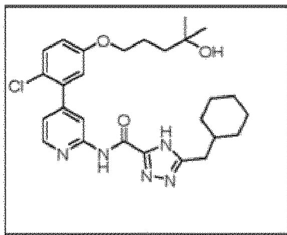 | 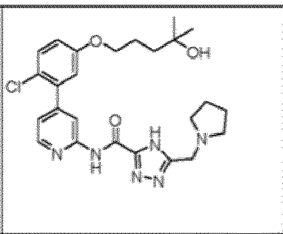 | 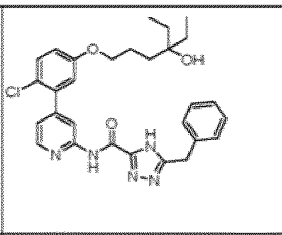 | 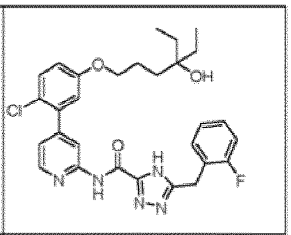 |
| 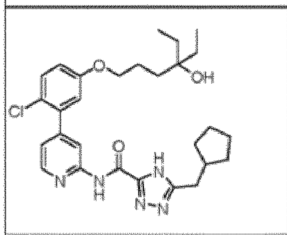 | 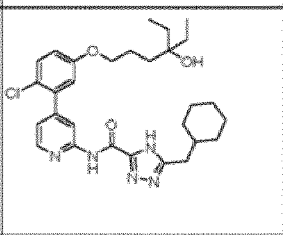 | 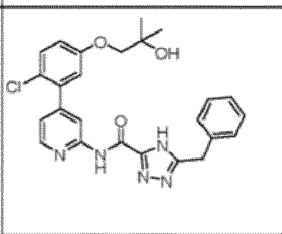 | 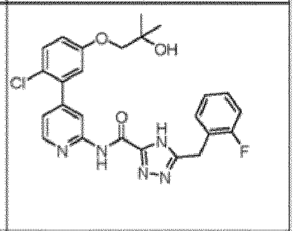 |
| 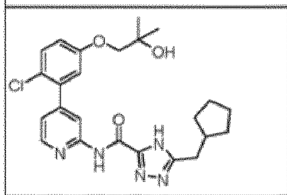 | 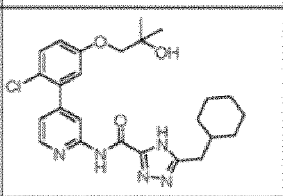 | 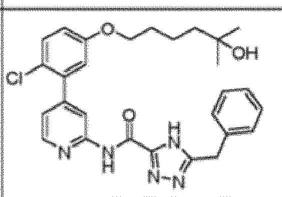 | 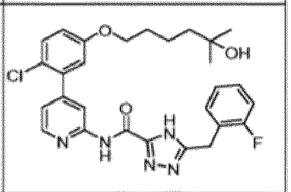 |
| 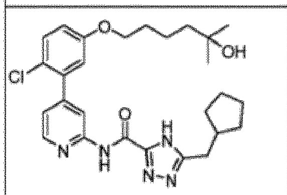 | 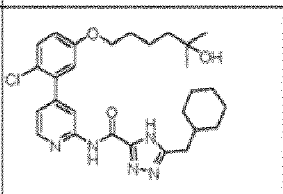 | 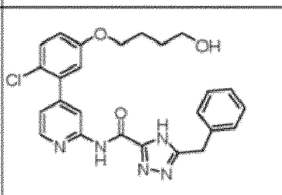 | 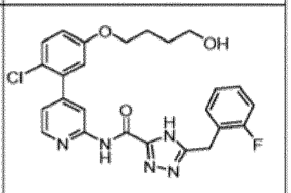 |
| 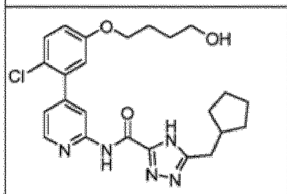 | 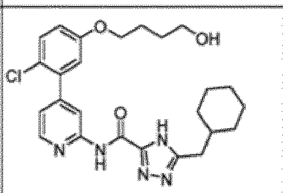 | 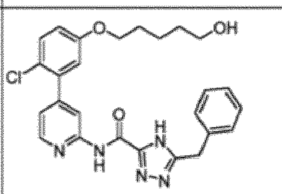 | 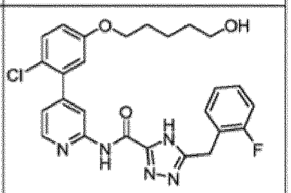 |
| 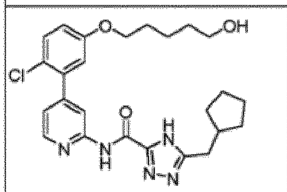 | 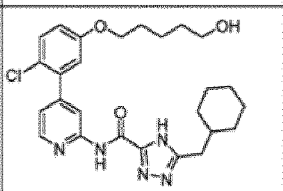 | 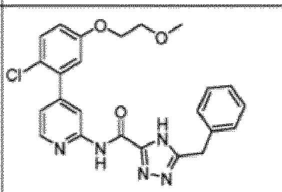 | 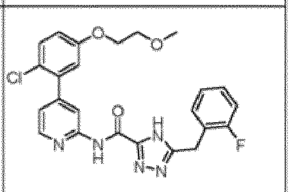 |
| 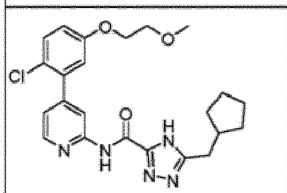 | 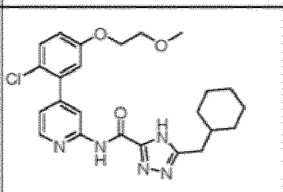 | 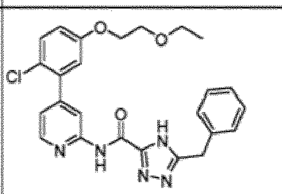 | 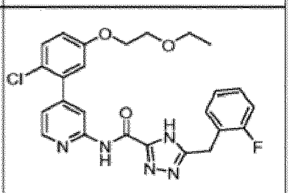 |
| 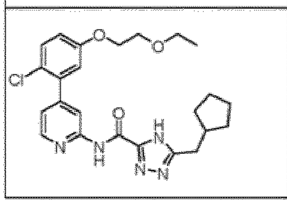 | 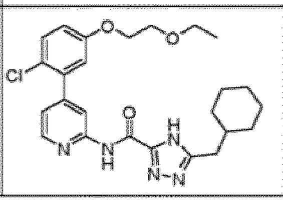 | 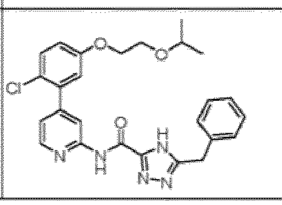 | 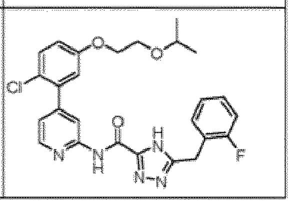 |

| | | | |
|---|---|---|---|
| 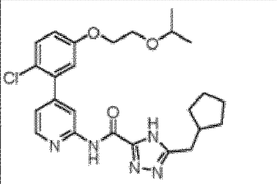 | 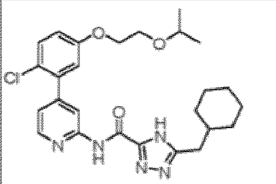 | 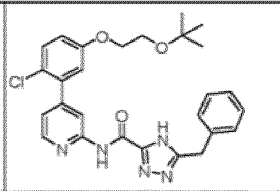 | 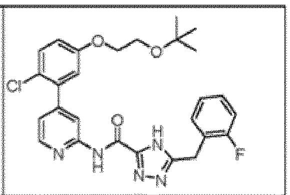 |
| 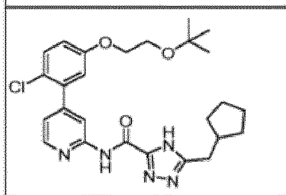 | 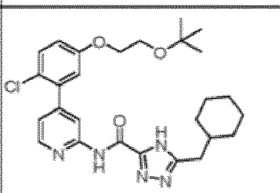 | 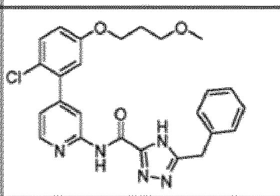 | 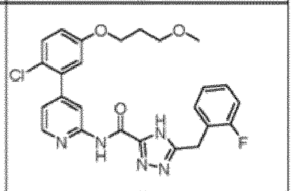 |
| 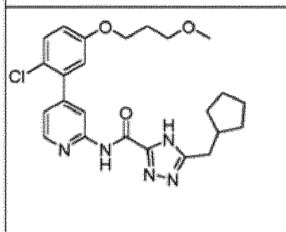 | 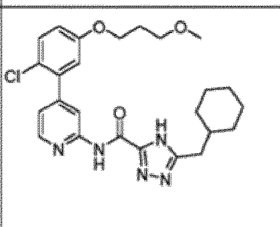 | 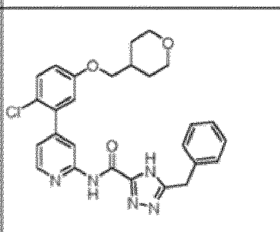 | 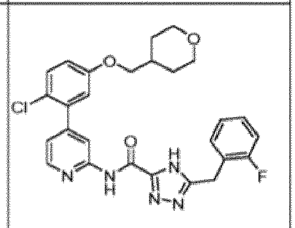 |
| 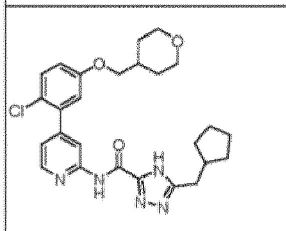 | 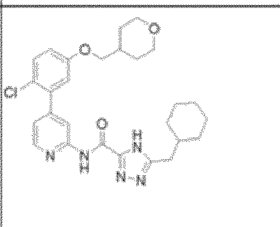 | 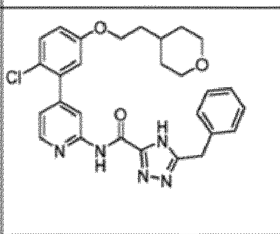 | 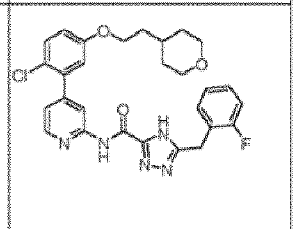 |
| 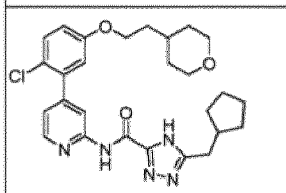 | 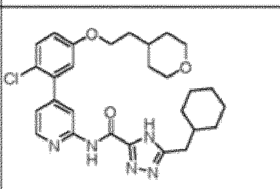 | 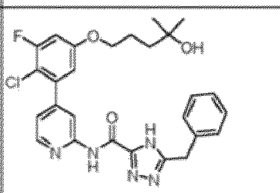 | 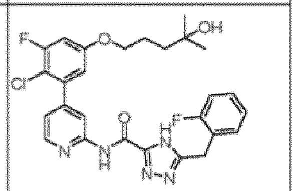 |
| 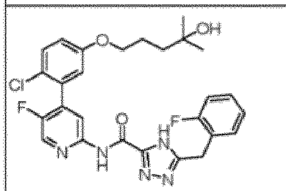 | 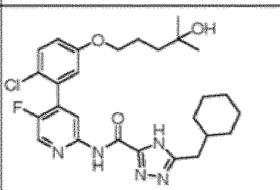 | 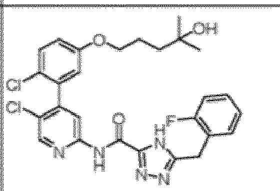 | 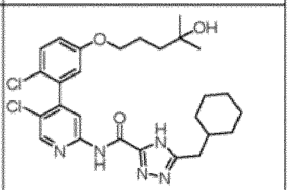 |
| 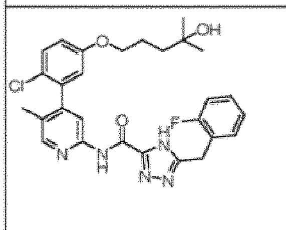 | 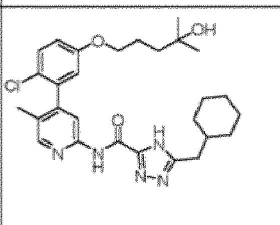 | 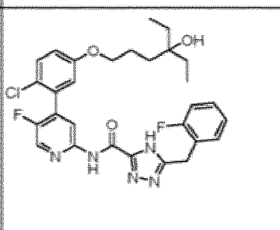 | 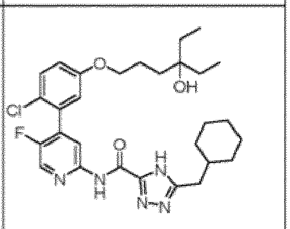 |
| 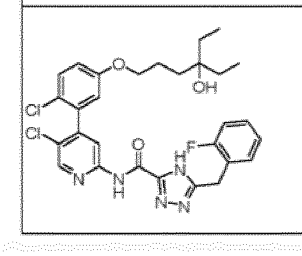 | 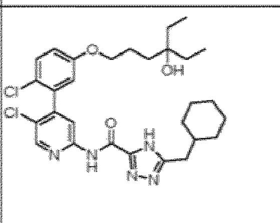 | 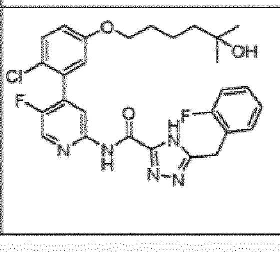 | 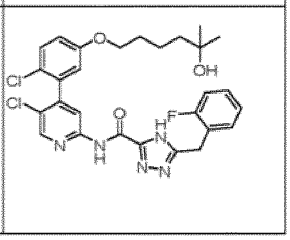 |

| | | | |
|---|---|---|---|
| 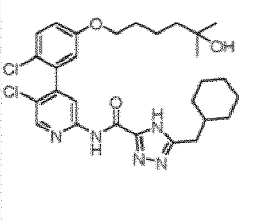 | 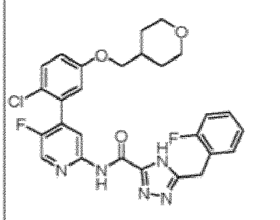 | 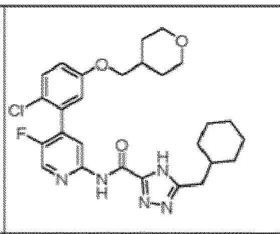 | 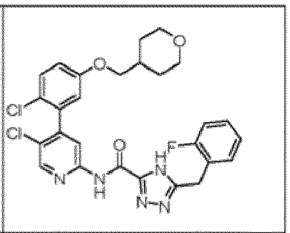 |
| 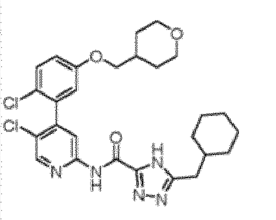 | 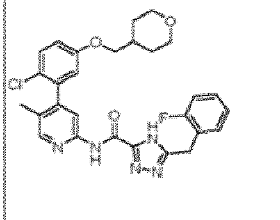 | 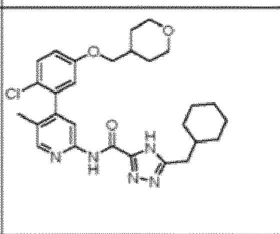 | 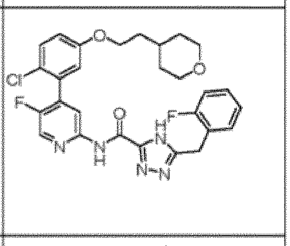 |
| 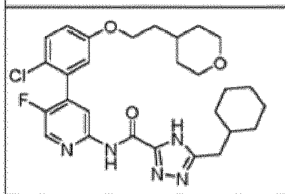 | 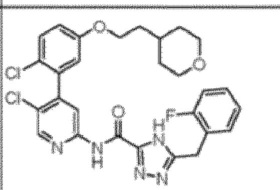 | 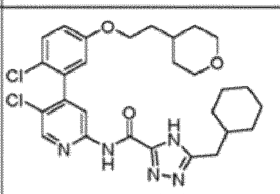 | 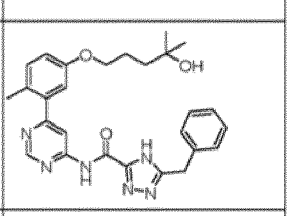 |
| 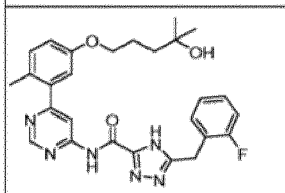 | 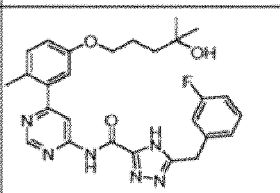 | 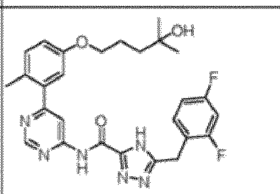 | 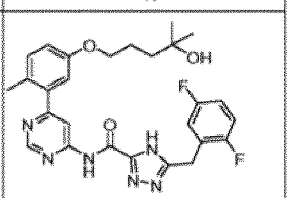 |
| 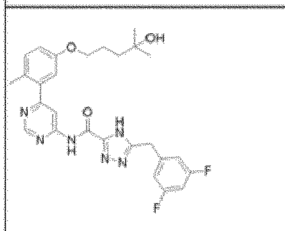 | 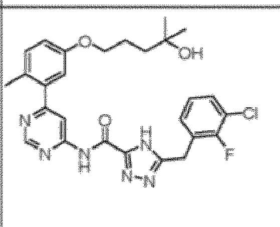 | 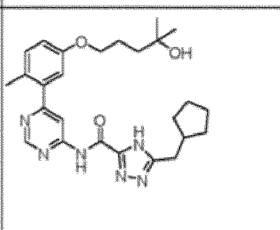 | 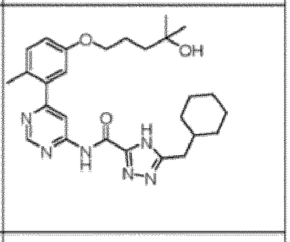 |
| 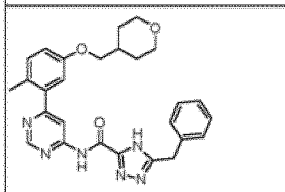 | 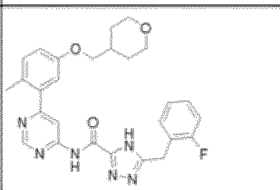 | 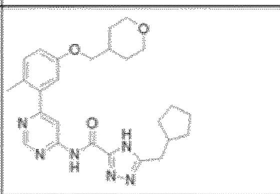 | 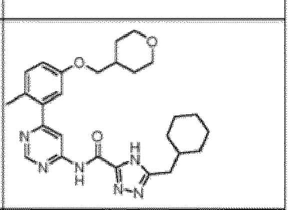 |
| 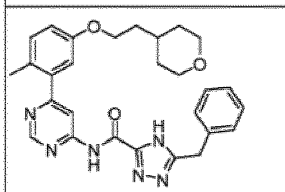 | 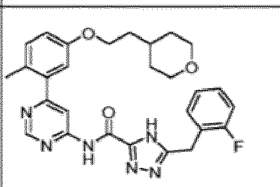 | 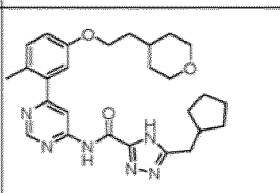 | 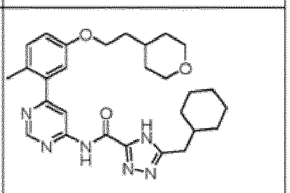 |
| 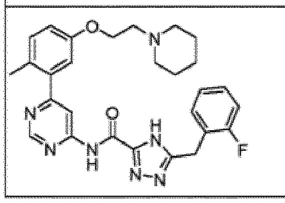 | 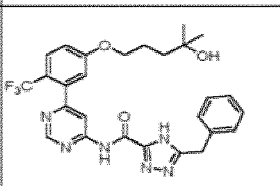 | 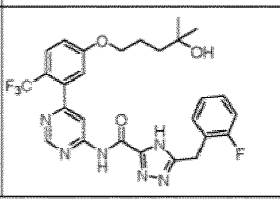 | 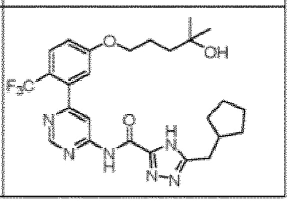 |

| | | | |
|---|---|---|---|
| 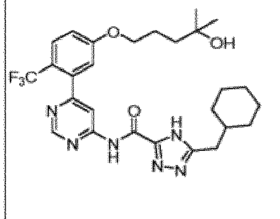 | 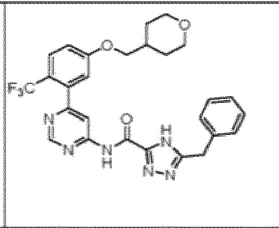 | 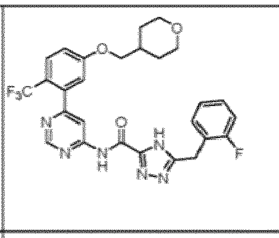 | 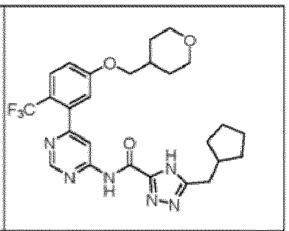 |
| 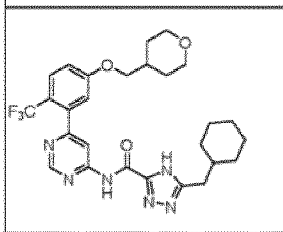 | 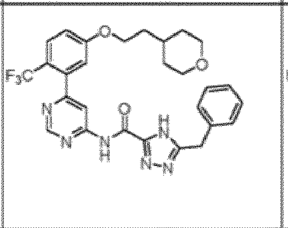 | 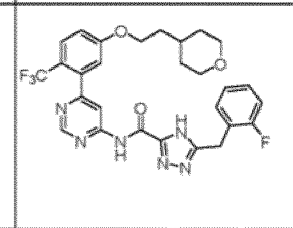 | 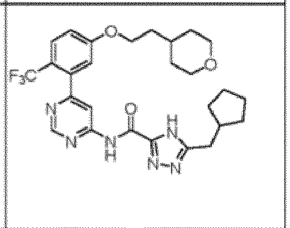 |
| 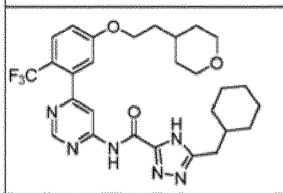 | 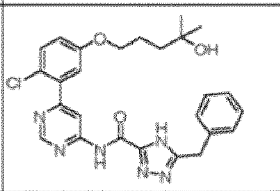 | 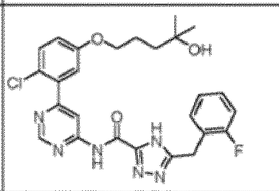 | 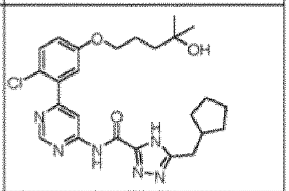 |
| 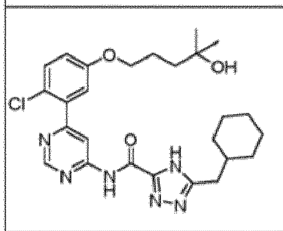 | 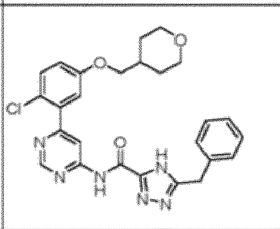 | 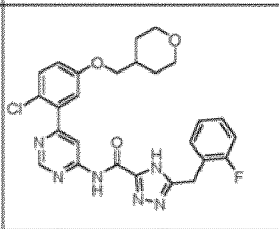 | 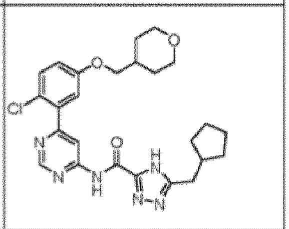 |
| 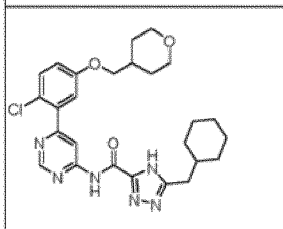 | 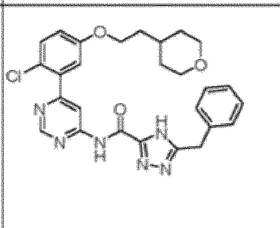 | 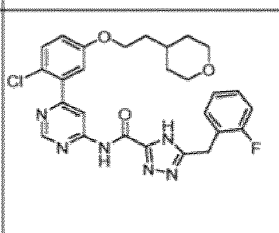 | 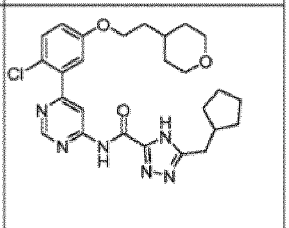 |
| 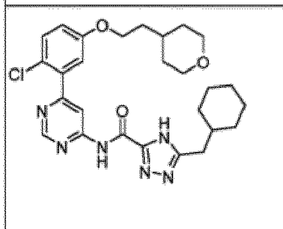 | 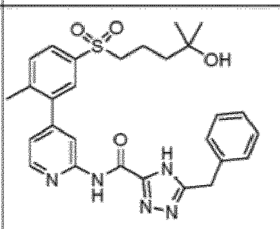 | 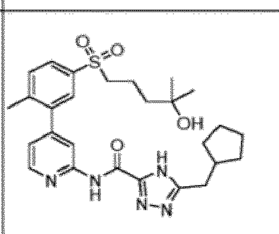 | 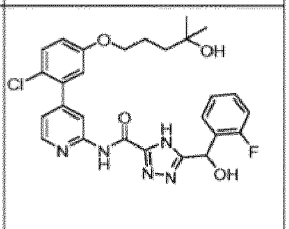 |
| 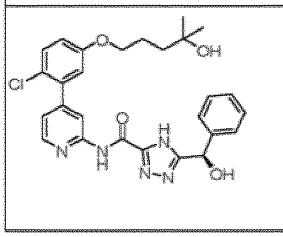 | 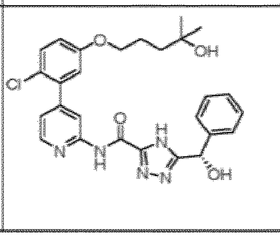 | 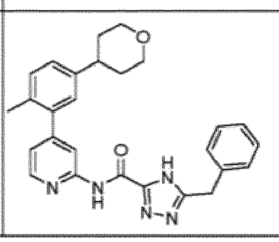 | 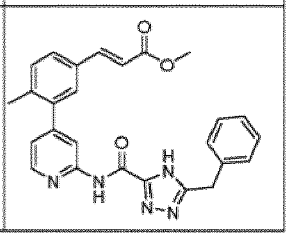 |

| | | | |
|---|---|---|---|
| 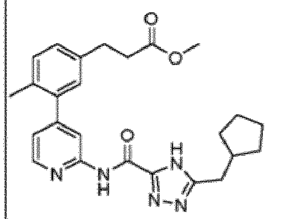 | 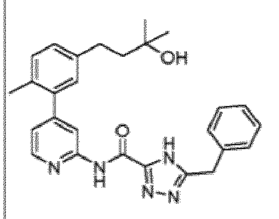 | 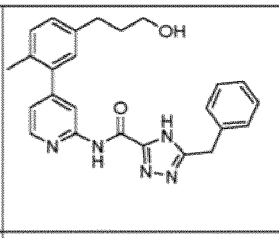 | 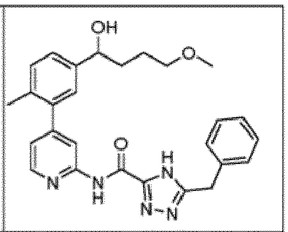 |
| 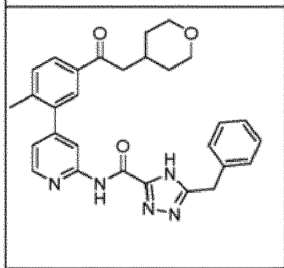 | 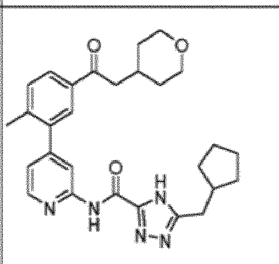 | 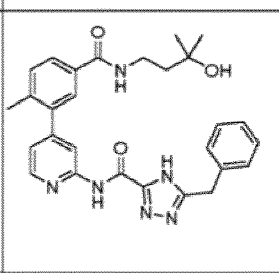 | 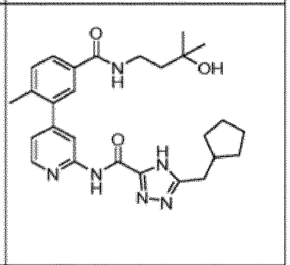 |
| 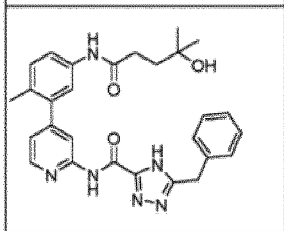 | 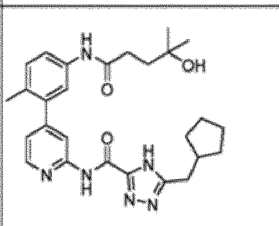 | 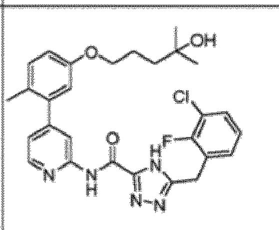 | 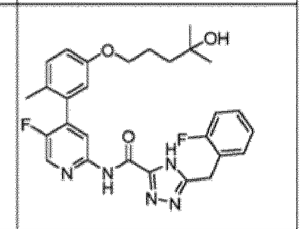 |
| 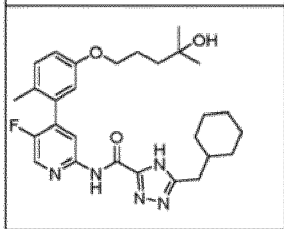 | 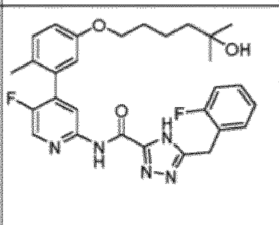 | 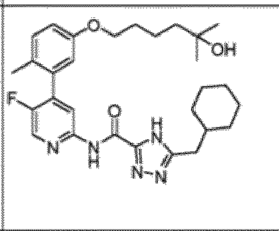 | 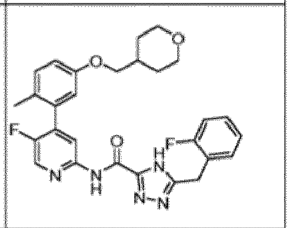 |
| 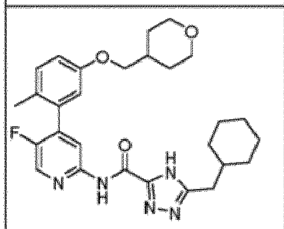 | 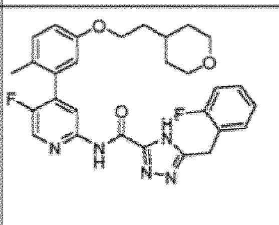 | 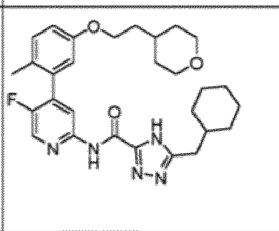 | 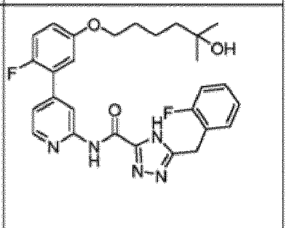 |
| 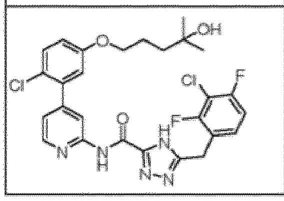 | 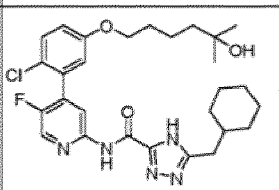 | 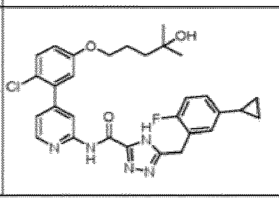 | 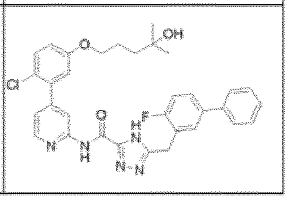 |

**13.** Verbindung, die pharmazeutisch verträglichen Salze, Diastereomere, Enantiomere oder Solvate davon nach einem der Ansprüche 1-12 zur Verwendung bei der Behandlung von RIP 1-bezogenen Krankheiten, wobei die RIP 1-bezogene Krankheit Augenfunduskrankheit, Xerophthalmie, Psoriasis, Leucoderma, Dermatitis, Alopecia areata, rheumatoide Arthritis, Colitis, multiple Sklerose, systemischem Lupus erythematodes, Morbus Crohn, Atherosklerose, Lungenfibrose, Leberfibrose, Myelofibrose, nicht-kleinzelligem Lungenkrebs, kleinzelligem Lungenkrebs, Brustkrebs, Bauchspeicheldrüsenkrebs, Gliom, Glioblastom, Eierstockkrebs, Gebärmutterhalskrebs, kolorektalem Krebs, Melanom, Endometriumkrebs, Prostatakrebs, Blasenkrebs, Leukämie, Magenkrebs, Leberkrebs, Magen-Darm-Stromatumor, Schilddrüsenkrebs, chronisch-myeloische Leukämie, akute myeloische Leukämie, Non-Hodgkin-Lymphom, Nasen-Rachen-Krebs, Speiseröhrenkrebs, Hirntumor, B-Zell- und T-Zell-Lymphom, Lymphom, multiples Myelom, Gallengangskrebs und -sarkom, Gallengangskarzinom, entzündliche Darmerkrankung, Colitis ulcerosa, Netzhautablösung, Retinitis pigmentosa, Makuladegeneration, Bauchspeicheldrüsenentzündung, atopische Dermatitis, Spondylarthritis, Gicht, SoJIA, Sjögren-Syndrom, systemische Sklerodermie, Antiphospholipid-Syndrom, Vaskulitis, Osteoarthritis, nichtalkoholische Steatohepatitis, alkoholische Steatohepatitis, Autoimmunhepatitis,

autoimmune hepatobiliäre Erkrankung, primär sklerosierende Cholangitis, Nephritis, Zöliakie, autoimmune ITP, Transplantatabstoßung, Ischämie-Reperfusionsschäden fester Organe, Sepsis, systemisches Entzündungssyndrom, zerebrovaskulärem Unfall, Myokardinfarkt, Huntington-Krankheit, Alzheimer-Krankheit, Parkinson-Krankheit, allergische Erkrankungen, Asthma, atopische Dermatitis, multiple Sklerose, Typ-I-Diabetes, Wegener-Granulomatose, Lungensarkoidose, Morbus Behcet, Interleukin-1-Converzym-bezogenes Fiebersyndrom, chronisch obstruktive Lungenerkrankung, Tumornekrosefaktor-Rezeptorbezogenes periodisches Syndrom und Parodontitis beinhaltet.

**14.** Pharmazeutische Zusammensetzung, umfassend eine Verbindung, die pharmazeutisch verträglichen Salze, Diastereomere, Enantiomere oder Solvate davon nach einem der Ansprüche 1-12 und einen oder mehrere pharmazeutisch verträgliche Träger oder Hilfsstoffe.

## Revendications

**1.** Composé de formule (I), ou les sels pharmaceutiquement acceptables, diastéréomères, énantiomères, ou solvates de celui-ci,

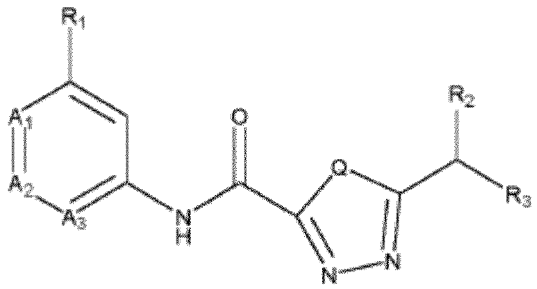

formule (I)

dans la formule (I),

Q est NH, O ou S ;
$A_1$, $A_2$, et $A_3$ sont sélectionnés chacun indépendamment parmi N ou $CR_4$ et au moins un parmi $A_1$, $A_2$, et $A_3$ est N, $R_4$ est H, F, Cl ou méthyle ;
$R_1$ est cycloalkyle en $C_3$-$C_8$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^aR^b$,
ou un groupe hétéroalicyclique de 4 à 8 chaînons, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^aR^b$,
ou aryle ou hétéroaryle, qui est non substitué ou substitué avec 1 à 3 substituants sélectionnés dans le groupe consistant en alkyle en $C_1$-$C_{10}$, halogène, cycloalkyle en $C_3$-$C_8$, alkyle halogéné en $C_1$-$C_{10}$, cyano, hydrogène, alkylthio en $C_1$-$C_6$, -$SO_2$-$R_5$, -SO-$R_5$, -CO-$R_5$, - CONH-$R_5$, -NHCO-$R_5$, -R'-COO-R″, -$NR^aR^b$, un groupe hétéroalicyclique de 4 à 8 chaînons, alcynyle en $C_2$-$C_6$, alcényle en $C_2$-$C_6$, alcoxy en $C_1$-$C_3$, alkylthio en $C_1$-$C_6$, alkyle en $C_1$-$C_{10}$ substitué avec hydroxyle et/ou alcoxy en $C_1$-$C_6$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons, et -O-$R_6$,
le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle, le groupe hétéroalicyclique de 4 à 8 chaînons est un groupe hétéroalicyclique de 4 à 8 chaînons contenant 1 à 2 atomes sélectionnés parmi N, O ou S en tant qu'atomes de cycle,
$R_5$ est hydrogène, hydroxyle, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,
$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ qui est substitué avec 1 à 3 substituants sélectionnés dans le groupe consistant en hydroxyle, alcoxy

en $C_1$-$C_6$, cyano, -$NR^aR^b$, cycloalkyloxy en $C_3$-$C_8$, - CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, et alkylthio en $C_1$-$C_6$,

R' représente alcénylène en $C_2$-$C_6$, ou alkylène en $C_1$-$C_6$,

R″ est hydrogène, alkyle en $C_1$-$C_6$, alcoxy en $C_1$-$C_6$, alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,

$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec monoou di-alkyle en $C_1$-$C_3$ ;

$R_2$ est cycloalkyle en $C_3$-$C_8$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$,

ou cyclyle ponté en $C_7$-$C_{12}$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$,

ou alkyle en $C_1$-$C_{10}$, qui est non substitué ou substitué avec 1 à 3 substituants sélectionnés dans le groupe consistant en alcoxy en $C_1$-$C_6$, alkylthio en $C_1$-$C_6$, acyle en $C_1$-$C_3$, hydroxyle, halogène, cyano, -$CONH_2$, cycloalkyle en $C_3$-$C_8$, et -$NR^cR^d$,

ou - $(CH_2)$n-$R^e$, dans lequel $R^e$ est aryle ou hétéroaryle, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, cycloalkyle en $C_3$-$C_6$, phényle, naphtyle, alcynyle en $C_2$-$C_6$, alcényle en $C_2$-$C_6$, et -$NR^cR^d$, dans lequel n est un nombre entier de 0 à 3,

ou -$(CH_2)$m-$R^f$, dans lequel $R^f$ est un groupe hétéroalicyclique de 4 à 8 chaînons, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$,

m est un nombre entier de 0 à 3,

le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,

le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle,

$R^c$ et $R^d$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec monoou di-alkyle en $C_1$-$C_3$ ;

$R_3$ est hydrogène, alkyle en $C_1$-$C_3$, hydroxyle, halogène, trifluorométhyle, ou cyano.

**2.** Composé, sels pharmaceutiquement acceptables, diastéréomères, énantiomères, ou solvates de celui-ci selon la revendication 1, dans lequel Q est NH, O ou S ;

$A_1$, $A_2$, et $A_3$ sont sélectionnés chacun indépendamment parmi N ou $CR_4$ et au moins un parmi $A_1$, $A_2$, et $A_3$ est N, $R_4$ est H, F, Cl ou méthyle ;

$R_1$ est cycloalkyle en $C_3$-$C_8$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^aR^b$,

ou un groupe hétéroalicyclique de 4 à 8 chaînons, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^aR^b$,

ou aryle ou hétéroaryle, qui est non substitué ou substitué avec 1 à 3 substituants sélectionnés dans le groupe constitué par alkyle en $C_1$-$C_{10}$, halogène, cycloalkyle en $C_3$-$C_8$, alkyle halogéné en $C_1$-$C_{10}$, cyano, hydroxyle, alkylthio en $C_1$-$C_6$, -$SO_2$-$R_5$, -SO-$R_5$, -CO-$R_5$, - CONH-$R_5$, -$NR^aR^b$, un groupe hétéroalicyclique de 4 à 8 chaînons, alcynyle en $C_2$-$C_6$, alcényle en $C_2$-$C_6$, alcoxy en $C_1$-$C_3$, alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en

$C_3$-$C_8$)-O-(alkyle en $C_1$-$C_6$), alkyle en $C_1$-$C_6$ substitué avec un groupe hétéroalicyclique de 4 à 8 chaînons, et -O-$R_6$,
le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle, le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,
$R_5$ est hydrogène, hydroxyle, alkyle en $C_1$-$C_6$, alcoxy en $C_1$-$C_6$, alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,
$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ qui est substitué avec 1 à 3 substituants sélectionnés dans le groupe consistant en hydroxyle, alcoxy en $C_1$-$C_6$, cyano, -$NR^aR^b$, cycloalkyloxy en $C_3$-$C_8$, - CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, et alkylthio en $C_1$-$C_6$,
$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué étant substitué avec mono- ou di-alkyle en $C_1$-$C_3$ ;
$R_2$ est cycloalkyle en $C_3$-$C_8$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$,
ou cyclyle ponté en $C_7$-$C_{12}$, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$,
ou alkyle en $C_1$-$C_{10}$, qui est non substitué ou substitué avec 1 à 3 substituants sélectionnés dans le groupe consistant en alcoxy en $C_1$-$C_6$, alkylthio en $C_1$-$C_6$, acyle en $C_1$-$C_3$, hydroxyle, halogène, cyano, -$CONH_2$, cycloalkyle en $C_3$-$C_8$, et -$NR^cR^d$,
ou -$(CH_2)n$-$R^e$, dans lequel $R^e$ est aryle ou hétéroaryle, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, alcynyle en $C_2$-$C_6$, alcényle en $C_2$-$C_6$, et - $NR^cR^d$, dans lequel n est un nombre entier de 0 à 3,
ou -$(CH_2)m$-$R^f$, dans lequel $R^f$ est un groupe hétéroalicyclique de 4 à 8 chaînons, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, oxo (=O) et -$NR^cR^d$, m est un nombre entier de 0 à 3,
le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,
le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle,
$R^c$ et $R^d$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$ ;
$R_3$ est hydrogène, alkyle en $C_1$-$C_3$, hydroxyle, halogène, trifluorométhyle ou cyano.

**3.** Composé, sels pharmaceutiquement acceptables, diastéréomères, énantiomères, ou solvates de celui-ci selon la revendication 1 ou 2, dans lequel $A_3$ est N, $A_1$ et $A_2$ sont sélectionnés chacun indépendamment parmi N ou $CR_4$, $R_4$ est H, F, Cl ou méthyle.

**4.** Composé, sels pharmaceutiquement acceptables, diastéréomères, énantiomères, ou solvates de celui-ci selon l'une des revendications 1 à 3, dans lequel Q est NH.

**5.** Composé, sels pharmaceutiquement acceptables, diastéréomères, énantiomères, ou solvates de celui-ci selon

l'une des revendications 1 à 4, dans lequel $R_1$ est cycloalkyle en $C_3$-$C_8$, un groupe hétéroalicyclique de 4 à 8 chaînons, ou aryle ou hétéroaryle qui est non substitué ou substitué avec 1 à 3 substituants sélectionnés dans le groupe consistant en alkyle en $C_1$-$C_{10}$, halogène, cycloalkyle en $C_3$-$C_8$, alkyle halogéné en $C_1$-$C_{10}$, cyano, hydroxyle, alkylthio en $C_1$-$C_6$, -CO-$R_5$, -$SO_2$-$R_5$, -SO-$R_5$, - CONH-$R_5$, -$NR^aR^b$, un groupe hétéroalicyclique de 4 à 8 chaînons, alcynyle en $C_2$-$C_6$ alcényle en $C_2$-$C_6$, alcoxy en $C_1$-$C_3$ alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons et -O-$R_6$,

le groupe aryle est phényle, naphtyle,

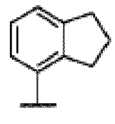,

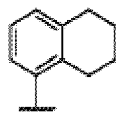,

le groupe hétéroaryle est pyrrolyle, furyle, pyridyle, thiényle, imidazolyle, thiazolyle, isothiazolyle, indazolyle, indolyle, isoindolyle, indolinyle, isoindolinyle, isoquinoléinyle, indolizinyle, isoxazolyle, 1,5-naphtyridinyle, 1,6-naphtyridinonyle, oxadiazolyle, oxazolyle, 1-phényl-1H-pyrrolyle, phénazinyle, phénothiazinyle, phénoxazinyle, phtalazinyle, ptéridinyle, purinyle, pyranyle, pyrazolyle, pyrazolo[3,4-d]pyrimidinyle, pyrido[3,2-d]pyrimidinyle, pyrido[3,4-d]pyrimidinyle, pyrazinyle, pyrimidinyle, pyridazinyle, quinazolinyle, quinoxalinyle, quinoléinyle,

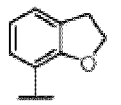, 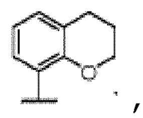, 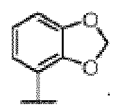,

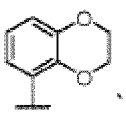 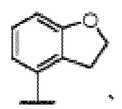 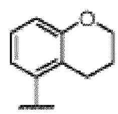,

le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,
$R_5$ est hydrogène, hydroxyle, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,
$R_6$ représente alkyle en $C_1$-$C_{10}$, cycloalkyle en $C_3$-$C_8$, un groupe hétéroalicyclique de 4 à 8 chaînons, ou alkyle en $C_1$-$C_{10}$ qui est non substitué ou substitué avec 1 à 3 substituants sélectionnés dans le groupe consistant en hydroxyle, alcoxy en $C_1$-$C_6$, cyano, -$NR^aR^b$, cycloalkyloxy en $C_3$-$C_8$, -CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -CO-$R_5$, -$SO_2$-$R_5$, -SO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, ou alkylthio en $C_1$-$C_6$,
$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono-ou di-alkyle en $C_1$-$C_3$ ;
de préférence, dans lequel, $R_1$ est cyclopropyle, cyclobutyle, cyclopentyle, cyclohexyle, oxétan-3-yle, tétrahydrofuran-3-yle, tétrahydropyran-4-yle, tétrahydropyran-3-yle, pyrrolidinyle, pipéridinyle, pipérazinyle, morpholinyle, thiomorpholinyle, ou aryle ou hétéroaryle qui est non substitué ou substitué avec 1 à 3 substituants sélectionnés dans le groupe consistant en alkyle en $C_1$-$C_{10}$, halogène, cycloalkyle en $C_3$-$C_8$, alkyle halogéné

en $C_1$-$C_{10}$, cyano, hydroxyle, alkylthio en $C_1$-$C_6$, -$SO_2$-$R_5$, -SO-$R_5$, -CO-$R_5$, -CONH-$R_5$, - $NR^aR^b$, un groupe hétéroalicyclique de 4 à 8 chaînons, alcynyle en $C_2$-$C_6$, alcényle en $C_2$-$C_6$, alcoxy en $C_1$-$C_3$ alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons, et -O-$R_6$,
le groupe aryle est phényle,

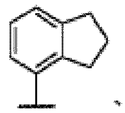 , 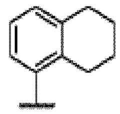 ,

le groupe hétéroaryle est pyrazolyle, pyridyle, pyrimidinyle, thiazolyle, oxazolyle,

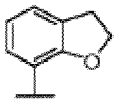 , 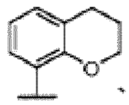 ,

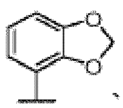 , 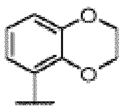 , 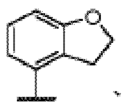 , 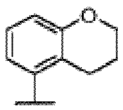

, le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,
$R_5$ est hydrogène, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,
$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ non substitué ou substitué avec 1 à 3 substituants sélectionnés dans le groupe consistant en hydroxyle, alcoxy en $C_1$-$C_6$, cyano, -$NR^aR^b$, cycloalkyloxy en $C_3$-$C_8$, - CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, et alkylthio en $C_1$-$C_6$,
$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$ ;
de préférence, dans lequel, $R_1$ est

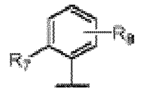 ,

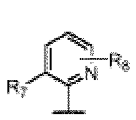 , 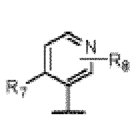 , 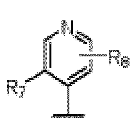 , 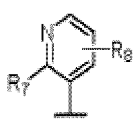 , 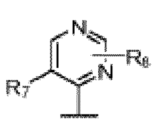 , 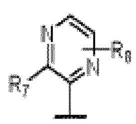 , ou

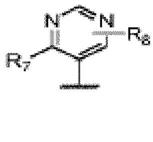 ,

$R_7$ est hydrogène, alkyle en $C_1$-$C_4$, alcoxy en $C_1$-$C_3$, halogène, hydroxyle, trifluorométhyle, trifluorométhoxy, cyano, alcynyle en $C_2$-$C_4$, alcényle en $C_2$-$C_4$, cycloalkyle en $C_3$-$C_4$, ou acyle en $C_1$-$C_3$ ;

$R_8$ est hydrogène, alkyle en $C_1$-$C_{10}$, halogène, cycloalkyle en $C_3$-$C_8$, alkyle halogéné en $C_1$-$C_{10}$, cyano, hydroxyle, alkylthio en $C_1$-$C_6$, -CO-$R_5$, -$SO_2$-$R_5$, -SO-$R_5$, - CONH-$R_5$, -$NR^aR^b$, un groupe hétéroalicyclique de 4 à 8 chaînons, alcynyle en $C_2$-$C_6$, alcényle en $C_2$-$C_6$, alcoxy en $C_1$-$C_3$, alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons, ou -O-$R_6$,

le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,

$R_5$ est hydrogène, hydroxyle, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,

$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ qui est non substitué ou substitué avec 1 à 3 substituants sélectionnés dans le groupe consistant en hydroxyle, alcoxy en $C_1$-$C_6$, cyano, -$NR^aR^b$, cycloalkyloxy en $C_3$-$C_8$, -CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, alkylthio en $C_1$-$C_6$,

$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$ ;

de préférence, dans lequel,

$R_1$ est

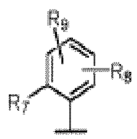 , 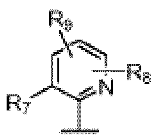 , 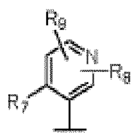 , 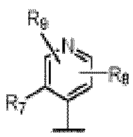 , 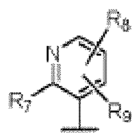 ,

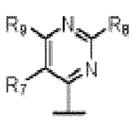 , 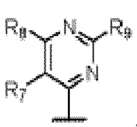 , 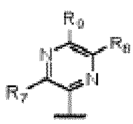 , 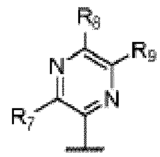 , 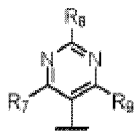 ou 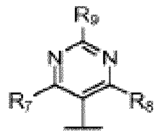 ;

$R_7$ est hydrogène, alkyle en $C_1$-$C_4$, alcoxy en $C_1$-$C_3$, halogène, hydroxyle, trifluorométhyle, trifluorométhoxy, cyano, alcynyle en $C_2$-$C_4$, alcényle en $C_2$-$C_4$, cycloalkyle en $C_3$-$C_4$, ou acyle en $C_1$-$C_3$ ;

$R_8$ est hydrogène, alkyle en $C_1$-$C_{10}$, halogène, cycloalkyle en $C_3$-$C_8$, alkyle halogéné en $C_1$-$C_{10}$, cyano, hydroxyle, alkylthio en $C_1$-$C_6$, -CO-$R_5$, -$SO_2$-$R_5$, -SO-$R_5$, - CONH-$R_5$, -$NR^aR^b$, un groupe hétéroalicyclique de 4 à 8 chaînons, alcynyle en $C_2$-$C_6$, alcényle en $C_2$-$C_6$, alcoxy en $C_1$-$C_3$, alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons, ou -O-$R_6$,

le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,

$R_5$ est hydrogène, hydroxyle, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, ou alkyle en $C_1$-$C_6$ substitué avec un groupe hétéroalicyclique de 4 à 8 chaînons,

$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ qui est non substitué ou substitué avec 1 à 3 substituants sélectionnés dans le groupe consistant en hydroxyle, alcoxy en $C_1$-$C_6$, cyano, -$NR^aR^b$, cycloalkyloxy en $C_3$-$C_8$, -CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un

groupe hétéroalicyclique de 4 à 8 chaînons substitué avec hydroxyle et/ou alkyle en $C_1$-$C_4$, et un alkylthio en $C_1$-$C_6$,

$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$ ;

$R_9$ est hydrogène, alkyle en $C_1$-$C_4$, alcoxy en $C_1$-$C_3$, halogène, hydroxy, trifluorométhyle, trifluorométhoxy, cyano, alcynyle en $C_2$-$C_4$, alcényle en $C_2$-$C_4$, cycloalkyle en $C_3$-$C_4$, ou un acyle en $C_1$-$C_3$ ;

de préférence, dans lequel, $R_1$ est :

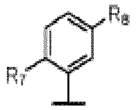,

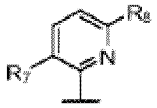, 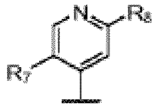, 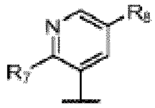, 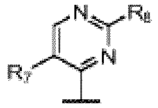, ou 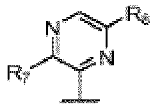,

$R_7$ est hydrogène, méthyle, éthyle, propyle, isopropyle, méthoxy, éthoxy, propoxy, isopropoxy, fluor, chlore, hydroxyle, trifluorométhyle, trifluorométhoxy, cyano, éthynyle, propynyle, vinyle, propènyle, cyclopropyle, cyclobutyle, formyle, ou acétyle ;

$R_8$ est hydrogène, alkyle en $C_1$-$C_{10}$, halogène, cycloalkyle en $C_3$-$C_8$, alkyle halogéné en $C_1$-$C_{10}$, cyano, hydroxyle, alkylthio en $C_1$-$C_6$, -CO-$R_5$, -$SO_2$-$R_5$, -SO-$R_5$, - CONH-$R_5$, -$NR^aR^b$, un groupe hétéroalicyclique de 4 à 8 chaînons, alcynyle en $C_2$-$C_6$, alcényle en $C_2$-$C_6$, alcoxy en $C_1$-$C_3$, alkylthio en $C_1$-$C_6$, alkyle en $C_1$-$C_{10}$ substitué avec hydroxyle, alcoxy en $C_1$-$C_6$ alkyle en $C_1$-$C_{10}$, cycloalkyle en $C_3$-$C_8$ alkyle en $C_1$-$C_6$, (cycloalkyle en $C_3$-$C_8$)-O-(alkyle en $C_1$-$C_6$), alkyle en $C_1$-$C_6$ substitué avec un groupe hétéroalicyclique de 4 à 8 chaînons, ou -O-$R_6$,

le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle,

$R_5$ est hydrogène, hydroxyle, alkyle en $C_1$-$C_6$, alcoxy en $C_1$-$C_6$ alkyle en $C_1$-$C_6$, alkyle en $C_1$-$C_6$ substitué avec hydroxyle, cycloalkyle en $C_3$-$C_8$, alkyle en $C_1$-$C_6$ substitué avec un groupe hétéroalicyclique de 4 à 8 chaînons,

$R_6$ est alkyle en $C_1$-$C_{10}$, cycloalkyle en $C_3$-$C_8$, un groupe hétéroalicyclique de 4 à 8 chaînons, ou alkyle en $C_1$-$C_{10}$ substitué avec 1 à 3 substituants sélectionnés parmi hydroxyle, alcoxy en $C_1$-$C_6$, cyano, -$NR^aR^b$, cycloalkyloxy en $C_3$-$C_8$, -CONH-$R_5$, cycloalkyle en $C_3$-$C_8$, cycloalkyle en $C_3$-$C_8$ substitué avec hydroxyle et/ou alkyle en $C_1$-$C_4$, carboxylique, halogène, alcoxy halogéné en $C_1$-$C_6$, -$SO_2$-$R_5$, -SO-$R_5$, -CO-$R_5$, alcynyle en $C_2$-$C_6$, alcényle en $C_2$-$C_6$, alcoxy en $C_1$-$C_4$ alcoxy en $C_1$-$C_6$, un groupe hétéroalicyclique de 4 à 8 chaînons, un groupe hétéroalicyclique de 4 à 8 chaînons substitué avec oxo, un groupe hétéroalicyclique de 4 à 8 chaînons substitué avec hydroxyle et/ou alkyle en $C_1$-$C_4$, et alkylthio en $C_1$-$C_6$,

$R^a$ et $R^b$ sont sélectionnés chacun indépendamment parmi hydrogène, alkyle en $C_1$-$C_6$, cycloalkyle en $C_3$-$C_8$, alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$.

**6.** Composé, sels pharmaceutiquement acceptables, diastéréomères, énantiomères, ou solvates de celui-ci selon l'une des revendications 1 à 5, dans lequel $R_2$ est cycloalkyle en $C_3$-$C_8$, qui est non substitué ou substitué avec 1 à 3 substituants sélectionnés dans le groupe consistant en méthyle, éthyle, propyle, isopropyle, méthoxy, éthoxy, propoxy, isopropoxy, méthylthio, éthylthio, propylthio, formyle, acétyle, hydroxyle, fluor, chlore, trifluorométhyle, cyano, -$CONH_2$, oxo (=O), amino, diméthylamino, et diéthylamino,

ou cyclyle ponté en $C_7$-$C_{10}$, qui est non substitué ou substitué avec 1 à 3 substituants sélectionnés dans le groupe consistant en méthyle, éthyle, propyle, isopropyle, méthoxy, éthoxy, propoxy, isopropoxy, méthylthio, éthylthio, propylthio, formyle, acétyle, hydroxyle, fluor, chlore, trifluorométhyle, cyano, - $CONH_2$, oxo (=O), amino, diméthylamino, et diéthylamino,

ou alkyle en $C_1$-$C_{10}$, qui est non substitué ou substitué avec 1 à 3 substituants sélectionnés dans le groupe consistant en méthoxy, éthoxy, propoxy, isopropoxy, méthylthio, éthylthio, propylthio, formyle, acétyle, hydroxyle, fluor, chlore, cyano, -$CONH_2$, cyclopropyle, cyclobutyle, cyclopentyle, cyclohexyle, amino, diméthylamino, et diéthylamino,
ou -$(CH_2)$n-$R^e$, dans lequel $R^e$ est aryle ou hétéroaryle, qui est non substitué ou substitué avec 1 à 3 substituants sélectionnés dans le groupe consistant en méthyle, éthyle, propyle, isopropyle, méthoxy, éthoxy, propoxy, isopropoxy, méthylthio, éthylthio, propylthio, formyle, acétyle, hydroxyle, fluor, chlore, trifluorométhyle, cyano, -$CONH_2$, éthynyle, vinyle, amino, diméthylamino, et diéthylamino, n est un nombre entier de 0 à 3,
ou -$(CH_2)$m-$R^f$, dans lequel $R^f$ est un groupe hétéroalicyclique de 4 à 8 chaînons, qui est non substitué ou substitué avec 1 à 3 substituants sélectionnés dans le groupe consistant en méthyle, éthyle, propyle, isopropyle, méthoxy, éthoxy, propoxy, isopropoxy, méthylthio, éthylthio, propylthio, formyle, acétyle, hydroxyle, fluor, chlore, trifluorométhyle, cyano, -$CONH_2$, oxo (=O), amino, diméthylamino, et diéthylamino, m est un nombre entier de 0 à 3,
le groupe hétéroalicyclique à 4 à 8 chaînons contient 1 à 2 atomes sélectionnés parmi N, O, ou S en tant qu'atomes de cycle, le groupe aryle est phényle, et le groupe hétéroaryle est pyridyle, pyrimidinyle, pyrazolyle, oxazolyle, isoxazolyle, thiazolyle.

**7.** Composé, sels pharmaceutiquement acceptables, diastéréomères, énantiomères, ou solvates de celui-ci selon la revendication 6, dans lequel $R_2$ est cyclopropyle, cyclobutyle, cyclopentyle, cyclohexyle, cycloheptyle, cyclooctyle, 4,4-difluorocyclohexyle, bicyclo[2.2.1]heptyle, adamantyle, méthyle, éthyle, propyle, isopropyle, butyle, isobutyle, sec-butyle, tert-butyle, pentyle, néopentyle, hexyle, 2-hydroxy-2-méthylpropyle, 3,3-diméthylbutyle, 3-hydroxy-3-méthylbutyle, cyclopropylméthyle, cyclopropyléthyle, cyclopropylpropyle, cyclobutylméthyle, cyclobutyléthyle, cyclobutylpropyle, cyclopentylméthyle, cyclopentyl-éthyle, cyclopentylpropyle, cyclohexylméthyle, cyclohexyléthyle, cyclohexylpropyle, benzyle, phénéthyle, phényle, 2-fluorophényle, 2-chlorophényle, 2-méthoxyphényle, 2-cyanophényle, 2-éthynylphényle, 3-fluorophényle, 3-chlorophényle, 3-méthoxyphényle, 3-cyanophényle, 3-éthynylphényle, 4-fluorophényle, 4-chlorophényle, 4-méthoxyphényle, 4-cyanophényle, 4-éthynylphényle, 3,4-difluorophényle, 3-cyano-4-méthylphényle, pyridin-2-yle, pyridin-3-yle, pyridin-4-yle, pyrimidin-2-yle, pyrimidin-4-yle, pyrimidin-5-yle, pyrazinyle, pyrazolyle, oxazolyle, isoxazolyle, thiazolyle, oxétan-3-yle, tétrahydrofuran-3-yle, tétrahydro-2H-pyran-4-yle, tétrahydropyrrolyle, pipéridin-1-yle, pipérazin-1-yle, morpholin-4-yle, méthylpipérazin-4-yle, 1-méthylpipéridin-4-yle, 1-acétylpipéridin-4-yle, 4-hydroxypipéridin-1-yle, 4-méthyl-4-hydroxypipéridin-1-yle,

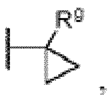, 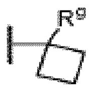,

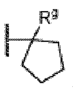, 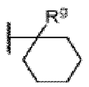, 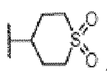, 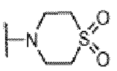, 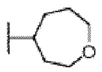, 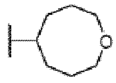, 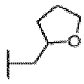, 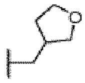, 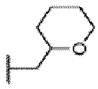,

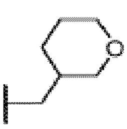, ou 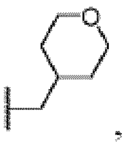,

$R^g$ est -$CH_3$ ou -OH.

**8.** Composé, sels pharmaceutiquement acceptables, diastéréomères, énantiomères, ou solvates de celui-ci selon la revendication 1, dans lequel $R_2$ est 2,3-difluorophényle, 3,5-difluorophényle, 2,5-difluorophényle, 2,4-difluorophényle, 2,3,4-trifluorophényle, 2,3,5-trifluorophényle, 2,3,6-trifluorophényle, 3,4,5-trifluorophényle, 2,4,5-trifluorophényle, 3-chloro-2-fluorophényle, 2-chloro-3-fluorophényle, 5-chloro-2-fluorophényle, 2-chloro-5-fluorophényle, 5-chloro-3-fluorophényle, 3-chloro-5-fluorophényle, 3-chloro-4-fluorophényle, 4-chloro-3-fluorophényle, 2-chloro-4-fluorophényle, 4-chloro-2-fluorophényle, 3-chloro-2,4-difluorophényle, 5-chloro-2,4-difluorophényle, 3-chloro-2,5-difluorophényle, 3-chloro-2,6-difluorophényle, 3-chloro-4,5-difluorophényle, 2-chloro-4,5-difluorophényle, 2-chloro-3,4-difluorophényle, 2-chloro-3,5-difluorophényle, 2-chloro-3,6-difluorophényle, 4-chloro-2,3-difluorophényle, 4-chloro-3,5-difluorophényle, 4-chloro-2,5-difluorophényle, 5-chloro-2,3-difluorophényle, 5-chloro-3,4-difluorophényle, 6-chloro-2,3-difluorophényle, 3-fluoro-5-méthylphényle, 4-fluoro-3-méthylphényle, 2-fluoro-3-méthylphényle, 2-fluoro-4-méthylphényle, 2-fluoro-5-méthylphényle, 3-fluoro-4-méthylphényle, 3-fluoro-2-méthylphényle, 3-fluoro-5-méthoxyphényle, 4-fluoro-3-méthoxyphényle, 2-fluoro-3-méthoxyphényle, 2-fluoro-4-méthoxyphényle, 2-fluoro-5-méthoxyphényle, 3-fluoro-4-méthoxyphényle, 3-fluoro-2-méthoxyphényle, 2-fluoro-3-trifluorométhylphényle, 2-fluoro-4-trifluorométhyl-phényle, 2-fluoro-5-trifluorométhylphényle, 3-fluoro-2-trifluorométhylphényle, 4-fluoro-2-trifluorométhyl-phényle, 5-fluoro-2-trifluorométhylphényle, 4-fluoro-3-trifluorométhylphényle, 3-fluoro-4-trifluorométhyl-phényle, 3-fluoro-5-trifluorométhylphényle, 2-fluoro-5-éthylphényle, 2-fluoro-5-cyclopropylphényle, ou 2-fluoro-5-phénylphényle.

**9.** Composé, sels pharmaceutiquement acceptables, diastéréomères, énantiomères, ou solvates de celui-ci selon la revendication 1, dans lesquels $R_3$ est hydrogène, méthyle, éthyle, hydroxyle, cyano, trifluorométhyle, fluor, ou un chlore.

**10.** Composé, sels pharmaceutiquement acceptables, diastéréomères, énantiomères, ou solvates de celui-ci selon la revendication 1,

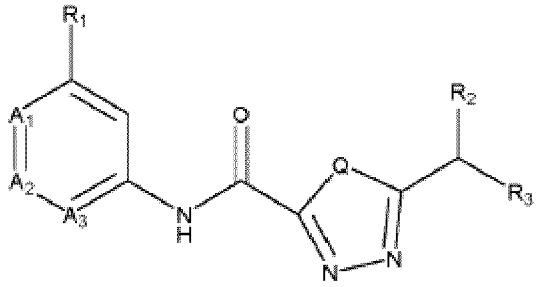

formule (I)

dans la formule (I),

Q est NH ;
$A_1$ et $A_2$ sont chacun CH ;
$A_3$ est N ;
$R_1$ est

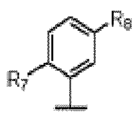 , 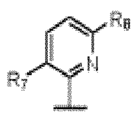 , 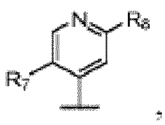 , 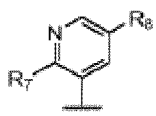 , 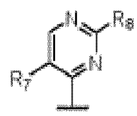 ,

ou 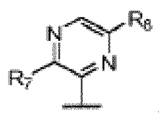 ;

$R_7$ est méthyle, éthyle, propyle, isopropyle, fluor, chlore, trifluorométhyle, vinyle, ou propènyle ;
$R_8$ est cyano, alcoxy en $C_1$-$C_3$ alkylthio en $C_1$-$C_6$, ou -O-$R_6$ ;
$R_5$ est alkyle en $C_1$-$C_6$ ;
$R_6$ est un groupe hétéroalicyclique de 4 à 8 chaînons, ou alkyle en $C_1$-$C_{10}$ qui est substitué avec 1 à 3 substituants sélectionnés dans le groupe consistant en hydroxyle, alcoxy en $C_1$-$C_6$, cyano, cycloalkyloxy en $C_3$-$C_8$, cycloalkyle en $C_3$-$C_8$, -$SO_2$-$R_5$, un groupe hétéroalicyclique de 4 à 8 chaînons, et alkylthio en $C_1$-$C_6$ ;
$R_2$ est -$(CH_2)$n-$R^e$, dans lequel $R^e$ est aryle ou hétéroaryle, qui est non substitué ou substitué avec 1 à 3 substituants sélectionnés dans le groupe consistant en alkyle en $C_1$-$C_6$, alcoxy en $C_1$-$C_6$, alkylthio en $C_1$-$C_6$, acyle en $C_1$-$C_3$, hydroxyle, halogène, trifluorométhyle, cyano, -$CONH_2$, alcynyle en $C_2$-$C_6$, alcényle en $C_2$-$C_6$, et - $NR^cR^d$, dans lequel n est un nombre entier de 0 à 3,
$R^c$ et $R^d$ sont sélectionnés chacun indépendamment parmi hydrogène, alkyle en $C_1$-$C_6$, cycloalkyle en $C_3$-$C_8$,

alkyle en $C_1$-$C_6$ substitué avec alcoxy en $C_1$-$C_6$, alkyle en $C_1$-$C_6$ substitué avec hydroxyle, cycloalkyle en $C_3$-$C_8$ alkyle en $C_1$-$C_6$, alkyle en $C_1$-$C_6$ substitué avec un groupe hétéroalicyclique de 4 à 8 chaînons, alkyle en $C_1$-$C_6$ substitué avec alkylthio en $C_1$-$C_3$, ou alkyle en $C_1$-$C_6$ substitué avec amino substitué ou amino non substitué, dans lequel l'amino substitué est substitué avec mono- ou di-alkyle en $C_1$-$C_3$ ;
le groupe hétéroalicyclique de 4 à 8 chaînons est un groupe hétéroalicyclique de 4 à 8 chaînons contenant 1 à 2 atomes sélectionnés parmi N, O, ou S en tant qu'atomes de cycle ;
le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle ;
$R_3$ est hydrogène.

**11.** Composé, sels pharmaceutiquement acceptables, diastéréomères, énantiomères, ou solvates de celui-ci selon la revendication 10, dans lequel

Q est NH ;
$A_1$ et $A_2$ sont chacun CH ;
$A_3$ est N ;
$R_1$ est

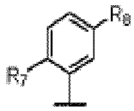, 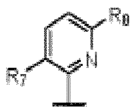, 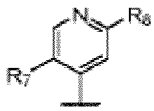, 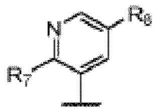, 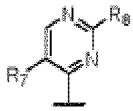,

ou

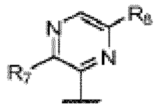 ;

$R_7$ est méthyle, chlore, ou trifluorométhyle ;
$R_8$ est cyano, alcoxy en $C_1$-$C_3$ alkylthio en $C_1$-$C_6$, ou -O-$R_6$ ;
$R_6$ est alkyle en $C_1$-$C_{10}$ qui est substitué avec 1 à 3 substituants sélectionnés parmi hydroxyle ;
$R_2$ est -$(CH_2)$n-$R^e$, dans lequel $R^e$ est aryle ou hétéroaryle, qui est non substitué ou substitué avec 1 à 3 substituants sélectionnés dans le groupe consistant méthyle, éthyle, propyle, isopropyle, méthoxy, éthoxy, propoxy, isopropoxy, méthylthio, éthylthio, propylthio, formyle, acétyle, hydroxyle, fluor, chlore, trifluorométhyle, cyano, -$CONH_2$, éthynyle, vinyle, amino, diméthylamino, et diéthylamino, n est un nombre entier de 0 à 3,
le groupe aryle est un groupe monocyclique ou bicyclique contenant 6 à 12 atomes de carbone de cycle et ayant au moins un cycle aromatique, l'hétéroaryle est un groupe monocyclique ou bicyclique ayant 5 à 10 atomes de cycle et contenant 1 à 3 hétéroatomes sélectionnés parmi N, O, ou S en tant qu'atomes de cycle ;
$R_3$ est hydrogène.

**12.** Composé, sels pharmaceutiquement acceptables, diastéréomères, énantiomères, ou solvates de celui-ci selon la revendication 1, dans lequel le composé est sélectionné parmi les structures suivantes :

| | | | |
|---|---|---|---|
| 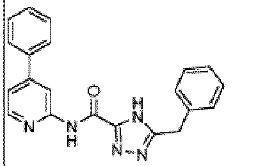 | 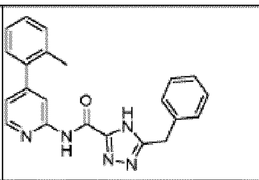 | 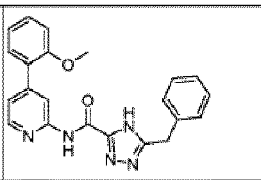 | 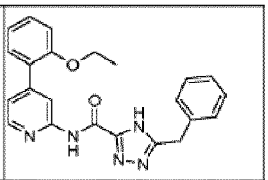 |
| 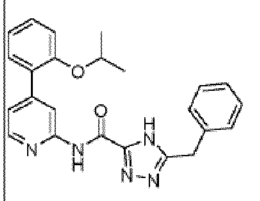 | 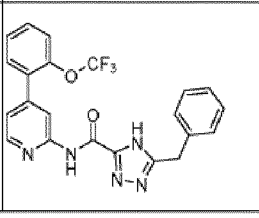 | 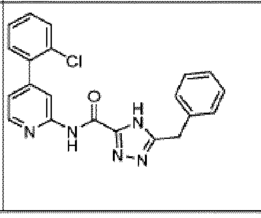 | 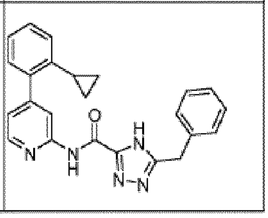 |

| | | | |
|---|---|---|---|
| 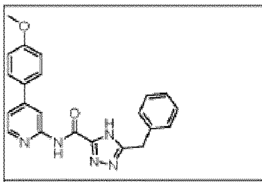 | 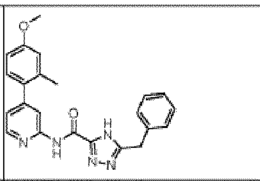 | 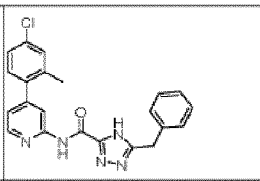 | 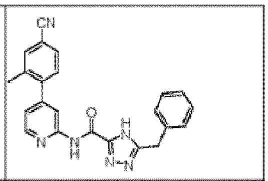 |
| 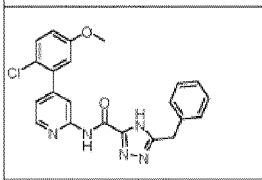 | 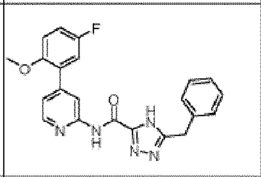 | 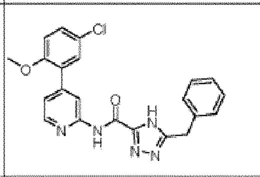 | 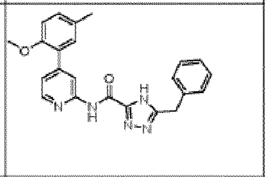 |
| 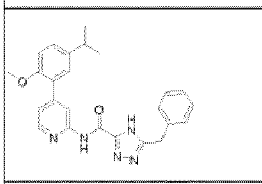 | 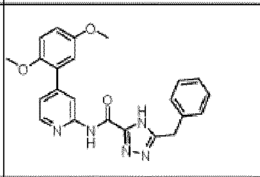 | 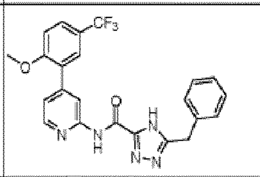 | 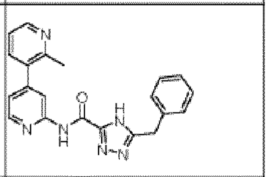 |
| 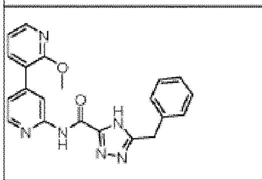 | 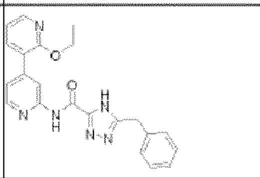 | 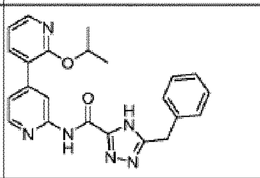 | 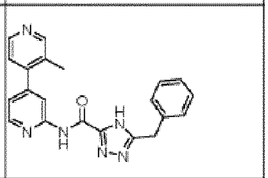 |
| 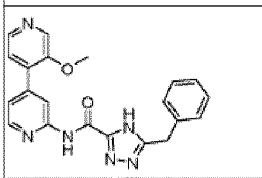 | 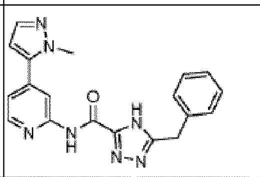 | 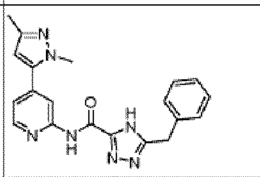 | 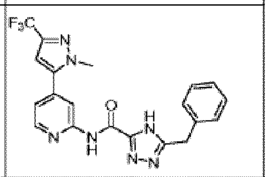 |
| 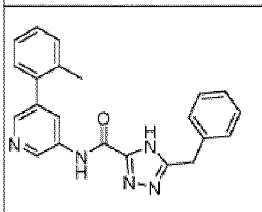 | 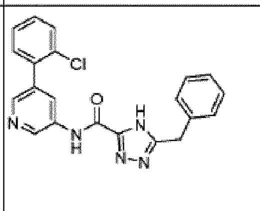 | 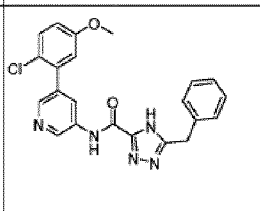 | 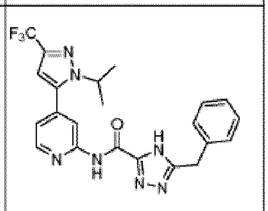 |
| 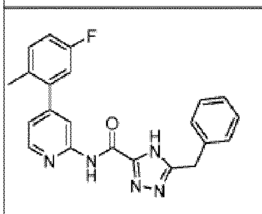 | 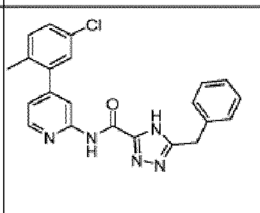 | 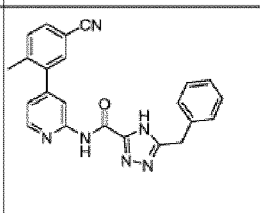 | 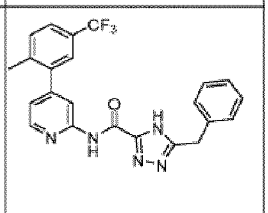 |
| 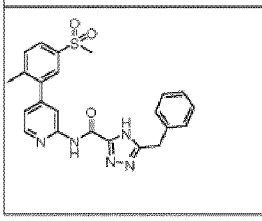 | 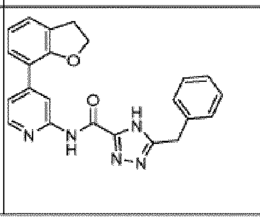 | 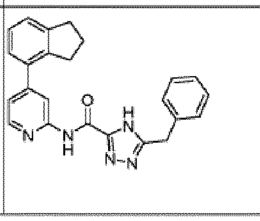 | 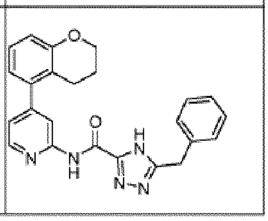 |

| | | | |
|---|---|---|---|
| 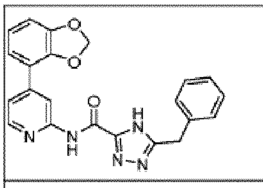 | 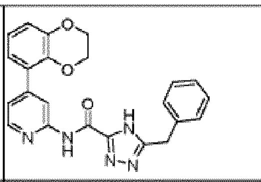 | 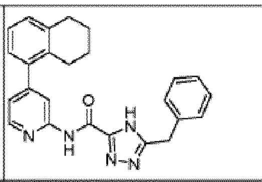 | 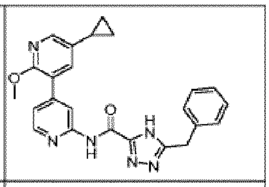 |
| 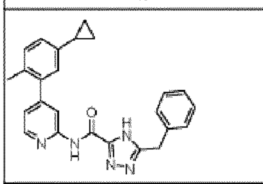 | 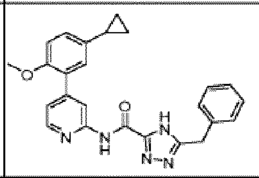 | 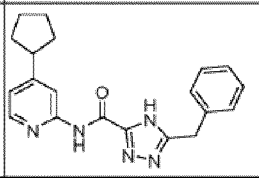 | 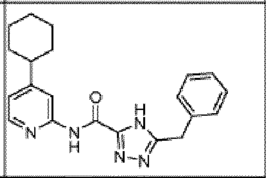 |
| 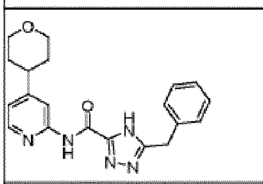 | 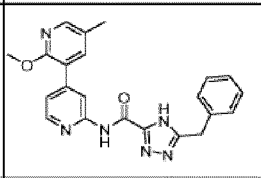 | 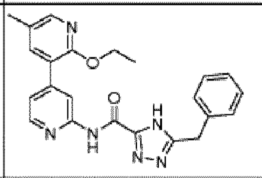 | 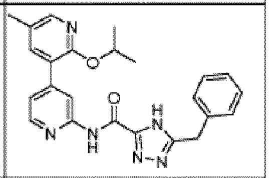 |
| 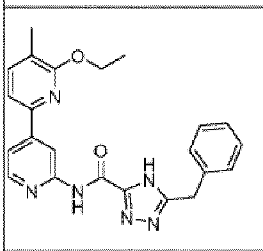 | 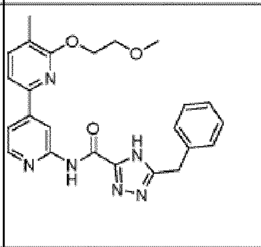 | 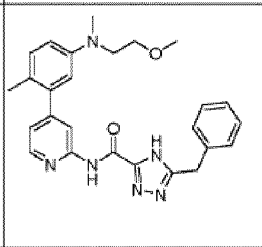 | 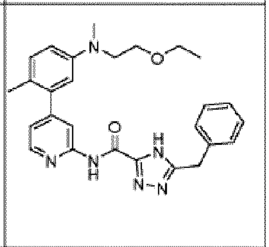 |
| 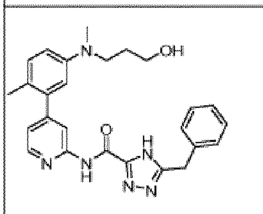 | 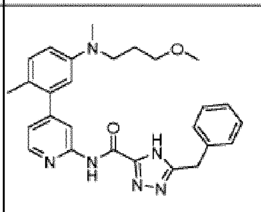 | 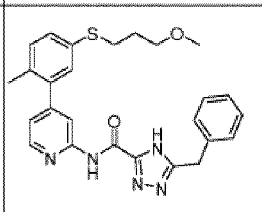 | 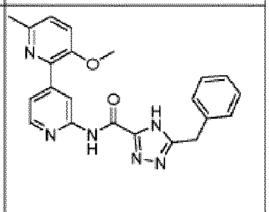 |
| 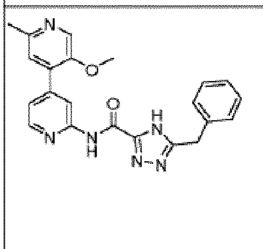 | 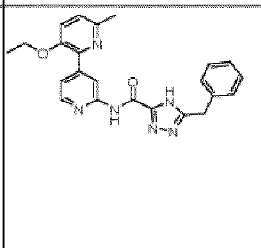 | 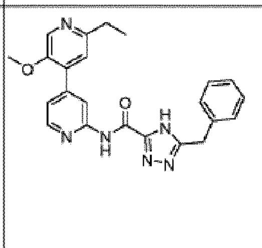 | 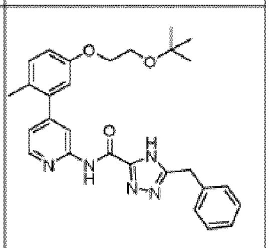 |
| 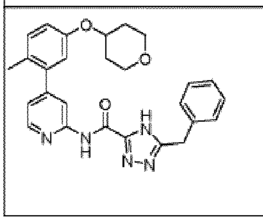 | 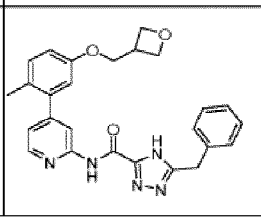 | 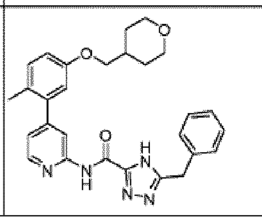 | 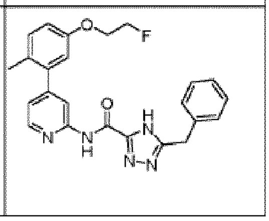 |

| | | | |
|---|---|---|---|
| 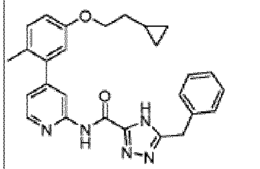 | 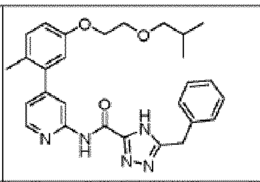 | 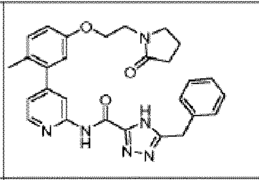 | 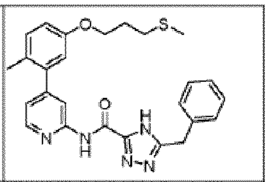 |
| 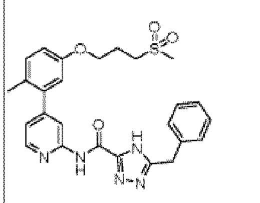 | 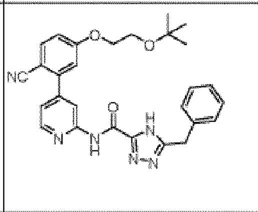 | 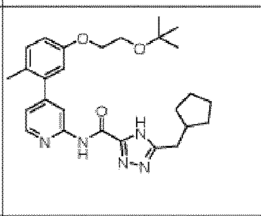 | 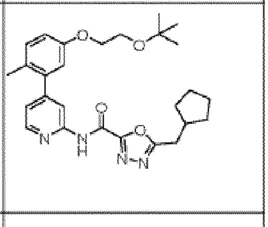 |
| 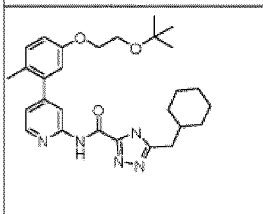 | 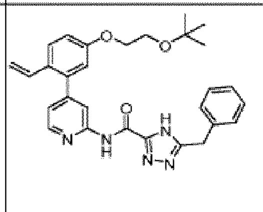 | 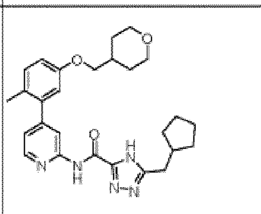 | 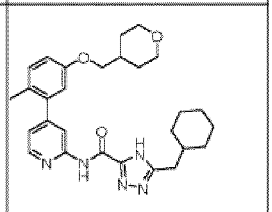 |
| 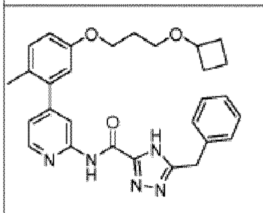 | 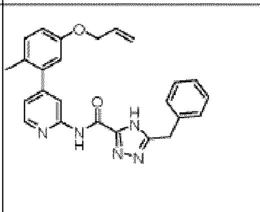 | 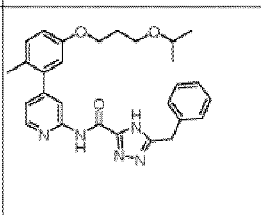 | 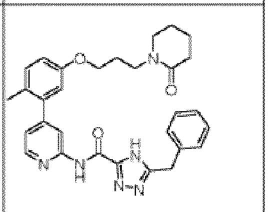 |
| 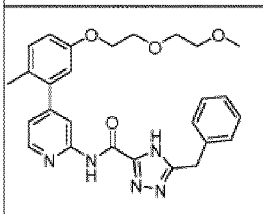 | 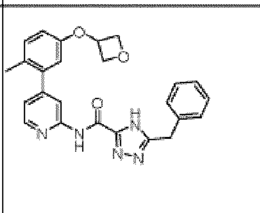 | 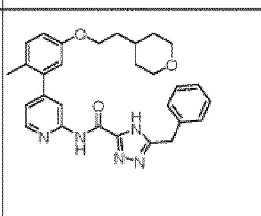 | 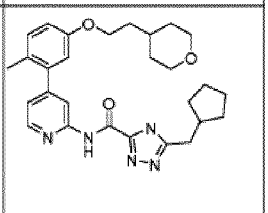 |
| 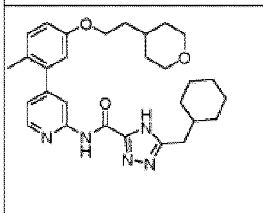 | 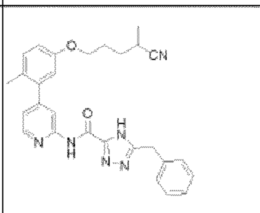 | 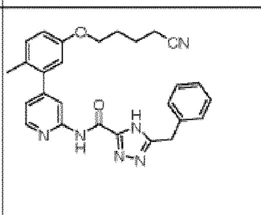 | 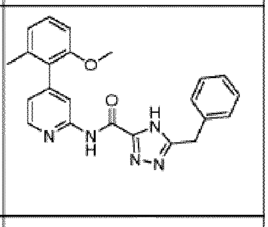 |
| 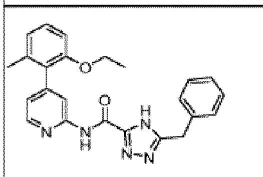 | 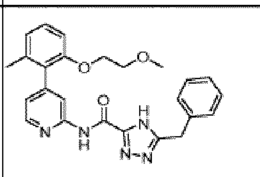 | 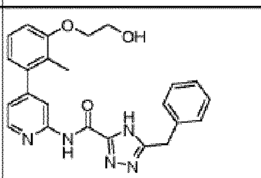 | 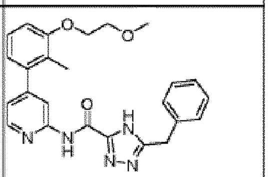 |
| 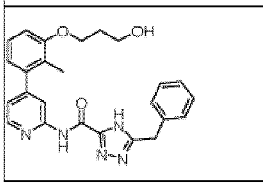 | 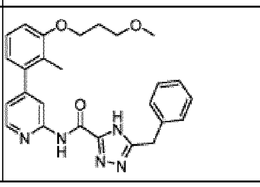 | 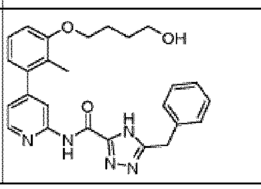 | 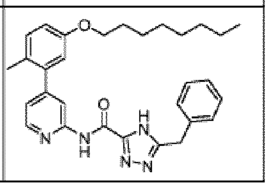 |

| | | | |
|---|---|---|---|
| 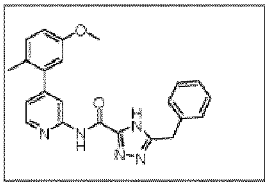 | 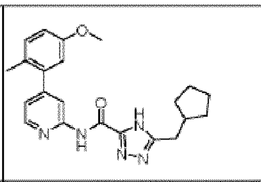 | 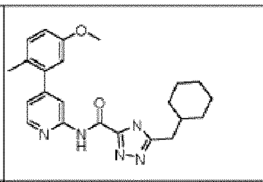 | 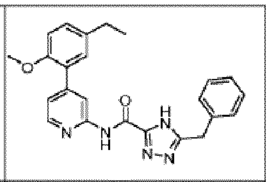 |
| 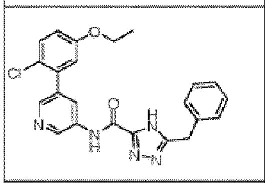 | 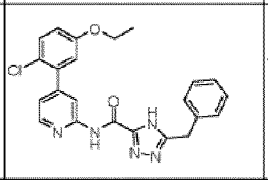 | 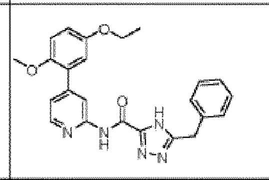 | 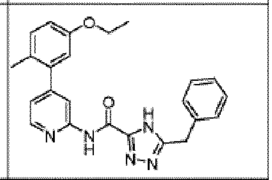 |
| 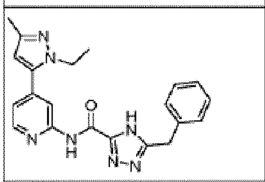 | 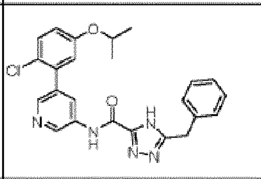 | 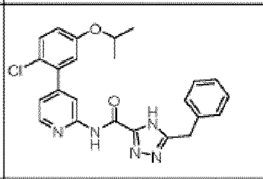 | 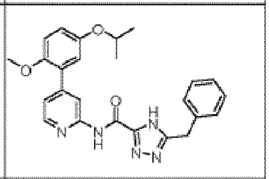 |
| 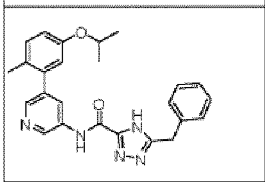 | 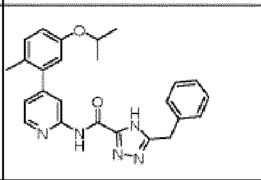 | 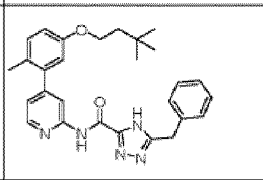 | 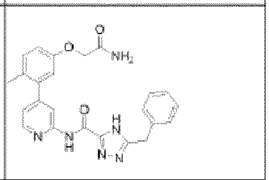 |
| 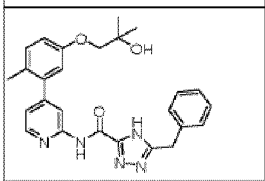 | 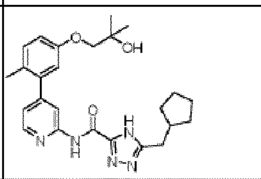 | 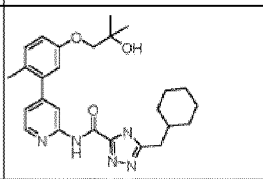 | 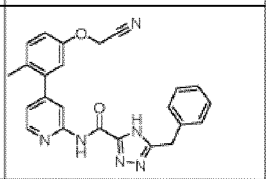 |
| 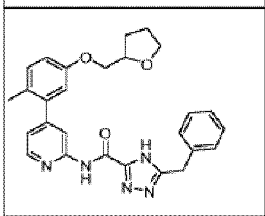 | 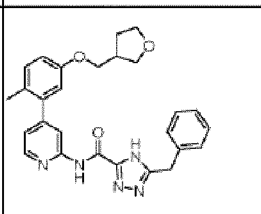 | 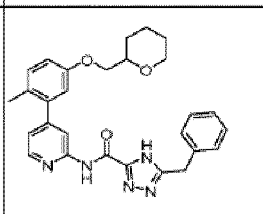 | 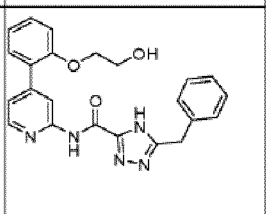 |
| 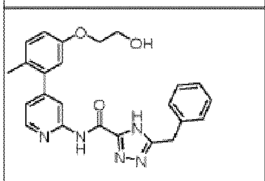 | 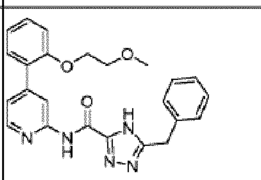 | 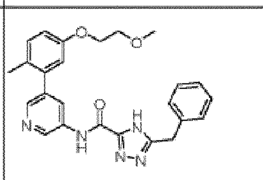 | 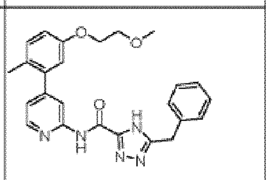 |
| 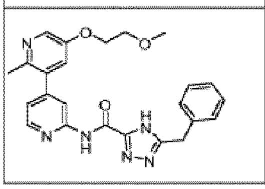 | 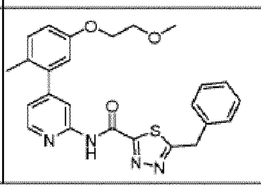 | 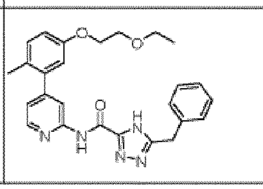 | 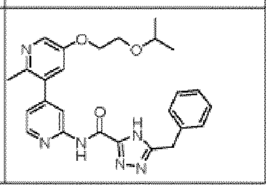 |

| | | | |
|---|---|---|---|
| 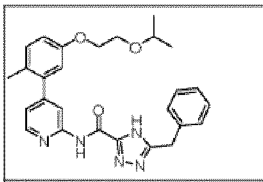 | 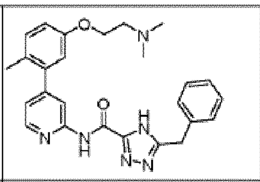 | 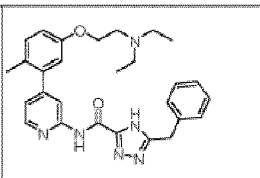 | 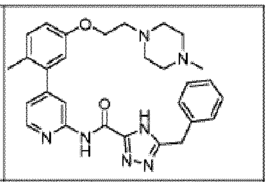 |
| 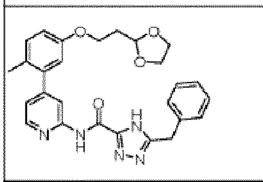 | 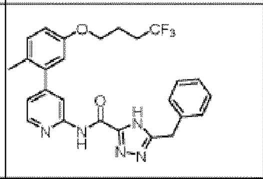 | 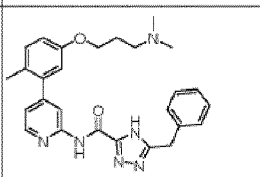 | 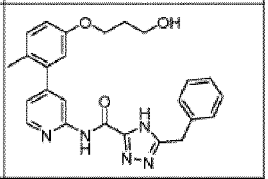 |
| 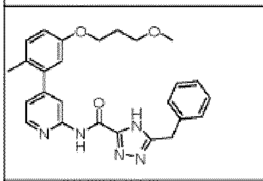 | 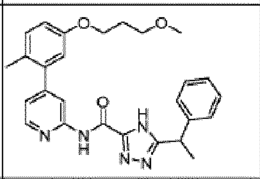 | 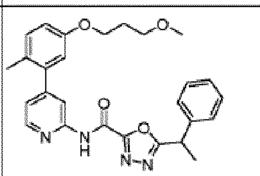 | 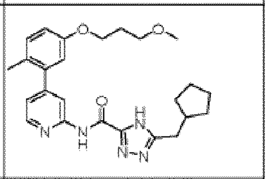 |
| 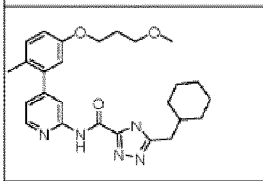 | 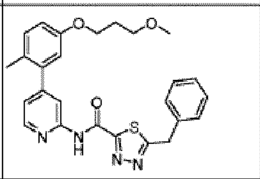 | 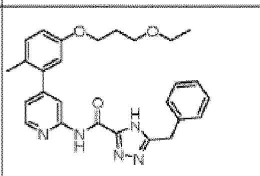 | 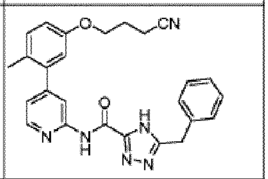 |
| 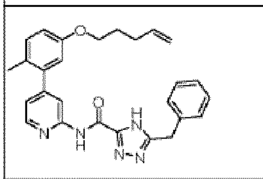 | 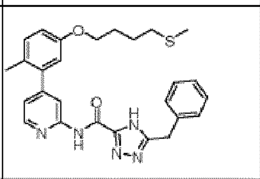 | 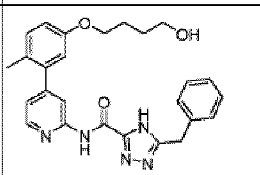 | 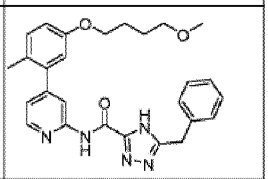 |
| 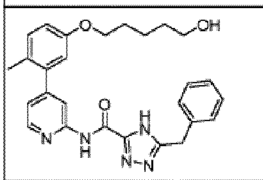 | 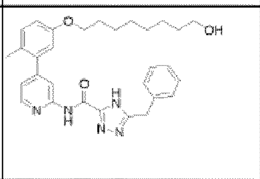 | 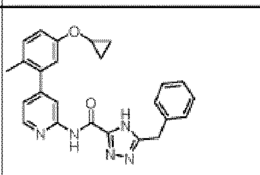 | 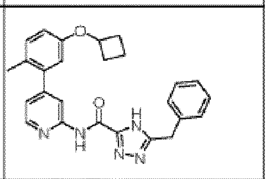 |
| 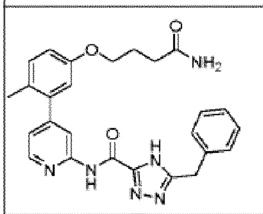 | 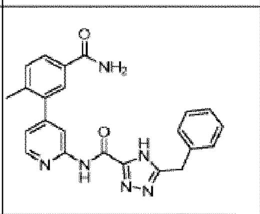 | 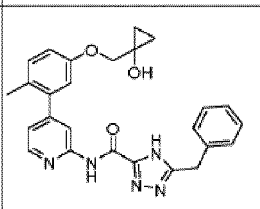 | 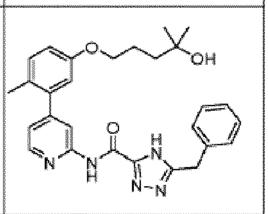 |
| 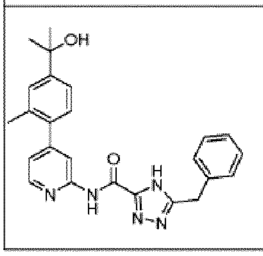 | 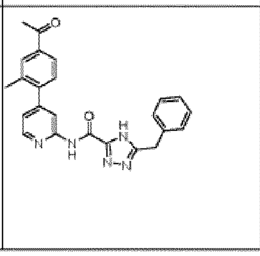 | 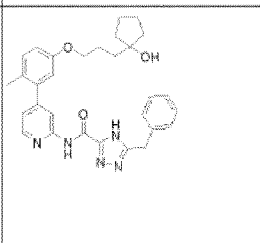 | 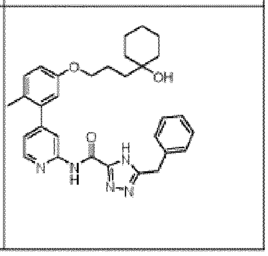 |

| | | | |
|---|---|---|---|
| 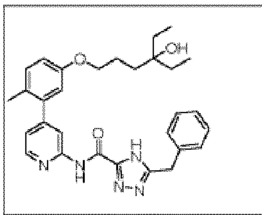 | 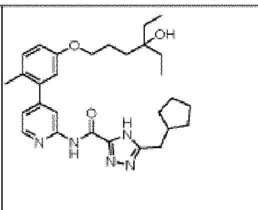 | 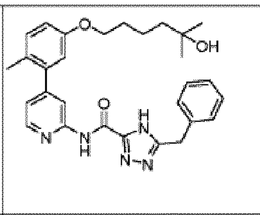 | 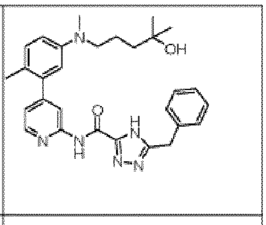 |
| 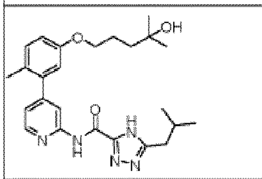 | 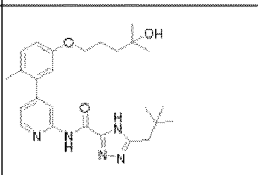 | 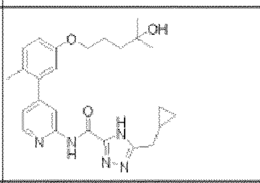 | 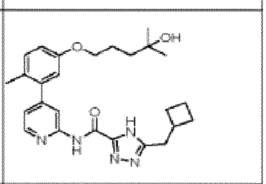 |
| 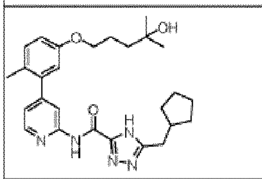 | 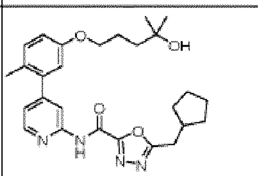 | 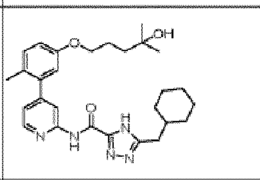 | 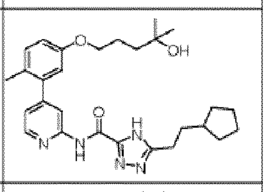 |
| 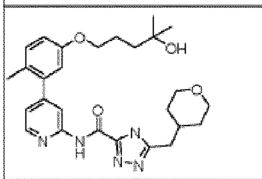 | 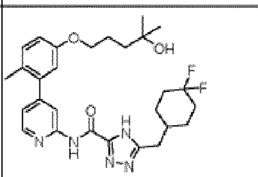 | 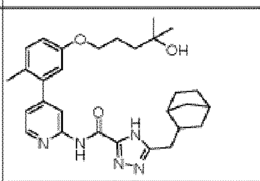 | 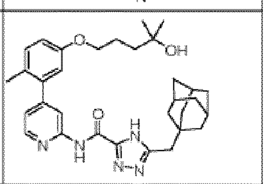 |
| 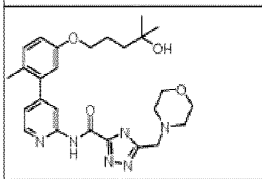 | 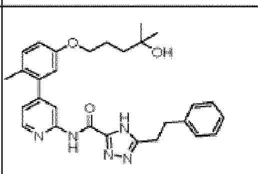 | 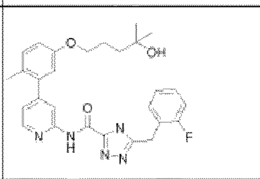 | 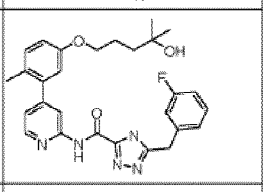 |
| 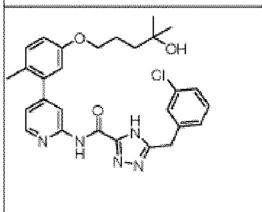 | 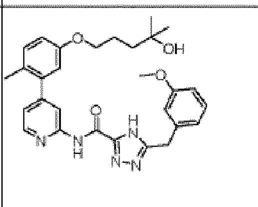 | 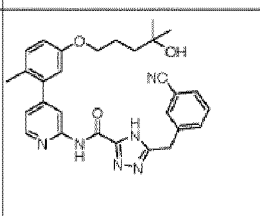 | 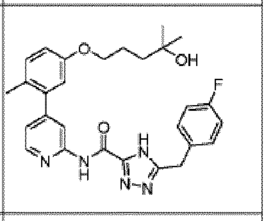 |
| 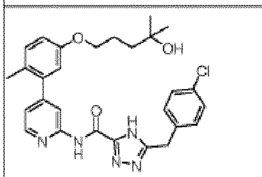 | 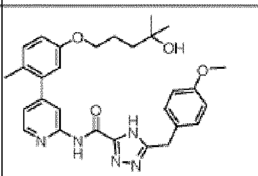 | 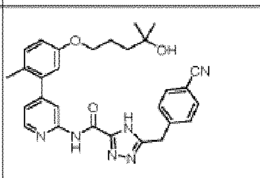 | 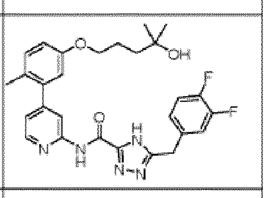 |
| 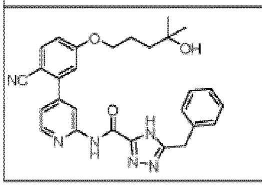 | 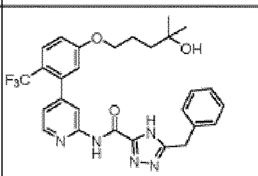 | 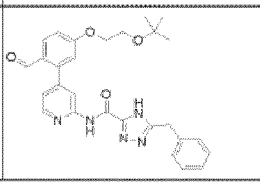 | 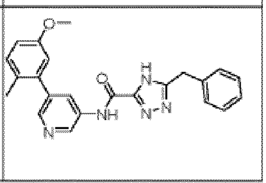 |

| | | | |
|---|---|---|---|
| 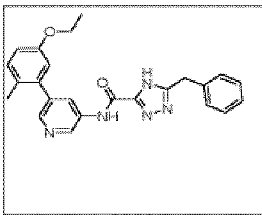 | 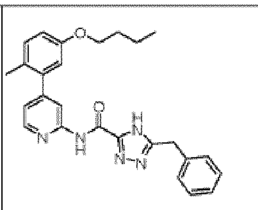 | 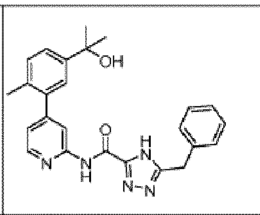 | 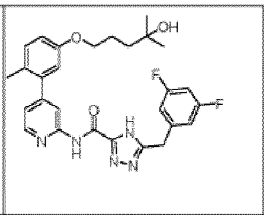 |
| 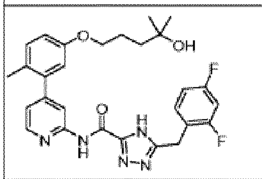 | 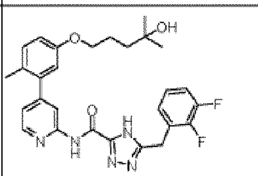 | 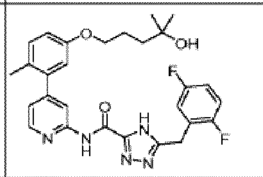 | 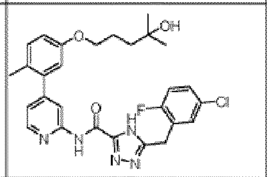 |
| 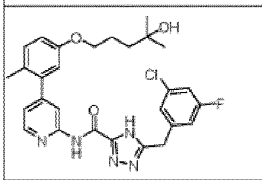 | 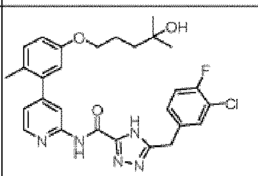 | 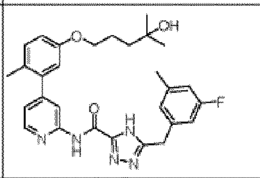 | 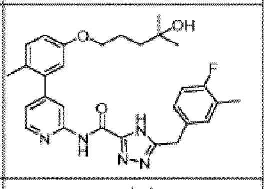 |
| 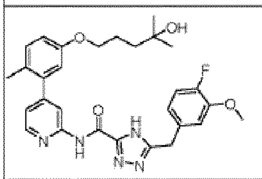 | 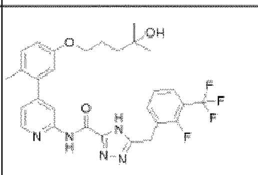 | 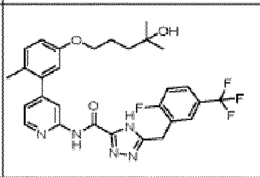 | 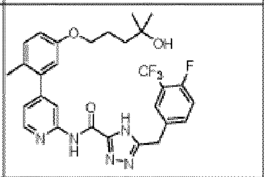 |
| 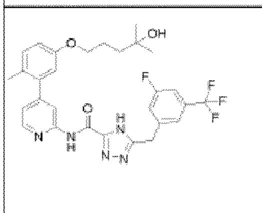 | 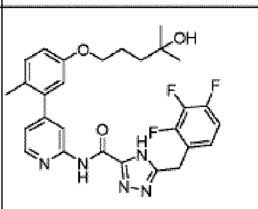 | 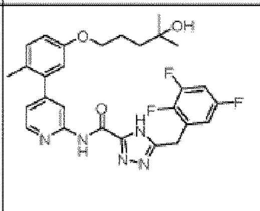 | 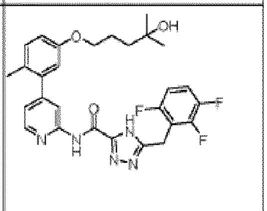 |
| 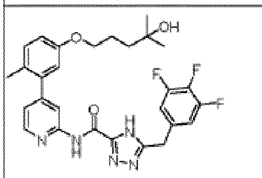 | 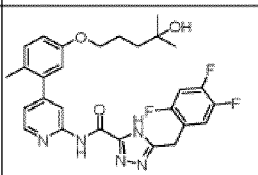 | 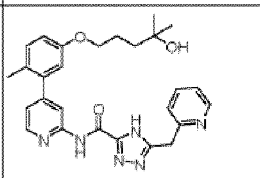 | 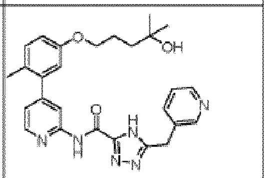 |
| 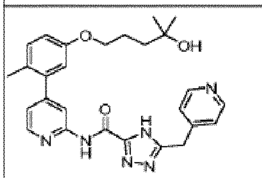 | 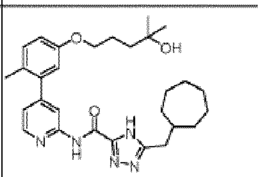 | 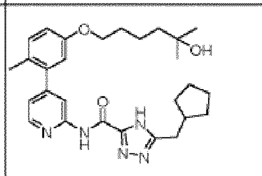 | 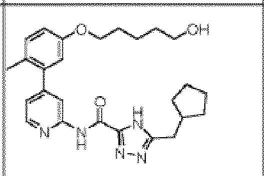 |
| 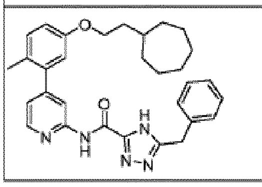 | 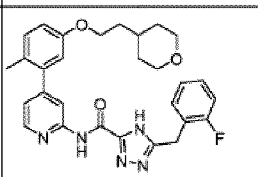 | 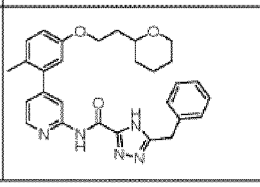 | 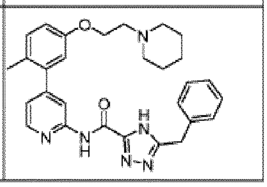 |

| | | | |
|---|---|---|---|
| 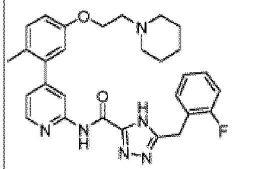 | 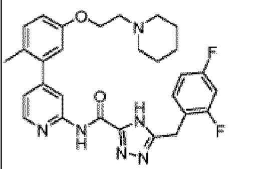 | 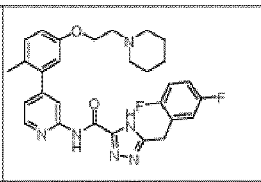 | 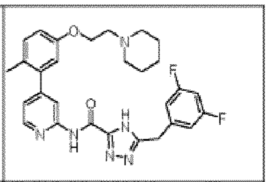 |
| 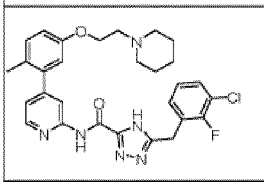 | 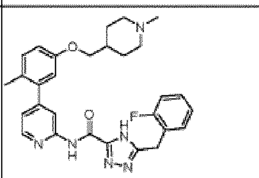 | 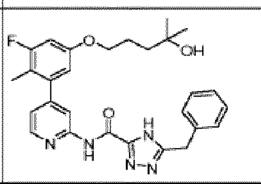 | 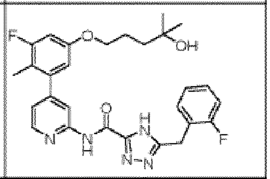 |
| 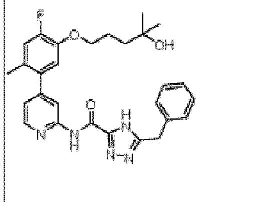 | 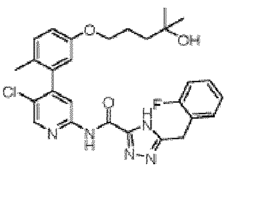 | 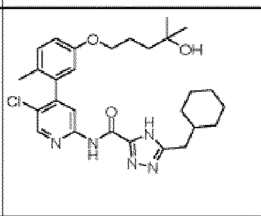 | 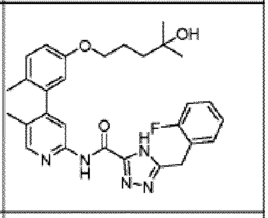 |
| 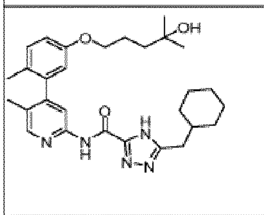 | 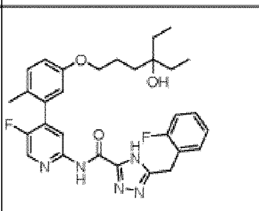 | 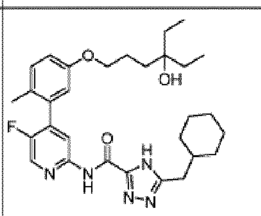 | 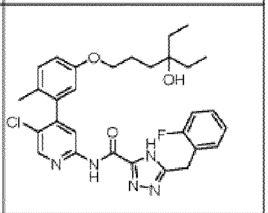 |
| 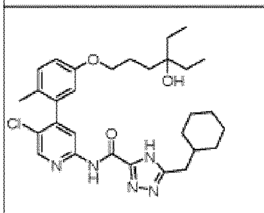 | 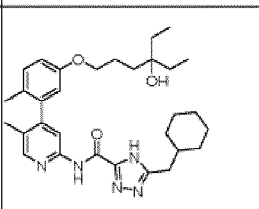 | 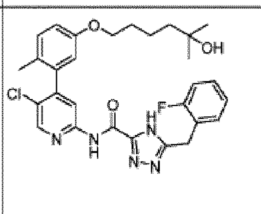 | 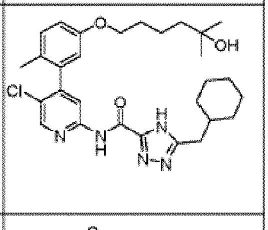 |
| 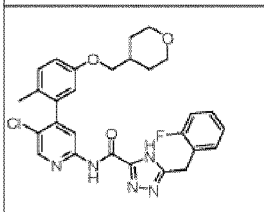 | 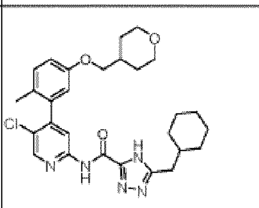 | 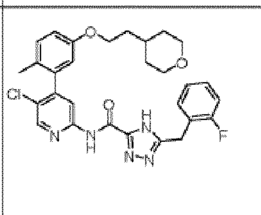 | 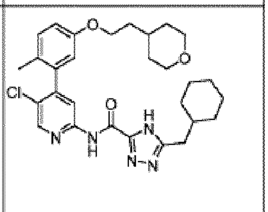 |
| 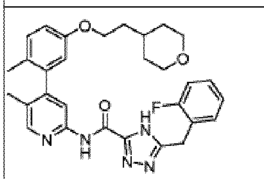 | 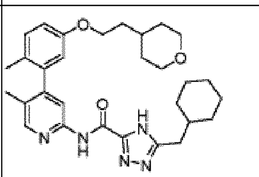 | 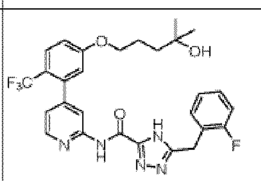 | 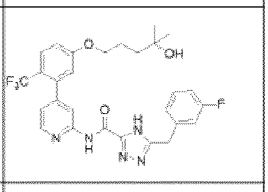 |
| 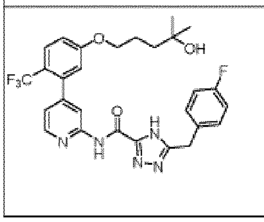 | 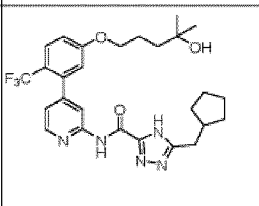 | 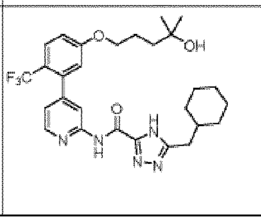 | 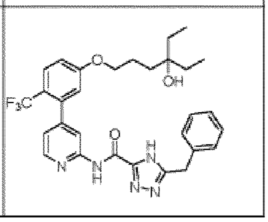 |

| | | | |
|---|---|---|---|
| 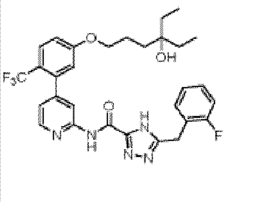 | 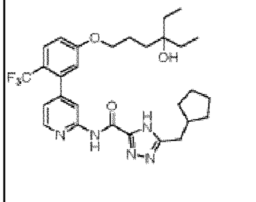 | 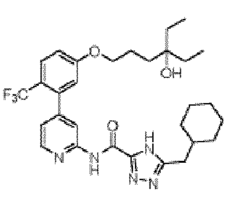 | 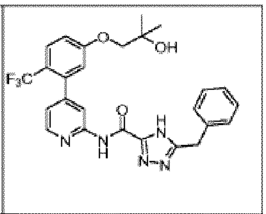 |
| 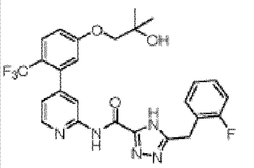 | 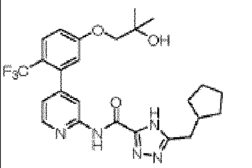 | 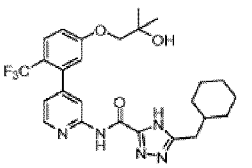 | 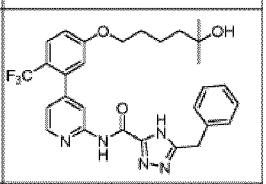 |
| 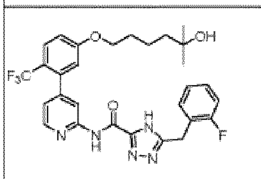 | 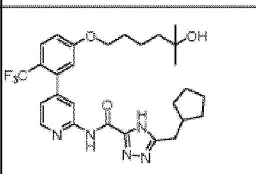 | 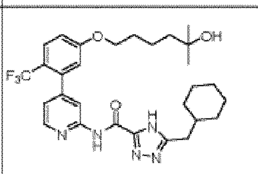 | 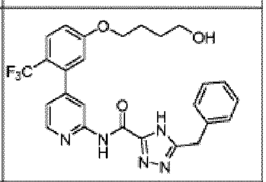 |
| 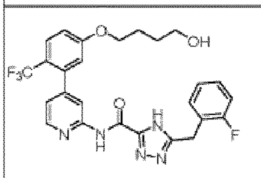 | 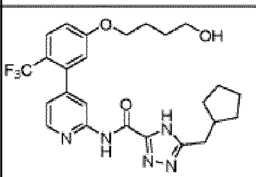 | 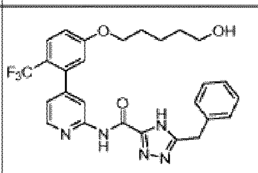 | 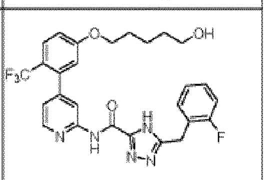 |
| 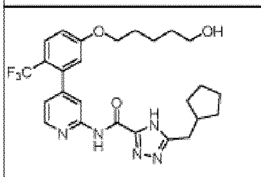 | 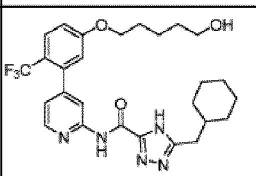 | 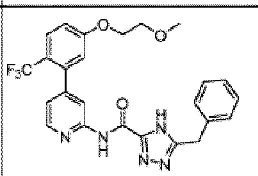 | 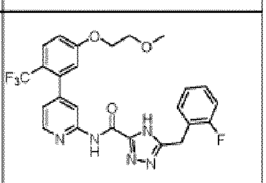 |
| 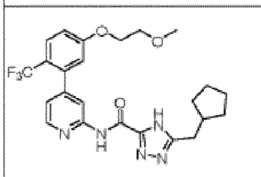 | 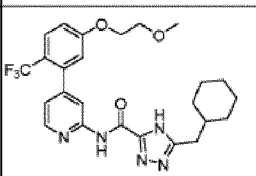 | 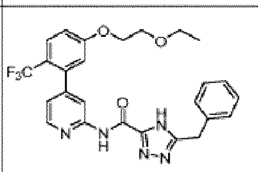 | 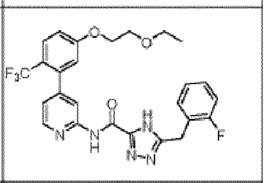 |
| 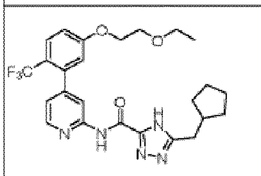 | 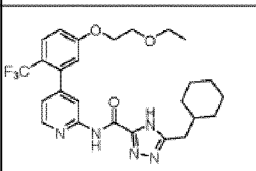 | 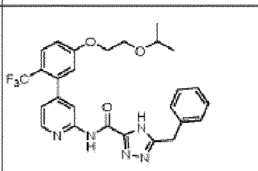 | 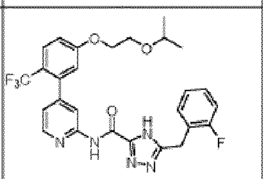 |
| 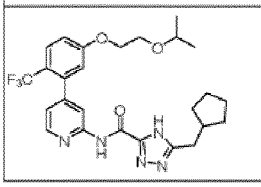 | 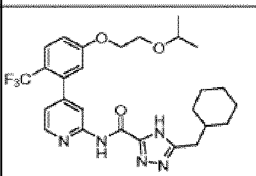 | 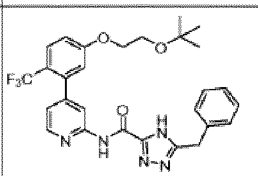 | 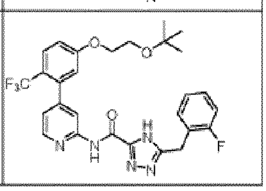 |

| | | | |
|---|---|---|---|
| 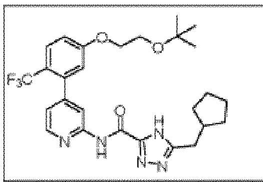 | 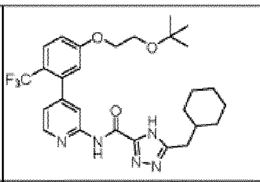 | 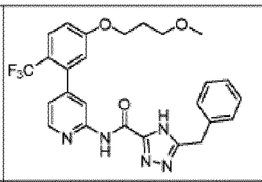 | 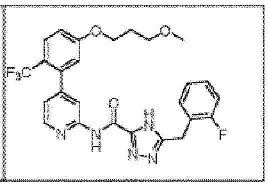 |
| 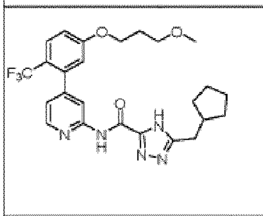 | 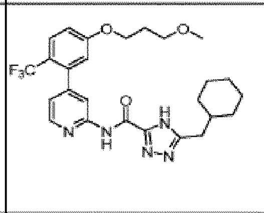 | 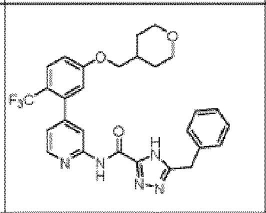 | 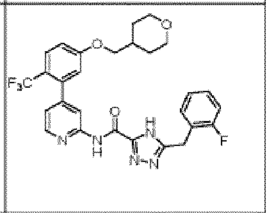 |
| 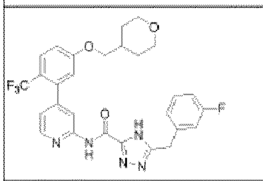 | 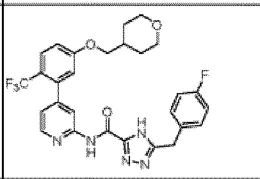 | 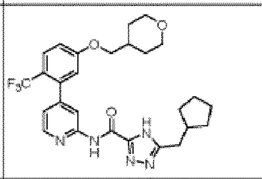 | 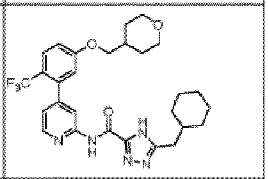 |
| 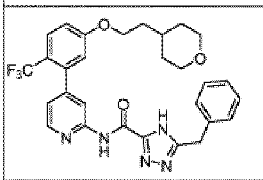 | 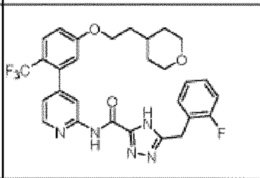 | 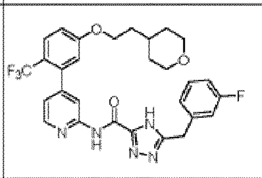 | 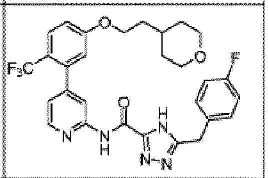 |
| 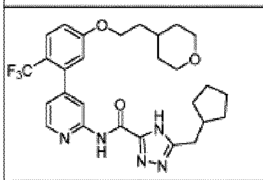 | 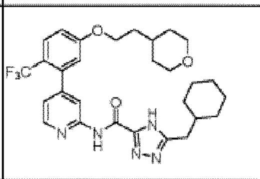 | 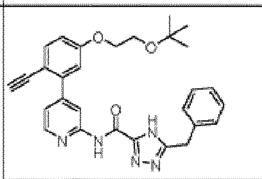 | 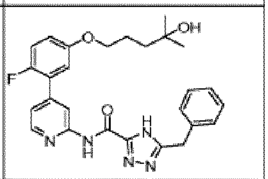 |
| 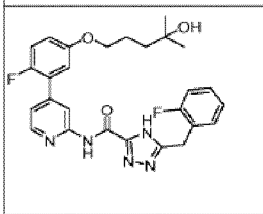 | 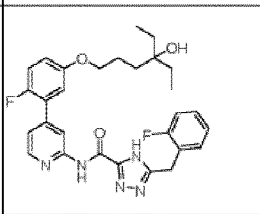 | 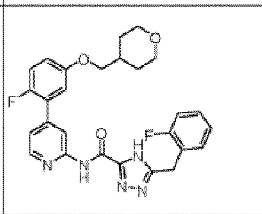 | 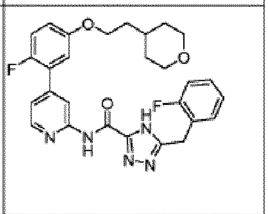 |
| 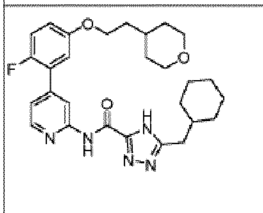 | 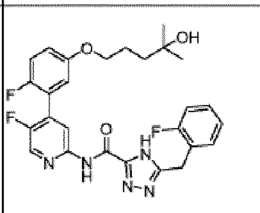 | 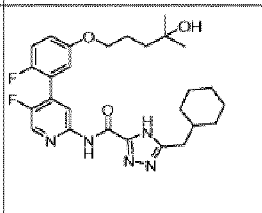 | 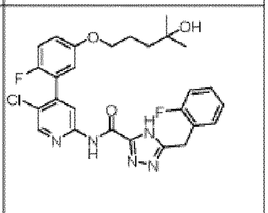 |
| 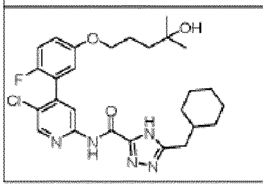 | 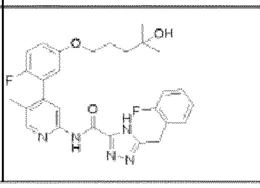 | 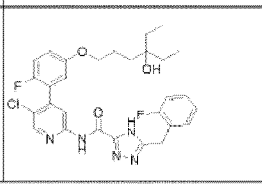 | 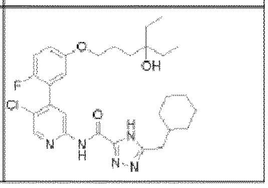 |

| | | | |
|---|---|---|---|
| 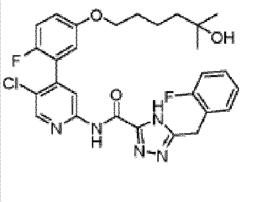 | 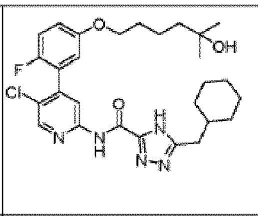 | 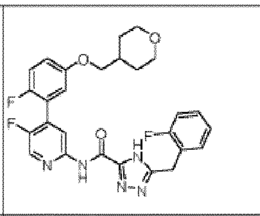 | 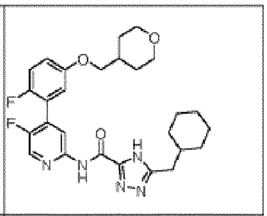 |
| 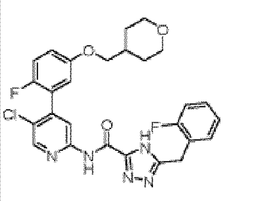 | 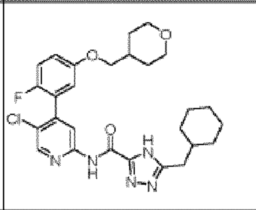 | 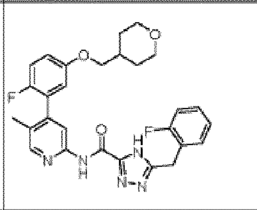 | 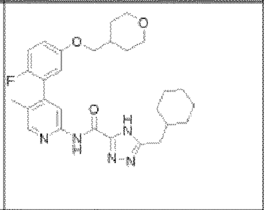 |
| 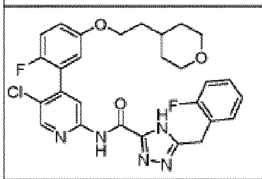 | 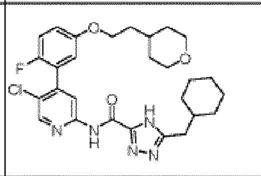 | 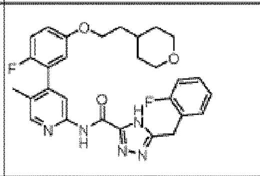 | 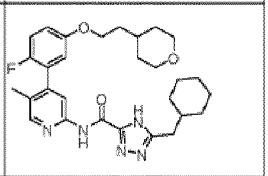 |
| 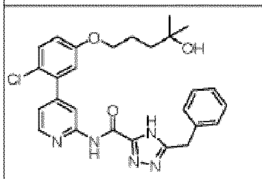 | 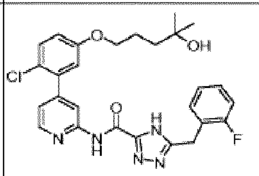 | 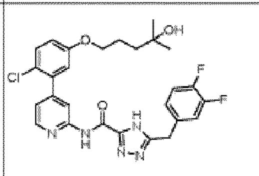 | 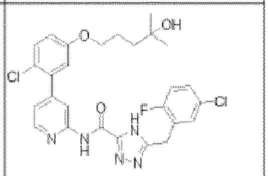 |
| 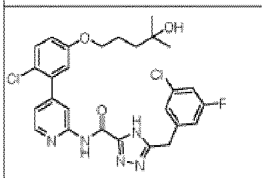 | 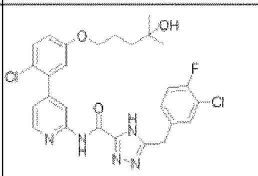 | 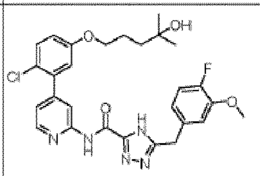 | 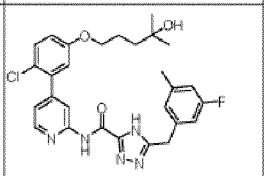 |
| 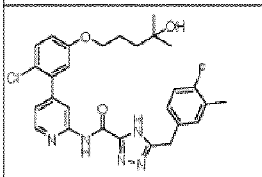 | 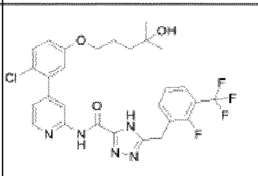 | 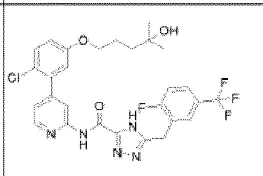 | 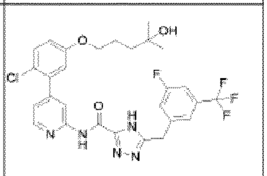 |
| 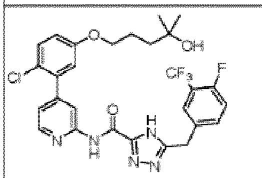 | 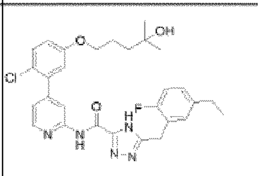 | 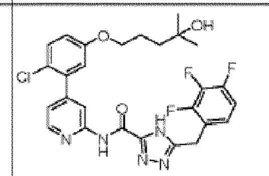 | 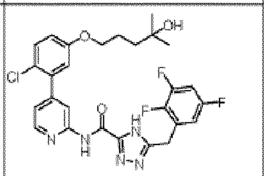 |
| 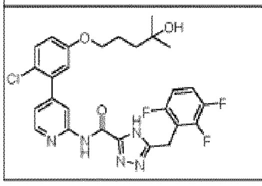 | 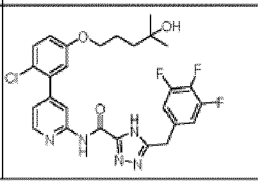 | 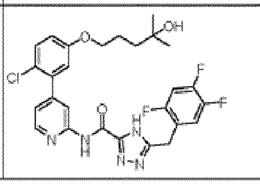 | 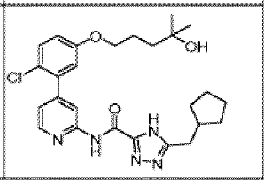 |

| | | | |
|---|---|---|---|
| 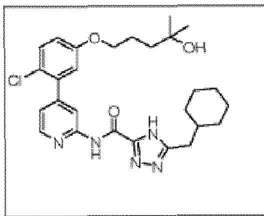 | 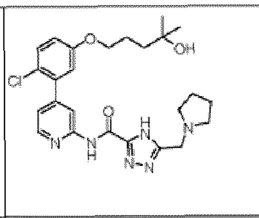 | 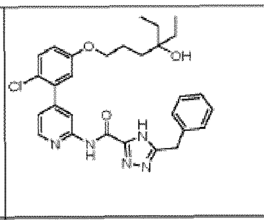 | 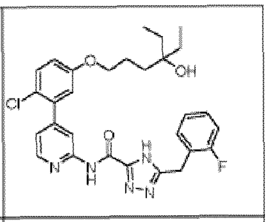 |
| 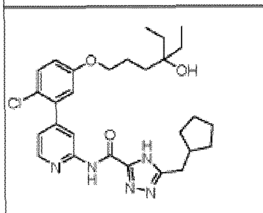 | 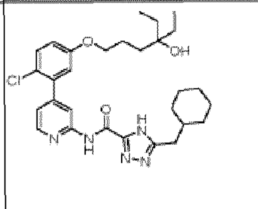 | 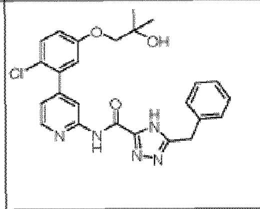 | 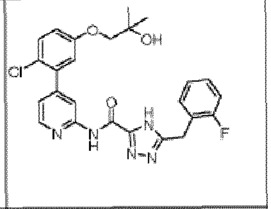 |
| 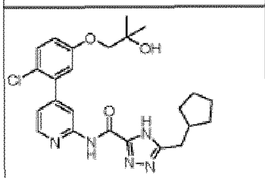 | 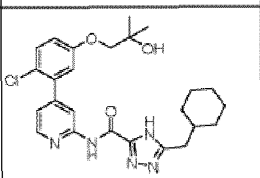 | 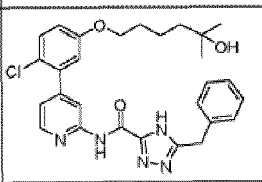 | 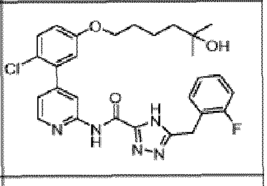 |
| 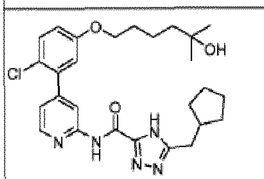 | 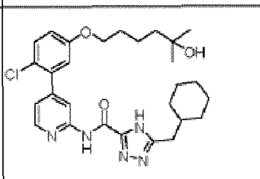 | 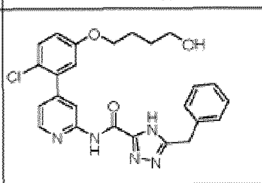 | 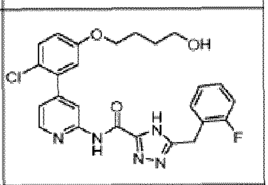 |
| 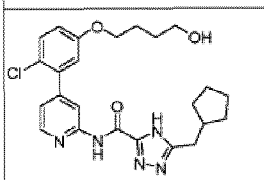 | 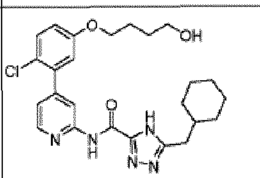 | 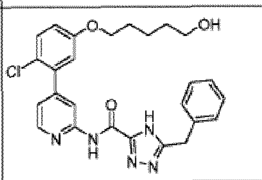 | 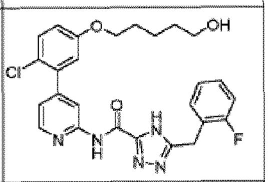 |
| 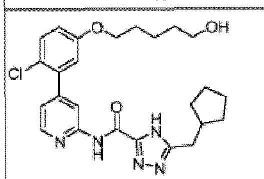 | 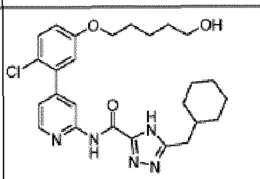 | 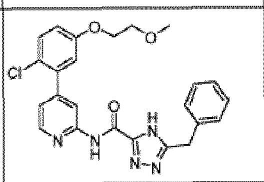 | 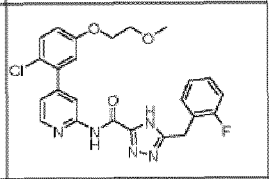 |
| 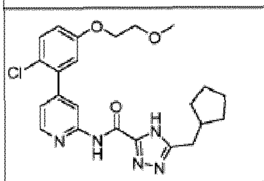 | 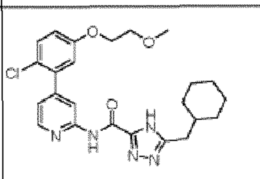 | 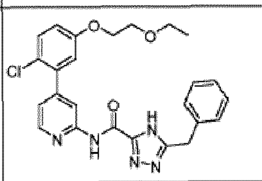 | 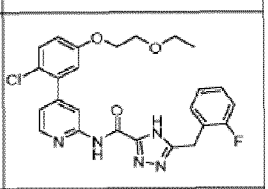 |
| 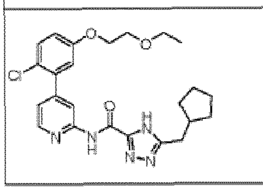 | 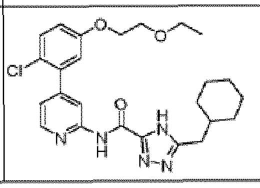 | 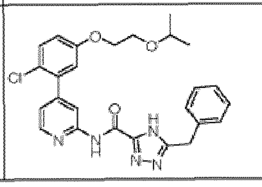 | 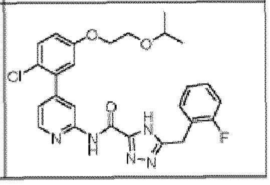 |

| | | | |
|---|---|---|---|
| 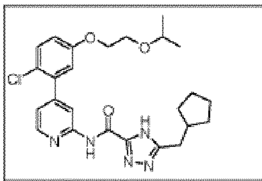 | 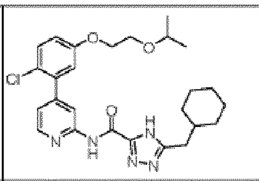 | 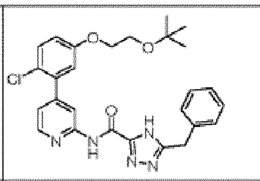 | 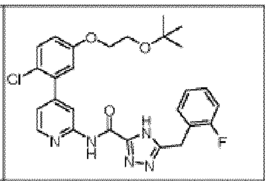 |
| 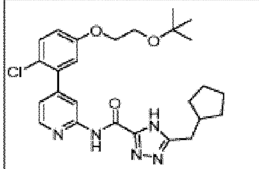 | 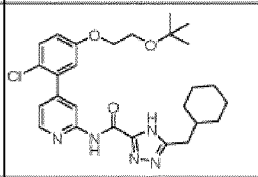 | 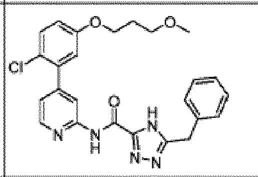 | 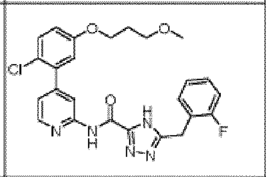 |
| 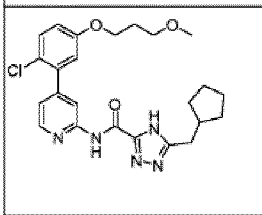 | 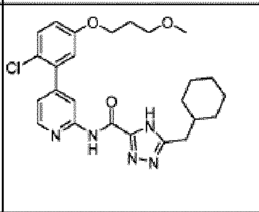 | 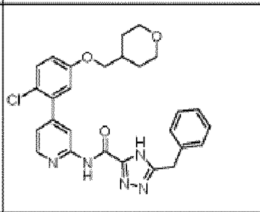 | 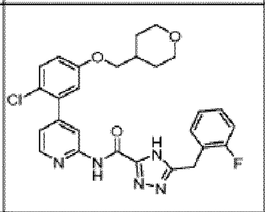 |
| 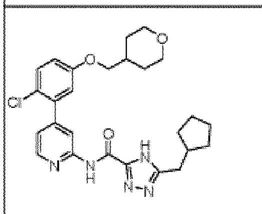 | 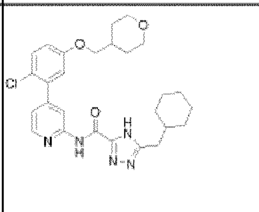 | 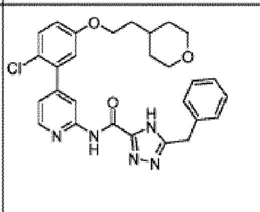 | 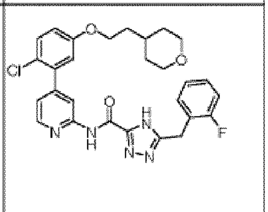 |
| 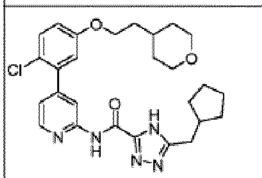 | 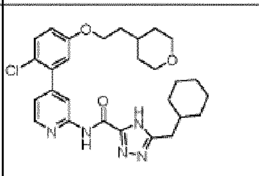 | 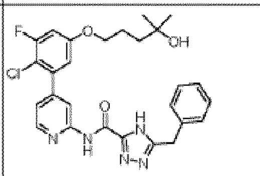 | 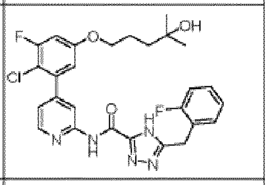 |
| 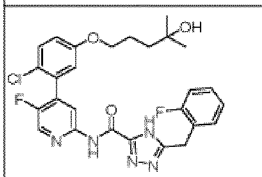 | 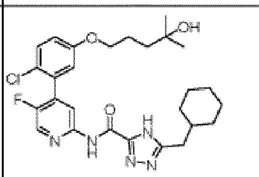 | 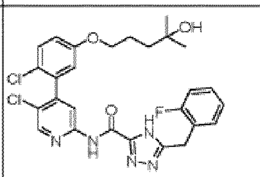 | 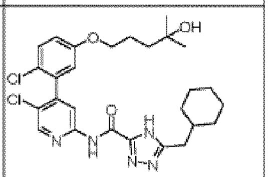 |
| 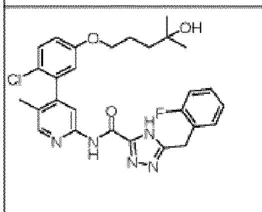 | 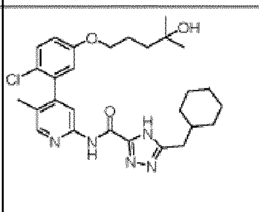 | 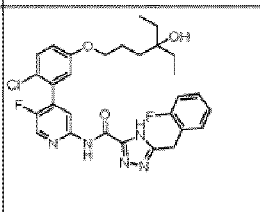 | 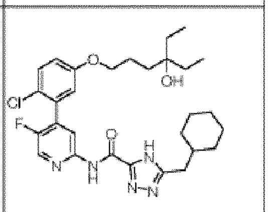 |
| 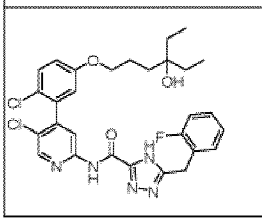 | 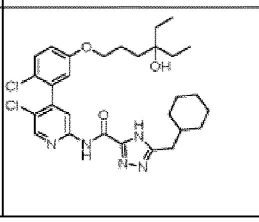 | 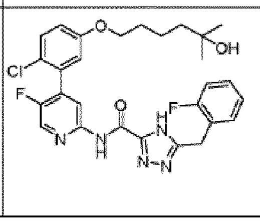 | 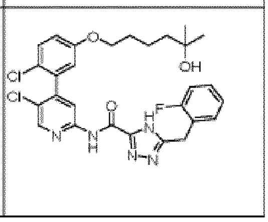 |

| | | | |
|---|---|---|---|
| 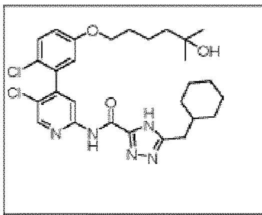 | 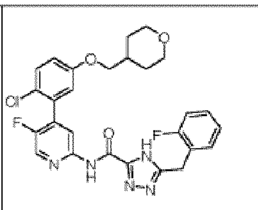 | 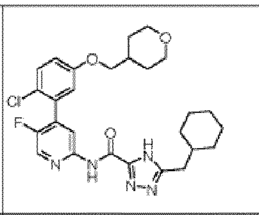 | 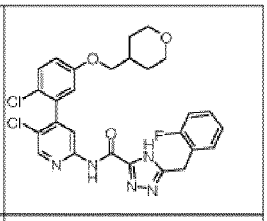 |
| 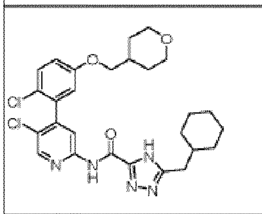 | 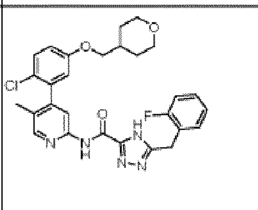 | 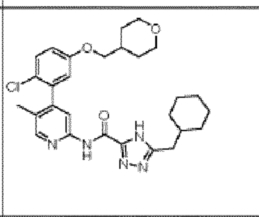 | 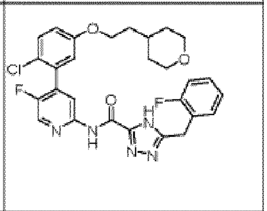 |
| 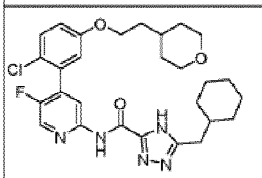 | 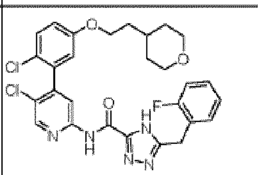 | 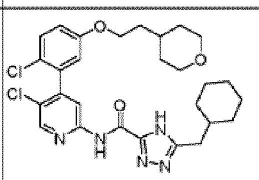 | 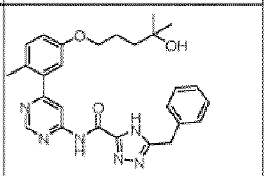 |
| 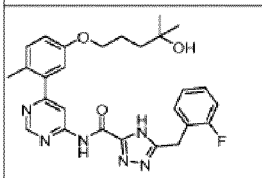 | 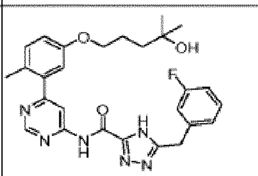 | 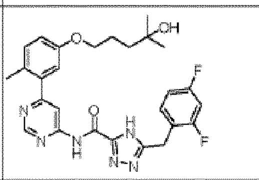 | 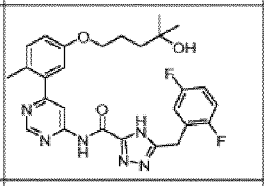 |
| 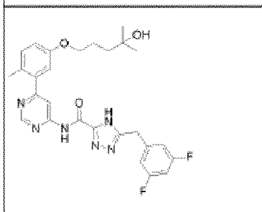 | 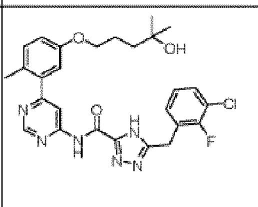 | 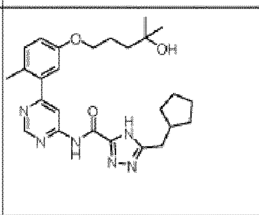 | 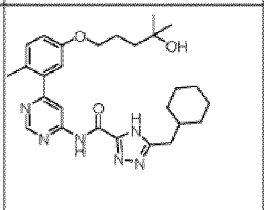 |
| 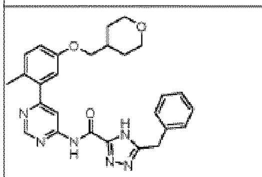 | 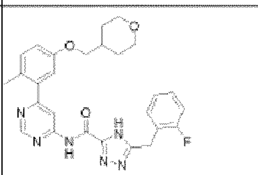 | 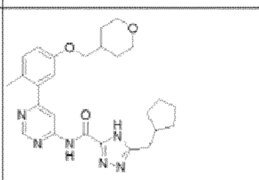 | 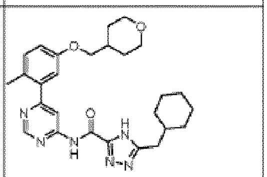 |
| 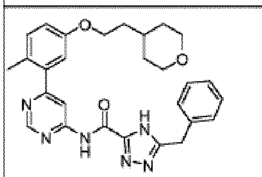 | 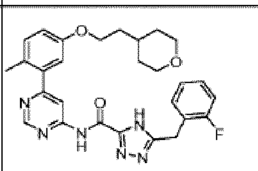 | 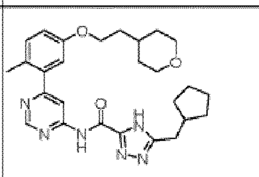 | 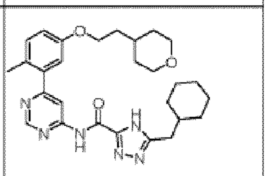 |
| 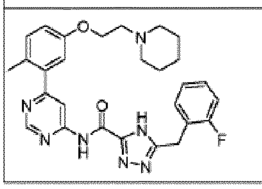 | 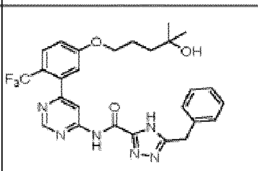 | 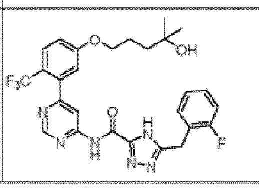 | 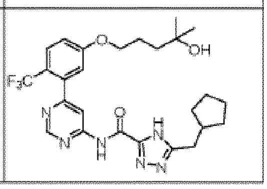 |

| | | | |
|---|---|---|---|
| 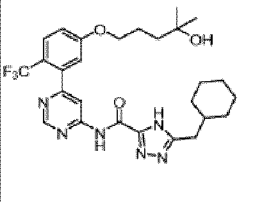 | 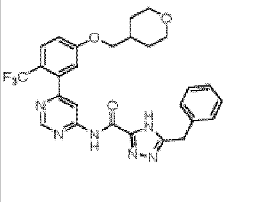 | 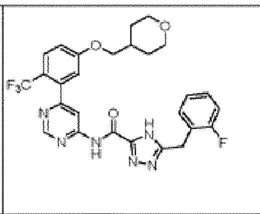 | 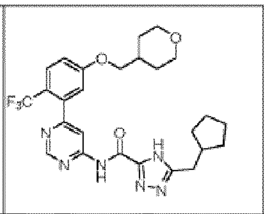 |
| 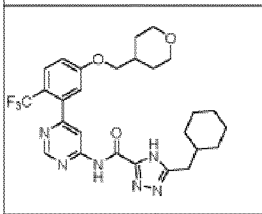 | 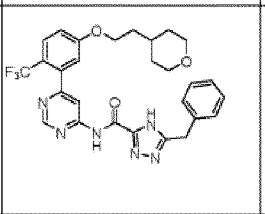 | 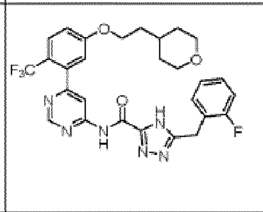 | 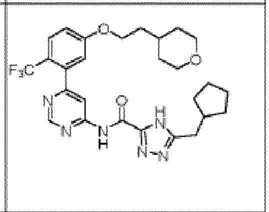 |
| 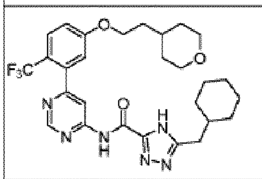 | 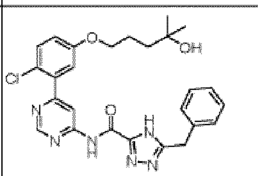 | 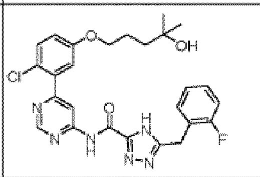 | 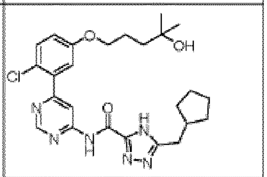 |
| 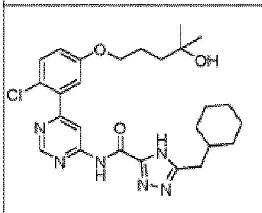 | 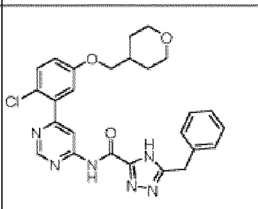 | 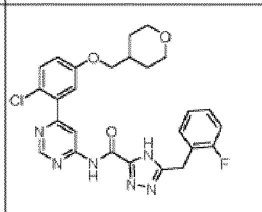 | 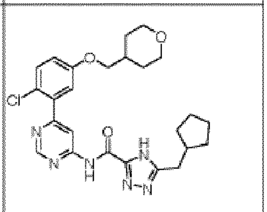 |
| 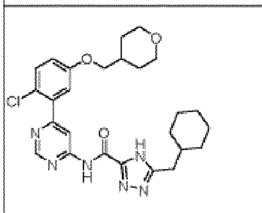 | 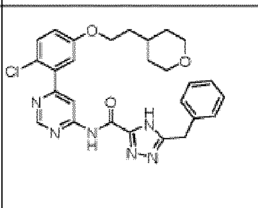 | 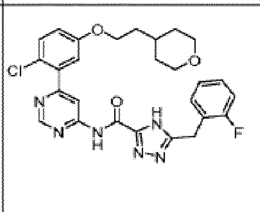 | 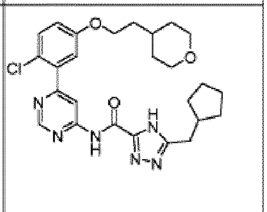 |
| 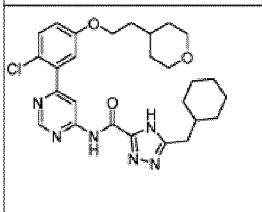 | 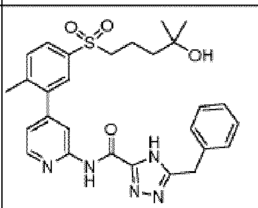 | 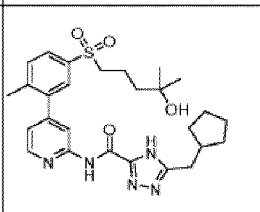 | 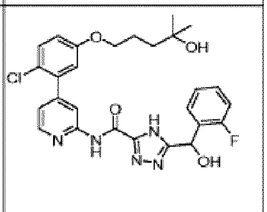 |
| 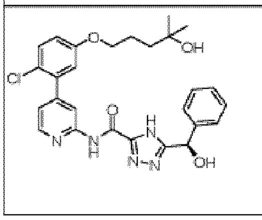 | 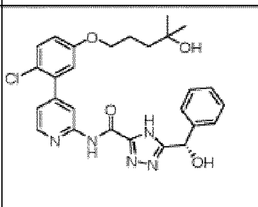 | 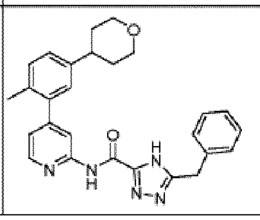 | 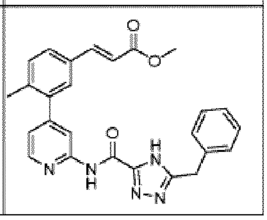 |

| | | | |
|---|---|---|---|
| 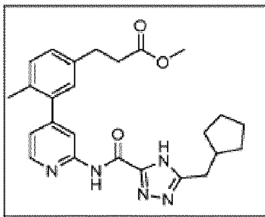 | 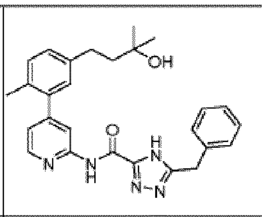 | 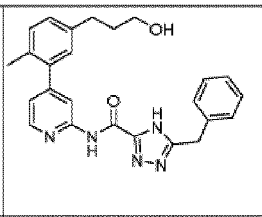 | 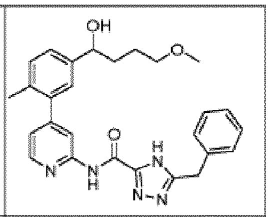 |
| 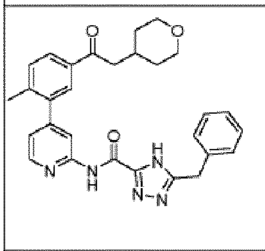 | 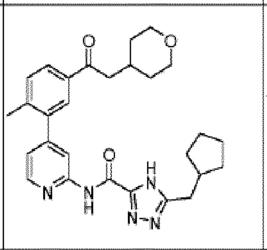 | 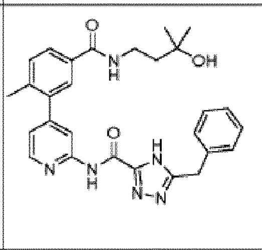 | 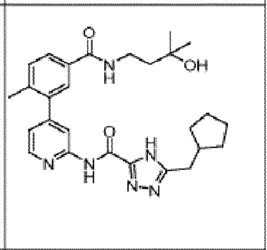 |
| 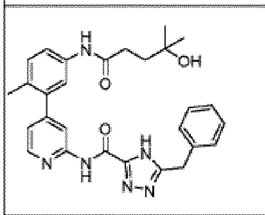 | 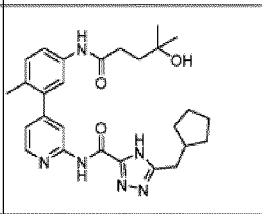 | 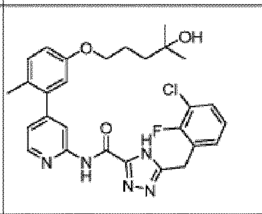 | 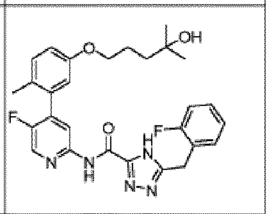 |
| 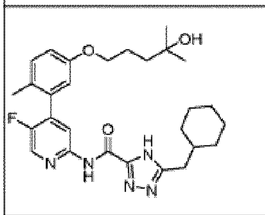 | 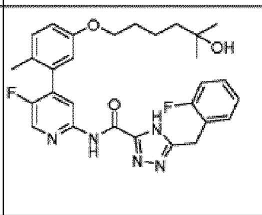 | 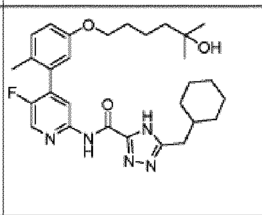 | 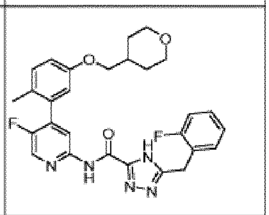 |
| 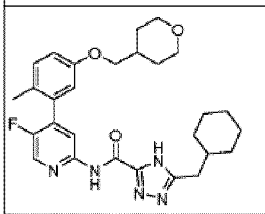 | 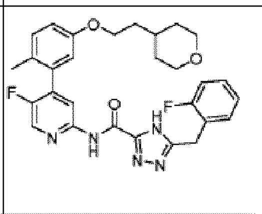 | 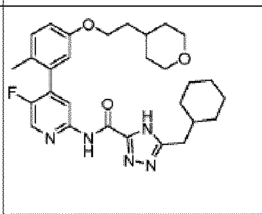 | 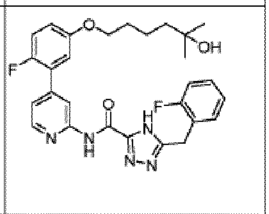 |
| 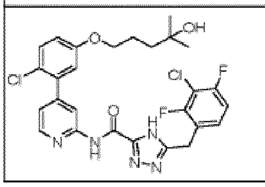 | 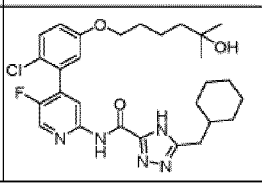 | 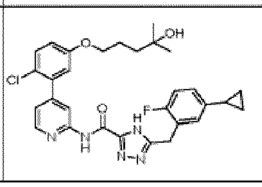 | 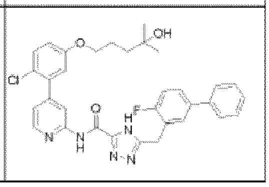 |

**13.** Composé, sels pharmaceutiquement acceptables, diastéréomères, énantiomères, ou solvates de celui-ci selon l'une des revendications 1 à 12, pour une utilisation dans le traitement des maladies associées à RIP1, dans lequel la maladie associée à RIP1 comprend une maladie de fond d'oeil, la xérophtalmie, le psoriasis, la leucodermie, la dermatite, la pelade, la polyarthrite rhumatoïde, la colite, la sclérose en plaques, le lupus érythémateux disséminé, la maladie de Crohn, l'athérosclérose, la fibrose pulmonaire, la fibrose hépatique, la myélofibrose, le cancer du poumon non à petites cellules, le cancer du poumon à petites cellules, le cancer du sein, le cancer du pancréas, le gliome, le glioblastome, le cancer des ovaires, le cancer du col de l'utérus, le cancer colorectal, le mélanome, cancer de l'endomètre, le cancer de la prostate, le cancer de la vessie, la leucémie, le cancer gastrique, le cancer du foie, une tumeur stromale gastro-intestinale, le cancer de la thyroïde, la leucémie myéloïde chronique, la leucémie myéloïde aiguë, un lymphome non hodgkinien, le cancer du nasopharynx, le cancer de l'œsophage, une tumeur cérébrale, un lymphome à cellules B et T, le lymphome, le myélome multiple, le cancer et sarcome biliaires, le cholangiocarcinome, une maladie inflammatoire de l'intestin, la colite ulcéreuse, le décollement de la rétine, la rétinite pigmentaire, la dégénérescence maculaire, la pancréatite, la dermatite atopique, la spondyloarthrite, la goutte, le SoJIA, le syndrome de Sjögren, la sclérodermie systémique, le syndrome des antiphospholipides, la vascularite, l'arthrose, la stéatohépatite non alcoolique, la stéatohépatite alcoolique, l'hépatite auto-immune, une maladie hépatobiliaire auto-immune, la cholangite sclérosante primitive, la néphrite, la maladie coeliaque, l'ITP auto-immun, le rejet de greffe, une lésion d'ischémie-reperfusion d'organes solides, la sepsie, le syndrome de réponse inflammatoire systémique, l'accident vasculaire cérébral, l'infarctus du myocarde, la maladie de Huntington, la maladie d'Alzheimer, la maladie de Parkinson, les maladies allergiques, l'asthme, la dermatite atopique, la sclérose en plaques, le diabète de type I, la granulomatose de Wegener, la sarcoïdose pulmonaire, la maladie de Behçet, le syndrome fébrile associé à l'interleukine-1 converzyme, la maladie pulmonaire obstructive chronique, le syndrome périodique associé aux récepteurs du facteur de nécrose tumorale et la parodontite.

**14.** Composition pharmaceutique, comprenant un composé, les sels pharmaceutiquement acceptables, diastéréomères, énantiomères, ou solvates de celui-ci selon l'une des revendications 1 à 12, et un ou plusieurs supports ou excipients pharmaceutiquement acceptables.

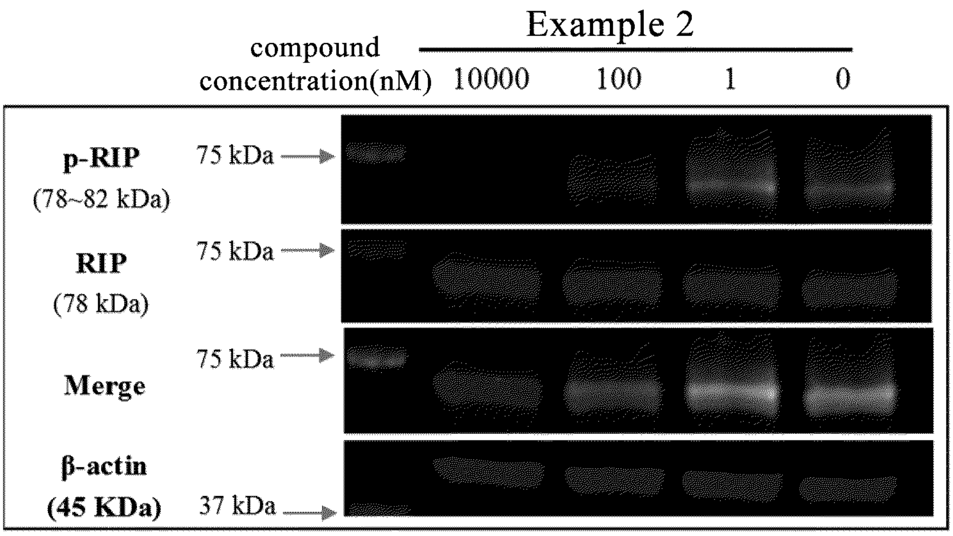


Figure 1

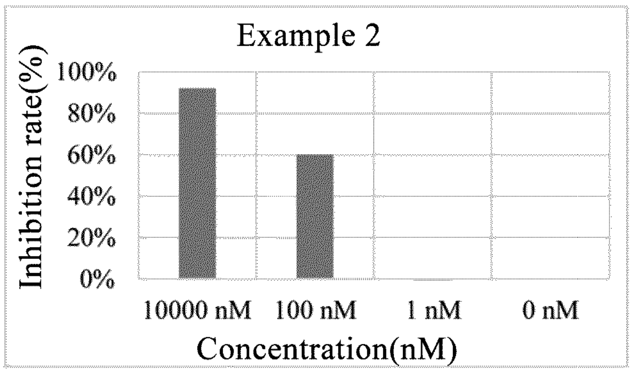


Figure 2

Compound concentration

4-fold dilution of compounds in DMSO( 15 µLcompound+ 45 µLDMSO)

| | | | | | | | | | | |
|---|---|---|---|---|---|---|---|---|---|---|
| 400× (µM) (DMSO:100%) | 10000 | 2500 | 625 | 156.25 | 39.0625 | 9.76563 | 2.44141 | 0.61035 | 0.15259 | 0 |

40-fold dilution (5 µLcompound+195 µL medium )

| | | | | | | | | | | |
|---|---|---|---|---|---|---|---|---|---|---|
| 10× (nM) (DMSO:2.5%) | 250000 | 62500 | 15625 | 3906.25 | 976.563 | 244.141 | 61.0352 | 15.2588 | 3.8147 | 0 |

10-fold dilution (10 µLcompound +90 µL medium )

| | | | | | | | | | | |
|---|---|---|---|---|---|---|---|---|---|---|
| 1× (nM) (DMSO:0.25%) | 25000 | 6250.000 | 1562.500 | 390.625 | 97.656 | 24.414 | 6.104 | 1.526 | 0.381 | 0 |

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018109097 A
- WO 2014125444 A
- WO 2017109724 A
- WO 2017136727 A
- WO 2018044783 A
- WO 2011025706 A
- WO 2005040152 A
- WO 2014125444 A1
- WO 2006018725 A, Gonzalez, Javier
- US 6437138 B, Lin, Nan-Homg


### Non-patent literature cited in the description


- **CLAUDIA BALDUCCI et al.** found that activated microglia plays an important role in the evolution of Alzheimer's disease. *Pharmacological Research.,* 2018, vol. 130, 402-413
- **M. V CHUDINOV et al.** *Bioorg. Med. Chem. Lett.,* 2016, vol. 26, 3223-3225